# EUROPEAN PATENT SPECIFICATION

(11) **EP 1 931 697 B1**
(45) Date of publication and mention of the grant of the patent: **15.09.2010**
(21) Application number: 06815733.8
(22) Date of filing: 28.09.2006
(51) Int. Cl.: C07K 19/00, C07K 14/715, C07K 16/28, A61K 38/17

(54) **IL-17A AND IL-17F ANTAGONISTS AND METHODS OF USING THE SAME**
IL-17A- UND IL-17F-ANTAGONISTEN UND VERWENDUNGSVERFAHREN
ANTAGONISTES ANTI IL-17A ET ANTI IL-17F ET LEURS METHODES D'UTILISATION

(30) Priority: 28.09.2005 US 721162 P; 22.12.2005 US 753794 P; 10.02.2006 US 772022 P; 14.03.2006 US 782247 P
(43) Date of publication of application: 18.06.2008
(73) Proprietor: ZymoGenetics, Inc., Seattle, Washington 98102 (US)
(72) Inventor: LEVIN, Steven, D., Seattle WA 98177 (US); RIXON, Mark, W., Issaquah WA 98029 (US); GAO, Zeren, Shanghai 201203 (CN)
(74) Representative: Tombling, Adrian George
(86) International application number: PCT/US2006/037950
(87) International publication number: WO 2007/038703

(56) References cited:
- WO-A2-02/058717
- WO-A2-2004/019866
- WO-A2-2005/010044
- WO-A2-2005/065711
- US-A1- 2005 147 609
- HAUDENSCHILD D ET AL: "Soluble and transmembrane isoforms of novel interleukin-17 receptor-like protein by RNA splicing and expression in prostate cancer" JOURNAL OF BIOLOGICAL CHEMISTRY, AMERICAN SOCIETY OF BIOLOCHEMICAL BIOLOGISTS, BIRMINGHAM,, US, vol. 277, no. 6, 8 February 2002 (2002-02-08), pages 4309-4316, XP002238284 ISSN: 0021-9258

## Description

### BACKGROUND OF THE INVENTION

Cytokines are soluble, small proteins that mediate a variety of biological effects, including the regulation of the growth and differentiation of many cell types (see, for example, Arai et al., Annu. Rev. Biochem. 59:783 (1990); Mosmann, Curr. Opin. Immunol. 3:311 (1991); Paul and Seder, Cell 76:241 (1994)). Proteins that constitute the cytokine group include interleukins, interferons, colony stimulating factors, tumor necrosis factors, and other regulatory molecules. For example, human interleukin-17 is a cytokine which stimulates the expression of interleukin-6, intracellular adhesion molecule 1, interleukin-8, granulocyte macrophage colony-stimulating factor, and prostaglandin E2 expression, and plays a role in the preferential maturation of CD34⁺ hematopoietic precursors into neutrophils (Yao et al., J. Immunol. 155:5483 (1995); Fossiez et al., J Exp. Med. 183:2593 (1996)).

Receptors that bind cytokines are typically composed of one or more integral membrane proteins that bind the cytokine with high affinity and transduce this binding event to the cell through the cytoplasmic portions of the certain receptor subunits. Cytokine receptors have been grouped into several classes on the basis of similarities in their extracellular ligand binding domains.

The demonstrated *in vivo* activities of cytokines and their receptors illustrate the clinical potential of, and need for, other cytokines, cytokine receptors, cytokine agonists, and cytokine antagonists. For example, demonstrated *in vivo* activities of the pro-inflammatory cytokine family illustrates the enormous clinical potential of, and need for antagonists of pro-inflammatory molecules. International Patent Application WO 2005/010044 discloses antibodies and small molecules for inhibiting IL-17A and IL-17F. International Patent Application WO 2005/065711 discloses chimeric IL-17 receptors and their ligands. However, there is no disclosure of a soluble IL-17C and IL-17F receptor that specifically binds to IL-17A and IL-17F.

### BRIEF DESCRI-PTION OF THE DRAWINGS

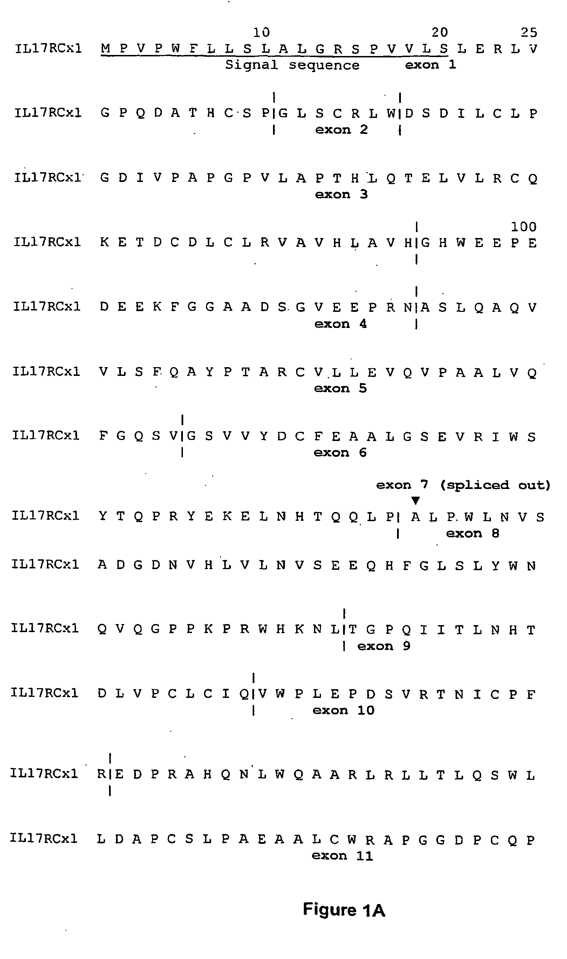

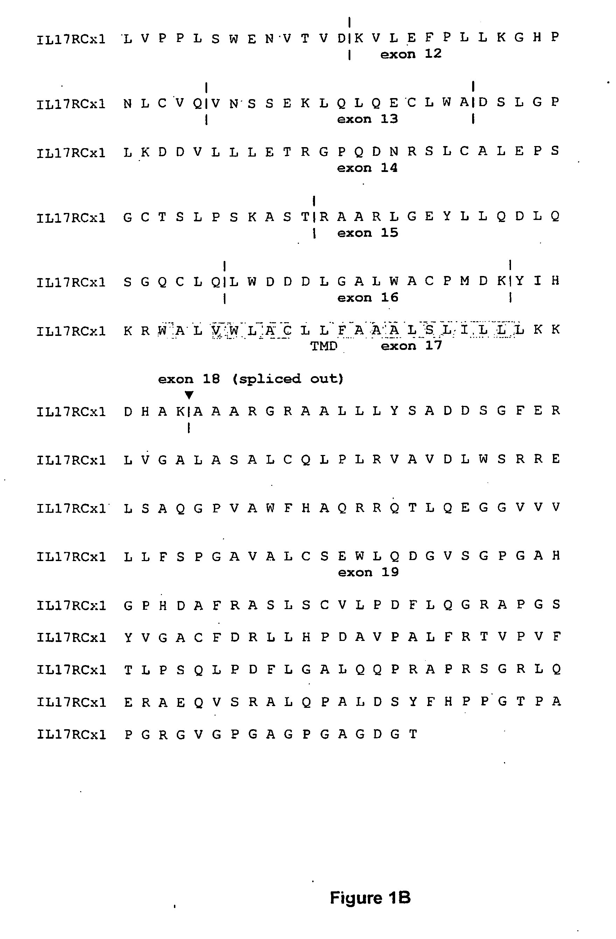

Figures 1A and 1B are graphic representations of the exon structure of human IL-17RCx1 (SEQ ID NO:2). For those amino acid where codon was spliced by exon/intron unction, the junction was moved to include the entire codon.

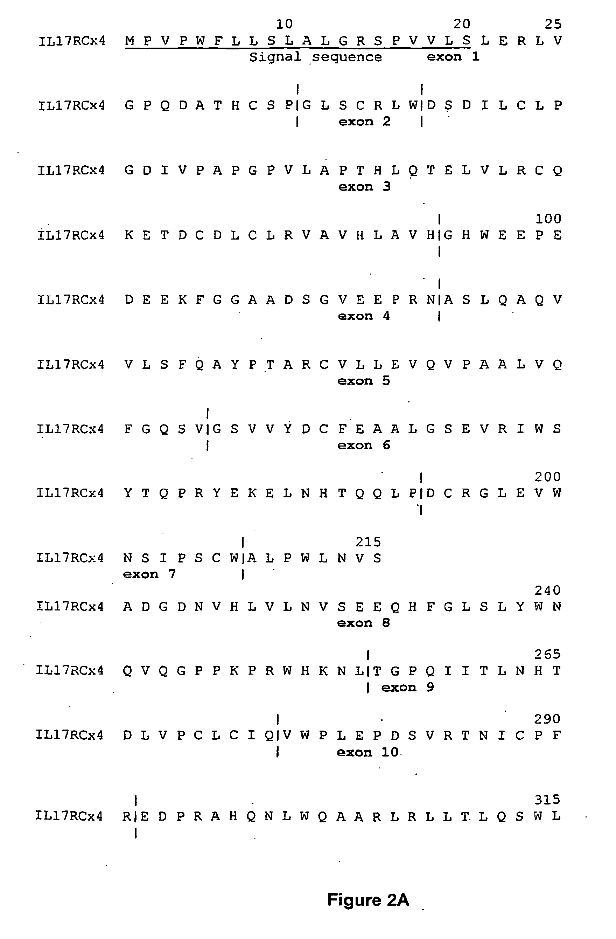

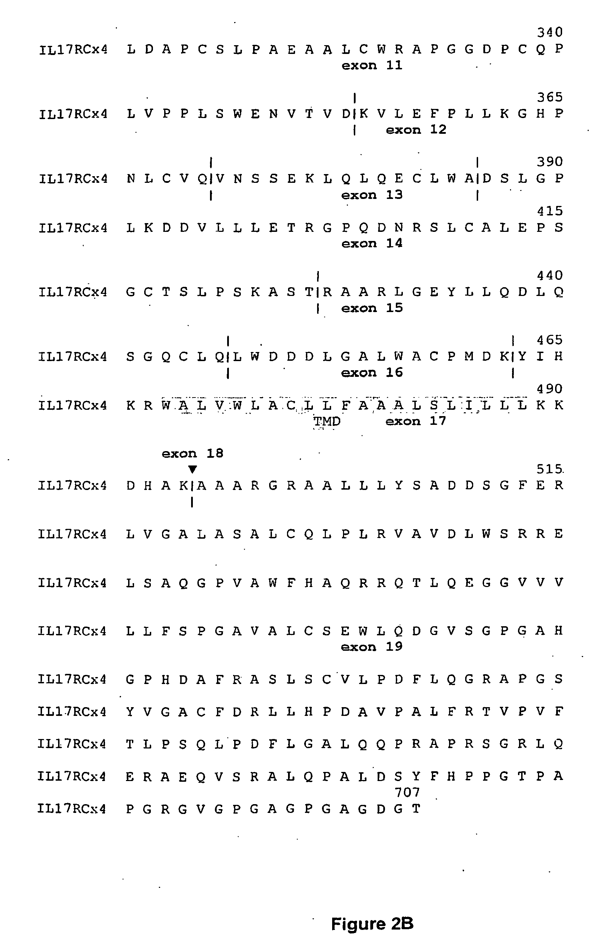

Figures 2A and 2B are graphic representations of the exon structure of human IL-17RCx4 (SEQ ID NO:166).

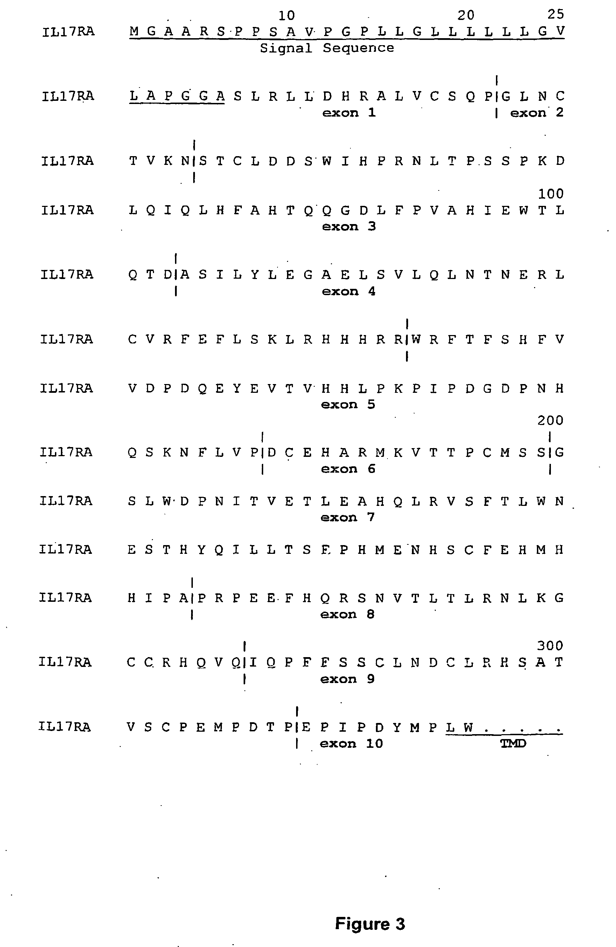

Figure 3 is a graphic representation of the exon structure of human IL-17RA (SEQ ID NO:21).

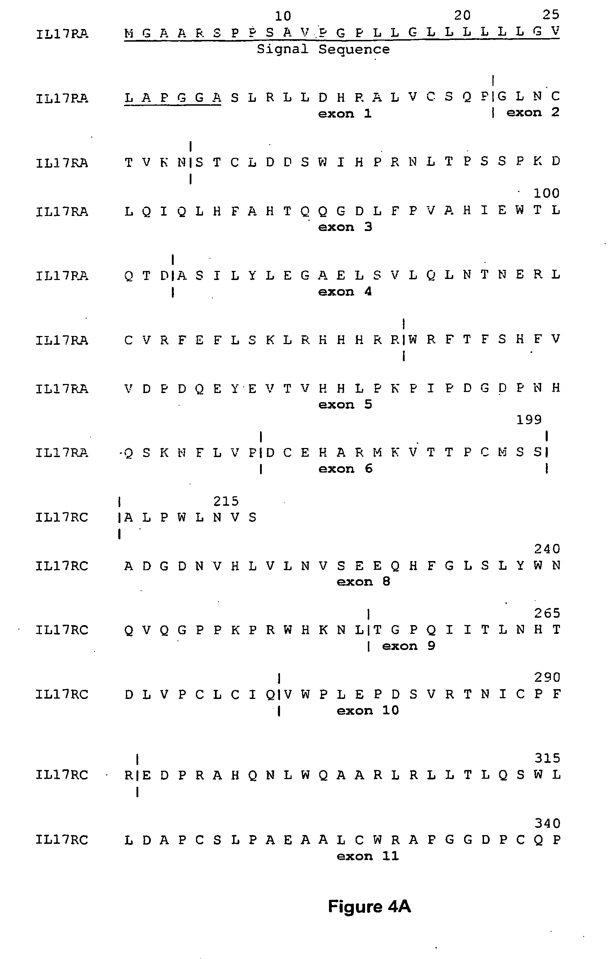

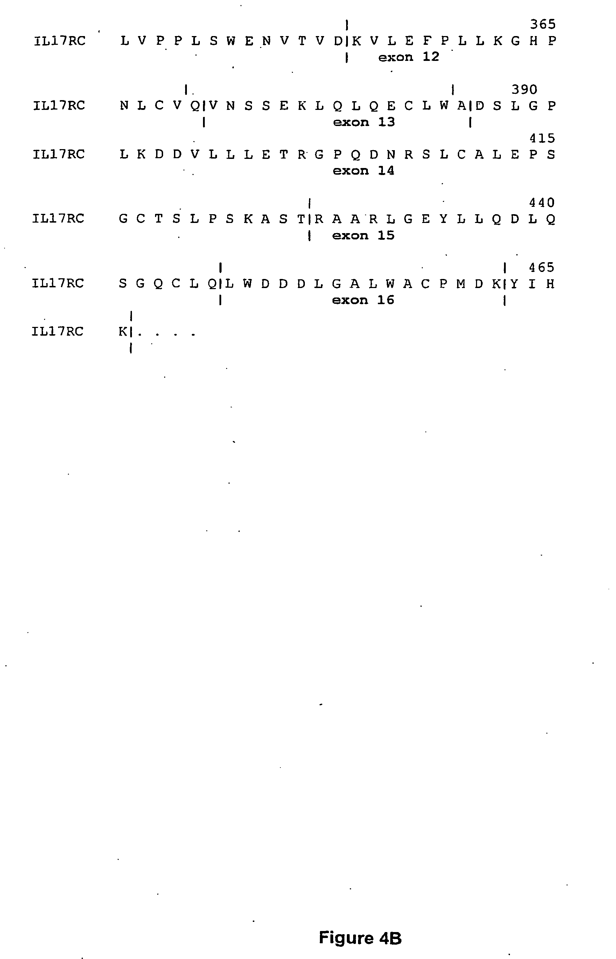

Figures 4A and 4B are graphic representations of the exon structure of a preferred soluble polypeptide of the present invention as described herein and in SEQ ID NOs:157 and 158. This soluble polypeptide comprises exons from both human IL-17RA (SEQ ID NO:21) and human IL-17RCx1 (SEQ ID NO:2).

Figure 5 is a graphical representation of a typical assay result using the protocol outlined in Example 34. The graph was generated using the Prizm software program. The Y values represent the MFI normalized to maximum and minimum (100% and 0%) based on ligand only and no ligand/no soluble receptor control wells, and thus the percent binding of the ligand to the cells. The software calculates the IC50 for each curve.

### DETAILED DESCRIPTION OF THE INVENTION

The present invention addresses these needs by providing antagonists to pro-inflammatory cytokines IL-17A and IL-17F. Specifically, the pro-inflammatory cytokines IL-17A and IL-17F have a high degree of sequence similarity, share many biological properties, and are both produced by activated T cells. They have both been implicated as factors that contribute to the progression of various autoimmune and inflammatory diseases including rheumatoid arthritis and asthma. In fact, reagents that negate IL-17A function significantly ameliorate disease incidence and severity in several mouse models of human disease. IL-17A mediates its effects through interaction with its cognate receptor, the IL-17 receptor (IL-17R), but the receptor for IL-17F had not yet been identified. Previously, we had reported that IL-17RC is a receptor for both IL-17A and IL-17F, and binds both with a similar high affinity. IL-17R on the other hand, binds IL-17A with high affinity, but binds IL-17F with very low affinity. Consistent with this, it has been shown that a soluble form of IL-17R blocks IL-17A binding and signaling in cells expressing either receptor, but does not interfere with binding or function of IL-17F to IL-17RC.

Since IL-17A intervention has been proposed as an effective therapy for several autoimmune diseases, using the antagonists of the present invention, which may block, inhibit, reduce, antagonize or neutralize the activity of both IL-17A and IL-17F, will have advantages over therapies that target only one of these two cytokines. The invention further provides uses therefor in inflammatory disease, as well as related compositions and methods.

### A) Overview

Immune related and inflammatory diseases are the manifestation or consequence of fairly complex, often multiple interconnected biological pathways which in normal physiology are critical to respond to insult or injury, initiate repair from insult or injury, and mount innate and acquired defense against foreign organisms. Disease or pathology occurs when these normal physiological pathways cause additional insult or injury either as directly related to the intensity of the response, as a consequence of abnormal regulation or excessive stimulation, as a reaction to self, or as a combination of these.

Though the genesis of these diseases often involves multi-step pathways and often multiple different biological systems/pathways, intervention at critical points in one or more of these pathways can have an ameliorative or therapeutic effect. Therapeutic intervention can occur by either antagonism of a detrimental process/pathway or stimulation of a beneficial process/pathway.

Many immune related diseases are known and have been extensively studied. Such diseases include immune-mediated inflammatory diseases (such as rheumatoid arthritis, immune mediated renal disease, hepatobiliary diseases, inflammatory bowel disease (IBD), psoriasis, and asthma), non-immune-mediated inflammatory diseases, infectious diseases, immunodeficiency diseases, neoplasia, etc.

T lymphocytes (T cells) are an important component of a mammalian immune response. T cells recognize antigens which are associated with a self-molecule encoded by genes within the major histocompatibility complex (MHC). The antigen may be displayed together with MHC molecules on the surface of antigen presenting cells, virus infected cells, cancer cells, grafts, etc. The T cell system eliminates these altered cells which pose a health threat to the host mammal. T cells include helper T cells and cytotoxic T cells. Helper T cells proliferate extensively following recognition of an antigen-MHC complex on an antigen presenting cell. Helper T cells also secrete a variety of cytokines, i.e., lymphokines, which play a central role in the activation of B cells, cytotoxic T cells and a variety of other cells which participate in the immune response.

A central event in both humoral and cell mediated immune responses is the activation and clonal expansion of helper T cells. Helper T cell activation is initiated by the interaction of the T cell receptor (TCR)-CD3 complex with an antigen-MHC on the surface of an antigen presenting cell. This interaction mediates a cascade of biochemical events that induce the resting helper T cell to enter a cell cycle (the G0 to G1 transition) and results in the expression of a high affinity receptor for IL-2 and sometimes IL-4. The activated T cell progresses through the cycle proliferating and differentiating into memory cells or effector cells.

In addition to the signals mediated through the TCR, activation of T cells involves additional costimulation induced by cytokines released by the antigen presenting cell or through interactions with membrane bound molecules on the antigen presenting cell and the T cell. The cytokine IL-1 and IL-6 have been shown to provide a costimulatory signal. Also, the interaction between the B7 molecule expressed on the surface of an antigen presenting cell and CD28 and CTLA-4 molecules expressed on the T cell surface effect T cell activation. Activated T cells express an increased number of cellular adhesion molecules, such as ICAM-1, integrins, VLA-4, LFA-1, CD56, etc.

T-cell proliferation in a mixed lymphocyte culture or mixed lymphocyte reaction (MLR) is an established indication of the ability of a compound to stimulate the immune system. In many immune responses, inflammatory cells infiltrate the site of injury or infection. The migrating cells may be neutrophilic, eosinophilic, monocytic or lymphocytic as can be determined by histologic examination of the affected tissues. Current Protocols in Immunology, ed. John E. Coligan, 1994, John Wiley & Sons, Inc.

Immune related diseases could be treated by suppressing the immune response. Using soluble receptors and/or neutralizing antibodies that inhibit molecules having immune stimulatory activity would be beneficial in the treatment of immune-mediated and inflammatory diseases. Molecules which inhibit the immune response can be utilized (proteins directly or via the use of antibody agonists) to inhibit the immune response and thus ameliorate immune related disease.

Interleukin-17 (IL-17A) has been identified as a cellular ortholog of a protein encoded by the T lymphotropic Herpes virus Saimiri (HSV) [see, Rouvier et al., J. Immunol., 150(12): 5445-5456 (19993); Yao et al., J. Immunol., 122(12):5483-5486 (1995) and Yao et al., Immunity, 3(6):811-821 (1995)]. Subsequent characterization has shown that this protein is a potent cytokine that acts to induce proinflammatory responses in a wide variety of peripheral tissues. IL-17A is a disulfide-linked homodimeric cytokine of about 32 kDa which is synthesized and secreted only by CD4+activated memory T cells (reviewed in Fossiez et al., Int. Rev. Immunol., 16: 541-551 [1998]). Specifically, IL-17 is synthesized as a precursor polypeptide of 155 amino acids with an N-terminal signal sequence of 19-33 residues and is secreted as a disulfide-linked homodimeric glycoprotein. Il-17A is disclosed in WO9518826 (1995), WO9715320 (1997) and WO9704097 (1997), as well as US Patent No. 6,063,372.

Despite its restricted tissue distribution, IL-17A exhibits pleitropic biological activities on various types of cells. IL-17A has been found to stimulate the production of many cytokines. It induces the secretion of IL-6, IL-8, IL-12, leukemia inhibitory factor (LIF), prostaglandin E2, MCP-1 and G-CSF by adherent cells like fibroblasts, keratinocytes, epithelial and endothelial cells. IL-17A also has the ability to induce ICAM-1 surface expression, proliferation of T cells, and growth and differentiation of CD34.sup.+ human progenitors into neutrophils. IL-17A has also been implicated in bone metabolism, and has been suggested to play an important role in pathological conditions characterized by the presence of activated T cells and TNF-.alpha. production such as rheumatoid arthritis and loosening of bone implants (Van Bezooijen et al., J. Bone Miner. Res. 14: 1513-1521 [1999]). Activated T cells of synovial tissue derived from rheumatoid arthritis patients were found to secrete higher amounts of IL-17A than those derived from normal individuals or osteoarthritis patients (Chabaud et al., Arthritis Rheum. 42: 963-970 [1999]). It was suggested that this proinflammatory cytokine actively contributes to synovial inflammation in rheumatoid arthritis. Apart from its proinflammatory role, IL-17A seems to contribute to the pathology of rheumatoid arthritis by yet another mechanism. For example, IL-17A has been shown to induce the expression of osteoclast differentiation factor (ODF) mRNA in osteoblasts (Kotake et al., J. Clin. Invest., 103: 1345-1352 [1999]). ODF stimulates differentiation of progenitor cells into osteoclasis, the cells involved in bone resorption.

Since the level of IL-17A is significantly increased in synovial fluid of rheumatoid arthritis patients, it appears that IL-17A induced osteoclast formation plays a crucial role in bone resorption in rheumatoid arthritis. IL-17A is also believed to play a key role in certain other autoimmune disorders such, as multiple sclerosis (Matusevicius et al., Mult. Scler., 5: 101-104 [1999]). IL-17A has further been shown, by intracellular signalling, to stimulate Ca.sup.2+ influx and a reduction in [cAMP], in human macrophages (Jovanovic et al., J. Immunol., 160:3513 [1998]). Fibroblasts treated with IL-17A induce the activation of NF-.kappa.B, [Yao et al., Immunity, 3:811 (1995), Jovanovic et al., supra], while macrophages treated with it activate NF-.kappa.B and mitogen-activated protein kinases (Shalom-Barek et al., J. Biol. Chem., 273:27467 [1998]).

Additionally, IL-17A also shares sequence similarity with mammalian cytokine-like factor 7 that is involved in bone and cartilage growth. Other proteins with which IL-17A polypeptides share sequence similarity are human embryo-derived interleukin-related factor (EDIRF) and interleukin-20.

Consistent with IL-17A's wide-range of effects, the cell surface receptor for IL-7A has been found to be widely expressed in many tissues and cell types (Yao et al., Cytokine, 9:794 [1997]). While the amino acid sequence of the human IL-17A receptor (IL-17R) (866 amino acids) predicts a protein with a single transmembrane domain and a long, 525 amino acid intracellular domain, the receptor sequence is unique and is not similar to that of any of the receptors from the cytokine/growth factor receptor family. This coupled with the lack of similarity of IL-17A itself to other known proteins indicates that IL-17A and its receptor may be part of a novel family of signalling proteins and receptors. It has been demonstrated that IL-17A activity is mediated through binding to its unique cell surface receptor, wherein previous studies have shown that contacting T cells with a soluble form of the IL-17A receptor polypeptide inhibited T cell proliferation and IL-2 production induce by PHA, concanavalin A and anti-TCR monoclonal antibody (Yao et al., J. Immunol., 155:5483-5486 [1995]). As such, there is significant interest in identifying and characterizing novel polypeptides having homology to the known cytokine receptors, specifically IL-17A receptors.

The expression pattern of IL-17F appears to be similar to that of IL-17A, such that it includes only activated CD4+ T cells and monocytes. (Starnes et al. J. Immunol. 167: 4137-4140 [2001]). IL-17F has been demonstrated to induce G-CSF, IL-6, and IL-8 in fibroblasts (Hymowitz et al, EMBO J. 20:5322-5341 [2001]) and TGF-b in endothelial cells (Starnes et al. J. Immunol. 167: 4137-4140 [2001]). It has recently been reported that IL-23, a cytokine produced by dendritic cell, can mediate the production of both IL-17A and IL-17F, primarily in memory T cells (Aggarwal et al. J. Biol. Chem. 278:1910-1914 [2003]).

Moreover, over expression or upregulation of both IL-17A and IL-17F have been shown in arthritic and asthmatic individuals (reviewed in Moseley et al. CytokineGrowth Factor Rev 14:155-174 [2003]). With regards to arthritis, these cytokines act in a manner characteristic to the cartilage and joint destruction that is associated with rheumatoid- and osteo-arthritis. For example, IL-117A and IL-17F have been demonstrated to enhance matrix degradation in articular cartilage explants via release of cartilage proteoglycan glycosaminoglycans and collagen fragments, while inhibiting the synthesis of new proteoglycans and collagens (Cai et al. Cytokine 16:10-21 [2001]; Attur et al Arthritis Rheum 44:2078-2083 [2001]).

Similar to IL-17A, overexpression of IL-17F in mice has also been shown to increase lung neutrophil recruitment and result in increased expression of Thl-associated cytokines in the lung, including IL-6, IFN-gamma, IP-10 and MIG (Starnes et al. J. Immunol. 167: 4137-4140 [2001]). IL-17F was also upregulated in T cells from allergen-challenged asthmatics (Kawaguchi et al J. Immunol 167:4430-4435 [2001]), and found to induce IL-6 and IL-8 production in NHBE. In contrast to IL-17A, IL-17F appears to inhibit angiogenesis in vitro (Starnes et al. J. Immunol. 167: 4137-4140 [2001]).

IL-7F mRNA was not detected by northern blot in various human tissues but was dramatically induced upon activation of CD4+ T cells and monocytes. Id. In mice, Th2 cells and mastr cells were found to express IL-17F upon activation. See Dumont, Expert Opin. Ther. Patents 13(3) (2003). Like IL-17A, the expression of IL-17F was also found to be upregulated by IL-23 in mouse.

The Il-17 cytokine/receptor families appear to represent a unique signaling system within the cytokine network that will offer innovative approaches to the manipulation of immune and inflammatory responses. Accordingly, the present invention is based on the discovery of a new IL-17 family receptor, IL-17RC and its ability to bind both IL-17A and IL-17F.

IL-17RC was initially identified using a bioinformatics approach to search for proteins related to IL-17RA and identified through a cDNA encoding the IL-17 receptor-related protein IL-17RC. In spite of its obvious similarity to the IL-17 receptor (IL-17RA), which binds to the prototypical member of the IL-17 family IL-17A, and the identification of five other members of the IL-17 cytokine family, a specific ligand for IL-17RC had not been previously reported. However, IL-17A and IL-17F were identified as the specific ligands for IL-17RC as described in US Patent Application No. 11/150,533, filed on June 10, 2005 and published as US Patent Publication No. 20060002925. Specifically, these ligands were identified using Baby Hamster Kidney cells (BHK) that were stably transfected with constructs encoding either human IL-17RA (hIL-17RA) or IL-17RC (hIL-17RC). Expression of receptors on the surface was confirmed by FACS analysis using either a monoclonal antibody to hIL-17RA or a polyclonal antiserum to hIL-17RC. To assess cytokine binding, biotinylated forms of human IL-17A, C, D, E, and F and fluorochrome-conjugated streptavidin were used to detect cytokine binding to transfected cells by flow cytometry. The results clearly showed that stably transfected BHK cells expressing hIL-17RA clearly bound human IL-17A (hIL-17A) as expected, whereas those transfected with empty expression vector failed to bind any members of the IL-17 family tested. Relatively weak binding of human IL-17F (hIL-17F) to hIL-17RA-transfected cells was also observed, but there was no significant binding of other members of the IL-17 family tested. Other IL-17 family members were examined for binding of to hIL-17RC-transfected cells and it was noted that these cells showed significant binding to hIL-17F. In addition, significant binding of hIL-17A to these cells was seen, but no binding of hIL-17C, D, or E. This data proved that hIL-17RC was the receptor for both hIL-17F and hIL-17A.

Additionally, the level of fluorescence over a range of cytokine concentrations was examined to determine relative affinities of hIL-17A and F for hIL-17RA and hIL-17RC. By comparing mean fluorescence intensities of the individual cytokines on each transfectant, it was noted that hIL-17A bound much better to hIL-17R.A than hIL-17F did, but that both cytokines seemed to bind equally well to hIL-17RC-transfected cells. Interestingly, cytokine binding to cells that expressed both receptors seemed to be additive, with no evidence of cooperativity.

Next, the specificity of this binding was investigated by attempting to compete for binding with unlabeled cytokine. Transfected BHK cells were incubated with a fixed concentration of biotinylated cytokine and increasing concentrations of unlabeled cytokine and the amount of bound biotinylated material was quantitated by FACS. It was shown that the binding of both hIL-17A and F to hIL-17RC was specific since increasing concentrations of unlabeled cytokine interfered with binding of the biotinylated material. In fact, unlabeled hIL-17A and F effectively cross-competed for binding of biotinylated forms of both cytokines to hIL-17RC-transfected cells, suggesting that the two cytokine were binding hIL-17RC with similar affinities, and that they were binding to overlapping, if not identical sites. Uunlabeled hIL-17A also effectively competed for binding of both biotinylated hIL-17A and F to hIL-17RA-transfected cells, while unlabeled hIL-17F showed essentially no ability to compete for hIL-17A binding to hIL-17RA. This indicated that although hIL-17F showed specific binding to hIL-17RA, the avidity of this interaction appeared to be significantly lower than the interaction of hIL-17A and hIL-17RA.

Saturation binding studies were done to measure the affinity of hIL-17A and F binding to hIL-17RC and hIL-17RA. BHK cell lines stably expressing hIL-17RA or hIL-17RC were incubated with iodinated hIL-17A or F under saturation binding conditions to determine the affinity constants of each cytokine for each receptor. hIL-17A bound both hIL-17RA and hIL-17RC with comparable affinities (Table 1). Specifically, BHK cells transfected with the indicated receptor were used to establish K_{d} values for hIL-17 A and hIL-17F as described in Methods. Results shown are mean K_{d} values derived from triplicate determinations.

**Table 1**

| | hIL-17A | hIL-17F |
|---|---|---|
| hIL-17RC (x1)¹ | 0.6 nM | 1.0 nM |
| hIL-17RA | 1.9 nM | 1.5 □M |

| | | |
|---|---|---|
| ¹Denotes the x1 splice variant of hIL-17RC. | | |

In addition, the affinity of hIL-17F for hIL-17RC was very similar to the affinity of hIL-17A for this receptor (see Table 1 above). However, consistent with results obtained using biotinylated cytokines, the affinity of hIL-17F for hIL-17RA was roughly 1000-fold lower relative to other affinities measured (*Id*.). This indicates that hIL-17A and F bind hIL-17RC with similar affinities, but their affinities for hIL-17RA differ dramatically.

The observation that hIL-17RC bound both hIL-17A and F with high affinity suggests that cells expressing hIL-17RC should be equally capable of responding to hIL-17A and F. On the other hand, since hIL-17RA bound hIL-17A with high affinity, but hIL-17F about 1000-fold less well, the implication is that cells expressing hIL-17RA would, under physiologic conditions, only respond to hIL-17A. Previously, it had been shown that hIL-17RA is expressed ubiquitously, but its expression has been reported to be higher in hematopoietic cells with lower expression in other tissues. Therefore, the expression of hIL-17RC was examined to determine the extent of overlap in the expression patterns. Northern blot analysis showed that hIL-17RC was expressed at high levels in glandular tissues such as adrenal gland, prostate, liver, and thyroid with no detectable expression in hematopoietic tissues.

To further investigate expression of these receptors in hematopoietic cells, the binding of biotinylated hIL-17A and F to peripheral blood mononuclear cells (PBMC) by multiparameter FACS analysis was also examined. Results indicated that hIL-17A bound to virtually all PBMC subsets examined, whereas hIL-17F failed to show detectable binding to any of these populations. This is consistent with the capacity of hIL-17RA to bind hIL-17A with high affinity, but not hIL-17F, and with the to detect hIL-17RC mRNA in PBMC. Collectively, these data indicate that IL-17RC is preferentially expressed in non-hematopoietic tissues, while IL-17RA is preferentially expressed in hematopoietic cells.

The high affinity binding of hIL-17A and F to hIL-17RC-transfected cells suggests that an efficiaous therapeutic might be a soluble form of hIL-17RC. Such a molecule would be an effective antagonist of these two cytokines. To test this directly, a soluble form of human hIL-17RC was produced as an Fc-fusion protein and tested its ability to inhibit the binding of both hIL-17A and F. These effects were then compared with results obtained using a soluble form of hIL-17RA. Increasing concentrations of hIL-17RC-Ig or hIL-17RA-Ig were included in binding reactions and FACS analysis was used to assess effects of the soluble receptors on binding of biotinylated cytokines to stably transfected BHK cells. Soluble hIL-17RC inhibited the binding of both hIL-17A and F to a similar extent, whereas an Fc-fusion protein of another member of the IL-17R family, hIL-17RD, had no effect. On the other hand, soluble hIL-17RA effectively blocked binding of hIL-17A, but had essentially no effect on the binding of hIL-17F. Similar results were obtained examining binding of hIL-17A to hematopoietic cells. This binding was effectively blocked using hIL-17RA-Ig and hIL-17RC-Ig, but not hIL-17RD-Ig. These data are consistent with results obtained from affinity measurements and indicate that the soluble receptors are behaving the same as their membrane-anchored forms.

As an additional assessment of the capacity of the human hIL-17RC-Ig to bind to hIL-17A and F, the affinity of the soluble receptor for these cytokines was assessed using Biacore analysis. Soluble hIL-17RC bound to both hIL-17A and F with high affinity (Table 2), providing additional support for the idea of using this reagent as an antagonist for the effects of both hIL-17A and F in vivo. Specifically, soluble receptors were captured onto chips and binding experiments were performed as described below: ND = no detectable binding.

**Table 2**

| **hIL-17RC-Ig** | **kₐ (on-rate)** | **k_{d} (off-rate)** | **K_{D}** |
|---|---|---|---|
| **mIL17A** | | ND | |
| | | | |
| **mIL17F** | | ND | |
| | | | |
| **hIL17A** | 1.05E+06 | 4.90E-04 | 0.469nM |
| | 1.24E+06 | 4.38E-04 | 0.352nM |
| | | | |
| **hIL17F** | 9.91 E+05 | 4.31 E-04 | 0.435nM |
| | 1.11E+06 | 3.84E-04 | 0.346nM |

| **mL-17RA-Ig** | **kₐ (on-rate)** | **k_{d} (off-rate)** | **K_{D}** |
|---|---|---|---|
| **mIL17A** | 9.78E+05 | 6.79E-05 | 0.069nM |
| | 1.12E+06 | 7.99E-05 | 0.072nM |
| | | | |
| **mIL17F** | | ND | |

The number of splice variants in humans is much greater and therefore we performed our initial experiments on only a subset of these molecules. Those chosen for this analysis also differed in their inclusion or exclusion of exon 7, but, unlike the mouse, all splice variants incorporated all of exon 8. The cryptic splice acceptor found in the middle of the mouse exon 8 sequence is not present in human exon 8. However, the other splice variants tested either included or excluded hIL-17RC exon 12. These variants were designated hIL-17RCx1 (identical in exon composition to mouse x1 above), hIL-17RCx4 (identical in exon composition to mouse x4 above), hIL-17RCx2, and hIL-17RCx7. Again, these splice variants were transiently expressed in 293F cells and were tested for their ability to bind biotinylated mouse and human IL-17A and F and the results are summarized in Table 3.

**Table 3**

| | | **Exons¹** | | | **Cytokine Binding²** | | | |
|---|---|---|---|---|---|---|---|---|
| | Variant | 7 | 8 | 12 | hIL-17A | hIL-17F | mIL-17A | mIL-17F |
| Human | IL-17RCx4 | + | + | + | + | + | - | + |
| | IL-17RCx1 | - | + | + | + | + | - | - |
| | IL-17RCx2 | - | + | - | - | - | - | - |
| | IL-17RCx7 | + | + | - | - | - | - | - |

| | | | | | | | | |
|---|---|---|---|---|---|---|---|---|
| ¹Denotes exons completely included in transcript. ²(+) indicates a detectable, significant cytokine binding as assessed by a significant increase in fluorescence by FACS. (-) indicates no significant change in fluorescence. | | | | | | | | |

Consistent with the experiments presented earlier, hIL-17RCx1 bound to both hIL-17A and F, but did not bind to either mouse cytokine. hIL-17RCx4 also bound to both human cytokines, and like its mouse counterpart, it bound to mIL-17F, but not mIL-17A. hIL-17RCx2 and x7 failed to bind any of the four cytokines tested, although they were clearly expressed on the surface of transfected cells since a polyclonal antiserum against hIL-17RC stained CD8⁺ cells (data not shown). These binding results were faithfully recapitulated in stably transfected BHK cells as well. Collectively, these data support conclusions regarding essential portions of the IL-17RC protein required for binding to the human cytokines.

Numerous publications have implicated IL-17A and, to a lesser extent, IL-17F as contributing to disease progression and severity in mouse collagen-induced arthritis (CIA) and human rheumatoid arthritis. The expression of both mIL-17A and F in the joints or draining lymph nodes (DLN) from mice that had been immunized with collagen to induce CIA was examined. Analysis by real-time PCR clearly demonstrated that both cytokines were upregulated in both tissues in diseased mice relative to unimmunized controls, clearly indicating that expression correlated with disease. In addition, the relative expression of mIL-17RA and mIL-17RC was also examined in the same tissues. However, in this case, there was not a reproducible correlation of expression of either receptor with disease. Moreover, what was obvious was the discrepancy in expression comparing DLN to non-hematopoietic tissue (hind foot). Consistent with the previous results looking at expression of the human receptors, mIL-17RA was found to be more highly expressed in hematopoietic tissue, and mIL-17RC to be more highly expressed in non-hematopoietic tissue. This data suggests that expression of mIL-17A and mIL-17F expression correlates with disease, that both of the requisite receptors are present in diseased and normal tissue, and suggests that neutralization of these cytokines may be an effective therapy to prevent disease progression.

Accordingly, the cognate receptor for IL-17A and F has been shown to be IL-17RC. Notably, hIL-17RC binds to hIL-17A and F with similar affinities. Since these two members of the IL-17 family share 55% sequence identity, it is perhaps not surprising that they share receptors. However, hIL-17RA binds hIL-17A with high affinity, but binds hIL-17F with an affinity that is nearly 1000-fold lower, suggesting that under physiologic conditions, hIL-lTRA would not bind hIL-17F. The implication is that cells that express hIL-17RC should respond to both hIL-17A and F, whereas cells that only express hIL-17RA will only respond to IL-17A. This difference has the potential to impact how these cytokines affect different tissues. Through expression analysis it was shown that although IL-17RA is expressed ubiquitously, it is more highly expressed in hematopoietic cells, whereas IL-17RC tends to be expressed in non-hematopoietic tissues with no expression in hematopoietic cells. Consistent with this, all subsets of human peripheral blood mononuclear cells bind hIL-17A, but do not bind hIL-17F. Moreover, this suggests that non-hematopoietic tissues should respond to both IL-17A and F, whereas hematopoietic cells should only respond to IL-17A.

This examination of cytokine binding to the different IL-17RC splice variants has revealed two portions of the receptor that are essential for cytokine binding, and there are subtle differences in the binding characteristics of the mouse and human cytokines. Moreover, these characteristics are consistent for the cytokines regardless of the species of the receptor examined. As shown from the data presented in Table 3, exon 12 and all of exon 8 are required for hIL-17A and F to bind to IL-17RC, since these cytokines only bind to the human x1 variants and the human x4 variants. Each of these isoforms includes all of exon 8 and exon 12, although they differ with respect to whether exon 7 is included or not. This implies that exon 7 is dispensable for binding of the human cytokines.

The importance of generating an antagonist to both IL-17A and IL-17F function seems clear from available information that shows a strong correlation between IL-17A and F expression and progression of a number of autoimmune and inflammatory diseases. These two cytokines induce other inflammatory cytokines and chemokines as well as matrix metalloproteases, which contribute to collagen and bone destruction in autoimmune arthritis. This reagent should serve as an effective therapeutic for rheumatoid arthritis and in other inflammatory diseases in which hL-17A and F play a role.

Thus, soluble forms of human IL-17RC were developed to serve as an antagonist to both IL-17A and IL-17F. Therapeutically, these soluble IL-17RC polypeptides were efficacious. However, due to numerous factors, soluble IL-17RC is not easily secreted from the numerous and varying production systems available in the art. Nor is it secreted in adequate quantities needed for manufacturing purposes. Thus, there is a need in the art to develop antagonists to IL-17A and IL-17F that can be expressed and secreted in quantities that can be scaled up for manufacturing.

Accordingly, the present invention answers this need by providing IL-17A and IL-17F antagonists that can be expressed and secreted. Specifically, the present invention is based on the development and discovery of an isolated soluble polypeptide comprising exons 8-16 of IL-17RC (amino acid residues 193-447 of SEQ ID NO:2) and exons 1-6 of IL-17RA, wherein said soluble polypeptide specifically binds to IL-17A and IL-17F.
As such, antagonists to IL-17F and IL-17A activity, such as the IL-17RC/IL-17RA soluble receptor of the present invention, are useful in therapeutic treatment of inflammatory diseases, particularly as antagonists to both IL-17F and IL-17A singly or together in the treatment of diseases involving these molecules. Moreover, antagonists to IL-17A and IL-17F activity are useful in therapeutic treatment of other inflammatory diseases for example as bind, block, inhibit, reduce, antagonize or neutralize IL-17F and IL-17A (either individually or together) in the treatment of psoriasis, contact dermatitis, IBD, IBS, colitis, arthritis, rheumatoid arthritis, asthma, chronic obstructive pulmonary disease (COPD), inflammatory bowel disease such as ulcerative colitis and Crohn's disease, multiple sclerosis, organ allograft rejection and kidney, lung, heart, etc. transplant rejection.

Cytokine receptors subunits are characterized by a multi-domain structure comprising a ligand-binding domain and an effector domain that is typically involved in signal transduction. Multimeric cytokine receptors include monomers, homodimers (*e*.*g*., PDGF receptor αα and ββ isoforms, erythropoietin receptor, MPL [thrombopoietin receptor], and G-CSF receptor), heterodimers whose subunits each have ligand-binding and effector domains (*e*.*g*., PDGF receptor αβ isoform), and multimers having component subunits with disparate functions (*e*.*g*., IL-2, IL-3, IL-4, IL-5, IL-6, IL-7, and GM-CSF receptors). Some receptor subunits are common to a plurality of receptors. For example, the AIC2B subunit, which cannot bind ligand on its own but includes an intracellular signal transduction domain, is a component of IL-3 and GM-CSF receptors. Many cytokine receptors can be placed into one of four related families on the basis of their structures and functions. Class I hematopoietic receptors, for example, are characterized by the presence of a domain containing conserved cysteine residues and the WSXWS motif. Additional domains, including protein kinase domains; fibronectin type III domains; and immunoglobulin domains, which are characterized by disulfide-bonded loops, are present in certain hematopoietic receptors. Cytokine receptor structure has been reviewed by Urdal, Ann. Reports Med. Chem. 26:221-228, 1991 and Cosman, Cytokine 5:95-106, 1993. It is generally believed that under selective pressure for organisms to acquire new biological functions, new receptor family members arose from duplication of existing receptor genes leading to the existence of multi-gene families. Family members thus contain vestiges of the ancestral gene, and these characteristic features can be exploited in the isolation and identification of additional family members.

Accordingly, the present invention is directed to an isolated soluble polypeptide comprising exons 8-16 of IL-17RC (amino acid residues 193-447 of SEQ ID NO:2) and exons 1-6 of IL-17RA, wherein said soluble polypeptide specifically binds to IL-17A and IL-17F.

The IL-17RC receptor has a large number of splice variants based on the inclusion or exclusion of specific exons. As described below, some of these exons are required for ligand (IL-17A and/or IL-17F) binding.

The present invention is based in part of the discovery of structural similarity ("domains") between IL-17RC and other members of the IL-17 family, such as IL-17RA (SEQ ID NO:21). Specifically, three domains were identified:
1) Domain 1 (SEQ ID NOs: 159 and 160) comprises exons 8-10 of IL-17RC. This corresponds to IL-17RCx1's amino acid residues 193-276 of (SEQ ID NO:2) and IL-17RCx4's amino acid residues 208-291 of (SEQ ID NO:166).
2) Domain 2 (SEQ ID NOs: 161 and 162) comprises exons 11-13 of IL-17RC. This corresponds to IL-17RCx1's amino acid residues 277-370 of (SEQ ID NO:2) and IL-17RCx4's amino acid residues 292-385 of (SEQ ID NO:166).
3) Domain 3 (SEQ ID NOs: 163 and 164) comprises exons 14-16 of IL-17RC. This corresponds to IL-17RCxl's amino acid residues 371-447 of (SEQ ID NO:2) and IL-17RCx4's amino acid residues 386-462 of (SEQ ID NO: 166).
The present invention also provides the soluble polypeptide of the present invention for use in human inflammatory and autoimmune diseases. The soluble polypeptide of the present invention can be used to block, inhibit, reduce, antagonize or neutralize the activity of both IL-17A and IL-17F in the treatment of inflammation and inflammatory dieases such as psoriasis, rheumatoid arthritis, IBD, IBS, colitis, asthma, allograft rejection, multiple sclerosis and other inflammatory conditions disclosed herein.

An illustrative nucleotide sequence that encodes human IL-17RC ("IL-17RCx1") is provided by SEQ ID NO:1; the encoded polypeptide is shown in SEQ ID NO:2. IL-17RC functions as a receptor for both IL-17A (SEQ ID NOs:13 and 14) and IL-17F (SEQ ID NOs:15 and 16). IL-17RC can act as a monomer, a homodimer or a heterodimer. As described in the present application, the soluble polypeptide of the present invention comprises IL-17RC and portions of IL-17RA ("IL-17RC/IL-17RA"). As such, the present invention encompasses soluble receptors that comprise portions of IL-17RC in combination with IL-17RA. IL-17RC is disclosed in commonly owned US Patent Application No. 10/458,647, and commonly owned WIPO publication WO 01/04304. Analysis of a human cDNA clone encoding IL-17RC (SEQ ID NO:1) revealed an open reading frame encoding 692 amino acids (SEQ ID NO:2) comprising a putative signal sequence of approximately 20 amino acid residues (amino acird residues 1 to 20 of SEQ ID NO:2), an extracellular ligand-binding domain of approximately 431 amino acid residues (amino acid residues 21-452 of SEQ ID NO:2; SEQ ID NO:3), a transmembrane domain of approximately 20 amino acid residues (amino acid residues 453-473 of SEQ ID NO:2), and an intracellular domain of approximately 203 amino acid residues (amino acid residues 474 to 677 of SEQ ID NO:2). Furthermore, a ligand binding domain is represented by SEQ ID NO:22.

Yet another illustrative nucleotide sequence that encodes a variant human IL-17RC, designated as "IL-17RCx4" is provided by SEQ ID NO:165, the encoded polypeptide is shown in SEQ ID NO:166. The predicted signal peptides is from residues 1-60 of SEQ ID NO:165 and 1-20 of SEQ ID NO:166; the extracellular domain from residues 61-1401 of SEQ ID NO:165 and 21-467 of SEQ ID NO:166; the transmembrane domain is from residues 1402-1464 of SEQ ID NO:165 and 468-488 of SEQ ID NO:166; and the intracellular domain is from residues 1465-2121 of SEQ ID NO:165 and 489-707 of SEQ ID NO: 166.

Yet another illustrative nucleotide sequence that encodes a variant human IL-17RC, designated as "IL-17RC-1" is provided by SEQ ID NO:4, the encoded polypeptide is shown in SEQ ID NO:5. IL-17RC-1 is disclosed in commonly owned US Patent Application No. 10/458,647, and commonly owned WIPO publication WO 01/04304.
Sequence analysis revealed that IL-17RC-1 is a truncated form of receptor polypeptide. That is, IL-17RC-1 lacks amino acid residues 1-113 of SEQ ID NO:2. SEQ ID NO:10 presents an amino acid sequence of a IL-17RC-1 polypeptide that includes the N-terminal portion of IL-17RC.

A comparison of the IL-17RC and IL-17RC-1 amino acid sequences also indicated that the two polypeptides represent alternatively spliced variants. The amino acid sequence of IL-17RC includes a 17 amino acid segment (amino acid residues 339 to 355 of SEQ ID NO:2), which IL-17RC-1 lacks, while IL-17RC lacks, following amino acid 479, a 13 amino acid segment found in IL-17RC-1 (amino acid residues 350 to 362 of SEQ ID NO:5). A polypeptide that contains both amino acid segments is provided by SEQ ID NO:11, whereas SEQ ID NO:12 presents the amino acid sequence of a polypeptide that lacks both 13 and 17 amino acid segments.

Yet another illustrative nucleotide sequence that encodes a variant human IL-17RC, designated as "IL-17RC-6" is provided by SEQ ID NO:23, the encoded polypeptide is shown in SEQ ID NO:24. IL-17RC-6 contains a 25 amino acid residue deletion as compared to IL-17RC as embodied in SEQ ID NO:2. Specifically, IL-17RC-6 does not contain amino acid residue 94 to amino acid residue 118 of SEQ ID NO:2. Analysis of a human cDNA clone encoding IL-17RC-6 (SEQ ID NO:23) revealed an extracellular ligand-binding domain of approximately 427 amino acid residues (amino acid residues 1-427 of SEQ ID NO:24), a transmembrane domain of approximately 20 amino acid residues (amino acid residues 428-448 of SEQ ID NO:24), and an intracellular domain of approximately 218 amino acid residues (amino acid residues 449 to 667 of SEQ NO:24).

An illustrative nucleotide sequence that encodes a variant murine IL-17RC is provided by SEQ ID NO:25; the encoded polypeptide is shown in SEQ ID NO:26. Murine IL-17RC functions as a receptor for both murine IL-17A (SEQ ID NOS:17 & 18) and murine IL-17F (SEQ ID NOS:19 & 20). Analysis of a murine cDNA clone encoding IL-17RC (SEQ ID NO:25) revealed an extracellular ligand-binding domain of approximately 449 amino acid residues SEQ ID NO:27). Furthermore, a ligand binding, domain is represented by SEQ ID NO:28.

Yet another illustrative nucleotide sequence that encodes a variant murine IL-17RC is provided by SEQ ID NO:29; the encoded polypeptide is shown in SEQ ID NO:30.

The IL-17RC gene resides in chromosome 3p25 - 3p24. As discussed below, this region is associated with various disorders and diseases.

Northern analyses indicate that there is strong expression of the IL-17RC gene in thyroid, adrenal gland, prostate, and liver tissues, and less expression in heart, small intestine, stomach, and trachea tissues. In contrast, there is little or no expression in brain, placenta, lung, skeletal muscle, kidney, pancreas, spleen, thymus, testis, ovary, colon, peripheral blood leukocytes, spinal cord, lymph node, and bone marrow. These observations show that IL-17RC sequences can be used differentiate between various tissues.
The present invention further includes isolated nucleic acid molecules that encode the isolated polypeptide of the present invention.

These and other aspects of the invention will become evident upon reference to the following detailed description. In addition, various references are identified below.

### B) Definitions

In the description that follows, a number of terms are used extensively. The following definitions are provided to facilitate understanding of the invention.

As used herein, "nucleic acid" or "nucleic acid-molecule" refers to polynucleotides, such as deoxyribonucleic acid (DNA) or ribonucleic acid (RNA), oligonucleotides, fragments generated by the polymerase chain reaction (PCR), and fragments generated by any of ligation, scission, endonuclease action, and exonuclease action. Nucleic acid molecules can be composed of monomers that are naturally-occurring nucleotides (such as DNA and RNA), or analogs of naturally-occurring nucleotides (*e.g*., α-enantiomeric forms of naturally-occurring nucleotides), or a combination of both. Modified nucleotides can have alterations in sugar moieties and/or in pyrimidine or purine base moieties. Sugar modifications include, for example, replacement of one or more hydroxyl groups with halogens, alkyl groups, amines, and azido groups, or sugars can be functionalized as ethers or esters. Moreover, the entire sugar moiety can be replaced with sterically and electronically similar structures, such as aza-sugars and carbocyclic sugar analogs. Examples of modifications in a base moiety include alkylated purines and pyrimidines, acylated purines or pyrimidines, or other well-known heterocyclic substitutes. Nucleic acid monomers can be linked by phosphodiester bonds or analogs of such linkages. Analogs of phosphodiester linkages include phosphorothioate, phosphorodithioate, phosphoroselenoate, phosphorodiselenoate, phosphoroanilothioate, phosphoranilidate, phosphoramidate, and the like. The term "nucleic acid molecule" also includes so-called "peptide nucleic acids," which comprise naturally-occurring or modified nucleic acid bases attached to a polyamide backbone. Nucleic acids can be either single stranded or double stranded.

The term "complement of a nucleic acid molecule" refers to a nucleic acid molecule having a complementary nucleotide sequence and reverse orientation as compared to a reference nucleotide sequence. For example, the sequence 5' ATGCACGGG 3' is complementary to 5' CCCGTGCAT 3'.

The term "degenerate nucleotide sequence" denotes a sequence of nucleotides that includes one or more degenerate codons as compared to a reference nucleic acid molecule that encodes a polypeptide. Degenerate codons contain different triplets of nucleotides, but encode the same amino acid residue (*i.e.*, GAU and GAC triplets each encode Asp).

The term "structural gene" refers to a nucleic acid molecule that is transcribed into messenger RNA (mRNA), which is then translated into a sequence of amino acids characteristic of a specific polypeptide.

An "isolated nucleic acid molecule" is a nucleic acid molecule that is not integrated in the genomic DNA of an organism. For example, a DNA molecule that encodes a growth factor that has been separated from the genomic DNA of a cell is an isolated DNA molecule. Another example of an isolated nucleic acid molecule is a chemically-synthesized nucleic acid molecule that is not integrated in the genome of an organism. A nucleic acid molecule that has been isolated from a particular species is smaller than the complete DNA molecule of a chromosome from that species.

A "nucleic acid molecule construct" is a nucleic acid molecule, either single- or double-stranded, that has been modified through human intervention to contain segments of nucleic acid combined and juxtaposed in an arrangement not existing in nature.

"Linear DNA" denotes non-circular DNA molecules having free 5' and 3' ends. Linear DNA can be prepared from closed circular DNA molecules, such as plasmids, by enzymatic digestion or physical disruption.

"Complementary DNA (cDNA)" is a single-stranded DNA molecule that is formed from an mRNA template by the enzyme reverse transcriptase. Typically, a primer complementary to portions of mRNA is employed for the initiation of reverse transcription. Those skilled in the art also use the term "cDNA" to refer to a double-stranded DNA molecule consisting of such a single-stranded DNA molecule and its complementary DNA strand. The term "cDNA" also refers to a clone of a cDNA molecule synthesized from an RNA template.

A "promoter" is a nucleotide sequence that directs the transcription of a structural gene. Typically, a promoter is located in the 5' non-coding region of a gene, proximal to the transcriptional start site of a structural gene. Sequence elements within promoters that function in the initiation of transcription are often characterized by consensus nucleotide sequences. These promoter elements include RNA polymerase binding sites, TATA sequences, CAAT sequences, differentiation-specific elements (DSEs; McGehee et al., Mol. Endocrinol. 7:551 (1993)), cyclic AMP response elements (CREs), serum response elements (SREs; Treisman, Seminars in Cancer Biol. 1:47 (1990)), glucocorticoid response elements (GREs), and binding sites for other transcription factors, such as CRE/ATF (O'Reilly et al., J. Biol. Chem. 267:19938 (1992)), AP2 (Ye et al., J. Biol. Chem. 269:25728 (1994)), SP1, cAMP response element binding protein (CREB; Loeken, Gene Expr. 3:253 (1993)) and octamer factors (see, in general, Watson et al., eds., Molecular Biology of the Gene, 4th ed.. (The Benjamin/Cummings Publishing Company, Inc. 1987), and Lemaigre and Rousseau, Biochem. J. 303:1 (1994)). If a promoter is an inducible promoter, then the rate of transcription increases in response to an inducing agent. In contrast, the rate of transcription is not regulated by an inducing agent if the promoter is a constitutive promoter. Repressible promoters are also known.

A "core promoter" contains essential nucleotide sequences for promoter function, including the TATA box and start of transcription. By this definition, a core promoter may or may not have detectable activity in the absence of specific sequences that may enhance the activity or confer tissue specific activity.

A "regulatory element" is a nucleotide sequence that modulates the activity of a core promoter. For example, a regulatory element may contain a nucleotide sequence that binds with cellular factors enabling transcription exclusively or preferentially in particular cells, tissues, or organelles. These types of regulatory elements are normally associated with genes that are expressed in a "cell-specific," "tissue-specific," or "organelle-specific" manner.

An "enhancer" is a type of regulatory element that can increase the efficiency of transcription, regardless of the distance or orientation of the enhancer relative to the start site of transcription.

"Heterologous DNA" refers to a DNA molecule, or a population of DNA molecules, that does not exist naturally within a given host cell. DNA molecules heterologous to a particular host cell may contain DNA derived from the host cell species (*i*.*e*., endogenous DNA) so long as that host DNA is combined with non-host DNA (*i*.*e*., exogenous DNA). For example, a DNA molecule containing a non-host DNA segment encoding a polypeptide operably linked to a host DNA segment comprising a transcription promoter is considered to be a heterologous DNA molecule. Conversely, a heterologous DNA molecule can comprise an endogenous gene operably linked with an exogenous promoter. As another illustration, a DNA molecule comprising a gene derived from a wild-type cell is considered to be heterologous DNA if that DNA molecule is introduced into a mutant cell that lacks the wild-type gene.

A "polypeptide" is a polymer of amino acid residues joined by peptide bonds, whether produced naturally or synthetically. Polypeptides of less than about 10 amino acid residues are commonly referred to as "peptides."

A "protein" is a macromolecule comprising one or more polypeptide chains. A protein may also comprise non-peptidic components, such as carbohydrate groups. Carbohydrates and other non-peptidic substituents may be added to a protein by the cell in which the protein is produced, and will vary with the type of cell. Proteins are defined herein in terms of their amino acid backbone structures; substituents such as carbohydrate groups are generally not specified, but may be present nonetheless.

A peptide or polypeptide encoded by a non-host DNA molecule is a "heterologous" peptide or polypeptide.

A. "cloning vector" is a nucleic acid molecule, such as a plasmid, cosmid, or bactetiophage, that has the capability of replicating autonomously in a host cell. Cloning vectors typically contain one or a small number of restriction endonuclease recognition sites that allow insertion of a nucleic acid molecule in a determinable fashion without loss of an essential biological function of the vector, as well as nucleotide sequences encoding a marker gene that is suitable for use in the identification and selection of cells transformed with the cloning vector. Marker genes typically include genes that provide tetracycline resistance or ampicillin resistance.

An "expression vector" is a nucleic acid molecule encoding a gene that is expressed in a host cell. Typically, an expression vector comprises a transcription promoter, a gene, and a transcription terminator. Gene expression is usually placed under the control of a promoter, and such a gene is said to be "operably linked to" the promoter. Similarly, a regulatory element and a core promoter are operably linked if the regulatory element modulates the activity of the core promoter.

A "recombinant host" is a cell that contains a heterologous nucleic acid molecule, such as a cloning vector or expression vector. In the present context, an example of a recombinant host is a cell that produces IL-17RC from an expression vector. In contrast, IL-17RC can be produced by a cell that is a "natural source" of IL-17RC, and that lacks an expression vector.

"Integrative transformants" are recombinant host cells, in which heterologous DNA has become integrated into the genomic DNA of the cells.

A "fusion protein" is a hybrid protein expressed by a nucleic acid molecule comprising nucleotide sequences of at least two genes. For example, a fusion protein can comprise at least part of a IL-17RC polypeptide fused with a polypeptide that binds an affinity matrix. Such a fusion protein provides a means to isolate large quantities of IL-17RC using affinity chromatography.

The term "receptor" denotes a cell-associated protein that binds to a bioactive molecule termed a "ligand." This interaction mediates the effect of the ligand on the cell. Receptors can be membrane bound, cytosolic or nuclear; monomeric (*e*.*g*., thyroid stimulating hormone receptor, beta-adrenergic receptor) or multimeric (*e*.*g*., PDGF receptor, growth hormone receptor, IL-3 receptor, GM-CSF receptor, G-CSF receptor, erythropoietin receptor and IL-6 receptor). Membrane-bound receptors are characterized by a multi-domain structure comprising an extracellular ligand-binding domain and an intracellular effector domain that is typically involved in signal transduction. In certain membrane-bound receptors, the extracellular ligand-binding domain and the intracellular effector domain are located in separate polypeptides that comprise the complete functional receptor.

In general, the binding of ligand to receptor results in a conformational change in the receptor that causes an interaction between the effector domain and other molecule(s) in the cell, which in turn leads to an alteration in the metabolism of the cell. Metabolic events that are often linked to receptor-ligand interactions include gene transcription, phosphorylation, dephosphorylation, increases in cyclic AMP production, mobilization of cellular calcium, mobilization of membrane lipids, cell adhesion, hydrolysis of inositol lipids and hydrolysis of phospholipids.

A "soluble receptor" is a receptor polypeptide that is not bound to a cell membrane. Soluble receptors are most commonly ligand-binding receptor polypeptides that lack transmembrane and cytoplasmic domains, and other linkage to the cell membrane such as via glycophosphoinositol (gpi). Soluble receptors can comprise additional amino acid residues, such as affinity tags that provide for purification of the polypeptide or provide sites for attachment of the polypeptide to a substrate, or immunoglobulin constant region sequences. Many cell-surface receptors have naturally occurring, soluble counterparts that are produced by proteolysis or translated from alternatively spliced mRNAs. Soluble receptors can be monomeric, homodimeric, heterodimeric, or multimeric, with multimeric receptors generally not comprising more than 9 subunits, preferably not comprising more than 6 subunits, and most preferably not comprising more than 3 subunits. Receptor polypeptides are said to be substantially free of transmembrane and intracellular polypeptide segments when they lack sufficient portions of these segments to provide membrane anchoring or signal transduction, respectively. Soluble receptors of cytokine receptors generally comprise the extracellular cytokine binding domain free of a transmsmbrane domain and intracellular domain. For example, representative soluble receptors include soluble receptors for IL-17RA as shown in SEQ ID NOs: 167 (polynucleotide) and 21 (polypeptide). It is well within the level of one of skill in the art to delineate what sequences of a known cytokine receptor sequence comprise the extracellular cytokine binding domain free of a transmsmbrane domain and intracellular domain. Moreover, one of skill in the art using the genetic code can readily determine polynucleotides that encode such soluble receptor polyptides.

The term "secretory signal sequence" denotes a DNA sequence that encodes a peptide (a "secretory peptide") that, as a component of a larger polypeptide, directs the larger polypeptide through a secretory pathway of a cell in which it is synthesized. The larger polypeptide is commonly cleaved to remove the secretory peptide during transit through the secretory pathway.

An "isolated polypeptide" is a polypeptide that is essentially free from contaminating cellular components, such as carbohydrate, lipid, or other proteinaceous impurities associated with the polypeptide in nature. Typically, a preparation of isolated polypeptide contains the polypeptide in a highly purified form, *i*.*e*., at least about 80% pure, at least about 90% pure, at least about 95% pure, greater than 95% pure, such as 96%, 97%, or 98% or more pure, or greater than 99% pure. One way to show that a particular protein preparation contains an isolated polypeptide is by the appearance of a single band following sodium dodecyl sulfate (SDS)-polyacrylamide gel electrophoresis of the protein preparation and Coomassie Brilliant Blue staining of the gel. However, the term "isolated" does not exclude the presence of the same polypeptide in alternative physical forms, such as dimers or alternatively glycosylated or derivatized forms.

The terms "amino-terminal" and "carboxyl-terminal" are used herein to denote positions within polypeptides. Where the context allows, these terms are used with reference to a particular sequence or portion of a polypeptide to denote proximity or relative position. For example, a certain sequence positioned carboxyl-terminal to a reference sequence within a polypeptide is located proximal to the carboxyl terminus of the reference sequence, but is not necessarily at the carboxyl terminus of the complete polypeptide.

The term "expression" refers to the biosynthesis of a gene product. For example, in the case of a structural gene, expression involves transcription of the structural gene into mRNA and the translation of mRNA into one or more polypeptides.

The term "splice variant" is used herein to denote alternative forms of RNA transcribed from a gene. Splice variation arises naturally through use of alternative splicing sites within a transcribed RNA molecule, or less commonly between separately transcribed RNA molecules, and may result in several mRNAs transcribed from the same gene. Splice variants may encode polypeptides having altered amino acid sequence. The term splice variant is also used herein to denote a polypeptide encoded by a splice variant of an mRNA transcribed from a gene.

As used herein, the term "immunomodulator" includes cytokines, stem cell growth factors, lymphotoxins, co-stimulatory molecules, hematopoietic factors, an dthe like, and synthetic analogs of these molecules.

The term "complement/anti-complement pair" denotes non-identical moieties that form a non-covalently associated, stable pair under appropriate conditions. For instance, biotin and avidin (or streptavidin) are prototypical members of a complement/anti-complement pair. Other exemplary complement/anti-complement pairs include receptor/ligand pairs, antibody/antigen (or hapten or epitope) pairs, sense/antisense polynucleotide pairs, and the like. Where subsequent dissociation of the complement/anti-complement pair is desirable, the complement/anti-complement pair preferably has a binding affinity of less than 10⁹ M⁻¹.

An "anti-idiotype antibody" is an antibody that binds with the variable region domain of an immunoglobulin. In the present context, an anti-idiotype antibody binds with the variable region of an anti-IL-17RC antibody, and thus, an anti-idiotype antibody mimics an epitope of IL-17RC.

An "antibody fragment" is a portion of an antibody such as F(ab')₂, F(ab)₂, Fab', Fab, and the like. Regardless of structure, an antibody fragment binds with the same antigen that is recognized by the intact antibody. For example, an anti-IL-17RC monoclonal antibody fragment binds with an epitope of IL-17RC.

The term "antibody fragment" also includes a synthetic or a genetically engineered polypeptide that binds to a specific antigen, such as polypeptides consisting of the light chain variable region, "Fv" fragments consisting of the variable regions of the heavy and light chains, recombinant single chain polypeptide molecules in which light and heavy variable regions are connected by a peptide linker ("scFv proteins"), and minimal recognition units consisting of the amino acid residues that mimic the hypervariable region.

A "chimeric antibody" is a recombinant protein that contains the variable domains and complementary determining regions derived from a rodent antibody, while the remainder of the antibody molecule is derived from a human antibody.

"Humanized antibodies" are recombinant proteins in which murine complementarity determining regions of a monoclonal antibody have been transferred from heavy and light variable chains of the murine immunoglobulin into a human variable domain. Construction of humanized antibodies for therapeutic use in humans that are derived from murine antibodies, such as those that bind to or neutralize a human protein, is within the skill of one in the art.

As used herein, a "therapeutic agent" is a molecule or atom which is conjugated to an antibody moiety to produce a conjugate which is useful for therapy. Examples of therapeutic agents include drugs; toxins, immunomodulators, chelators, boron compounds, photoactive agents or dyes, and radioisotopes.

A "detectable label" is a molecule or atom which can be conjugated to an antibody moiety to produce a molecule useful for diagnosis. Examples of detectable labels include chelators, photoactive agents, radioisotopes, fluorescent agents, paramagnetic ions, or other marker moieties.

The term "affinity tag" is used herein to denote a polypeptide segment that can be attached to a second polypeptide to provide for purification or detection of the second polypeptide or provide sites for attachment of the second polypeptide to a substrate. In principal, any peptide or protein for which an antibody or other specific binding agent is available can be used as an affinity tag. Affinity tags include a poly-histidine tract, protein A (Nilsson et al., EMBO J. 4:1075 (1985); Nilsson et al., Methods Enzymol. 198:3 (1991)), glutathione S transferase (Smith and Johnson, Gene 67:31 (1988)), Glu-Glu affinity tag (Grussenmeyer et al., Proc. Natl. Acad. Sci. USA 82:7952 (1985)), substance P, FLAG peptide (Hopp et al., Biotechnology 6:1204 (1988)), streptavidin binding peptide, or other antigenic epitope or binding domain. See, in general, Ford et al., Protein Expression and Purification 2:95 (1991). DNA molecules encoding affinity tags are available from commercial suppliers (*e*.*g*., Pharmacia Biotech, Piscataway, NJ).

A "naked antibody" is an entire antibody, as opposed to an antibody fragment, which is not conjugated with a therapeutic agent. Naked antibodies include both polyclonal and monoclonal antibodies, as well as certain recombinant antibodies, such as chimeric and humanized antibodies.

As used herein, the term "antibody component" includes both an entire antibody and an antibody fragment.

An "immunoconjugate" is a conjugate of an antibody component with a therapeutic agent or a detectable label.

As used herein, the term "antibody fusion protein" refers to a recombinant molecule that comprises an antibody component and a IL-17RC polypeptide component. Examples of an antibody fusion protein include a protein that comprises a IL-17RC extracellular domain, and either an Fc domain or an antigen-binding region.

A "target polypeptide" or a "target peptide" is an amino acid sequence that comprises at least one epitope, and that is expressed on a target cell, such as a tumor cell, or a cell that carries an infectious agent antigen. T cells recognize peptide epitopes presented by a major histocompatibility complex molecule to a target polypeptide or target peptide and typically lyse the target cell or recruit other immune cells to the site of the target cell, thereby killing the target cell.

An "antigenic peptide" is a peptide which will bind a major histocompatibility complex molecule to form an MHC-peptide complex which is recognized by a T cell, thereby inducing a cytotoxic lymphocyte response upon presentation to the T cell. Thus, antigenic peptides are capable of binding to an appropriate major histocompatibility complex molecule and inducing a cytotoxic T cells response; such as cell lysis or specific cytokine release against the target cell which binds or expresses the antigen: The antigenic peptide can be bound in the context of a class I or class II major histocompatibility complex molecule, on an antigen presenting cell or on a target cell.

In eukaryotes, RNA polymerase II catalyzes the transcription of a structural gene to produce mRNA. A nucleic acid molecule can be designed to contain an RNA polymerase II template in which the RNA transcript has a sequence that is complementary to that of a specific mRNA. The RNA transcript is termed an "anti-sense RNA" and a nucleic acid molecule that encodes the anti-sense RNA is termed an "anti-sense gene." Anti-sense RNA molecules are capable of binding to mRNA molecules, resulting in an inhibition of mRNA translation.

An "anti-sense oligonucleotide specific for IL-17RC" or a "IL-17RC anti-sense oligonucleotide" is an oligonucleotide having a sequence (a) capable of forming a stable triplex with a portion of the *IL-17RC* gene, or (b) capable of forming a stable duplex with a portion of an mRNA transcript of the *IL-17RC* gene.

A "ribozyme" is a nucleic acid molecule that contains a catalytic center. The term includes RNA enzymes, self-splicing RNAs, self-cleaving RNAs, and nucleic acid molecules that perform these catalytic functions. A nucleic acid molecule that encodes a ribozyme is termed a "ribozyme gene."

An "external guide sequence" is a nucleic acid molecule that directs the endogenous ribozyme, RNase P, to a particular species of intracellular mRNA, resulting in the cleavage of the mRNA by RNase P. A nucleic acid molecule that encodes an external guide sequence is termed an "external guide sequence gene."

The term "variant IL-17RC gene" refers to nucleic acid molecules that encode a polypeptide having an amino acid sequence that is a modification of SEQ ID NO:2. Such variants include naturally-occurring polymorphisms of IL-17RC genes, as well as synthetic genes that contain conservative amino acid substitutions of the amino acid sequence of SEQ ID NO:2. Additional variant forms of IL-17RC genes are nucleic acid molecules that contain insertions or deletions of the nucleotide sequences described herein. A variant IL-17RC gene can be identified, for example, by determining whether the gene hybridizes with a nucleic acid molecule having the nucleotide sequence of SEQ ID NO:1 OR SEQ ID NO:4, or its complement, under stringent conditions.

Alternatively, variant IL-17RC genes can be identified by sequence comparison. Two amino acid sequences have "100% amino acid sequence identity" if the amino acid residues of the two amino acid sequences are the same when aligned for maximal correspondence. Similarly, two nucleotide sequences have "100% nucleotide sequence identity" if the nucleotide residues of the two nucleotide sequences are the same when aligned for maximal correspondence. Sequence comparisons can be performed using standard software programs such as those included in the LASERGENE bioinformatics computing suite, which is produced by DNASTAR (Madison, Wisconsin). Other methods for comparing two nucleotide or amino acid sequences by determining optimal alignment are well-known to those of skill in the art (see, for example, Peruski and Peruski, The Internet and the New Biology: Tools for Genomic and Molecular Research (ASM Press, Inc. 1997), Wu et al. (eds.), "Information Superhighway and Computer Databases of Nucleic Acids and Proteins," in Methods in Gene Biotechnology, pages 123-151 (CRC Press, Inc. 1997), and Bishop (ed.), Guide to Human Genome Computing, 2nd Edition (Academic Press, Inc. 1998)). Particular methods for determining sequence identity are described below.

Regardless of the particular method used to identify a variant *IL*-17RC gene or variant IL-17RC polypeptide, a variant gene or polypeptide encoded by a variant gene may be functionally characterized the ability to bind specifically to an anti-IL-17RC antibody. A variant IL-17RC gene or variant IL-17RC polypeptide may also be functionally characterized the ability to bind to its ligand, for example; IL-17A and/or IL-17F, using a biological or biochemical assay described herein.

The term "allelic variant" is used herein to denote any of two or more alternative forms of a gene occupying the same chromosomal locus. Allelic variation arises naturally through mutation, and may result in phenotypic polymorphism within populations. Gene mutations can be silent (no change in the encoded polypeptide) or may encode polypeptides having altered amino acid sequence. The term allelic variant is also used herein to denote a protein encoded by an allelic variant of a gene.

The term "ortholog" denotes a polypeptide or protein obtained from one species that is the functional counterpart of a polypeptide or protein from a different species. Sequence differences among orthologs are the result of speciation.

"Paralogs" are distinct but structurally related proteins made by an organism. Paralogs are believed to arise through gene duplication. For example, α-globin, β-globin, and myoglobin are paralogs of each other.

The present invention includes functional fragments of IL-17RC genes. Within the context of this invention, a "functional fragment" of a IL-17RC gene refers to a nucleic acid molecule that encodes a portion of a IL-17RC polypeptide which is a domain described herein or at least specifically binds with an anti-IL-17RC antibody.

Due to the imprecision of standard analytical methods, molecular weights and lengths of polymers are understood to be approximate values. When such a value is expressed as "about" X or "approximately" X, the stated value of X will be understood to be accurate to ±10%.

### C) Production of IL-17RA and IL-17RC Polynucleotides or Genes

Nucleic acid molecules encoding a human IL-17RA or IL-17RC gene or polynucleotides encoding any of the soluble polypeptides of the present invention can be obtained by screening a human cDNA or genomic library using polynucleotide probes based upon SEQ ID NO:1, SEQ ID NO:4. These techniques are standard and well-established, and may be accomplished using cloning kits available by commercial suppliers. See, for example, Ausubel et al. (eds.), Short Protocols in Molecular Biology, 3rd Edition, John Wiley & Sons 1995; Wu et al., Methods in Gene Biotechnology, CRC Press, Inc. 1997; Aviv and Leder, Proc. Nat'l Acad. Sci. USA 69:1408 (1972); Huynh et al., "Constructing and Screening cDNA Libraries in λgt10 and λgt11," in DNA Cloning: A Practical Approach Vol. I, Glover (ed.), page 49 (IRL Press, 1985); Wu (1997) at pages 47-52.

Nucleic acid molecules that encode a human IL-17RA or IL-17RC gene can also be obtained using the polymerase chain reaction (PCR) with oligonucleotide primers having nucleotide sequences that are based upon the nucleotide sequences of the IL-17RA or IL-17RC gene or cDNA. General methods for screening libraries with PCR are provided by, for example, Yu et al., "Use of the Polymerase Chain Reaction to Screen Phage Libraries," in Methods in Molecular Biology, Vol. 15: PCR Protocols: Current Methods and Applications, White (ed.), Humana Press, Inc., 1993. Moreover, techniques for using PCR to isolate related genes are described by, for example, Preston, "Use of Degenerate Oligonucleotide Primers and the Polymerase Chain Reaction to Clone Gene Family Members," in Methods in Molecular Biology, Vol. 15: PCR Protocols: Current Methods and Applications, White (ed.), Humana Press, Inc. 1993. As an alternative, an IL-17RA or IL-17RC gene can be obtained by synthesizing nucleic acid molecules using mutually priming long oligonucleotides and the nucleotide sequences described herein (see, for example, Ausubel (1995)). Established techniques using the polymerase chain reaction provide the ability to synthesize DNA molecules at least two kilobases in length (Adang et al., Plant Molec. Biol. 21:1131 (1993), Bambot et al., PCR Methods and Applications 2:266 (1993), Dillon et al., "Use of the Polymerase Chain Reaction for the Rapid Construction of Synthetic Genes," in Methods in Molecular Biology, Vol. 15: PCR Protocols: Current Methods and Applications, White (ed.), pages 263-268, (Humana Press, Inc. 1993), and Holowachuk et al., PCR Methods Appl. 4:299 (1995)). For reviews on polynucleotide synthesis, see, for example, Glick and Pasternak, Molecular Biotechnology, Principles and Applications of Recombinant DNA (ASM Press 1994), Itakura et al., Annu. Rev. Biochem. 53:323 (1984), and Climie et al., Proc. Nat'l Acad. Sci. USA 87:633 (1990).

### D) Production of IL-17RA or IL-17RC Gene Variants

The present invention provides a variety of nucleic acid molecules, including DNA and RNA molecules, that encode the IL-17RA or IL-17RC polypeptides disclosed herein. Those skilled in the art will readily recognize that, in view of the degeneracy of the genetic code, considerable sequence variation is possible among these polynucleotide molecules. Moreover, the present invention also provides isolated soluble monomeric, homodimeric, heterodimeric and multimeric receptor polypeptides that comprise at least a portion of IL-17RC that is substantially homologous to the receptor polypeptide of SEQ ID NO:2. Thus, the present invention contemplates IL-17RA or IL-17RC polypeptide-encoding nucleic acid molecules comprising degenerate nucleotides of SEQ ID NO:1 or SEQ ID NO:4, and their RNA equivalents.

Those skilled in the art will readily recognize that, in view of the degeneracy of the genetic code, considerable sequence variation is possible among these polynucleotide molecules. SEQ ID NO:7 is a degenerate nucleotide sequence that encompasses all nucleic acid molecules that encode the IL-17RC polypeptide of SEQ ID NO:2. Those skilled in the art will recognize that the degenerate sequence of SEQ ID NO:7 also provides all RNA sequences encoding SEQ ID NO:2, by substituting U for T. Thus, the present invention contemplates IL-17RC polypeptide-encoding nucleic acid molecules comprising nucleotide 154 to nucleotide 2229 of SEQ ID NO:1, and their RNA equivalents. Similarly, the IL-17RC-1 degenerate sequence of SEQ ID NO:6 also provides all RNA sequences encoding SEQ ID NO:5, by substituting U for T.

Table 4 sets forth the one-letter codes to denote degenerate nucleotide positions. "Resolutions" are the nucleotides denoted by a code letter. "Complement" indicates the code for the complementary nucleotide(s). For example, the code Y denotes either C or T, and its complement R denotes A or G, A being complementary to T, and G being complementary to C.

**Table 4**

| Nucleotide | Resolution | Complement | Resolution |
|---|---|---|---|
| A | A | T | T |
| C | C | G | G |
| G | G | C | C |
| T | T | A | A |
| R | A\|G | Y | C\|T |
| Y | C\|T | R | A\|G |
| M | A\|C | K | G\|T |
| K | G\|T | M | A\|C |
| S | C\|G | S | C\|G |
| W | A\|T | W | A\|T |
| H | A\|C\|T | D | A\|G\|T |
| B | C\|G\|T | V | A\|C\|G |
| V | A\|C\|G | B | C\|G\|T |
| D | A\|G\|T | H | A\|C\|T |
| N | A\|C\|G\|T | N | A\|C\|G\|T |

The degenerate codons, encompassing all possible codons for a given amino acid, are set forth in Table 5.

**Table 5**

| Amino Acid | One Letter Code | Codons | Degenerate Codon |
|---|---|---|---|
| Cys | C | TGC TGT | TGY |
| Ser | S | AGC AGT TCA TCC TCG TCT | WSN |
| Thr | T | ACA ACG ACG ACT | ACN |
| Pro | P | CCA CCC CCG CCT | CCN |
| Ala | A | GCA GCC GCG GCT | GCN |
| Gly | G | GGA GGC GGG GGT | GGN |
| Asn | N | AAC AAT | AAY |
| Asp | D | GAC GAT | GAY |
| Glu | E | GAA GAG | GAR |
| Gln | Q | CAA CAG | CAR |
| His | H | CAC CAT | CAY |
| Arg | R | AGA AGG CGA CGC CGG CGT | MGN |
| Lys | K | AAA AAG | AAR |
| Met | M | ATG | ATG |
| Ile | I | ATA ATC ATT | ATH |
| Leu | L | CTA CTC CTG CTT TTA TTG | YTN |
| Val | V | GTA GTC GTG GTT | GTN |
| Phe | F | TTC TTT | TTY |
| Tyr | Y | TAC TAT | TAY |
| Trp | W | TGG | TGG |
| Ter | | TAA TAG TGA | TRR |
| Asn\|Asp | B | | RAY |
| Glu\|Gln | Z | | SAR |
| Any | X | | NNN |

One of ordinary skill in the art will appreciate that some ambiguity is introduced in determining a degenerate codon, representative of all possible codons encoding an amino acid. For example, the degenerate codon for serine (WSN) can, in some circumstances, encode arginine (AGR), and the degenerate codon for arginine (MGN) can, in some circumstances, encode serine (AGY). A similar relationship exists between codons encoding phenylalanine and leucine. Thus, some polynucleotides encompassed by the degenerate sequence may encode variant amino acid sequences, but one of ordinary skill in the art can easily identify such variant sequences by reference to the amino acid sequences of SEQ ID NO:6. Variant sequences can be readily tested for functionality as described herein.

Different species can exhibit "preferential codon usage." In general, see, Grantham et al., Nucl. Acids Res. 8:1893 (1980), Haas et al. Curr. Biol. 6:315 (1996), Wain-Hobson et al., Gene 13:355 (1981), Grosjean and Fiers, Gene 18:199 (1982), Holm, Nuc. Acids Res. 14:3075 (1986), Ikemura, J. Mol. Biol. 158:573 (1982), Sharp and Matassi, Curr. Opin. Genet. Dev. 4:851 (1994), Kane, Curr. Opin. Biotechnol. 6:494 (1995), and Makrides, Microbio/. Rev. 60:512 (1996). As used herein, the term "preferential codon usage" or "preferential codons" is a term of art referring to protein translation codons that are most frequently used in cells of a certain species, thus favoring one or a few representatives of the possible codons encoding each amino acid (See Table 5). For example, the amino acid threonine (Thr) may be encoded by ACA, ACC, ACG, or ACT, but in mammalian cells ACC is the most commonly used codon; in other species, for example, insect cells, yeast, viruses or bacteria, different Thr codons may be preferential. Preferential codons for a particular species can be introduced into the polynucleotides of the present invention by a variety of methods known in the art. Introduction of preferential codon sequences into recombinant DNA can, for example, enhance production of the protein by making protein translation more efficient within a particular cell type or species. Therefore, the degenerate codon sequences disclosed herein serve as a template for optimizing expression of polynucleotides in various cell types and species commonly used in the art and disclosed herein. Sequences containing preferential codons can be tested and optimized for expression in various species, and tested for functionality as disclosed herein.

An IL-17RA or IL-17RC-encoding cDNA can be isolated by a variety of methods, such as by probing with a complete or partial human cDNA or with one or more sets of degenerate probes based on the disclosed sequences. A cDNA can also be cloned using the polymerase chain reaction with primers designed from the representative human IL-17RA or IL-17RC sequences disclosed herein. In addition, a cDNA library can be used to transform or transfect host cells, and expression of the cDNA of interest can be detected with an antibody to IL-17RA or IL-17RC polypeptide.

Those skilled in the art will recognize that the sequence disclosed in SEQ ID NO:1 represents a single allele of human IL-17RC, and that allelic variation and alternative splicing are expected to occur. Allelic variants of this sequence can be cloned by probing DNA or genomic libraries from different individuals according to standard procedures. Allelic variants of the nucleotide sequences disclosed herein, including those containing silent mutations and those in which mutations result in amino acid sequence chages, are within the scope of the present invention, as are proteins which are allelic variants of the amino acid sequences disclosed herein. cDNA molecules generated from alternatively spliced mRNAs, which retain the properties of the IL-17RC polypeptide are included within the scope of the present invention, as are polypeptides encoded by such cDNAs and mRNAs. Allelic variants and splice variants of these sequences can be cloned by probing cDNA or genomic libraries from different individuals or tissues according to standard procedures known in the art.

Using the methods discussed above, one of ordinary skill in the art can prepare the polypeptide of the present invention.

### E) Production of IL-17RC, IL-17RA and IL-17RC/IL-17RA Polypeptides

The polypeptide of the present invention can be produced in recombinant host cells following conventional techniques. To express an IL-17RC/IL-17RA gene, a nucleic acid molecule encoding the polypeptide must be operably linked to regulatory sequences that control transcriptional expression in an expression vector and then, introduced into a host cell. In addition to transcriptional regulatory sequences, such as promoters and enhancers, expression vectors can include translational regulatory sequences and a marker gene which is suitable for selection of cells that carry the expression vector.

Expression vectors that are suitable for production of a foreign protein in eukaryotic cells typically contain (1) prokaryotic DNA elements coding for a bacterial replication origin and an antibiotic resistance marker to provide for the growth and selection of the expression vector in a bacterial host; (2) eukaryotic DNA elements that control initiation of transcription, such as a promoter; and (3) DNA elements that control the processing of transcripts, such as a transcription termination/polyadenylation sequence. As discussed above, expression vectors can also include nucleotide sequences encoding a secretory sequence that directs the heterologous polypeptide into the secretory pathway of a host cell. For example, an expression vector may comprise an IL-17RC/IL-17RA gene and a secretory sequence derived from any secreted gene.

The polypeptide of the present invention may be expressed in mammalian cells. Examples of suitable mammalian host cells include African green monkey kidney cells (Vero; ATCC CRL 1587), human embryonic kidney cells (293-HEK; ATCC CRL 1573), baby hamster kidney cells (BHK-21, BHK-570; ATCC CRL 8544, ATCC CRL 10314), canine kidney cells (MDCK; ATCC CCL 34), Chinese hamster ovary cells (CHO-K1; ATCC CCL61;
CHO DG44 (Chasin et al., Som. Cell. Molec. Genet. 12:555, 1986)), rat pituitary cells (GH1; ATCC CCL82), HeLa S3 cells (ATCC CCL2.2), rat hepatoma cells (H-4-II-E; ATCC CRL 1548) SV40-transformed monkey kidney cells (COS-1; ATCC CRL 1650) and murine embryonic cells (NIH-3T3; ATCC CRL 1658).

For a mammalian host, the transcriptional and translational regulatory signals may be derived from mammalian viral sources, for example, adenovirus, bovine papilloma virus, simian virus, or the like, in which the regulatory signals are associated with a particular gene which has a high level of expression. Suitable transcriptional and translational regulatory sequences also can be obtained from mammalian genes, for example, actin, collagen, myosin, and metallothionein genes.

Transcriptional regulatory sequences include a promoter region sufficient to direct the initiation of RNA synthesis. Suitable eukaryotic promoters include the promoter of the mouse *metallothionein I* gene (Hamer et al., J. Molec. Appl. Genet. 1:273 (1982)), the TK promoter of *Herpes* virus (McKnight, Cell 31:355 (1982)), the *SV40* early promoter (Benoist et al., Nature 290:304 (1981)), the *Rous* sarcoma virus promoter (Gorman et al., Proc. Nat'l Acad. Sci. USA 79:6777 (1982)), the cytomegalovirus promoter (Foecking et al., Gene 45:101 (1980)), and the mouse mammary tumor virus promoter (see, generally, Etcheverry, "Expression of Engineered Proteins in Mammalian Cell Culture," in Protein Engineering: Principles and Practice, Cleland et al. (eds.), pages 163-181 (John Wiley & Sons, Inc. 1996)).

Alternatively, a prokaryotic promoter, such as the bacteriophage T3 RNA polymerase promoter, can be used to control gene expression in mammalian cells if the prokaryotic promoter is regulated by a eukaryotic promoter (Zhou et al., Mol. Cell. Biol. 10:4529 (1990), and Kaufman et al., Nucl. Acids Res. 19:4485 (1991)).

In certain embodiments, a DNA sequence encoding the soluble receptor polypeptide is operably linked to other genetic elements required for its expression, generally including a transcription promoter and terminator, within an expression vector. The vector will also commonly contain one or more selectable markers and one or more origins of replication, although those skilled in the art will recognize that within certain systems selectable markers may be provided on separate vectors, and replication of the exogenous DNA may be provided by integration into the host cell genome. Selection of promoters, terminators, selectable markers, vectors and other elements is a matter of routine design within the level of ordinary skill in the art. Many such elements are described in the literature and are available through commercial suppliers. Multiple components of a soluble receptor complex can be co-transfected on individual expression vectors or be contained in a single expression vector. Such techniques of expressing multiple components of protein complexes are well known in the art.

An expression vector can be introduced into host cells using a variety of standard techniques including calcium phosphate transfection, liposome-mediated transfection, microprojectile-mediated delivery, electroporation, and the like. The transfected cells can be selected and propagated to provide recombinant host cells that comprise the expression vector stably integrated in the host cell genome. Techniques for introducing vectors into eukaryotic cells and techniques for selecting such stable transformants using a dominant selectable marker are described, for example, by Ausubel (1995) and by Murray (ed.), Gene Transfer and Expression Protocols (Human Press 1991).

For example, one suitable selectable marker is a gene that provides resistance to the antibiotic neomycin. In this case, selection is carried out in the presence of a neomycin-type drug, such as G-418 or the like. Selection systems can also be used to increase the expression level of the gene of interest, a process referred to as "amplification." Amplification is carried out by culturing transfectants in the presence of a low level of the selective agent and then increasing the amount of selective agent to select for cells that produce high levels of the products of the introduced genes. A suitable amplifiable selectable marker is dihydrofolate reductase (DHFR), which confers resistance to methotrexate. Other drug resistance genes (*e*.*g*., hygromycin resistance, multi-drug resistance, puromycin acetyltransferase) can also be used. Alternatively, markers that introduce an altered phenotype, such as green fluorescent protein, or cell surface proteins such as CD4, CD8, Class I MHC, placental alkaline phosphatase may be used to sort transfected cells from untransfected cells by such means as FACS sorting or magnetic bead separation technology.

The polypeptide of the invention can also be produced by cultured mammalian cells using a viral delivery system. Exemplary viruses for this purpose include adenovirus, retroviruses, herpesvirus, vaccinia virus and adeno-associated virus (AAV). Adenovirus, a double-stranded DNA virus, is currently the best studied gene transfer vector for delivery of heterologous nucleic acid (for a review, see Becker et al., Meth. Cell Biol. 43:161 (1994), and Douglas and Curiel, Science & Medicine 4:44 (1997)). Advantages of the adenovirus system include the accommodation of relatively large DNA inserts, the ability to grow to high-titer, the ability to infect a broad range of mammalian cell types, and flexibility that allows use with a large number of available vectors containing different promoters.

By deleting portions of the adenovirus genome, larger inserts (up to 7 kb) of heterologous DNA can be accommodated. These inserts can be incorporated into the viral DNA by direct ligation or by homologous recombination with a co-transfected plasmid. An option is to delete the essential *E1* gene from the viral vector, which results in the inability to replicate unless the *E1* gene is provided by the host cell. Adenovirus vector-infected human 293 cells (ATCC Nos. CRL-1573, 45504, 45505), for example, can be grown as adherent cells or in suspension culture at relatively high cell density to produce significant amounts of protein (see Garnier et al., Cytotechnol. 15:145 (1994)).

The polypeptide of the invention can also be expressed in other higher eukaryotic cells, such as avian, fungal, insect, yeast, or plant cells. The baculovirus system provides an efficient means to introduce cloned genes into insect cells. Suitable expression vectors are based upon the *Autographa californica* multiple nuclear polyhedrosis virus (AcMNPV), and contain well-known promoters such as *Drosophila heat shock protein* (*hsp*) 70 promoter, *Autographa californica nuclear polyhedrosis virus immediate-early* gene promoter (*ie-1*) and the *delayed early 39K* promoter, baculovirus *p10* promoter, and the *Drosophila metallothionein* promoter. A second method of making recombinant baculovirus utilizes a transposon-based system described by Luckow (Luckow, et al., J. Virol. 67:4566 (1993)). This system, which utilizes transfer vectors, is sold in the BAC-to-BAC kit (Life Technologies, Rockville, MD). This system utilizes a transfer vector, PFASTBAC (Life Technologies) containing a Tn7 transposon to move the DNA encoding a polypeptide into a baculovirus genome maintained in E. *coli* as a large plasmid called a "bacmid" See, Hill-Perkins and Possee, J. Gen. Virol. 71:971 (1990), Bonning, et al., J. Gen. Virol. 75:1551 (1994), and Chazenbalk, and Rapoport, J. Biol. Chem. 270:1543 (1995). In addition, transfer vectors can include an in-frame fusion with DNA encoding an epitope tag at the C- or N-terminus of the expressed the polypeptide, for example, a Glu-Glu epitope tag (Grussenmeyer et al., Proc. Nat'l Acad. Sci. 82:7952 (1985)). Using a technique known in the art, a transfer vector containing a gene encoding a polypeptide of the present invention is transformed into E. *coli,* and screened for bacmids which contain an interrupted *lacZ* gene indicative of recombinant baculovirus. The bacmid DNA containing the recombinant baculovirus genome is then isolated using common techniques.

The illustrative PFASTBAC vector can be modified to a considerable degree. For example, the polyhedrin promoter can be removed and substituted with the baculovirus basic protein promoter (also known as *Pcor,* p6.9 or MP promoter) which is expressed earlier in the baculovirus infection, and has been shown to be advantageous for expressing secreted proteins (see, for example, Hill-Perkins and Possee, J. Gen. Virol. 71:971 (1990), Bonning, et al., J. Gen. Virol. 75:1551 (1994), and Chazenbalk and Rapoport, J. Biol. Chem. 270:1543 (1995). In such transfer vector constructs, a short or long version of the basic protein promoter can be used. Moreover, transfer vectors can be constructed which replace the native secretory signal sequences with secretory signal sequences derived from insect proteins. For example, a secretory signal sequence from Ecdysteroid Glucosyltransferase (EGT), honey bee Melittin (Invitrogen Corporation; Carlsbad, CA), or baculovirus gp67 (PharMingen: San Diego, CA) can be used in constructs to replace the native IL-17RC secretory signal sequence.

The recombinant virus or bacmid is used to transfect host cells. Suitable insect host cells include cell lines derived from IPLB-*Sf*-21, a *Spodoptera frugiperda* pupal ovarian cell line, such as *Sf*9 (ATCC CRL 1711), *Sf*21AE, and *Sf*21 (Invitrogen Corporation; San Diego, CA), as well as *Drosophila* Schneider-2 cells, and the HIGH FIVEO cell line (Invitrogen) derived from *Trichoplusia ni* (U.S. Patent No. 5,300;435). Commercially available serum-free media can be used to grow and to maintain the cells. Suitable media are Sf900 II^{™} (Life Technologies) or ESF 921^{™} (Expression Systems) for the Sf9 cells; and Ex-cellO405™ (JRH Biosciences, Lenexa, KS) or Express FiveO™ (Life Technologies) for the *T. ni* cells. When recombinant virus is used, the cells are typically grown up from an inoculation density of approximately 2-5 x 10⁵ cells to a density of 1-2 x 10⁶ cells at which time a recombinant viral stock is added at a multiplicity of infection (MOI) of 0.1 to 10, more typically near 3.

Established techniques for producing recombinant proteins in baculovirus systems are provided by Bailey et al., "Manipulation of Baculovirus Vectors," in Methods in Molecular Biology, Volume 7: Gene Transfer and Expression Protocols, Murray (ed.), pages 147-168 (The Humana Press, Inc. 1991), by Patel et al., "The baculovirus expression system," in DNA Cloning 2: Expression Systems, 2nd Edition, Glover et al. (eds.), pages 205-244 (Oxford University Press 1995), by Ausubel (1995) at pages 16-37 to 16-57, by Richardson (ed.), Baculovirus Expression Protocols (The Humana Press, Inc. 1995), and by Lucknow, "Insect Cell Expression Technology," in Protein Engineering: Principles and Practice, Cleland et al. (eds.), pages 183-218 (John Wiley & Sons, Inc. 1996).

Fungal cells, including yeast cells, can also be used to express the genes described herein. Yeast species of particular interest in this regard include *Saccharomyces cerevisiae, Pichia pastoris,* and *Pichia methanolica.* Suitable promoters for expression in yeast include promoters from *GAL1* (galactose), *PGK* (phosphoglycerate kinase), ADH (alcohol dehydrogenase), *AOX1* (alcohol oxidase), HIS4 (histidinol dehydrogenase), and the like. Many yeast cloning vectors have been designed and are readily available. These vectors include YIp-based vectors, such as YIp5, YRp vectors, such as YRp 17, YEp vectors such as YEp13 and YCp vectors, such as YCp 19. Methods for transforming *S. cerevisiae* cells with exogenous DNA and producing recombinant polypeptides therefrom are disclosed by, for example, Kawasaki, U.S. Patent No. 4,599,311, Kawasaki et al., U.S. Patent No. 4,931,373, Brake, U.S. Patent No. 4,870,008, Welch et al., U.S. Patent No. 5,037,743, and Murray et al., U.S. Patent No. 4,845,075. Transformed cells are selected by phenotype determined by the selectable marker, commonly drug resistance or the ability to grow in the absence of a particular nutrient (*e*.*g*., leucine). A suitable vector system for use in *Saccharomyces cerevisiae* is the *POT1* vector system disclosed by Kawasaki et al. (U.S. Patent No. 4,931,373), which allows transformed cells to be selected by growth in glucose-containing media. Additional suitable promoters and terminators for use in yeast include those from glycolytic enzyme genes (see, *e*.*g*., Kawasaki, U.S. Patent No. 4,599,311, Kingsman et al., U.S. Patent No. 4,615,974, and Bitter, U.S. Patent No. 4,977,092) and alcohol dehydrogenase genes. See also U.S. Patents Nos. 4,990,446, 5,063,154, 5,139,936, and 4,661,454.

Transformation systems for other yeasts, including *Hansenula polymorpha, Schizosaccharomyces pombe, Kluyveromyces lactis, Kluyveromyces fragilis, Ustilago maydis, Pichia pastoris, Pichia methanolica, Pichia guillermondii* and *Candida maltosa* are known in the art. See, for example, Gleeson et al., J. Gen. Microbiol. 132:3459 (1986), and Cregg, U.S. Patent No. 4,882,279. *Aspergillus* cells may be utilized according to the methods of McKnight et al., U.S. Patent No. 4,935,349. Methods for transforming *Acremonium chrysogenum* are disclosed by Sumino et al., U.S. Patent No. 5,162,228. Methods for transforming *Neurospora* are disclosed by Lambowitz, U.S. Patent No. 4,486,533.

For example, the use of *Pichia methanolica* as host for the production of recombinant proteins is disclosed by Raymond, U.S. Patent No. 5,716,808, Raymond, U.S. Patent No. 5,736,383, Raymond et al., Yeast 14:11-23 (1998), and in international publication Nos. WO 97/17450, WO 97/17451, WO 98/02536, and WO 98/02565. DNA molecules for use in transforming *P. methanolica* will commonly be prepared as double-stranded, circular plasmids, which are preferably linearized prior to transformation. For polypeptide production in *P. methanolica,* the promoter and terminator in the plasmid can be that of a *P. methanolica* gene, such as a *P. methanolica* alcohol utilization gene (*AUG1* or *AUG2*). Other useful promoters include those of the dihydroxyacetone synthase (DHAS), formate dehydrogenase (FMD), and catalase (CAT) genes. To facilitate integration of the DNA into the host chromosome, it is preferred to have the entire expression segment of the plasmid flanked at both ends by host DNA sequences. A suitable selectable marker for use in *Pichia methanolica* is a *P. methanolica ADE2* gene, which encodes phosphoribosyl-5-aminoimidazole carboxylase (AIRC; EC 4.1.121), and which allows *ade2* host cells to grow in the absence of adenine. For large-scale, industrial processes where it is desirable to minimize the use of methanol, host cells can be used in which both methanol utilization genes (*AUG1* and *AUG2*) are deleted. For production of secreted proteins, host cells can be deficient in vacuolar protease genes (*PEP4* and *PRB1*). Electroporation is used to facilitate the introduction of a plasmid containing DNA encoding a polypeptide of interest into *P. methanolica* cells. *P. methanolica* cells can be transformed by electroporation using an exponentially decaying, pulsed electric field having a field strength of from 2.5 to 4.5 kV/cm, preferably about 3.75 kV/cm, and a time constant (t) of from 1 to 40 milliseconds, most preferably about 20 milliseconds.

Expression vectors can also be introduced into plant protoplasts, intact plant tissues, or isolated plant cells. Methods for introducing expression vectors into plant tissue include the direct infection or co-cultivation of plant tissue with *Agrobacterium tumefaciens,* microprojectile-mediated delivery, DNA injection, electroporation, and the like. See, for example, Horsch et al., Science 227:1229 (1985), Klein et al., Biotechnology 10:268 (1992), and Miki et al., "Procedures for Introducing Foreign DNA into Plants," in Methods in Plant Molecular Biology and Biotechnology, Glick et al. (eds.), pages 67-88 (CRC Press, 1993).

Alternatively, genes encoding the polypeptide of the present invention can be expressed in prokaryotic host cells. Suitable promoters that can be used to express the polypeptide in a prokaryotic host are well-known to those of skill in the art and include promoters capable of recognizing the T4, T3, Sp6 and T7 polymerases, the P_{R} and P_{L} promoters of bacteriophage lambda, the *trp, recA,* heat shock, *lacUV5, tac, lpp-lacSpr, phoA,* and *lacZ* promoters of E. *coli,* promoters of *B. subtilis,* the promoters of the bacteriophages of *Bacillus, Streptomyces* promoters, the *int* promoter of bacteriophage lambda, the *bla* promoter of pBR322, and the CAT promoter of the chloramphenicol acetyl transferase gene. Prokaryotic promoters have been reviewed by Glick, J. Ind. Microbiol. 1:277 (1987), Watson et al., Molecular Biology of the Gene, 4th Ed. (Benjamin Cummins 1987), and by Ausubel *et al.* (1995).

Suitable prokaryotic hosts include E. *coli* and *Bacillus subtilus.* Suitable strains of E. *coli* include BL21(DE3), BL21(DE3)pLysS, BL21(DE3)pLysE, DH1, DH4I, DH5, DH5I, DH5IF', DH5IMCR, DH10B, DH10B/p3, DH11S, C600, HB101, JM101, JM105, JM109, JM110, K38, RR1, Y1088, Y1089, CSH18, ER1451, and ER1647 (see, for example, Brown (ed.), Molecular Biology Labfax (Academic Press 1991)). Suitable strains of *Bacillus subtilus* include BR151, YB886, MI119, MI120, and B170 (see, for example, Hardy, "Bacillus Cloning Methods," in DNA Cloning: A Practical Approach, Glover (ed.) (IRL Press 1985)).

When expressing a polypeptide of the present invention in bacteria such as E. *coli,* the polypeptide may be retained in the cytoplasm, typically as insoluble granules, or may be directed to the periplasmic space by a bacterial secretion sequence. In the former case, the cells are lysed, and the granules are recovered and denatured using, for example, guanidine isothiocyanate or urea. The denatured polypeptide can then be refolded and dimerized by diluting the denaturant, such as by dialysis against a solution of urea and a combination of reduced and oxidized glutathione, followed by dialysis against a buffered saline solution. In the latter case, the polypeptide can be recovered from the periplasmic space in a soluble and functional form by disrupting the cells (by, for example, sonication or osmotic shock) to release the contents of the periplasmic space and recovering the protein, thereby obviating the need for denaturation and refolding.

Methods for expressing proteins in prokaryotic hosts are well-known to those of skill in the art (see, for example, Williams et al., "Expression of foreign proteins in E. coli using plasmid vectors and purification of specific polyclonal antibodies," in DNA Cloning 2: Expression Systems, 2nd Edition, Glover et al. (eds.), page 15 (Oxford University Press 1995), Ward et al., "Genetic Manipulation and Expression of Antibodies," in Monoclonal Antibodies: Principles and Applications, page 137 (Wiley-Liss, Inc. 1995), and Georgiou, "Expression of Proteins in Bacteria," in Protein Engineering: Principles and Practice, Cleland et al. (eds.), page 101 (John Wiley & Sons, Inc. 1996)).

Standard methods for introducing expression vectors into bacterial, yeast, insect, and plant cells are provided, for example, by Ausubel (1995).

General methods for expressing and recovering foreign protein produced by a mammalian cell system are provided by, for example, Etcheverry, "Expression of Engineered Proteins in Mammalian Cell Culture," in Protein Engineering: Principles and Practice, Cleland et al. (eds.), pages 163 (Wiley-Liss, Inc. 1996). Standard techniques for recovering protein produced by a bacterial system is provided by, for example, Grisshammer et al., "Purification of over-produced proteins from E. coli cells," in DNA Cloning 2: Expression Systems, 2nd Edition, Glover et al. (eds.), pages 59-92 (Oxford University Press 1995). Established methods for isolating recombinant proteins from a baculovirus system are described by Richardson (ed.), Baculovirus Expression Protocols (The Humana Press, Inc. 1995).

As an alternative, the polypeptide of the present invention can be synthesized by exclusive solid phase synthesis, partial solid phase methods, fragment condensation or classical solution synthesis. These synthesis methods are well-known to those of skill in the art (see, for example, Merrifield, J. Am. Chem. Soc. 85:2149 (1963), Stewart et al., "Solid Phase Peptide Synthesis" (2nd Edition), (Pierce Chemical Co. 1984), Bayer and Rapp, Chem. Pept. Prot. 3:3 (1986), Atherton et al., Solid Phase Peptide Synthesis: A Practical Approach (IRL Press 1989), Fields and Colowick, "Solid-Phase Peptide Synthesis," Methods in Enzymology Volume 289 ((Academic Press 1997), and Lloyd-Williams et al., Chemical Approaches to the Synthesis of Peptides and Proteins (CRC Press, Inc. 1997)). Variations in total chemical synthesis strategies, such as "native chemical ligation" and "expressed protein ligation" are also standard (see, for example, Dawson et al., Science 266:776 (1994), Hackeng et al., Proc. Nat'l Acad. Sci. USA 94:7845 (1997), Dawson, Methods Enzymol. 287: 34 (1997), Muir et al, Proc. Nat'l Acad. Sci. USA 95:6705 (1998), and Severinov and Muir, J. Biol. Chem. 273:16205 (1998)).

The present invention also contemplates chemically modified compositions, in which the polypeptide is linked with a polymer. Typically, the polymer is water soluble so that the conjugate does not precipitate in an aqueous environment, such as a physiological environment. An example of a suitable polymer is one that has been modified to have a single reactive group, such as an active ester for acylation, or an aldehyde for alkylation. In this way, the degree of polymerization can be controlled. An example of a reactive aldehyde is polyethylene glycol propionaldehyde, or mono-(C1-C10) alkoxy, or aryloxy derivatives thereof (see, for example, Harris, et al., U.S. Patent No. 5,252,714). The polymer may be branched or unbranched. Moreover, a mixture of polymers can be used to produce the conjugates.

The conjugates of the present invention used for therapy can comprise pharmaceutically acceptable water-soluble polymer moieties. Suitable water-soluble polymers include polyethylene glycol (PEG), monomethoxy-PEG, mono-(C1-C10)alkoxy-PEG, aryloxy-PEG, poly-(N-vinyl pyrrolidone)PEG, tresyl monomethoxy PEG, PEG propionaldehyde, *bis-*succinimidyl carbonate PEG, propylene glycol homopolymers, a polypropylene oxide/ethylene oxide co-polymer, polyoxyethylated polyols (*e*.*g*., glycerol), polyvinyl alcohol, dextran, cellulose, or other carbohydrate-based polymers. Suitable PEG may have a molecular weight from about 600 to about 60,000, including, for example, 5,000, 12,000, 20,000 and 25,000. The conjugate can also comprise a mixture of such water-soluble polymers.

One example of a conjugate comprises the polypeptide of the present invetion and a polyalkyl oxide moiety attached to the *N*-terminus of the polypeptide. PEG is one suitable polyalkyl oxide. As an illustration, the polypeptide can be modified with PEG, a process known as "PEGylation." PEGylation of the polypeptide can be carried out by any of the PEGylation reactions known in the art (see, for example, EP 0 154 316, Delgado et al., Critical Reviews in Therapeutic Drug Carrier Systems 9:249 (1992), Duncan and Spreafico, Clin. Pharmacokinet. 27:290 (1994), and Francis et al., Int J Hematol 68:1 (1998)). For example, PEGylation can be performed by an acylation reaction or by an alkylation reaction with a reactive polyethylene glycol molecule. In an alternative approach, conjugates are formed by condensing activated PEG, in which a terminal hydroxy or amino group of PEG has been replaced by an activated linker (see, for example, Karasiewicz et al., U.S. Patent No. 5,382,657).

PEGylation by acylation typically requires reacting an active ester derivative of PEG with polypeptide. An example of an activated PEG ester is PEG esterified to *N-*hydroxysuccinimide. As used herein, the term "acylation" includes the following types of linkages between the polypeptide and a water soluble polymer: amide, carbamate, urethane, and the like. Methods for preparing PEGylated IL-17RG/IL-17RA by acylation will typically comprise the steps of (a) reacting a IL-17RC/IL-17RA polypeptide with PEG (such as a reactive ester of an aldehyde derivative of PEG) under conditions whereby one or more PEG groups attach to IL-17RC/IL-17RA, and (b) obtaining the reaction product(s). Generally, the optimal reaction conditions for acylation reactions will be determined based upon known parameters and desired results. For example, the larger the ratio of PEG:IL-17RC/IL-17RA, the greater the percentage of polyPEGylated IL-17RC/IL-17RA product.

The product of PEGylation by acylation is typically a polyPEGylated product, wherein the lysine ε-amino groups are PEGylated via an acyl linking group. An example of a connecting linkage is an amide. Typically, the resulting IL-17RC/IL-17RA will be at least 95% mono-, di-, or tri-pegylated, although some species with higher degrees of PEGylation may be formed depending upon the reaction conditions. PEGylated species can be separated from unconjugated IL-17RC/IL-17RA polypeptides using standard purification methods, such as dialysis, ultrafiltration, ion exchange chromatography, affinity chromatography, and the like.

PEGylation by alkylation generally involves reacting a terminal aldehyde derivative of PEG with IL-17RC/1L-17RA in the presence of a reducing agent. PEG groups can be attached to the polypeptide via a -CH₂-NH group.

Derivatization via reductive alkylation to produce a monoPEGylated product takes advantage of the differential reactivity of different types of primary amino groups available for derivatization. Typically, the reaction is performed at a pH that allows one to take advantage of the pKa differences between the ε-amino groups of the lysine residues and the α-amino group of the *N-*terminal residue of the protein. By such selective derivatization, attachment of a water-soluble polymer that contains a reactive group such as an aldehyde, to a protein is controlled. The conjugation with the polymer occurs predominantly at the *N*-terminus of the protein without significant modification of other reactive groups such as the lysine side chain amino groups. The present invention provides a substantially homogenous preparation of IL-17RC/IL-17RA monopolymer conjugates.

Reductive alkylation to produce a substantially homogenous population of monopolymer IL-17RC/IL-17RA conjugate molecule can comprise the steps of: (a) reacting a IL, 17RC/IL-17RA polypeptide with a reactive PEG under reductive alkylation conditions at a pH suitable to permit selective modification of the α-amino group at the amino terminus of IL-17RC/IL-17RA, and (b) obtaining the reaction product(s). The reducing agent used for reductive alkylation should be stable in aqueous solution and able to reduce only the Schiff base formed in the initial process of reductive alkylation. Illustrative reducing agents include sodium borohydride, sodium cyanoborohydride, dimethylamine borane, trimethylamine borane, and pyridine borane.

For a substantially homogenous population of monopolymer IL-17RC/IL-17RA conjugates, the reductive alkylation reaction conditions are those that permit the selective attachment of the water-soluble polymer moiety to the *N*-terminus of IL-17RC/IL-17RA. Such reaction conditions generally provide for pKa differences between the lysine amino groups and the α-amino group at the *N*-terminus. The pH also affects the ratio of polymer to protein to be used. In general, if the pH is lower, a larger excess of polymer to protein will be desired because the less reactive the *N*-terminal α-group, the more polymer is needed to achieve optimal conditions. If the pH is higher, the polymer: IL-17RC/IL-17RA need not be as large because more reactive groups are available. Typically, the pH will fall within the range of 3 to 9, or 3 to 6. This method can be employed for making IL-17RC/IL-17RA-comprising homodimeric, heterodimeric or multimeric soluble receptor conjugates.

Another factor to consider is the molecular weight of the water-soluble polymer. Generally, the higher the molecular weight of the polymer, the fewer number of polymer molecules which may be attached to the protein. For PEGylation reactions, the typical molecular weight is about 2 kDa to about 100 kDa, about 5 kDa to about 50 kDa, or about 12 kDa to about 25 kDa. The molar ratio of water-soluble polymer to IL-17RC/IL-17RA will generally be in the range of 1:1 to 100:1. Typically, the molar ratio of water-soluble polymer to IL-17RC/IL-17RA will be 1:1 to 20:1 for polyPEGylation, and 1:1 to 5:1 for monoPEGylation.

General methods for producing conjugates comprising a polypeptide and water-soluble polymer moieties are known in the art. See, for example, Karasiewicz et al., U.S. Patent No. 5,382,657, Greenwald et al., U.S. Patent No. 5,738,846, Nieforth et al., Clin. Pharmacol. Ther. 59:636 (1996), Monkarsh et al., Anal. Biochem. 247:434 (1997)).

The present invention contemplates compositions comprising the polypeptide of the present invention. Such compositions can further comprise a carrier. The carrier can be a conventional organic or inorganic carrier. Examples of carriers include water, buffer solution, alcohol, propylene glycol, macrogol, sesame oil, corn oil, and the like..

### G) Isolation of IL-17RC or IL-17RC/IL-17RA Polypeptides

The polypeptide of the present invention can be purified to at least about 80% purity, to at least about 90% purity, to at least about 95% purity, or greater than 95%, such as 96%, 97%, 98%, or greater than 99% purity with respect to contaminating macromolecules, particularly other proteins and nucleic acids, and free of infectious and pyrogenic agents. The polypeptide of the present invention may also be purified to a pharmaceutically pure state, which is greater than 99.9% pure. In certain preparations, purified polypeptide is substantially free of other polypeptides, particularly other polypeptides of animal origin.

Fractionation and/or conventional purification methods can be used to obtain preparations of IL-17RC/IL-17RA purified from recombinant host cells. In general, ammonium sulfate precipitation and acid or chaotrope extraction may be used for fractionation of samples. Exemplary purification steps may include hydroxyapatite, size exclusion, FPLC and reverse-phase high performance liquid chromatography. Suitable chromatographic media include derivatized dextrans, agarose, cellulose, polyacrylamide, specialty silicas, and the like. PEI, DEAE, QAE and Q derivatives are suitable. Exemplary chromatographic media include those media derivatized with phenyl, butyl, or octyl groups, such as Phenyl-Sepbarose FF (Pharmacia), Toyopearl butyl 650 (Toso Haas, Montgomeryville, PA), Octyl-Sepharose (Pharmacia) and the like; or polyacrylic resins, such as Amberchrom CG 71 (Toso Haas) and the like. Suitable solid supports include glass beads, silica-based resins, cellulosic resins, agarose beads, cross-linked agarose beads, polystyrene beads, cross-linked polyacrylamide resins and the like that are insoluble under the conditions in which they are to be used. These supports may be modified with reactive groups that allow attachment of proteins by amino groups, carboxyl groups, sulfhydryl groups, hydroxyl groups and/or carbohydrate moieties.

Examples of coupling chemistries include cyanogen bromide activation, N-hydroxysuccinimide activation, epoxide activation, sulfhydryl activation, hydrazide activation, and carboxyl and amino derivatives for carbodiimide coupling chemistries. These and other solid media are well known and widely used in the art, and are available from commercial suppliers. Selection of a particular method for polypeptide isolation and purification is a matter of routine design and is determined in part by the properties of the chosen support. See, for example, Affinity Chromatography: Principles & Methods (Pharmacia LKB Biotechnology 1988), and Doonan, Protein Purification Protocols (The Humana Press 1996).

Additional variations in IL-17RC/IL-17RA isolation and purification can be devised by those of skill in the art.

The polypeptide of the present invention can also be isolated by exploitation of particular properties. For example, immobilized metal ion adsorption (IMAC) chromatography can be used to purify histidine-rich proteins, including those comprising polyhistidine tags. Briefly, a gel is first charged with divalent metal ions to form a chelate (Sulkowski, Trends in Biochem. 3:1 (1985)). Histidine-rich proteins will be adsorbed to this matrix with differing affinities, depending upon the metal ion used, and will be eluted by competitive elution, lowering the pH, or use of strong chelating agents. Other methods of purification include purification of glycosylated proteins by lectin affinity chromatography and ion exchange chromatography (M. Deutscher, (ed.), Meth. Enzymol. 182:529 (1990)). Within additional embodiments of the invention, a fusion of the polypeptide of interest and an affinity tag (*e.g.,* maltose-binding protein, an immunoglobulin domain) may be constructed to facilitate purification. Moreover, the ligand-binding properties of the soluble IL-17RC/IL-17RA polypeptide of the present invention can be exploited for purification, for example, of IL-17RC-comprising soluble receptors; for example, by using affinity chromatography wherein IL-17F ligand is bound to a column and the IL-17RC-comprising receptor is bound and subsequently eluted using standard chromatography methods.

IL-17RC/IL-17RA polypeptides or fragments thereof may also be prepared through chemical synthesis, as described above. These polypeptides may be monomers or multimers; glycosylated or non-glycosylated; PEGylated or non-PEGylated; and may or may not include an initial methionine amino acid residue.

### I) Therapeutic Uses of the IL-17RC/IL-17RA Polypeptide of the Invention

The polypeptide of the present invention can be used to modulate the immune system by binding ligands IL-17A and IL-17F (either singly or together), and thus, preventing the binding of these ligands with endogenous IL-17RC and/or IL-17RA receptor. The polypeptide of the present invention can also be used to modulate the immune system by inhibiting the binding of IL-17A and IL-17F with the endogenous IL-17RC and IL-17RA receptor. Accordingly, the present invention includes the use of the polypeptide of the present invention in administering to a subject which lacks an adequate amount of this polypeptide, or which produces an excess of IL-17A and/or IL-17F. The polypeptide of the present invention (*e.g*., IL-17RC/IL-17RA) can be also used to treat a subject which produces an excess of either IL-17A, IL-17F, IL-17RA or IL-17RC. Suitable subjects include mammals, such as humans. For example, such soluble polypeptides are useful in binding, blocking, inhibiting, reducing, antagonizing or neutralizing IL-17A and IL-17F (either singly or together), in the treatment of inflammation and inflammatory dieases such as psoriasis, rheumatoid arthritis, IBD, IBS, colitis, asthma, allograft rejection, multiple sclerosis and other inflammatory conditions disclosed herein.

The polypeptide of the present invention binds to, blocks, inhibits, reduces, antagonizes or neutralizes IL-17F and IL-17A (individually or together) *in vivo.*

Analysis of the tissue distribution of the mRNA corresponding IL-17RC cDNA showed that mRNA from the *IL-17RC* gene is strongly expressed in thyroid, adrenal gland, prostate, and liver tissues, and expressed to a lesser extent in heart, small intestine, stomach, and trachea tissues. In particular, IL-17RC is consistently expressed in non-T cell peripheral blood cell lines, including monocytes, B-cells, and cells of the myeloid lineage. Also, IL-17RC mRNA is reliably expressed in cell lines derived from skin. Other cell lines that express IL-17RC are all 5 of the large intestine cell lines that were present on the array. In contrast, there is little or no expression in brain, placenta, lung, skeletal muscle, kidney, pancreas, spleen, thymus, testis, ovary, colon, peripheral blood leukocytes, spinal cord, lymph node, and bone marrow. The ligand to which IL-17RC binds (IL-17F and/or IL-17A) is implicated in inducing inflammatory response and contributing to inflammatory diseases, primarily via its ability to enhance production of inflammatory mediators, including IL-1β, IL-6 and TNF-α, as well as those mediators that are involved in the proliferation, maturation and chemotaxis of neutrophils (reviewed in Witowski et al., Cell. Mol. Life Sci. 61:567-579 (2004)).

Thus, particular embodiments of the present invention are directed toward use of the soluble IL-17RC/IL-17RA polypeptide as antagonists in inflammatory and immune diseases or conditions such as psoriasis, inflammatory skin conditions, rheumatoid arthritis, IBD, IBS, Crohn's Disease, asthma, autoimmune disease, organ or bone marrow transplant; inflammation due to trauma, surgery or infection; graft versus host disease; and where inhibition of inflammation, immune suppression, reduction of proliferation of hematopoietic, immune, inflammatory or lymphoid cells, macrophages, T-cells (including Th1 and Th2 cells), suppression of immune response to a pathogen or antigen, or other instances where inhibition of IL-17F and/or IL-17A is desired.

Moreover, the soluble IL-17RC/IL-17RA polypeptide is useful to:

(1) Block, inhibit, reduce, antagonize or neutralize signaling via IL-17RA or IL-17RC in the treatment of acute inflammation, inflammation as a result of trauma, tissue injury, surgery, infection, and chronic inflammatory diseases such as asthma, inflammatory bowel disease (IBD), IBS, chronic colitis, rheumatoid arthritis, asthma, and other diseases associated with the induction of acute-phase response.

(2) Block, inhibit, reduce, antagonize or neutralize signaling IL-17RA or IL-17RC in the treatment of autoimmune diseases such as IDDM, multiple sclerosis (MS), rheumatoid arthritis, IBS and IBD to prevent or inhibit signaling in immune cells (*e*.*g*. lymphocytes, monocytes, leukocytes). Blocking, inhibiting, reducing, or antagonizing signaling via IL-17RC and/or IL-17RA, using the polypeptide of the present invention, may also benefit diseases of the pancreas, kidney, pituitary and neuronal cells.

(3) Agonize, enhance, increase or initiate signaling via IL-17RA or IL-17RC in the treatment of autoimmune diseases such as MS, rheumatoid arthritis, IBS and IBD. The soluble polypeptide of the present invention may signal lymphocytes or other immune cells to differentiate, alter proliferation, or change production of cytokines or cell surface proteins that ameliorate autoimmunity. Specifically, modulation of a T-helper cell response to an alternate pattern of cytokine secretion may deviate an autoimmune response to ameliorate disease (Smith JA et al., J. Immunol. 160:4841-4849, 1998). Similarly, agonistic soluble polypeptides may be used to signal, deplete and deviate immune cells involved in asthma, allergy and atopoic disease. Signaling via IL-17RC and/or IL-17RA may also benefit diseases of the pancreas, kidney, pituitary and neuronal cells.

The soluble IL-17RC/IL-17RA polypeptide described herein can be used to bind, block, inhibit, reduce, antagonize or neutralize IL-17F or IL-17A activity, either singly or together, in the treatment of autoimmune disease and atopic disease as described above. The soluble form of IL-17RC/IL-17RA may be used to promote an antibody response mediated by Th cells and/or to promote the production of IL-4 or other cytokines by lymphocytes or other immune cells.

The soluble polypeptide of the present invention is useful as an antagonist of IL-17A and/or IL-17F. Such antagonistic effects can be achieved by direct neutralization or binding of IL-17A or IL-17F. In addition to antagonistic uses, the soluble receptor of the present invention can bind IL-17F or IL-17A and act as carrier proteins for the ligand, in order to transport it to different tissues, organs, and cells within the body. As such, the soluble receptor of the present invention can be fused or coupled to molecules, polypeptides or chemical moieties that direct the soluble-receptor-Ligand complex to a specific site, such as a tissue, specific immune cell, or tumor. For example, in acute infection or some cancers, benefit may result from induction of inflammation and local acute phase response proteins by the action of IL-17F. Thus, the soluble receptors of the present invention can be used to specifically direct the action of IL-17A or IL-17F. See, Cosman, D. Cytokine 5: 95-106,1993; and Femandez-Botran, R. Exp. Onion. Invest. Drugs 9:497-513, 2000.

Inflammation is a protective response by an organism to fend off an invading agent. Inflammation is a cascading event that involves many cellular and humoral mediators. On one hand, suppression of inflammatory responses can leave a host immunocompromised; however, if left unchecked, inflammation can lead to serious complications including chronic inflammatory diseases (e.g., psoriasis, arthritis, rheumatoid arthritis, multiple sclerosis, inflammatory bowel disease and the like). Importantly, these diverse disease states share common inflammatory mediators. The collective diseases that are characterized by inflammation have a large impact on human morbidity and mortality. Therefore it is clear that anti-inflammatory proteins, such as the soluble polypeptide of the present invention could have crucial therapeutic potential for a vast number of human and animal diseases, from asthma and allergy to autoimmunity.

### 1. Arthritis

Arthritis, including osteoarthritis, rheumatoid arthritis, arthritic joints as a result of injury, and the like, are common inflammatory conditions which would benefit from the therapeutic use of anti-inflammatory proteins, such as the soluble polypeptides of the present invention. For example, rheumatoid arthritis (RA) is a systemic disease that affects the entire body and is one of the most common forms of arthritis. It is characterized by the inflammation of the membrane lining the joint, which causes pain, stiffness, warmth, redness and swelling. Inflammatory cells release enzymes that may digest bone and cartilage. As a result of rheumatoid arthritis, the inflamed joint lining, the synovium, can invade and damage bone and cartilage leading to joint deterioration and severe pain amongst other physiologic effects. The involved joint can lose its shape and alignment, resulting in pain and loss of movement.

Rheumatoid arthritis (RA) is an immune-mediated disease particularly characterized by inflammation and subsequent tissue damage leading to severe disability and increased mortality. A variety of cytokines are produced locally in the rheumatoid joints. Numerous studies have demonstrated that IL-1 and TNF-alpha, two prototypic pro-inflammatory cytokines, play an important role in the mechanisms involved in synovial inflammation and in progressive joint destruction. Indeed, the administration of TNF-alpha and IL-1 inhibitors in patients with RA has led to a dramatic improvement of clinical and biological signs of inflammation and a reduction of radiological signs of bone erosion and cartilage destruction. However, despite these encouraging results, a significant percentage of patients do not respond to these agents, suggesting that other mediators are also involved in the pathophysiology of arthritis (Gabay, Expert. Opin. Biol. Ther. 2(2):135-149. 2002). One of those mediators could be IL-17A or IL-17F, and as such a molecule that binds or inhibits IL-17F or IL-17A activity, such as soluble IL-17RC/IL-17RA, could serve as a valuable therapeutic to reduce inflammation in rheumatoid arthritis, and other arthritic diseases.

There are several animal models for rheumatoid arthritis known in the art. For example, in the collagen-induced arthritis (CIA) model, mice develop chronic inflammatory arthritis that closely resembles human rheumatoid arthritis. Since CIA shares similar immunological and pathological features with R.A, this makes it an ideal model for screening potential human anti-inflammatory compounds. The CIA model is a well-known model in mice that depends on both an immune response, and an inflammatory response, in order to occur. The immune response comprises the interaction of B-cells and CD4+ T-cells in response to collagen, which is given as antigen, and leads to the production of anti-collagen antibodies. The inflammatory phase is the result of tissue responses from mediators of inflammation, as a consequence of some of these antibodies cross-reacting to the mouse's native collagen and activating the complement cascade. An advantage in using the CIA model is that the basic mechanisms of pathogenesis are known. The relevant T-cell and B-cell epitopes on type II collagen have been identified, and various immunological (e.g., delayed-type hypersensitivity and anti-collagen antibody) and inflammatory (e.g., cytokines, chemokines, and matrix-degrading enzymes) parameters relating to immune-mediated arthritis have been determined, and can thus be used to assess test compound efficacy in the CIA model (Wooley, Curr. Opin. Rheum. 3:407-20, 1999; Williams et al., Immunol. 89:9784-788, 1992; Myers et al., Life Sci. 61:1861-78, 1997; and Wang et al., Immunol. 92:8955-959, 1995).

One group has shown that an anti-mouse IL-17 antibody reduces symptoms in a mouse CIA-model relative to control mice, thus showing conceptually that the soluble polypeptides of the present invention would be beneficial in treating human disease. The administration of a single mouse-IL-17-specific rat antisera reduced the symptoms of arthritis in the animals when introduced prophylactically or after symptoms of arthritis were already present in the model (Lubberts et al, Arthritis Rheum. 50:650-9, 2004). Therefore, IL-17RC/IL-17RA-Fc can be used to neutralize IL-17A and/or IL-17F in the treatment of specific human diseases such as arthritis, psoriasis, inflammatory bowel disease (IBD), IBS, and other inflammatory conditions disclosed herein.

The administration of the soluble polypeptide of the present invention, to these CIA model mice is used to evaluate its use as an antagonist to IL-17F and IL-17A to ameliorate symptoms and alter the course of disease. Moreover, results showing inhibition or neutralization of IL-17F and/or IL-17A by the soluble polypeptide of the present invention would provide proof of concept that other IL-17A or Il-17F antagonists can also be used to ameliorate symptoms and alter the course of disease. Furthermore, since IL-17A and/or IL-17F induces production of IL-16 and TNF-a, both of which are implicated in the pathogenesis and progression of rheumatoid arthritis, the systemic or local administration of these soluble polypeptides can potentially suppress the inflammatory response in RA. By way of example and without limitation, the injection of 10 - 200 ug IL-17RC-Fc per mouse (one to seven times a week for up to but not limited to 4 weeks via s.c., i.p., or i.m route of administration) can significantly reduce the disease score (paw score, incident of inflammation, or disease). Depending on the initiation of IL-17RC-Fc administration (e.g. prior to or at the time of collagen immunization, or at any time point following the second collagen immunization, including those time points at which the disease has already progressed), IL-17RC can be efficacious in preventing rheumatoid arthritis, as well as preventing its progression. Other potential therapeutics include IL-17RC/IL-17RA polypeptides, and the like.

### 2. Inflammatory Bowel Disease IBD

In the United States approximately 500,000 people suffer from Inflammatory Bowel Disease (IBD) which can affect either colon and rectum (Ulcerative colitis) or both, small and large intestine (Crohn's Disease). The pathogenesis of these diseases is unclear, but they involve chronic inflammation of the affected tissues. The soluble polypeptides of the present invention could serve as a valuable therapeutic to reduce inflammation and pathological effects in IBD, UC and related diseases.

Ulcerative colitis (UC) is an inflammatory disease of the large intestine, commonly called the colon, characterized by inflammation and ulceration of the mucosa or innermost lining of the colon. This inflammation causes the colon to empty frequently, resulting in diarrhea. Symptoms include loosening of the stool and associated abdominal cramping, fever and weight loss. Although the exact cause of UC is unknown, recent research suggests that the body's natural defenses are operating against proteins in the body which the body thinks are foreign (an "autoimmune reaction"). Perhaps because they resemble bacterial proteins in the gut, these proteins may either instigate or stimulate the inflammatory process that begins to destroy the lining of the colon. As the lining of the colon is destroyed, ulcers form releasing mucus, pus and blood. The disease usually begins in the rectal area and may eventually extend through the entire large bowel. Repeated episodes of inflammation lead to thickening of the wall of the intestine and rectum with scar tissue. Death of colon tissue or sepsis may occur with severe disease. The symptoms of ulcerative colitis vary in severity and their onset may be gradual or sudden. Attacks may be provoked by many factors, including respiratory infections or stress.

Although there is currently no cure for UC available, treatments are focused on suppressing the abnormal inflammatory process in the colon lining. Treatments including corticosteroids immunosuppressives (eg. azathioprine, mercaptopurine, and methotrexate) and aminosalicytates are available to treat the disease. However, the long-term use of immunosuppressives such as corticosteroids and azathioprine can result in serious side effects including thinning of bones, cataracts, infection, and liver and bone marrow effects. In the patients in whom current therapies are not successful, surgery is an option. The surgery involves the removal of the entire colon and the rectum.

There are several animal models that can partially mimic chronic ulcerative colitis. The most widely used model is the 2,4,6-trinitrobenesulfonic acid/ethanol (TNBS) induced colitis model, which induces chronic inflammation and ulceration in the colon. When TNBS is introduced into the colon of susceptible mice via intra-rectal instillation, it induces T-cell mediated immune response in the colonic mucosa, in this case leading to a massive mucosal inflammation characterized by the dense infiltration of T-cells and macrophages throughout the entire wall of the large bowel. Moreover, this histopathologic picture is accompanies by the clinical picture of progressive weight loss (wasting), bloody diarrhea, rectal prolapse, and large bowel wall thickening (Neurath et al. Intern. Rev. Immunol. 19:51-62,2000).

Another colitis model uses dextran sulfate sodium (DSS), which induces an acute colitis manifested by bloody diarrhea, weight loss, shortening of the colon and mucosal ulceration with neutrophil infiltration. DSS-induced colitis is characterized histologically by infiltration of inflammatory cells into the lamina propria, with lymphoid hyperplasia, focal crypt damage, and epithelial ulceration. These changes are thought to develop due to a toxic effect of DSS on the epithelium and by phagocytosis of lamina propria cells and production of TNF-alpha and IFN-gamma. Despite its common use, several issues regarding the mechanisms of DSS about the relevance to the human disease remain unresolved. DSS is regarded as a T cell-independent model because it is observed in T cell-deficient animals such as SCID mice.

The administration of the soluble polypeptide of the present invention to these TNBS or DSS models can be used to evaluate its use to ameliorate symptoms and alter the course of gastrointestinal disease. Moreover, the results showing inhibition or neutralization of IL-17F and/or IL-17A by these soluble polypeptides provide proof of concept that they (or similar molecules) can also be used to ameliorate symptoms in the colitis/IBD models and alter the course of disease.

### 3. Psoriasis

Psoriasis is a chronic skin condition that affects more than seven million Americans. Psoriasis occurs when new skin cells grow abnormally, resulting in inflamed, swollen, and scaly patches of skin where the old skin has not shed quickly enough. Plaque psoriasis, the most common form, is characterized by inflamed patches of skin ("lesions") topped with silvery white scales. Psoriasis may be limited to a few plaques or involve moderate to extensive areas of skin, appearing most commonly on the scalp, knees, elbows and trunk. Although it is highly visible, psoriasis is not a contagious disease. The pathogenesis of the diseases involves chronic inflammation of the affected tissues. The soluble polypeptides of the present invention could serve as a valuable therapeutic to reduce inflammation and pathological effects in psoriasis, other inflammatory skin diseases, skin and mucosal allergies, and related diseases.

Psoriasis is a T-cell mediated inflammatory disorder of the skin that can cause considerable discomfort. It is a disease for which there is no cure and affects people of all ages. Psoriasis affects approximately two percent of the populations of European and North America. Although individuals with mild psoriasis can often control their disease with topical agents, more than one million patients worldwide require ultraviolet or systemic immunosuppressive therapy. Unfortunately, the inconvenience and risks of ultraviolet radiation and the toxicities of many therapies limit their long-term use. Moreover, patients usually have recurrence of psoriasis, and in some cases rebound, shortly after stopping immunosuppressive therapy.

The soluble polypeptide of the present invention may also be used within diagnostic systems for the detection of circulating levels of IL-17F or IL-17A, and in the detection of IL-17F or IL-17A associated with acute phase inflammatory response. Within a related embodiment, the soluble polypeptide of the present invention can be used to detect circulating or locally-acting IL-17F or IL-17A polypeptides. Elevated or depressed levels of ligand or receptor polypeptides may be indicative of pathological conditions, including inflammation or cancer. IL-17F is known to induce associated acute phase inflammatory response. Moreover, detection of acute phase proteins or molecules such as IL-17A or IL-17F can be indicative of a chronic inflammatory condition in certain disease states (e.g., asthma, psoriasis, rheumatoid arthritis, IBD, IBS). Detection of such conditions serves to aid in disease diagnosis as well as help a physician in choosing proper therapy.

In addition to other disease models described herein, the activity of the soluble polypeptides of the present invention on inflammatory tissue derived from human psoriatic lesions can be measured *in vivo* using a severe combined immune deficient (SCID) mouse model. Several mouse models have been developed in which human cells are implanted into immunodeficient mice (collectively referred to as xenograft models); see, for example, Cattan AR, Douglas E, Leuk. Res. 18:513-22, 1994 and Flavell, DJ, Hematological Oncology 14:67-82, 1996. As an in vivo xenograft model for psoriasis, human psoriatic skin tissue is implanted into the SCID mouse model, and challenged with an appropriate antagonist. Moreover, other psoriasis animal models in ther art may be used to evaluate IL-17A and IL-17F antagonists, such as human psoriatic skin grafts implanted into AGR129 mouse model, and challenged with an appropriate antagonist (e.g., see, Boyman, O. et al., J. Exp. Med. Online publication #20031482, 2004). The soluble polypeptide of the present invention that binds, blocks, inhibits, reduces, antagonizes or neutralizes the activity of both IL-17A and IL-17F can be used in this model. Similarly, tissues or cells derived from IBD, IBS, arthritis, or other inflammatory lesions can be used in the SCID model to assess the anti-inflammatory properties of the IL-17A and IL-17F antagonists described herein.

Therapies designed to abolish, retard, or reduce inflammation using the soluble polypeptide of the present invention can be tested by administration to SCID mice bearing human inflammatory tissue (e.g., psoriatic lesions and the like), or other models described herein. Efficacy of treatment is measured and statistically evaluated as increased anti-inflammatory effect within the treated population over time using methods well known in the art. Some exemplary methods include, but are not limited to measuring for example, in a psoriasis model, epidermal thickness, the number of inflammatory cells in the upper dermis, and the grades of parakeratosis. Such methods are known in the art and described herein. For example, see Zeigler, M. et al. Lab Invest 81:1253, 2001; Zollner, T. M. et al. J. Clin. Invest. 109:671, 2002; Yamanaka, N. et al. Microbio.l Immunol. 45:507, 2001; Raychaudhuri, S. P. et al. Br. J. Dermatol. 144:931, 2001; Boehncke, W. H et al. Arch. Dermatol. Res. 291:104, 1999; Boehncke, W. H et aL. J. Invest. Dermatol. 116:596, 2001; Nickoloff, B. J. et al. Am. J. Pathol. 146:580, 1995; Boehncke, W. H et al. J. Cutan. Pathol. 24:1, 1997; Sugai, J., M. et al. J. Dermatol. Sci. 17:85, 1998; and Villadsen L.S. et al. J. Clin. Invest. 112:1571, 2003. Inflammation may also be monitored over time using well-known methods such as flow cytometry (or PCR) to quantitate the number of inflammatory or lesional cells present in a sample, score (weight loss, diarrhea, rectal bleeding, colon length) for IBD, paw disease score and inflammation score for CIA RA model. For example, therapeutic strategies appropriate for testing in such a model include direct treatment using soluble IL-17RC or IL-ITRC/IL-17RA, or other IL-17A and IL-17F antagonists (singly or together), or related conjugates or antagonists based on the disrupting interaction of IL-17RC and/or IL-17RA with their corresponding ligands.

Psoriasis is a chronic inflammatory skin disease that is associated with hyperplastic epidermal keratinocytes and infiltrating mononuclear cells, including CD4+ memory T cells, neutrophils and macrophages (Christophers, Int. Arch. Allergy Immunol., 110:199, 1996). It is currently believed that environmental antigens play a significant role in initiating and contributing to the pathology of the disease. However, it is the loss of tolerance to self-antigens that is thought to mediate the pathology of psoriasis. Dendritic cells and CD4⁺ T cells are thought to play an important role in antigen presentation and recognition that mediate the immune response leading to the pathology. We have recently developed a model of psoriasis based on the CD4+CD45RB transfer model (Davenport et al., Internat. Immunopharmacol., 2:653-672). The soluble polypeptide of the present invention is administered to the mice. Inhibition of disease scores (skin lesions, inflammatory cytokines) indicates the effectiveness of those soluble polypeptides in psoriasis.

### 4. Asthma

IL-17 plays an important role in allergen-induced T cell activation and neutrophilic influx in the airways. The receptor for IL-17 is expressed in the airways (Yao, et al. Immunity 3:811 (1995)) and IL-17 mediated neutrophil recruitment in allergic asthma is largely induced by the chemoattractant IL-8, GRO-β and macrophage inflammatory protein-2 (MIP-2) produced by IL-17 stimulated human bronchial epithelial cells (HBECs) and human bronichial fibroblasts ( Yao, et al. J Immunol 155:5483 (1995)); Molet, et al. J Allergy Clin Immunol 108:430 (2001*)*). IL-17 also stimulates HBECs to release IL-6, a neutrophil-activating factor (Fossiez, et al, J Exp Med 183:2593 (1996*),* and Linden, et al. Int Arch Allergy Immunol 126:179 (2001)) and has been shown to synergize with TNF-α to prolong the survival of human neutrophils *in vitro* (Laan et al., Eur Respir J 21:387 (2003)). Moreover, IL-17 is capable of amplifying the inflammatory responses in asthma by its ability to enhance the secretion of cytokines implicated in airway remodeling such as the profibrotic cytokines, IL-6 and IL-11 and inflammatory mediators granulocyte colony-stimulating factor (G-CSF) and granulocyte macrophage colony-stimulating factor (GM-CSF) (Molet et al., J Allergy Clin Immunol 108:430 (2001)).

Clinical evidence shows that acute, severe exacerbations of asthma are associated with recruitment and activation of neutrophils in the airways, thus IL-17 is likely to play a significant role in asthma. Patients with mild asthma display a detectable increase in the local concentration of free, soluble IL-17A protein (Molet et al., J Allergy Clin Immunol 108:430 (2001)) while healthy human volunteers with induced, severe airway inflammation due to the exposure to a swine confinement, display a pronounced increase in the concentration of free, soluble IL-17A protein in the bronchoalveolar space (Fossiez et al., J Exp Med 183:2593 (1996), and Linden el al., Int Arch Allergy Immuno I 126:179 (2001)). Furthermore, IL-17 levels in sputum have correlated with individuals who have increased airway hyper-reactivity Barczyk et al., Respir Med 97:726 (2003).

In animal models of airway hyper-responsiveness, chronic inhalation of ovalbumin by sensitized mice resulted in bronchial eosinophilic inflammation and early induction of IL-17 mRNA expression in inflamed lung tissue, together with a bronchial neutrophilia. (Hellings et al., Am J Respir Cell Mol Biol 28:42 (2003).) Anti-IL-17 monoclonal antibodies strongly reduced bronchial neutrophilic influx but significantly enhanced IL-5 levels in both bronchoalveolar lavage fluid and serum, and aggravated allergen-induced bronchial eosinophilic influx, suggesting that IL-17A may be involved in determining the balance between neutrophil and eosinophil accumulation following antigen insult. (*Id.*)

Among the IL-17 family members, IL-17F is most closely related to IL-17A. The biological activities mediated by IL-17F are similar to those of IL-17A, where IL-17F stimulates production of IL-6, IL-8 and G-CSF. (Hurst et al., J Immunol 169:443 (2002).) IL-17F also induces production of IL-2, transforming growth factor (TGF)-β, and monocyte chemoattractant protein (MCP) in endothelial cells. (Starnes et al., J Immunol 167:4137 (2001).) Similarly, allergen challenge can increase local IL-17F in patients with allergic asthma. (Kawaguchi et al., J Immunol 167:4430 (2001).) Gene delivery of IL-17F in murine lung increases neutrophils in the bronchoalveolar space, while mucosal transfer of the IL-17F gene enhances the levels of Ag-induced pulmonary neutrophilia and airway responsiveness to methacholine. (Oda et al., Am J Respir Crit Care Med 171:12 (2005).)

Apart from asthma, several chronic inflammatory airway diseases are characterized by neutrophil recruitment in the airways and IL-17 has been reported to play an important role in the pathogenesis of respiratory conditions such as chronic obstructive pulmonary disease (COPD), and cystic fibrosis (Linden et al., Eur Respir J 15:973 (2000); Ye et al., Am J Respir Cell Mol Biol 25:335 (2001), Rahman, et al. Clin Immunol 115:268 (2005)). An anti-IL-17A and/or anti-IL-17F therapeutic molecule could be demonstrated to be efficacious for chronic inflammatory airway disease in an in vitro model of inflammation. The ability of antagonists to IL-17F and/or IL-17A activity, such as IL-17RC soluble receptors and antibodies thereto including the anti-human-IL-17RC monoclonal and neutralizing antibodies of the present invention to inhibit IL-17A or and/or IL-17F-induced cytokine and chemokine production from cultured HBECs or bronchial fibroblasts could be used as a measure of efficacy for such antagonists in the prevention of the production of inflammatory mediators directly resulting from IL-17A and/or F stimulation. If the addition of antagonists, such as the soluble polypeptide of the present invention, to IL-17F and/or IL-17A activity, markedly reduces the production and expression of inflammatory mediators, it would be expected to be efficacious in inflammatory aspects associated with chronic airway inflammation.

### 5. Irritable Bowel Syndrome ("IBS")

Irritable bowel syndrome represents a disease characterized by abdominal pain or discomfort and an erratic bowel habit. IBS patients can be characterized into three main groups based on bowel habits: those with predominantly loose or frequent stools, those with predominantly hard or infrequent stools, and those with variable or normal stools (Talley et al., 2002). Altered intestinal motility, abnormalities in epithelial function, abnormal transit of stool and gas, and stress, may contribute to symptoms, while visceral hypersensitivity is a key feature in most patients. Genetic factors affecting pain-signaling and disturbances in central processing of afferent signals are postulated to predispose individuals to IBS following specific environmental exposures. Studies have also demonstrated that inflammatory responses in the colon may contribute to increased sensitivity of smooth muscle and enteric nerves and therefore perturb sensory-motor functions in the intestine (Collins et al., 2001). There is clinical overlap between IBS and IBD, with IBS-like symptoms frequently reported in patients before the diagnosis of IBD, and a higher than expected IBS symptoms in patients in remission from established IBD. Thus, these conditions may coexist with a higher than expected frequency, or may exist on a continuum, with IBS and IBD at different ends of the same spectrum. However, it should be noted that in most IBS patients, colonic biopsy specimens appear normal. Nevertheless, IBS significantly affects a very large number of individuals (U.S. prevalence in 2000, approximately 16 million individuals), resulting in a total cost burden of 1.7 billion dollars (year 2000). Thus, among the most prevalent and costly gastrointestinal diseases and disorders, IBS is second only to gastroesophageal reflux disease (GERD). Yet unlike GERD, treatment for IBS remains unsatisfactory (Talley et al., 2002; Farhadi et al., 21001; Collins et al., 2001), demonstrating that IBS clearly represents an unmet medical need.

Converging- disease models have been proposed that postulate an enhanced responsiveness of neural, immune or neuroimmune circuits in the central nervous system (CNS) or in the gut to central (psychosocial) or peripheral (tissue irritation, inflammation, infection) perturbations of normal homeostasis (Talley et al., 2002). This enhanced responsiveness results in dysregulation of gut motility, epithelial function (immune, permeability), and visceral hypersensitivity, which in turn results in IBS symptoms.

There may be a role for a number of different molecules in the pathogenesis of IBS including a role for molecules that stimulate neurons and those that are involved in initiation of inflammatory process. A number of our in-house molecules are known to be linked to possible activity on neurons due to their direct expression by neurons or expression of their receptors on neurons, including IL-17D, IL-17B and IL-31. Moreover, a number of IL-17 family members and related molecules have been associated with inflammation in the gut, including IL-17A, IL-17F, IL-23 and IL-31.

Efficacy of inhibitors of these molecules could be tested *in vivo* in animal models of disease. Several animal models have been proposed that mimic key features of IBS and involve centrally targeted stimuli (stress) or peripherally targeted stimuli (infection, inflammation). Two examples of in vivo animal models that can be used to determined the effectiveness of inhibitors in the treatment of IBS are (i) models focusing on primary CNS-directed pathogeneisis of IBS (stress models), and (ii) models focusing on gut-directed inducers of stress (i.e. gut inflammation, infection or physical stress). It should be noted however, that events within the CNS or in the gastrointestinal (GI) tract do not occur in isolation and that symptoms of IBS most likely result from a complex interaction between signals from the CNS on the GI and vice versa.

### J) Pharmaceutical Formulations

For pharmaceutical use, the soluble polypeptide of the present invention is formulated for parenteral, particularly intravenous or subcutaneous, delivery according to conventional methods. Intravenous administration will be by bolus injection, controlled release, e.g, using mini-pumps or other appropriate technology, or by infusion over a typical period of one to several hours. In general, pharmaceutical formulations will include a hematopoietic protein in combination with a pharmaceutically acceptable vehicle, such as saline, buffered saline, 5% dextrose in water or the like. Formulations may further include one or more excipients, preservatives, solubilizers, buffering agents, albumin to provent protein loss on vial surfaces, etc. When utilizing such a combination therapy, the cytokines may be combined in a single formulation or may be administered in separate formulations. Methods of formulation are well known in the art and are disclosed, for example, in Remington's Pharmaceutical Sciences, Gennaro, ed., Mack Publishing Co., Easton PA, 1990. Therapeutic doses will generally be in the range of 0.1 to 100 mg/kg of patient weight per day, preferably 0.5-20 mg/kg per day, with the exact dose determined by the clinician according to accented standards, taking into account the nature and severity of the condition to be treated, patient traits, etc. Determination of dose is within the level of ordinary skill in the art. The proteins will commonly be administered over a period of up to 28 days following chemotherapy or bone-marrow transplant or until a platelet count of >20,000/mm³, preferably >50,000/mm³, is achieved. More commonly, the proteins will be administered over one week or less, often over a period of one to three days. In general, a therapeutically effective amount of the soluble polypeptide of the present invention in an amount sufficient to produce a clinically significant increase in the proliferation and/or differentiation of lymphoid or myeloid progenitor cells, which will be manifested as an increase in circulating levels of mature cells (e.g. platelets or neutrophils). Treatment of platelet disorders will thus be continued until a platelet count of at least 20,000/mm³, preferably 50,000/mm³, is reached. The soluble polypeptide of the present invention can also be administered in combination with other cytokines such as IL-3, -6 and -11; stem cell factor, erythropoietin, G-CSF and GM-CSF. Within regimens of combination therapy, daily doses of other cytokines will in general be: EPO, 150 U/kg; GM-CSF, 5-15 1g/kg; IL-3,1-5 1g/kg; and G-CSF, 1-25 1g/kg. Combination therapy with EPO, for example, is indicated in anemic patients with low EPO levels.

Generally, the dosage of administered soluble polypeptide will vary depending upon such factors as the patient's age, weight, height, sex, general medical condition and previous medical history. Typically, it is desirable to provide the recipient with a dosage of such soluble polypeptide which is in the range of from about 1 pg/kg to 10 mg/kg (amount of agent/body weight of patient), although a lower or higher dosage also may be administered as circumstances dictate.

Administration of the soluble polypeptide of the present invention to a subject can be intravenous, intraarterial, intraperitoneal, intramuscular, subcutaneous, intrapleural, intrathecal, by perfusion through a regional catheter, or by direct intralesional injection. When administering therapeutic proteins by injection, the administration may be by continuous infusion or by single or multiple boluses.

Additional routes of administration include oral, mucosal-membrane, pulmonary, and transcutaneous. Oral delivery is suitable for polyester microspheres, zein microspheres, proteinoid microspheres, polycyanoacrylate microspheres, and lipid-based systems (see, for example, DiBase and Morrel, "Oral Delivery of Microencapsulated Proteins," in Protein Delivery: Physical Systems, Sanders and Hendren (eds.), pages 255-288 (Plenum Press 1997)). The feasibility of an intranasal delivery is exemplified by such a mode of insulin administration (see, for example, Hinchcliffe and Illum, Adv. Drug Deliv. Rev. 35:199 (1999)). Dry or liquid particles comprising the soluble polypeptide can be prepared and inhaled with the aid of dry-powder dispersers, liquid aerosol generators, or nebulizers (*e.g*., Pettit and Gombotz, TIBTECH 16:343 (1998); Patton et al., Adv. Drug De/iv. Rev. 35:235 (1999)). This approach is illustrated by the AERX diabetes management system, which is a hand-held electronic inhaler that delivers aerosolized insulin into the lungs. Studies have shown that proteins as large as 48,000 kDa have been delivered across skin at therapeutic concentrations with the aid of low-frequency ultrasound, which illustrates the feasibility of trascutaneous administration (Mitragotri et al., Science 269:850 (1995)). Transdermal delivery using electroporation provides another means to administer the soluble polypeptide of the present invention (Potts et al., Pharm. Biotechnol. 10:213 (1997)).

A pharmaceutical composition comprising the soluble polypeptide of the present invention can be formulated according to known methods to prepare pharmaceutically useful compositions, whereby the therapeutic proteins are combined in a mixture with a pharmaceutically acceptable carriers. A composition is said to be a "pharmaceutically acceptable carrier" if its administration can be tolerated by a recipient patient. Sterile phosphate-buffered saline is one example of a pharmaceutically acceptable carrier. Other suitable carriers are well-known to those in the art. See, for example, Gennaro (ed.), Remington's Pharmaceutical Sciences, 19th Edition (Mack Publishing Company 1995).

For purposes of therapy, the soluble polypeptide of the present invention and a pharmaceutically acceptable carrier are administered to a patient in a therapeutically effective amount. A combination of a therapeutic molecule of the present invention and a pharmaceutically acceptable carrier is said to be administered in a "therapeutically effective amount" if the amount administered is physiologically significant. An agent is physiologically significant if its presence results in a detectable change in the physiology of a recipient patient. For example, an agent used to treat inflammation is physiologically significant if its presence alleviates the inflammatory response.

A pharmaceutical composition comprising a soluble polypeptide of the present invention can be furnished in liquid form, in an aerosol, or in solid form. Liquid forms, are illustrated by injectable solutions and oral suspensions. Exemplary solid forms include capsules, tablets, and controlled-release forms. The latter form is illustrated by miniosmotic pumps and implants (Bremer et al., Pharm. Biotechnol. 10:239 (1997); Ranade, "Implants in Drug Delivery," in Drug Delivery Systems, Ranade and Hollinger (eds.), pages 95-123 (CRC Press 1995); Bremer et al., "Protein Delivery with Infusion Pumps," in Protein Delivery: Physical Systems, Sanders and Hendren (eds.), pages 239-254 (Plenum Press 1997); Yewey et al., "Delivery of Proteins from a Controlled Release Injectable Implant," in Protein Delivery: Physical Systems, Sanders and Hendren (eds.), pages 93-117 (Plenum Press 1997)).

Liposomes provide one means to deliver therapeutic polypeptides to a subject intravenously, intraperitoneally, intrathecally, intramuscularly, subcutaneously, or via oral administration, inhalation, or intranasal administration. Liposomes are microscopic vesicles that consist of one or more lipid bilayers surrounding aqueous compartments (see, generally, Bakker-Woudenberg et al., Eur. J. Clin. Microbiol. Infect. Dis. 12 (Suppl. 1):S61 (1993), Kim, Drugs 46:618 (1993), and Ranade, "Site-Specific Drug Delivery Using Liposomes as Carriers," in Drug Delivery Systems, Ranade and Hollinger (eds.), pages 3-24 (CRC Press 1995)). Liposomes are similar in composition to cellular membranes and as a result, liposomes can be administered safely and are biodegradable. Depending on the method of preparation, liposomes may be unilamellar or multilamellar, and liposomes can vary in size with diameters ranging from 0.02 µm to greater than 10 µm. A variety of agents can be encapsulated in liposomes: hydrophobic agents partition in the bilayers and hydrophilic agents partition within the inner aqueous space(s) (see, for example, Machy el al., Liposomes In Cell Biology And Pharmacology (John Libbey 1987), and Ostro et al., American J. Hosp. Pharm. 46:1576 (1989)). Moreover, it is possible to control the therapeutic availability of the encapsulated agent by varying liposome size, the number of bilayers, lipid composition, as well as the charge and surface characteristics of the liposomes.

Liposomes can adsorb to virtually any type of cell and then slowly release the encapsulated agent. Alternatively, an absorbed liposome may be endocytosed by cells that are phagocytic. Endocytosis is followed by intralysosomal degradation of liposomal lipids and release of the encapsulated agents (Scherphof et al., Ann. N.Y. Acad. Sci. 446:368 (1985)). After intravenous administration, small liposomes (0.1 to 1.0 µm) are typically taken up by cells of the reticuloendothelial system, located principally in the liver and spleen, whereas liposomes larger than 3.0 µm are deposited in the lung. This preferential uptake of smaller liposomes by the cells of the reticuloendothelial system has been used to deliver chemotherapeutic agents to macrophages and .to tumors of the liver.

The reticuloendothelial system can be circumvented by several methods including saturation with large doses of liposome particles, or selective macrophage inactivation by pharmacological means (Claassen et al.. Biochim. Biophys. Acta 802:428 (1984)). In addition, incorporation of glycolipid- or polyethelene glycol-derivatized phospholipids into liposome membranes has been shown to result in a significantly reduced uptake by the reticuloendothelial system (Allen et al., Biochim. Biophys. Acta 1068:133 (1991); Allen et al., Biochim. Biophys. Acta 1150:9 (1993)).

Liposomes can also be prepared to target particular cells or organs by varying phospholipid composition or by inserting receptors or ligands into the liposomes. For example, liposomes, prepared with a high content of a nonionic surfactant, have been used to target the liver (Hayakawa *et al.,* Japanese Patent 04-244,018; Kato et al., Biol. Pharm. Bull. 16:960 (1993)). These formulations were prepared by mixing soybean phospatidylcholine, α-tocopherol, and ethoxylated hydrogenated castor oil (HCO-60) in methanol, concentrating the mixture under vacuum, and then reconstituting the mixture with water. A liposomal formulation of dipalmitoylphosphatidylcholine (DPPC) with a soybean-derived sterylglucoside mixture (SG) and cholesterol (Ch) has also been shown to target the liver (Shimizu et al., Biol. Pharm. Bull. 20:881 (1997)).

Alternatively, various targeting ligands can be bound to the surface of the liposome, such as antibodies, antibody fragments, carbohydrates, vitamins, and transport proteins. For example, liposomes can be modified with branched type galactosyllipid derivatives to target asialoglycoprotein (galactose) receptors, which are exclusively expressed on the surface of liver cells (Kato and Sugiyama, Crit. Rev. Ther. Drug Carrier Syst. 14:287 (1997); Murahashi et al., Biol. Pharm. Bull.20:259 (1997)). Similarly, Wu et al., Hepatology 27:772 (1998), have shown that labeling liposomes with asialofetuin led to a shortened liposome plasma half-life and greatly enhanced uptake of asialofetuin-labeled liposome by hepatocytes. On the other hand, hepatic accumulation of liposomes comprising branched type galactosyllipid derivatives can be inhibited by preinjection of asialofetuin (Murahashi et al., Biol. Pharm. Bull.20:259 (1997)). Polyaconitylated human serum albumin liposomes provide another approach for targeting liposomes to liver cells (Kamps et al., Proc. Nat'l Acad. Sci. USA 94:11681 (1997)). Moreover, Geho, et al. U.S. Patent No. 4,603,044, describe a hepatocyte-directed liposome vesicle delivery system, which has specificity for hepatobiliary receptors associated with the specialized metabolic cells of the liver.

In a more general approach to tissue targeting, target cells are prelabeled with biotinylated antibodies specific for a ligand expressed by the target cell (Harasym et al., Adv. Drug Deliv. Rev. 32:99 (1998)). After plasma elimination of free antibody, streptavidin-conjugated liposomes are administered. In another approach, targeting antibodies are directly attached to liposomes (Harasym et al., Adv. Drug Deliv. Rev. 32:99 (1998)).

Polypeptides and antibodies can be encapsulated within liposomes using standard techniques of protein microencapsulation (see, for example, Anderson et al., Infect. Immun. 31:1099 (1981), Anderson et al., Cancer Res. 50:1853 (1990), and Cohen et al., Biochim. Biophys. Acta 1063:95 (1991), Alving et al. "Preparation and Use of Liposomes in Immunological Studies," in Liposome Technology, 2nd Edition, Vol. III, Gregoriadis (ed.), page 317 (CRC Press 1993), Wassef et al., Meth. Enzymol. 149:124 (1987)). As noted above, therapeutically useful liposomes may contain a variety of components. For example, liposomes may comprise lipid derivatives of poly(ethylene glycol) (Allen et al., Biochim. Biophys. Acta 1150:9 (1993)).

Degradable polymer microspheres have been designed to maintain high systemic levels of therapeutic proteins. Microspheres are prepared from degradable polymers such as poly(lactide-co-glycolide) (PLG), polyanhydrides, poly (ortho esters), nonbiodegradable ethylvinyl acetate polymers, in which proteins are entrapped in the polymer (Gombotz and Pettit, Bioconjugate Chem. 6:332 (1995); Ranade, "Role of Polymers in Drug Delivery," in Drug Delivery Systems, Ranade and Hollinger (eds.), pages 51-93 (CRC Press 1995); Roskos and Maskiewicz, "Degradable Controlled Release Systems Useful for Protein Delivery," in Protein Delivery: Physical Systems, Sanders and Hendren (eds.), pages 45-92 (Plenum Press 1997); Bartus et al., Science 281:1161 (1998); Putney and Burke, Nature Biotechnology 16:153 (1998); Putney, Curr. Opin. Chem. Biol. 2:548 (1998)). Polyethylene glycol (PEG)-coated nanospheres can also provide carriers for intravenous administration, of therapeutic proteins (see, for example, Gref et al., Pharm. Biotechnol. 10:167 (1997)).

Other dosage forms can be devised by those skilled in the art, as shown, for example, by Ansel and Popovich, Pharmaceutical Dosage Forms and Drug Delivery Systems, 5th Edition (Lea & Febiger 1990), Gennaro (ed.), Remington's Pharmaceutical Sciences, 19th Edition (Mack Publishing Company 1995), and by Ranade and Hollinger, Drug Delivery Systems (CRC Press 1996).

As an illustration, pharmaceutical compositions may be supplied as a kit comprising a container that comprises the soluble polypeptide of the present invention. Therapeutic polypeptide can be provided in the form of an injectable solution for single or multiple doses, or as a sterile powder that will be reconstituted before injection. Alternatively, such a kit can include a dry-powder disperser, liquid aerosol generator, or nebulizer for administration of a therapeutic polypeptide. Such a kit may further comprise written information on indications and usage of the pharmaceutical composition. Moreover, such information may include a statement that the composition is contraindicated in patients with known hypersensitivity to IL-17RC or IL-17RA.

The present invention contemplates compositions of the soluble polypeptide of the present invention, and methods and therapeutic uses comprising the same polypeptide described herein. Such compositions can further comprise a carrier. The carrier can be a conventional organic or inorganic carrier. Examples of carriers include water, buffer solution, alcohol, propylene glycol, macrogol, sesame oil, corn oil, and the like.

### K) Production of Transgenic Mice

Transgenic mice can be engineered to over-express the either IL-17F, IL-17A, IL-17RA or the IL-17RC gene in all tissues or under the control of a tissue-specific or tissue-preferred regulatory element. These over-producers can be used to characterize the phenotype that results from over-expression, and the transgenic animals can serve as models for human disease caused by excess IL-17F, IL-17A, IL-17RA or IL-17RC. Transgenic mice that over-express any of these also provide model bioreactors for production of IL-17RA or IL-17RC, such as any of the soluble polypeptides of the present invention in milk or blood of larger animals. Methods for producing transgenic mice are well-known to those of skill in the art (see, for example, Jacob, "Expression and Knockout of Interferons in Transgenic Mice," in Overexpression and Knockout of Cytokines in Transgenic Mice, Jacob (ed.), pages 111-124 (Academic Press, Ltd. 1994), Monastersky and Rob1 (eds.), Strategies in Transgenic Animal Science (ASM Press 1995), and Abbud and Nilson, recombinant Protein Expression in Transgenic Mice," in Gene Expression Systems: Using Nature for the Art of Expression, Fernandez and Hoeffler (eds.), pages 367-397 (Academic Press, Inc. 1999)).

For example, a method for producing a transgenic mouse that expresses a IL-17RC gene can begin with adult, fertile males (studs) (B6C3f1, 2-8 months of age (Taconic Farms, Germantown, NY)), vasectomized males (duds) (B6D2f1, 2-8 months, (Taconic Farms)), prepubescent fertile females (donors) (B6C3f1, 4-5 weeks, (Taconic Farms)) and adult fertile females (recipients) (B6D2f1, 2-4 months, (Taconic Farms)). The donors are acclimated for one week and then injected with approximately 8 IU/mouse of Pregnant Mare's Serum gonadotrophin (Sigma Chemical Company; St. Louis, MO) I.P., and 46-47 hours later, 8 IU/mouse of human Chorionic Gonadotropin (hCG (Sigma)) I.P. to induce superovulation. Donors are mated with studs subsequent to hormone injections. Ovulation generally occurs within 13 hours of hCG injection. Copulation is confirmed by the presence of a vaginal plug the morning following mating.

Fertilized eggs are collected under a surgical scope. The oviducts are collected and eggs are released into urinanalysis slides containing hyaluronidase (Sigma). Eggs are washed once in hyaluronidase, and twice in Whitten's W640 medium (described, for example, by Menino and O'Claray, Biol. Reprod. 77:159 (1986), and Dienhart and Downs, Zygote 4:129 (1996)) that has been incubate with 5% CO2, 5% O2, and 90% N2 at 37°C. The eggs are then stored in a 37°C/5% CO2 incubator until microinjection.

Ten to twenty micrograms of plasmid DNA containing a IL-17RC encoding sequence is linearized, gel-purified, and resuspended in 10 mM Tris-HCl (pH 7.4), 0.25 mM EDTA (pH 8.0), at a final concentration of 5-10 nanograms per microliter for microinjection. For example, the IL-17RC encoding sequences can encode a polypeptide comprising amino acid residues 21 to 452 of SEQ ID NO:2.

Plasmid DNA is microinjected into harvested eggs contained in a drop of W640 medium overlaid by warm, CO2-equilibrated mineral oil. The DNA is drawn into an injection needle (pulled from a 0.75mm in, 1mm OD borosilicate glass capillary), and injected into individual eggs. Each egg is penetrated with the injection needle, into one or both of the haploid pronuclei.

Picoliters of DNA are injected into the pronuclei, and the injection needle withdrawn without coming into contact with the nucleoli. The procedure is repeated until all the eggs are injected. Successfully microinjected eggs are transferred into an organ tissue-culture dish with pre-gassed W640 medium for storage overnight in a 37°C/5% CO2 incubator.

The following day, two-cell embryos are transferred into pseudopregnant recipients. The recipients are identified by the presence of copulation plugs, after copulating with vasectomized duds. Recipients are anesthetized and shaved on the dorsal left side and transferred to a surgical microscope. A small incision is made in the skin and through the muscle wall in the middle of the abdominal area outlined by the ribcage, the saddle, and the hind leg, midway between knee and spleen. The reproductive organs are exteriorized onto a small surgical drape. The fat pad is stretched out over the surgical drape, and a baby serrefine (Roboz, Rockville, MD) is attached to the fat pad and left hanging over the back of the mouse, preventing the organs from sliding back in.

With a fine transfer pipette containing mineral oil followed by alternating W640 and air bubbles, 12-17 healthy two-cell embryos from the previous day's injection are transferred into the recipient. The swollen ampulla is located and holding the oviduct between the ampulla and the bursa, a nick in the oviduct is made with a 28 g needle close to the bursa, making sure not to tear the ampulla or the bursa.

The pipette is transferred into the nick in the oviduct, and the embryos are blown in, allowing the first air bubble to escape the pipette. The fat pad is gently pushed into the peritoneum, and the reproductive organs allowed to slide in. The peritoneal wall is closed with one suture and the skin closed with a wound clip. The mice recuperate on a 37°C slide warmer for a minimum of four hours.

The recipients are returned to cages in pairs, and allowed 19-21 days gestation. After birth, 19-21 days postpartum is allowed before weaning. The weanlings are sexed and placed into separate sex cages, and a 0.5 cm biopsy (used for genotyping) is snipped off the tail with clean scissors.

Genomic DNA is prepared from the tail snips using, for example, a Qiagen Dneasy kit following the manufacturer's instructions. Genomic DNA is analyzed by PCR using primers designed to amplify a IL-17RC gene or a selectable marker gene that was introduced in the same plasmid. After animals are confirmed to be transgenic, they are back-crossed into an inbred strain by placing a transgenic female with a wild-type male, or a transgenic male with one or two wild-type female(s). As pups are born and weaned, the sexes are separated, and their tails snipped for genotyping.

To check for expression of a transgene in a live animal, a partial hepatectomy is performed. A surgical prep is made of the upper abdomen directly below the zyphoid process. Using sterile technique, a small 1.5-2 cm incision is made below the sternum and the left lateral lobe of the liver exteriorized. Using 4-0 silk, a tie is made around the lower lobe securing it outside the body cavity. An atraumatic clamp is used to hold the tie while a second loop of absorbable Dexon (American Cyanamid; Wayne, N.J.) is placed proximal to the first tie. A distal cut is made from the Dexon tie and approximately 100 mg of the excised liver tissue is placed in a sterile petri dish. The excised liver section is transferred to a 14 ml polypropylene round bottom tube and snap frozen in liquid nitrogen and then stored on dry ice. The surgical site is closed with suture and wound clips, and the animal's cage placed on a 37°C heating pad for 24 hours post operatively. The animal is checked daily post operatively and the wound clips removed 7-10 days after surgery. The expression level of IL-17RC mRNA is examined for each transgenic mouse using an RNA solution hybridization assay or polymerase chain reaction.

In addition to producing transgenic mice that over-express IL-17F, IL-17A, IL-17RA or IL-17RC, it is useful to engineer transgenic mice with either abnormally low or no expression of any of these genes. Such transgenic mice provide useful models for diseases associated with a lack of IL-17F, IL-17A, IL-17RA or IL-17RC. As discussed above, IL-17RC gene expression can be inhibited using anti-sense genes, ribozyme genes, or external guide sequence genes. For example, to produce transgenic mice that under-express the IL-17RC gene, such inhibitory sequences are targeted to IL-17RC mRNA. Methods for producing transgenic mice that have abnormally low expression of a particular gene are known to those in the art (see, for example, Wu et al., "Gene Underexpression in Cultured Cells and Animals by Antisense DNA and RNA Strategies," in Methods in Gene Biotechnology, pages 205-224 (CRC Press 1997)).

An alternative approach to producing transgenic mice that have little or no IL-17RC gene expression is to generate mice having at least one normal IL-17RC allele replaced by a nonfunctional IL-17RC gene. One method of designing a nonfunctional IL-17RC gene is to insert another gene, such as a selectable marker gene, within a nucleic acid molecule that encodes IL-17RC. Standard methods for producing these so-called "knockout mice" are known to those skilled in the art (see, for example, Jacob, "Expression and Knockout of Interferons in Transgenic Mice," in Overexpression and Knockout of Cytokines in Transgenic Mice, Jacob (ed.), pages 111-124 (Academic Press, Ltd. 1994), and Wu et al., "New Strategies for Gene Knockout," in Methods in Gene Biotechnology, pages 339-365 (CRC Press 1997)).

The invention is further illustrated by the following non-limiting examples.

### EXAMPLES

### EXAMPLE 1

### Expression of the IL-17RC Gene

Northern analyses were performed using Human Multiple Tissue Blots (Clontech Laboratories, Inc., Palo Alto, CA). Two probes were generated from gel purified PCR products. The first probe was made using C21798 (5'CGG CGT GGT GGT CTT GCT CTT 3'; SEQ ID NO:8) and ZC21808 (5'TCC CGT CCC CCG CCC CAG GTC 3'; SEQ ID NO:31) as primers. The probe was a radioactively labeled using the Multiprime labeling kit from Amersham (Arlington Heights, IL) according to the manufacturer's protocol. The probe was purified using a NucTrap push column (Stratagene, La Jolla, CA). ExpressHyb (Clontech) solution was used for the prehybridization and hybridization solutions for the northern blots. Hybridization took place overnight at 65□C. Following hybridization, the blots were washed for 30 minutes each in solutions that contained 0.1 % SDS and SSC as follows: twice in 2xSSC at room temperature, three times in 0.1x SSC at 50C°, once in 0.1x SSC at 55°C, and once in O.lx SSC at 65°C. The results demonstrated the IL-17RC gene is strongly expressed in thyroid, adrenal gland, prostate, and liver tissues, and expressed to a lesser extent in heart, small intestine, stomach, and trachea tissues. In contrast, there is little or no expression in brain, placenta, lung, skeletal muscle, kidney, pancreas, spleen, thymus, testis, ovary, colon, peripheral blood leukocytes, spinal cord, lymph node, and bone marrow.

### EXAMPLE 2

### Distribution of mRNA in Cell Line Panels Using PCR

Total RNA was purified from resting and stimulated cell lines grown in-house and purified using a Qiagen (Valencia, CA) RNeasy kit according to the manufacturer's instructions, or an acid-phenol purification protocol (Chomczynski and Sacchi, Analytical Biochemistry, 162:156-9, 1987). The quality of the RNA was assessed by running an aliquot on an Agilent Bioanalyzer. If the RNA was significantly degraded, it was not used for subsequent creation of first strand cDNA. Presence of contaminating genomic DNA was assessed by a PCR assay on an aliquot of the RNA with zc41011 (5'CTCTCCATCCTTATCTTTCATCAAC 3'; SEQ ID NO:32) and zc41012 (5'CTCTCTGCTGGCTAAACAAAACAC 3'; SEQ ID NO:33), primers that amplify a single site of intergenic genomic DNA. The PCR conditions for the contaminating genomic DNA assay were as follows: 2.5µl 10X buffer and 0.5µl Advantage 2 DNA polymerase mix (BD Biosciences Clontech, Palo Alto, CA), 2ul 2.5mM dNTP mix (Applied Biosystems, Foster City, CA), 2.5µl 10X Rediload (Invitrogen, Carlsbad, CA), and 0.5µl 20uM zc41011 and zc41012, in a final volume of 25 ul. Cycling parameters were 94oC 20", 40 cycles of 94oC 20" 60oC 1'20" and one cycle of 72oC 7'. 10ul of each reaction was subjected to agarose gel electrophoresis and gels were examined for presence of a PCR product from contaminating genomic DNA. If contaminating genomic DNA was observed, the total RNA was. DNAsed using DNA-free reagents (Ambion, Inc, Austin, TX) according to the manufacturer's instructions, then retested as described above. Only RNAs which appeared to be free of contaminating genomic DNA were used for subsequent creation of first strand cDNA.

20µg total RNA from 82 human cell lines were each brought to 98µl with H2O, then split into two 49ul aliquots, each containing 10µg total RNA, and placed in two 96-well PCR plates. To each aliquot was added reagents for first strand cDNA synthesis (Invitrogen First Strand cDNA Synthesis System, Carlsbad, CA): 20µl 25mM MgCl2, 10ul 10X RT buffer, 10ul 0.1M DTT, 2µl oligo dT, 2ul RNAseOut. Then, to one aliquot from each cell line 2µl Superscript II Reverse Transcriptase was added, and to the corresponding cell line aliquot 2µl H2O was added to make a minus Reverse Transcriptase negative control. All samples were incubated as follows: 25oC 10', 42oC 50', 70oC 15'. Samples were arranged in deep well plates and diluted to 1.7ml with H2O. A Multipette (Saigan) robot was used to aliquot 16.5µl into each well of a 96-well PCR plate multiple times, generating numerous one-use PCR panels of the cell lines, which were then sealed and stored at -20oC. Each well in these panels represents first strand cDNA from approximately 100ng total RNA. The 82 cell lines are spread across two panels, array #118A and #118B. Quality of first strand cDNA on the panels was assessed by a multiplex PCR assay on one set of the panels using primers to two widely expressed, but only moderately abundant genes, CLTC (clathrin) and TFRC (transferrin receptor C). 0.5ul each of Clathrin primers zc42901 (5'CTCATATTGCTCAACTGTGTGAAAAG 3'; SEQ ID NO:34), zc42902(5'TAGAAGCCACCTGAACACAAATCTG3'; SEQ ID NO:35), and TFRC primers zc42599 (5'ATGTTGCGTTGTATGTTGAAAATCAATT3'; SEQ ID NO:36), zc42600 (5'TTCTCCACGAGGTAAACAAGTCTAC3'; SEQ ID NO:37), were mixed with 2.5µl 10X buffer and 0.5µl Advantage 2 cDNA polymerase mix (BD Biosciences Clontech, Palo Alto, CA), 2µl 2.5mM dNTP mix (Applied Biosystems,, Foster City, CA), 2.5µl 10X Rediload (Invitrogen, Carlsbad, CA), and added to each well of a panel of array#118A and array #118B. Cycling parameters were as follows: 94oC 20", 35 cycles of 94oC 20", 67oC 80", and one cycle of 72oC 7'. 10µl of each reaction was subjected to agarose gel electrophoresis and gels were scored for the presence of a robust PCR product for each gene specific to the +RT wells for each cell line.

Expression of mRNA in the human first strand cDNA panels for IL-I7RC was assayed by PCR with sense oligo ZC42756 (5'ctctccaggcccaagtcgtgctct3'; SEQ ID NO:38) and antisense oligo ZC42757 (5'ttgtcctgggggcctcgtgtctcc3'; SEQ ID NO:39) under these PCR conditions per sample: 2.5µl 10X buffer and 0.5µl advantage 2 cDNA polymerase mix (BD Biosciences Clontech, Palo Alto, CA), 2µl 2.5mM dNTP mix (Applied Biosystems, ), 2.5ul 10X Rediload (Invitrogen, Carlsbad, CA), and 0.5µl 20uM each sense and antisense primer. Cycling conditions were 94oC 2', 35 cycles of 94oC 1', 66oC 30", 72oC 1.5', and one cycle of 72oC 7'. 10µl of each reaction was subjected to agarose gel electrophoresis and gels were scored for positive or negative expression of IL-17RC.

IL-17RC mRNA is widely expressed in many cell lines representing a broad spectrum of tissue and cell types. In particular, IL-17RC is consistently expressed in non-T cell peripheral blood cell lines, including monocytes, B-cells, and cells of the myeloid lineage. Also, IL-17RC mRNA is reliably expressed in cell lines derived from skin. Other cell lines that express IL-17RC are all 5 of the large intestine cell lines that were present on the array.

### EXAMPLE3

### Distribution of mRNA in Mouse Cell Line Panels Using RT PCR

Total RNA was purified from 60 resting and stimulated cell lines grown in-house and purified using a Qiagen (Valencia, CA) RNeasy kit according to the manufacturer's instructions, an acid-phenol purification protocol (Chomczynski and Sacchi, Analytical Biochemistry, 162:156-9, 1987), or a Trizol reagent protocol (Invitrogen, Carlsbad, CA).

5µg of total RNA from each cell line was arranged in a deep well 96-well plate, 125µl 3M NaOAc and 100µl Pellet Paint (Novagen, Madison, WI) were added to each well, then the final volume was adjusted to 1.25ml with H20. A Multipette (Saigan) robot was used to aliquot 2µl of the RNA mixture followed by 75ul EtOH into each well of a 96-well PCR plate multiple times, generating numerous one-use RT PCR panels of the cell lines, which were then sealed and stored at-20oC. RT PCR screening was performed by first centrifuging a panel in a Qiagen (Valencia, CA) 96-well centrifuge for 10' at 6000 RPM. Supernatant was removed by inverting the plate onto absorbent paper. RNA pellets were washed with 100µl 70% EtOH, followed by a 5' centrifugation at 6000 RPM. Supernatant was again removed and plates allowed to air-dry until the remaining EtOH was evaporated. RNA pellets were resuspended in 15µl H20.

Expression of IL-17RC mRNA in the mouse cell line RNA panels was assayed by RT PCR with zc38910 (5'acgaagcccaggtaccagaaagag3'; SEQ ID NO:40) and zc38679 (5'aaaagcgccgcagccaagagtagg3'; SEQ ID NO:41) under these RT PCR conditions per sample: Superscript One-Step PCR with Platinum Taq kit, Invitrogen, Carlsbad, CA. Cycling conditions were:1 cycle of 48°C for 30 minutes, 94°C for 2 minutes, followed by 35 cycles of 94°C for 15 seconds, 55°C for 30 seconds, 72°C for 1.5 minutes, followed by 1 cycle of 72°C for 7 minutes. 10µl of each reaction was subjected to agarose gel electrophoresis and gels were scored for positive or negative expression of IL-17RC.

Murine IL-17RCmRNA is expressed in several mouse cell lines, notably in cell lines derived from bone marrow, including osteoblast, adipocyte, and preadipocyte cell lines. Also, mouse IL-17RC is mRNA is represented in several samples from the endocrine system, such as pancreas stromal cell lines, pancreas islet cell lines, and hypothalamus, salivary gland, and testis cell lines.

### EXAMPLE 4

### Refolding and Purification pIl-17F Produced in E.coli

### A) Inclusion body isolation and extraction of pIL-17F

Following induction of protein expression in either batch ferment or shaker flask culture, the E.coli broth is centrifuged in 1 liter bottles @ 3000 RPM in a Sorvall swinging bucket rotor. Washing of the cell paste to remove any broth contaminants is performed with 50 mM Tris pH 8.0 containing 200 mM NaCl and 5 mM EDTA until the supemate is clear.

The cell pellets are then suspended in ice-cold lysis buffer (50 mM Tris pH 8.0; 5 mM EDTA; 200 mM NaCl, 10% sucrose (w/v); 5mM DTT; 5 mM Benzamidine;) to 10-20 Optical Density units at 600 nm. This slurry is then subjected to 3 passes at 8500-9000 psi in a chilled APV 2000 Lab Homogenizer producing a disrupted cell lysate. The insoluble fraction (inclusion bodies) is recovered by centrifugation of the cell lysate at 20,000 X G for 1 hour at 4°C.

. The inclusion body pellet resulting from the 20,000 X G spin is weighed and then re-suspended in wash buffer (50 mM Tris pH 8 containing 200 mM NaCl, 5 mM EDTA, 5uM DTT, 5mM Benzamidine ) at 10 ml wash buffer per gram inclusion bodies. Complete dispersion is achieved by homogenizing with an OMNI international rotor stator generator. This suspension is centrifuged at 20,000 X G. for 30 minutes at 4°C. The wash cycle is repeated 3-5 times until the supernatant is clear.

The final washed pellet is solubilized in 7M Guanidine HCl in 40 mM Tris buffer at pH 8 containing 0.1M Sodium Sulfite and 0.02 M Sodium Tetrathionate. The extraction and sulfitolysis reaction is allowed to proceed with gentle stirring at 4°C overnight. The resulting pinkish colored solution is centrifuged at 35,000 X g for 1 hour at 4°C and the clarified supemate, containing the soluble pIL-17F, is 0.45 um filtered.

### B) pIL-17F refolding procedure

The solubilized, sulfitolyzed pIL-17F is refolded by drop wise dilution into ice cold refolding buffer containing 55 mM MES, 10.56 mM NaCl, 0.44 mM KCl, 0.055% PEG (3400 K), 1.1 mM EDTA, 20% Glycerol, 0.5M Guanidine HCl, 0.75 M Arginine and the Glutathione redox pair at a 1:1 ratio (1mM GSH : 1mM GSSG). The pH of the refolding buffer is adjusted to 6.5 with HCl and the pIL-17F is added to a final concentration of 100 ug/ml. Once diluted, the mixture is allowed to stir slowly in the cold room for 72 hours.

### C) Product recovery & purification

The refolded pIL-17F is concentrated 10X vs. a 10kD cutoff membrane on a lab scale TFF system. Next it is filtered using a 0.45 micron membrane and the pH is adjusted to 5.1 with the addition of Acetic acid. The pH-adjusted material is captured by cation exchange chromatography on a Pharmacia SP Fast Flow column equilibrated in 50 mM Acetate buffer, pH 5.1. The pIL-17F is loaded by inline proportioning at 1:5 with equilibration buffer at a flow rate of 190 cm/hr. This dilution lowers the ionic strength enabling efficient binding of the target to the matrix. After sample loading is complete, the column is washed to baseline absorbance with equilibration buffer. The column is washed with 0.4 M NaCl in 50 mM Acetate buffer at pH 5.1 and then the bound protein is eluted with a 5 CV gradient from 0.4 M to 1.5 M NaCl in 50 mM Acetate buffer at pH 5.1. The protein elutes at ∼ 1M NaCl and is approximately 85% dimeric by SDS PAGE analysis of eluate fractions. The fractions containing pIL-17F are pooled and concentrated against a 10 kDa cutoff ultrafiltration membrane using an Amicon stirred cell in preparation for the final purification and buffer exchange by size exclusion chromatography.

### D) Size exclusion buffer exchange and formulation

The concentrated cation pool (at a volume of 3-4% of CV) is injected at a flow rate of 30 cm/hr onto a Pharmacia Superdex 75 size exclusion column equilibrated in 50 mM Sodium Phosphate buffer containing 109 mM NaCl, pH 7.2. The symmetric eluate peak containing the product is diluted to a concentration of 1 mg/ml in 50 mM Sodium Phosphate buffer containing 109 mM NaCl, pH 7.2. Finally the pIL-17F is 0.2 micron sterile filtered, aliquoted and stored at -80°C. The final process yield is 20%.

### EXAMPLE 5

### Construction of Mammalian Soluble IL-17RC Expression Construct

An expression construct containing human IL-17RC [L21-K451]-mFc1 (mouse BALB/c µ2a Fc) is constructed via overlap PCR and homologous recombination using a DNA fragment (SEQ ID NO:42) encoding a IL-17RC polypeptide (SEQ ID NO:43), a DNA fragment encoding mFc1 (SEQ ID NO:44), and the expression vector pZMP20. The fragments are generated by PCR amplification.

The PCR fragment encoding IL-17RC [L21-K451] contains a 5' overlap with the pZMP20 vector sequence in the optimized tissue plasminogen activator pre-pro secretion leader sequence coding region, the IL-17RC extracellular domain coding [L21-K451], and a 3' overlap with the mFc1 coding region. The PCR amplification reaction uses the 5' oligonucleotide [GTTTCGCTCAGCCAGGAAATCCATGCCGAGTTGAGACGCTTCCGTAGACTGGAGAGGCT TGTGGGGCCT; SEQ ID NO:46], the 3' oligonucleotide [TGTGGGCCCTCTGGGCTCCTTGTGGATGTATTTGTC; SEQ ID NO:47], and a previously generated DNA clone of IL-17RC as the template.

The PCR fragment encoding mFc1 contains a 5' overlap with the IL-17RC sequence, the mFc1 coding region, and a 3' overlap with the pZMP20 vector in the poliovirus internal ribosome entry site region. The PCR amplification reaction uses the 5 oligonucleotide [GACAAATACATCCACAAGGAGCCCAGAGGGCCCACA; SEQ ID NO:48], the 3' oligonucleotide [CAACCCCAGAGCTGTTTTAAGGCGCGCCTCTAGATTATTTACCCGGAGTCCGGGA; SEQ ID NO:49], and a previously generated DNA clone of mFc1 as the template.

The PCR amplification reaction conditions are as follows: 1 cycle, 94 °C, 5 minutes; 35 cycles, 94°C, 1 minute, followed by 55 °C, 2 minutes, followed by 72 °C, 3 minutes; 1 cycle, 72 °C, 10 minutes. The PCR reaction mixtures are run on a 1% agarose gel and the DNA fragments corresponding to the expected sizes are extracted from the gel using a QLAquick™ Gel Extraction Kit (Qiagen, Cat. No. 28704).

The two PCR fragments are joined by overlap PCR. Approximately 1 µl each of the two gel extracted fragments are combined in a PCR amplification reaction using the 5' oligonucleotide [GTTTCGCTCAGCCAGGAAATCGATGCCGAGTTGAGACGCTTGCGTAGACTGGAGAGGGT TGTGGGGCCT; SEQ ID NO: 46] and the 3' oligonucleotide [CAACCCCAGAGCTGTTTTAAGGCGCGCCTCTAGATTATTTACCCGGAGTCCGGGA; SEQ ID NO:49]. PCR conditions used are as follows: 1 cycle, 94 °C, 5 minutes; 35 cycles, 94 °C, 1 minute, followed by 55 °C, 2 minutes, followed by 72 °C, 3 minutes; 1 cycle, 72 °C, 10 minutes. The PCR reaction mixture is run on a 1% agarose gel and the DNA fragment corresponding to the size of the insert is extracted from the gel using a QlAquick™ Gel Extraction Kit (Qiagen, Cat. No. 28704).

Plasmid pZMP20 is a mammalian expression vector containing an expression cassette having the MPSV promoter, a Begin site for linearization prior to yeast recombination, an otPA signal peptide sequence, an internal ribosome entry element from poliovirus, the extracellular domain of CD8 truncated at the C-terminal end of the transmembrane domain; an E. coli origin of replication; a mammalian selectable marker expression unit comprising an SV40 promoter, enhancer and origin of replication, a DHFR gene, and the SV40 terminator; and URA3 and CEN-ARS sequences required for selection and replication in S. cerevisiae.

The plasmid pZMP20 is digested with BglII prior to recombination in yeast with the gel extracted IL-17RC[L21-K451]-mFc1 PCR fragment. 100µl of competent yeast (S. cerevisiae) cells are combined with 10µl of the IL-17RC[L21-K451]-mFc1 insert DNA and 100 ng of BglII digested pZMP20 vector, and the mix is transferred to a 0.2 cm electroporation cuvette. The yeast/DNA mixture is electropulsed using power supply (BioRad Laboratories, Hercules, CA) settings of 0.75 kV (5 kV/cm), ∞ ohms, and 25 µF. Six hundred µl of 1.2 M sorbitol is added to the cuvette, and the yeast is plated in 100 µl and 300 µl aliquots onto two URA-D plates and incubated at 30°C. After about 72 hours, the Ura+ yeast transformants from a single plate are resuspended in 1 ml H2O and spun briefly to pellet the yeast cells. The cell pellet is resuspended in 0.5 ml of lysis buffer (2% Triton X-100, 1% SDS, 100 mM NaCl, 10 mM Tris, pH 8.0, 1 mM EDTA). The five hundred µl of the lysis mixture is added to an Eppendorf tube containing 250 µl acid-washed glass beads and 300 µl phenol-chloroform, is vortexed for 3 minutes, and spun for 5 minutes in an Eppendorf centrifuge at maximum speed: Three hundred µl of the aqueous phase is transferred to a fresh tube, and the DNA is precipitated with 600 µl ethanol, followed by centrifugation for 30 minutes at maximum speed. The tube is decanted and the pellet is washed with 1 mL of 70% ethanol. The tube is decanted and the DNA pellet is resuspended in 30 µl 10 mM Tris, pH 8.0, 1 mM EDTA.

Transformation of electrocompetent E. coli host cells (DH12S) is done using 5 µl of the yeast DNA preparation and 50 µl of E. coli cells. The cells are electropulsed at 2.0 kV, 25 µF, and 400 ohms. Following electroporation, 1 ml SOC (2% Bacto^{™} Tryptone (Difco, Detroit, MI), 0.5% yeast extract (Difco), 10 mM NaCl, 2.5 mM KCl, 10 mM MgCl2, 10 mM MgSO4, 20 mM glucose) is added and then the cells are plated in 50 µl and 200 µl aliquots on two LB AMP plates (LB broth (Lennox), 1.8% Bacto^{™} Agar (Difco), 100 mg/L Ampicillin).

The inserts of three DNA clones for the construct is subjected to sequence analysis and one clone containing the correct sequence is selected. Large scale plasmid DNA is isolated using a commercially available kit (QIAGEN Plasmid Mega Kit, Qiagen, Valencia, CA) according to manufacturer's instructions.

### EXAMPLE 6

### Construction of Mammalian Soluble IL-17RC Expression Constructs that Express IL-17RC-CEE, IL-17RC-CHIS, and IL-17RC-CFLAG

An expression construct containing human IL-17RC [L21-K451] with a C-terminal tag, either Glu-Glu (CEE), six His (CHIS), or FLAG (CFLAG), is constructed via PCR and homologous recombination using a DNA fragment encoding IL-17RC [L21-K451] (SEQ ID NO:42) and the expression vector pZMP20.

The PCR fragment encoding IL-17RCCEE contains a 5' overlap with the pZMP20 vector sequence in the optimized tissue plasminogen activator pre-pro secretion leader sequence coding region, the IL-17RC extracellular domain coding [L21-K451], the sequence of the Glu-Glu tag (Glu Glu Tyr Met Pro Met Glu; SEQ ID NO:53), and a 3' overlap with the pZMP20 vector in the poliovirus internal ribosome entry site region. The PCR amplification reaction uses the 5' oligonucleotide [GTTTCGCTCAGCCAGGAAATCCATGCCGAGTTGAGACGCTTCCGTAGACTGGAGAGGCT TGTGGGGCCT; SEQ ID NO:46], the 3' oligonucleotide [CAACCCCAGAGCTGTITTAAGGCGCGCCTCTAGATTATTCCATGGGGATGTATTCTTCCT TGTGGATGTATTTGTC; SEQ ID NO:50], and a previously generated DNA clone of IL-17RC as the template.

The PCR amplification reaction condition is as follows: 1 cycle, 94 °C, 5 minutes; 35 cycles, 94 °C, 1 minute, followed by 55 °C, 2 minutes, followed by 72 °C, 3 minutes; 1 cycle, 72 °C, 10 minutes. The PCR reaction mixture is run on a 1% agarose gel and the DNA fragment corresponding to the expected size is extracted from the gel using .a QIAquick™ Gel Extraction Kit (Qiagen, Cat. No. 28704).

The plasmid pZMP20 is digested with BglII prior to recombination in yeast with the gel extracted IL-17RCCEE PCR fragment. One hundred µl of competent yeast (S. cerevisiae) cells are combined with 10 µl of the IL-17RCCEE insert DNA and 100 ng of BglII digested pZMP20 vector, and the mix is transferred to a 0.2 cm electroporation cuvette. The yeast/DNA mixture is electropulsed using power supply (BioRad Laboratories, Hercules, CA) settings of 0.75 kV (5 kV/cm), ∞ ohms, and 25 µF. Six hundred µl of 1.2 M sorbitol is added to the cuvette, and the yeast is plated in 100 µl and 300 µl aliquots onto two URA-D plates and incubated at 30°C. After about 72 hours, the Ura+ yeast transformants from a single plate are resuspended in 1 ml H2O and spun briefly to pellet the yeast cells. The cell pellet is resuspended in 0.5 ml of lysis buffer (2% Triton X-100, 1% SDS, 100 mM NaCl, 10 mM Tris, pH 8.0, 1 mM EDTA). The five hundred µl of the lysis mixture is added to an Eppendorf tube containing 250 µl acid-washed glass beads and 300 µl phenol-chloroform, is vortexed for 3 minutes, and spun for 5 minutes in an Eppendorf centrifuge at maximum speed. Three hundred µl of the aqueous phase is transferred to a fresh tube, and the DNA is precipitated with 600 µl ethanol, followed by centrifugation for 30 minutes at maximum speed. The tube is decanted and the pellet is washed with 1 mL of 70% ethanol. The tube is decanted and the DNA pellet is resuspended in 30 µl 10 mM Tris, pH 8.0, 1 mM EDTA.

Transformation of electrocompetent E. coli host cells (DH12S) is done using 5 µl of the yeast DNA preparation and 50 µl of E. coli cells. The cells are electropulsed at 2.0 kV, 25 µF, and 400 ohms. Following electroporation, 1 ml SOC (2% Bacto^{™} Tryptone (Difco, Detroit, MI), 0.5% yeast extract (Difco), 10 mM NaCl, 2.5 mM KCl, 10 mM MgCl2, 10 mM MgSO4, 20 mM glucose) is added and then the cells are plated in 50 µl and 200 µl aliquots on two LB AMP plates (LB broth (Lennox), 1.8% Bacto^{™} Agar (Difco), 100 mg/L Ampicillin).

The inserts of three DNA clones for the construct is subjected to sequence analysis and one clone containing the correct sequence is selected. Large scale Plasmid DNA is isolated using a commercially available kit (QIAGEN Plasmid Mega Kit, Qiagen, Valencia, CA) according to manufacturer's instructions.

The same process is used to prepare the IL-17RC with a C-terminal his tag, composed of Gly Ser Gly Gly His His His His His His (IL-17RCCHIS; SEQ ID NO:51) or the C-terminal FLAG tag , composed of Gly Ser Asp Tyr Lys Asp Asp Asp Asp Lys (IL-17RCCFLAG; SEQ ID NO:52). To prepare these constructs, instead of the 3' oligonucleotide of SEQ ID NO:50; the 3' oligonucleotide [CAACCCCAGAGCTGTTTTAAGGCGCGCCTCTAGATTAGTGATGGTGATGGTGATGTCCA CCAGATCCCTTGTGGATGTATTTGTC; SEQ ID NO:54] is used to generate IL-17RCCHIS or the 3' oligonucleotide [CAACCCCAGAGCTGTTTTAAGGCGCGCCTCTAGATTACTTATCATCATCATCCTTATAAT CGGATCCCTTGTGGATGTATTTGTC; SEQ ID NO:55] is used to generate IL-17RCCFLAG.

### EXAMPLE 7

### Transfection and Expression of Soluble IL-17RC Receptor Expression Constructs that Express the IL-17RC-mFc1 Fusion Protein, and the IL-17RC-CEE, IL-17RC-CHIS, and IL-17RC-CFLAG C-Terminal Tagged Proteins

Three sets of 200 µg of each of the soluble IL-17RC fusion or tagged expression constructs are separately digested with 200 units of PvuI at 37°C for three hours, precipitated with isopropyl alcohol, and centrifuged in a 1.5 mL microfuge tube. The supernatant is decanted off the pellet, and the pellet is washed with 1 mL of 70% ethanol and allowed to incubate for 5 minutes at room temperature. The tube is spun in a microfuge for 10 minutes at 14,000 RPM and the supernatant is decanted off the pellet. The pellet is then resuspended in 750 µl of CHO cell tissue culture medium in a sterile environment, allowed to incubate at 60o C for 30 minutes, and is allowed to cool to room temperature. Approximately 5 x 106 CHO cells are pelleted in each of three tubes and are resuspended using the DNA-medium solution. The DNA/cell mixtures are placed in a 0.4 cm gap cuvette and electroporated using the following parameters; 950 µF, high capacitance, at 300 V. The contents of the cuvettes are then removed, pooled, and diluted to 25 mLs with CHO cell tissue culture medium and placed in a 125 mL shake flask. The flask is placed in an incubator on a shaker at 37 °C, 6% CO2 with shaking at 120 RPM.

The CHO cells are subjected to nutrient selection followed by step amplification to 200 nM methotrexate (MTX), and then to 1 µM MTX. Fusion or tagged protein expression is confined by Western blot, and the CHO cell pool is scaled-up for harvests for protein purification.

### EXAMPLE 8

### Expression of Soluble IL-17RC

An expression plasmid containing IL-17RC-Tbx-C(Fc9) (SEQ ID NO:64) was constructed via homologous recombination using a DNA fragment of IL-17RC _Tbx and the expression vector pZMP40. The fragment was generated by PCR amplification using primers zc44531 and zc44545.

The PCR fragment IL-17RC_Tbx contains a partial IL-17RC extracellular domain coding region, which was made using a previously generated clone of IL-17RC as the template. The fragment includes a 5' overlap with the pZMP40 vector sequence in the otPA coding region, the IL-17RC segment (amino acid residue 21 to 451 of SEQ ID NO:2), a linker sequence, a thrombin cleavage site, and a 3' overlap with the pZMP40 vector in the Fc9 coding region. PCR conditions used were as follows: 1 cycle, 94°C, 5 minutes; 35 cycles, 94°C, 1 minute, followed by 55°C, 2 minutes, followed by 72°C, 3 minutes; 1 cycle, 72°C, 10 minutes.

The PCR reaction mixtures were run on a 1% agarose gel and a band corresponding to the sizes of the inserts were gel-extracted using a QIAquick™ Gel Extraction Kit (Qiagen, Cat. No. 28704).

Plasmid pZMP40 is a mammalian expression vector containing an expression cassette having the MPSV promoter, multiple restriction sites for insertion of coding sequences, an otPA signal peptide sequence, and the sequence for Fc9; an internal ribosome entry site (IRES) element from poliovirus, and the extracellular domain of CD8 truncated at the C-terminal end of the transmembrane domain; an E. coli origin of replication; a mammalian selectable marker expression unit comprising an SV40 promoter, enhancer and origin of replication, a DHFR gene, and the SV40 terminator; and URA3 and CEN-ARS sequences required for selection and replication in S. cerevisiae. It was constructed from pZMP21 (Patent Pub. No. US 2003/0232414 A1; deposited at the American Type Culture Collection and designated as ATCC# PTA-5266).

The plasmid pZMP40 was cut with BglII prior to recombination in yeast with the PCR fragment. One hundred microliters of competent yeast (S. cerevisiae) cells were independently combined with 10 µl of the insert DNA (SEQ ID NO:66) and 100ng of cut pZMP40 vector, and the mix was transferred to a 0.2-cm electroporation cuvette. The yeast/DNA mixture was electropulsed using power supply (BioRad Laboratories, Hercules, CA) settings of 0.75 kV (5 kV/cm), ∞ ohms, and 25 µF. Six hundred µl of 1.2 M sorbitol was added to the cuvette, and the yeast was plated in a 100-µl and 300µl aliquot onto two URA-D plates and incubated at 30°C. After about 72 hours, the Ura+ yeast transformants from a single plate were resuspended in 1 ml H2O and spun briefly to pellet the yeast cells. The cell pellet was resuspended in 0.5 ml of lysis buffer (2% Triton X-100, 1% SDS, 100 mM NaCl, 10 mM Tris, pH 8.0, 1 mM EDTA). The five hundred microliters of the lysis mixture was added to an Eppendorf tube containing 250 µl acid-washed glass beads and 300 µl phenol-chloroform, was vortexed for 3 minutes, and spun for 5 minutes in an Eppendorf centrifuge at maximum speed. Three hundred microliters of the aqueous phase was transferred to a fresh tube, and the DNA was precipitated with 600 µl ethanol (EtOH), followed by centrifugation for 30 minutes at maximum speed. The tube was decanted and the pellet was washed with 1 mL of 70% ethanol. The tube was decanted and the DNA pellet was resuspended in 30 µl TE.

Transformation of electrocompetent E. coli host cells (DH12S) was done using 5 µl of the yeast DNA prep and 50 µl of cells. The cells were electropulsed at 2.0 kV, 25 µF, and 400 ohms. Following electroporation, 1 ml SOC (2% Bacto^{™} Tryptone (Difco, Detroit, Mn, 0.5% yeast extract (Difco), 10 mM NaCl, 2.5 mM KCl, 10 mM MgCl2, 10 mM MgSO4, 20 mM glucose) was added and then the cells were plated in a 50 µl and a 200 µl aliquot on two LB AMP plates (LB broth (Lennox), 1.8% Bacto^{™} Agar (Difco), 100 mg/L Ampicillin).

The inserts of three clones for the construct was subjected to sequence analysis and one clone for each construct, containing the correct sequence, was selected. Larger scale plasmid DNA was isolated using a commercially available kit (QIAGEN Plasmid Mega Kit, Qiagen, Valencia, CA) according to manufacturer's instructions.

Three sets of 200µg of the IL-17RC[L21-K451]_Tbx_C(Fc9) construct were then each digested with 200 units of Pvu I at 37°C for three hours and then were precipitated with IPA and spun down in a 1.5 mL microfuge tube. The supernatant was decanted off the pellet, and the pellet was washed with 1 mL of 70% ethanol and allowed to incubate for 5 minutes at room temperature. The tube was spun in a microfuge for 10 minutes at 14,000 RPM and the supernatants was decanted off the pellet. The pellet was then resuspended in 750 µl of PF-CHO media in a sterile environment, allowed to incubate at 60oC for 30 minutes, and was allowed to cool to room temperature. 5E6 APFDXB11 cells were spun down in each of three tubes and were resuspended using the DNA-media solution. The DNA/cell mixtures were placed in a 0.4 cm gap cuvette and electroporated using the following parameters: 950 µF, high capacitance, and 300 V. The contents of the cuvettes were then removed, pooled, and diluted to 25 mLs with PF-CHO media and placed in a 125 mL shake flask. The flask was placed in an incubator on a shaker at 37°C, 6% CO2, and shaking at 120 RPM.

The cell line was subjected to nutrient selection followed by step amplification to 200nM methotrexate (MTX), and then to 1 µM MTX. Expression was confirmed by western blot, and the cell line was scaled-up and protein purification followed.

### EXAMPLE 9

### Purification of Soluble IL-17RC from CHO Cells

Conditioned media from CHO cells expressing IL-17RC-TbX-Fc9 (SEQ ID NO:64) was concentrated approximately 10-fold with a Pellicon-II tangential flow system against two Biomax 0.1 m2 30kD molecular weight cutoff membrane cassettes (Millipore, Bedford, MA). The concentrated media was pH adjusted to 5.5 with glacial acetic acid, 0.2 □m sterile filtered then loaded onto a Protein G sepharose fast flow resin (Pharmacia, Piscataway, NJ) via batch chromatography overnight at 4C. Prior to loading the pH adjusted conditioned media, the Protein G resin was pre-equilibrated with, 5 column volumes (approximately 150ml) of 25mM sodium acetate, 150mM NaCl, pH5.5. The ratio of filtered, pH adjusted conditioned media to resin was 33:1 (v/v).

The batched chromatography process was performed at ambient room temperature (approximately 21C). The batched, pH adjusted, 0.22 µm filtered, conditioned media was poured into an empty 5.5 x 20.5 cm glass column (BioRad, Hercules, CA) and packed via gravity. The column was washed with 10 column volumes (approximately 300ml) of 25mM sodium acetate, 150mM NaCl, pH5.5. Bound protein was then pH eluted with 100mM glycine, pH 2.7. 9.0ml fractions were collected and immediately neutralized with 1.0 ml 2.0M Tris, pH 8.0. The collected fractions were analyzed via SDS-PAGE Coomassie staining. Fractions containing IL-17RC-Tbx-Fc9 were pooled and concentrated approximately 6-fold using a 5kD molecular weight cutoff Biomax membrane spin concentrator (Millipore, Bedford, MA) according to the manufacturer's instructions.

The pooled, concentrated fractions were then dialyzed ,at 4C, extensively against 1X phosphate buffered saline, pH 7.3 (Sigma, St. Louis, MO) using a 7kD molecular weight cutoff membrane Slide-A-Lyzer (Pierce, Rockford, IL). IL-17RG-TbX-Fc9 as formulated in 1x phosphate buffered saline, pH 7.3 was 0.22 µm sterile filtered prior to aliquoting and storage at -80C.

### EXAMPLE 10

### Binding of IL-17A and IL-17F to Human IL-17RC

### A) Binding of biotinylated cytokines to transfected cells

Baby Hamster Kidney (BHK) cells that had been transfected with expression vectors encoding human IL-17 receptor (SEQ ID NO:21), human IL-17RC (SEQ ID NO:2), or both of these receptors are assessed for their ability to bind biotinylated human IL-17A and human IL-17F. Cells are harvested with versene, counted and diluted to 107 cells per ml in staining media (SM), which is HBSS plus 1 mg/ml bovine serum albumin (BSA), 10 mM Hepes, and 0.1% sodium azide (w/v). Biotinylated human IL-17A (SEQ ID NO:14) and human IL-17F (SEQ ID NO:16) are incubated with the cells on ice for 30 minutes at various concentrations. After 30 minutes, excess cytokine is washed away with SM and the cells are incubated with a 1:100 dilution of streptavidin conjugated to phycoerythrin (SA-PE) for 30 minutes on ice. Excess SA-PE is washed away and cells are analyzed by flow cytometry. The amount of cytokine binding was quantitated from the mean fluorescence intensity of the cytokine staining. From this analysis, we find that human IL-17A binds both the human IL-17R and IL-17RC to a similar extent. Also, human IL-17F binds IL-17RC to a similar level, but binds IL-17R detectably, but to a much lower level than was seen with IL-17A.

### B) Binding of biotinylated cytokines to human peripheral blood mononuclear cells

Human peripheral blood mononuclear cells (PBMC) were prepared from whole blood by ficoll density gradient centrifugation. PBMC at 107 cells per ml were simultaneously incubated with biotinylated IL-17A or IL-17F at 1 µg/ml and fluorochrome conjugated antibodies to specific cell surface proteins that were designed to distinguish various white blood cell lineages lineages. These markers include CD4, CD8, CD19, CD11b, CD56 and CD16. Excess antibody and cytokine are washed away, and specific cytokine binding is detected by incubating with SA-PE as described above. Samples were analyzed by flow cytometry and from this analysis, we find that human IL-17A binds to virtually all PBMC populations examined, but that human IL-17F does not detectably bind to any population.

### C) Inhibition of specific binding of biotinlyated human IL-17A and IL-17F with unlabeled cytokine

Binding studies are performed as discussed above, but excess unlabeled human IL-17A and IL-17F are included in the binding reaction. In studies with BHK cells, the amount of unlabeled cytokine was varied over a range of concentrations and we fmd that addition of unlabeled IL-17A competed for binding of both IL-17A and IL-17F to both IL-17RC and IL-17R. However, unlabeled IL-17F competed for binding of both IL-17A and IL-17F to IL-17RC, but it did not compete effectively for binding to IL-17R. This indicates that both IL-17A and IL-17F specifically bind to IL-17RC, and that they bind at a site that is either identical or overlaps significantly since they cross-compete for binding. Also, IL-17A competes for the relatively weak binding of IL-17F for IL-17R, indicating these two cytokine also bind to a similar region in the IL-17R, but IL-17F binds IL-17R with much reduced affinity relative to IL-17RC.

### D) Inhibition of specific binding of biotinylated human IL-17A and IL-17F with soluble IL-17RC and IL-17R

Binding studies are performed as discussed above, except that a soluble form of IL-17RC or IL-17R are included in the binding reactions. These soluble receptors are fusion proteins derived from the extracellular domain of each receptor fused to the human IgG1 constant (Fc) region. We find that soluble IL-17RC inhibits binding of both human IL-17A and IL-17F to both IL-17R and IL-17RC transfected BHK cells. However, soluble IL-17R inhibits binding of IL-17A to either receptor, but does not effectively block binding of IL-17F to IL-17RC, consistent with the poor binding of IL-17F for the IL-17R.

### EXAMPLE 11

### IL-17A and IL-17F Bind to IL-17RC

### A) Binding Inhibition with Cold Ligand

BHK cells transfected with hIL-17RC (SEQ ID NO:2) and IL-17R (SEQ ID NO:21) were plated at 40,000 cells/well in a 24-well dish (Costar 3527)two days prior to assay. IL-17A (SEX ID NO:14) and IL-17F(SEQ ID NO:16) that had been radiolabeled by the iodobead method were added independently to wells in triplicate at 10ng/ml with a total of 250ul/well in binding buffer (RPMI 1640 media (JRH 51502-500M) with 10mg/ml bovine serum albumin(Gibco 15260-037)). Cold competitors were added in 100 fold molar excess. Competitors tested included IL-17A, IL-17B, IL-17C, IL=17D, IL-17E, IL-17F and IL-21. Wells were incubated on ice for 1-hour followed by two washes with PBS (Invitrogen 20012-027) and one wash with a high salt solution (1.5M NaCL, 50mM HEPES pH 7.4). Wells were extracted with 500ul of 0.8M NaOH for 30min. at room temperature and counts per minute were measured in a gamma counter (Packard Cobra II A5005).

The results indicated that 100x molar cold IL-17A and IL-17F were able to reduce binding of 125I IL-17A to BHK hIL-17RC by approximately 7 fold while IL-17B,C,D,E and IL-21 had no effect on binding. 100x molar cold IL-17A reduced the binding of 125I IL-17A to BHK IL-17R by approximately 4 fold while IL-17B,C,D,E,F and IL-21 had no effect on binding. 100x molar cold IL-17A and IL-17F reduced the binding of 125IL-17F to BHK hIL-17RC by approximately 4 fold and 5 fold, respectively, while IL-17B,C,D,E and IL-21 had no effect on binding. B) Binding Inhibition with Soluble Receptor:

Binding to hzytor14 (SEQ ID NO:2) and IL-17R (SEQ ID NO:21) transfected BHK cells was performed as in one, but 100 fold molar excess soluble hIL-17RCx1/Fc9 (Example 8) and soluble IL-17R/Fc (obtained from R&D; Ref. 177-IR) were used in place of cold ligand in the competition. Cells were washed, extracted and counted as in part one.

Soluble hIL-17RC/Fc inhibited binding of 125IL-17F to BHK hIL-17RC with an IC50 of 10X molar excess average from three experiments. Soluble hIL-17RC/Fc inhibition of 125IIL-17A on the same cell line gave an average IC50 of 20X molar excess and soluble IL-17R/Fc inhibition of 125I IL-17A gave an average IC50 of 20X molar excess.

### C) Binding Saturation

Transfected BHK cells were plated into 24-well dishes as in one. Radiolabeled IL-17A and IL-17F were added starting at a concentration of 4nM in eight 1:3 dilutions (to a concentration of 1.83 pM) in triplicate with a total of 250µl/well in binding buffer. Separately, 100 fold molar excess of cold ligand was added at each dilution point. Cells were washed, extracted and counted as in one. Specific counts per minute were plotted against concentration of radiolabeled ligand added by subtracting the 100 fold excess counts from the the uncompeted counts at each dilution point. These normalized data were plotted to generate saturation binding curves for each combination of radiolabeled ligand and transfected BHK cells. Table 7 shows the affinity values calculated from all three experiments.

**Table 7**

| | | | |
|---|---|---|---|
| 125I IL-17A + BHK hIL-17RC | | 125I IL-17A + BHK IL-17R | |
| 1. | 180pM | 1. | 2.5 +/- 0.2nM |
| 2. | 200pM | 2. | 4.5 +/- 0.3nM |
| 3. | 370pM | 3. | 5.9 +/- 0.1nM |
| 125I IL-17F + BHK hIL-17RC | | 125I IL-17F + BHK IL-17R | |
| 1. | 50pM | 1. | Very low affinity |
| 2. | 60pM | 2. | Very low affinity |
| 3. | 80pM | 3. | Very low affinity |

One-site binding curve fits agreed most closely with IL-17A & IL-17F binding to IL-17R. Two-site binding curve fits agreed most closely with IL-17A and IL-17F binding to hIL-17RC. The high affinity binding site is the value shown above. The low affinity binding site had very low affinity and varied widely between the three experiments.

### EXAMPLE 12

### Murine Nih3t3 Cells Respond to Human IL-17A and IL-17F

### A) Cell plating and kz142 adenovirus reporter infection.

Nih3t3 cells, derived from mouse fibroblasts (described in ATCC) Nih3t3 were plated at 5000 cells/well in solid white, cell culture coated 96 well plates, (Cat. #3917. Costar) using DMEM/10% FBS, containing glutamine and amended witch pyruvate and cultured overnight at 37oC and 5% C02. On this second day, the plating media was removed and Kz142 adenovirus particles at a multiplicity of infection of 5000 particles/cell were prepared in DMEM/1% FBS, containing glutamine and amended with pyruvate and cultured overnight at 37oC and 5% C02. B) Luciferase assay measuring IL-17A and F activation of kz142 adenovirus reporter infected nih3t3 cells.

Following the overnight incubation with the adenovirus particle reporter, human IL-17A and IL-17F Ligand treatments were prepared in serum free media ()amended to .28% BSA. The adenovirus particles and media were removed and the appropriate ligand doses were given in triplicates. Incubation at 37°C and 5% C02 was continued for 4 hours, after which the media was removed, cells lysed for 15 minutes and mean fluorescence intensity (MFI) measured using the luciferase assay system and reagents. (Cat.#e1531 Promega. Madison, WI.) and a Microplate luminometer. Activity was detected at concentrations ranging from .1-1000ng/ml human IL-17A and IL-17F, generating EC50 values of about 50ng/ml for both ligands. These data suggest that nih3t3 cells carry receptors to these ligands and that IL-17A and IL-17F activate the NfKb/Ap-1 transcription factor.

### EXAMPLE 13

### Murine Nih3t3 Cells Express Both IL-17RA and IL-17RC

RTPCR analysis of nih3t3 RNA demonstrated that these cells are positive for both IL-17 RA and IL-17RC, consistent with their nfkb/ap1 response to human IL-17A and IL-17F mediation being mediated through one or both of these receptors.

### RTPCR DETAILS:

### A) Murine IL-17RC PCR

First strand cDNA was prepared from total RNA isolated from nih3t3 cells using standard methods. PCR was applied using hot star polymerase and the manufacturer's recommendations (Qiagen, Valencia, CA) using sense primer, zc38910, 5' ACGAAGCCCAGGTACCAGAAAGAG 3' (SEQ ID NO:56) and antisense primer, zc 38679, 5' AAAAGCGCCGCAGCCAAGAGTAGG 3' (SEQ ID NO:57) and 35 cycles of amplification. Agarose gel electrophoresis revealed a single, robust amplicon of the expected, 850 bp size.

### B) Murine IL-17RA PCR

First strand cDNA was prepared from total RNA isolated from nih3t3 cells using standard methods. PCR was applied using hot star polymerase and the manufacturer's recommendations (Qiagen, Valencia, CA) using sense primer, zc38520, 5' CGTAAGCGGTGGCGGTTTTC 3'(SEQ ID NO:58) and antisense primer, zc 38521, 5' TGGGCAGGGCACAGTCACAG 3' (SEQ ID NO:59) and 35 cycles of amplification. Agarose gel electrophoresis revealed a single, robust amplicon of the expected, 498 bp size.

### EXAMPLE 14

### Creation of a Stable Nih3t3 Assay Clone Expressing the ap1/nfkb Transcription Factor

The murine nih3t3 cell line described above was stably transfected with the kz142 ap1/nfkb reporter construct, containing a neomycin-selectible marker. The Neo resistant transfection pool was plated at clonal density. Clones were isolated using cloning rings and screened by luciferase assay using the human IL-17A ligand as an inducer. Clones with the highest mean fluorescence intensity (MFI) (via ap1/NflcB luciferase) and the lowest background were selected. A stable transfectant cell line was selected and called nih3t3/kz142.8.

### EXAMPLE 15

### Inhibition of Activation by Human IL-17A and IL-17F in Murine Nih3t3 Cells Using Soluble IL-17RC and IL-17RA/FC Chimeras

Soluble forms of IL-17RC or IL-17RA were used as antagonists of human IL-17A and IL-17F activation of ap1/nfkb elements in a luciferase assay. These soluble receptors are fusion proteins derived from the extracellular domain of each receptor fused to the human IgG1 constant (Fc) region. The soluble human IL-17R FC fusion protein was purchased. (recombinant human IL-17R/FC chimera, catalog number 177-IR-100, R&D Systems, Inc., Minneapolis, Mn.) The soluble human IL-17RC FC chimera (IL-17RCsR/FC9) was constructed as described above. We find that an excess IL-17RCsR/FC9 and human IL17RsR/FC chimera inhibit EC50 levels of both human IL-17A and IL-17F mediation of ap1/nfkb activation of the murine nih3t3/kz142.8 assay cell line.

The IL-17RCsR/FC9 protein showed the greatest potency in antagonizing IL-17F activation and IL17RsR/FC chimera showed the greatest potency in antagonizing IL-17A activation.

### EXAMPLE 16

### IL-17F mRNA is Upregulated in a Murine Model of Asthma

IL-17F mRNA levels were measured in a sensitization and airway challenge model in mice. Groups of mice, 8 to 10 wks of age, were sensitized by intraperitoneal injection of 10 ug of recombinant Dermatophagoides pteronyssinus allergen 1 (DerP1) (Indoor biotechnologies, Cardiff, UK) in 50 % Imject Alum (Pierce) on days 0 and 7. Seven days later, mice were challenged on 3 consecutive days (days 14, 15 and 16) with 20 ug of DerP1 in 50 ul PBS. There were 4 mice representing this group. Negative controls included 5 mice given phosphate buffered saline (PBS) sensitization, followed by PBS challenge. In addition to 3 mice given DerP1 sensitization, followed by PBS challenge. Forty-eight hours following allergen, or control challenge whole lung tissue was harvested and total RNA was isolated.

First strand cDNA was prepared using identical amounts of total RNA from each subject. IL-17F PCR was applied using Qiagen hotstar polymerase (Qiagen, Valencia, CA) and the manufacturer's recommendations. The IL-17F PCR utilized 35 cycles of amplification with sense primer, zc46098, 5' ACTTGCCATTCTGAGGGAGGTAGC 3' (SEQ ID NO:60) and antisense primer, 46099, 5' CACAGGTGCAGCCAACTTTTAGGA 3' (SEQ ID NO:61). In order to establish that the template quality was uniform amongst all subjects, Beta Actin PCR was applied to the same amount of each template used in the IL-17F amplification. B actin PCR included 25 cycles of PCR with sense primer, zc44779, 5' GTGGGCCGCTCTAGGCACCA 3' (SEQ ID NO:62) and antisense primer, zcc44776, 5' CGGTTGGCCTTAGGGTTCAGGGGGG 3' (SEQ ID NO:63).

All 4 mice from the DerP1 sensitized, DerP1 challenged treatment group (the asthma simulation) showed robust IL-17F amplification. In contrast, weak IL-17F amplification was seen from the negative controls, including 3 of 3 subjects representing the DerP1 sensitized/PBS challenged treatment group and 5 of 5 subjects from the PBS sensitized/PBS challenged treatment group. B actin amplification was at least as robust for the negative controls as for the asthma-simulated subjects, demonstrating that the weak negative control IL-17F amplification was not due to template problems.

### EXAMPLE 17

### COS Cell Transfection and Secretion Trap

### A) Cos cell transfection and secretion trap assays show that IL-17RCsR/Fc9 and IL-17F is a receptor/ligand pair

A secretion trap assay was used to match the human IL-17RC (SEQ ID NO:2) to the human IL-17F (SE QID NO: 16). The soluble IL-17RCsR/Fc9 fusion protein (Example 8) was used as a binding reagent in a secretion assay. SV40 ori containing expression vectors containing cDNA of human IL-17B,C,D,E, and F was transiently transfected into COS cells. The binding of IL-17RCsR/Fc9 to transfected COS cells was carried out using the secretion trap assay described below. Positive binding of IL-17RCsR/Fc9 was only seen to human IL-17F. These results demonstrate the novel finding that human IL-17RC and IL-17F is a receptor/ligand pair.

### B) COS Cell Transfections

The COS cell transfection was performed as follows: Mix 3ul pooled DNA and 5ul Lipofectamine^{™} in 92ul serum free DMEM media (55mg sodium pyruvate, 146mg L-glutamine, 5mg transferrin, 2.5mg insulin, 1µg selenium and 5mg fetuin in 500ml DMEM), incubate at room temperature for 30 minutes and then add 400ul serum free DMEM media. Add this 500ul mixture onto 1.5x105 COS cells/well plated on 12-well tissue culture plate and incubate for 5 hours at 37°C. Add 500ul 20% FBS DMEM media (100 ml FBS, 55 mg sodium pyruvate and 146mg L-glutamine in 500ml DMEM) and incubate overnight.

### C) Secretion Trap Assay

The secretion trap was performed as follows: Media was rinsed off cells with PBS and then fixed for 15 minutes with 1.8% Formaldehyde in PBS. Cells were then washed with TNT (0.1M Tris-HCL, 0.15M NaCl, and 0.05% Tween-20 in H2O), and permeated with 0.1% Triton-X in PBS for 15 minutes, and again washed with TNT. Cells were blockd for 1 hour with TNB (0.1M Tris-HCL, 0.15M NaCl and 0.5% Blocking Reagent (NEN Renaissance TSA-Direct Kit) in H2O), and washed again with TNT. The cells were incubated for 1 hour with 1µg/ml human IL-17RCxlsR/FC9 soluble receptor fusion protein Cells were then washed with TNT. Cells were incubated for another hour with 1:200 diluted goat-anti-human Ig-HRP (Fc specific). Again cells were washed with TNT.

Positive binding was detected with fluorescein tyramide reagent diluted 1:50 in dilution buffer (NEN kit) and incubated for 4-6 minutes, and washed with TNT. Cells were preserved with Vectashield Mounting Media (Vector Labs Burlingame, CA) diluted 1:5 in TNT. Cells were visualized using a FITC filter on fluorescent microscope.

### EXAMPLE 18

### Generation of Murine Anti-Human IL-17RC Monoclonal Antibodies

### A. Immunization for generation of anti-IL-17RC Antibodies

### 1. Soluble IL-17RC-muFc

Six to twelve week old intact or IL-17RC knockout mice are immunized by intraperitoneal injection with 25-50 ug of soluble human IL-17RC-muFc protein (Example 23) mixed 1:1 (v:v) with Ribi adjuvant (Sigma) on a biweekly schedule. Seven to ten days following the third immunization, blood samples were taken via retroorbital bleed, the serum harvested and evaluated for its ability to inhibit the binding of IL-17 or IL-17F to IL-17RC in neutralization assays (e.g., described herein) and to stain IL-17RC transfected versus untransfected 293 cells in a FACS staining assay. Mice continued to be immunized and blood samples taken and evaluated as described above until neutralization titers reached a plateau. At that time, mice with the highest neutralization titers were injected intravascularly with 25-50 ug of soluble IL-17RC-Fc protein in PBS. Three days later, the spleen and lymph nodes from these mice were harvested and used for hybridoma generation, for example using mouse myeloma (P3-X63-Ag8.653-3.12.11) cells or other appropriate cell lines in the art, using standard methods known in the art (e.g., see Kearney, J.F. et al., J Immunol. 123:1548-50, 1979; and Lane, R.D. J Immunol Methods 81:223-8, 1985).

### 2. Soluble IL-17RC, IL-17RC-CEE, IL-17RC-CHIS, IL-17RC-CFLAG

Six to twelve week old intact or IL-17RC knockout mice are immunized by intraperitoneal injection with 25-50 ug of soluble human IL-17RC-CEE, IL-17RC-CHIS, or IL-17RC-CFLAG mixed 1:1 (v:v) with Ribi adjuvant (Sigma) on a biweekly schedule. Seven to ten days following the third immunization, blood samples are taken via retroorbital bleed, the serum harvested and evaluated for its ability to inhibit the binding of IL-17 or IL-17F to IL-17RC in neutralization assays (e.g., described herein) and to stain IL-17RC transfected versus untransfected 293 cells in a FACS staining assay. Mice are continued to be immunized and blood samples taken and evaluated as described above until neutralization titers reached a plateau. At that time, mice with the highest neutralization titers are injected intravascularly with 25-50 ug of soluble IL-17RC, IL-17RC-CEE, zcytor-CHIS, or IL-17RC-CFLAG antigen protein in PBS. Three days later, the spleen and lymph nodes from these mice are harvested and used for hybridoma generation, for example using mouse myeloma (P3-X63-Ag8.653.3.12.11) cells or other appropriate cell lines in the art, using standard methods known in the art (e.g., see Kearney, J.F. et. al., J Immunol. 123:1548-50, 1979; and Lane, R.D. J Immunol Methods 81:223-8, 1985).

### 3. P815 transfectants that express the IL-17RC

Six to ten week old female DBA/2 mice are immunized by intraperitoneal injection of 1 x 105 live, transfected P815 cells, for example P815/IL-17RC cells (e.g., 0.5 ml at a cell density of 2 x 105 cells/ml). Prior to injection, the cells are maintained in the exponential growth phase. For injection the cells are harvested, washed three times with PBS and then resuspended in PBS to a density of 2 x 105 cells/ml. In this model, the mice develop an ascites tumor within 2-3 weeks and progress to death by 4-6 weeks unless an immune response to the transfected target antigen has been mounted. At three weeks mice with no apparent abdominal swelling (indicative of ascites) are re-immunized as above at 2-3 week intervals. Seven to ten days following the second immunization, blood samples are taken via retroorbital bleed, the serum harvested and evaluated for its ability to inhibit the binding of IL-17 or IL-17F to IL-17 or IL-17RC in neutralization assays (e.g., described herein) and to stain IL-17RC transfected versus untransfected 293 cells in a FACS staining assay. Mice continue to be immunized and blood samples taken and evaluated as described above until neutralization titers reach a plateau. At that time, the mice with the highest neutralization titers are injected intraperitonealy with 1 x 105 live, transfected P815 cells. Four days later, the spleen and lymph nodes from these mice are harvested and used for hybridoma generation, for example using mouse myeloma (P3-X63-Ag8.653.3.12.11) cells or other appropriate cell lines in the art, using standard methods known in the art (e.g., see Kearney, J.F. et al., supra.; and Lane, R.D. supra.).

An alternative to the above immunization scheme with live, transfected P815 cells involves intraperitoneal injection of 1-5 x 106 irradiated, transfected cells every 2-3 weeks. In this approach, no animals develop and die of ascites. Instead, animals are monitored for a neutralizing immune response to IL-17RC in their serum as outlined above, starting with a bleed after the second immunization. Once neutralization titers have reached a maximal level, the mice with highest titers are given a pre-fusion, intraperitoneal injection of 5 x 106 irradiated cells and four days later, the spleen and lymph nodes from these mice are harvested and used for hybridoma generation, for example using mouse myeloma (P3-X63-Ag8.653.3.12.11) cells or other appropriate cell lines in the art, using standard methods known in the art (e.g., see Kearney, J.F. et al., supra.; and Lane, R.D. supra.).

### B. Screening the Hybridoma Fusions for Antibodies that bind IL-17RC and Inhibit the Binding of IL-17 or IL-17F to IL-17RC

Three different primary screens are performed on the hybridoma supernatants at 8-10 days post-fusion. For the first assay, antibodies in supernatants were tested for, their ability to bind to plate bound soluble human IL-17RC, IL-17RC-muFc, IL-17RC-CEE, IL-17RC-CHIS, or IL-17RC-CFLAG protein by ELISA using HRP-conjugated goat anti-mouse kappa and anti-lambda light chain second step reagents to identify bound mouse antibodies. To demonstrate specificity for the IL-17RC portion of the IL-17RC fusion proteins, positive supernatants in the initial assay were evaluated on an irrelevant protein fused to the same murine Fc region (mG2a), EE sequence, HIS sequence, or FLAG sequence. Antibody in those supernatants that bound to IL-17RC-fusion protein and not the irrelevant muFc or other proteins containing fusion protein sequence were deemed to be specific for IL-17RC. For the second assay, antibodies in all hybridoma supernatants were evaluated by ELISA for their ability to inhibit the binding of biotinylated human IL-17 or biotinylated human IL-17F to plate bound IL-17RC-muFc or IL-17RC-fusion proteins.

All supernatants containing antibodies that bound specifically to IL-17RC, whether they inhibited the binding of IL-17 or IL-17F to IL-17RC or not in the ELISA assay, were subsequently tested for their ability to inhibit the binding of IL-17 or IL-17F to IL-17RC transfected Baf3 or BHK cells or normal human bronchial epithelial cells. All supernatants that were neutralization positive in either the IL-17 or IL-17F inhibition assays or both the IL-17 and IL-17F inhibition assays were subsequently evaluated for their ability to stain IL-17RC transfected versus non-transfected Baf3 or BHK cells by FACS analysis. This analysis was designed to confirm that inhibition of IL-17 or IL-17F binding to IL-17RC, was indeed due to an antibody that specifically binds the IL-17RC receptor. Additionally, since the FACS analysis was performed with an anti-IgG second step reagent, specific FACS positive results indicate that the neutralizing antibody was likely to be of the IgG class. By these means, a master well was identified that bound IL-17RC in the plate bound ELISA, inhibited the binding of IL-17 or IL-17F to IL-17RC in the ELISA based inhibition assay, blocked the interaction of IL-17 and IL-17F with IL-17RC transfected Baf3 or BHK cells, respectively, and was strongly positive for the staining of IL-17RC transfected Baf3 or BHK cells with an anti-mouse IgG second step reagent.

The third assay consists of primary human bronchial epithelial cells which express IL-17RC and can be induced to secrete IL-8 or IL-6 in response to IL-17F treatment. The specific monoclonal antibody is assayed by its ability to inhibit the IL-17 or IL-17F stimulated IL-8 or IL-6 production by these cells. IL-8 and IL-6 production is assayed in response to IL-17 or IL-17F as described herein.

Alternatively, the monoclonal antibody; anti-IL-17RC, mediated inhibition of IL-17 or IL-17F induced luciferase production in NIH 3T3 or other IL-17RC containing cells can be used with or in place of one of the bioactivity neutralization assays noted above. The NFkB mediated luciferase assay in NIH 3T3 cells is described herein.

### C) Cloning Anti-IL-17RC Specific Antibody Producing Hybridomas

Hybridoma cell lines producing a specific anti-IL-17RC mAb that cross-neutralized the binding of IL-17 and IL-17F to appropriately transfected BaF3 or BHK cells are cloned by a standard low-density dilution (less than 1 cell per well) approach. Approximately 5-7 days after plating, the clones are screened by ELISA on, for example, plate bound human IL-17RC-muFc followed by a retest of positive wells by ELISA on irrelevant muFc containing fusion protein as described above.. Selected clones, whose supernatants bind to IL-17RC-muFc and not the irrelevant muFc containing fusion protein, are further confirmed for specific antibody activity by repeating both neutralization assays as well as the FACS analysis. All selected IL-17RC antibody positive clones are cloned a minimum of two times to help insure clonality and to assess stability of antibody production. Further rounds of cloning are performed and screened as described until, preferably, at least 95% of the resulting clones were positive for neutralizing anti-IL-17RC antibody production.

### D) Biochemical Characterization of the Molecule Recognized by Anti-IL-17RC mAbs

Biochemical confirmation that the target molecule, IL-17RC, recognized by the putative anti-IL-17RC mAbs is indeed IL-17RC are performed by standard immunoprecipitation followed by SDS-PAGE analysis or western blotting procedures, both employing soluble membrane preparations from IL-17RC transfected versus untransfected Baf3 or BHK cells. Moreover, soluble membrane preparations of non-transfected cell lines that express IL-17RC are used show that the mAbs recognize the native receptor chain as well as the transfected one. Alternatively, the mAbs are tested for their ability to specifically immunoprecipitate or western blot the soluble IL-17RC-muFc protein.

### EXAMPLE 19

### Neutralization of Human IL-17RC by Sera from Mice Injected with P815 Cells Transfected with Human IL-17RC

Using a cell based neutralization assay, serum from mice injected with live human IL-17RC transfected P815 cells (Example 17) is added as a serial dilution at 1%, 0.5%, 0.25%, 0.13%, 0.06%, 0.03%, 0.02%, and 0%. The assay plates are incubated at 37□C, 5% CO2 for 4 days at which time Alamar Blue (Accumed, Chicago, IL) is added at 20µl/well. Plates are again incubated at 37□C, 5% CO2 for 16 hours. Results showed that serum from four of the animals could neutralize signaling of both huIL-17 and huIL-17F through human IL-17RC.

Results such as these provide additional evidence that effectively blocking IL-17RC by binding, blocking, inhibiting, reducing, antagonizing or neutralizing IL-17 or IL-17F activity (individually or together), for example via a neutralizing monoclonal antibody to IL-17RC of the present invention, could be advantageous in reducing the effects of IL-17 and IL-17F (alone or together) in vivo and may reduce IL-17 and/or IL-17F-induced inflammation, such as that seen in, for example in psoriasis, IBD, colitis, chronic obstructive pulmonary disease, cystic fibrosis or other inflammatory diseases induced by IL-17, and or IL-17F including IBD, arthritis, asthma, psoriatic arthritis, colitis, inflammatory skin conditions, and atopic dermatitis.

### EXAMPLE 20

### Pharmacokinetics of an Anti-human IL-17RC Monoclonal Antibody

The test monoclonal antibody, anti-human IL-17RC mAb, is provided in, for example, 3x 3 mL aliquots at a concentration of approximately 1 mg/mL (determined by UV Absorbance at 280 nM) and was stored at -80 °C until use. The vehicle is 1X PBS (50mM NaPO4, 109mM NaCl), pH 7.3. The mAb is thawed at room temperature before use and aliquots 1 and 2 are used as provided for the 100 µg IV and SC dosing groups, respectively. Half of aliquot 3 is diluted 1:2 in 1X PBS for the 50 µg SC dose group and the second half of aliquot 3 is diluted 1:10 in 1X PBS for the 10 µg SC dose group. Female SCID mice (n=96) are obtained from Charles River Labs. Animals are checked for health on arrival and group-housed (3 animals per cage). The mice are 12 weeks old with an average body weight of approximately 22 g at the beginning of the study.

### A) Dosing Protocol

Female SCID mice (n=24/dose group) are randomly placed into four dosing groups (Table 8). Group 1 was administered the anti-human IL-17RC mAb via IV injection of approximately 93 µL in a tail vein and Groups 2, 3, and 4 are administered the mAb via SC injection of approximately 93 µL in the scruff of the neck.

### B) Sample Collection

Prior to blood collection, mice were fully anesthetized with halothane or isofluorane. Blood samples were collected via cardiac stick for all time points except the 168 hr timepoint (collected via eye bleed and the same animals were bled again at the 504 hr timepoint via cardiac stick). Blood was collected into serum separator tubes and allowed to clot for 15 minutes. Samples were subsequently centrifuged for 3 minutes at 14,000 rpm. Following centrifugation, aliquots of 125-150uL were dispensed into labeled eppendorf tubes and immediately stored at -80 °C until analysis.

**Table 8**

| **Group #** | **Dose (ROA)** | **Animals** | **PK Timepoints** |
|---|---|---|---|
| 1 | 100 µg (IV) | 3 mice/timepoint* | 0.25, 1, 4, 8, 24, 72, 168, 336 and 504 hr |
| 2 | 100 µg (SC) | 3 mice/timepoint* | 0.25, 1, 4, 8, 24, 72, 168, 336 and 504 hr |
| 3 | 50 µg (SC) | 3 mice/timepoint* | 0.25, 1, 4, 8, 24, 72, 168, 336 and 504 hr |
| 4 | 10 µg (SC) | 3 mice/timepoint* | 0.25, 1, 4, 8, 24, 72, 168, 336 and 504 hr |

| | | | |
|---|---|---|---|
| * The same animals were used for the 168 and 504 hr timepoints. | | | |

### C) Quantification of Serum Anti-human IL-17RC mAb Concentrations by ELISA

An Enzyme Linked Immunosorbant Assay (ELISA) is developed and qualified to analyze mouse serum samples from animals dosed with anti-IL-17RC mAb during pharmacokinetic studies. This assay is designed to take advantage of a commercially available secondary antibody and colorimetric detection using TMB. The dilutions used for the standard curve were modified to improve the definition of the linear portion of the standard curve. A standard curve in the range of 100 ng/mL to 0.231 ng/mL with 2-fold dilutions allows for quantitation of the mouse serum samples. QC samples are diluted to 1:100, 1:1000 and 1:10000 in 10% SCID mouse serum and back calculated from the standard curve.

### D) Pharmacokinetic Analysis

Serum concentration versus time data are downloaded into WinNonlin Professional 4.0 software (Pharsight, Inc.; Cary, NC) for pharmacokinetic analysis. Noncompartmental analysis is used to determine pharmacokinetic parameters based on the mean data at each time point.

### EXAMPLE 21

### Neutralization of IL-17A and IL-17F Activity by a Anti- Human IL-17RC Monoclonal Antibody

Using a cell-based neutralization assay, a purified mouse anti-human IL-17RC monoclonal antibody is added as a serial dilution, for example, at 10µg/ml, 5µg/ml, 2.5µg/ml, 1.25µg/ml, 625ng/ml, 313ng/ml, 156ng/ml and 78ng/ml. The assay plates are incubated at 37°C, 5% CO₂ for 4 days at which time Alamar Blue (Accumed, Chicago, IL) is added at 20µl/well Plates are again incubated at 37°C, 5% CO₂ for 16 hours. This assay is able to demonstrate that the purified anti-human IL-17RC monoclonal antibody is able neutralize signaling of both huIL-17 and huIL-17F through human IL-17RC. For highly effective antibodies, when used at approx. 10µg/ml concentration, the antibody completely neutralizes proliferation induced by huIL-17 or huIL-17F, with the inhibition of proliferation decreasing in a dose dependent fashion at the lower concentrations. An isotype-matched negative control mouse mAb, tested at the concentrations described above, is exected to provide no inhibition of proliferation of either cytokine. These results are able to further demonstrate that monoclonal antibodies to IL-17RC could indeed antagonize the activity of the pro-inflammatory ligands, IL-17 and IL-17F at low concentrations.

### EXAMPLE 22

### IL-17A Induces Elevated Levels of IFN-gamma and TNF-alpha in Human Peripheral Blood Mononuclear Cells

Human peripheral blood mononuclear cells (PBMC) are purified by ficoll density gradient centrifugation and then incubated overnight at 37°C in media alone, 50 ng/ml anti-human CD3 antibody, or the combination of 50 ng/ml anti-human CD3 antibody plus 1 µg/ml anti-human CD28 antibody. Replicate cultures for each of these conditions are set up and are given no cytokine, 25 ng/ml human IL-17A, or 25 ng/ml human IL-17F. After 24-hour incubations, supernatants from each culture are harvested and assayed for cytokine content using B-D Bioscience's human Th1/Th2 Cytometric Bead Array (CBA). We found that cultures that had been stimulated with either anti-CD3 or anti-CD3 plus anti-CD28 and had been supplemented with IL-17A contained significantly elevated levels of IFN-gamma and TNF-alpha (3-5-fold elevation of each) over cultures with no cytokine added or those that received IL-17F. Cultures in which no anti-CD3 stimulation was added did not show significant changes in cytokine levels. In addition, IL-17A addition induced no significant changes in other cytokines assayed for with the CBA including IL-2, IL-4, IL-5, and IL-10. This data indicates that IL-17A, but not IL-17F, can augment the production of IFN-gamma and TNF-alpha in PBMC cultures stimulated with anti-CD3 or anti-CD3 plus anti-CD28.

### EXAMPLE 23

### IL-17RC-Fc Decreases Disease Incidence and Progression

### in Mouse Collagen Induced Arthritis (CIA) Model

### A) Mouse Collagen Induced Arthritis (CIA) Model

Ten week old male DBA/1J mice (Jackson Labs) are divided into 3 groups of 13 mice/group. On day-21, animals are given an intradermal tail injection of 50-100 µl of 1mg/ml chick Type II collagen formulated in Complete Freund's Adjuvant (prepared by Chondrex, Redmond, WA), and three weeks later on Day 0 they are given the same injection except prepared in Incomplete Freund's Adjuvant. IL-17RC-Fc is administered as an intraperitoneal injection 3 times a week for 4 weeks, at different time points ranging from Day 0, to a day in which the majority of mice exhibit moderate symptoms of disease. Groups receive either 10 or 100 µg of IL-17RC-Fc per animal per dose, and control groups receive the vehicle control, PBS (Life Technologies, Rockville, MD). Animals begin to show symptoms of arthritis following the second collagen injection, with most animals developing inflammation within 1.5-3 weeks. The extent of disease is evaluated in each.paw by using a caliper to measure paw thickness, and by assigning a clinical score (0-3) to each paw: 0=Normal, 0.5=Toe(s) inflamed, 1=Mild paw inflammation, 2=Moderate paw inflammation, and 3=Severe paw inflammation as detailed below.

### B) Monitoring Disease

Animals can begin to show signs of paw inflammation soon after the second collagen injection, and some animals may even begin to have signs of toe inflammation prior to the second collagen injection. Most animals develop arthritis within 1.5-3 weeks of the boost injection, but some may require a longer period of time. Incidence of disease in this model is typically 95-100%, and 0-2 non-responders (determined after 6 weeks of observation) are typically seen in a study using 40 animals. Note that as inflammation begins; a common transient occurrence of variable low-grade paw or toe inflammation can occur. For this reason, an animal is not considered to have established disease until marked, persistent paw swelling has developed.

All animals are observed daily to assess the status of the disease in their paws, which is done by assigning a qualitative clinical score to each of the paws. Every day, each animal has its 4 paws scored according to its state of clinical disease. To determine the clinical score, the paw can be thought of as having 3 zones, the toes, the paw itself (manus or pes), and the wrist or ankle joint. The extent and severity of the inflammation relative to these zones is noted including: observation of each toe for swelling; torn nails or redness of toes; notation of any evidence of edema or redness in any of the paws; notation of any loss of fine anatomic demarcation of tendons or bones; evaluation of the wrist or ankle for any edema or redness; and notation if the inflammation extends proximally up the leg. A paw score of 1, 2, or 3 is based first on the overall impression of severity, and second on how many zones are involved. The scale used for clinical scoring is shown below.

### C) Clinical Score

0 = Normal
0.5 = One or more toes involved, but only the toes are inflamed
1 = mild inflammation involving the paw (1 zone), and may include a toe or toes
2 = moderate inflammation in the paw and may include some of the toes and/or the wrist/ankle (2 zones)
3 = severe inflammation in the paw, wrist/ankle, and some or all of the toes (3 zones)

Established disease is defined as a qualitative score of paw inflammation ranking 2 or more, that persists for two days in a row. Once established disease is present, the date is recorded and designated as that animal's first day with "established disease".

Blood is collected throughout the experiment to monitor serum levels of anti-collagen antibodies, as well as serum immunoglobulin and cytokine levels. Serum anti-collagen antibodies correlate well with severity of disease. Animals are euthanized on Day 21, and blood collected for serum and CBC's. From each animal, one affected paw is collected in 10%NBF for histology and one is frozen in liquid nitrogen and stored at -800C for mRNA analysis. Also, 1/2 spleen, 1/2 thymus, 1/2 mesenteric lymph node, one liver lobe and the left kidney are collected in RNAlater for RNA analysis, and .1/2 spleen, 1/2 thymus, 1/2 mesenteric lymph node, the remaining liver, and the right kidney are collected in 10% NBF for histology. Serum is collected and frozen at -800C for immunoglobulin and cytokine assays.

Groups of mice receiving IL-17RC-Fc at all time points are characterized by a delay in the onset and/or progression of paw inflammation. These results indicate that IL-17RC can reduce inflammation, as well as disease incidence and progression associated with this model. These results are further supported by the observation that IL-17RC-Fc resulted in decreased levels of serum TNFa, IL-1b, and anti-collagen antibodies.

### EXAMPLE 24

### Stable Over-Expression of IL-17RC in the Murine Assay Cell Line, Nih3t3/kz142.8 Expressing the ap1/nfkb Transcription Factor

The murine nih3t3/kz142.8 assay cell line was transfected with a human IL-17RCx1 (SEQ ID NO:2) in an expression vector with a methotrexate resistance gene (dihydrofolate reductase,DHFR) This transfection was performed using a commercially available kit and the manufacturer's recommendations. (Mirus, Madison,WI. Cat. #MIR218) Cells were placed in 1 µM mtx amended growth medium to select for the expression vector containing the human IL-17RCX1 transgene. After selection a human IL-17RCx1 transfection pool was generated, and called nih3t3/kz142.8/hcytor14x1.

### A) Luciferase assay using the nih3t3/kz142.8 assay cell line

Since nih3t3/kz142.8 has a stable kz142 reporter, there is no need for adenovirus infection to add this reporter. Thus the luciferase assay protocol was shorted and done the following way:

### 1. Cell plating

nih3t3/kz142.8 cells were plated at 5000 cells/well in solid white, cell culture coated 96 well plates, (Cat. #3917. Costar) using DMEM/10% FBS, containing glutamine and amended with pyruvate and cultured overnight at 37oC and 5% C02. On this second day, the plating media was removed and exchanged for DMEM/1% FBS, containing glutamine and amended with pyruvate and cultured overnight at 37oC and 5% C02.

### 2. Luciferase assay measuring IL-17A and F activation of the stable kz142 reporter

Following the overnight incubation in the 1% fbs, DMEM media, human IL-17A,and IL-17F ligand dilutions were made in serum free media, amended with BSA to a .28% level. After adding the ligand dilutions, cells were incubated at 37oC and 5% C02 for 4 hours, after which the media was removed, cells lysed for 15 minutes and mean fluorescence intensity (MFI) measured using the luciferase assay system and reagents, (Cat.#e1531 Promega. Madison, WI.) and a Microplate luminometer. Activity was detected for both ligands at concentrations ranging from .1-1000ng/ml. The nih3t3/kz142.8/hcytor14x1 transfection pool showed similar activity for the murine IL-17A ligand as did the parental cell line. (example 14) However, the cytor14x1 transfectant pool showed an elevated responsiveness to human IL-17A and F treatments, even when these ligand concentrations were as low as 20 femptograms. The fact that the mIL-17A signaling is comparable to that in the parental cell line (example14) suggests that there isn't a general, non-specific problem with human IL-17RC-expressing cells and that the murine IL-17A is probably signaling through the endogenous murine nih3t3 cell IL-17R or IL-17RC receptor. Thus, the fact that human IL-17A and IL-17F cause an elevation of MFI at such low ligand concentrations may indicate a specific hyper-responsiveness of the cells to those ligands, which is mediated through the over-expressed human IL-17RC receptor.

This result has significant clinical and biological ramifications and utility. For example, physiological situations could cause local up-regulation of the IL-17RC receptors which could then make these areas hyper-responsive to IL-17A and IL-17F, resulting in biological activation at much lower ligand concentrations than those suggested without IL-17RC over-expression. Thus, far lower soluble receptor levels may be sufficient to antagonize these hypothetically lower ligand concentrations, than previously thought or recognized by those in the field.

### EXAMPLE 25

### Antagonists to IL-17F and IL-17A Activity Decrease Disease Incidence and Progression in an Inflammatory Bowel Disease (IBD) Model

This model is designed to show that cultured intestinal tissue from patients with IBD produce higher levels of inflammatory mediators compared to tissue from healthy controls. This enhanced production of inflammatory mediators (including but not limited to IL-1b, IL-4, IL-5, IL-6, IL-8, IL-12, IL-13, IL-15, IL-17 A and F, IL-18, IL-23, TNF-a, IFN-g, MIP family members, MCP-1, G- and GM-CSF, etc.) contributes to the symptoms and pathology associated with IBDs such as Crohn's disease (CD) and ulcerative colitis (UC) by way of their effect(s) on activating inflammatory pathways and downstream effector cells. These pathways and components then lead to tissue and cell damage/destruction observed in vivo. Therefore, this model can simulate this enhanced inflammatory mediator aspect of IBD. Furthermore, when intestinal tissue from healthy controls or from human intestinal epithelial cell (IEC) lines is cultured in the presence of these inflammatory components, inflammatory pathway signaling can be observed, as well as evidence of tissue and cell damage.

Therapeutics that would be efficacious in human IBD in vivo would work in the above ex vivo or IEC models by inhibiting and/or neutralizing the production and/or presence of inflammatory mediators.

In this model, human intestinal tissue is collected from patients with IBD or from healthy controls undergoing intestinal biopsy, re-sectioning or from post-mortem tissue collection, and processed using a modification of Alexakis et al (Gut 53:85-90; 2004). Under aseptic conditions, samples are gently cleaned with copious amounts of PBS, followed by culturing of minced sections of tissue, in the presence of complete tissue culture media (plus antibiotics to prevent bacterial overgrowth). Samples from the same pool of minced tissue are treated with one of the following: vehicle (PBS); recombinant human (rh) IL-17A; rhIL-17F; or rhIL-17A+rhIL-17F. In addition, these are treated with or without an antagonist of either IL-17A or IL-17F, alone or in combination (such as a soluble IL-17RC). This experimental protocol is followed for studies with human IEC lines, with the exception that cells are passaged from existing stocks. After varying times in culture (from 1 h to several days), supernatants are collected and analyzed for levels of inflammatory mediators, including those listed above. In samples from patients with IBD or in samples treated with rhIL-17A and/or F, levels of inflammatory cytokines and chemokines are elevated compared to untreated healthy control tissue samples. The addition of antagonists to IL-17F and/or IL-17A activity, such as IL-17RC soluble receptors and antibodies thereto including the anti-human-IL-17RC monoclonal and neutralizing antibodies of the present invention markedly reduces the production of inflammatory mediators, and thus, would expect to be efficacious in human IBD.

### EXAMPLE 26

### Antagonists to IL-17F and IL-17A activity Decrease Disease Incidence and Progression in a Multiple Sclerosis (MS) Model

Multiple sclerosis (MS) is a complex disease that is thought to be mediated by a number of factors, including the presence of lymphocytic and mononuclear cell inflammatory infiltrates and demyelination throughout the CNS. Microglia are macrophage-like cells that populate the central nervous system (CNS) and become activated upon injury or infection. Microglia have been implicated as playing critical roles in various CNS diseases including MS, and may be used to study mechanism(s) of initiation, progression, and therapy of the disease (Nagai et al. Neurobiol Dis 8:1057-1068; 2001; Olson et al. J Neurosci Methods 128:33-43; 2003). Immortalized human microglial cell lines and/or established human astroglia cell lines can, therefore, be used to study some of the effects of inflammatory mediators on these cell types and their potential for neutralization. Inflammatory mediators (including but not limited to IL-1b, IL-6, IL-8, IL-12, IL-13, IL-15, IL-17 A and F, IL-18, IL-23, TNF-a, IFN-g, MIP family members, RANTES, IP-10, MCP-1, G- and GM-CSF, etc.) can contribute to the symptoms and pathology associated with MS by way of their effect(s) on activating inflammatory pathways and downstream effector cells.

In order to evaluate the pro-inflammatory actions of IL-17A and IL-17F, and the ability of an antagonist to IL-17F and/or IL-17A activity, such as IL-17RC soluble receptors and antibodies thereto including the anti-human-IL-17RG monoclonal and neutralizing antibodies of the present invention to neutralize or decrease these effects, cultured glial cells are treated with one of the following: vehicle; rhIL-17A; rhIL-17F; rhIL-17A+IL-17F. In addition, these are treated with or without an antagonist of either IL-17A or IL-17F, alone or in combination (such as a soluble IL-17RC). After varying times in culture (from 1 h to several days), supernatants and cells are collected and analyzed for levels and/or expression of inflammatory mediators, including those listed above. Levels of inflammatory cytokines and chemokines are elevated in the presence of rhIL-17A and/or IL-17F compared to cultures treated with vehicle alone. The addition of antagonists to IL-17F and/or IL-17A activity, such as IL-17RC soluble receptors and antibodies thereto including the anti-human-IL-17RC monoclonal and neutralizing antibodies of the present invention markedly reduces the production and expression of inflammatory mediators, and thus, would expect to be efficacious in inflammatory aspects associated with human MS.

### EXAMPLE 27

### Antagonists to IL-17F and IL-17A activity Decrease Disease Incidence and Progression in a Rheumatoid Arthritis (RA) and Osteoarthritis (OA) Model

This model is designed to show that human synovial cultures (including synovial macrophages, synovial fibroblasts, and articular chondrocytes) and explants from patients with RA and OA produce higher levels of inflammatory mediators compared to cultures/explants from healthy controls. This enhanced production of inflammatory mediators (including but not limited to oncostatin M, IL-1b, IL-6, IL-8, IL-12, IL-15, IL-17 A and F, IL-18, IL-23, TNF-a, IFN-g, IP-10, RANTS, RANKL, MIP family members; MCP-1, G- and GM-CSF, nitric oxide, etc.) contributes to the symptoms and pathology associated with RA and OA by way of their effect(s) on activating inflammatory pathways and downstream effector cells. These pathways and components then lead to inflammatory infiltrates, cartilage and matrix loss/destruction, bone loss, and upregulation of prostaglandins and cyclooxygenases. Therefore, this model can simulate the destructive inflammatory aspects of RA and OA in in vitro and ex vivo experiments. Furthermore, when explants and synovial cultures from healthy controls are cultured in the presence of several of these inflammatory components (e.g. oncostatin M, TNF-a, IL-1b, IL-6, IL-17A and F, IL-15, etc.), inflammatory pathway signaling can be observed. Therapeutics that would be efficacious in human RA in vivo would work in the above in vitro and ex vivo models by inhibiting and/or neutralizing the production and/or presence of inflammatory mediators.

In this model, human synovial explants are collected from patients with RA, OA, or from healthy controls undergoing joint replacement or from post-mortem tissue collection, and processed using a modification of Wooley and Tetlow (Arthritis Res 2: 65-70; 2000) and van 't Hof et al (Rheumatology 39:1004-1008; 2000). Cultures of synovial fibroblasts, synovial macrophages and articular chondrocytes are also studied. Replicate samples are treated with one of the following: vehicle (PBS); recombinant human (rh) IL-17A; rhIL-17F; or rhIL-17A+rhIL-17F, and some samples contain various combinations of oncostatin M, TNF-a, IL-1b IL-6, IL-17A, IL-17F, and IL-15. In addition, these are treated with or without an antagonist to IL-17F and/or IL-17A activity, such as IL-17RC soluble receptors and antibodies thereto including the anti-human-IL-17RC monoclonal and neutralizing antibodies of the present invention. After varying time of culture (from 1 h to several days), supernatants are collected and analyzed for levels of inflammatory mediators, including those listed above. In samples from patients with RA or OA, or in samples treated with rhIL-17A and/or F (either alone or in combination with other inflammatory cytokines), levels of inflammatory cytokines and chemokines are elevated compared to untreated healthy control explants or in untreated cell cultures. The addition of antagonists to IL-17F and/or IL-17A activity, such as IL-17RC. soluble receptors and antibodies thereto including the anti-human-IL-17RC monoclonal and neutralizing antibodies of the present invention markedly reduces the production of inflammatory mediators, and thus, would expect to be efficacious in human RA and OA.

### EXAMPLE 28

### IL-17A and IL-17F Functional Responses

NIH-3T3/KZ142 cells were stably transfected with human IL-17RCx1 (SEQ ID NO:1) and mouse IL-17RCx1 (SEQ ID NO:25). As described above, each line was treated for 7 and 15 minutes with a dose response of IL-17A, IL-17F, murine IL-17F, and appropriate controls. Both IL-17A and IL-17F gave a dose dependent response in phosphorylated IκB-α and p38 MAPK transcription factors when IL-17RCx1 (SEQ ID NO:1) was transfected, approximately 30% greater then the inherent signaling from the control line. IL-17A and IL-17F gave no increase in signaling when the murime IL-17RCx1 (SEQ ID NO:25) was transfected. Murine IL-17F gave no increase in signaling for either human or murine IL-17RCx1.

### EXAMPLE 29

### IL-17A, IL-17F, IL-17RA and IL-7RC Expression in Murine Disease Models

Four murine models of disease (asthma, DSS colitis, atopic dermatitis and experimental allergic encephalomyelitis) were analyzed using know techniques for the expression of IL-17A, IL-17F, IL-17R and IL-17RC.

In the asthma model, IL-17A and IL-17F are expressed at very low to undetectable levels in lung, spleen, lung draining lymph nodes and lung infiltrating cells in diseased and non-diseased mice. IL-17RC message was found to be more highly expressed in lung compared to spleen and lymph node but was not regulated with disease. IL-17R was more highly expressed in spleen and lung draining lymph node compared to lung but was also not regulated with disease.

Contrary to the asthma model, IL-17A and IL-17F were highly up-regulated in diseased but not normal mice in the DSS-colitis model in both proximal and distal colon. Neither cytokine was significantly up-regulated in the mesenteric lymph node. Further, it was found that up-regulation of both cytokines in the context of acute DSS-induced colitis and not in chronic DSS-induced colitis. IL-17R was found to be prominently expressed in mesenteric lymph nodes as compared to proximal and distal colon, but was not regulated with disease. In contrast, IL-17RC was more highly expressed in proximal distal colon tissue compared to mesenteric lymph nodes. IL-17RC expression was also not regulated with disease.

In atopic dermatitis, IL-17A mRNA was not detectable. IL-17F was found to be expressed in both skin and skin-draining lymph nodes but did not appear to be significantly regulated with disease. IL-17R mRNA was more highly expressed in skin-draining lymph nodes as compared to skin but was not regulated with disease. IL-17RC was more highly expressed in skin compared to skin-draining lymph nodes but was also not regulated with disease.

In experimental allergic encephalomyelitis, both IL-17A and IL-17F appeared to up-regulated in spinal chord in diseased but not healthy mice. IL-17F may have been more highly expressed in lymph nodes compared to spinal cord but expression in the lymph nodes was not regulated with disease. However, overall levels of expression in these tissues was quite low. IL-17R was more highly expressed in lymph node tissue compared to brain and spinal cord. IL-17RC was not tested.

In short, IL-17A and IL-17F expression appears to be regulated with disease in the context of the DSS-induced colitis and experimental allergic encephalomyelitis models but apparently not for asthma or atopic dermatitis. IL-17R and IL-17RC expression does not appear to be regulated with disease but IL-17R expression appears to be enriched in lymphoid tissues while IL-17RC expression appears to be enriched in non-lymphoid tissues.

### EXAMPLE 30

### IL-17RC is a Mediator of Activation to Both IL-17A and IL-17F

The murine nih3t3/kz142.8 assay cell line was transfected with a human IL-17RCX (SEQ ID NO:2) in an expression vector with a methotrexate resistance gene. (dihydrofolate reductase,DHFR) Human IL-17RA (SEQ ID NO:21) was similarly tranfected into this cell line. Transfections were performed using a commercially available kit and the manufacturer's recommendations. (Mirus, Madison,WI. Cat. #MIR218) Cells were placed in 1µM mtx amended growth medium to select for the expression vector containing the expression constructs. After selection transfection pools were generated, and called nih3t3/kz142.8/hcytor14Xl and nih3t3/kz142.8/IL-17R.

### A) Luciferase assay using the nih3t3/kz142.8- based cell lines.

Since nih3t3/lz142.8 based cell lines have stable apl/nfkb reporters (kz142), there is no need for adenovirus infection to add this reporter. Thus the luciferase assay protocol was shorted and done the following way:

### 1. Cell plating

Cells were plated at 5000 cells/well in solid white, cell culture coated 96 well plates, (Cat. #3917. Costar) using DMEM/10% FBS, containing glutamine and amended with pyruvate and cultured overnight at 37°C and 5% CO₂. On this second day, the plating media was removed and exchanged for DMEM/1% FBS, containing glutamine and amended with pyruvate and cultured overnight at 37°C and 5% CO₂.

### 2. Luciferase assay measuring IL-17A and F activation of the stable kz142 reporter

Following the overnight incubation in the 1% fbs, DMEM media, human IL-17A,and IL-17F ligand dilutions were made in serum free media, amended with BSA to a .28% level. After adding the ligand dilutions, cells were incubated at 37°C and 5% CO₂ for 4 hours, after which the media was removed, cells lysed for 15 minutes and mean fluorescence intensity (MFI) measured using the luciferase assay system and reagents, (Cat.#e1531 Promega. Madison, WI.) and a Microplate luminometer. Activity was detected for both ligands at concentrations ranging from 1-100ng/ml.

The EC50s discussed below are averages of at least 4 experiments. The nih3t3/kz142.8/hcytor14x1 transfection pool showed similar activity for the murine IL-17A ligand as did the parental cell line, with an EC50 of about 4ng/ml. (example 14) The fact that the mIL-17A signaling in the hcytor14x1 recombinant line is comparable to that in the parental cell line (example14) suggests that murine IL-17A is probably signaling through the endogenous murine nih3t3 cell IL-17RA or IL-17RC receptors and does not activate the cells through hcytor14X1. However, the hIL-17RCX1 transfectant pool showed an elevated responsiveness to human IL-17A treatment, with an EC50 of 41 ng/ml Vs 2.8 ng/ml (averages of 4 experiments) in the parental line (a 6.8 fold more potent EC50 in the recombinant line) In addition, the hIL-17RCX1 recombinant line had an enhanced responsiveness to hIL-17F, with an EC50 of .61ng/ml in the recombinant line Vs 10ng/ml in the parental line. (a 17 fold more potent EC50 in the recombinant line). The increased potency to hIL-17A and F in the hIL-17RCX1 line is consistent with human IL-17RCX1 being a high affinity receptor for both human IL-17A and IL-17F. In contrast, the hIL-17RA recombinant line had enhanced sensitivity only to hIL-17A, with an EC50 of .6ng/ml vs 2.8 ng/ml for the parental line. There was not an enhancement of the hIL-17F EC50 in the hIL-17RA recombinant line, with an IL-17F EC50 of 12.4 ng/ml vs 8.9ng/ml in the parental line.

This result is significant because it specifically implicates hIL-17RCX1 as a mediator of activation to both hIL-17A and hIL-17F and suggests that hIL-17RA mediates signaling only to hIL-17A activation and not hIL-17F.

### EXAMPLE 31

### Intravenous Administration of IL-17A and IL-17F

To determine the effect of i.v. delivery of murine or human IL-17A or IL-17F on complete blood counts (CBC) and serum cytokines/chemokines in BALB/c mice at various time points.

I.V. administration of 1 ug mIL-17A resulted in an approximate 2-fold increase in circulating neutrophils (by CBC) and approximate 10-fold increase in serum KC and MCP-1 (by Luminex) 1-2 h following administration; similar results in these chemokines were observed with 5 ug hIL-17A. Blood monocyte levels were also significantly increased in mice treated with 1 ug mIL-17A (showed the greatest increase), 5 ug hIL-17A or 5 ug hIL-17F at the 2 h timepoint. I.V. administration of m and hIL-17F resulted in marked increases in serum IL-15 (by Luminex) at the 1 and 2 h time points, and small increases in serum KC and MCP-1 at these same timepoints.

### EXAMPLE 32

### Neutralization of Intravenous Administration IL-17A and IL-17F

To neutralize the i.v. IL-17A and IL-17F-mediated increases in cytokines and chemokines with i.p. soluble receptors (mIL-17RA:Fc for murine ligands; soluble human IL-17RC for human ligands). Female BALB/c mice were administered by i.p. injection either PBS, 100 ug mIL-17RA:Fc, or 100 ug soluble human IL-17RC three hours prior to receiving by i.v. tail injection: PBS; 2 ug of either mIL-17A, mIL-17F, or 2 ug of both mIL-17A and F (for mice that received mIL-17RA:Fc); or 2 ug of either hIL-17A, hIL-17F, or 2 ug of both hIL-17A and F (for mice that received soluble human IL-17RC). Serum was collected 1 h following ligand administration and analyzed for a small number of serum cytokines and chemokines.

Mice pretreated with i.p. soluble receptor had marked reductions in IL-17A-mediated increases in serum concentrations of IL-17A and KC compared to mice treated with PBS +IL-17A.

### EXAMPLE 33

### Plate Based Protein binding Assays of the Soluble IL-17RC and IL-17RC/IL-17RA Polypeptides

The format of the Capture EIA is as follows: Coat the ELISA plate with Goat anti Human IgG at 1 µg/ml and incubate overnight at 4°C. Wash and block the plate with 200 µl per well 1% BSA for 1 hour at room temperature. Wash, add the soluble receptor variants (A1586F, A1587F) or IL17RCx1 (A1034F) dilution series (100 µg/ml through 0.10 µg/ml) to the plate and incubate for 1 hour at room temperature. Wash, add biotin labeled ligand @ 10:1 (IL17A) or 6:1 (IL17F) and incubate for 1 hour at room temperature. Wash, add Strept Avidin -Horse Radish Peroxidase @ 0.5 µg/mL and incubate for 1 hour at room temperature. Wash, add TMB substrate for 4 minutes. Stop the reaction by adding Stop Solution. (Note: All reagents volumes were 50 µl per well unless stated otherwise). A positive result would be high OD values, generally above 0.5. The results indicated that construct 1342 (SEQ ID NO:74) does not bind IL-17A and weakly binds IL-17F in this assay. Construct 1341 (SEQ ID NO:72) binds both IL-17A and IL-17F very strongly. IL-17RCx1 binds IL-17A and IL-17F.

The format of the Neutralization EIA is as follows: Coat the ELISA plate with soluble receptor (A1034F) at 1 µg/ml and incubate overnight at 4°C. Wash and block the plate with 200 µl per well 1% BSA for I hour at room temperature. While blocking, in a separate plate incubate the soluble receptor variants (A1586F, A1587F) dilution series (50 µg/ml through 0.05 µg/ml) with biotin labeled ligand @ 10:1 (IL17A) or 6:1 (IL17F) in equal volumes for 1 hour at room temperature. Wash the blocked plate, add the receptor-ligand complex to the blocked plate and incubate for 1 hour at room temperature. Wash, add Strept Avidin -Horse Radish Peroxidase @ 0.5 µg/mL and incubate for 1 hour at room temperature. Wash, add TMB substrate for 7 minutes. Stop the reaction by adding Stop Solution. (Note: All reagents volumes were 50 µl per well unless stated otherwise). A positive result would be low OD values, generally below 0.5. The results indicated that construct 1342 (SEQ ID NO:74) weakly neutralizes binding of IL17A to IL17RCx1and strongly neutralizes binding of IL17F to IL17RCx1. Construct 1341 (SEQ ID NO:72) weakly neutralizes binding of IL17A to IL17RCx1 and weakly neutralizes binding of IL17F to IL17RCx1. Neutalization indicates that the variant protein is binding the biotinylated ligand.

### EXAMPLE 34

### FACS Binding Assay Protocol

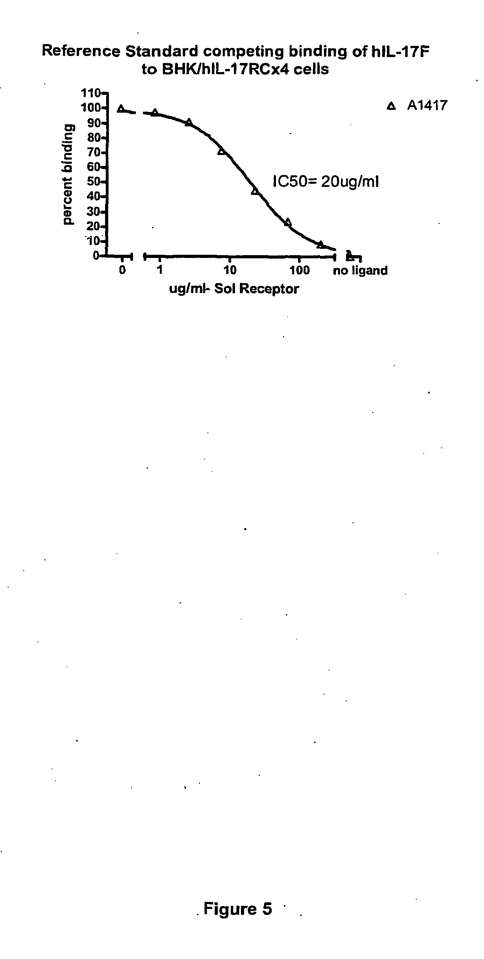

To assess the ability of the soluble IL-17RC and IL-17RC/IL-17RA polypeptides of the present invention to bind the ligands IL-17A and IL-17F, a Flow Cytometry-based competitive binding assay was utilized. Incubation of a BHK cell line stably transfected with full length IL17RCx4 in the presence of the ligands IL17A or IL17F, and the soluble receptor targeted to bind the ligands allows for detection and relative quantification of ligand bound to the cell surface (and therefore unbound by the soluble receptor). The biotinylation of the ligand allows for FACS detection using a secondary Streptavidin conjugated fluorophore. A reduction in cell bound ligand over a titration of the soluble receptor is recorded as a reduction in the mean fluorescence of the cells. Biotinylated ligands are individually pre-mixed at 1ug/ml with titrating amounts of soluble receptor in staining media (HBSS + 1%BSA + 0.1% NaAzide + 10mM HEPES) in 100ul volumes and incubated at RT for 15 minutes. A BHK cell line stably transfected with full length IL17RCx4 is prepared for ligand staining by resuspension with Versene (Invitrogen cat.15040-066), equilibrating to 2 x 10e5 cells/100ul, pelleting, and resuspension in the ligand/soluble receptor pre-mix. Stained cells are incubated at 4° for 30 minutes, washed 1x in staining media, and stained with Streptavidin-PE (BD Pharmingen cat. 554061) at a 1:100 ratio. Cells are incubated at 4° in the dark for 30 minutes, washed 2x in staining media, and re-suspended in a 1:1 ratio of staining media and Cytofix (BD Bioscience 554655). The BD LSRII Flow Cytometer or similar instrument is used for data collection and analysis. Figure 5 depicts a standard graph. The graph was generated using the Prizm software program. The Y values represent the MFI normalized to maximum and minimum (100% and 0%) based on ligand only and no ligand/no soluble receptor control wells, and thus the percent binding of the ligand to the cells. The software calculates the IC50 for each curve.

### EXAMPLE 35

### Inhibition of Specific Binding of Biotinylated Human IL-17A and IL17F with a Soluble IL-17RC/IL-17RA Polypeptide

The binding assay used to determine the ability of the soluble IL-17RC and IL-17RC/IL-17RA polypeptides to bind IL-17A and IL17F is described herein. Binding studies are performed as discussed above, except that additional soluble polypeptides, such as SEQ ID NOs: 157 and 158 was included in the binding reaction. This soluble polypeptide inhibited binding of both human IL-17A and IL-17F to IL-17RC transfected BHK cells to the same extent as soluble human IL-17RCx1 Fc fusion protein (SEQ ID NO:64). The remainder of soluble polypeptides, including the soluble polypeptide of SEQ ID Nos: 157 and 158, are included in Table 9 below.

**Table 9***

| Soluble Polypeptide | Variant | IC50 - IL17A | | Soluble Polypeptide | Variant | IC50 - IL17F |
|---|---|---|---|---|---|---|
| IL17RA/RC | 1407 | 7 | | IL17RC | 1390 | 9 |
| IL17RA/RC | 1407 | 9 | | IL17RA/RC | 1454 | 18 |
| IL17RA/RC | 1454 | 4 | | IL17RA/RC | 1454 | 31 |
| IL17RA/RC | 1454 | 17 | | IL17RA/RC | 1454 | 95 |
| IL17RA/RC | 1454 | 20 | | IL17RA/RC | 1407 | 33 |
| IL17RC | 1390 | 12 | | IL17RA/RC | 1407 | 42 |
| IL17RA/RC | 1341 | 30 | | IL17RC | 1210 | 31 |
| IL17RC | 1210 | 35 | | IL17RC | 1210 | 61 |
| IL17RC | 1210 | 47 | | IL17RC | 1210 | 67 |
| IL17RC | 1210 | 74 | | IL17RA/RC | 1341 | 47 |
| IL17RC | 1459 | 126 | | IL17RC | 1459 | 103 |
| IL17RC | 1342 | 217 | | IL17RC | 1342 | 313 |

| | | | | | | |
|---|---|---|---|---|---|---|
| * Cell-based Competition Binding IC50 (ng/uL); ordering of Constructs from strongest binders to weakest based on IC50's for each ligand | | | | | | |

### EXAMPLE 36

### Binding Affinity of the IL-17RC and IL-17RC/IL-17RA Soluble Polypeptides to IL-17A and IL-17F

IL-17RCxl, IL-17RA and the soluble IL-17RC/IL-17RA soluble polypeptide (SEQ ID Nos: 157 and 158) were tested for binding affinity to both IL-17A and IL-17F as follows: Gt-anti-Hu IgG-Fc specific Antibody (Jackson #109-005-008) was diluted to 50ug/ml in pH 5.0 Na Acetate and immobilized onto a CM5 Biacore chip. The protocol was optimized to capture receptor at a theoretical binding max. before injecting a concentration series of each ligand to observe association and dissociation. The soluble receptors and the IL-17RC/IL-17RA polypeptide were tested for binding of a concentration series of each ligand. The surface was regenerated with 2 x 30 sec. injections of pH 1.75 glycine between cycles. Data was evaluated using Biacore Evaluation software to define kinetic values and is hown in Table 10 below.

**Table 10***

| | | | | |
|---|---|---|---|---|
| Human IL17RCx1 Affinity for Human IL-17A | | | | 05-2005 |
| ka (1/Ms) | kd (1/s) | KD(M) | Rmax (RU) | Chi² (RU²) |
| 1.05E+06 | 4.90E-04 | 4.69E-10 | 9.02 | 0.424 |
| 1.24E+06 | 4.38E-04 | 3.52E-10 | 8.86 | 0.324 |
| Human IL17RCx1 Affinity for Human IL-17F | | | | 05-2005 |
| ka(1/Ms) | kd(1/s) | KD (M) | Rmax (RU) | Chi² (RU²) |
| 9.91E+05 | 4.31E-04 | 4.35E-10 | 7.22 | 0.378 |
| 1.11E+06 | 3.84E-04 | 3.46E-10 | 7.57 | 0.549 |
| Soluble IL-17RC/IL-17RA Polypeptide for Human IL-17A | | | | 04-2006 |
| ka(1/Ms) | kd(1/s) | KD (M) | Rmax(RU) | Chi² (RU²) |
| 1.42E+06 | 6.22E-05 | 4.39E-11 | 20.5 | 0.460 |
| 2.61E+06 | 9.95E-05 | 3.82E-11 | 18.3 | 0.888 |
| Soluble IL-17RC/IL-17RA Polypeptide for Human IL-17F | | | | 04-2006 |
| ka (1/Ms) | kd (1/s) | KD (M) | Rmax | (RU) Chi² (RU²) |
| 1.82E+06 | 2.61E-04 | 1.43E-10 | 10.2 | 0.495 |
| 2.49E+06 | 3.15E-04 | 1.26E-10 | 11.2 | 0.544 |
| Human IL-17RA Affinity for Human IL-17A | | | | 06-2006 |
| ka (1/Ms) | kd (1/s) | KD (M) Rmax | (RU) | Chi² (RU²) |
| 3.70E+05 | 8.65E-05 | 2.34E-10 | 29.5 | 0.249 |
| 2.89E+05 | 8.57E-05 | 2.96E-10 | 35.1 | 0.197 |
| Human IL-17RA Affinity for Human IL-17F | | | | 07-2006 |
| ka(1/Ms) | kd (1/s) | KD(M) | Rmax (RU) | Chi² (RU²) |
| 2.09E+04 | 5.56E-04 | 2.66E-08 | 20.3 | 0.071 |
| 2.55E+04 | 4.40E-04 | 1.72E-08 | 9.9 | 0.076 |

| | | | | |
|---|---|---|---|---|
| *Equilibrium and rate constants are shown and values fall within machine limits. | | | | |

Chi2 refers to the sum of the square of the residuals between the binding curves and the evaluation fitting curves. The closer to 0, the more confidence we have in the data. This data is shown with good confidence.

These data demonstrates the binding of human IL-17A and human IL-17F to human IL-17RA and human IL-17RC. Specifically, human IL-17RC demonstrates similar binding affinity for both human IL-17A and human IL-17F with dissociation equilibrium constants (KD) in the 400 picomolar (pM) range. The soluble IL-17RC/IL-17RA polypeptide bound human IL-17A with slightly higher affinity, KD- 40pM, than human IL-17F, KD∼ 140pM. Human IL-17RA produced the largest discrepancy of ligand affinity with a 100-fold difference between human IL-17A, KD∼ 300pM, and human IL-17F, KD∼ 30 nanomolar (nM),binding.

### EXAMPLE 37

### Creation of Recombinant Human IL-17RA/NIH3T3/KZ142.8 and IL-17RCx4/NIH3T3/KZ142.8 Reporter Assay Cell Lines

The murine NIH3T3/KZ142.8 reporter cell line described herein was used to create new assay cell lines, recombinant for either human IL-17RA (SEQ ID NO:21) or IL-17RCx4 (SEQ ID NO:166). This was accomplished by transfection of these cells with expression constructions containing each of these cDNAs. The expression vector utilized, pzmp11, which contains the dihydrofolate reductase gene. Thus transfectants were selected using 1uM methotrexate amended growth medium to create stable pools. These assay cell lines were called hIL-17RA/IH3T3/KZ142.8 and hIL-17RCX4/NIH3T3/KZ142.8.

### EXAMPLE 38

### A Soluble IL-17RC/IL-17RA Polypeptde Antagonizes Human IL-17A Activation of Recombinant Human IL-17RA/NIH3T3/KZ142.8 Cells

The efficacy of soluble IL-17RC/IL-17RA soluble polypeptide (SEQ ID Nos: 157 and 158) competition for human IL-17A activation of recombinant hIL-17RA/NIH3T3/KZ142.8 cells was measured as follows: Cell plating and preparation for a luciferase assay was the same as that described herein. The day of the assay, these cells were first given a triplicate 2 fold dose series of one volume of soluble receptors at 2 fold the final concentration including the soluble polypeptide above, IL-17RA and IL-17RC beginning at a 2ug/ml, (which results in a 1ug/ml final concentration once combined with the ligand). Next one volume of IL-17A was applied at 1ng/ml, which is 2 fold the final concentration of .5ng/ml which results from the receptor-ligands mixing together. The maximum activation was determined using a triplicate set which received .5ng/ml of IL-17A without receptor. The basal activation was determined using a triplicate set which received only assay medium which contained neither ligand nor soluble receptor. Data analysis revealed IC50 for IL-17A activation of the above cell line by the soluble polypeptide was 7ng/ml. There wasn't sufficient potency of soluble IL-17RA or IL-17RC to convincingly antagonize .5ng/ml hIL-17A activation of this cell line with even the highest dose of 1ug/ml soluble receptor.

### EXAMPLE 39

### A Soluble IL-17RC/IL-17RA Polypeptide Antagonizes Human IL-17F Activation of Recombinant Human IL-17RA/NIH3T3/KZ142.8 cells

The efficacy of the soluble IL-17RC/IL-17RA polypeptide (SEQ ID Nos: 157 and 158) competition for human IL-17F activation of recombinant hIL-17RA/NIH3T3/KZ142.8 cells (described above) was measured as follows: Cell plating and preparation for a luciferase assay was the same as that described herein. The day of the assay, these cells were first given a triplicate 2 fold dose series of one volume of soluble polypeptide at 2 fold the final concentration including the soluble polypeptide above, IL-17RA and IL-17RC beginning at a 4ug/ml, (which results in a 2ug/ml final concentration once combined with the ligand). Next one volume of IL-17F was applied at 40ng/ml, which is 2 fold the final concentration of 20ng/ml which results from the receptor-ligands mixing together. The maximum activation was determined using a triplicate set which received 20ng/ml of IL-17F without receptor. The basal activation was determined using a triplicate set which received only assay medium which contained neither ligand nor soluble receptor. Data analysis revealed IC50 for IL-17F activation of the above cell line by the IL-17RC/IL-17RA soluble polypeptide of 0.48ug/ml. There wasn't sufficient potency of soluble IL-17RA or IL-17RC to show any antagonism of 20ng/ml IL-17F activation of this cell line with even the highest dose of 2ug/ml soluble receptor.

### EXAMPLE 40

### A Soluble IL-17RC/IL-17RA Polypeptide Antagonizes Human IL-17F Activation of Recombinant Human IL-17RCx4/NIH3T3/KZ142.8 cells

The efficacy of soluble IL-17RC/IL-17RA polypeptide (SEQ ID Nos: 157 and 158) competition for IL-17F activation of recombinant hIL-17RCX4/NIH3T3/KZ142.8 cells (described above) was measured as follows: Cell plating and preparation for a luciferase assay was the same as that described herein. The day of the assay, these cells were first given triplicate 5 fold serial doses of one volume of soluble receptors at 2 fold the final concentration including the above soluble polypeptide, IL-17RA and IL-17RC beginning at a 4ug/ml. Next one volume of IL-17F lot A1275F was applied at 2ng/ml, which is 2 fold the final concentration of 1ng/ml which results from the receptor-ligands mixing together. The maximum activation was determined using a triplicate set which received 1ng/ml of IL-17F without receptor. The basal activation was determined using a triplicate set which received only assay medium which contained neither ligand nor soluble receptor. Data analysis revealed IC50 for IL-17F activation of the soluble IL-17RC/IL-17RA polypeptide of 0.8ug/ml, IL-17RC was 6ug/ml, and IL-17RA had no antagonism at any dose.

### EXAMPLE 41

### Soluble IL-17RC/IL-17RA Polypeptide Neutralizes the Activity of Both Human IL-17A and IL-17F Induction of G-CSF, IL-6 and IL-8

Human small airway epithelial cells (SAEC) were treated with human IL-17A or with human IL-17F and 48hr supernatants were collected. These supernatants were assayed and showed a dose-dependent induction of G-CSF, IL-6, and IL-8, as shown in Table 11 below:

**Table 11**

| | | Fold Induction in 48hr supernatants | | |
|---|---|---|---|---|
| | | G-CSF | IL-6 | IL-8 |
| SAEC treated with: | | | | |
| huIL-17A | 50 ng/ml | 26 | 13 | 8 |
| | 10 ng/ml | 24 | 14 | 6 |
| | 2 ng/ml | 14 | 8 | 3 |
| | 0.4 ng/ml | 13 | 8 | 3 |
| huIL-17F | 250 ng/ml | 15 | 11 | 4 |
| | 50 ng/ml | 10 | 8 | 3 |
| | 10 ng/ml | 8 | 8 | 2 |
| | 2 ng/ml | 4 | 5 | 2 |

SAEC were also treated with 0.01 - 10 ug/ml doses of soluble IL-17RC/IL-17RA polypeptide (SEQ ID Nos: 157 and 158) in combination with 10 ng/ml human IL-17A or 50 ng/mi human IL-17F (both ligand and soluble polypeptide were incubated together for 30 minutes at 37°C before adding to cells), and 48hr supernatants collected. As shown in Table 12 below, these supernatants showed decreased G-CSF, IL-6, and IL-8, demoristrating that the soluble IL-17RC/IL-17RA polypeptide was able to effectively neutralize the activity of both human IL-17A and human IL-17F induction of these cytokines. It is noted that IC50 values were not able to be determined for the neutralization of IL-6, because at the lowest dose (0.01 ug/ml) of the soluble IL-17RC/IL-17RA polypeptide tested, neutralization had only returned to approximately 50% of max.).

**Table 12**

| **Soluble IL-17RA/RC receptor neutralizes activity of huIL-17A/F :** | **IC50 of IL-17RA/RC (ug/ml)** |
|---|---|
| huIL-17A(10 ng/ml) induction of G-CSF | 0.14 |
| huIL-17F(50 ng/ml) induction of G-CSF | 1.20 |
| huIL-17A(10 ng/ml) induction of IL-8 | 0.03 |
| huIL-17F(50 ng/ml) induction of IL-8 | 0.57 |
| huIL-17A(10 ng/ml) induction of IL-6 | 94% neutralized at 10 ug/ml |
| | 49% neutralized at 0.01 ug/ml |
| huIL-17F(50 ng/ml) induction of IL-6 | 72% neutralized at 10 ug/ml |
| | 57% neutralized at 0.01 ug/ml |

### EXAMPLE 42

### Efficacy of the Soluble IL-17RC and IL-17RC/IL-17RA Polypeptides in Human Multiple Sclerosis Samples

Multiple sclerosis (MS) is a complex disease that is thought to be mediated by a number of factors, including the presence of lymphocytic and mononuclear cell inflammatory infiltrates and demyelination throughout the CNS. Microglia are macrophage-like cells that populate the central nervous system (CNS) and become activated upon injury or infection. Microglia and neuronal cells have both been implicated as playing critical roles in various CNS diseases including MS, and may be used to study mechanism(s) of initiation, progression, and therapy of the disease (Nagai et al. Neurobiol Dis 8:1057-1068; 2001; Olson et al. J Neurosci Methods 128:33-43; 2003; Giuliani et al. J Neuroimmunol 165: 83 - 91; 2005). Primary neuronal cell cultures, immortalized human microglial cell lines and/or established human astroglia cell lines can, therefore, be used to study some of the effects of inflammatory mediators on these cell types and their potential for neutralization. Inflammatory mediators (including but not limited to IL-1b, IL-6, IL-8, IL-12, IL-13, IL-15, IL-17 A and F, IL-18, IL-23, TNF-a, IFN-g, MIP family members, RANTES, IP-10, MCP-1, G- and GM-CSF, etc.) can contribute to the symptoms and pathology associated with MS by way of their effect(s) on activating inflammatory pathways and downstream effector cells.

In order to evaluate the pro-inflammatory actions of IL-17A and IL-17F on these cells types, and the ability of the soluble polypeptides of the present invention, such as the soluble IL-17RC/IL-17RA polypeptide (SEQ ID NO:158) to neutralize or decrease these effects, cultured neuronal or glial cells are treated with one of the following: vehicle; rhIL-17A; rhIL-17F; rhIL-17A+IL-17F. In addition, these are treated with or without a soluble polypeptide of the present invention, such as the soluble IL-17RC/IL-17RA polypeptide (SEQ ID NO:158). In a separate set of cultures, circulating T cells isolated from human subjects and activated with anti-CD3, are added to the cultured neuronal and glial cells in the absence of exogenous IL-17A or IL17-F, thus providing a co-culture method of investigating the destructive effects of activated T cells on these cell types. The T cells are treated with or without a soluble polypeptide of the present invention, such as the soluble IL-17RC/IL-17RA polypeptide (SEQ ID NO:158). After varying times in culture (from 1 h to several days), supernatants and cells are collected and analyzed for levels and/or expression of inflammatory mediators, including those listed above, and also analyzed for cell survival. Levels of inflammatory cytokines and chemokines, and death of neuronal cells, are elevated in the presence of rhIL-17A and/or IL-17F compared to cultures treated with vehicle alone. The addition of a soluble polypeptide of the present invention, such as the soluble IL-17RC/IL-17RA polypeptide (SEQ ID NO:158) markedly reduces the production and expression of inflammatory mediators in these cultures, and increases cell survival in the neuronal cells.

Therefore, because these *ex vivo* experiments demonstrate that a soluble polypeptide of the present invention, such as the soluble IL-17RC/IL-17RA polypeptide (SEQ ID NO:158) can reduce the destructive and inflammatory actions that are associated with the pathobiology of human MS, treatment with such soluble polypeptides would be expected to be efficacious in reducing the inflammatory aspects, neuronal death, and/or demyelination associated with human MS.

### EXAMPLE 43

### Efficacy of the Soluble IL-17RC and IL-17RC/IL-17RA Polypeptides in Human Rheumatoid Arthritis ("RA") and Osteoartritis ("OA") Samples

These models are designed to show that human synovial cultures (including synovial macrophages, synovial fibroblasts, and articular chondrocytes) and explants from patients with RA and OA produce higher levels of inflammatory mediators compared to cultures/explants from healthy controls, which in turn can contribute to the degradation of extracellular matrix components (e.g. bone, cartilage, etc), which is a hallmark of these diseases. In addition, the co-culture models described below are designed to show that inflammatory mediators present in RA/OA synovial fluid and/or activated T cells can also result in greater inflammation and matrix degradation.

The enhanced production of inflammatory mediators (including but not limited to oncostatin M, IL-1b, IL-6, IL-8, IL-12, IL-15, IL-17 A and F, IL-18, IL-23, TNF-a, IFN-g, IP-10, RANTES, RANKL, MIP family members, MCP-1, MMP-9, G- and GM-CSF, nitric oxide, etc.) contributes to the symptoms and pathology associated with RA and OA by way of their effect(s) on activating inflammatory pathways and downstream effector cells. These pathways and components then lead to inflammatory infiltrates, cartilage and matrix loss/destruction, bone loss, and upregulation of matrix metalloproteases, prostaglandins and cyclooxygenases. Therefore, these models can simulate the destructive inflammatory aspects of RA and OA in *in vitro* and *ex vivo* experiments. Furthermore, when explants and synovial cultures from healthy controls are cultured in the presence of exogenously added inflammatory components (e.g. oncostatin M, TNF-a, IL-1b, IL-6, IL-17A and F, IL-15, etc.), or alternatively, in the presence of synovial fluid from RA patients (which would contain inflammatory components endogenously), inflammatory and degradative pathway signaling can be observed. Therapeutics that would be efficacious in human RA *in vivo* would work in the above *in vitro* and *ex vivo* models by inhibiting and/or neutralizing the production and/or presence of inflammatory mediators.

In these models, human synovial explants are collected from patients with RA, OA, or from healthy controls undergoing joint replacement or from post-mortem tissue collection, and processed using a modification of Wooley and Tetlow (Arthritis Res 2: 65-70; 2000) and van 't Hof et al (Rheumatology 39:1004-1008; 2000). Cultures of synovial fibroblasts, synovial macrophages and articular chondrocytes are also studied. Replicate samples are treated with one of the following: vehicle (PBS); recombinant human (rh) IL-17A; rhIL-17F; or rhIL-17A+rhIL-17F, and some samples contain various combinations of oncostatin M, TNF-a, IL-1b, IL-6, IL-17A, IL-17F, and IL-15. A separate set of samples are treated with activated human T cells, or synovial fluid from healthy controls or patients with RA or OA. In addition, all of these samples are treated with or without a soluble polypeptide of the present invention, such as a soluble IL-17RC polypeptide or a soluble IL-17RC/IL-17RA polypeptide (SEQ ID NO:158). After varying time of culture (from 1 h to several days), supernatants and cells are collected and analyzed for levels of inflammatory mediators and cartilage/bone/matrix biomarkers, including those listed above. In samples from patients with RA or OA, or in samples treated with RA/OA synovial fluid, activated T cells, rhIL-17A and/or rhIL-17F (either alone or in combination with other inflammatory cytokines), levels of inflammatory cytokines and chemokines and cartilage/bone/matrix degradative markers are elevated compared to untreated healthy control explants or in untreated cell cultures, The addition of a soluble polypeptide of the present invention markedly reduces the production of inflammatory and cartilage/bone/matrix degradative mediators, and thus, would expect to be efficacious in human RA and OA.

### EXAMPLE 44

### Efficacy of the Soluble IL-17RC and IL-17RC/IL-17RA Polypeptides in Human Inflammatory Bowel Disease ("IBD") Samples via Mucosal Biopsy Cultures

This model is designed to show that cultured intestinal tissue from patients with IBD produce higher levels of inflammatory mediators compared to tissue from healthy controls. This enhanced production of inflammatory mediators (including but not limited to IL-1b, IL-4, IL-5, IL-6, IL-8, IL-12, IL-13, IL-15, IL-17 A and F, IL-18, IL-23, TNF-a, IFN-g, MIP family members, MCP-1, G- and GM-CSF, etc.) contributes to the symptoms and pathology associated with IBD such as Crohn's disease (CD) and ulcerative colitis (UC) by way of their effect(s) on activating inflammatory pathways and downstream effector cells. These pathways and components then lead to tissue and cell damage/destruction observed in vivo. Therefore, this model can simulate this enhanced inflammatory mediator aspect of IBD. Furthermore, when intestinal tissue from healthy controls or from human intestinal epithelial cell (IEC) lines is cultured in the presence of these inflammatory components, inflammatory pathway signaling can be observed, as well as evidence of tissue and cell damage.

Therapeutics that would be efficacious in human IBD in vivo would work in the above ex vivo or IEC models by inhibiting and/or neutralizing the production and/or presence of inflammatory mediators.

In this model, human intestinal tissue is collected from patients with IBD or from healthy controls undergoing intestinal biopsy, re-sectioning or from post-mortem tissue collection, and processed using a modification of Alexakis et al (Gut 53:85-90; 2004). Under aseptic conditions, samples are gently cleaned with copious amounts of PBS, followed by culturing of minced sections of tissue, in the presence of complete tissue culture media (plus antibiotics to prevent bacterial overgrowth). Samples from the same pool of minced tissue are treated with one of the following: vehicle (PBS); recombinant human (rh) IL-17A; rhIL-17F; or rhIL-17A+rhIL-17F. In addition, these are treated with or without a soluble polypeptide of the present invention, such as a soluble IL-17RC polypeptide or a soluble IL-17RC/IL-17RA polypeptide (SEQ ID NO:158). This experimental protocol is followed for studies with human IEC lines, with the exception that cells are passaged from existing stocks. After varying times in culture (from 1 h to several days), supernatants are collected and analyzed for levels of inflammatory mediators, including those listed above. In samples from patients with IBD or in samples treated with rhIL-17A and/or F, levels of inflammatory cytokines and chemokines are elevated compared to untreated healthy control tissue samples. The addition of a soluble polypeptide of the present invention markedly reduces the production of inflammatory mediators, and thus, would expect to be efficacious in human IBD.

An additional arm of this study can include comparisons of the production of inflammatory mediators from tissue biopsies of IBD patients undergoing effective treatment, and those either not currently taking medications or considered non-responders to treatment.

### EXAMPLE 45

### Efficacy of the Soluble IL-17RC and IL-17RC/IL-17RA Polypeptides in Human IBD Samples via Epithelial Barrier Function

Maintenance of epithelial barrier integrity is a critical factor in the preservation of a healthy gastrointestinal tract. Experimental evidence suggests that leakiness of the epithelial barrier in the gut may contribute to the development of IBD. Immune cells located in the intestinal lamina propria generally interact with intestinal epithelial cells via cell to cell contact or production of soluble factors to maintain immune surveillance and contribute to epithelial barrier integrity. However, prolonged or dysregulated immune-mediated inflammation may contribute to defects in epithelial barrier cell integrity and function. The following study is designed to measure the direct effect(s) of T cell-derived IL-17A and/or IL-17F on epithelial barrier integrity.

In this example, intestinal epithelial cell lines, like Caco-2 cells, are differentiated on semipermeable membranes and co-cultured on the basolateral side with either T cells or monocytes derived from biopsies from IBD patients or normal individuals. Epithlelial monolayer integrity is monitored over time using assessment of transepithelial electrical resistance or resistance of the monolayer to dye diffusion. Decreases in transepithial resistance of monolayers in co-cultures would suggest a disruption in the monolayer induced by the activity of the T cells or monocytes in the co-culture. Inhibitors of IL-17A and IL-17F such as the soluble polypeptides of the present invention, such as a soluble IL-17RC polypeptide or a soluble IL-17RC/IL-17RA polypeptide (SEQ ID NO:158) could be used to determine the relative contribution of IL-17A and IL-17F to the disruption of the epithelial monolayer and test whether inhibitors of IL-17A and IL-17F would be effective in maintaining epithelial barrier integrity. Prevention of epithelial monolayer disruption induced by activated T cells by such molecules would suggest that the soluble IL-17RC and IL-17RC/IL-17RA polypeptides of the present invention may be effective for the therapeutic treatment of IBD in humans.

Co-culture systems could also be generated using monolayers formed by primary epithelium from IBD patients to determine whether these cells are more sensitive to IL-17A and IL-17F compared to epithelial cells derived from healthy individuals. If so, these data would suggest that inhibiting IL-17A and IL-17F would be a suitable strategy for the therapeutic treatment of IBD.

### EXAMPLE 46

### Effects of IL-17A and IL-17F on Lamina PropPria T cells and Monocytes/Macrophages from Normal and Human IBD Samples

Dysregulated or sustained immune-mediated inflammation may contribute to the symptoms and pathology associated with IBD by way of tissue damage or permanent skewing to inappropriate or prolonged immune responses. This model can determine the potential down-stream consequences of exposure of disease-associated T cells and monocytes to IL-17A and IL-17F which may be present in the immediate environmental cytokine mileu of the intestinal tissue.

Therapeutics that would be efficacious in human IBD in vivo would work in the above ex vivo models by inhibiting and/or neutralizing the production and/or presence of inflammatory mediators (including but not limited to IL-lb, IL-4, IL-5, IL-6, IL-8, IL-12, IL-13, IL-15, IL-17 A and F, IL-18, IL-23, TNF-a, IFN-g, MIP family members, MCP-1, G- and GM-CSF, etc.).

In this model, T cells and monocytes/macrophages are isolated from biopsy samples by carefully mincing biopsies with scissors in HBSS, treating with collagense and Dispase II and incubating for 1 hr at 37oC in a shaker. The cell suspension is filtered through nylon mesh to remove debris and cell clumps and washed multiple times in HBSS. T cells and macrophage/monocytes can be isolated using direct cell sorting or bead-depletion/enrichment protocols. Isolated cells are incubated in the presence of IL-17A and IL-17F. This induces the production of inflammatory mediators by T cells and monocytes/macrophages or results in skewing subsequent T cell responses to highly pro-inflammatory responses. Comparisons between the types of inflammatory mediators produced by cells from IBD patients and those from cells of normal individuals can be made and might suggest that T cells and monocyte/macrophages from IBD patients produce a more pro-inflammatory profile in the presence of IL-17A and IL-17F. The addition of a soluble polypeptide of the present invention, such as a soluble IL-17RC polypeptide or a soluble IL-17RG/IL-17RA polypeptide (SEQ ID NO:158) to neutralize the production of downstream inflammatory mediators induced by IL-17A and IL-17F suggests that such soluble IL-17RC and IL-17RC/IL-17RA polypeptides may be efficacious in the therapeutic treatment of patients with IBD.

### EXAMPLE 47

### Efficacy of the Soluble IL-17RC and IL-17RC/IL-17RA Polypeptides in Irritable Bowl Syndrome ("IBS"): CNS-Directed Pathogenesis

A model focusing on primary CNS-directed pathogenesis of IBS which employs stress stimuli to induce symptoms characteristic of IBS. The neonatal psychosocial stress model mimics some clinical features associated with IBS patients including visceral hyperalgesia, diarrhea and stress-sensitivity. Daily separation of the litter from their mothers for 180 minutes each day during postnatal days 4-18 will result in an alteration of maternal behaviour and significantly reduce times of the licking/grooming behaviour. The stress on the neonates results in permanent changes in the CNS resulting in altered stress-induced visceral and somatic pain sensitivity. Colonic motor function in response to stress is enhanced in these animals and preliminary data shows evidence of increased intestinal permeability (Mayer et al., 2002). Treatment with a soluble polypeptide of the present invention, such as a soluble IL-17RC polypeptide or a soluble IL-17RC/IL-17RA polypeptide (SEQ ID NO:158) and subsequent analysis of colonic motor function, epithelial permeability and response to stress stimuli could determine efficacy in this animal model of IBS. Decreases in the incidence of symptoms following treatment with these inhibitors would suggest potential efficacy in the treatment of IBS.

### EXAMPLE 48

### Efficacy of the Soluble IL-17RC and IL-17RC/IL-17RA Polypeptides in Irritable Bowl Syndrome ("IBS"): Primary Gut-Directed Inducers of Stress

This is a model focusing on primary gut-directed inducers of stress (ie. gut inflammation, infection or physical stress). Animal studies have indicated that low-grade inflammation or immune activation may be a basis for altered motility, and/or afferent and epithelial function of the gut (Mayer et al,. 2002). In this model, daily colon irritation is produced in neonatal animals (days 8-21) in the form of daily intracolonic injection of mustard oil. Mustard oil is a neural stimulant and has been shown to induce visceral hyperalgesia following intracolonic administration. This model mimics key features of the IBS including visceral hypersensitivity and alteration in bowel habits. Animals also present with diarrhea or constipation, a key feature of EBS patients (Mayer et al., 2002; Kimball et al., 2005). A soluble polypeptide of the present invention, such as a soluble IL-17RC polypeptide or a soluble IL-17RC/IL-17RA polypeptide (SEQ ID NO:158) could be delivered to determine changes in the development of symptoms associated with this model. Decreases in the incidence or magnitude of visceral hypersensitivity and altered gut motility following therapeutic treatment with our inhibitors would suggest a potential for these molecules to be efficacious in the treatment of IBS.

### EXAMPLE 49

### Designing a Scalable Protein Production Process for a Soluble IL-17A and IL-17F Antagonist

In designing strategies focused on developing a scaleable protein production process for a soluble form of IL-17RC, many difficulties were encountered with identifying an expression system that allowed high level protein concentrations in the conditioned media. Western blot analysis demonstrated low levels of protein secretion with protein accumulating in the cell. In the discovery of the soluble polypeptides of the present invention, more than seventy different expression constructs were designed, generated, and tested for expression in either BHK cells, CHO cells, or HEK 293 cells. Several were tested in more than one host cell lines. Variations of tested soluble IL-17RC expression cassette included:
1) Alternative signal sequences such as: a) native; b) otPA; c) mouse immunoglobulin heavy chain variable region; d) human growth hormone; e) mouse IL17RA.
2) Two different naturally occurring splice variants (IL-17RCx1, SEQ ID NO:2; and IL-17RCx4, SEQ ID NO:166).
3) Addition of linker sequences between the IL-17RC extracellular domain (ECD) and the Fc portion, such as: a) no linker; b) a 9 amino acid linker based on GlyGlyGlySer; and c) a 20 amino acid linker based on GlyGlyGlySer.
4) His tagged monomeric forms.
5) Both amino- and carboxyl-terminal Fc fusion proteins.
6) Removal of N-linked carbohydrate attachment sites.
7) Gln for Asn amino acid substitutions.
8) Hybrid fusion proteins between IL17RA and IL17RC

All of the soluble IL-17RC variant expression constructs were tested for protein expression by transient transfection in HEK 293 cells. Western blot analysis was used to detect protein secreted into the conditioned medium compared to protein retained in the cell by sampling cell lysates. Most of the constructs expressed protein secreted into the conditioned medium that was barely detectable by Western Blot. Additionally, the signal was greater from the cell lysate sample in comparison to the conditioned media sample indicating an inability for the protein to be efficiently secreted. Those expression constructs that resulted in the highest signals in the conditioned media were used to transfect-stable CHO cell pools. Protein titers were measured from the stable CHO pools and where possible, purified protein was analyzed for IL-17A and IL-17F binding in a cell based competition binding assay. The following table shows protein expression results from the highest expressing constructs in CHO cell stable pools. Where absolute protein concentration measurements were below the level of detection, the protein titer is indicated as < 0.5 mg/mL.

IL-17RC and IL-17RC/RA protein expression constructs number designation, brief description of exons included, protein titer from stably transfeced CHO cell pools, and IL17A and IL17F binding ability. Not all the sequences of the variants included in Table 13 were included herewith.

| Description | Protein Titer (mg/L) | Binding |
|---|---|---|
| x1 splice variant | 3.0 | Ability to Block IL17A and IL17F and IL17F |
| IL17RC exons 1-6, exons | | |
| 8-16 (Variant 1210) | | |
| X4 splice variant | < 0.5 | Unable to obtain enough sample |
| IL17RC exons 1-16 | | |
| IL17RC exons 1-6 | < 0.5 | Inactive |
| IL17RC exons 8-13 | 1.6 | Inactive |
| IL17RC exons 7-16 | < 0.5 | Ability to Block IL17A AND IL1 7F and IL17F |
| IL17RA exons 1-10 | 32.5 | Ability to Block IL17A and IL17F |
| IL17RC exons 8-16 | | |
| (Variant 1407) | | |
| IL17RA exons 1-6 | < 0.5 | Inactive |
| IL17RC exons 8-16 | | |
| IL17RA exons 7-10 | | |
| IL17RA exons 1-3 | < 0.5 | Unable to obtain enough sample |
| IL17RC exons 4-16 | | |
| IL17RA exons 1 | < 0.5 | Unable to obtain enough sample |
| IL17RC exons 2-16 | | |
| IL17RA exons 1-6 | 19 | Ability to Block IL17A and IL17F |
| IL17RC exons 8-16 | | |
| (Variant 1454) | | |

## Claims

1. An isolated soluble polypeptide comprising exons 8-16 of IL-17RC (amino acid residues 193-447 of SEQ ID NO:2) and exons 1-6 of IL-17RA, wherein said soluble polypeptide specifically binds to IL-17A and IL-17F.

2. The isolated soluble polypeptide of claim 1, wherein the polypeptide comprises amino acid residues 1-458 of SEQ ID NO:158.

3. The isolated soluble polypeptide of claim 2, wherein the polypeptide comprises SEQ ID NO:158.

4. The isolated soluble polypeptide of claim 2, wherein the polypeptide consists of amino acid residues 1-458 of SEQ ID NO:158.

5. The isolated soluble polypeptide of claim 3, wherein the polypeptide consists of SEQ ID NO:158.

6. The isolated soluble polypeptide of claim 1, wherein the polypeptide comprises an amino acid sequence selected from the group consisting of SEQ ID NO:72 and SEQ ID NO:140.

7. An isolated nucleic acid molecule encoding a polypeptide according to any one of claims 1-6.

8. The nucleic acid molecule of claim 7, wherein the nucleic acid molecule comprises the nucleotide sequence shown in SEQ ID NO:157.

9. An expression vector comprising the following operably linked elements:
a) a transcription promoter;
b) a DNA segment encoding a polypeptide according to any one of claims 1-6; and
c) a transcription terminator.

10. An isolated cell into which has been introduced an expression vector of claim 9, wherein the cell expresses the polypeptide encoded by the DNA segment.

11. A method of producing a polypeptide comprising:
a) culturing a cell into which has been introduced an expression vector of claim 9, wherein the cell expresses the polypeptide encoded by the DNA segment; and
b) recovering the expressed polypeptide.

12. The method of claim 11, wherein the cell is a eukaryotic cell, and wherein the expressed polypeptide is secreted from the cell.

13. The method of claim 11 or 12, wherein the encoded polypeptide comprises the amino acid sequence shown in SEQ ID NO:158 or residues 1-458 of SEQ ID NO: 158.

14. The isolated polypeptide of any one of claims 1-6, wherein the polypeptide further comprises PEGylation.

15. A soluble polypeptide according to any one of claims 1-6 for reducing or inhibiting either IL-17A-induced or IL-17F-induced inflammation in a mammal.

16. A soluble polypeptide according to any one of claims 1-6 for reducing IL-17A-induced and IL-17F-induced inflammation in a mammal.

17. Use of a soluble polypeptide according to any one of claims 1-6
in the manufacture of a medicament for treating a mammal afflicted with an inflammatory disease in which IL-17A and/or IL-17F plays a role.

18. The use of claim 17, wherein the disease is selected from the group consisting of a chronic inflammatory disease, an acute inflammatory disease, an autoimmune disease, and a chronic inflammatory airway disease.

19. The use of claim 18, wherein the disease is a chronic inflammatory disease selected from the group consisting of inflammatory bowel disease, ulcerative colitis, Crohn's disease, arthritis, and psoriasis.

20. The use of claim 18, wherein the disease is an autoimmune disease selected from the group consisting of multiple sclerosis, rheumatoid arthritis, and inflammatory bowel disease (IBD).

21. The use of claim 18, wherein the disease is a chronic inflammatory airway disease selected from the group consisting of asthma, chronic obstructive pulmonary disease (COPD), and cystic fibrosis.

22. The use of claim 17, wherein the disease is selected from the group consisting of psoriasis; rheumatoid arthritis; osteoarthritis; ulcerative colitis; Crohn's disease; irritable bowel syndrome (IBS); contact dermatitis; multiple sclerosis (MS); transplant rejection; asthma; chronic obstructive pulmonary disease (COPD); cystic fibrosis; and allergic asthma.

23. The use of any one of claims 18 to 22, wherein the soluble polypeptide is a polypeptide produced by the method of claim 13.

## Patentansprüche

1. Isoliertes lösliches Polypeptid, das Exons 8-16 von IL-17RC (Aminosäurereste 193-447 von SEQ ID NR: 2) und Exons 1-6 von IL-17RA aufweist, wobei das lösliche Polypeptid spezifisch an IL-17A und IL-17F bindet.

2. Isoliertes lösliches Polypeptid nach Anspruch 1, wobei das Polypeptid die Aminoäurereste 1-458 von SEQ ID NR: 158 aufweist.

3. Isoliertes lösliches Polypeptid nach Anspruch 2, wobei das Polypeptid SEQ ID NR: 158 aufweist.

4. Isoliertes lösliches Polypeptid nach Anspruch 2, wobei das Polypeptid aus den Aminosäureresten 1-458 von SEQ ID NR: 158 besteht.

5. Isoliertes lösliches Polypeptid nach Anspruch 3, wobei das Polpeptid aus SEQ ID NR: 158 besteht.

6. Isoliertes lösliches Polypeptid nach Anspruch 1, wobei das Polypeptid eine Aminosäuresequenz aufweist, ausgewählt aus der Gruppe bestehend aus SEQ ID NR: 72 und SEQ ID NR: 140.

7. Isoliertes Nukleinsäuremolekül, das ein Polypeptid gemäß einem der Ansprüche 1-6 kodiert.

8. Nukleinsäuremolekül nach Anspruch 7, wobei das Nukleinsäuremolekül die Nukleotidsequenz, die in SEQ ID NR: 157 gezeigt ist, aufweist.

9. Expressionsvektor, der die folgenden funktionsfähig verbundenen Elemente aufweist:
a) einen Transkriptionspromotor
b) ein DNA-Segment, das ein Polypeptid gemäß einem der Ansprüche 1-6 kodiert; und
c) einen Transkriptionsterminator.

10. Isolierte Zelle, in die ein Expressionsvektor nach Anspruch 9 eingebracht wurde, wobei die Zelle das Polypeptid exprimiert, für welches das DNA-Segment kodiert.

11. Verfahren zum Herstellen eines Polypeptids, aufweisend:
a) Kultivieren einer Zelle, in welche ein Expressionsvektor nach Anspruch 9 eingebracht wurde, wobei die Zelle das Polypeptid exprimiert, für welches das DNA-Segment kodiert; und
b) Gewinnen des exprimierten Polypeptids.

12. Verfahren nach Anspruch 11, wobei die Zelle eine eukaryotische Zelle ist, und wobei das exprimierte Polypeptid von der Zelle sezerniert wird.

13. Verfahren nach Anspruch 11 oder 12, wobei das kodierte Polypeptid die Aminosäuresequenz, die in SEQ ID NR: 158 gezeigt ist oder die Reste 1-458 von SEQ ID NR: 158 aufweist.

14. Isoliertes Polypeptid nach einem der Ansprüche 1-6, wobei das Polypeptid weiterhin eine PEGylierung aufweist.

15. Lösliches Polypeptid gemäß einem der Ansprüche 1-6 zum Vermindern oder Inhibieren von entweder IL-17A-induzierter oder IL-17F-induzierter Entzündung in einem Säugetier.

16. Lösliches Polypeptid gemäß einem der Ansprüche 1-6 zum Vermindern von IL-17A-imduzierer und IL-17F-induzierter Entzündung in einem Säugetier.

17. Verwendung eines löslichen Polypeptids gemäß einem der Ansprüche 1-6 für die Herstellung eines Medikaments zum Behandeln eines Säugetiers, das an einer entzündlichen Erkrankung, bei der IL-17A und/oder IL-17F eine Rolle spielt, leidet.

18. Verwendung nach Anspruch 17, wobei die Erkrankung ausgewählt ist aus der Gruppe bestehend aus einer chronisch entzündlichen Erkrankung, einer akut entzündlichen Erkrankung, einer Autoimmunerkrankung und einer chronisch entzündlichen Atemwegserkrankung.

19. Verwendung nach Anspruch 18, wobei die Erkrankung eine chronisch entzündliche Erkrankung ist, die ausgewählt ist aus der Gruppe bestehend aus entzündlicher Darmerkrankung, Colitis ulcerosa, Morbus Crohn, Arthrose und Psoriasis.

20. Verwendung nach Anspruch 18, wobei die Erkrankung eine Autoimmunerkrankung ist, die ausgewählt ist aus der Gruppe bestehend aus Multipler Sklerose, rheumatoider Arthritis und entzündlicher Darmerkrankung (IBD).

21. Verwendung nach Anspruch 18, wobei die Erkrankung eine chronisch entzündliche Atemwegserkrankung ist, die ausgewählt ist aus der Gruppe bestehend aus Asthma, chronisch obstruktiver Lungenerkrankung (COPD) und Zystofibrose.

22. Verwendung nach Anspruch 17, wobei die Erkrankung ausgewählt ist aus der Gruppe bestehend aus Psoriasis; rheumatoider Arthritis; Osteoarthritis; Colitis ulcerosa; Morbus Crohn; Reizdarmsyndrom (IBS); Kontaktdermatitis; Multipler Sklerose (MS); Transplantatabstoßung; Asthma; chronisch obstruktive Lungenerkrankung (COPD); Zystofibrose; und allergischem Asthma.

23. Verwendung nach einem der Ansprüche 18 bis 22, wobei das lösliche Polypeptid ein Polypeptid ist, das nach dem Verfahren von Anspruch 13 hergestellt wird.

## Revendications

1. Polypeptide soluble isolé renfermant les exons 8-16 de IL-17RC (résidus d'acides aminés 193-447 de SEQ ID NO : 2) et les exons 1-6 de IL-17RA, ce polypeptide soluble se liant spécifiquement à IL-17A et à IL-17F.

2. Polypeptide soluble isolé selon la revendication 1, renfermant les résidus d'acides aminés 1-458 de SEQ ID NO : 158.

3. Polypeptide soluble isolé selon la revendication 2, renfermant SEQ ID NO : 158.

4. Polypeptide soluble isolé selon la revendication 2, constitué par les résidus d'acides aminés 1-458 de SEQ ID NO : 158.

5. Polypeptide soluble isolé selon la revendication 3, constitué par SEQ ID NO : 158.

6. Polypeptide soluble isolé selon la revendication 1, renfermant une séquence d'acides aminés choisie dans le groupe formé par SEQ ID NO : 72 et SEQ ID NO : 140.

7. Molécule d'acide nucléique isolée codant pour un polypeptide selon l'une quelconque des revendications 1 à 6.

8. Molécule d'acide nucléique selon la revendication 7, renfermant la séquence nucléotidique représentée dans SEQ ID NO : 157.

9. Vecteur d'expression renfermant les éléments suivants opérative-ment liés :
a) un promoteur de la transcription,
b) un segment d'ADN codant pour un polypeptide selon l'une quelconque des revendications 1 à 6, et
c) un terminateur de transcription.

10. Cellule isolée dans laquelle a été introduit un vecteur d'expression selon la revendication 9, cette cellule exprimant le polypeptide codé par le segment d'ADN.

11. Procédé d'obtention d'un polypeptide comprenant les étapes consistant à :
a) cultiver une cellule dans laquelle a été introduit un vecteur d'expression selon la revendication 9, cette cellule exprimant le polypeptide codé par le segment d'ADN, et
b) récupérer le polypeptide exprimé.

12. Procédé conforme à la revendication 11, selon lequel la cellule est une cellule eucaryote et le polypeptide exprimé est sécrété par la cellule.

13. Procédé conforme à la revendication 11 ou à la revendication 12, selon lequel le polypeptide codé comprend la séquence d'acides aminés représentée dans SEQ ID NO : 158 ou les résidus 1-458 de SEQ ID NO : 158.

14. Polypeptide isolé selon l'une quelconque des revendications 1 à 6, comprenant en outre une réaction de Pégylation.

15. Polypeptide soluble selon l'une quelconque des revendications 1 à 6, pour réduire ou inhiber soit l'inflammation induite par IL-17A soit l'inflammation induite par IL-17F chez le mammifère.

16. Polypeptide soluble selon l'une quelconque des revendications 1 à 6, pour réduite l'inflammation induite par IL-17A et l'inflammation induite par IL-17F chez le mammifère.

17. Utilisation d'un polypeptide soluble conforme à l'une quelconque des revendications 1 à 6, pour l'obtention d'un médicament pour le traitement d'un mammifère atteint par une maladie inflammatoire dans laquelle IL-17A et/ou IL-17F joue un rôle.

18. Utilisation conforme à la revendication 17, selon laquelle la maladie est choisie dans le groupe formé par une maladie inflammatoire chronique, une maladie inflammatoire aiguë, une maladie auto immune et une maladie inflammatoire chronique des voies aériennes.

19. Utilisation conforme à la revendication 18, selon laquelle la maladie est une maladie inflammatoire chronique choisie dans le groupe formé par les maladies chroniques des intestins, la colite ulcéreuse, la maladie de Crohn, l'arthrite et le psoriasis.

20. Utilisation conforme à la revendication 18, selon laquelle la maladie est une maladie auto immune choisie dans le groupe formé par la sclérose en plaque, l'arthrite rhumatoïde et les maladies inflammatoires des intestins (IBD).

21. Utilisation conforme à la revendication 18, selon laquelle la maladie est une maladie inflammatoire chronique des voix aériennes choisie dans le groupe formé par l'asthme, la broncho-pneumopathie chronique obstructive (COPD) et la fibrose kystique.

22. Utilisation conforme à la revendication 17, selon laquelle la maladie est choisie dans le groupe formé par le psoriasis, l'arthrite rhumatoïde, l'ostéo-arthrite, la colite ulcéreuse, la maladie de Crohn, le syndrome de l'intestin irritable (IBS), la dermatite de contact, la sclérose en plaque (MS), le rejet de greffe, l'asthme, la broncho-pneumopathie chronique obstructive (COPD), la fibrose kystique et l'asthme allergique.

23. Utilisation conforme à l'une quelconque des revendications 18 à 22, selon laquelle le polypeptide soluble est un polypeptide obtenu par le procédé conforme à la revendication 13.


### SEQUENCE LISTING

<110> Levin, Steven D.
   Rixon, Mark W.
   Gao, Zeren
   Lewis, Katherine E.
   Bilsborough, Janine
   Taft, David W.
<120> IL-17A and IL-17F Antagonists and Methods of Using the Same
<130> 05-30
<140> Not Yet Assigned
   <141> 2006-09-28
<150> 60/721,162
   <151> 2005-09-28
<150> 60/753,794
   <151> 2005-12-22
<150> 60/772,022
   <151> 2006-02-10
<150> 60/782,247
   <151> 2006-03-14
<160> 181
<170> FastSEQ for Windows Version 4.0
<210> 1
   <211> 2255
   <212> DNA
   <213> Homo sapiens
<220>
   <221> CDS
   <222> (154)...(2229)
<220>
   <223> Optimized tissue Plasminogen Activator (otPA) pre-pro signal sequence and exons 7-10 of human IL-17RC, and Fc5
<400> 1
   aactacccag cacagccccc tccgccccct ctggaggctg aagagggatt ccagcccctg 60
   ccacccacag acacgggctg actggggtgt ctgcccccct tggggggggg cagcacaggg 120
   cctcaggcct gggtgccacc tggcacctag aag atg cct gtg ccc tgg ttc ttg  174
   ctg tcc ttg gca ctg ggc cga agc cca gtg gtc ctt tct ctg gag agg    222
   ctt gtg ggg cct cag gac gct acc cac tgc tct ccg ggc ctc tcc tgc    270
   cgc ctc tgg gac agt gac ata ctc tgc ctg cct ggg gac atc gtg cct    318
   gct ccg ggc ccc gtg ctg gcg cct acg cac ctg cag aca gag ctg gtg    366
   ctg agg tgc cag aag gag acc gac tgt gac ctc tgt ctg cgt gtg gct    414
   gtc cac ttg gcc gtg cat ggg cac tgg gaa gag cct gaa gat gag gaa    462
   aag ttt gga gga gca gct gac tca ggg gtg gag gag cct agg aat gcc    510
   tct ctc cag gcc caa gtc gtg ctc tcc ttc cag gcc tac cct act gcc    558
   cgc tgc gtc ctg ctg gag gtg caa gtg cct gct gcc ctt gtg cag ttt    606
   ggt cag tct gtg ggc tct gtg gta tat gac tgc ttc gag gct gcc cta    654
   ggg agt gag gta cga atc tgg tcc tat act cag ccc agg tac gag aag    702
   gaa ctc aac cac aca cag cag ctg cct gcc ctg ccc tgg ctc aac gtg    750
   tca gca gat ggt gac aac gtg cat ctg gtt ctg aat gtc tct gag gag    798
   cag cac ttc ggc ctc tcc ctg tac tgg aat cag gtc cag ggc ccc cca    846
   aaa ccc cgg tgg cac aaa aac ctg act gga ccg cag atc att acc ttg    894
   aac cac aca gac ctg gtt ccc tgc ctc tgt att cag gtg tgg cct ctg     942
   gaa cct gac tcc gtt agg acg aac atc tgc ccc ttc agg gag gac ccc     990
   cgc gca cac cag aac ctc tgg caa gcc gcc cga ctg cga ctg ctg acc    1038
   ctg cag agc tgg ctg ctg gac gca ccg tgc tcg ctg ccc gca gaa gcg    1086
   gca ctg tgc tgg cgg gct ccg ggt ggg gac ccc tgc cag cca ctg gtc    1134
   cca ccg ctt tcc tgg gag aac gtc act gtg gac aag gtt ctc gag ttc    1182
   cca ttg ctg aaa ggc cac cct aac ctc tgt gtt cag gtg aac agc tcg    1230
   gag aag ctg cag ctg cag gag tgc ttg tgg gct gac tcc ctg ggg cct    1278
   ctc aaa gac gat gtg cta ctg ttg gag aca cga ggc ccc cag gac aac    1326
   aga tcc ctc tgt gcc ttg gaa ccc agt ggc tgt act tca cta ccc agc    1374
   aaa gcc tcc acg agg gca gct cgc ctt gga gag tac tta cta caa gac    1422
   ctg cag tca ggc cag tgt ctg cag cta tgg gac gat gac ttg gga gcg    1470
   cta tgg gcc tgc ccc atg gac aaa tac atc cac aag cgc tgg gcc ctc    1518
   gtg tgg ctg gcc tgc cta ctc ttt gcc gct gcg ctt tcc ctc atc ctc    1566
   ctt ctc aaa aag gat cac gcg aaa gcg gcc gcc agg ggc cgc gcg gct    1614
   ctg ctc ctc tac tca gcc gat gac tcg ggt ttc gag cgc ctg gtg ggc      1662
   gcc ctg gcg tcg gcc ctg tgc cag ctg ccg ctg cgc gtg gcc gta gac      1710
   ctg tgg agc cgt cgt gaa ctg agc gcg cag ggg ccc gtg gct tgg ttt      1758
   cac gcg cag cgg cgc cag acc ctg cag gag ggc ggc gtg gtg gtc ttg      1806
   ctc ttc tct ccc ggt gcg gtg gcg ctg tgc agc gag tgg cta cag gat      1854
   ggg gtg tcc ggg ccc ggg gcg cac ggc ccg cac gac gcc ttc cgc gcc      1902
   tcg ctc agc tgc gtg ctg ccc gac ttc ttg cag ggc cgg gcg ccc ggc      1950
   agc tac gtg ggg gcc tgc ttc gac agg ctg ctc cac ccg gac gcc gta      1998
   ccc gcc ctt ttc cgc acc gtg ccc gtc ttc aca ctg ccc tcc caa ctg      2046
   cca gac ttc ctg ggg gcc ctg cag cag cct cgc gcc ccg cgt tcc ggg      2094
   cgg ctc caa gag aga gcg gag caa gtg tcc cgg gcc ctt cag cca gcc      2142
   ctg gat agc tac ttc cat ccc ccg ggg act ccc gcg ccg gga cgc ggg      2190
   gtg gga cca ggg gcg gga cct ggg gcg ggg gac ggg act taaataaagg       2239
   cagacgctgt ttttct                                                    2255
<210> 2
   <211> 692
   <212> PRT
   <213> Homo sapiens
<400> 2
<210> 3
   <211> 432
   <212> PRT
   <213> homo sapiens
<400> 3
   Leu Glu Arg Leu Val Gly Pro Gln Asp Ala Thr His Cys Ser Pro Gly
   1               5                   10                  15
   Leu Ser Cys Arg Leu Trp Asp Ser Asp Ile Leu Cys Leu Pro Gly Asp
               20                  25                  30
   Ile Val Pro Ala Pro Gly Pro Val Leu Ala Pro Thr His Leu Gln Thr
           35                  40                  45
   Glu Leu Val Leu Arg Cys Gln Lys Glu Thr Asp Cys Asp Leu Cys Leu
       50                  55                  60
   Arg Val Ala Val His Leu Ala Val His Gly His Trp Glu Glu Pro Glu
   65                  70                  75                  80
   Asp Glu Glu Lys Phe Gly Gly Ala Ala Asp Ser Gly Val Glu Glu Pro
                   85                  90                  95
   Arg Asn Ala Ser Leu Gln Ala Gln Val Val Leu Ser Phe Gln Ala Tyr
               100                 105                 110
   Pro Thr Ala Arg Cys Val Leu Leu Glu Val Gln Val Pro Ala Ala Leu
           115                 120                 125
   Val Gln Phe Gly Gln Ser Val Gly Ser Val Val Tyr Asp Cys Phe Glu
       130                 135                 140
   Ala Ala Leu Gly Ser Glu Val Arg Ile Trp Ser Tyr Thr Gln Pro Arg
   145                 150                 155                 160
   Tyr Glu Lys Glu Leu Asn His Thr Gln Gln Leu Pro Ala Leu Pro Trp
                   165                 170                 175
   Leu Asn Val Ser Ala Asp Gly Asp Asn Val His Leu Val Leu Asn Val
               180                 185                 190
   Ser Glu Glu Gln His Phe Gly Leu Ser Leu Tyr Trp Asn Gln Val Gln
           195                 200                 205
   Gly Pro Pro Lys Pro Arg Trp His Lys Asn Leu Thr Gly Pro Gln Ile
       210                 215                 220
   Ile Thr Leu Asn His Thr Asp Leu Val Pro Cys Leu Cys Ile Gln Val
   225                 230                 235                 240
   Trp Pro Leu Glu Pro Asp Ser Val Arg Thr Asn Ile Cys Pro Phe Arg
                   245                 250                 255
   Glu Asp Pro Arg Ala His Gln Asn Leu Trp Gln Ala Ala Arg Leu Arg
               260                 265                 270
   Leu Leu Thr Leu Gln Ser Trp Leu Leu Asp Ala Pro Cys Ser Leu Pro
           275                 280                 285
   Ala Glu Ala Ala Leu Cys Trp Arg Ala Pro Gly Gly Asp Pro Cys Gln
       290                 295                 300
   Pro Leu Val Pro Pro Leu Ser Trp Glu Asn Val Thr Val Asp Lys Val
   305                 310                 315                 320
   Leu Glu Phe Pro Leu Leu Lys Gly His Pro Asn Leu Cys Val Gln Val
                   325                 330                 335
   Asn Ser Ser Glu Lys Leu Gln Leu Gln Glu Cys Leu Trp Ala Asp Ser
               340                 345                 350
   Leu Gly Pro Leu Lys Asp Asp Val Leu Leu Leu Glu Thr Arg Gly Pro
           355                 360                 365
   Gln Asp Asn Arg Ser Leu Cys Ala Leu Glu Pro Ser Gly Cys Thr Ser
       370                 375                 380
   Leu Pro Ser Lys Ala Ser Thr Arg Ala Ala Arg Leu Gly Glu Tyr Leu
   385                 390                 395                 400
   Leu Gln Asp Leu Gln Ser Gly Gln Cys Leu Gln Leu Trp Asp Asp Asp
                   405                 410                 415
   Leu Gly Ala Leu Trp Ala Cys Pro Met Asp Lys Tyr Ile His Lys Arg
               420                 425                 430
<210> 4
   <211> 1753
   <212> DNA
   <213> Homo sapiens
<220>
   <221> CDS
   <222> (2)...(1726)
<400> 4
   g gag gag cct agg aat gcc tct ctc cag gcc caa gtc gtg ctc tcc ttc 49
     Glu Glu Pro Arg Asn Ala Ser Leu Gln Ala Gln Val Val Leu Ser Phe
      1               5                  10                  15
   cag gcc tac cct act gcc cgc tgc gtc ctg ctg gag gtg caa gtg cct   97
   Gln Ala Tyr Pro Thr Ala Arg Cys Val Leu Leu Glu Val Gln Val Pro
               20                  25                  30
   gct gcc ctt gtg cag ttt ggt cag tct gtg ggc tct gtg gta tat gac  145
   Ala Ala Leu Val Gln Phe Gly Gln Ser Val Gly Ser Val Val Tyr Asp
           35                  40                  45
   tgc ttc gag gct gcc cta ggg agt gag gta cga atc tgg tcc tat act  193
   Cys Phe Glu Ala Ala Leu Gly Ser Glu Val Arg Ile Trp Ser Tyr Thr
       50                  55                  60
   cag ccc agg tac gag aag gaa ctc aac cac aca cag cag ctg cct gcc  241
   Gln Pro Arg Tyr Glu Lys Glu Leu Asn His Thr Gln Gln Leu Pro Ala
    65                   70                   75                   80
   ctg ccc tgg ctc aac gtg tca gca gat ggt gac aac gtg cat ctg gtt    289
   Leu Pro Trp Leu Asn Val Ser Ala Asp Gly Asp Asn Val His Leu Val
                    85                  90                  95
   ctg aat gtc tct gag gag cag cac ttc ggc ctc tcc ctg tac tgg aat    337
   Leu Asn Val Ser Glu Glu Gln His Phe Gly Leu Ser Leu Tyr Trp Asn
               100                 105                 110
   cag gtc cag ggc ccc cca aaa ccc cgg tgg cac aaa aac ctg act gga    385
   Gln Val Gln Gly Pro Pro Lys Pro Arg Trp His Lys Asn Leu Thr Gly
           115                 120                 125
   ccg cag atc att acc ttg aac cac aca gac ctg gtt ccc tgc ctc tgt    433
   Pro Gln Ile Ile Thr Leu Asn His Thr Asp Leu Val Pro Cys Leu Cys
       130                 135                 140
   att cag gtg tgg cct ctg gaa cct gac tcc gtt agg acg aac atc tgc    481
   Ile Gln Val Trp Pro Leu Glu Pro Asp Ser Val Arg Thr Asn Ile Cys
   145                 150                 155                 160
   ccc ttc agg gag gac ccc cgc gca cac cag aac ctc tgg caa gcc gcc    529
   Pro Phe Arg Glu Asp Pro Arg Ala His Gln Asn Leu Trp Gln Ala Ala
                   165                 170                 175
   cga ctg cga ctg ctg acc ctg cag agc tgg ctg ctg gac gca ccg tgc    577
   Arg Leu Arg Leu Leu Thr Leu Gln Ser Trp Leu Leu Asp Ala Pro Cys
               180                 185                 190
   tcg ctg ccc gca gaa gcg gca ctg tgc tgg cgg gct ccg ggt ggg gac    625
   Ser Leu Pro Ala Glu Ala Ala Leu Cys Trp Arg Ala Pro Gly Gly Asp
           195                 200                 205
   ccc tgc cag cca ctg gtc cca ccg ctt tcc tgg gag aac gtc act gtg    673
   Pro Cys Gln Pro Leu Val Pro Pro Leu Ser Trp Glu Asn Val Thr Val
       210                 215                 220
   gac gtg aac agc tcg gag aag ctg cag ctg cag gag tgc ttg tgg gct    721
   Asp Val Asn Ser Ser Glu Lys Leu Gln Leu Gln Glu Cys Leu Trp Ala
   225                 230                 235                 240
   gac tcc ctg ggg cct ctc aaa gac gat gtg cta ctg ttg gag aca cga    769
   Asp Ser Leu Gly Pro Leu Lys Asp Asp Val Leu Leu Leu Glu Thr Arg
                   245                 250                 255
   ggc ccc cag gac aac aga tcc ctc tgt gcc ttg gaa ccc agt ggc tgt    817
   Gly Pro Gln Asp Asn Arg Ser Leu Cys Ala Leu Glu Pro Ser Gly Cys
               260                 265                 270
   act tca cta ccc agc aaa gcc tcc acg agg gca gct cgc ctt gga gag    865
   Thr Ser Leu Pro Ser Lys Ala Ser Thr Arg Ala Ala Arg Leu Gly Glu
           275                 280                 285
   tac tta cta caa gac ctg cag tca ggc cag tgt ctg cag cta tgg gac    913
   Tyr Leu Leu Gln Asp Leu Gln Ser Gly Gln Cys Leu Gln Leu Trp Asp
       290                 295                 300
   gat gac ttg gga gcg cta tgg gcc tgc ccc atg gac aaa tac atc cac    961
   Asp Asp Leu Gly Ala Leu Trp Ala Cys Pro Met Asp Lys Tyr Ile His
   305                 310                 315                 320
   aag cgc tgg gcc ctc gtg tgg ctg gcc tgc cta ctc ttt gcc gct gcg    1009
   Lys Arg Trp Ala Leu Val Trp Leu Ala Cys Leu Leu Phe Ala Ala Ala
                   325                 330                 335
   ctt tcc ctc atc ctc ctt ctc aaa aag gat cac gcg aaa ggg tgg ctg    1057
   Leu Ser Leu Ile Leu Leu Leu Lys Lys Asp His Ala Lys Gly Trp Leu
               340                 345                 350
   agg ctc ttg aaa cag gac gtc cgc tcg ggg gcg gcc gcc agg ggc cgc    1105
   Arg Leu Leu Lys Gln Asp Val Arg Ser Gly Ala Ala Ala Arg Gly Arg
           355                 360                 365
   gcg gct ctg ctc ctc tac tca gcc gat gac tcg ggt ttc gag cgc ctg    1153
   Ala Ala Leu Leu Leu Tyr Ser Ala Asp Asp Ser Gly Phe Glu Arg Leu
       370                 375                 380
   gtg ggc gcc ctg gcg tcg gcc ctg tgc cag ctg ccg ctg cgc gtg gcc    1201
   Val Gly Ala Leu Ala Ser Ala Leu Cys Gln Leu Pro Leu Arg Val Ala
   385                 390                 395                 400
   gta gac ctg tgg agc cgt cgt gaa ctg agc gcg cag ggg ccc gtg gct    1249
   Val Asp Leu Trp Ser Arg Arg Glu Leu Ser Ala Gln Gly Pro Val Ala
                   405                 410                 415
   tgg ttt cac gcg cag cgg cgc cag acc ctg cag gag ggc ggc gtg gtg    1297
   Trp Phe His Ala Gln Arg Arg Gln Thr Leu Gln Glu Gly Gly Val Val
               420                 425                 430
   gtc ttg ctc ttc tct ccc ggt gcg gtg gcg ctg tgc agc gag tgg cta    1345
   Val Leu Leu Phe Ser Pro Gly Ala Val Ala Leu Cys Ser Glu Trp Leu
           435                 440                 445
   cag gat ggg gtg tcc ggg ccc ggg gcg cac ggc ccg cac gac gcc ttc    1393
   Gln Asp Gly Val Ser Gly Pro Gly Ala His Gly Pro His Asp Ala Phe
       450                 455                 460
   cgc gcc tcg ctc agc tgc gtg ctg ccc gac ttc ttg cag ggc cgg gcg    1441
   Arg Ala Ser Leu Ser Cys Val Leu Pro Asp Phe Leu Gln Gly Arg Ala
   465                 470                 475                 480
   ccc ggc agc tac gtg ggg gcc tgc ttc gac agg ctg ctc cac ccg gac    1489
   Pro Gly Ser Tyr Val Gly Ala Cys Phe Asp Arg Leu Leu His Pro Asp
                   485                 490                 495
   gcc gta ccc gcc ctt ttc cgc acc gtg ccc gtc ttc aca ctg ccc tcc    1537
   Ala Val Pro Ala Leu Phe Arg Thr Val Pro Val Phe Thr Leu Pro Ser
               500                 505                 510
   caa ctg cca gac ttc ctg ggg gcc ctg cag cag cct cgc gcc ccg cgt    1585
   Gln Leu Pro Asp Phe Leu Gly Ala Leu Gln Gln Pro Arg Ala Pro Arg
           515                 520                 525
   tcc ggg cgg ctc caa gag aga gcg gag caa gtg tcc cgg gcc ctt cag    1633
   Ser Gly Arg Leu Gln Glu Arg Ala Glu Gln Val Ser Arg Ala Leu Gln
       530                 535                 540
   cca gcc ctg gat agc tac ttc cat ccc ccg ggg act ccc gcg ccg gga    1681
   Pro Ala Leu Asp Ser Tyr Phe His Pro Pro Gly Thr Pro Ala Pro Gly
   545 550 555 560
   cgc ggg gtg gga cca ggg gcg gga cct ggg gcg ggg gac ggg act 1726
   Arg Gly Val Gly Pro Gly Ala Gly Pro Gly Ala Gly Asp Gly Thr
   565 570 575
   taaataaagg cagacgctgt ttttcta 1753
<210> 5
   <211> 575
   <212> PRT
   <213> Homo sapiens
<400> 5
   Glu Glu Pro Arg Asn Ala Ser Leu Gln Ala Gln Val Val Leu Ser Phe
   1               5                   10                  15
   Gln Ala Tyr Pro Thr Ala Arg Cys Val Leu Leu Glu Val Gln Val Pro
               20                  25                  30
   Ala Ala Leu Val Gln Phe Gly Gln Ser Val Gly Ser Val Val Tyr Asp
           35                  40                  45
   Cys Phe Glu Ala Ala Leu Gly Ser Glu Val Arg Ile Trp Ser Tyr Thr
       50                  55                  60
   Gln Pro Arg Tyr Glu Lys Glu Leu Asn His Thr Gln Gln Leu Pro Ala
   65                  70                  75                  80
   Leu Pro Trp Leu Asn Val Ser Ala Asp Gly Asp Asn Val His Leu Val
                   85                  90                  95
   Leu Asn Val Ser Glu Glu Gln His Phe Gly Leu Ser Leu Tyr Trp Asn
               100                 105                 110
   Gln Val Gln Gly Pro Pro Lys Pro Arg Trp His Lys Asn Leu Thr Gly
           115                 120                 125
   Pro Gln Ile Ile Thr Leu Asn His Thr Asp Leu Val Pro Cys Leu Cys
       130                 135                 140
   Ile Gln Val Trp Pro Leu Glu Pro Asp Ser Val Arg Thr Asn Ile Cys
   145                 150                 155                 160
   Pro Phe Arg Glu Asp Pro Arg Ala His Gln Asn Leu Trp Gln Ala Ala
                   165                 170                 175
   Arg Leu Arg Leu Leu Thr Leu Gln Ser Trp Leu Leu Asp Ala Pro Cys
               180                 185                 190
   Ser Leu Pro Ala Glu Ala Ala Leu Cys Trp Arg Ala Pro Gly Gly Asp
           195                 200                 205
   Pro Cys Gln Pro Leu Val Pro Pro Leu Ser Trp Glu Asn Val Thr Val
       210                 215                 220
   Asp Val Asn Ser Ser Glu Lys Leu Gln Leu Gln Glu Cys Leu Trp Ala
   225                 230                 235                 240
   Asp Ser Leu Gly Pro Leu Lys Asp Asp Val Leu Leu Leu Glu Thr Arg
                   245                 250                 255
   Gly Pro Gln Asp Asn Arg Ser Leu Cys Ala Leu Glu Pro Ser Gly Cys
               260                 265                 270
   Thr Ser Leu Pro Ser Lys Ala Ser Thr Arg Ala Ala Arg Leu Gly Glu
           275                 280                 285
   Tyr Leu Leu Gln Asp Leu Gln Ser Gly Gln Cys Leu Gln Leu Trp Asp
       290                 295                 300
   Asp Asp Leu Gly Ala Leu Trp Ala Cys Pro Met Asp Lys Tyr Ile His
   305                 310                 315                 320
   Lys Arg Trp Ala Leu Val Trp Leu Ala Cys Leu Leu Phe Ala Ala Ala
                   325                 330                 335
   Leu Ser Leu Ile Leu Leu Leu Lys Lys Asp His Ala Lys Gly Trp Leu
               340                 345                 350
   Arg Leu Leu Lys Gln Asp Val Arg Ser Gly Ala Ala Ala Arg Gly Arg
           355                 360                 365
   Ala Ala Leu Leu Leu Tyr Ser Ala Asp Asp Ser Gly Phe Glu Arg Leu
       370                 375                 380
   Val Gly Ala Leu Ala Ser Ala Leu Cys Gln Leu Pro Leu Arg Val Ala
   385                 390                 395                 400
   Val Asp Leu Trp Ser Arg Arg Glu Leu Ser Ala Gln Gly Pro Val Ala
                   405                 410                 415
   Trp Phe His Ala Gln Arg Arg Gln Thr Leu Gln Glu Gly Gly Val Val
               420                 425                 430
   Val Leu Leu Phe Ser Pro Gly Ala Val Ala Leu Cys Ser Glu Trp Leu
           435                 440                 445
   Gln Asp Gly Val Ser Gly Pro Gly Ala His Gly Pro His Asp Ala Phe
       450                 455                 460
   Arg Ala Ser Leu Ser Cys Val Leu Pro Asp Phe Leu Gln Gly Arg Ala
   465                 470                 475                 480
   Pro Gly Ser Tyr Val Gly Ala Cys Phe Asp Arg Leu Leu His Pro Asp
                   485                 490                 495
   Ala Val Pro Ala Leu Phe Arg Thr Val Pro Val Phe Thr Leu Pro Ser
               500                 505                 510
   Gln Leu Pro Asp Phe Leu Gly Ala Leu Gln Gln Pro Arg Ala Pro Arg
           515                 520                 525
   Ser Gly Arg Leu Gln Glu Arg Ala Glu Gln Val Ser Arg Ala Leu Gln
       530                 535                 540
   Pro Ala Leu Asp Ser Tyr Phe His Pro Pro Gly Thr Pro Ala Pro Gly
   545                 550                 555                 560
   Arg Gly Val Gly Pro Gly Ala Gly Pro Gly Ala Gly Asp Gly Thr
                   565                 570                 575
<210> 6
   <211> 1725
   <212> DNA
   <213> Artificial Sequence
<220>
   <223> degenerate sequence
<220>
   <221> misc feature
   <222> (1)...(1725)
   <223> n = A,T,C or G
<400> 6
   gargarccnm gnaaygcnws nytncargcn cargtngtny tnwsnttyca rgcntayccn 60
   acngcnmgnt gygtnytnyt ngargtncar gtnccngcng cnytngtnca rttyggncar 120
   wsngtnggnw sngtngtnta ygaytgytty gargcngcny tnggnwsnga rgtnmgnath 180
   tggwsntaya cncarccnmg ntaygaraar garytnaayc ayacncarca rytnccngcn 240
   ytnccntggy tnaaygtnws ngcngayggn gayaaygtnc ayytngtnyt naaygtnwsn 300
   gargarcarc ayttyggnyt nwsnytntay tggaaycarg tncarggncc nccnaarccn 360
   mgntggcaya araayytnac nggnccncar athathacny tnaaycayac ngayytngtn 420
   ccntgyytnt gyathcargt ntggccnytn garccngayw sngtnmgnac naayathtgy 480
   ccnttymgng argayccnmg ngcncaycar aayytntggc argcngcnmg nytnmgnytn 540
   ytnacnytnc arwsntggyt nytngaygcn ccntgywsny tnccngcnga rgcngcnytn 600
   tgytggmgng cnccnggngg ngayccntgy carccnytng tnccnccnyt nwsntgggar 660
   aaygtnacng tngaygtnaa ywsnwsngar aarytncary tncargartg yytntgggcn 720
   gaywsnytng gnccnytnaa rgaygaygtn ytnytnytng aracnmgngg nccncargay 780
   aaymgnwsny tntgygcnyt ngarccnwsn ggntgyacnw snytnccnws naargcnwsn 840
   acnmgngcng cnmgnytngg ngartayytn ytncargayy tncarwsngg ncartgyytn 900
   carytntggg aygaygayyt nggngcnytn tgggcntgyc cnatggayaa rtayathcay 960
   aarmgntggg cnytngtntg gytngcntgy ytnytnttyg cngcngcnyt nwsnytnath 1020
   ytnytnytna araargayca ygcnaarggn tggytnmgny tnytnaarca rgaygtnmgn 1080
   wsnggngcng cngcnmgngg nmgngcngcn ytnytnytnt aywsngcnga ygaywsnggn 1140
   ttygarmgny tngtnggngc nytngcnwsn gcnytntgyc arytnccnyt nmgngtngcn 1200
   gtngayytnt ggwsnmgnmg ngarytnwsn gcncarggnc cngtngcntg gttycaygcn 1260
   carmgnmgnc aracnytnca rgarggnggn gtngtngtny tnytnttyws nccnggngcn 1320
   gtngcnytnt gywsngartg gytncargay ggngtnwsng gnccnggngc ncayggnccn 1380
   caygaygcnt tymgngcnws nytnwsntgy gtnytnccng ayttyytnca rggnmgngcn 1440
   ccnggnwsnt aygtnggngc ntgyttygay mgnytnytnc ayccngaygc ngtnccngcn 1500
   ytnttymgna cngtnccngt nttyacnytn ccnwsncary tnccngaytt yytnggngcn 1560
   ytncarcarc cnmgngcncc nmgnwsnggn mgnytncarg armgngcnga rcargtnwsn 1620
   mgngcnytnc arccngcnyt ngaywsntay ttycayccnc cnggnacncc ngcnccnggn 1680
   mgnggngtng gnccnggngc nggnccnggn gcnggngayg gnacn                 1725
<210> 7
   <211> 2076
   <212> DNA
   <213> Artificial Sequence
<220>
   <223> degenerate sequence
<220>
   <221> misc_feature
   <222> (1)..(2076)
   <223> n = A,T,C or G
<400> 7
   atgccngtnc cntggttyyt nytnwsnytn gcnytnggnm gnwsnccngt ngtnytnwsn 60
   ytngarmgny tngtnggncc ncargaygcn acncaytgyw snccnggnyt nwsntgymgn 120
   ytntgggayw sngayathyt ntgyytnccn ggngayathg tnccngcncc nggnccngtn 180
   ytngcnccna cncayytnca racngarytn gtnytnmgnt gycaraarga racngaytgy 240
   gayytntgyy tnmgngtngc ngtncayytn gcngtncayg gncaytggga rgarccngar 300
   gaygargara arttyggngg ngcngcngay wsnggngtng argarccnmg naaygcnwsn 360
   ytncargcnc argtngtnyt nwsnttycar gcntayccna cngcnmgntg ygtnytnytn 420
   gargtncarg tnccngcngc nytngtncar ttyggncarw sngtnggnws ngtngtntay 480
   gaytgyttyg argcngcnyt nggnwsngar gtnmgnatht ggwsntayac ncarccnmgn 540
   taygaraarg arytnaayca yacncarcar ytnccngcny tnccntggyt naaygtnwsn 600
   gcngayggng ayaaygtnca yytngtnytn aaygtnwsng argarcarca yttyggnytn 660
   wsnytntayt ggaaycargt ncarggnccn ccnaarccnm gntggcayaa raayytnacn 720
   ggnccncara thathacnyt naaycayacn gayytngtnc cntgyytntg yathcargtn 780
   tggccnytng arccngayws ngtnmgnacn aayathtgyc cnttymgnga rgayccnmgn 840
   gcncaycara ayytntggca rgcngcnmgn ytnmgnytny tnacnytnca rwsntggytn 900
   ytngaygcnc cntgywsnyt nccngcngar gcngcnytnt gytggmgngc nccnggnggn 960
   gayccntgyc arccnytngt nccnccnytn wsntgggara aygtnacngt ngayaargtn 1020
   ytngarttyc cnytnytnaa rggncayccn aayytntgyg tncargtnaa ywsnwsngar 1080
   aarytncary tncargartg yytntgggcn gaywsnytng gnccnytnaa rgaygaygtn 1140
   ytnytnytng aracnmgngg nccncargay aaymgnwsny tntgygcnyt ngarccnwsn 1200
   ggntgyacnw snytnccnws naargcnwsn acnmgngcng cnmgnytngg ngartayytn 1260
   ytncargayy tncarwsngg ncartgyytn carytntggg aygaygayyt nggngcnytn 1320
   tgggcntgyc cnatggayaa rtayathcay aarmgntggg cnytngtntg gytngcntgy 1380
   ytnytnttyg cngcngcnyt nwsnytnath ytnytnytna araargayca ygcnaargcn 1440
   gcngcnmgng gnmgngcngc nytnytnytn taywsngcng aygaywsngg nttygarmgn 1500
   ytngtnggng cnytngcnws ngcnytntgy carytnccny tnmgngtngc ngtngayytn 1560
   tggwsnmgnm gngarytnws ngcncarggn ccngtngcnt ggttycaygc ncarmgnmgn 1620
   caracnytnc argarggngg ngtngtngtn ytnytnttyw snccnggngc ngtngcnytn 1680
   tgywsngart ggytncarga yggngtnwsn ggnccnggng cncayggncc ncaygaygcn 1740
   ttymgngcnw snytnwsntg ygtnytnccn gayttyytnc arggnmgngc nccnggnwsn 1800
   taygtnggng cntgyttyga ymgnytnytn cayccngayg cngtnccngc nytnttymgn 1860
   acngtnccng tnttyacnyt nccnwsncar ytnccngayt tyytnggngc nytncarcar 1920
   ccnmgngcnc cnmgnwsngg nmgnytncar garmgngcng arcargtnws nmgngcnytn 1980
   carccngcny tngaywsnta yttycayccn ccnggnacnc cngcnccngg nmgnggngtn 2040
   ggnccnggng cnggnccngg ngcnggngay ggnacn 2076
<210> 8
   <211> 21
   <212> DNA
   <213> Artificial Sequence
<220>
   <223> PCR primer
<400> 8
   cggcgtggtg gtcttgctct t 21
<210> 9
   <211> 16
   <212> PRT
   <213> Artificial Sequence
<220>
   <223> peptide linker
<400> 9
   Gly Gly Ser Gly Gly Ser Gly Gly Gly Gly Ser Gly Gly Gly Gly Ser
    1               5                  10                  15
<210> 10
   <211> 688
   <212> PRT
   <213> Artificial Sequence
<220>
   <223> Chimeric Zcytor14 protein
<400> 10
   Met Pro Val Pro Trp Phe Leu Leu Ser Leu Ala Leu Gly Arg Ser Pro
    1               5                  10                  15
   Val Val Leu Ser Leu Glu Arg Leu Val Gly Pro Gln Asp Ala Thr His
               20                  25                  30
   Cys Ser Pro Gly Leu Ser Cys Arg Leu Trp Asp Ser Asp Ile Leu Cys
           35                  40                  45
   Leu Pro Gly Asp Ile Val Pro Ala Pro Gly Pro Val Leu Ala Pro Thr
       50                  55                  60
   His Leu Gln Thr Glu Leu Val Leu Arg Cys Gln Lys Glu Thr Asp Cys
   65                  70                  75                  80
   Asp Leu Cys Leu Arg Val Ala Val His Leu Ala Val His Gly His Trp
                   85                  90                  95
   Glu Glu Pro Glu Asp Glu Glu Lys Phe Gly Gly Ala Ala Asp Ser Gly
               100                 105                 110
   Val Glu Glu Pro Arg Asn Ala Ser Leu Gln Ala Gln Val Val Leu Ser
           115                 120                 125
   Phe Gln Ala Tyr Pro Thr Ala Arg Cys Val Leu Leu Glu Val Gln Val
       130                 135                 140
   Pro Ala Ala Leu Val Gln Phe Gly Gln Ser Val Gly Ser Val Val Tyr
   145                 150                 155                 160
   Asp Cys Phe Glu Ala Ala Leu Gly Ser Glu Val Arg Ile Trp Ser Tyr
                   165                 170                 175
   Thr Gln Pro Arg Tyr Glu Lys Glu Leu Asn His Thr Gln Gln Leu Pro
               180                 185                 190
   Ala Leu Pro Trp Leu Asn Val Ser Ala Asp Gly Asp Asn Val His Leu
                   195                 200                 205
   Val Leu Asn Val Ser Glu Glu Gln His Phe Gly Leu Ser Leu Tyr Trp
       210                 215                 220
   Asn Gln Val Gln Gly Pro Pro Lys Pro Arg Trp His Lys Asn Leu Thr
   225                 230                 235                 240
   Gly Pro Gln Ile Ile Thr Leu Asn His Thr Asp Leu Val Pro Cys Leu
                   245                 250                 255
   Cys Ile Gln Val Trp Pro Leu Glu Pro Asp Ser Val Arg Thr Asn Ile
               260                 265                 270
   Cys Pro Phe Arg Glu Asp Pro Arg Ala His Gln Asn Leu Trp Gln Ala
           275                 280                 285
   Ala Arg Leu Arg Leu Leu Thr Leu Gln Ser Trp Leu Leu Asp Ala Pro
       290                 295                 300
   Cys Ser Leu Pro Ala Glu Ala Ala Leu Cys Trp Arg Ala Pro Gly Gly
   305                 310                 315                 320
   Asp Pro Cys Gln Pro Leu Val Pro Pro Leu Ser Trp Glu Asn Val Thr
                   325                 330                 335
   Val Asp Val Asn Ser Ser Glu Lys Leu Gln Leu Gln Glu Cys Leu Trp
               340                 345                 350
   Ala Asp Ser Leu Gly Pro Leu Lys Asp Asp Val Leu Leu Leu Glu Thr
           355                 360                 365
   Arg Gly Pro Gln Asp Asn Arg Ser Leu Cys Ala Leu Glu Pro Ser Gly
       370                 375                 380
   Cys Thr Ser Leu Pro Ser Lys Ala Ser Thr Arg Ala Ala Arg Leu Gly
   385                 390                 395                 400
   Glu Tyr Leu Leu Gln Asp Leu Gln Ser Gly Gln Cys Leu Gln Leu Trp
                   405                 410                 415
   Asp Asp Asp Leu Gly Ala Leu Trp Ala Cys Pro Met Asp Lys Tyr Ile
               420                 425                 430
   His Lys Arg Trp Ala Leu Val Trp Leu Ala Cys Leu Leu Phe Ala Ala
           435                 440                 445
   Ala Leu Ser Leu Ile Leu Leu Leu Lys Lys Asp His Ala Lys Gly Trp
       450                 455                 460
   Leu Arg Leu Leu Lys Gln Asp Val Arg Ser Gly Ala Ala Ala Arg Gly
   465                 470                 475                 480
   Arg Ala Ala Leu Leu Leu Tyr Ser Ala Asp Asp Ser Gly Phe Glu Arg
                   485                 490                 495
   Leu Val Gly Ala Leu Ala Ser Ala Leu Cys Gln Leu Pro Leu Arg Val
               500                 505                 510
   Ala Val Asp Leu Trp Ser Arg Arg Glu Leu Ser Ala Gln Gly Pro Val
           515                 520                 525
   Ala Trp Phe His Ala Gln Arg Arg Gln Thr Leu Gln Glu Gly Gly Val
       530                 535                 540
   Val Val Leu Leu Phe Ser Pro Gly Ala Val Ala Leu Cys Ser Glu Trp
   545                 550                 555                 560
   Leu Gln Asp Gly Val Ser Gly Pro Gly Ala His Gly Pro His Asp Ala
                   565                 570                 575
   Phe Arg Ala Ser Leu Ser Cys Val Leu Pro Asp Phe Leu Gln Gly Arg
               580                 585                 590
   Ala Pro Gly Ser Tyr Val Gly Ala Cys Phe Asp Arg Leu Leu His Pro
           595                 600                 605
   Asp Ala Val Pro Ala Leu Phe Arg Thr Val Pro Val Phe Thr Leu Pro
       610                 615                 620
   Ser Gln Leu Pro Asp Phe Leu Gly Ala Leu Gln Gln Pro Arg Ala Pro
   625                 630                 635                 640
   Arg Ser Gly Arg Leu Gln Glu Arg Ala Glu Gln Val Ser Arg Ala Leu
                   645                 650                 655
   Gln Pro Ala Leu Asp Ser Tyr Phe His Pro Pro Gly Thr Pro Ala Pro
               660                 665                 670
   Gly Arg Gly Val Gly Pro Gly Ala Gly Pro Gly Ala Gly Asp Gly Thr
   675 680 685
<210> 11
   <211> 705
   <212> PRT
   <213> Artificial Sequence
<220>
   <223> Chimeric Zcytor14 protein
<400> 11
   Met Pro Val Pro Trp Phe Leu Leu Ser Leu Ala Leu Gly Arg Ser Pro
   1 5 10 15
   Val Val Leu Ser Leu Glu Arg Leu Val Gly Pro Gln Asp Ala Thr His
   20 25 30
   Cys Ser Pro Gly Leu Ser Cys Arg Leu Trp Asp Ser Asp Ile Leu Cys
   35 40 45
   Leu Pro Gly Asp Ile Val Pro Ala Pro Gly Pro Val Leu Ala Pro Thr
   50 55 60
   His Leu Gln Thr Glu Leu Val Leu Arg Cys Gln Lys Glu Thr Asp Cys
   65 70 75 80
   Asp Leu Cys Leu Arg Val Ala Val His Leu Ala Val His Gly His Trp
   85 90 95
   Glu Glu Pro Glu Asp Glu Glu Lys Phe Gly Gly Ala Ala Asp Ser Gly
   100 105 110
   Val Glu Glu Pro Arg Asn Ala Ser Leu Gln Ala Gln Val Val Leu Ser
   115 120 125
   Phe Gln Ala Tyr Pro Thr Ala Arg Cys Val Leu Leu Glu Val Gln Val
   130 135 140
   Pro Ala Ala Leu Val Gln Phe Gly Gln Ser Val Gly Ser Val Val Tyr
   145 150 155 160
   Asp Cys Phe Glu Ala Ala Leu Gly Ser Glu Val Arg Ile Trp Ser Tyr
   165 170 175
   Thr Gln Pro Arg Tyr Glu Lys Glu Leu Asn His Thr Gln Gln Leu Pro
   180 185 190
   Ala Leu Pro Trp Leu Asn Val Ser Ala Asp Gly Asp Asn Val His Leu
   195 200 205
   Val Leu Asn Val Ser Glu Glu Gln His Phe Gly Leu Ser Leu Tyr Trp
   210 215 220
   Asn Gln Val Gln Gly Pro Pro Lys Pro Arg Trp His Lys Asn Leu Thr
   225 230 235 240
   Gly Pro Gln Ile Ile Thr Leu Asn His Thr Asp Leu Val Pro Cys Leu
   245 250 255
   Cys Ile Gln Val Trp Pro Leu Glu Pro Asp Ser Val Arg Thr Asn Ile
   260 265 270
   Cys Pro Phe Arg Glu Asp Pro Arg Ala His Gln Asn Leu Trp Gln Ala
   275 280 285
   Ala Arg Leu Arg Leu Leu Thr Leu Gln Ser Trp Leu Leu Asp Ala Pro
   290 295 300
   Cys Ser Leu Pro Ala Glu Ala Ala Leu Cys Trp Arg Ala Pro Gly Gly
   305 310 315 320
   Asp Pro Cys Gln Pro Leu Val Pro Pro Leu Ser Trp Glu Asn Val Thr
   325 330 335
   Val Asp Lys Val Leu Glu Phe Pro Leu Leu Lys Gly His Pro Asn Leu
   340 345 350
   Cys Val Gln Val Asn Ser Ser Glu Lys Leu Gln Leu Gln Glu Cys Leu
   355 360 365
   Trp Ala Asp Ser Leu Gly Pro Leu Lys Asp Asp Val Leu Leu Leu Glu
   370 375 380
   Thr Arg Gly Pro Gln Asp Asn Arg Ser Leu Cys Ala Leu Glu Pro Ser
   385                 390                 395                 400
   Gly Cys Thr Ser Leu Pro Ser Lys Ala Ser Thr Arg Ala Ala Arg Leu
                   405                 410                 415
   Gly Glu Tyr Leu Leu Gln Asp Leu Gln Ser Gly Gln Cys Leu Gln Leu
               420                 425                 430
   Trp Asp Asp Asp Leu Gly Ala Leu Trp Ala Cys Pro Met Asp Lys Tyr
           435                 440                 445
   Ile His Lys Arg Trp Ala Leu Val Trp Leu Ala Cys Leu Leu Phe Ala
       450                 455                 460
   Ala Ala Leu Ser Leu Ile Leu Leu Leu Lys Lys Asp His Ala Lys Gly
   465                 470                 475                 480
   Trp Leu Arg Leu Leu Lys Gln Asp Val Arg Ser Gly Ala Ala Ala Arg
                   485                 490                 495
   Gly Arg Ala Ala Leu Leu Leu Tyr Ser Ala Asp Asp Ser Gly Phe Glu
               500                 505                 510
   Arg Leu Val Gly Ala Leu Ala Ser Ala Leu Cys Gln Leu Pro Leu Arg
           515                 520                 525
   Val Ala Val Asp Leu Trp Ser Arg Arg Glu Leu Ser Ala Gln Gly Pro
       530                 535                 540
   Val Ala Trp Phe His Ala Gln Arg Arg Gln Thr Leu Gln Glu Gly Gly
   545                 550                 555                 560
   Val Val Val Leu Leu Phe Ser Pro Gly Ala Val Ala Leu Cys Ser Glu
                   565                 570                 575
   Trp Leu Gln Asp Gly Val Ser Gly Pro Gly Ala His Gly Pro His Asp
               580                 585                 590
   Ala Phe Arg Ala Ser Leu Ser Cys Val Leu Pro Asp Phe Leu Gln Gly
           595                 600                 605
   Arg Ala Pro Gly Ser Tyr Val Gly Ala Cys Phe Asp Arg Leu Leu His
       610                 615                 620
   Pro Asp Ala Val Pro Ala Leu Phe Arg Thr Val Pro Val Phe Thr Leu
   625                 630                 635                 640
   Pro Ser Gln Leu Pro Asp Phe Leu Gly Ala Leu Gln Gln Pro Arg Ala
                   645                 650                 655
   Pro Arg Ser Gly Arg Leu Gln Glu Arg Ala Glu Gln Val Ser Arg Ala
               660                 665                 670
   Leu Gln Pro Ala Leu Asp Ser Tyr Phe His Pro Pro Gly Thr Pro Ala
           675                 680                 685
   Pro Gly Arg Gly Val Gly Pro Gly Ala Gly Pro Gly Ala Gly Asp Gly
       690                 695                 700
   Thr
   705
<210> 12
   <211> 675
   <212> PRT
   <213> Artificial Sequence
<220>
   <223> Chimeric Zcytor14 protein
<400> 12
   Met Pro Val Pro Trp Phe Leu Leu Ser Leu Ala Leu Gly Arg Ser Pro
    1               5                  10                  15
   Val Val Leu Ser Leu Glu Arg Leu Val Gly Pro Gln Asp Ala Thr His
               20                  25                  30
   Cys Ser Pro Gly Leu Ser Cys Arg Leu Trp Asp Ser Asp Ile Leu Cys
           35                  40                  45
   Leu Pro Gly Asp Ile Val Pro Ala Pro Gly Pro Val Leu Ala Pro Thr
       50                  55                  60
   His Leu Gln Thr Glu Leu Val Leu Arg Cys Gln Lys Glu Thr Asp Cys
   65                  70                  75                  80
   Asp Leu Cys Leu Arg Val Ala Val His Leu Ala Val His Gly His Trp
                   85                  90                  95
   Glu Glu Pro Glu Asp Glu Glu Lys Phe Gly Gly Ala Ala Asp Ser Gly
               100                 105                 110
   Val Glu Glu Pro Arg Asn Ala Ser Leu Gln Ala Gln Val Val Leu Ser
           115                 120                 125
   Phe Gln Ala Tyr Pro Thr Ala Arg Cys Val Leu Leu Glu Val Gln Val
       130                 135                 140
   Pro Ala Ala Leu Val Gln Phe Gly Gln Ser Val Gly Ser Val Val Tyr
   145                 150                 155                 160
   Asp Cys Phe Glu Ala Ala Leu Gly Ser Glu Val Arg Ile Trp Ser Tyr
                   165                 170                 175
   Thr Gln Pro Arg Tyr Glu Lys Glu Leu Asn His Thr Gln Gln Leu Pro
               180                 185                 190
   Ala Leu Pro Trp Leu Asn Val Ser Ala Asp Gly Asp Asn Val His Leu
           195                 200                 205
   Val Leu Asn Val Ser Glu Glu Gln His Phe Gly Leu Ser Leu Tyr Trp
       210                 215                 220
   Asn Gln Val Gln Gly Pro Pro Lys Pro Arg Trp His Lys Asn Leu Thr
   225                 230                 235                 240
   Gly Pro Gln Ile Ile Thr Leu Asn His Thr Asp Leu Val Pro Cys Leu
                   245                 250                 255
   Cys Ile Gln Val Trp Pro Leu Glu Pro Asp Ser Val Arg Thr Asn Ile
               260                 265                 270
   Cys Pro Phe Arg Glu Asp Pro Arg Ala His Gln Asn Leu Trp Gln Ala
           275                 280                 285
   Ala Arg Leu Arg Leu Leu Thr Leu Gln Ser Trp Leu Leu Asp Ala Pro
       290                 295                 300
   Cys Ser Leu Pro Ala Glu Ala Ala Leu Cys Trp Arg Ala Pro Gly Gly
   305                 310                 315                 320
   Asp Pro Cys Gln Pro Leu Val Pro Pro Leu Ser Trp Glu Asn Val Thr
                   325                 330                 335
   Val Asp Val Asn Ser Ser Glu Lys Leu Gln Leu Gln Glu Cys Leu Trp
               340                 345                 350
   Ala Asp Ser Leu Gly Pro Leu Lys Asp Asp Val Leu Leu Leu Glu Thr
           355                 360                 365
   Arg Gly Pro Gln Asp Asn Arg Ser Leu Cys Ala Leu Glu Pro Ser Gly
       370                 375                 380
   Cys Thr Ser Leu Pro Ser Lys Ala Ser Thr Arg Ala Ala Arg Leu Gly
   385                 390                 395                 400
   Glu Tyr Leu Leu Gln Asp Leu Gln Ser Gly Gln Cys Leu Gln Leu Trp
                   405                 410                 415
   Asp Asp Asp Leu Gly Ala Leu Trp Ala Cys Pro Met Asp Lys Tyr Ile
               420                 425                 430
   His Lys Arg Trp Ala Leu Val Trp Leu Ala Cys Leu Leu Phe Ala Ala
           435                 440                 445
   Ala Leu Ser Leu Ile Leu Leu Leu Lys Lys Asp His Ala Lys Ala Ala
       450                 455                 460
   Ala Arg Gly Arg Ala Ala Leu Leu Leu Tyr Ser Ala Asp Asp Ser Gly
   465                 470                 475                 480
   Phe Glu Arg Leu Val Gly Ala Leu Ala Ser Ala Leu Cys Gln Leu Pro
                   485                 490                 495
   Leu Arg Val Ala Val Asp Leu Trp Ser Arg Arg Glu Leu Ser Ala Gln
               500                 505                 510
   Gly Pro Val Ala Trp Phe His Ala Gln Arg Arg Gln Thr Leu Gln Glu
           515                 520                 525
   Gly Gly Val Val Val Leu Leu Phe Ser Pro Gly Ala Val Ala Leu Cys
           530                     535                     540
   Ser Glu Trp Leu Gln Asp Gly Val Ser Gly Pro Gly Ala His Gly Pro
   545                 550                 555                 560
   His Asp Ala Phe Arg Ala Ser Leu Ser Cys Val Leu Pro Asp Phe Leu
                   565                 570                 575
   Gln Gly Arg Ala Pro Gly Ser Tyr Val Gly Ala Cys Phe Asp Arg Leu
               580                 585                 590
   Leu His Pro Asp Ala Val Pro Ala Leu Phe Arg Thr Val Pro Val Phe
           595                 600                 605
   Thr Leu Pro Ser Gln Leu Pro Asp Phe Leu Gly Ala Leu Gln Gln Pro
       610                 615                 620
   Arg Ala Pro Arg Ser Gly Arg Leu Gln Glu Arg Ala Glu Gln Val Ser
   625                 630                 635                 640
   Arg Ala Leu Gln Pro Ala Leu Asp Ser Tyr Phe His Pro Pro Gly Thr
                   645                 650                 655
   Pro Ala Pro Gly Arg Gly Val Gly Pro Gly Ala Gly Pro Gly Ala Gly
               660                 665                 670
   Asp Gly Thr
           675
<210> 13
   <211> 1874
   <212> DNA
   <213> Homo sapiens
<400> 13
   gaattccggc aggcacaaac tcatccatcc ccagttgatt ggaagaaaca acgatgactc 60
   ctgggaagac ctcattggtg tcactgctac tgctgctgag cctggaggcc atagtgaagg 120
   caggaatcac aatcccacga aatccaggat gcccaaattc tgaggacaag aacttccccc 180
   ggactgtgat ggtcaacctg aacatccata accggaatac caataccaat cccaaaaggt 240
   cctcagatta ctacaaccga tccacctcac cttggaatct ccaccgcaat gaggaccctg 300
   agagatatcc ctctgtgatc tgggaggcaa agtgccgcca cttgggctgc atcaacgctg 360
   atgggaacgt ggactaccac atgaactctg tccccatcca gcaagagatc ctggtcctgc 420
   gcagggagcc tccacactgc cccaactcct tccggctgga gaagatactg gtgtccgtgg 480
   gctgcacctg tgtcaccccg attgtccacc atgtggccta agagctctgg ggagcccaca 540
   ctccccaaag cagttagact atggagagcc gacccagccc ctcaggaacc ctcatccttc 600
   aaagacagcc tcatttcgga ctaaactcat tagagttctt aaggcagttt gtccaattaa 660
   agcttcagag gtaacacttg gccaagatat gagatctgaa ttacctttcc ctctttccaa 720
   gaaggaaggt ttgactgagt accaatttgc ttcttgttta cttttttaag ggctttaagt 780
   tatttatgta tttaatatgc cctgagataa ctttggggta taagattcca ttttaatgaa 840
   ttacctactt tattttgttt gtctttttaa agaagataag attctgggct tgggaatttt 900
   attatttaaa aggtaaaacc tgtatttatt tgagctattt aaggatctat ttatgtttaa 960
   gtatttagaa aaaggtgaaa aagcactatt atcagttctg cctaggtaaa tgtaagatag 1020
   aattaaatgg cagtgcaaaa tttctgagtc tttacaacat acggatatag tatttcctcc 1080
   tctttgtttt taaaagttat aacatggctg aaaagaaaga ttaaacctac tttcatatgt 1140
   attaatttaa attttgcaat ttgttgaggt tttacaagag atacagcaag tctaactctc 1200
   tgttccatta aacccttata ataaaatcct tctgtaataa taaagtttca aaagaaaatg 1260
   tttatttgtt ctcattaaat gtattttagc aaactcagct cttccctatt gggaagagtt 1320
   atgcaaattc tcctataagc aaaacaaagc atgtctttga gtaacaatga cctggaaata 1380
   cccaaaattc caagttctcg atttcacatg ccttcaagac tgaacaccga ctaaggtttt 1440
   catactatta gccaatgctg tagacagaag cattttgata ggaatagagc aaataagata 1500
   atggccctga ggaatggcat gtcattatta aagatcatat ggggaaaatg aaaccctccc 1560
   caaaatacaa gaagttctgg gaggagacat tgtcttcaga ctacaatgtc cagtttctcc 1620
   cctagactca ggcttccttt ggagattaag gcccctcaga gatcaacaga ccaacatttt 1680
   tctcttcctc aagcaacact cctagggcct ggcttctgtc tgatcaaggc accacacaac 1740
   ccagaaagga gctgatgggg cagaatgaac tttaagtatg agaaaagttc agcccaagta 1800
   aaataaaaac tcaatcacat tcaattccag agtagtttca agtttcacat cgtaaccatt 1860
   ttcgcccgga attc                                                  1874
<210> 14
   <211> 155
   <212> PRT
   <213> Homo sapiens
<400> 14
   Met Thr Pro Gly Lys Thr Ser Leu Val Ser Leu Leu Leu Leu Leu Ser
    1               5                  10                  15
   Leu Glu Ala Ile Val Lys Ala Gly Ile Thr Ile Pro Arg Asn Pro Gly
               20                  25                  30
   Cys Pro Asn Ser Glu Asp Lys Asn Phe Pro Arg Thr Val Met Val Asn
           35                  40                  45
   Leu Asn Ile His Asn Arg Asn Thr Asn Thr Asn Pro Lys Arg Ser Ser
       50                  55                  60
   Asp Tyr Tyr Asn Arg Ser Thr Ser Pro Trp Asn Leu His Arg Asn Glu
   65                  70                  75                  80
   Asp Pro Glu Arg Tyr Pro Ser Val Ile Trp Glu Ala Lys Cys Arg His
                   85                  90                  95
   Leu Gly Cys Ile Asn Ala Asp Gly Asn Val Asp Tyr His Met Asn Ser
               100                 105                 110
   Val Pro Ile Gln Gln Glu Ile Leu Val Leu Arg Arg Glu Pro Pro His
           115                 120                 125
   Cys Pro Asn Ser Phe Arg Leu Glu Lys Ile Leu Val Ser Val Gly Cys
       130                 135                 140
   Thr Cys Val Thr Pro Ile Val His His Val Ala
   145                 150                 155
<210> 15
   <211> 923
   <212> DNA
   <213> Homo sapiens
<400> 15
   ggcttcagtt actagctagg ctactgagtt tagttctcag tttggcacct tgataccttt 60
   aggtgtgagt gttcccattt ccaggtgagg aactgaggtg caaagagaag ccctgatccc 120
   ataaaaggac aggaatgctg agttccgcca gaccatgcat ctcttgctag taggtgaggc 180
   gagtctctaa ctgattgcag cgtcttctat tttccaggtc aagtacttgc tgctgtcgat 240
   attggggctt gcctttctga gtgaggcggc agctcggaaa atccccaaag taggacatac 300
   ttttttccaa aagcctgaga gttgcccgcc tgtgccagga ggtagtatga agcttgacat 360
   tggcatcatc aatgaaaacc agcgcgtttc catgtcacgt aacatcgaga gccgctccac 420
   ctccccctgg aattacactg tcacttggga ccccaaccgg tacccctcgg aagttgtaca 480
   ggcccagtgt aggaacttgg gctgcatcaa tgctcaagga aaggaagaca tctccatgaa 540
   ttccgttccc atccagcaag agaccctggt cgtccggagg aagcaccaag gctgctctgt 600
   ttctttccag ttggagaagg tgctggtgac tgttggctgc acctgcgtca cccctgtcat 660
   ccaccatgtg cagtaagagg tgcatatcca ctcagctgaa gaagctgtag aaatgccact 720
   ccttacccag tgctctgcaa caagtcctgt ctgaccccca attccctcca cttcacagga 780
   ctcttaataa gacctgcacg gatggaaaca taaaatattc acaatgtatg tgtgtatgta 840
   ctacacttta tatttgatat ctaaaatgtt aggagaaaaa ttaatatatt cagtgctaat 900
   ataataaagt attaataatg tta                                         923
<210> 16
   <211> 153
   <212> PRT
   <213> Homo sapiens
<400> 16
   Met Val Lys Tyr Leu Leu Leu Ser Ile Leu Gly Leu Ala Phe Leu Ser
    1               5                  10                  15
   Glu Ala Ala Ala Arg Lys Ile Pro Lys Val Gly His Thr Phe Phe Gln
                   20                  25                  30
   Lys Pro Glu Ser Cys Pro Pro Val Pro Gly Gly Ser Met Lys Leu Asp
               35                  40                  45
   Ile Gly Ile Ile Asn Glu Asn Gln Arg Val Ser Met Ser Arg Asn Ile
       50                  55                  60
   Glu Ser Arg Ser Thr Ser Pro Trp Asn Tyr Thr Val Thr Trp Asp Pro
   65                  70                  75                  80
   Asn Arg Tyr Pro Ser Glu Val Val Gln Ala Gln Cys Arg Asn Leu Gly
                   85                  90                  95
   Cys Ile Asn Ala Gln Gly Lys Glu Asp Ile Ser Met Asn Ser Val Pro
               100                 105                 110
   Ile Gln Gln Glu Thr Leu Val Val Arg Arg Lys His Gln Gly Cys Ser
           115                 120                 125
   Val Ser Phe Gln Leu Glu Lys Val Leu Val Thr Val Gly Cys Thr Cys
       130                 135                 140
   Val Thr Pro Val Ile His His Val Gln
   145                 150
<210> 17
   <211> 1172
   <212> DNA
   <213> Mus musculus
<400> 17
   gatccacctc acacgaggca caagtgcacc cagcaccagc tgatcaggac gcgcaaacat 60
   gagtccaggg agagcttcat ctgtgtctct gatgctgttg ctgctgctga gcctggcggc 120
   tacagtgaag gcagcagcga tcatccctca aagctcagcg tgtccaaaca ctgaggccaa 180
   ggacttcctc cagaatgtga aggtcaacct caaagtcttt aactcccttg gcgcaaaagt 240
   gagctccaga aggccctcag actacctcaa ccgttccacg tcaccctgga ctctccaccg 300
   caatgaagac cctgatagat atccctctgt gatctgggaa gctcagtgcc gccaccagcg 360
   ctgtgtcaat gcggagggaa agctggacca ccacatgaat tctgttctca tccagcaaga 420
   gatcctggtc ctgaagaggg agcctgagag ctgccccttc actttcaggg tcgagaagat 480
   gctggtgggt gtgggctgca cctgcgtggc ctcgattgtc cgccaggcag cctaaacaga 540
   gacccgcggc tgacccctaa gaaaccccca cgtttctcag caaacttact tgcattttta 600
   aaacagttcg tgctattgat tttcagcaag gaatgtggat tcagaggcag attcagaatt 660
   gtctgccctc cacaatgaaa agaaggtgta aaggggtccc aaactgcttc gtgtttgttt 720
   ttctgtggac tttaaattat ttgtgtattt acaatatccc aagatagctt tgaagcgtaa 780
   cttattttaa tgaagtatct acattattat tatgtttctt tctgaagaag acaaaattca 840
   agactcagaa attttattat ttaaaaggta aagcctatat ttatatgagc tatttatgaa 900
   tctatttatt tttcttcagt atttgaagta ttaagaacat gattttcaga tctacctagg 960
   gaagtcctaa gtaagattaa atattaatgg aaatttcagc tttactattt gtttatttaa 1020
   ggttctctcc tctgaatggg gtgaaaacca aacttagttt tatgtttaat aactttttaa 1080
   attattgaag attcaaaaaa ttggataatt tagctcccta ctctgtttta aaaaaaaatt 1140
   gtaacaatat cactgtaata ataaagtttt gg                               1172
<210> 18
   <211> 158
   <212> PRT
   <213> Mus musculus
<400> 18
   Met Ser Pro Gly Arg Ala Ser Ser Val Ser Leu Met Leu Leu Leu Leu
   1               5                   10                  15
   Leu Ser Leu Ala Ala Thr Val Lys Ala Ala Ala Ile Ile Pro Gln Ser
               20                  25                  30
   Ser Ala Cys Pro Asn Thr Glu Ala Lys Asp Phe Leu Gln Asn Val Lys
           35                  40                  45
   Val Asn Leu Lys Val Phe Asn Ser Leu Gly Ala Lys Val Ser Ser Arg
       50                  55                  60
   Arg Pro Ser Asp Tyr Leu Asn Arg Ser Thr Ser Pro Trp Thr Leu His
   65                  70                  75                  80
   Arg Asn Glu Asp Pro Asp Arg Tyr Pro Ser Val Ile Trp Glu Ala Gln
                   85                  90                  95
   Cys Arg His Gln Arg Cys Val Asn Ala Glu Gly Lys Leu Asp His His
               100                 105                 110
   Met Asn Ser Val Leu Ile Gln Gln Glu Ile Leu Val Leu Lys Arg Glu
           115                 120                 125
   Pro Glu Ser Cys Pro Phe Thr Phe Arg Val Glu Lys Met Leu Val Gly
       130                 135                 140
   Val Gly Cys Thr Cys Val Ala Ser Ile Val Arg Gln Ala Ala
   145                 150                 155
<210> 19
   <211> 462
   <212> DNA
   <213> Mus musculus
<400> 19
   atggtcaagt ctttgctact gttgatgttg ggacttgcca ttctgaggga ggtagcagct 60
   cggaagaacc ccaaagcagg ggttcctgcc ttgcagaagg ctgggaactg tcctcccctg 120
   gaggataaca ctgtgagagt tgacattcga atcttcaacc aaaaccaggg catttctgtc 180
   ccacgtgaat tccagaaccg ctccagttcc ccatgggatt acaacatcac tcgagacccc 240
   caccggttcc cctcagagat cgctgaggcc cagtgcagac actcaggctg catcaatgcc 300
   cagggtcagg aagacagcac catgaactcc gtcgccattc agcaagaaat cctggtcctt 360
   cggagggagc cccagggctg ttctaattcc ttcaggttgg agaagatgct cctaaaagtt 420
   ggctgcacct gtgtcaagcc cattgtccac caagcggcct ga                    462
<210> 20
   <211> 153
   <212> PRT
   <213> Mus musculus
<400> 20
   Met Val Lys Ser Leu Leu Leu Leu Met Leu Gly Leu Ala Ile Leu Arg
    1               5                  10                  15
   Glu Val Ala Ala Arg Lys Asn Pro Lys Ala Gly Val Pro Ala Leu Gln
               20                  25                  30
   Lys Ala Gly Asn Cys Pro Pro Leu Glu Asp Asn Thr Val Arg Val Asp
           35                  40                  45
   Ile Arg Ile Phe Asn Gln Asn Gln Gly Ile Ser Val Pro Arg Glu Phe
       50                  55                  60
   Gln Asn Arg Ser Ser Ser Pro Trp Asp Tyr Asn Ile Thr Arg Asp Pro
   65                  70                  75                  80
   His Arg Phe Pro Ser Glu Ile Ala Glu Ala Gln Cys Arg His Ser Gly
                   85                  90                  95
   Cys Ile Asn Ala Gln Gly Gln Glu Asp Ser Thr Met Asn Ser Val Ala
               100                 105                 110
   Ile Gln Gln Glu Ile Leu Val Leu Arg Arg Glu Pro Gln Gly Cys Ser
           115                 120                 125
   Asn Ser Phe Arg Leu Glu Lys Met Leu Leu Lys Val Gly Cys Thr Cys
       130                 135                 140
   Val Lys Pro Ile Val His Gln Ala Ala
   145                 150
<210> 21
   <211> 320
   <212> PRT
   <213> Homo sapien
<400> 21
   Met Gly Ala Ala Arg Ser Pro Pro Ser Ala Val Pro Gly Pro Leu Leu
   1 5 10 15
   Gly Leu Leu Leu Leu Leu Leu Gly Val Leu Ala Pro Gly Gly Ala Ser
   20 25 30
   Leu Arg Leu Leu Asp His Arg Ala Leu Val Cys Ser Gln Pro Gly Leu
   35 40 45
   Asn Cys Thr Val Lys Asn Ser Thr Cys Leu Asp Asp Ser Trp Ile His
   50 55 60
   Pro Arg Asn Leu Thr Pro Ser Ser Pro Lys Asp Leu Gln Ile Gln Leu
   65 70 75 80
   His Phe Ala His Thr Gln Gln Gly Asp Leu Phe Pro Val Ala His Ile
   85 90 95
   Glu Trp Thr Leu Gln Thr Asp Ala Ser Ile Leu Tyr Leu Glu Gly Ala
   100 105 110
   Glu Leu Ser Val Leu Gln Leu Asn Thr Asn Glu Arg Leu Cys Val Arg
   115 120 125
   Phe Glu Phe Leu Ser Lys Leu Arg His His His Arg Arg Trp Arg Phe
   130 135 140
   Thr Phe Ser His Phe Val Val Asp Pro Asp Gln Glu Tyr Glu Val Thr
   145 150 155 160
   Val His His Leu Pro Lys Pro Ile Pro Asp Gly Asp Pro Asn His Gln
   165 170 175
   Ser Lys Asn Phe Leu Val Pro Asp Cys Glu His Ala Arg Met Lys Val
   180 185 190
   Thr Thr Pro Cys Met Ser Ser Gly Ser Leu Trp Asp Pro Asn Ile Thr
   195 200 205
   Val Glu Thr Leu Glu Ala His Gln Leu Arg Val Ser Phe Thr Leu Trp
   210 215 220
   Asn Glu Ser Thr His Tyr Gln Ile Leu Leu Thr Ser Phe Pro His Met
   225 230 235 240
   Glu Asn His Ser Cys Phe Glu His Met His His Ile Pro Ala Pro Arg
   245 250 255
   Pro Glu Glu Phe His Gln Arg Ser Asn Val Thr Leu Thr Leu Arg Asn
   260 265 270
   Leu Lys Gly Cys Cys Arg His Gln Val Gln Ile Gln Pro Phe Phe Ser
   275 280 285
   Ser Cys Leu Asn Asp Cys Leu Arg His Ser Ala Thr Val Ser Cys Pro
   290 295 300
   Glu Met Pro Asp Thr Pro Glu Pro Ile Pro Asp Tyr Met Pro Leu Trp
   305 310 315 320
<210> 22
   <211> 221
   <212> PRT
   <213> Homo sapien
<400> 22
   Lys Pro Arg Trp His Lys Asn Leu Thr Gly Pro Gln Ile Ile Thr Leu
   1 5 10 15
   Asn His Thr Asp Leu Val Pro Cys Leu Cys Ile Gln Val Trp Pro Leu
   20 25 30
   Glu Pro Asp Ser Val Arg Thr Asn Ile Cys Pro Phe Arg Glu Asp Pro
   35 40 45
   Arg Ala His Gln Asn Leu Trp Gln Ala Ala Arg Leu Arg Leu Leu Thr
   50 55 60
   Leu Gln Ser Trp Leu Leu Asp Ala Pro Cys Ser Leu Pro Ala Glu Ala
   65                  70                       75                  80
   Ala Leu Cys Trp Arg Ala Pro Gly Gly Asp Pro Cys Gln Pro Leu Val
                   85                  90                  95
   Pro Pro Leu Ser Trp Glu Asn Val Thr Val Asp Lys Val Leu Glu Phe
               100                 105                 110
   Pro Leu Leu Lys Gly His Pro Asn Leu Cys Val Gln Val Asn Ser Ser
           115                 120                 125
   Glu Lys Leu Gln Leu Gln Glu Cys Leu Trp Ala Asp Ser Leu Gly Pro
       130                 135                 140
   Leu Lys Asp Asp Val Leu Leu Leu Glu Thr Arg Gly Pro Gln Asp Asn
   145                 150                 155                 160
   Arg Ser Leu Cys Ala Leu Glu Pro Ser Gly Cys Thr Ser Leu Pro Ser
                   165                 170                 175
   Lys Ala Ser Thr Arg Ala Ala Arg Leu Gly Glu Tyr Leu Leu Gln Asp
               180                 185                 190
   Leu Gln Ser Gly Gln Cys Leu Gln Leu Trp Asp Asp Asp Leu Gly Ala
           195                 200                 205
   Leu Trp Ala Cys Pro Met Asp Lys Tyr Ile His Lys Arg
       210                 215                 220
<210> 23
   <211> 2180
   <212> DNA
   <213> Homo sapien
<400> 23
   aactacccag cacagccccc tccgccccct ctggaggctg aagagggatt ccagcccctg 60
   ccacccacag acacgggctg actggggtgt ctgcccccct tggggggggg cagcacaggg 120
   cctcaggcct gggtgccacc tggcacctag aagatgcctg tgccctggtt cttgctgtcc 180
   ttggcactgg gccgaagccc agtggtcctt tctctggaga ggcttgtggg gcctcaggac 240
   gctacccact gctctccggg cctctcctgc cgcctctggg acagtgacat actctgcctg 300
   cctggggaca tcgtgcctgc tccgggcccc gtgctggcgc ctacgcacct gcagacagag 360
   ctggtgctga ggtgccagaa ggagaccgac tgtgacctct gtctgcgtgt ggctgtccac 420
   ttggccgtgc atgcctctct ccaggcccaa gtcgtgctct ccttccaggc ctaccctact 480
   gcccgctgcg tcctgctgga ggtgcaagtg cctgctgccc ttgtgcagtt tggtcagtct 540
   gtgggctctg tggtatatga ctgcttcgag gctgccctag ggagtgaggt acgaatctgg 600
   tcctatactc agcccaggta cgagaaggaa ctcaaccaca cacagcagct gcctgccctg 660
   ccctggctca acgtgtcagc agatggtgac aacgtgcatc tggttctgaa tgtctctgag 720
   gagcagcact tcggcctctc cctgtactgg aatcaggtcc agggcccccc aaaaccccgg 780
   tggcacaaaa acctgactgg accgcagatc attaccttga accacacaga cctggttccc 840
   tgcctctgta ttcaggtgtg gcctctggaa cctgactccg ttaggacgaa catctgcccc 900
   ttcagggagg acccccgcgc acaccagaac ctctggcaag ccgcccgact gcgactgctg 960
   accctgcaga gctggctgct ggacgcaccg tgctcgctgc ccgcagaagc ggcactgtgc 1020
   tggcgggctc cgggtgggga cccctgccag ccactggtcc caccgctttc ctgggagaac 1080
   gtcactgtgg acaaggttct cgagttccca ttgctgaaag gccaccctaa cctctgtgtt 1140
   caggtgaaca gctcggagaa gctgcagctg caggagtgct tgtgggctga ctccctgggg 1200
   cctctcaaag acgatgtgct actgttggag acacgaggcc cccaggacaa cagatccctc 1260
   tgtgccttgg aacccagtgg ctgtacttca ctacccagca aagcctccac gagggcagct 1320
   cgccttggag agtacttact acaagacctg cagtcaggcc agtgtctgca gctatgggac 1380
   gatgacttgg gagcgctatg ggcctgcccc atggacaaat acatccacaa gcgctgggcc 1440
   ctcgtgtggc tggcctgcct actctttgcc gctgcgcttt ccctcatcct ccttctcaaa 1500
   aaggatcacg cgaaagcggc cgccaggggc cgcgcggctc tgctcctcta ctcagccgat 1560
   gactcgggtt tcgagcgcct ggtgggcgcc ctggcgtcgg ccctgtgcca gctgccgctg 1620
   cgcgtggccg tagacctgtg gagccgtcgt gaactgagcg cgcaggggcc cgtggcttgg 1680
   tttcacgcgc agcggcgcca gaccctgcag gagggcggcg tggtggtctt gctcttctct 1740
   cccggtgcgg tggcgctgtg cagcgagtgg ctacaggatg gggtgtccgg gcccggggcg 1800
   cacggcccgc acgacgcctt ccgcgcctcg ctcagctgcg tgctgcccga cttcttgcag 1860
   ggccgggcgc ccggcagcta cgtgggggcc tgcttcgaca ggctgctcca cccggacgcc 1920
   gtacccgccc ttttccgcac cgtgcccgtc ttcacactgc cctcccaact gccagacttc 1980
   ctgggggccc tgcagcagcc tcgcgccccg cgttccgggc ggctccaaga gagagcggag 2040
   caagtgtccc gggcccttca gccagccctg gatagctact tccatccccc ggggactccc 2100
   gcgccgggac gcggggtggg accaggggcg ggacctgggg cgggggacgg gacttaaata 2160
   aaggcagacg ctgtttttct 2180
<210> 24
   <211> 667
   <212> PRT
   <213> Homo sapien
<400> 24
<210> 25
   <211> 2269
   <212> DNA
   <213> Mus musculus

<400> 25
   aaatcgaaag cactccagct gaaactgggc ctggagtcca ggctcactgg agtggggaag 60
   catggctgga gaggaattct agcccttgct ctctcccagg gacacggggc tgattgtcag 120
   caggggcgag gggtctgccc ccccttgggg gggcaggacg gggcctcagg cctgggtgct 180
   gtccggcacc tggaagatgc ctgtgtcctg gttcctgctg tccttggcac tgggccgaaa 240
   ccctgtggtc gtctctctgg agagactgat ggagcctcag gacactgcac gctgctctct 300
   aggcctctcc tgccacctct gggatggtga cgtgctctgc ctgcctggaa gcctccagtc 360
   tgccccaggc cctgtgctag tgcctacccg cctgcagacg gagctggtgc tgaggtgtcc 420
   acagaagaca gattgcgccc tctgtgtccg tgtggtggtc cacttggccg tgcatgggca 480
   ctgggcagag cctgaagaag ctggaaagtc tgattcagaa ctccaggagt ctaggaacgc 540
   ctctctccag gcccaggtgg tgctctcctt ccaggcctac cccatcgccc gctgtgccct 600
   gctggaggtc caggtgcccg ctgacctggt gcagcctggt cagtccgtgg gttctgcggt 660
   atttgactgt ttcgaggcta gtcttggggc tgaggtacag atctggtcct acacgaagcc 720
   caggtaccag aaagagctca acctcacaca gcagctgcct gtcctgccct ggctcaatgt 780
   gtctacagat ggtgacaatg tccttctgac actggatgtc tctgaggagc aggactttag 840
   cttcttactg tacctgcgtc cagtcccgga tgctctcaaa tccttgtggt acaaaaacct 900
   gactggacct cagaacatta ctttaaacca cacagacctg gttccctgcc tctgcattca 960
   ggtgtggtcg ctagagccag actctgagag ggtcgaattc tgccccttcc gggaagatcc 1020
   cggtgcacac aggaacctct ggcacatagc caggctgcgg gtactgtccc caggggtatg 1080
   gcagctagat gcgccttgct gtctgccggg caaggtaaca ctgtgctggc aggcaccaga 1140
   ccagagtccc tgccagccac ttgtgccacc agtgccccag aagaacgcca ctgtgaatga 1200
   gccacaagat ttccagttgg tggcaggcca ccccaacctc tgtgtccagg tgagcacctg 1260
   ggagaaggtt cagctgcaag cgtgcttgtg ggctgactcc ttggggccct tcaaggatga 1320
   tatgctgtta gtggagatga aaaccggcct caacaacaca tcagtctgtg ccttggaacc 1380
   cagtggctgt acaccactgc ccagcatggc ctccacgaga gctgctcgcc tgggagagga 1440
   gttgctgcaa gacttccgat cacaccagtg tatgcagctg tggaacgatg acaacatggg 1500
   atcgctatgg gcctgcccca tggacaagta catccacagg cgctgggtcc tagtatggct 1560
   ggcctgccta ctcttggctg cggcgctttt cttcttcctc cttctaaaaa aggaccgcag 1620
   gaaagcggcc cgtggctccc gcacggcctt gctcctccac tccgccgacg gagcgggcta 1680
   cgagcgtctg gtgggagcac tggcgtccgc gttgagccag atgccactgc gcgtggccgt 1740
   ggacctgtgg agccgccgcg agctgagcgc gcacggagcc ctagcctggt tccaccacca 1800
   gcgacgccgt atcctgcagg agggtggcgt ggtaatcctt ctcttctcgc ccgcggccgt 1860
   ggcgcagtgt cagcagtggc tgcagctcca gacagtggag cccgggccgc atgacgccct 1920
   cgccgcctgg ctcagctgcg tgctacccga tttcctgcaa ggccgggcga ccggccgcta 1980
   cgtcggggtc tacttcgacg ggctgctgca cccagactct gtgccctccc cgttccgcgt 2040
   cgccccgctc ttctccctgc cctcgcagct gccggctttc ctggatgcac tgcagggagg 2100
   ctgctccact tccgcggggc gacccgcgga ccgggtggaa cgagtgaccc aggcgctgcg 2160
   gtccgccctg gacagctgta cttctagctc ggaagcccca ggctgctgcg aggaatggga 2220
   cctgggaccc tgcactacac tagaataaaa gccgatacag tattcctaa 2269
<210> 26
   <211> 683
   <212> PRT
   <213> Mus musculus
<400> 26
   Met Pro Val Ser Trp Phe Leu Leu Ser Leu Ala Leu Gly Arg Asn Pro
    1               5                  10                  15
   Val Val Val Ser Leu Glu Arg Leu Met Glu Pro Gln Asp Thr Ala Arg
               20                  25                  30
   Cys Ser Leu Gly Leu Ser Cys His Leu Trp Asp Gly Asp Val Leu Cys
           35                  40                  45
   Leu Pro Gly Ser Leu Gln Ser Ala Pro Gly Pro Val Leu Val Pro Thr
       50                  55                  60
   Arg Leu Gln Thr Glu Leu Val Leu Arg Cys Pro Gln Lys Thr Asp Cys
   65                  70                  75                  80
   Ala Leu Cys Val Arg Val Val Val His Leu Ala Val His Gly His Trp
                   85                  90                  95
   Ala Glu Pro Glu Glu Ala Gly Lys Ser Asp Ser Glu Leu Gln Glu Ser
               100                 105                 110
   Arg Asn Ala Ser Leu Gln Ala Gln Val Val Leu Ser Phe Gln Ala Tyr
           115                 120                 125
   Pro Ile Ala Arg Cys Ala Leu Leu Glu Val Gln Val Pro Ala Asp Leu
       130                 135                 140
   Val Gln Pro Gly Gln Ser Val Gly Ser Ala Val Phe Asp Cys Phe Glu
   145                 150                 155                 160
   Ala Ser Leu Gly Ala Glu Val Gln Ile Trp Ser Tyr Thr Lys Pro Arg
                   165                 170                 175
   Tyr Gln Lys Glu Leu Asn Leu Thr Gln Gln Leu Pro Val Leu Pro Trp
               180                 185                 190
   Leu Asn Val Ser Thr Asp Gly Asp Asn Val Leu Leu Thr Leu Asp Val
           195                 200                 205
   Ser Glu Glu Gln Asp Phe Ser Phe Leu Leu Tyr Leu Arg Pro Val Pro
       210                 215                 220
   Asp Ala Leu Lys Ser Leu Trp Tyr Lys Asn Leu Thr Gly Pro Gln Asn
   225                 230                 235                 240
   Ile Thr Leu Asn His Thr Asp Leu Val Pro Cys Leu Cys Ile Gln Val
                   245                 250                 255
   Trp Ser Leu Glu Pro Asp Ser Glu Arg Val Glu Phe Cys Pro Phe Arg
               260                 265                 270
   Glu Asp Pro Gly Ala His Arg Asn Leu Trp His Ile Ala Arg Leu Arg
           275                 280                 285
   Val Leu Ser Pro Gly Val Trp Gln Leu Asp Ala Pro Cys Cys Leu Pro
       290                 295                 300
   Gly Lys Val Thr Leu Cys Trp Gln Ala Pro Asp Gln Ser Pro Cys Gln
   305                 310                 315                 320
   Pro Leu Val Pro Pro Val Pro Gln Lys Asn Ala Thr Val Asn Glu Pro
                   325                 330                 335
   Gln Asp Phe Gln Leu Val Ala Gly His Pro Asn Leu Cys Val Gln Val
               340                 345                 350
   Ser Thr Trp Glu Lys Val Gln Leu Gln Ala Cys Leu Trp Ala Asp Ser
           355                 360                 365
   Leu Gly Pro Phe Lys Asp Asp Met Leu Leu Val Glu Met Lys Thr Gly
       370                 375                 380
   Leu Asn Asn Thr Ser Val Cys Ala Leu Glu Pro Ser Gly Cys Thr Pro
   385                 390                 395                 400
   Leu Pro Ser Met Ala Ser Thr Arg Ala Ala Arg Leu Gly Glu Glu Leu
                   405                 410                 415
   Leu Gln Asp Phe Arg Ser His Gln Cys Met Gln Leu Trp Asn Asp Asp
               420                 425                 430
   Asn Met Gly Ser Leu Trp Ala Cys Pro Met Asp Lys Tyr Ile His Arg
           435                 440                 445
   Arg Trp Val Leu Val Trp Leu Ala Cys Leu Leu Leu Ala Ala Ala Leu
       450                 455                 460
   Phe Phe Phe Leu Leu Leu Lys Lys Asp Arg Arg Lys Ala Ala Arg Gly
   465                 470                 475                 480
   Ser Arg Thr Ala Leu Leu Leu His Ser Ala Asp Gly Ala Gly Tyr Glu
                   485                 490                 495
   Arg Leu Val Gly Ala Leu Ala Ser Ala Leu Ser Gln Met Pro Leu Arg
               500                 505                 510
   Val Ala Val Asp Leu Trp Ser Arg Arg Glu Leu Ser Ala His Gly Ala
           515                 520                 525
   Leu Ala Trp Phe His His Gln Arg Arg Arg Ile Leu Gln Glu Gly Gly
       530                 535                 540
   Val Val Ile Leu Leu Phe Ser Pro Ala Ala Val Ala Gln Cys Gln Gln
   545                 550                 555                 560
   Trp Leu Gln Leu Gln Thr Val Glu Pro Gly Pro His Asp Ala Leu Ala
                   565                 570                 575
   Ala Trp Leu Ser Cys Val Leu Pro Asp Phe Leu Gln Gly Arg Ala Thr
               580                 585                 590
   Gly Arg Tyr Val Gly Val Tyr Phe Asp Gly Leu Leu His Pro Asp Ser
           595                 600                 605
   Val Pro Ser Pro Phe Arg Val Ala Pro Leu Phe Ser Leu Pro Ser Gln
       610                 615                 620
   Leu Pro Ala Phe Leu Asp Ala Leu Gln Gly Gly Cys Ser Thr Ser Ala
   625                 630                 635                 640
   Gly Arg Pro Ala Asp Arg Val Glu Arg Val Thr Gln Ala Leu Arg Ser
                   645                 650                 655
   Ala Leu Asp Ser Cys Thr Ser Ser Ser Glu Ala Pro Gly Cys Cys Glu
               660                 665                 670
   Glu Trp Asp Leu Gly Pro Cys Thr Thr Leu Glu
           675                 680
<210> 27
   <211> 449
   <212> PRT
   <213> Mus musculus
<400> 27
   Met Pro Val Ser Trp Phe Leu Leu Ser Leu Ala Leu Gly Arg Asn Pro
   1               5                   10                  15
   Val Val Val Ser Leu Glu Arg Leu Met Glu Pro Gln Asp Thr Ala Arg
               20                  25                  30
   Cys Ser Leu Gly Leu Ser Cys His Leu Trp Asp Gly Asp Val Leu Cys
           35                  40                  45
   Leu Pro Gly Ser Leu Gln Ser Ala Pro Gly Pro Val Leu Val Pro Thr
       50                  55                  60
   Arg Leu Gln Thr Glu Leu Val Leu Arg Cys Pro Gln Lys Thr Asp Cys
   65                  70                  75                  80
   Ala Leu Cys Val Arg Val Val Val His Leu Ala Val His Gly His Trp
                   85                  90                  95
   Ala Glu Pro Glu Glu Ala Gly Lys Ser Asp Ser Glu Leu Gln Glu Ser
               100                 105                 110
   Arg Asn Ala Ser Leu Gln Ala Gln Val Val Leu Ser Phe Gln Ala Tyr
           115                 120                 125
   Pro Ile Ala Arg Cys Ala Leu Leu Glu Val Gln Val Pro Ala Asp Leu
       130                 135                 140
   Val Gln Pro Gly Gln Ser Val Gly Ser Ala Val Phe Asp Cys Phe Glu
   145                 150                 155                 160
   Ala Ser Leu Gly Ala Glu Val Gln Ile Trp Ser Tyr Thr Lys Pro Arg
                   165                 170                 175
   Tyr Gln Lys Glu Leu Asn Leu Thr Gln Gln Leu Pro Val Leu Pro Trp
               180                 185                 190
   Leu Asn Val Ser Thr Asp Gly Asp Asn Val Leu Leu Thr Leu Asp Val
           195                 200                 205
   Ser Glu Glu Gln Asp Phe Ser Phe Leu Leu Tyr Leu Arg Pro Val Pro
       210                 215                 220
   Asp Ala Leu Lys Ser Leu Trp Tyr Lys Asn Leu Thr Gly Pro Gln Asn
   225                 230                 235                 240
   Ile Thr Leu Asn His Thr Asp Leu Val Pro Cys Leu Cys Ile Gln Val
                   245                 250                 255
   Trp Ser Leu Glu Pro Asp Ser Glu Arg Val Glu Phe Cys Pro Phe Arg
               260                 265                 270
   Glu Asp Pro Gly Ala His Arg Asn Leu Trp His Ile Ala Arg Leu Arg
           275                 280                 285
   Val Leu Ser Pro Gly Val Trp Gln Leu Asp Ala Pro Cys Cys Leu Pro
       290                 295                 300
   Gly Lys Val Thr Leu Cys Trp Gln Ala Pro Asp Gln Ser Pro Cys Gln
   305                 310                 315                 320
   Pro Leu Val Pro Pro Val Pro Gln Lys Asn Ala Thr Val Asn Glu Pro
                   325                 330                 335
   Gln Asp Phe Gln Leu Val Ala Gly His Pro Asn Leu Cys Val Gln Val
               340                 345                 350
   Ser Thr Trp Glu Lys Val Gln Leu Gln Ala Cys Leu Trp Ala Asp Ser
           355                 360                 365
   Leu Gly Pro Phe Lys Asp Asp Met Leu Leu Val Glu Met Lys Thr Gly
       370                 375                 380
   Leu Asn Asn Thr Ser Val Cys Ala Leu Glu Pro Ser Gly Cys Thr Pro
   385                 390                 395                 400
   Leu Pro Ser Met Ala Ser Thr Arg Ala Ala Arg Leu Gly Glu Glu Leu
                   405                 410                 415
   Leu Gln Asp Phe Arg Ser His Gln Cys Met Gln Leu Trp Asn Asp Asp
               420                 425                 430
   Asn Met Gly Ser Leu Trp Ala Cys Pro Met Asp Lys Tyr Ile His Arg
           435                 440                 445
   Arg
<210> 28
   <211> 222
   <212> PRT
   <213> Mus musculus
<400> 28
   Lys Ser Leu Trp Tyr Lys Asn Leu Thr Gly Pro Gln Asn Ile Thr Leu
    1               5                  10                  15
   Asn His Thr Asp Leu Val Pro Cys Leu Cys Ile Gln Val Trp Ser Leu
               20                  25                  30
   Glu Pro Asp Ser Glu Arg Val Glu Phe Cys Pro Phe Arg Glu Asp Pro
           35                  40                  45
   Gly Ala His Arg Asn Leu Trp His Ile Ala Arg Leu Arg Val Leu Ser
       50                  55                  60
   Pro Gly Val Trp Gln Leu Asp Ala Pro Cys Cys Leu Pro Gly Lys Val
   65                  70                  75                  80
   Thr Leu Cys Trp Gln Ala Pro Asp Gln Ser Pro Cys Gln Pro Leu Val
                   85                  90                  95
   Pro Pro Val Pro Gln Lys Asn Ala Thr Val Asn Glu Pro Gln Asp Phe
               100                 105                 110
   Gln Leu Val Ala Gly His Pro Asn Leu Cys Val Gln Val Ser Thr Trp
           115                 120                 125
   Glu Lys Val Gln Leu Gln Ala Cys Leu Trp Ala Asp Ser Leu Gly Pro
       130                 135                 140
   Phe Lys Asp Asp Met Leu Leu Val Glu Met Lys Thr Gly Leu Asn Asn
   145                 150                 155                 160
   Thr Ser Val Cys Ala Leu Glu Pro Ser Gly Cys Thr Pro Leu Pro Ser
                   165                 170                 175
   Met Ala Ser Thr Arg Ala Ala Arg Leu Gly Glu Glu Leu Leu Gln Asp
               180                 185                 190
   Phe Arg Ser His Gln Cys Met Gln Leu Trp Asn Asp Asp Asn Met Gly
           195                 200                 205
   Ser Leu Trp Ala Cys Pro Met Asp Lys Tyr Ile His Arg Arg
       210                 215                 220
<210> 29
   <211> 2287
   <212> DNA
   <213> Mus musculus
<400> 29
   aaatcgaaag cactccagct gaaactgggc ctggagtcca ggctcactgg agtggggaag 60
   catggctgga gaggaattct agcccttgct ctctcccagg gacacggggc tgattgtcag 120
   caggggcgag gggtctgccc ccccttgggg gggcaggacg gggcctcagg cctgggtgct 180
   gtccggcacc tggaagatgc ctgtgtcctg gttcctgctg tccttggcac tgggccgaaa 240
   ccctgtggtc gtctctctgg agagactgat ggagcctcag gacactgcac gctgctctct 300
   aggcctctcc tgccacctct gggatggtga cgtgctctgc ctgcctggaa gcctccagtc 360
   tgccccaggc cctgtgctag tgcctacccg cctgcagacg gagctggtgc tgaggtgtcc 420
   acagaagaca gattgcgccc tctgtgtccg tgtggtggtc cacttggccg tgcatgggca 480
   ctgggcagag cctgaagaag ctggaaagtc tgattcagaa ctccaggagt ctaggaacgc 540
   ctctctccag gcccaggtgg tgctctcctt ccaggcctac cccatcgccc gctgtgccct 600
   gctggaggtc caggtgcccg ctgacctggt gcagcctggt cagtccgtgg gttctgcggt 660
   atttgactgt ttcgaggcta gtcttggggc tgaggtacag atctggtcct acacgaagcc 720
   caggtaccag aaagagctca acctcacaca gcagctgcct gactgcaggg gtcttgaagt 780
   ccgggacagc atccagagct gctgggatgg tgacaatgtc cttctgacac tggatgtctc 840
   tgaggagcag gactttagct tcttactgta cctgcgtcca gtcccggatg ctctcaaatc 900
   cttgtggtac aaaaacctga ctggacctca gaacattact ttaaaccaca cagacctggt 960
   tccctgcctc tgcattcagg tgtggtcgct agagccagac tctgagaggg tcgaattctg 1020
   ccccttccgg gaagatcccg gtgcacacag gaacctctgg cacatagcca ggctgcgggt 1080
   actgtcccca ggggtatggc agctagatgc gccttgctgt ctgccgggca aggtaacact 1140
   gtgctggcag gcaccagacc agagtccctg ccagccactt gtgccaccag tgccccagaa 1200
   gaacgccact gtgaatgagc cacaagattt ccagttggtg gcaggccacc ccaacctctg 1260
   tgtccaggtg agcacctggg agaaggttca gctgcaagcg tgcttgtggg ctgactcctt 1320
   ggggcccttc aaggatgata tgctgttagt ggagatgaaa accggcctca acaacacatc 1380
   agtctgtgcc ttggaaccca gtggctgtac accactgccc agcatggcct ccacgagagc 1440
   tgctcgcctg ggagaggagt tgctgcaaga cttccgatca caccagtgta tgcagctgtg 1500
   gaacgatgac aacatgggat cgctatgggc ctgccccatg gacaagtaca tccacaggcg 1560
   ctgggtccta gtatggctgg cctgcctact cttggctgcg gcgcttttct tcttcctcct 1620
   tctaaaaaag gaccgcagga aagcggcccg tggctcccgc acggccttgc tcctccactc 1680
   cgccgacgga gcgggctacg agcgtctggt gggagcactg gcgtccgcgt tgagccagat 1740
   gccactgcgc gtggccgtgg acctgtggag ccgccgcgag ctgagcgcgc acggagccct 1800
   agcctggttc caccaccagc gacgccgtat cctgcaggag ggtggcgtgg taatccttct 1860
   cttctcgccc gcggccgtgg cgcagtgtca gcagtggctg cagctccaga cagtggagcc 1920
   cgggccgcat gacgccctcg ccgcctggct cagctgcgtg ctacccgatt tcctgcaagg 1980
   ccgggcgacc ggccgctacg tcggggtcta cttcgacggg ctgctgcacc cagactctgt 2040
   gccctccccg ttccgcgtcg ccccgctctt ctccctgccc tcgcagctgc cggctttcct 2100
   ggatgcactg cagggaggct gctccacttc cgcggggcga cccgcggacc gggtggaacg 2160
   agtgacccag gcgctgcggt ccgccctgga cagctgtact tctagctcgg aagccccagg 2220
   ctgctgcgag gaatgggacc tgggaccctg cactacacta gaataaaagc cgatacagta 2280
   ttcctaa                                                           2287
<210> 30
   <211> 689
   <212> PRT
   <213> Mus musculus
<400> 30
   Met Pro Val Ser Trp Phe Leu Leu Ser Leu Ala Leu Gly Arg Asn Pro
    1               5                  10                  15
   Val Val Val Ser Leu Glu Arg Leu Met Glu Pro Gln Asp Thr Ala Arg
               20                  25                  30
   Cys Ser Leu Gly Leu Ser Cys His Leu Trp Asp Gly Asp Val Leu Cys
           35                  40                  45
   Leu Pro Gly Ser Leu Gln Ser Ala Pro Gly Pro Val Leu Val Pro Thr
       50                  55                  60
   Arg Leu Gln Thr Glu Leu Val Leu Arg Cys Pro Gln Lys Thr Asp Cys
   65                  70                  75                  80
   Ala Leu Cys Val Arg Val Val Val His Leu Ala Val His Gly His Trp
                   85                  90                  95
   Ala Glu Pro Glu Glu Ala Gly Lys Ser Asp Ser Glu Leu Gln Glu Ser
               100                 105                 110
   Arg Asn Ala Ser Leu Gln Ala Gln Val Val Leu Ser Phe Gln Ala Tyr
           115                 120                 125
   Pro Ile Ala Arg Cys Ala Leu Leu Glu Val Gln Val Pro Ala Asp Leu
       130                 135                 140
   Val Gln Pro Gly Gln Ser Val Gly Ser Ala Val Phe Asp Cys Phe Glu
   145                 150                 155                 160
   Ala Ser Leu Gly Ala Glu Val Gln Ile Trp Ser Tyr Thr Lys Pro Arg
                   165                 170                 175
   Tyr Gln Lys Glu Leu Asn Leu Thr Gln Gln Leu Pro Asp Cys Arg Gly
               180                 185                 190
   Leu Glu Val Arg Asp Ser Ile Gln Ser Cys Trp Asp Gly Asp Asn Val
           195                 200                 205
   Leu Leu Thr Leu Asp Val Ser Glu Glu Gln Asp Phe Ser Phe Leu Leu
       210                 215                 220
   Tyr Leu Arg Pro Val Pro Asp Ala Leu Lys Ser Leu Trp Tyr Lys Asn
   225                 230                 235                 240
   Leu Thr Gly Pro Gln Asn Ile Thr Leu Asn His Thr Asp Leu Val Pro
                   245                 250                 255
   Cys Leu Cys Ile Gln Val Trp Ser Leu Glu Pro Asp Ser Glu Arg Val
               260                 265                 270
   Glu Phe Cys Pro Phe Arg Glu Asp Pro Gly Ala His Arg Asn Leu Trp
           275                 280                 285
   His Ile Ala Arg Leu Arg Val Leu Ser Pro Gly Val Trp Gln Leu Asp
       290                 295                 300
   Ala Pro Cys Cys Leu Pro Gly Lys Val Thr Leu Cys Trp Gln Ala Pro
   305                 310                 315                 320
   Asp Gln Ser Pro Cys Gln Pro Leu Val Pro Pro Val Pro Gln Lys Asn
                   325                 330                 335
   Ala Thr Val Asn Glu Pro Gln Asp Phe Gln Leu Val Ala Gly His Pro
               340                 345                 350
   Asn Leu Cys Val Gln Val Ser Thr Trp Glu Lys Val Gln Leu Gln Ala
           355                 360                 365
   Cys Leu Trp Ala Asp Ser Leu Gly Pro Phe Lys Asp Asp Met Leu Leu
       370                 375                 380
   Val Glu Met Lys Thr Gly Leu Asn Asn Thr Ser Val Cys Ala Leu Glu
   385                 390                 395                 400
   Pro Ser Gly Cys Thr Pro Leu Pro Ser Met Ala Ser Thr Arg Ala Ala
                   405                 410                 415
   Arg Leu Gly Glu Glu Leu Leu Gln Asp Phe Arg Ser His Gln Cys Met
               420                 425                 430
   Gln Leu Trp Asn Asp Asp Asn Met Gly Ser Leu Trp Ala Cys Pro Met
           435                 440                 445
   Asp Lys Tyr Ile His Arg Arg Trp Val Leu Val Trp Leu Ala Cys Leu
       450                 455                 460
   Leu Leu Ala Ala Ala Leu Phe Phe Phe Leu Leu Leu Lys Lys Asp Arg
   465                 470                 475                 480
   Arg Lys Ala Ala Arg Gly Ser Arg Thr Ala Leu Leu Leu His Ser Ala
                   485                 490                 495
   Asp Gly Ala Gly Tyr Glu Arg Leu Val Gly Ala Leu Ala Ser Ala Leu
               500                 505                 510
   Ser Gln Met Pro Leu Arg Val Ala Val Asp Leu Trp Ser Arg Arg Glu
           515                 520                 525
   Leu Ser Ala His Gly Ala Leu Ala Trp Phe His His Gln Arg Arg Arg
       530                 535                 540
   Ile Leu Gln Glu Gly Gly Val Val Ile Leu Leu Phe Ser Pro Ala Ala
   545                 550                 555                 560
   Val Ala Gln Cys Gln Gln Trp Leu Gln Leu Gln Thr Val Glu Pro Gly
                   565                 570                 575
   Pro His Asp Ala Leu Ala Ala Trp Leu Ser Cys Val Leu Pro Asp Phe
               580                 585                 590
   Leu Gln Gly Arg Ala Thr Gly Arg Tyr Val Gly Val Tyr Phe Asp Gly
           595                 600                 605
   Leu Leu His Pro Asp Ser Val Pro Ser Pro Phe Arg Val Ala Pro Leu
       610                 615                 620
   Phe Ser Leu Pro Ser Gln Leu Pro Ala Phe Leu Asp Ala Leu Gln Gly
   625                 630                 635                 640
   Gly Cys Ser Thr Ser Ala Gly Arg Pro Ala Asp Arg Val Glu Arg Val
                   645                 650                 655
   Thr Gln Ala Leu Arg Ser Ala Leu Asp Ser Cys Thr Ser Ser Ser Glu
               660                 665                 670
   Ala Pro Gly Cys Cys Glu Glu Trp Asp Leu Gly Pro Cys Thr Thr Leu
           675                 680                 685
   Glu
<210> 31
   <211> 21
   <212> DNA
   <213> Artificial Sequence
<220>
   <223> oligonucleotide primer
<400> 31
   tcccgtcccc cgccccaggt c 21
<210> 32
   <211> 25
   <212> DNA
   <213> Artificial Sequence
<220>
   <223> oligonucleotide primer
<400> 32
   ctctccatcc ttatctttca tcaac 25
<210> 33
   <211> 24
   <212> DNA
   <213> Artificial Sequence
<220>
   <223> oligonucleotide primer
<400> 33
   ctctctgctg gctaaacaaa acac 24
<210> 34
   <211> 26
   <212> DNA
   <213> Artificial Sequence
<220>
   <223> oligonucleotide primer
<400> 34
   ctcatattgc tcaactgtgt gaaaaa 26
<210> 35
   <211> 25 '
   <212> DNA
   <213> Artificial Sequence
<220>
   <223> oligonucleotide primer
<400> 35
   tagaagccac ctgaacacaa atctg 25
<210> 36
   <211> 28
   <212> DNA
   <213> Artificial Sequence
<220>
   <223> oligonucleotide primer
<400> 36
   atcttgcgtt gtatgttgaa aatcaatt 28
<210> 37
   <211> 25
   <212> DNA
   <213> Artificial Sequence
<220>
   <223> oligonucleotide primer
<400> 37
   ttctccacca ggtaaacaag tctac 25
<210> 38
   <211> 24
   <212> DNA
   <213> Artificial Sequence
<220>
   <223> oligonucleotide primer
<400> 38
   ctctccaggc ccaagtcgtg ctct 24
<210> 39
   <211> 24
   <212> DNA
   <213> Artificial Sequence
<220>
   <223> oligonucleotide primer
<400> 39
   ttgtcctggg ggcctcgtgt ctcc 24
<210> 40
   <211> 24
   <212> DNA
   <213> Artificial Sequence
<220>
   <223> oligonucleotide primer
<400> 40
   acgaagccca ggtaccagaa agag 24
<210> 41
   <211> 24
   <212> DNA
   <213> Artificial Sequence
<220>
   <223> oligonucleotide primer
<400> 41
   aaaagcgccg cagccaagag tagg 24
<210> 42
   <211> 1293
   <212> DNA
   <213> Homo sapiens
<400> 42
   ctggagaggc ttgtggggcc tcaggacgct acccactgct ctccgggcct ctcctgccgc 60
   ctctgggaca gtgacatact ctgcctgcct ggggacatcg tgcctgctcc gggccccgtg 120
   ctggcgccta cgcacctgca gacagagctg gtgctgaggt gccagaagga gaccgactgt 180
   gacctctgtc tgcgtgtggc tgtccacttg gccgtgcatg ggcactggga agagcctgaa 240
   gatgaggaaa agtttggagg agcagctgac tcaggggtgg aggagcctag gaatgcctct 300
   ctccaggccc aagtcgtgct ctccttccag gcctacccta ctgcccgctg cgtcctgctg 360
   gaggtgcaag tgcctgctgc ccttgtgcag tttggtcagt ctgtgggctc tgtggtatat 420
   gactgcttcg aggctgccct agggagtgag gtacgaatct ggtcctatac tcagcccagg 480
   tacgagaagg aactcaacca cacacagcag ctgcctgccc tgccctggct caacgtgtca 540
   gcagatggtg acaacgtgca tctggttctg aatgtctctg aggagcagca cttcggcctc 600
   tccctgtact ggaatcaggt ccagggcccc ccaaaacccc ggtggcacaa aaacctgact 660
   ggaccgcaga tcattacctt gaaccacaca gacctggttc cctgcctctg tattcaggtg 720
   tggcctctgg aacctgactc cgttaggacg aacatctgcc ccttcaggga ggacccccgc 780
   gcacaccaga acctctggca agccgcccga ctgcgactgc tgaccctgca gagctggctg 840
   ctggacgcac cgtgctcgct gcccgcagaa gcggcactgt gctggcgggc tccgggtggg 900
   gacccctgcc agccactggt cccaccgctt tcctgggaga acgtcactgt ggacaaggtt 960
   ctcgagttcc cattgctgaa aggccaccct aacctctgtg ttcaggtgaa cagctcggag 1020
   aagctgcagc tgcaggagtg cttgtgggct gactccctgg ggcctctcaa agacgatgtg 1080
   ctactgttgg agacacgagg cccccaggac aacagatccc tctgtgcctt ggaacccagt 1140
   ggctgtactt cactacccag caaagcctcc acgagggcag ctcgccttgg agagtactta 1200
   ctacaagacc tgcagtcagg ccagtgtctg cagctatggg acgatgactt gggagcgcta 1260
   tgggcctgcc ccatggacaa atacatccac aag 1293
<210> 43
   <211> 431
   <212> PRT
   <213> Homo sapiens
<400> 43
   Leu Glu Arg Leu Val Gly Pro Gln Asp Ala Thr His Cys Ser Pro Gly
    1               5                  10                  15
   Leu Ser Cys Arg Leu Trp Asp Ser Asp Ile Leu Cys Leu Pro Gly Asp
               20                  25                  30
   Ile Val Pro Ala Pro Gly Pro Val Leu Ala Pro Thr His Leu Gln Thr
           35                  40                  45
   Glu Leu Val Leu Arg Cys Gln Lys Glu Thr Asp Cys Asp Leu Cys Leu
       50                  55                  60
   Arg Val Ala Val His Leu Ala Val His Gly His Trp Glu Glu Pro Glu
   65                  70                  75                  80
   Asp Glu Glu Lys Phe Gly Gly Ala Ala Asp Ser Gly Val Glu Glu Pro
                   85                  90                  95
   Arg Asn Ala Ser Leu Gln Ala Gln Val Val Leu Ser Phe Gln Ala Tyr
               100                 105                 110
   Pro Thr Ala Arg Cys Val Leu Leu Glu Val Gln Val Pro Ala Ala Leu
           115                 120                 125
   Val Gln Phe Gly Gln Ser Val Gly Ser Val Val Tyr Asp Cys Phe Glu
       130                 135                 140
   Ala Ala Leu Gly Ser Glu Val Arg Ile Trp Ser Tyr Thr Gln Pro Arg
   145                 150                 155                 160
   Tyr Glu Lys Glu Leu Asn His Thr Gln Gln Leu Pro Ala Leu Pro Trp
                   165                 170                 175
   Leu Asn Val Ser Ala Asp Gly Asp Asn Val His Leu Val Leu Asn Val
               180                 185                 190
   Ser Glu Glu Gln His Phe Gly Leu Ser Leu Tyr Trp Asn Gln Val Gln
           195                 200                 205
   Gly Pro Pro Lys Pro Arg Trp His Lys Asn Leu Thr Gly Pro Gln Ile
       210                 215                 220
   Ile Thr Leu Asn His Thr Asp Leu Val Pro Cys Leu Cys Ile Gln Val
   225                 230                 235                 240
   Trp Pro Leu Glu Pro Asp Ser Val Arg Thr Asn Ile Cys Pro Phe Arg
                   245                 250                 255
   Glu Asp Pro Arg Ala His Gln Asn Leu Trp Gln Ala Ala Arg Leu Arg
               260                 265                 270
   Leu Leu Thr Leu Gln Ser Trp Leu Leu Asp Ala Pro Cys Ser Leu Pro
           275                 280                 285
   Ala Glu Ala Ala Leu Cys Trp Arg Ala Pro Gly Gly Asp Pro Cys Gln
       290                 295                 300
   Pro Leu Val Pro Pro Leu Ser Trp Glu Asn Val Thr Val Asp Lys Val
   305                 310                 315                 320
   Leu Glu Phe Pro Leu Leu Lys Gly His Pro Asn Leu Cys Val Gln Val
                   325                 330                 335
   Asn Ser Ser Glu Lys Leu Gln Leu Gln Glu Cys Leu Trp Ala Asp Ser
               340                 345                 350
   Leu Gly Pro Leu Lys Asp Asp Val Leu Leu Leu Glu Thr Arg Gly Pro
           355                 360                 365
   Gln Asp Asn Arg Ser Leu Cys Ala Leu Glu Pro Ser Gly Cys Thr Ser
       370                 375                 380
   Leu Pro Ser Lys Ala Ser Thr Arg Ala Ala Arg Leu Gly Glu Tyr Leu
   385                 390                 395                 400
   Leu Gln Asp Leu Gln Ser Gly Gln Cys Leu Gln Leu Trp Asp Asp Asp
                   405                 410                 415
   Leu Gly Ala Leu Trp Ala Cys Pro Met Asp Lys Tyr Ile His Lys
               420                 425                 430
<210> 44
   <211> 699
   <212> DNA
   <213> Homo sapiens
<400> 44
   gagcccagag ggcccacaat caagccctgt cctccatgca aatgcccagc acctaacctc 60
   ttgggtggac catccgtctt catcttccct ccaaagatca aggatgtact catgatctcc 120
   ctgagcccca tagtcacatg tgtggtggtg gatgtgagcg aggatgaccc agatgtccag 180
   atcagctggt ttgtgaacaa cgtggaagta cacacagctc agacacaaac ccatagagag 240
   gattacaaca gtactctccg ggtggtcagt gccctcccca tccagcacca ggactggatg 300
   agtggcaagg agttcaaatg caaggtcaac aacaaagacc tcccagcgcc catcgagaga 360
   accatctcaa aacccaaagg gtcagtaaga gctccacagg tatatgtctt gcctccacca 420
   gaagaagaga tgactaagaa acaggtcact ctgacctgca tggtcacaga cttcatgcct 480
   gaagacattt acgtggagtg gaccaacaac gggaaaacag agctaaacta caagaacact 540
   gaaccagtcc tggactctga tggttcttac ttcatgtaca gcaagctgag agtggaaaag 600
   aagaactggg tggaaagaaa tagctactcc tgttcagtgg tccacgaggg tctgcacaat 660
   caccacacga ctaagagctt ctcccggact ccgggtaaa                        699
<210> 45
   <211> 233
   <212> PRT
   <213> Homo sapiens
<400> 45
   Glu Pro Arg Gly Pro Thr Ile Lys Pro Cys Pro Pro Cys Lys Cys Pro
   1               5                   10                  15
   Ala Pro Asn Leu Leu Gly Gly Pro Ser Val Phe Ile Phe Pro Pro Lys
                   20                      25                      30
   Ile Lys Asp Val Leu Met Ile Ser Leu Ser Pro Ile Val Thr Cys Val
           35                      40                      45
   Val Val Asp Val Ser Glu Asp Asp Pro Asp Val Gln Ile Ser Trp Phe
       50                      55                      60
   Val Asn Asn Val Glu Val His Thr Ala Gln Thr Gln Thr His Arg Glu
   65                      70                      75                  80
   Asp Tyr Asn Ser Thr Leu Arg Val Val Ser Ala Leu Pro Ile Gln His
                   85                      90                      95
   Gln Asp Trp Met Ser Gly Lys Glu Phe Lys Cys Lys Val Asn Asn Lys
               100                     105                     110
   Asp Leu Pro Ala Pro Ile Glu Arg Thr Ile Ser Lys Pro Lys Gly Ser
           115                     120                     125
   Val Arg Ala Pro Gln Val Tyr Val Leu Pro Pro Pro Glu Glu Glu Met
       130                     135                     140
   Thr Lys Lys Gln Val Thr Leu Thr Cys Met Val Thr Asp Phe Met Pro
   145                     150                     155                 160
   Glu Asp Ile Tyr Val Glu Trp Thr Asn Asn Gly Lys Thr Glu Leu Asn
                   165                     170                     175
   Tyr Lys Asn Thr Glu Pro Val Leu Asp Ser Asp Gly Ser Tyr Phe Met
               180                     185                     190
   Tyr Ser Lys Leu Arg Val Glu Lys Lys Asn Trp Val Glu Arg Asn Ser
           195                     200                     205
   Tyr Ser Cys Ser Val Val His Glu Gly Leu His Asn His His Thr Thr
       210                     215                     220
   Lys Ser Phe Ser Arg Thr Pro Gly Lys
   225                     230
<210> 46
   <211> 69
   <212> DNA
   <213> Artificial Sequence
<220>
   <223> oligonucleotide primer
<400> 46
   gtttcgctca gccaggaaat ccatgccgag ttgagacgct tccgtagact ggagaggctt 60
   gtggggcct                                                          69
<210> 47
   <211> 36
   <212> DNA
   <213> Artificial Sequence
<220>
   <223> oligonucleotide primer
<400> 47
   tgtgggccct ctgggctcct tgtggatgta tttgtc 36
<210> 48
   <211> 36
   <212> DNA
   <213> Artificial Sequence
<220>
   <223> oligonucleotide primer
<400> 48
   gacaaataca tccacaagga gcccagaggg cccaca 36
<210> 49
   <211> 55
   <212> DNA
   <213> Artificial Sequence
<220>
   <223> oligonucleotide primer
<400> 49
   caaccccaga gctgttttaa ggcgcgcctc tagattattt acccggagtc cggga 55
<210> 50
   <211> 76
   <212> DNA
   <213> Artificial Sequence
<220>
   <223> oligonucleotide primer
<400> 50
   caaccccaga gctgttttaa ggcgcgcctc tagattattc catgggcatg tattcttcct 60
   tgtggatgta tttgtc 76
<210> 51
   <211> 10
   <212> PRT
   <213> Artificial Sequence
<220>
   <223> C-terminal his tag
<400> 51
   Gly Ser Gly Gly His His His His His His
   1 5 10
<210> 52
   <211> 10
   <212> PRT
   <213> Artificial Sequence
<220>
   <223> C-terminal FLAG tag
<400> 52
   Gly Ser Asp Tyr Lys Asp Asp Asp Asp Lys
   1 5 10
<210> 53
   <211> 7
   <212> PRT
   <213> Artificial Sequence
<220>
   <223> Glu-Glu tag
<400> 53
   Glu Glu Tyr Met Pro Met Glu
   1 5
<210> 54
   <211> 85
   <212> DNA
   <213> Artificial Sequence
<220>
   <223> oligonucleotide primer
<400> 54
   caaccccaga gctgttttaa ggcgcgcctc tagattagtg atggtgatgg tgatgtccac 60
   cagatccctt gtggatgtat ttgtc 85
<210> 55
   <211> 85
   <212> DNA
   <213> Artificial Sequence
<220>
   <223> oligonucleotide primer
<400> 55
   caaccccaga gctgttttaa ggcgcgcctc tagattactt atcatcatca tccttataat 60
   cggatccctt gtggatgtat ttgtc 85
<210> 56
   <211> 24
   <212> DNA
   <213> Artificial Sequence
<220>
   <223> oligonucleotide primer
<400> 56
   acgaagccca ggtaccagaa agag 24
<210> 57
   <211> 24
   <212> DNA
   <213> Artificial Sequence
<220>
   <223> oligonucleotide primer
<400> 57
   aaaagcgccg cagccaagag tagg 24
<210> 58
   <211> 20
   <212> DNA
   <213> Artificial Sequence
<220>
   <223> oligonucleotide primer
<400> 58
   cgtaagcggt ggcggttttc 20
<210> 59
   <211> 20
   <212> DNA
   <213> Artificial Sequence
<220>
   <223> oligonucleotide primer
<400> 59
   tgggcagggc acagtcacag 20
<210> 60
   <211> 24
   <212> DNA
   <213> Artificial Sequence
<220>
   <223> oligonucleotide primer
<400> 60
   acttgccatt ctgagggagg tagc 24
<210> 61
   <211> 24
   <212> DNA
   <213> Artificial Sequence
<220>
   <223> oligonucleotide primer
<400> 61
   cacaggtgca gccaactttt agga 24
<210> 62
   <211> 20
   <212> DNA
   <213> Artificial Sequence
<220>
   <223> oligonucleotide primer
<400> 62
   gtgggccgct ctaggcacca 20
<210> 63
   <211> 25
   <212> DNA
   <213> Artificial Sequence
<220>
   <223> oligonucleotide primer
<400> 63
   cggttggcct tagggttcag ggggg 25
<210> 64
   <211>.2127
   <212> DNA
   <213> Homo sapiens
<400> 64
<210> 65
   <211> 708
   <212> PRT
   <213> Homo sapiens
<400> 65
   Met Asp Ala Met Lys Arg Gly Leu Cys Cys Val Leu Leu Leu Cys Gly
    1               5                  10                  15
   Ala Val Phe Val Ser Leu Ser Gln Glu Ile His Ala Glu Leu Arg Arg
               20                  25                  30
   Phe Arg Arg Leu Glu Arg Leu Val Gly Pro Gln Asp Ala Thr His Cys
           35                  40                  45
   Ser Pro Gly Leu Ser Cys Arg Leu Trp Asp Ser Asp Ile Leu Cys Leu
       50                  55                  60
   Pro Gly Asp Ile Val Pro Ala Pro Gly Pro Val Leu Ala Pro Thr His
   65                  70                  75                  80
   Leu Gln Thr Glu Leu Val Leu Arg Cys Gln Lys Glu Thr Asp Cys Asp
                   85                  90                  95
   Leu Cys Leu Arg Val Ala Val His Leu Ala Val His Gly His Trp Glu
               100                 105                 110
   Glu Pro Glu Asp Glu Glu Lys Phe Gly Gly Ala Ala Asp Ser Gly Val
           115                 120                 125
   Glu Glu Pro Arg Asn Ala Ser Leu Gln Ala Gln Val Val Leu Ser Phe
       130                 135                 140
   Gln Ala Tyr Pro Thr Ala Arg Cys Val Leu Leu Glu Val Gln Val Pro
   145                 150                 155                 160
   Ala Ala Leu Val Gln Phe Gly Gln Ser Val Gly Ser Val Val Tyr Asp
                   165                 170                 175
   Cys Phe Glu Ala Ala Leu Gly Ser Glu Val Arg Ile Trp Ser Tyr Thr
               180                 185                 190
   Gln Pro Arg Tyr Glu Lys Glu Leu Asn His Thr Gln Gln Leu Pro Ala
           195                 200                 205
   Leu Pro Trp Leu Asn Val Ser Ala Asp Gly Asp Asn Val His Leu Val
       210                 215                 220
   Leu Asn Val Ser Glu Glu Gln His Phe Gly Leu Ser Leu Tyr Trp Asn
   225                 230                 235                 240
   Gln Val Gln Gly Pro Pro Lys Pro Arg Trp His Lys Asn Leu Thr Gly
                   245                 250                 255
   Pro Gln Ile Ile Thr Leu Asn His Thr Asp Leu Val Pro Cys Leu Cys
               260                 265                 270
   Ile Gln Val Trp Pro Leu Glu Pro Asp Ser Val Arg Thr Asn Ile Cys
           275                 280                 285
   Pro Phe Arg Glu Asp Pro Arg Ala His Gln Asn Leu Trp Gln Ala Ala
       290                 295                 300
   Arg Leu Arg Leu Leu Thr Leu Gln Ser Trp Leu Leu Asp Ala Pro Cys
   305                 310                 315                 320
   Ser Leu Pro Ala Glu Ala Ala Leu Cys Trp Arg Ala Pro Gly Gly Asp
                   325                 330                 335
   Pro Cys Gln Pro Leu Val Pro Pro Leu Ser Trp Glu Asn Val Thr Val
               340                 345                 350
   Asp Lys Val Leu Glu Phe Pro Leu Leu Lys Gly His Pro Asn Leu Cys
           355                 360                 365
   Val Gln Val Asn Ser Ser Glu Lys Leu Gln Leu Gln Glu Cys Leu Trp
       370                 375                 380
   Ala Asp Ser Leu Gly Pro Leu Lys Asp Asp Val Leu Leu Leu Glu Thr
   385                 390                 395                 400
   Arg Gly Pro Gln Asp Asn Arg Ser Leu Cys Ala Leu Glu Pro Ser Gly
                   405                 410                 415
   Cys Thr Ser Leu Pro Ser Lys Ala Ser Thr Arg Ala Ala Arg Leu Gly
               420                 425                 430
   Glu Tyr Leu Leu Gln Asp Leu Gln Ser Gly Gln Cys Leu Gln Leu Trp
           435                 440                 445
   Asp Asp Asp Leu Gly Ala Leu Trp Ala Cys Pro Met Asp Lys Tyr Ile
       450                 455                 460
   His Lys Gly Gly Ser Gly Gly Gly Thr Gly Ser Leu Val Pro Arg Gly
   465                 470                 475                 480
   Ser Asp Lys Thr His Thr Cys Pro Pro Cys Pro Ala Pro Glu Leu Leu
                   485                 490                 495
   Gly Gly Pro Ser Val Phe Leu Phe Pro Pro Lys Pro Lys Asp Thr Leu
               500                 505                 510
   Met Ile Ser Arg Thr Pro Glu Val Thr Cys Val Val Val Asp Val Ser
           515                 520                 525
   His Glu Asp Pro Glu Val Lys Phe Asn Trp Tyr Val Asp Gly Val Glu
       530                 535                 540
   Val His Asn Ala Lys Thr Lys Pro Arg Glu Glu Gln Tyr Asn Ser Thr
   545                 550                 555                 560
   Tyr Arg Val Val Ser Val Leu Thr Val Leu His Gln Asp Trp Leu Asn
                   565                 570                 575
   Gly Lys Glu Tyr Lys Cys Lys Val Ser Asn Lys Ala Leu Pro Ala Pro
               580                 585                 590
   Ile Glu Lys Thr Ile Ser Lys Ala Lys Gly Gln Pro Arg Glu Pro Gln
           595                 600                 605
   Val Tyr Thr Leu Pro Pro Ser Arg Asp Glu Leu Thr Lys Asn Gln Val
       610                 615                 620
   Ser Leu Thr Cys Leu Val Lys Gly Phe Tyr Pro Ser Asp Ile Ala Val
   625                 630                 635                 640
   Glu Trp Glu Ser Asn Gly Gln Pro Glu Asn Asn Tyr Lys Thr Thr Pro
                   645                 650                 655
   Pro Val Leu Asp Ser Asp Gly Ser Phe Phe Leu Tyr Ser Lys Leu Thr
               660                 665                 670
   Val Asp Lys Ser Arg Trp Gln Gln Gly Asn Val Phe Ser Cys Ser Val
           675                 680                 685
   Met His Glu Ala Leu His Asn His Tyr Thr Gln Lys Ser Leu Ser Leu
       690                 695                 700
   Ser Pro Gly Lys
   705
<210> 66
   <211> 1416
   <212> DNA
   <213> Artificial Sequence
<220>
   <223> synthetic insert
<400> 66
   agccaggaaa tccatgccga gttgagacgc ttccgtagac tggagaggct tgtggggcct 60
   caggacgcta cccactgctc tccgggcctc tcctgccgcc tctgggacag tgacatactc 120
   tgcctgcctg gggacatcgt gcctgctccg ggccccgtgc tggcgcctac gcacctgcag 180
   acagagctgg tgctgaggtg ccagaaggag accgactgtg acctctgtct gcgtgtggct 240
   gtccacttgg ccgtgcatgg gcactgggaa gagcctgaag atgaggaaaa gtttggagga 300
   gcagctgact caggggtgga ggagcctagg aatgcctctc tccaggccca agtcgtgctc 360
   tccttccagg cctaccctac tgcccgctgc gtcctgctgg aggtgcaagt gcctgctgcc 420
   cttgtgcagt ttggtcagtc tgtgggctct gtggtatatg actgcttcga ggctgcccta 480
   gggagtgagg tacgaatctg gtcctatact cagcccaggt acgagaagga actcaaccac 540
   acacagcagc tgcctgccct gccctggctc aacgtgtcag cagatggtga caacgtgcat 600
   ctggttctga atgtctctga ggagcagcac ttcggcctct ccctgtactg gaatcaggtc 660
   cagggccccc caaaaccccg gtggcacaaa aacctgactg gaccgcagat cattaccttg 720
   aaccacacag acctggttcc ctgcctctgt attcaggtgt ggcctctgga acctgactcc 780
   gttaggacga acatctgccc cttcagggag gacccccgcg cacaccagaa cctctggcaa 840
   gccgcccgac tgcgactgct gaccctgcag agctggctgc tggacgcacc gtgctcgctg 900
   cccgcagaag cggcactgtg ctggcgggct ccgggtgggg acccctgcca gccactggtc 960
   ccaccgcttt cctgggagaa cgtcactgtg gacaaggttc tcgagttccc attgctgaaa 1020
   ggccacccta acctctgtgt tcaggtgaac agctcggaga agctgcagct gcaggagtgc 1080
   ttgtgggctg actccctggg gcctctcaaa gacgatgtgc tactgttgga gacacgaggc 1140
   ccccaggaca acagatccct ctgtgccttg gaacccagtg gctgtacttc actacccagc 1200
   aaagcctcca cgagggcagc tcgccttgga gagtacttac tacaagacct gcagtcaggc 1260
   cagtgtctgc agctatggga cgatgacttg ggagcgctat gggcctgccc catggacaaa 1320
   tacatccaca agggaggaag tggcggagga acaggaagtt tggtccctcg tggaagcgac 1380
   aaaactcaca catgcccacc gtgcccagca cctgaa 1416
<210> 67
   <211> 2154
   <212> DNA
   <213> homo sapians
<400> 67
   atggatgcaa tgaagagagg gctctgctgt gtgctgctgc tgtgtggcgc cgtcttcgtt 60
   tcgctcagcc aggaaatcca tgccgagttg agacgcttcc gtagactgga gaggcttgtg 120
   gggcctcagg acgctaccca ctgctctccg ggcctctcct gccgcctctg ggacagtgac 180
   atactctgcc tgcctgggga catcgtgcct gctccgggcc ccgtgctggc gcctacgcac 240
   ctgcagacag agctggtgct gaggtgccag aaggagaccg actgtgacct ctgtctgcgt 300
   gtggctgtcc acttggccgt gcatgggcac tgggaagagc ctgaagatga ggaaaagttt 360
   ggaggagcag ctgactcagg ggtggaggag cctaggaatg cctctctcca ggcccaagtc 420
   gtgctctcct tccaggccta ccctactgcc cgctgcgtcc tgctggaggt gcaagtgcct 480
   gctgcccttg tgcagtttgg tcagtctgtg ggctctgtgg tatatgactg cttcgaggct 540
   gccctaggga gtgaggtacg aatctggtcc tatactcagc ccaggtacga gaaggaactc 600
   aaccacacac agcagctgcc tgccctgccc tggctcaacg tgtcagcaga tggtgacaac 660
   gtgcatctgg ttctgaatgt ctctgaggag cagcacttcg gcctctccct gtactggaat 720
   caggtccagg gccccccaaa accccggtgg cacaaaaacc tgactggacc gcagatcatt 780
   accttgaacc acacagacct ggttccctgc ctctgtattc aggtgtggcc tctggaacct 840
   gactccgtta ggacgaacat ctgccccttc agggaggacc cccgcgcaca ccagaacctc 900
   tggcaagccg cccgactgcg actgctgacc ctgcagagct ggctgctgga cgcaccgtgc 960
   tcgctgcccg cagaagcggc actgtgctgg cgggctccgg gtggggaccc ctgccagcca 1020
   ctggtcccac cgctttcctg ggagaacgtc actgtggaca aggttctcga gttcccattg 1080
   ctgaaaggcc accctaacct ctgtgttcag gtgaacagct cggagaagct gcagctgcag 1140
   gagtgcttgt gggctgactc cctggggcct ctcaaagacg atgtgctact gttggagaca 1200
   cgaggccccc aggacaacag atccctctgt gccttggaac ccagtggctg tacttcacta 1260
   cccagcaaag cctccacgag ggcagctcgc cttggagagt acttactaca agacctgcag 1320
   tcaggccagt gtctgcagct atgggacgat gacttgggag cgctatgggc ctgccccatg 1380
   gacaaataca tccacaaggg aggtgggggc tccggcgggg gtggaagcgg tggaggcggg 1440
   tcggggggcg gaggtagtga gcccaaatct tcagacaaaa ctcacacatg cccaccgtgc 1500
   ccagcacctg aactcctggg gggaccgtca gtcttcctct tccccccaaa acccaaggac 1560
   accctcatga tctcccggac ccctgaggtc acatgcgtgg tggtggacgt gagccacgaa 1620
   gaccctgagg tcaagttcaa ctggtacgtg gacggcgtgg aggtgcataa tgccaagaca 1680
   aagccgcggg aggagcagta caacagcacg taccgtgtgg tcagcgtcct caccgtcctg 1740
   caccaggact ggctgaatgg caaggagtac aagtgcaagg tctccaacaa agccctccca 1800
   gcccccatcg agaaaaccat ctccaaagcc aaagggcagc cccgagaacc acaggtgtac 1860
   accctgcccc catcccggga tgagctgacc aagaaccagg tcagcctgac ctgcctggtc 1920
   aaaggcttct atcccagcga catcgccgtg gagtgggaga gcaatgggca gccggagaac 1980
   aactacaaga ccacgcctcc cgtgctggac tccgacggct ccttcttcct ctacagcaag 2040
   ctcaccgtgg acaagagcag gtggcagcag gggaacgtct tctcatgctc cgtgatgcat 2100
   gaggctctgc acaaccacta cacgcagaag agcctctccc tgtctccggg taaa       2154
<210> 68
   <211> 718
   <212> PRT
   <213> homosapians
<400> 68
<210> 69
   <211> 2052
   <212> DNA
   <213> homo sapians
<400> 69
   atgcctgtgc cctggttctt gctgtccttg gcactgggcc gaagcccagt ggtcctttct 60
   ctggagaggc ttgtggggcc tcaggacgct acccactgct ctccgggcct ctcctgccgc 120
   ctctgggaca gtgacatact ctgcctgcct ggggacatcg tgcctgctcc gggccccgtg 180
   ctggcgccta cgcacctgca gacagagctg gtgctgaggt gccagaagga gaccgactgt 240
   gacctctgtc tgcgtgtggc tgtccacttg gccgtgcatg ggcactggga agagcctgaa 300
   gatgaggaaa agtttggagg agcagctgac tcaggggtgg aggagcctag gaatgcctct 360
   ctccaggccc aagtcgtgct ctccttccag gcctacccta ctgcccgctg cgtcctgctg 420
   gaggtgcaag tgcctgctgc ccttgtgcag tttggtcagt ctgtgggctc tgtggtatat 480
   gactgcttcg aggctgccct agggagtgag gtacgaatct ggtcctatac tcagcccagg 540
   tacgagaagg aactcaacca cacacagcag ctgcctgccc tgccctggct caacgtgtca 600
   gcagatggtg acaacgtgca tctggttctg aatgtctctg aggagcagca cttcggcctc 660
   tccctgtact ggaatcaggt ccagggcccc ccaaaacccc ggtggcacaa aaacctgact 720
   ggaccgcaga tcattacctt gaaccacaca gacctggttc cctgcctctg tattcaggtg 780
   tggcctctgg aacctgactc cgttaggacg aacatctgcc ccttcaggga ggacccccgc 840
   gcacaccaga acctctggca agccgcccga ctgcgactgc tgaccctgca gagctggctg 900
   ctggacgcac cgtgctcgct gcccgcagaa gcggcactgt gctggcgggc tccgggtggg 960
   gacccctgcc agccactggt cccaccgctt tcctgggaga acgtcactgt ggacaaggtt 1020
   ctcgagttcc cattgctgaa aggccaccct aacctctgtg ttcaggtgaa cagctcggag 1080
   aagctgcagc tgcaggagtg cttgtgggct gactccctgg ggcctctcaa agacgatgtg 1140
   ctactgttgg agacacgagg cccccaggac aacagatccc tctgtgcctt ggaacccagt 1200
   ggctgtactt cactacccag caaagcctcc acgagggcag ctcgccttgg agagtactta 1260
   ctacaagacc tgcagtcagg ccagtgtctg cagctatggg acgatgactt gggagcgcta 1320
   tgggcctgcc ccatggacaa atacatccac aaggagccca aatcttcaga caaaactcac 1380
   acatgcccac cgtgcccagc acctgaagcc gagggggcac cgtcagtctt cctcttcccc 1440
   ccaaaaccca aggacaccct catgatctcc cggacccctg aggtcacatg cgtggtggtg 1500
   gacgtgagcc acgaagaccc tgaggtcaag ttcaactggt acgtggacgg cgtggaggtg 1560
   cataatgcca agacaaagcc gcgggaggag cagtacaaca gcacgtaccg tgtggtcagc 1620
   gtcctcaccg tcctgcacca ggactggctg aatggcaagg agtacaagtg caaggtctcc 1680
   aacaaagccc tcccatcctc catcgagaaa accatctcca aagccaaagg gcagccccga 1740
   gaaccacagg tgtacaccct gcccccatcc cgggatgagc tgaccaagaa ccaggtcagc 1800
   ctgacctgcc tggtcaaagg cttctatccc agcgacatcg ccgtggagtg ggagagcaat 1860
   gggcagccgg agaacaacta caagaccacg cctcccgtgc tggactccga cggctccttc 1920
   ttcctctaca gcaagctcac cgtggacaag agcaggtggc agcaggggaa cgtcttctca 1980
   tgctccgtga tgcatgaggc tctgcacaac cactacacgc agaagagcct ctccctgtct 2040
   ccgggtaaat aa                                                    2052
<210> 70
   <211> 683
   <212> PRT
   <213> homo sapians

<400> 70
   Met Pro Val Pro Trp Phe Leu Leu Ser Leu Ala Leu Gly Arg Ser Pro
    1               5                  10                  15
   Val Val Leu Ser Leu Glu Arg Leu Val Gly Pro Gln Asp Ala Thr His
               20                  25                  30
   Cys Ser Pro Gly Leu Ser Cys Arg Leu Trp Asp Ser Asp Ile Leu Cys
           35                  40                  45
   Leu Pro Gly Asp Ile Val Pro Ala Pro Gly Pro Val Leu Ala Pro Thr
       50                  55                  60
   His Leu Gln Thr Glu Leu Val Leu Arg Cys Gln Lys Glu Thr Asp Cys
   65                  70                  75                  80
   Asp Leu Cys Leu Arg Val Ala Val His Leu Ala Val His Gly His Trp
                   85                  90                  95
   Glu Glu Pro Glu Asp Glu Glu Lys Phe Gly Gly Ala Ala Asp Ser Gly
               100                 105                 110
   Val Glu Glu Pro Arg Asn Ala Ser Leu Gln Ala Gln Val Val Leu Ser
           115                 120                 125
   Phe Gln Ala Tyr Pro Thr Ala Arg Cys Val Leu Leu Glu Val Gln Val
       130                 135                 140
   Pro Ala Ala Leu Val Gln Phe Gly Gln Ser Val Gly Ser Val Val Tyr
   145                 150                 155                 160
   Asp Cys Phe Glu Ala Ala Leu Gly Ser Glu Val Arg Ile Trp Ser Tyr
                   165                 170                 175
   Thr Gln Pro Arg Tyr Glu Lys Glu Leu Asn His Thr Gln Gln Leu Pro
               180                 185                 190
   Ala Leu Pro Trp Leu Asn Val Ser Ala Asp Gly Asp Asn Val His Leu
           195                 200                 205
   Val Leu Asn Val Ser Glu Glu Gln His Phe Gly Leu Ser Leu Tyr Trp
       210                 215                 220
   Asn Gln Val Gln Gly Pro Pro Lys Pro Arg Trp His Lys Asn Leu Thr
   225                 230                 235                 240
   Gly Pro Gln Ile Ile Thr Leu Asn His Thr Asp Leu Val Pro Cys Leu
                   245                 250                 255
   Cys Ile Gln Val Trp Pro Leu Glu Pro Asp Ser Val Arg Thr Asn Ile
               260                 265                 270
   Cys Pro Phe Arg Glu Asp Pro Arg Ala His Gln Asn Leu Trp Gln Ala
           275                 280                 285
   Ala Arg Leu Arg Leu Leu Thr Leu Gln Ser Trp Leu Leu Asp Ala Pro
       290                 295                 300
   Cys Ser Leu Pro Ala Glu Ala Ala Leu Cys Trp Arg Ala Pro Gly Gly
   305                 310                 315                 320
   Asp Pro Cys Gln Pro Leu Val Pro Pro Leu Ser Trp Glu Asn Val Thr
                   325                 330                 335
   Val Asp Lys Val Leu Glu Phe Pro Leu Leu Lys Gly His Pro Asn Leu
               340                 345                 350
   Cys Val Gln Val Asn Ser Ser Glu Lys Leu Gln Leu Gln Glu Cys Leu
           355                 360                 365
   Trp Ala Asp Ser Leu Gly Pro Leu Lys Asp Asp Val Leu Leu Leu Glu
       370                 375                 380
   Thr Arg Gly Pro Gln Asp Asn Arg Ser Leu Cys Ala Leu Glu Pro Ser
   385                 390                 395                 400
   Gly Cys Thr Ser Leu Pro Ser Lys Ala Ser Thr Arg Ala Ala Arg Leu
                   405                 410                 415
   Gly Glu Tyr Leu Leu Gln Asp Leu Gln Ser Gly Gln Cys Leu Gln Leu
               420                 425                 430
   Trp Asp Asp Asp Leu Gly Ala Leu Trp Ala Cys Pro Met Asp Lys Tyr
           435                 440                 445
   Ile His Lys Glu Pro Lys Ser Ser Asp Lys Thr His Thr Cys Pro Pro
       450                 455                 460
   Cys Pro Ala Pro Glu Ala Glu Gly Ala Pro Ser Val Phe Leu Phe Pro
   465                 470                 475                 480
   Pro Lys Pro Lys Asp Thr Leu Met Ile Ser Arg Thr Pro Glu Val Thr
                   485                 490                 495
   Cys Val Val Val Asp Val Ser His Glu Asp Pro Glu Val Lys Phe Asn
               500                 505                 510
   Trp Tyr Val Asp Gly Val Glu Val His Asn Ala Lys Thr Lys Pro Arg
           515                 520                 525
   Glu Glu Gln Tyr Asn Ser Thr Tyr Arg Val Val Ser Val Leu Thr Val
       530                 535                 540
   Leu His Gln Asp Trp Leu Asn Gly Lys Glu Tyr Lys Cys Lys Val Ser
   545                 550                 555                 560
   Asn Lys Ala Leu Pro Ser Ser Ile Glu Lys Thr Ile Ser Lys Ala Lys
                   565                 570                 575
   Gly Gln Pro Arg Glu Pro Gln Val Tyr Thr Leu Pro Pro Ser Arg Asp
               580                 585                 590
   Glu Leu Thr Lys Asn Gln Val Ser Leu Thr Cys Leu Val Lys Gly Phe
           595                 600                 605
   Tyr Pro Ser Asp Ile Ala Val Glu Trp Glu Ser Asn Gly Gln Pro Glu
       610                 615                 620
   Asn Asn Tyr Lys Thr Thr Pro Pro Val Leu Asp Ser Asp Gly Ser Phe
   625                 630                 635                 640
   Phe Leu Tyr Ser Lys Leu Thr Val Asp Lys Ser Arg Trp Gln Gln Gly
                   645                 650                 655
   Asn Val Phe Ser Cys Ser Val Met His Glu Ala Leu His Asn His Tyr
               660                 665                 670
   Thr Gln Lys Ser Leu Ser Leu Ser Pro Gly Lys
           675                 680
<210> 71
   <211> 2130
   <212> DNA
   <213> Artificial Sequence
<220>
   <223> Murine signal peptide and exons 1-6 of murine IL-17RA, exons 8-16 of human IL-17RC, linker and Fc10
<400> 71
   atggcgattc ggcgctgctg gccacgggtc gtccccgggc ccgcgctggg atggctgctt 60
   ctgctgctga acgttctggc cccgggccgc gcctccccgc gcctcctcga cttcccggct 120
   ccggtctgcg cgcaggaggg gctgagctgc agagtcaaga atagtacttg tctggatgac 180
   agctggatcc accccaaaaa cctgaccccg tcttccccaa aaaacatcta tatcaatctt 240
   agtgtttcct ctacccagca cggagaatta gtccctgtgt tgcatgttga gtggaccctg 300
   cagacagatg ccagcatcct gtacctcgag ggtgcagagc tgtccgtcct gcagctgaac 360
   accaatgagc ggctgtgtgt caagttccag tttctgtcca tgctgcagca tcaccgtaag 420
   cggtggcggt tttccttcag ccactttgtg gtagatcctg gccaggagta tgaagtgact 480
   gttcaccacc tgccgaagcc catccctgat ggggacccaa accacaaatc caagatcatc 540
   tttgtgcctg actgtgagga cagcaagatg aagatgacta cctcatgcgt gagctcagcc 600
   ctgccctggc tcaacgtgtc agcagatggt gacaacgtgc atctggttct gaatgtctct 660
   gaggagcagc acttcggcct ctccctgtac tggaatcagg tccagggccc cccaaaaccc 720
   cggtggcaca aaaacctgac tggaccgcag atcattacct tgaaccacac agacctggtt 780
   ccctgcctct gtattcaggt gtggcctctg gaacctgact ccgttaggac gaacatctgc 840
   cccttcaggg aggacccccg cgcacaccag aacctctggc aagccgcccg actgcgactg 900
   ctgaccctgc agagctggct gctggacgca ccgtgctcgc tgcccgcaga agcggcactg 960
   tgctggcggg ctccgggtgg ggacccctgc cagccactgg tcccaccgct ttcctgggag 1020
   aacgtcactg tggacaaggt tctcgagttc ccattgctga aaggccaccc taacctctgt 1080
   gttcaggtga acagctcgga gaagctgcag ctgcaggagt gcttgtgggc tgactccctg 1140
   gggcctctca aagacgatgt gctactgttg gagacacgag gcccccagga caacagatcc 1200
   ctctgtgcct tggaacccag tggctgtact tcactaccca gcaaagcctc cacgagggca 1260
   gctcgccttg gagagtactt actacaagac ctgcagtcag gccagtgtct gcagctatgg 1320
   gacgatgact tgggagcgct atgggcctgc cccatggaca aatacatcca caagggaggt 1380
   gggggctccg gcgggggtgg aagcggtgga ggcgggtcgg ggggcggagg tagtgagccc 1440
   aaatcttcag acaaaactca cacatgccca ccgtgcccag cacctgaact cctgggggga 1500
   ccgtcagtct tcctcttccc cccaaaaccc aaggacaccc tcatgatctc ccggacccct 1560
   gaggtcacat gcgtggtggt ggacgtgagc cacgaagacc ctgaggtcaa gttcaactgg 1620
   tacgtggacg gcgtggaggt gcataatgcc aagacaaagc cgcgggagga gcagtacaac 1680
   agcacgtacc gtgtggtcag cgtcctcacc gtcctgcacc aggactggct gaatggcaag 1740
   gagtacaagt gcaaggtctc caacaaagcc ctcccagccc ccatcgagaa aaccatctcc 1800
   aaagccaaag ggcagccccg agaaccacag gtgtacaccc tgcccccatc ccgggatgag 1860
   ctgaccaaga accaggtcag cctgacctgc ctggtcaaag gcttctatcc cagcgacatc 1920
   gccgtggagt gggagagcaa tgggcagccg gagaacaact acaagaccac gcctcccgtg 1980
   ctggactccg acggctcctt cttcctctac agcaagctca ccgtggacaa gagcaggtgg 2040
   cagcagggga acgtcttctc atgctccgtg atgcatgagg ctctgcacaa ccactacacg 2100
   cagaagagcc tctccctgtc tccgggtaaa                                  2130
<210> 72
   <211> 710
   <212> PRT
   <213> Artificial Sequence
<220>
   <223> Murine signal peptide and exons 1-6 of murine IL-17RA, exons 8-16 of human IL-17RC, linker and Fc10
<400> 72
   Met Ala Ile Arg Arg Cys Trp Pro Arg Val Val Pro Gly Pro Ala Leu
    1               5                  10                  15
   Gly Trp Leu Leu Leu Leu Leu Asn Val Leu Ala Pro Gly Arg Ala Ser
               20                  25                  30
   Pro Arg Leu Leu Asp Phe Pro Ala Pro Val Cys Ala Gln Glu Gly Leu
           35                  40                  45
   Ser Cys Arg Val Lys Asn Ser Thr Cys Leu Asp Asp Ser Trp Ile His
       50                  55                  60
   Pro Lys Asn Leu Thr Pro Ser Ser Pro Lys Asn Ile Tyr Ile Asn Leu
   65                  70                  75                  80
   Ser Val Ser Ser Thr Gln His Gly Glu Leu Val Pro Val Leu His Val
                   85                  90                  95
   Glu Trp Thr Leu Gln Thr Asp Ala Ser Ile Leu Tyr Leu Glu Gly Ala
               100                 105                 110
   Glu Leu Ser Val Leu Gln Leu Asn Thr Asn Glu Arg Leu Cys Val Lys
           115                 120                 125
   Phe Gln Phe Leu Ser Met Leu Gln His His Arg Lys Arg Trp Arg Phe
       130                 135                 140
   Ser Phe Ser His Phe Val Val Asp Pro Gly Gln Glu Tyr Glu Val Thr
   145                 150                 155                 160
   Val His His Leu Pro Lys Pro Ile Pro Asp Gly Asp Pro Asn His Lys
                   165                 170                 175
   Ser Lys Ile Ile Phe Val Pro Asp Cys Glu Asp Ser Lys Met Lys Met
               180                 185                 190
   Thr Thr Ser Cys Val Ser Ser Ala Leu Pro Trp Leu Asn Val Ser Ala
           195                 200                 205
   Asp Gly Asp Asn Val His Leu Val Leu Asn Val Ser Glu Glu Gln His
       210                 215                 220
   Phe Gly Leu Ser Leu Tyr Trp Asn Gln Val Gln Gly Pro Pro Lys Pro
   225                 230                 235                 240
   Arg Trp His Lys Asn Leu Thr Gly Pro Gln Ile Ile Thr Leu Asn His
                   245                 250                 255
   Thr Asp Leu Val Pro Cys Leu Cys Ile Gln Val Trp Pro Leu Glu Pro
               260                 265                 270
   Asp Ser Val Arg Thr Asn Ile Cys Pro Phe Arg Glu Asp Pro Arg Ala
           275                 280                 285
   His Gln Asn Leu Trp Gln Ala Ala Arg Leu Arg Leu Leu Thr Leu Gln
       290                 295                 300
   Ser Trp Leu Leu Asp Ala Pro Cys Ser Leu Pro Ala Glu Ala Ala Leu
   305                 310                 315                 320
   Cys Trp Arg Ala Pro Gly Gly Asp Pro Cys Gln Pro Leu Val Pro Pro
                   325                 330                 335
   Leu Ser Trp Glu Asn Val Thr Val Asp Lys Val Leu Glu Phe Pro Leu
               340                 345                 350
   Leu Lys Gly His Pro Asn Leu Cys Val Gln Val Asn Ser Ser Glu Lys
           355                 360                 365
   Leu Gln Leu Gln Glu Cys Leu Trp Ala Asp Ser Leu Gly Pro Leu Lys
       370                 375                 380
   Asp Asp Val Leu Leu Leu Glu Thr Arg Gly Pro Gln Asp Asn Arg Ser
   385                 390                 395                 400
   Leu Cys Ala Leu Glu Pro Ser Gly Cys Thr Ser Leu Pro Ser Lys Ala
                   405                 410                 415
   Ser Thr Arg Ala Ala Arg Leu Gly Glu Tyr Leu Leu Gln Asp Leu Gln
               420                 425                 430
   Ser Gly Gln Cys Leu Gln Leu Trp Asp Asp Asp Leu Gly Ala Leu Trp
           435                 440                 445
   Ala Cys Pro Met Asp Lys Tyr Ile His Lys Gly Gly Gly Gly Ser Gly
       450                 455                 460
   Gly Gly Gly Ser Gly Gly Gly Gly Ser Gly Gly Gly Gly Ser Glu Pro
   465                 470                 475                 480
   Lys Ser Ser Asp Lys Thr His Thr Cys Pro Pro Cys Pro Ala Pro Glu
                   485                 490                 495
   Leu Leu Gly Gly Pro Ser Val Phe Leu Phe Pro Pro Lys Pro Lys Asp
               500                 505                 510
   Thr Leu Met Ile Ser Arg Thr Pro Glu Val Thr Cys Val Val Val Asp
           515                 520                 525
   Val Ser His Glu Asp Pro Glu Val Lys Phe Asn Trp Tyr Val Asp Gly
       530                 535                 540
   Val Glu Val His Asn Ala Lys Thr Lys Pro Arg Glu Glu Gln Tyr Asn
   545                 550                 555                 560
   Ser Thr Tyr Arg Val Val Ser Val Leu Thr Val Leu His Gln Asp Trp
                   565                 570                 575
   Leu Asn Gly Lys Glu Tyr Lys Cys Lys Val Ser Asn Lys Ala Leu Pro
               580                 585                 590
   Ala Pro Ile Glu Lys Thr Ile Ser Lys Ala Lys Gly Gln Pro Arg Glu
           595                 600                 605
   Pro Gln Val Tyr Thr Leu Pro Pro Ser Arg Asp Glu Leu Thr Lys Asn
       610                 615                 620
   Gln Val Ser Leu Thr Cys Leu Val Lys Gly Phe Tyr Pro Ser Asp Ile
   625                 630                 635                 640
   Ala Val Glu Trp Glu Ser Asn Gly Gln Pro Glu Asn Asn Tyr Lys Thr
                   645                 650                 655
   Thr Pro Pro Val Leu Asp Ser Asp Gly Ser Phe Phe Leu Tyr Ser Lys
               660                 665                 670
   Leu Thr Val Asp Lys Ser Arg Trp Gln Gln Gly Asn Val Phe Ser Cys
           675                 680                 685
   Ser Val Met His Glu Ala Leu His Asn His Tyr Thr Gln Lys Ser Leu
       690                 695                 700
   Ser Leu Ser Pro Gly Lys
   705                 710
<210> 73
   <211> 1638
   <212> DNA
   <213> Artificial Sequence
<220>
   <223> otPA (optimized tissue Plasminogen Activator) signal peptide and exons 8-16 of human IL-17RC, linker and Fc10
<400> 73
   atggatgcaa tgaagagagg gctctgctgt gtgctgctgc tgtgtggcgc cgtcttcgtt 60
   tcgctcagcc aggaaatcca tgccgagttg agacgcttcc gtagagccct gccctggctc 120
   aacgtgtcag cagatggtga caacgtgcat ctggttctga atgtctctga ggagcagcac 180
   ttcggcctct ccctgtactg gaatcaggtc cagggccccc caaaaccccg gtggcacaaa 240
   aacctgactg gaccgcagat cattaccttg aaccacacag acctggttcc ctgcctctgt 300
   attcaggtgt ggcctctgga acctgactcc gttaggacga acatctgccc cttcagggag 360
   gacccccgcg cacaccagaa cctctggcaa gccgcccgac tgcgactgct gaccctgcag 420
   agctggctgc tggacgcacc gtgctcgctg cccgcagaag cggcactgtg ctggcgggct 480
   ccgggtgggg acccctgcca gccactggtc ccaccgcttt cctgggagaa cgtcactgtg 540
   gacaaggttc tcgagttccc attgctgaaa ggccacccta acctctgtgt tcaggtgaac 600
   agctcggaga agctgcagct gcaggagtgc ttgtgggctg actccctggg gcctctcaaa 660
   gacgatgtgc tactgttgga gacacgaggc ccccaggaca acagatccct ctgtgccttg 720
   gaacccagtg gctgtacttc actacccagc aaagcctcca cgagggcagc tcgccttgga 780
   gagtacttac tacaagacct gcagtcaggc cagtgtctgc agctatggga cgatgacttg 840
   ggagcgctat gggcctgccc catggacaaa tacatccaca agggaggtgg gggctccggc 900
   gggggtggaa gcggtggagg cgggtcgggg ggcggaggta gtgagcccaa atcttcagac 960
   aaaactcaca catgcccacc gtgcccagca cctgaactcc tggggggacc gtcagtcttc 1020
   ctcttccccc caaaacccaa ggacaccctc atgatctccc ggacccctga ggtcacatgc 1080
   gtggtggtgg acgtgagcca cgaagaccct gaggtcaagt tcaactggta cgtggacggc 1140
   gtggaggtgc ataatgccaa gacaaagccg cgggaggagc agtacaacag cacgtaccgt 1200
   gtggtcagcg tcctcaccgt cctgcaccag gactggctga atggcaagga gtacaagtgc 1260
   aaggtctcca acaaagccct cccagccccc atcgagaaaa ccatctccaa agccaaaggg 1320
   cagccccgag aaccacaggt gtacaccctg cccccatccc gggatgagct gaccaagaac 1380
   caggtcagcc tgacctgcct ggtcaaaggc ttctatccca gcgacatcgc cgtggagtgg 1440
   gagagcaatg ggcagccgga gaacaactac aagaccacgc ctcccgtgct ggactccgac 1500
   ggctccttct tcctctacag caagctcacc gtggacaaga gcaggtggca gcaggggaac 1560
   gtcttctcat gctccgtgat gcatgaggct ctgcacaacc actacacgca gaagagcctc 1620
   tccctgtctc cgggtaaa 1638
<210> 74
   <211> 546
   <212> PRT
   <213> Artificial Sequence
<220>
   <223> otPA (optimized tissue Plasminogen Activator) signal peptide and exons 8-16 of human IL-17RC, linker and Fc10
<400> 74
   Met Asp Ala Met Lys Arg Gly Leu Cys Cys Val Leu Leu Leu Cys Gly
    1               5                  10                  15
   Ala Val Phe Val Ser Leu Ser Gln Glu Ile His Ala Glu Leu Arg Arg
               20                  25                  30
   Phe Arg Arg Ala Leu Pro Trp Leu Asn Val Ser Ala Asp Gly Asp Asn
           35                  40                  45
   Val His Leu Val Leu Asn Val Ser Glu Glu Gln His Phe Gly Leu Ser
       50                  55                  60
   Leu Tyr Trp Asn Gln Val Gln Gly Pro Pro Lys Pro Arg Trp His Lys
   65                  70                  75                  80
   Asn Leu Thr Gly Pro Gln Ile Ile Thr Leu Asn His Thr Asp Leu Val
                   85                  90                  95
   Pro Cys Leu Cys Ile Gln Val Trp Pro Leu Glu Pro Asp Ser Val Arg
               100                 105                 110
   Thr Asn Ile Cys Pro Phe Arg Glu Asp Pro Arg Ala His Gln Asn Leu
           115                 120                 125
   Trp Gln Ala Ala Arg Leu Arg Leu Leu Thr Leu Gln Ser Trp Leu Leu
       130                 135                 140
   Asp Ala Pro Cys Ser Leu Pro Ala Glu Ala Ala Leu Cys Trp Arg Ala
   145                 150                 155                 160
   Pro Gly Gly Asp Pro Cys Gln Pro Leu Val Pro Pro Leu Ser Trp Glu
                   165                 170                 175
   Asn Val Thr Val Asp Lys Val Leu Glu Phe Pro Leu Leu Lys Gly His
               180                 185                 190
   Pro Asn Leu Cys Val Gln Val Asn Ser Ser Glu Lys Leu Gln Leu Gln
           195                 200                 205
   Glu Cys Leu Trp Ala Asp Ser Leu Gly Pro Leu Lys Asp Asp Val Leu
       210                 215                 220
   Leu Leu Glu Thr Arg Gly Pro Gln Asp Asn Arg Ser Leu Cys Ala Leu
   225                 230                 235                 240
   Glu Pro Ser Gly Cys Thr Ser Leu Pro Ser Lys Ala Ser Thr Arg Ala
                   245                 250                 255
   Ala Arg Leu Gly Glu Tyr Leu Leu Gln Asp Leu Gln Ser Gly Gln Cys
               260                 265                 270
   Leu Gln Leu Trp Asp Asp Asp Leu Gly Ala Leu Trp Ala Cys Pro Met
           275                 280                 285
   Asp Lys Tyr Ile His Lys Gly Gly Gly Gly Ser Gly Gly Gly Gly Ser
       290                 295                 300
   Gly Gly Gly Gly Ser Gly Gly Gly Gly Ser Glu Pro Lys Ser Ser Asp
   305                 310                 315                 320
   Lys Thr His Thr Cys Pro Pro Cys Pro Ala Pro Glu Leu Leu Gly Gly
                   325                 330                 335
   Pro Ser Val Phe Leu Phe Pro Pro Lys Pro Lys Asp Thr Leu Met Ile
               340                 345                 350
   Ser Arg Thr Pro Glu Val Thr Cys Val Val Val Asp Val Ser His Glu
           355                 360                 365
   Asp Pro Glu Val Lys Phe Asn Trp Tyr Val Asp Gly Val Glu Val His
       370                 375                 380
   Asn Ala Lys Thr Lys Pro Arg Glu Glu Gln Tyr Asn Ser Thr Tyr Arg
   385                 390                 395                 400
   Val Val Ser Val Leu Thr Val Leu His Gln Asp Trp Leu Asn Gly Lys
                   405                 410                 415
   Glu Tyr Lys Cys Lys Val Ser Asn Lys Ala Leu Pro Ala Pro Ile Glu
               420                 425                 430
   Lys Thr Ile Ser Lys Ala Lys Gly Gln Pro Arg Glu Pro Gln Val Tyr
           435                 440                 445
   Thr Leu Pro Pro Ser Arg Asp Glu Leu Thr Lys Asn Gln Val Ser Leu
       450                 455                 460
   Thr Cys Leu Val Lys Gly Phe Tyr Pro Ser Asp Ile Ala Val Glu Trp
   465                 470                 475                 480
   Glu Ser Asn Gly Gln Pro Glu Asn Asn Tyr Lys Thr Thr Pro Pro Val
                   485                 490                 495
   Leu Asp Ser Asp Gly Ser Phe Phe Leu Tyr Ser Lys Leu Thr Val Asp
               500                 505                 510
   Lys Ser Arg Trp Gln Gln Gly Asn Val Phe Ser Cys Ser Val Met His
           515                 520                 525
   Glu Ala Leu His Asn His Tyr Thr Gln Lys Ser Leu Ser Leu Ser Pro
       530                 535                 540
   Gly Lys
   545
<210> 75
   <211> 622
   <212> DNA
   <213> homo sapians
<400> 75
   atgcctgtgc cctggttctt gctgtccttg gcactgggcc gaagcccagt ggtcctttct 60
   ctggagaggc ttgtggggcc tcaggacgct acccactgct ctccgggcct ctcctgccgc 120
   ctctgggaca gtgacatact ctgcctgcct ggggacatcg tgcctgctcc gggccccgtg 180
   ctggcgccta cgcacctgca gacagagctg gtgctgaggt gccagaagga gaccgactgt 240
   gacctctgtc tgcgtgtggc tgtccacttg gccgtgcatg ggcactggga agagcctgaa 300
   gatgaggaaa agtttggagg agcagctgac tcaggggtgg aggagcctag gaatgcctct 360
   ctccaggccc aagtcgtgct ctccttccag gcctacccta ctgcccgctg cgtcctgctg 420
   gaggtgcaag tgcctgctgc ccttgtgcag tttggtcagt ctgtgggctc tgtggtatat 480
   gactgcttcg aggctgccct agggagtgag gtacgaatct ggtcctatac tcagcccagg 540
   tacgagaagg aactcaacca cacacagcag ctgcctgact gcagggggct cgaagtctgg 600
   aacagcatcc cgagctgctg gg                                        622
<210> 76
   <211> 207
   <212> PRT
   <213> homo sapians
<400> 76
   Met Pro Val Pro Trp Phe Leu Leu Ser Leu Ala Leu Gly Arg Ser Pro
   1               5                   10                  15
   Val Val Leu Ser Leu Glu Arg Leu Val Gly Pro Gln Asp Ala Thr His
               20                  25                  30
   Cys Ser Pro Gly Leu Ser Cys Arg Leu Trp Asp Ser Asp Ile Leu Cys
           35                  40                  45
   Leu Pro Gly Asp Ile Val Pro Ala Pro Gly Pro Val Leu Ala Pro Thr
       50                  55                  60
   His Leu Gln Thr Glu Leu Val Leu Arg Cys Gln Lys Glu Thr Asp Cys
   65                  70                  75                  80
   Asp Leu Cys Leu Arg Val Ala Val His Leu Ala Val His Gly His Trp
                   85                  90                  95
   Glu Glu Pro Glu Asp Glu Glu Lys Phe Gly Gly Ala Ala Asp Ser Gly
               100                 105                 110
   Val Glu Glu Pro Arg Asn Ala Ser Leu Gln Ala Gln Val Val Leu Ser
           115                 120                 125
   Phe Gln Ala Tyr Pro Thr Ala Arg Cys Val Leu Leu Glu Val Gln Val
       130                 135                 140
   Pro Ala Ala Leu Val Gln Phe Gly Gln Ser Val Gly Ser Val Val Tyr
   145                 150                 155                 160
   Asp Cys Phe Glu Ala Ala Leu Gly Ser Glu Val Arg Ile Trp Ser Tyr
                   165                 170                 175
   Thr Gln Pro Arg Tyr Glu Lys Glu Leu Asn His Thr Gln Gln Leu Pro
               180                 185                 190
   Asp Cys Arg Gly Leu Glu Val Trp Asn Ser Ile Pro Ser Cys Trp
           195                 200                 205
<210> 77
   <211> 1318
   <212> DNA
   <213> Artificial Sequence
<220>
<223> IL-17RC signal peptide and exons 1-7 of human IL-17RC, and Fc5
<400> 77
   atgcctgtgc cctggttctt gctgtccttg gcactgggcc gaagcccagt ggtcctttct 60
   ctggagaggc ttgtggggcc tcaggacgct acccactgct ctccgggcct ctcctgccgc 120
   ctctgggaca gtgacatact ctgcctgcct ggggacatcg tgcctgctcc gggccccgtg 180
   ctggcgccta cgcacctgca gacagagctg gtgctgaggt gccagaagga gaccgactgt 240
   gacctctgtc tgcgtgtggc tgtccacttg gccgtgcatg ggcactggga agagcctgaa 300
   gatgaggaaa agtttggagg agcagctgac tcaggggtgg aggagcctag gaatgcctct 360
   ctccaggccc aagtcgtgct ctccttccag gcctacccta ctgcccgctg cgtcctgctg 420
   gaggtgcaag tgcctgctgc ccttgtgcag tttggtcagt ctgtgggctc tgtggtatat 480
   gactgcttcg aggctgccct agggagtgag gtacgaatct ggtcctatac tcagcccagg 540
   tacgagaagg aactcaacca cacacagcag ctgcctgact gcagggggct cgaagtctgg 600
   aacagcatcc cgagctgctg gggagcccaa atcttcagac aaaactcaca catgcccacc 660
   gtgcccagca cctgaagccg agggggcacc gtcagtcttc ctcttccccc caaaacccaa 720
   ggacaccctc atgatctccc ggacccctga ggtcacatgc gtggtggtgg acgtgagcca 780
   cgaagaccct gaggtcaagt tcaactggta cgtggacggc gtggaggtgc ataatgccaa 840
   gacaaagccg cgggaggagc agtacaacag cacgtaccgt gtggtcagcg tcctcaccgt 900
   cctgcaccag gactggctga atggcaagga gtacaagtgc aaggtctcca acaaagccct 960
   cccatcctcc atcgagaaaa ccatctccaa agccaaaggg cagccccgag aaccacaggt 1020
   gtacaccctg cccccatccc gggatgagct gaccaagaac caggtcagcc tgacctgcct 1080
   ggtcaaaggc ttctatccca gcgacatcgc cgtggagtgg gagagcaatg ggcagccgga 1140
   gaacaactac aagaccacgc ctcccgtgct ggactccgac ggctccttct tcctctacag 1200
   caagctcacc gtggacaaga gcaggtggca gcaggggaac gtcttctcat gctccgtgat 1260
   gcatgaggct ctgcacaacc actacacgca gaagagcctc tccctgtctc cgggtaaa   1318
<210> 78
   <211> 439
   <212> PRT
   <213> Artificial Sequence
<220>
   <223> IL-17RC signal peptide and exons 1-7 of human IL-17RC, and Fc5
<400> 78
   Met Pro Val Pro Trp Phe Leu Leu Ser Leu Ala Leu Gly Arg Ser Pro
   1 5 10 15
   Val Val Leu Ser Leu Glu Arg Leu Val Gly Pro Gln Asp Ala Thr His
   20 25 30
   Cys Ser Pro Gly Leu Ser Cys Arg Leu Trp Asp Ser Asp Ile Leu Cys
   35 40 45
   Leu Pro Gly Asp Ile Val Pro Ala Pro Gly Pro Val Leu Ala Pro Thr
   50 55 60
   His Leu Gln Thr Glu Leu Val Leu Arg Cys Gln Lys Glu Thr Asp Cys
   65 70 75 80
   Asp Leu Cys Leu Arg Val Ala Val His Leu Ala Val His Gly His Trp
   85 90 95
   Glu Glu Pro Glu Asp Glu Glu Lys Phe Gly Gly Ala Ala Asp Ser Gly
   100 105 110
   Val Glu Glu Pro Arg Asn Ala Ser Leu Gln Ala Gln Val Val Leu Ser
   115 120 125
   Phe Gln Ala Tyr Pro Thr Ala Arg Cys Val Leu Leu Glu Val Gln Val
   130 135 140
   Pro Ala Ala Leu Val Gln Phe Gly Gln Ser Val Gly Ser Val Val Tyr
   145 150 155 160
   Asp Cys Phe Glu Ala Ala Leu Gly Ser Glu Val Arg Ile Trp Ser Tyr
   165 170 175
   Thr Gln Pro Arg Tyr Glu Lys Glu Leu Asn His Thr Gln Gln Leu Pro
   180 185 190
   Asp Cys Arg Gly Leu Glu Val Trp Asn Ser Ile Pro Ser Cys Trp Glu
   195 200 205
   Pro Lys Ser Ser Asp Lys Thr His Thr Cys Pro Pro Cys Pro Ala Pro
   210 215 220
   Glu Ala Glu Gly Ala Pro Ser Val Phe Leu Phe Pro Pro Lys Pro Lys
   225 230 235 240
   Asp Thr Leu Met Ile Ser Arg Thr Pro Glu Val Thr Cys Val Val Val
   245 250 255
   Asp Val Ser His Glu Asp Pro Glu Val Lys Phe Asn Trp Tyr Val Asp
   260 265 270
   Gly Val Glu Val His Asn Ala Lys Thr Lys Pro Arg Glu Glu Gln Tyr
   275 280 285
   Asn Ser Thr Tyr Arg Val Val Ser Val Leu Thr Val Leu His Gln Asp
   290 295 300
   Trp Leu Asn Gly Lys Glu Tyr Lys Cys Lys Val Ser Asn Lys Ala Leu
   305 310 315 320
   Pro Ser Ser Ile Glu Lys Thr Ile Ser Lys Ala Lys Gly Gln Pro Arg
   325 330 335
   Glu Pro Gln Val Tyr Thr Leu Pro Pro Ser Arg Asp Glu Leu Thr Lys
   340 345 350
   Asn Gln Val Ser Leu Thr Cys Leu Val Lys Gly Phe Tyr Pro Ser Asp
   355 360 365
   Ile Ala Val Glu Trp Glu Ser Asn Gly Gln Pro Glu Asn Asn Tyr Lys
   370 375 380
   Thr Thr Pro Pro Val Leu Asp Ser Asp Gly Ser Phe Phe Leu Tyr Ser
   385 390 395 400
   Lys Leu Thr Val Asp Lys Ser Arg Trp Gln Gln Gly Asn Val Phe Ser
   405 410 415
   Cys Ser Val Met His Glu Ala Leu His Asn His Tyr Thr Gln Lys Ser
   420 425 430
   Leu Ser Leu Ser Pro Gly Lys
   435
<210> 79
   <211> 762
   <212> DNA
   <213> homo sapians
<400> 79
   atgcctgtgc cctggttctt gctgtccttg gcactgggcc gaagcccagt ggtcctttct 60
   ctggagaggc ttgtggggcc tcaggacgct acccactgct ctccgggcct ctcctgccgc 120
   ctctgggaca gtgacatact ctgcctgcct ggggacatcg tgcctgctcc gggccccgtg 180
   ctggcgccta cgcacctgca gacagagctg gtgctgaggt gccagaagga gaccgactgt 240
   gacctctgtc tgcgtgtggc tgtccacttg gccgtgcatg ggcactggga agagcctgaa 300
   gatgaggaaa agtttggagg agcagctgac tcaggggtgg aggagcctag gaatgcctct 360
   ctccaggccc aagtcgtgct ctccttccag gcctacccta ctgcccgctg cgtcctgctg 420
   gaggtgcaag tgcctgctgc ccttgtgcag tttggtcagt ctgtgggctc tgtggtatat 480
   gactgcttcg aggctgccct agggagtgag gtacgaatct ggtcctatac tcagcccagg 540
   tacgagaagg aactcaacca cacacagcag ctgcctgact gcagggggct cgaagtctgg 600
   aacagcatcc cgagctgctg ggccctgccc tggctcaacg tgtcagcaga tggtgacaac 660
   gtgcatctgg ttctgaatgt ctctgaggag cagcacttcg gcctctccct gtactggaat 720
   caggtccagg gccccccaaa accccggtgg cacaaaaacc tg                  762
<210> 80
   <211> 254
   <212> PRT
   <213> homo sapians
<400> 80
   Met Pro Val Pro Trp Phe Leu Leu Ser Leu Ala Leu Gly Arg Ser Pro
   1               5                   10                  15
   Val Val Leu Ser Leu Glu Arg Leu Val Gly Pro Gln Asp Ala Thr His
               20                  25                  30
   Cys Ser Pro Gly Leu Ser Cys Arg Leu Trp Asp Ser Asp Ile Leu Cys
           35                  40                  45
   Leu Pro Gly Asp Ile Val Pro Ala Pro Gly Pro Val Leu Ala Pro Thr
       50                  55                  60
   His Leu Gln Thr Glu Leu Val Leu Arg Cys Gln Lys Glu Thr Asp Cys
   65                  70                  75                  80
   Asp Leu Cys Leu Arg Val Ala Val His Leu Ala Val His Gly His Trp
                   85                  90                  95
   Glu Glu Pro Glu Asp Glu Glu Lys Phe Gly Gly Ala Ala Asp Ser Gly
               100                 105                 110
   Val Glu Glu Pro Arg Asn Ala Ser Leu Gln Ala Gln Val Val Leu Ser
           115                 120                 125
   Phe Gln Ala Tyr Pro Thr Ala Arg Cys Val Leu Leu Glu Val Gln Val
       130                 135                 140
   Pro Ala Ala Leu Val Gln Phe Gly Gln Ser Val Gly Ser Val Val Tyr
   145                 150                 155                 160
   Asp Cys Phe Glu Ala Ala Leu Gly Ser Glu Val Arg Ile Trp Ser Tyr
                   165                 170                 175
   Thr Gln Pro Arg Tyr Glu Lys Glu Leu Asn His Thr Gln Gln Leu Pro
               180                 185                 190
   Asp Cys Arg Gly Leu Glu Val Trp Asn Ser Ile Pro Ser Cys Trp Ala
           195                 200                 205
   Leu Pro Trp Leu Asn Val Ser Ala Asp Gly Asp Asn Val His Leu Val
       210                 215                 220
   Leu Asn Val Ser Glu Glu Gln His Phe Gly Leu Ser Leu Tyr Trp Asn
   225                 230                 235                 240
   Gln Val Gln Gly Pro Pro Lys Pro Arg Trp His Lys Asn Leu
                   245                 250
<210> 81
   <211> 1458
   <212> DNA
   <213> Artificial Sequence
<220>
   <223> IL-17RC signal peptide and exons 1-8 of human IL-17RC, and Fc5
<400> 81
   atgcctgtgc cctggttctt gctgtccttg gcactgggcc gaagcccagt ggtcctttct 60
   ctggagaggc ttgtggggcc tcaggacgct acccactgct ctccgggcct ctcctgccgc 120
   ctctgggaca gtgacatact ctgcctgcct ggggacatcg tgcctgctcc gggccccgtg 180
   ctggcgccta cgcacctgca gacagagctg gtgctgaggt gccagaagga gaccgactgt 240
   gacctctgtc tgcgtgtggc tgtccacttg gccgtgcatg ggcactggga agagcctgaa 300
   gatgaggaaa agtttggagg agcagctgac tcaggggtgg aggagcctag gaatgcctct 360
   ctccaggccc aagtcgtgct ctccttccag gcctacccta ctgcccgctg cgtcctgctg 420
   gaggtgcaag tgcctgctgc ccttgtgcag tttggtcagt ctgtgggctc tgtggtatat 480
   gactgcttcg aggctgccct agggagtgag gtacgaatct ggtcctatac tcagcccagg 540
   tacgagaagg aactcaacca cacacagcag ctgcctgact gcagggggct cgaagtctgg 600
   aacagcatcc cgagctgctg ggccctgccc tggctcaacg tgtcagcaga tggtgacaac 660
   gtgcatctgg ttctgaatgt ctctgaggag cagcacttcg gcctctccct gtactggaat 720
   caggtccagg gccccccaaa accccggtgg cacaaaaacc tggagcccaa atcttcagac 780
   aaaactcaca catgcccacc gtgcccagca cctgaagccg agggggcacc gtcagtcttc 840
   ctcttccccc caaaacccaa ggacaccctc atgatctccc ggacccctga ggtcacatgc 900
   gtggtggtgg acgtgagcca cgaagaccct gaggtcaagt tcaactggta cgtggacggc 960
   gtggaggtgc ataatgccaa gacaaagccg cgggaggagc agtacaacag cacgtaccgt 1020
   gtggtcagcg tcctcaccgt cctgcaccag gactggctga atggcaagga gtacaagtgc 1080
   aaggtctcca acaaagccct cccatcctcc atcgagaaaa ccatctccaa agccaaaggg 1140
   cagccccgag aaccacaggt gtacaccctg cccccatccc gggatgagct gaccaagaac 1200
   caggtcagcc tgacctgcct ggtcaaaggc ttctatccca gcgacatcgc cgtggagtgg 1260
   gagagcaatg ggcagccgga gaacaactac aagaccacgc ctcccgtgct ggactccgac 1320
   ggctccttct tcctctacag caagctcacc gtggacaaga gcaggtggca gcaggggaac 1380
   gtcttctcat gctccgtgat gcatgaggct ctgcacaacc actacacgca gaagagcctc 1440
   tccctgtctc cgggtaaa                                               1458
<210> 82
   <211> 486
   <212> PRT
   <213> Artificial Sequence
<220>
   <223> IL-17RC signal peptide and exons 1-8 of human IL-17RC, and Fc5
<400> 82
   Met Pro Val Pro Trp Phe Leu Leu Ser Leu Ala Leu Gly Arg Ser Pro
    1               5                  10                  15
   Val Val Leu Ser Leu Glu Arg Leu Val Gly Pro Gln Asp Ala Thr His
               20                  25                  30
   Cys Ser Pro Gly Leu Ser Cys Arg Leu Trp Asp Ser Asp Ile Leu Cys
           35                  40                  45
   Leu Pro Gly Asp Ile Val Pro Ala Pro Gly Pro Val Leu Ala Pro Thr
       50                  55                  60
   His Leu Gln Thr Glu Leu Val Leu Arg Cys Gln Lys Glu Thr Asp Cys
   65                  70                  75                  80
   Asp Leu Cys Leu Arg Val Ala Val His Leu Ala Val His Gly His Trp
                   85                  90                  95
   Glu Glu Pro Glu Asp Glu Glu Lys Phe Gly Gly Ala Ala Asp Ser Gly
               100                 105                 110
   Val Glu Glu Pro Arg Asn Ala Ser Leu Gln Ala Gln Val Val Leu Ser
           115                 120                 125
   Phe Gln Ala Tyr Pro Thr Ala Arg Cys Val Leu Leu Glu Val Gln Val
       130                 135                 140
   Pro Ala Ala Leu Val Gln Phe Gly Gln Ser Val Gly Ser Val Val Tyr
   145                 150                 155                 160
   Asp Cys Phe Glu Ala Ala Leu Gly Ser Glu Val Arg Ile Trp Ser Tyr
                   165                 170                 175
   Thr Gln Pro Arg Tyr Glu Lys Glu Leu Asn His Thr Gln Gln Leu Pro
               180                 185                 190
   Asp Cys Arg Gly Leu Glu Val Trp Asn Ser Ile Pro Ser Cys Trp Ala
           195                 200                 205
   Leu Pro Trp Leu Asn Val Ser Ala Asp Gly Asp Asn Val His Leu Val
       210                 215                 220
   Leu Asn Val Ser Glu Glu Gln His Phe Gly Leu Ser Leu Tyr Trp Asn
   225                 230                 235                 240
   Gln Val Gln Gly Pro Pro Lys Pro Arg Trp His Lys Asn Leu Glu Pro
                   245                 250                 255
   Lys Ser Ser Asp Lys Thr His Thr Cys Pro Pro Cys Pro Ala Pro Glu
               260                 265                 270
   Ala Glu Gly Ala Pro Ser Val Phe Leu Phe Pro Pro Lys Pro Lys Asp
           275                 280                 285
   Thr Leu Met Ile Ser Arg Thr Pro Glu Val Thr Cys Val Val Val Asp
       290                 295                 300
   Val Ser His Glu Asp Pro Glu Val Lys Phe Asn Trp Tyr Val Asp Gly
   305                 310                 315                 320
   Val Glu Val His Asn Ala Lys Thr Lys Pro Arg Glu Glu Gln Tyr Asn
                   325                 330                 335
   Ser Thr Tyr Arg Val Val Ser Val Leu Thr Val Leu His Gln Asp Trp
               340                 345                 350
   Leu Asn Gly Lys Glu Tyr Lys Cys Lys Val Ser Asn Lys Ala Leu Pro
           355                 360                 365
   Ser Ser Ile Glu Lys Thr Ile Ser Lys Ala Lys Gly Gln Pro Arg Glu
       370                 375                 380
   Pro Gln Val Tyr Thr Leu Pro Pro Ser Arg Asp Glu Leu Thr Lys Asn
   385                 390                 395                 400
   Gln Val Ser Leu Thr Cys Leu Val Lys Gly Phe Tyr Pro Ser Asp Ile
                   405                 410                 415
   Ala Val Glu Trp Glu Ser Asn Gly Gln Pro Glu Asn Asn Tyr Lys Thr
               420                 425                 430
   Thr Pro Pro Val Leu Asp Ser Asp Gly Ser Phe Phe Leu Tyr Ser Lys
           435                 440                 445
   Leu Thr Val Asp Lys Ser Arg Trp Gln Gln Gly Asn Val Phe Ser Cys
       450                 455                 460
   Ser Val Met His Glu Ala Leu His Asn His Tyr Thr Gln Lys Ser Leu
   465                 470                 475                 480
   Ser Leu Ser Pro Gly Lys
                   485
<210> 83
   <211> 822
   <212> DNA
   <213> homo sapians
<400> 83 <210> 84
   <211> 274
   <212> PRT
   <213> homo sapians
<400> 84
<210> 85
   <211> 1518
   <212> DNA
   <213> Artificial Sequence
<220>
   <223> IL-17RC signal peptide and exons 1-9 of human IL-17RC, and Fc5
<400> 85
   atgcctgtgc cctggttctt gctgtccttg gcactgggcc gaagcccagt ggtcctttct 60
   ctggagaggc ttgtggggcc tcaggacgct acccactgct ctccgggcct ctcctgccgc 120
   ctctgggaca gtgacatact ctgcctgcct ggggacatcg tgcctgctcc gggccccgtg 180
   ctggcgccta cgcacctgca gacagagctg gtgctgaggt gccagaagga gaccgactgt 240
   gacctctgtc tgcgtgtggc tgtccacttg gccgtgcatg ggcactggga agagcctgaa 300
   gatgaggaaa agtttggagg agcagctgac tcaggggtgg aggagcctag gaatgcctct 360
   ctccaggccc aagtcgtgct ctccttccag gcctacccta ctgcccgctg cgtcctgctg 420
   gaggtgcaag tgcctgctgc ccttgtgcag tttggtcagt ctgtgggctc tgtggtatat 480
   gactgcttcg aggctgccct agggagtgag gtacgaatct ggtcctatac tcagcccagg 540
   tacgagaagg aactcaacca cacacagcag ctgcctgact gcagggggct cgaagtctgg 600
   aacagcatcc cgagctgctg ggccctgccc tggctcaacg tgtcagcaga tggtgacaac 660
   gtgcatctgg ttctgaatgt ctctgaggag cagcacttcg gcctctccct gtactggaat 720
   caggtccagg gccccccaaa accccggtgg cacaaaaacc tgactggacc gcagatcatt 780
   accttgaacc acacagacct ggttccctgc ctctgtattc aggagcccaa atcttcagac 840
   aaaactcaca catgcccacc gtgcccagca cctgaagccg agggggcacc gtcagtcttc 900
   ctcttccccc caaaacccaa ggacaccctc atgatctccc ggacccctga ggtcacatgc 960
   gtggtggtgg acgtgagcca cgaagaccct gaggtcaagt tcaactggta cgtggacggc 1020
   gtggaggtgc ataatgccaa gacaaagccg cgggaggagc agtacaacag cacgtaccgt 1080
   gtggtcagcg tcctcaccgt cctgcaccag gactggctga atggcaagga gtacaagtgc 1140
   aaggtctcca acaaagccct cccatcctcc atcgagaaaa ccatctccaa agccaaaggg 1200
   cagccccgag aaccacaggt gtacaccctg cccccatccc gggatgagct gaccaagaac 1260
   caggtcagcc tgacctgcct ggtcaaaggc ttctatccca gcgacatcgc cgtggagtgg 1320
   gagagcaatg ggcagccgga gaacaactac aagaccacgc ctcccgtgct ggactccgac 1380
   ggctccttct tcctctacag caagctcacc gtggacaaga gcaggtggca gcaggggaac 1440
   gtcttctcat gctccgtgat gcatgaggct ctgcacaacc actacacgca gaagagcctc 1500
   tccctgtctc cgggtaaa                                               1518
<210> 86
   <211> 506
   <212> PRT
   <213> Artificial Sequence
<220>
   <223> IL-17RC signal peptide and exons 1-9 of human IL-17RC, and Fc5
<400> 86
   Met Pro Val Pro Trp Phe Leu Leu Ser Leu Ala Leu Gly Arg Ser Pro
    1               5                  10                  15
   Val Val Leu Ser Leu Glu Arg Leu Val Gly Pro Gln Asp Ala Thr His
               20                  25                  30
   Cys Ser Pro Gly Leu Ser Cys Arg Leu Trp Asp Ser Asp Ile Leu Cys
           35                  40                  45
   Leu Pro Gly Asp Ile Val Pro Ala Pro Gly Pro Val Leu Ala Pro Thr
       50                  55                  60
   His Leu Gln Thr Glu Leu Val Leu Arg Cys Gln Lys Glu Thr Asp Cys
   65                  70                  75                  80
   Asp Leu Cys Leu Arg Val Ala Val His Leu Ala Val His Gly His Trp
                   85                  90                  95
   Glu Glu Pro Glu Asp Glu Glu Lys Phe Gly Gly Ala Ala Asp Ser Gly
               100                 105                 110
   Val Glu Glu Pro Arg Asn Ala Ser Leu Gln Ala Gln Val Val Leu Ser
           115                 120                 125
   Phe Gln Ala Tyr Pro Thr Ala Arg Cys Val Leu Leu Glu Val Gln Val
       130                 135                 140
   Pro Ala Ala Leu Val Gln Phe Gly Gln Ser Val Gly Ser Val Val Tyr
   145                 150                 155                 160
   Asp Cys Phe Glu Ala Ala Leu Gly Ser Glu Val Arg Ile Trp Ser Tyr
                   165                 170                 175
   Thr Gln Pro Arg Tyr Glu Lys Glu Leu Asn His Thr Gln Gln Leu Pro
               180                 185                 190
   Asp Cys Arg Gly Leu Glu Val Trp Asn Ser Ile Pro Ser Cys Trp Ala
           195                 200                 205
   Leu Pro Trp Leu Asn Val Ser Ala Asp Gly Asp Asn Val His Leu Val
       210                 215                 220
   Leu Asn Val Ser Glu Glu Gln His Phe Gly Leu Ser Leu Tyr Trp Asn
   225                 230                 235                 240
   Gln Val Gln Gly Pro Pro Lys Pro Arg Trp His Lys Asn Leu Thr Gly
                   245                 250                 255
   Pro Gln Ile Ile Thr Leu Asn His Thr Asp Leu Val Pro Cys Leu Cys
               260                 265                 270
   Ile Gln Glu Pro Lys Ser Ser Asp Lys Thr His Thr Cys Pro Pro Cys
           275                 280                 285
   Pro Ala Pro Glu Ala Glu Gly Ala Pro Ser Val Phe Leu Phe Pro Pro
       290                 295                 300
   Lys Pro Lys Asp Thr Leu Met Ile Ser Arg Thr Pro Glu Val Thr Cys
   305                 310                 315                 320
   Val Val Val Asp Val Ser His Glu Asp Pro Glu Val Lys Phe Asn Trp
                   325                 330                 335
   Tyr Val Asp Gly Val Glu Val His Asn Ala Lys Thr Lys Pro Arg Glu
               340                 345                 350
   Glu Gln Tyr Asn Ser Thr Tyr Arg Val Val Ser Val Leu Thr Val Leu
           355                 360                 365
   His Gln Asp Trp Leu Asn Gly Lys Glu Tyr Lys Cys Lys Val Ser Asn
       370                 375                 380
   Lys Ala Leu Pro Ser Ser Ile Glu Lys Thr Ile Ser Lys Ala Lys Gly
   385                 390                 395                 400
   Gln Pro Arg Glu Pro Gln Val Tyr Thr Leu Pro Pro Ser Arg Asp Glu
                   405                 410                 415
   Leu Thr Lys Asn Gln Val Ser Leu Thr Cys Leu Val Lys Gly Phe Tyr
               420                 425                 430
   Pro Ser Asp Ile Ala Val Glu Trp Glu Ser Asn Gly Gln Pro Glu Asn
           435                 440                 445
   Asn Tyr Lys Thr Thr Pro Pro Val Leu Asp Ser Asp Gly Ser Phe Phe
       450                 455                 460
   Leu Tyr Ser Lys Leu Thr Val Asp Lys Ser Arg Trp Gln Gln Gly Asn
   465                 470                 475                 480
   Val Phe Ser Cys Ser Val Met His Glu Ala Leu His Asn His Tyr Thr
                   485                 490                 495
   Gln Lys Ser Leu Ser Leu Ser Pro Gly Lys
               500                 505
<210> 87
   <211> 873
   <212> DNA
   <213> homo sapians
<400> 87
   atgcctgtgc cctggttctt gctgtccttg gcactgggcc gaagcccagt ggtcctttct 60
   ctggagaggc ttgtggggcc tcaggacgct acccactgct ctccgggcct ctcctgccgc 120
   ctctgggaca gtgacatact ctgcctgcct ggggacatcg tgcctgctcc gggccccgtg 180
   ctggcgccta cgcacctgca gacagagctg gtgctgaggt gccagaagga gaccgactgt 240
   gacctctgtc tgcgtgtggc tgtccacttg gccgtgcatg ggcactggga agagcctgaa 300
   gatgaggaaa agtttggagg agcagctgac tcaggggtgg aggagcctag gaatgcctct 360
   ctccaggccc aagtcgtgct ctccttccag gcctacccta ctgcccgctg cgtcctgctg 420
   gaggtgcaag tgcctgctgc ccttgtgcag tttggtcagt ctgtgggctc tgtggtatat 480
   gactgcttcg aggctgccct agggagtgag gtacgaatct ggtcctatac tcagcccagg 540
   tacgagaagg aactcaacca cacacagcag ctgcctgact gcagggggct cgaagtctgg 600
   aacagcatcc cgagctgctg ggccctgccc tggctcaacg tgtcagcaga tggtgacaac 660
   gtgcatctgg ttctgaatgt ctctgaggag cagcacttcg gcctctccct gtactggaat 720
   caggtccagg gccccccaaa accccggtgg cacaaaaacc tgactggacc gcagatcatt 780
   accttgaacc acacagacct ggttccctgc ctctgtattc aggtgtggcc tctggaacct 840
   gactccgtta ggacgaacat ctgccccttc agg                              873
<210> 88
   <211> 291
   <212> PRT
   <213> homo sapians
<400> 88
   Met Pro Val Pro Trp Phe Leu Leu Ser Leu Ala Leu Gly Arg Ser Pro
   1 5 10 15
   Val Val Leu Ser Leu Glu Arg Leu Val Gly Pro Gln Asp Ala Thr His
   20 25 30
   Cys Ser Pro Gly Leu Ser Cys Arg Leu Trp Asp Ser Asp Ile Leu Cys
   35 40 45
   Leu Pro Gly Asp Ile Val Pro Ala Pro Gly Pro Val Leu Ala Pro Thr
   50 55 60
   His Leu Gln Thr Glu Leu Val Leu Arg Cys Gln Lys Glu Thr Asp Cys
   65 70 75 80
   Asp Leu Cys Leu Arg Val Ala Val His Leu Ala Val His Gly His Trp
   85 90 95
   Glu Glu Pro Glu Asp Glu Glu Lys Phe Gly Gly Ala Ala Asp Ser Gly
   100 105 110
   Val Glu Glu Pro Arg Asn Ala Ser Leu Gln Ala Gln Val Val Leu Ser
   115 120 125
   Phe Gln Ala Tyr Pro Thr Ala Arg Cys Val Leu Leu Glu Val Gln Val
   130 135 140
   Pro Ala Ala Leu Val Gln Phe Gly Gln Ser Val Gly Ser Val Val Tyr
   145 150 155 160
   Asp Cys Phe Glu Ala Ala Leu Gly Ser Glu Val Arg Ile Trp Ser Tyr
   165 170 175
   Thr Gln Pro Arg Tyr Glu Lys Glu Leu Asn His Thr Gln Gln Leu Pro
   180 185 190
   Asp Cys Arg Gly Leu Glu Val Trp Asn Ser Ile Pro Ser Cys Trp Ala
   195 200 205
   Leu Pro Trp Leu Asn Val Ser Ala Asp Gly Asp Asn Val His Leu Val
   210 215 220
   Leu Asn Val Ser Glu Glu Gln His Phe Gly Leu Ser Leu Tyr Trp Asn
   225 230 235 240
   Gln Val Gln Gly Pro Pro Lys Pro Arg Trp His Lys Asn Leu Thr Gly
   245 250 255
   Pro Gln Ile Ile Thr Leu Asn His Thr Asp Leu Val Pro Cys Leu Cys
   260 265 270
   Ile Gln Val Trp Pro Leu Glu Pro Asp Ser Val Arg Thr Asn Ile Cys
   275 280 285
   Pro Phe Arg
   290
<210> 89
   <211> 1569
   <212> DNA
   <213> Artificial Sequence
<220>
   <223> IL-17RC signal peptide and exons 1-10 of human IL-17RC, and Fc5
<400> 89
   atgcctgtgc cctggttctt gctgtccttg gcactgggcc gaagcccagt ggtcctttct 60
   ctggagaggc ttgtggggcc tcaggacgct acccactgct ctccgggcct ctcctgccgc 120
   ctctgggaca gtgacatact ctgcctgcct ggggacatcg tgcctgctcc gggccccgtg 180
   ctggcgccta cgcacctgca gacagagctg gtgctgaggt gccagaagga gaccgactgt 240
   gacctctgtc tgcgtgtggc tgtccacttg gccgtgcatg ggcactggga agagcctgaa 300
   gatgaggaaa agtttggagg agcagctgac tcaggggtgg aggagcctag gaatgcctct 360
   ctccaggccc aagtcgtgct ctccttccag gcctacccta ctgcccgctg cgtcctgctg 420
   gaggtgcaag tgcctgctgc ccttgtgcag tttggtcagt ctgtgggctc tgtggtatat 480
   gactgcttcg aggctgccct agggagtgag gtacgaatct ggtcctatac tcagcccagg 540
   tacgagaagg aactcaacca cacacagcag ctgcctgact gcagggggct cgaagtctgg 600
   aacagcatcc cgagctgctg ggccctgccc tggctcaacg tgtcagcaga tggtgacaac 660
   gtgcatctgg ttctgaatgt ctctgaggag cagcacttcg gcctctccct gtactggaat 720
   caggtccagg gccccccaaa accccggtgg cacaaaaacc tgactggacc gcagatcatt 780
   accttgaacc acacagacct ggttccctgc ctctgtattc aggtgtggcc tctggaacct 840
   gactccgtta ggacgaacat ctgccccttc agggagccca aatcttcaga caaaactcac 900
   acatgcccac cgtgcccagc acctgaagcc gagggggcac cgtcagtctt cctcttcccc 960
   ccaaaaccca aggacaccct catgatctcc cggacccctg aggtcacatg cgtggtggtg 1020
   gacgtgagcc acgaagaccc tgaggtcaag ttcaactggt acgtggacgg cgtggaggtg 1080
   cataatgcca agacaaagcc gcgggaggag cagtacaaca gcacgtaccg tgtggtcagc 1140
   gtcctcaccg tcctgcacca ggactggctg aatggcaagg agtacaagtg caaggtctcc 1200
   aacaaagccc tcccatcctc catcgagaaa accatctcca aagccaaagg gcagccccga 1260
   gaaccacagg tgtacaccct gcccccatcc cgggatgagc tgaccaagaa ccaggtcagc 1320
   ctgacctgcc tggtcaaagg cttctatccc agcgacatcg ccgtggagtg ggagagcaat 1380
   gggcagccgg agaacaacta caagaccacg cctcccgtgc tggactccga cggctccttc 1440
   ttcctctaca gcaagctcac cgtggacaag agcaggtggc agcaggggaa cgtcttctca 1500
   tgctccgtga tgcatgaggc tctgcacaac cactacacgc agaagagcct ctccctgtct 1560
   ccgggtaaa                                                         1569
<210> 90
   <211> 523
   <212> PRT
   <213> Artificial Sequence
<220>
   <223> IL-17RC signal peptide and exons 1-10 of human IL-17RC, and Fc5
<400> 90
   Met Pro Val Pro Trp Phe Leu Leu Ser Leu Ala Leu Gly Arg Ser Pro
   1               5                   10                  15
   Val Val Leu Ser Leu Glu Arg Leu Val Gly Pro Gln Asp Ala Thr His
               20                  25                  30
   Cys Ser Pro Gly Leu Ser Cys Arg Leu Trp Asp Ser Asp Ile Leu Cys
           35                  40                  45
   Leu Pro Gly Asp Ile Val Pro Ala Pro Gly Pro Val Leu Ala Pro Thr
       50                  55                  60
   His Leu Gln Thr Glu Leu Val Leu Arg Cys Gln Lys Glu Thr Asp Cys
   65                  70                  75                  80
   Asp Leu Cys Leu Arg Val Ala Val His Leu Ala Val His Gly His Trp
                   85                  90                  95
   Glu Glu Pro Glu Asp Glu Glu Lys Phe Gly Gly Ala Ala Asp Ser Gly
               100                 105                 110
   Val Glu Glu Pro Arg Asn Ala Ser Leu Gln Ala Gln Val Val Leu Ser
           115                 120                 125
   Phe Gln Ala Tyr Pro Thr Ala Arg Cys Val Leu Leu Glu Val Gln Val
       130                 135                 140
   Pro Ala Ala Leu Val Gln Phe Gly Gln Ser Val Gly Ser Val Val Tyr
   145                 150                 155                 160
   Asp Cys Phe Glu Ala Ala Leu Gly Ser Glu Val Arg Ile Trp Ser Tyr
                   165                 170                 175
   Thr Gln Pro Arg Tyr Glu Lys Glu Leu Asn His Thr Gln Gln Leu Pro
               180                 185                 190
   Asp Cys Arg Gly Leu Glu Val Trp Asn Ser Ile Pro Ser Cys Trp Ala
           195                 200                 205
   Leu Pro Trp Leu Asn Val Ser Ala Asp Gly Asp Asn Val His Leu Val
       210                 215                 220
   Leu Asn Val Ser Glu Glu Gln His Phe Gly Leu Ser Leu Tyr Trp Asn
   225                 230                 235                 240
   Gln Val Gln Gly Pro Pro Lys Pro Arg Trp His Lys Asn Leu Thr Gly
                   245                 250                 255
   Pro Gln Ile Ile Thr Leu Asn His Thr Asp Leu Val Pro Cys Leu Cys
               260                 265                 270
   Ile Gln Val Trp Pro Leu Glu Pro Asp Ser Val Arg Thr Asn Ile Cys
           275                 280                 285
   Pro Phe Arg Glu Pro Lys Ser Ser Asp Lys Thr His Thr Cys Pro Pro
       290                 295                 300
   Cys Pro Ala Pro Glu Ala Glu Gly Ala Pro Ser Val Phe Leu Phe Pro
   305                 310                 315                 320
   Pro Lys Pro Lys Asp Thr Leu Met Ile Ser Arg Thr Pro Glu Val Thr
                   325                 330                 335
   Cys Val Val Val Asp Val Ser His Glu Asp Pro Glu Val Lys Phe Asn
               340                 345                 350
   Trp Tyr Val Asp Gly Val Glu Val His Asn Ala Lys Thr Lys Pro Arg
           355                 360                 365
   Glu Glu Gln Tyr Asn Ser Thr Tyr Arg Val Val Ser Val Leu Thr Val
       370                 375                 380
   Leu His Gln Asp Trp Leu Asn Gly Lys Glu Tyr Lys Cys Lys Val Ser
   385                 390                 395                 400
   Asn Lys Ala Leu Pro Ser Ser Ile Glu Lys Thr Ile Ser Lys Ala Lys
                   405                 410                 415
   Gly Gln Pro Arg Glu Pro Gln Val Tyr Thr Leu Pro Pro Ser Arg Asp
               420                 425                 430
   Glu Leu Thr Lys Asn Gln Val Ser Leu Thr Cys Leu Val Lys Gly Phe
           435                 440                 445
   Tyr Pro Ser Asp Ile Ala Val Glu Trp Glu Ser Asn Gly Gln Pro Glu
       450                 455                 460
   Asn Asn Tyr Lys Thr Thr Pro Pro Val Leu Asp Ser Asp Gly Ser Phe
   465                 470                 475                 480
   Phe Leu Tyr Ser Lys Leu Thr Val Asp Lys Ser Arg Trp Gln Gln Gly
                   485                 490                 495
   Asn Val Phe Ser Cys Ser Val Met His Glu Ala Leu His Asn His Tyr
               500                 505                 510
   Thr Gln Lys Ser Leu Ser Leu Ser Pro Gly Lys
           515                 520
<210> 91
   <211> 1059
   <212> DNA
   <213> homo sapians
<400> 91
   atgcctgtgc cctggttctt gctgtccttg gcactgggcc gaagcccagt ggtcctttct 60
   ctggagaggc ttgtggggcc tcaggacgct acccactgct ctccgggcct ctcctgccgc 120
   ctctgggaca gtgacatact ctgcctgcct ggggacatcg tgcctgctcc gggccccgtg 180
   ctggcgccta cgcacctgca gacagagctg gtgctgaggt gccagaagga gaccgactgt 240
   gacctctgtc tgcgtgtggc tgtccacttg gccgtgcatg ggcactggga agagcctgaa 300
   gatgaggaaa agtttggagg agcagctgac tcaggggtgg aggagcctag gaatgcctct 360
   ctccaggccc aagtcgtgct ctccttccag gcctacccta ctgcccgctg cgtcctgctg 420
   gaggtgcaag tgcctgctgc ccttgtgcag tttggtcagt ctgtgggctc tgtggtatat 480
   gactgcttcg aggctgccct agggagtgag gtacgaatct ggtcctatac tcagcccagg 540
   tacgagaagg aactcaacca cacacagcag ctgcctgact gcagggggct cgaagtctgg 600
   aacagcatcc cgagctgctg ggccctgccc tggctcaacg tgtcagcaga tggtgacaac 660
   gtgcatctgg ttctgaatgt ctctgaggag cagcacttcg gcctctccct gtactggaat 720
   caggtccagg gccccccaaa accccggtgg cacaaaaacc tgactggacc gcagatcatt 780
   accttgaacc acacagacct ggttccctgc ctctgtattc aggtgtggcc tctggaacct 840
   gactccgtta ggacgaacat ctgccccttc agggaggacc cccgcgcaca ccagaacctc 900
   tggcaagccg cccgactgcg actgctgacc ctgcagagct ggctgctgga cgcaccgtgc 960
   tcgctgcccg cagaagcggc actgtgctgg cgggctccgg gtggggaccc ctgccagcca 1020
   ctggtcccac cgctttcctg ggagaacgtc actgtggac                        1059
<210> 92
   <211> 353
   <212> PRT
   <213> homo sapians
<400> 92
   Met Pro Val Pro Trp Phe Leu Leu Ser Leu Ala Leu Gly Arg Ser Pro
   1               5                   10                  15
   Val Val Leu Ser Leu Glu Arg Leu Val Gly Pro Gln Asp Ala Thr His
               20                  25                  30
   Cys Ser Pro Gly Leu Ser Cys Arg Leu Trp Asp Ser Asp Ile Leu Cys
           35                  40                  45
   Leu Pro Gly Asp Ile Val Pro Ala Pro Gly Pro Val Leu Ala Pro Thr
       50                  55                  60
   His Leu Gln Thr Glu Leu Val Leu Arg Cys Gln Lys Glu Thr Asp Cys
   65                  70                  75                  80
   Asp Leu Cys Leu Arg Val Ala Val His Leu Ala Val His Gly His Trp
                   85                  90                  95
   Glu Glu Pro Glu Asp Glu Glu Lys Phe Gly Gly Ala Ala Asp Ser Gly
               100                 105                 110
   Val Glu Glu Pro Arg Asn Ala Ser Leu Gln Ala Gln Val Val Leu Ser
           115                 120                 125
   Phe Gln Ala Tyr Pro Thr Ala Arg Cys Val Leu Leu Glu Val Gln Val
       130                 135                 140
   Pro Ala Ala Leu Val Gln Phe Gly Gln Ser Val Gly Ser Val Val Tyr
   145                 150                 155                 160
   Asp Cys Phe Glu Ala Ala Leu Gly Ser Glu Val Arg Ile Trp Ser Tyr
                   165                 170                 175
   Thr Gln Pro Arg Tyr Glu Lys Glu Leu Asn His Thr Gln Gln Leu Pro
               180                 185                 190
   Asp Cys Arg Gly Leu Glu Val Trp Asn Ser Ile Pro Ser Cys Trp Ala
           195                 200                 205
   Leu Pro Trp Leu Asn Val Ser Ala Asp Gly Asp Asn Val His Leu Val
       210                 215                 220
   Leu Asn Val Ser Glu Glu Gln His Phe Gly Leu Ser Leu Tyr Trp Asn
   225                 230                 235                 240
   Gln Val Gln Gly Pro Pro Lys Pro Arg Trp His Lys Asn Leu Thr Gly
                   245                 250                 255
   Pro Gln Ile Ile Thr Leu Asn His Thr Asp Leu Val Pro Cys Leu Cys
               260                 265                 270
   Ile Gln Val Trp Pro Leu Glu Pro Asp Ser Val Arg Thr Asn Ile Cys
           275                 280                 285
   Pro Phe Arg Glu Asp Pro Arg Ala His Gln Asn Leu Trp Gln Ala Ala
       290                 295                 300
   Arg Leu Arg Leu Leu Thr Leu Gln Ser Trp Leu Leu Asp Ala Pro Cys
   305                 310                 315                 320
   Ser Leu Pro Ala Glu Ala Ala Leu Cys Trp Arg Ala Pro Gly Gly Asp
                   325                 330                 335
   Pro Cys Gln Pro Leu Val Pro Pro Leu Ser Trp Glu Asn Val Thr Val
               340                 345                 350
   Asp
<210> 93
   <211> 1755
   <212> DNA
   <213> Artificial Sequence
<220>
   <223> IL-17RC signal peptide and exons 1-11 of human IL-17RC, and Fc5
<400> 93
   atgcctgtgc cctggttctt gctgtccttg gcactgggcc gaagcccagt ggtcctttct   60
   ctggagaggc ttgtggggcc tcaggacgct acccactgct ctccgggcct ctcctgccgc  120
   ctctgggaca gtgacatact ctgcctgcct ggggacatcg tgcctgctcc gggccccgtg  180
   ctggcgccta cgcacctgca gacagagctg gtgctgaggt gccagaagga gaccgactgt  240
   gacctctgtc tgcgtgtggc tgtccacttg gccgtgcatg ggcactggga agagcctgaa  300
   gatgaggaaa agtttggagg agcagctgac tcaggggtgg aggagcctag gaatgcctct  360
   ctccaggccc aagtcgtgct ctccttccag gcctacccta ctgcccgctg cgtcctgctg  420
   gaggtgcaag tgcctgctgc ccttgtgcag tttggtcagt ctgtgggctc tgtggtatat  480
   gactgcttcg aggctgccct agggagtgag gtacgaatct ggtcctatac tcagcccagg  540
   tacgagaagg aactcaacca cacacagcag ctgcctgact gcagggggct cgaagtctgg  600
   aacagcatcc cgagctgctg ggccctgccc tggctcaacg tgtcagcaga tggtgacaac  660
   gtgcatctgg ttctgaatgt ctctgaggag cagcacttcg gcctctccct gtactggaat  720
   caggtccagg gccccccaaa accccggtgg cacaaaaacc tgactggacc gcagatcatt  780
   accttgaacc acacagacct ggttccctgc ctctgtattc aggtgtggcc tctggaacct  840
   gactccgtta ggacgaacat ctgccccttc agggaggacc cccgcgcaca ccagaacctc  900
   tggcaagccg cccgactgcg actgctgacc ctgcagagct ggctgctgga cgcaccgtgc  960
   tcgctgcccg cagaagcggc actgtgctgg cgggctccgg gtggggaccc ctgccagcca 1020
   ctggtcccac cgctttcctg ggagaacgtc actgtggacg agcccaaatc ttcagacaaa 1080
   actcacacat gcccaccgtg cccagcacct gaagccgagg gggcaccgtc agtcttcctc 1140
   ttccccccaa aacccaagga caccctcatg atctcccgga cccctgaggt cacatgcgtg 1200
   gtggtggacg tgagccacga agaccctgag gtcaagttca actggtacgt ggacggcgtg 1260
   gaggtgcata atgccaagac aaagccgcgg gaggagcagt acaacagcac gtaccgtgtg 1320
   gtcagcgtcc tcaccgtcct gcaccaggac tggctgaatg gcaaggagta caagtgcaag 1380
   gtctccaaca aagccctccc atcctccatc gagaaaacca tctccaaagc caaagggcag 1440
   ccccgagaac cacaggtgta caccctgccc ccatcccggg atgagctgac caagaaccag 1500
   gtcagcctga cctgcctggt caaaggcttc tatcccagcg acatcgccgt ggagtgggag 1560
   agcaatgggc agccggagaa caactacaag accacgcctc ccgtgctgga ctccgacggc 1620
   tccttcttcc tctacagcaa gctcaccgtg gacaagagca ggtggcagca ggggaacgtc 1680
   ttctcatgct ccgtgatgca tgaggctctg cacaaccact acacgcagaa gagcctctcc 1740
   ctgtctccgg gtaaa                                                  1755
<210> 94
   <211> 585
   <212> PRT
   <213> Artificial Sequence
<220>
   <223> IL-17RC signal peptide and exons 1-11 of human IL-17RC, and Fc5
<400> 94
   Met Pro Val Pro Trp Phe Leu Leu Ser Leu Ala Leu Gly Arg Ser Pro
    1               5                   10                  15
   Val Val Leu Ser Leu Glu Arg Leu Val Gly Pro Gln Asp Ala Thr His
               20                  25                  30
   Cys Ser Pro Gly Leu Ser Cys Arg Leu Trp Asp Ser Asp Ile Leu Cys
           35                  40                  45
   Leu Pro Gly Asp Ile Val Pro Ala Pro Gly Pro Val Leu Ala Pro Thr
       50                  55                  60
   His Leu Gln Thr Glu Leu Val Leu Arg Cys Gln Lys Glu Thr Asp Cys
   65                  70                  75                  80
   Asp Leu Cys Leu Arg Val Ala Val His Leu Ala Val His Gly His Trp
                   85                  90                  95
   Glu Glu Pro Glu Asp Glu Glu Lys Phe Gly Gly Ala Ala Asp Ser Gly
               100                 105                 110
   Val Glu Glu Pro Arg Asn Ala Ser Leu Gln Ala Gln Val Val Leu Ser
           115                 120                 125
   Phe Gln Ala Tyr Pro Thr Ala Arg Cys Val Leu Leu Glu Val Gln Val
       130                 135                 140
   Pro Ala Ala Leu Val Gln Phe Gly Gln Ser Val Gly Ser Val Val Tyr
   145                 150                 155                 160
   Asp Cys Phe Glu Ala Ala Leu Gly Ser Glu Val Arg Ile Trp Ser Tyr
                   165                 170                 175
   Thr Gln Pro Arg Tyr Glu Lys Glu Leu Asn His Thr Gln Gln Leu Pro
               180                 185                 190
   Asp Cys Arg Gly Leu Glu Val Trp Asn Ser Ile Pro Ser Cys Trp Ala
           195                 200                 205
   Leu Pro Trp Leu Asn Val Ser Ala Asp Gly Asp Asn Val His Leu Val
       210                 215                 220
   Leu Asn Val Ser Glu Glu Gln His Phe Gly Leu Ser Leu Tyr Trp Asn
   225                 230                 235                 240
   Gln Val Gln Gly Pro Pro Lys Pro Arg Trp His Lys Asn Leu Thr Gly
                   245                 250                 255
   Pro Gln Ile Ile Thr Leu Asn His Thr Asp Leu Val Pro Cys Leu Cys
               260                 265                 270
   Ile Gln Val Trp Pro Leu Glu Pro Asp Ser Val Arg Thr Asn Ile Cys
           275                 280                 285
   Pro Phe Arg Glu Asp Pro Arg Ala His Gln Asn Leu Trp Gln Ala Ala
       290                 295                 300
   Arg Leu Arg Leu Leu Thr Leu Gln Ser Trp Leu Leu Asp Ala Pro Cys
   305                 310                 315                 320
   Ser Leu Pro Ala Glu Ala Ala Leu Cys Trp Arg Ala Pro Gly Gly Asp
                   325                 330                 335
   Pro Cys Gln Pro Leu Val Pro Pro Leu Ser Trp Glu Asn Val Thr Val
               340                 345                 350
   Asp Glu Pro Lys Ser Ser Asp Lys Thr His Thr Cys Pro Pro Cys Pro
           355                 360                 365
   Ala Pro Glu Ala Glu Gly Ala Pro Ser Val Phe Leu Phe Pro Pro Lys
       370                 375                 380
   Pro Lys Asp Thr Leu Met Ile Ser Arg Thr Pro Glu Val Thr Cys Val
   385                 390                 395                 400
   Val Val Asp Val Ser His Glu Asp Pro Glu Val Lys Phe Asn Trp Tyr
                   405                 410                 415
   Val Asp Gly Val Glu Val His Asn Ala Lys Thr Lys Pro Arg Glu Glu
               420                 425                 430
   Gln Tyr Asn Ser Thr Tyr Arg Val Val Ser Val Leu Thr Val Leu His
           435                 440                 445
   Gln Asp Trp Leu Asn Gly Lys Glu Tyr Lys Cys Lys Val Ser Asn Lys
       450                 455                 460
   Ala Leu Pro Ser Ser Ile Glu Lys Thr Ile Ser Lys Ala Lys Gly Gln
   465                 470                 475                 480
   Pro Arg Glu Pro Gln Val Tyr Thr Leu Pro Pro Ser Arg Asp Glu Leu
                   485                 490                 495
   Thr Lys Asn Gln Val Ser Leu Thr Cys Leu Val Lys Gly Phe Tyr Pro
               500                 505                 510
   Ser Asp Ile Ala Val Glu Trp Glu Ser Asn Gly Gln Pro Glu Asn Asn
           515                 520                 525
   Tyr Lys Thr Thr Pro Pro Val Leu Asp Ser Asp Gly Ser Phe Phe Leu
       530                 535                 540
   Tyr Ser Lys Leu Thr Val Asp Lys Ser Arg Trp Gln Gln Gly Asn Val
   545                 550                 555                 560
   Phe Ser Cys Ser Val Met His Glu Ala Leu His Asn His Tyr Thr Gln
                   565                 570                 575
   Lys Ser Leu Ser Leu Ser Pro Gly Lys
               580                 585
<210> 95
   <211> 303
   <212> DNA
   <213> homo sapians
<400> 95
   atgcctgtgc cctggttctt gctgtccttg gcactgggcc gaagcccagt ggtcctttct 60
   gactccctgg ggcctctcaa agacgatgtg ctactgttgg agacacgagg cccccaggac 120
   aacagatccc tctgtgcctt ggaacccagt ggctgtactt cactacccag caaagcctcc 180
   acgagggcag ctcgccttgg agagtactta ctacaagacc tgcagtcagg ccagtgtctg 240
   cagctatggg acgatgactt gggagcgcta tgggcctgcc ccatggacaa atacatccac 300
   aag                                                               303
<210> 96
   <211> 101
   <212> PRT
   <213> homo sapians

<400> 96
   Met Pro Val Pro Trp Phe Leu Leu Ser Leu Ala Leu Gly Arg Ser Pro
    1               5                   10                  15
   Val Val Leu Ser Asp Ser Leu Gly Pro Leu Lys Asp Asp Val Leu Leu
               20                  25                  30
   Leu Glu Thr Arg Gly Pro Gln Asp Asn Arg Ser Leu Cys Ala Leu Glu
           35                  40                  45
   Pro Ser Gly Cys Thr Ser Leu Pro Ser Lys Ala Ser Thr Arg Ala Ala
       50                  55                  60
   Arg Leu Gly Glu Tyr Leu Leu Gln Asp Leu Gln Ser Gly Gln Cys Leu
   65                  70                  75                  80
   Gln Leu Trp Asp Asp Asp Leu Gly Ala Leu Trp Ala Cys Pro Met Asp
                   85                  90                  95
   Lys Tyr Ile His Lys
               100
<210> 97
   <211> 999
   <212> DNA
   <213> Artificial Sequence
<220>
   <223> IL-17RC signal peptide and exons 14-16 of human IL-17RC, and Fc5
<400> 97
   atgcctgtgc cctggttctt gctgtccttg gcactgggcc gaagcccagt ggtcctttct 60
   gactccctgg ggcctctcaa agacgatgtg ctactgttgg agacacgagg cccccaggac 120
   aacagatccc tctgtgcctt ggaacccagt ggctgtactt cactacccag caaagcctcc 180
   acgagggcag ctcgccttgg agagtactta ctacaagacc tgcagtcagg ccagtgtctg 240
   cagctatggg acgatgactt gggagcgcta tgggcctgcc ccatggacaa atacatccac 300
   aaggagccca aatcttcaga caaaactcac acatgcccac cgtgcccagc acctgaagcc 360
   gagggggcac cgtcagtctt cctcttcccc ccaaaaccca aggacaccct catgatctcc 420
   cggacccctg aggtcacatg cgtggtggtg gacgtgagcc acgaagaccc tgaggtcaag 480
   ttcaactggt acgtggacgg cgtggaggtg cataatgcca agacaaagcc gcgggaggag 540
   cagtacaaca gcacgtaccg tgtggtcagc gtcctcaccg tcctgcacca ggactggctg 600
   aatggcaagg agtacaagtg caaggtctcc aacaaagccc tcccatcctc catcgagaaa 660
   accatctcca aagccaaagg gcagccccga gaaccacagg tgtacaccct gcccccatcc 720
   cgggatgagc tgaccaagaa ccaggtcagc ctgacctgcc tggtcaaagg cttctatccc 780
   agcgacatcg ccgtggagtg ggagagcaat gggcagccgg agaacaacta caagaccacg 840
   cctcccgtgc tggactccga cggctccttc ttcctctaca gcaagctcac cgtggacaag 900
   agcaggtggc agcaggggaa cgtcttctca tgctccgtga tgcatgaggc tctgcacaac 960
   cactacacgc agaagagcct ctccctgtct ccgggtaaa 999
<210> 98
   <211> 333
   <212> PRT
   <213> Artificial Sequence
<220>
   <223> IL-17RC signal peptide and exons 14-16 of human IL-17RC, and Fc5
<400> 98
   Met Pro Val Pro Trp Phe Leu Leu Ser Leu Ala Leu Gly Arg Ser Pro
    1               5                  10                  15
   Val Val Leu Ser Asp Ser Leu Gly Pro Leu Lys Asp Asp Val Leu Leu
               20                  25                  30
   Leu Glu Thr Arg Gly Pro Gln Asp Asn Arg Ser Leu Cys Ala Leu Glu
           35                  40                  45
   Pro Ser Gly Cys Thr Ser Leu Pro Ser Lys Ala Ser Thr Arg Ala Ala
       50                  55                  60
   Arg Leu Gly Glu Tyr Leu Leu Gln Asp Leu Gln Ser Gly Gln Cys Leu
   65                  70                  75                  80
   Gln Leu Trp Asp Asp Asp Leu Gly Ala Leu Trp Ala Cys Pro Met Asp
                   85                  90                  95
   Lys Tyr Ile His Lys Glu Pro Lys Ser Ser Asp Lys Thr His Thr Cys
               100                 105                 110
   Pro Pro Cys Pro Ala Pro Glu Ala Glu Gly Ala Pro Ser Val Phe Leu
           115                 120                 125
   Phe Pro Pro Lys Pro Lys Asp Thr Leu Met Ile Ser Arg Thr Pro Glu
       130                 135                 140
   Val Thr Cys Val Val Val Asp Val Ser His Glu Asp Pro Glu Val Lys
   145                 150                 155                 160
   Phe Asn Trp Tyr Val Asp Gly Val Glu Val His Asn Ala Lys Thr Lys
                   165                 170                 175
   Pro Arg Glu Glu Gln Tyr Asn Ser Thr Tyr Arg Val Val Ser Val Leu
               180                 185                 190
   Thr Val Leu His Gln Asp Trp Leu Asn Gly Lys Glu Tyr Lys Cys Lys
           195                 200                 205
   Val Ser Asn Lys Ala Leu Pro Ser Ser Ile Glu Lys Thr Ile Ser Lys
       210                 215                 220
   Ala Lys Gly Gln Pro Arg Glu Pro Gln Val Tyr Thr Leu Pro Pro Ser
   225                 230                 235                 240
   Arg Asp Glu Leu Thr Lys Asn Gln Val Ser Leu Thr Cys Leu Val Lys
                   245                 250                 255
   Gly Phe Tyr Pro Ser Asp Ile Ala Val Glu Trp Glu Ser Asn Gly Gln
               260                 265                 270
   Pro Glu Asn Asn Tyr Lys Thr Thr Pro Pro Val Leu Asp Ser Asp Gly
           275                 280                 285
   Ser Phe Phe Leu Tyr Ser Lys Leu Thr Val Asp Lys Ser Arg Trp Gln
       290                 295                 300
   Gln Gly Asn Val Phe Ser Cys Ser Val Met His Glu Ala Leu His Asn
   305                 310                 315                 320
   His Tyr Thr Gln Lys Ser Leu Ser Leu Ser Pro Gly Lys
                   325                 330
<210> 99
   <211> 585
   <212> DNA
   <213> homo sapians
<400> 99
   atgcctgtgc cctggttctt gctgtccttg gcactgggcc gaagcccagt ggtcctttct 60
   gaggaccccc gcgcacacca gaacctctgg caagccgccc gactgcgact gctgaccctg 120
   cagagctggc tgctggacgc accgtgctcg ctgcccgcag aagcggcact gtgctggcgg 180
   gctccgggtg gggacccctg ccagccactg gtcccaccgc tttcctggga gaacgtcact 240
   gtggacaagg ttctcgagtt cccattgctg aaaggccacc ctaacctctg tgttcaggtg 300
   aacagctcgg agaagctgca gctgcaggag tgcttgtggg ctgactccct ggggcctctc 360
   aaagacgatg tgctactgtt ggagacacga ggcccccagg acaacagatc cctctgtgcc 420
   ttggaaccca gtggctgtac ttcactaccc agcaaagcct ccacgagggc agctcgcctt 480
   ggagagtact tactacaaga cctgcagtca ggccagtgtc tgcagctatg ggacgatgac 540
   ttgggagcgc tatgggcctg ccccatggac aaatacatcc acaag                 585
<210> 100
   <211> 195
   <212> PRT
   <213> homo sapians
<400> 100
   Met Pro Val Pro Trp Phe Leu Leu Ser Leu Ala Leu Gly Arg Ser Pro
   1               5                   10                  15
   Val Val Leu Ser Glu Asp Pro Arg Ala His Gln Asn Leu Trp Gln Ala
               20                  25                  30
   Ala Arg Leu Arg Leu Leu Thr Leu Gln Ser Trp Leu Leu Asp Ala Pro
           35                  40                  45
   Cys Ser Leu Pro Ala Glu Ala Ala Leu Cys Trp Arg Ala Pro Gly Gly
       50                  55                  60
   Asp Pro Cys Gln Pro Leu Val Pro Pro Leu Ser Trp Glu Asn Val Thr
   65                  70                  75                  80
   Val Asp Lys Val Leu Glu Phe Pro Leu Leu Lys Gly His Pro Asn Leu
                   85                  90                  95
   Cys Val Gln Val Asn Ser Ser Glu Lys Leu Gln Leu Gln Glu Cys Leu
               100                 105                 110
   Trp Ala Asp Ser Leu Gly Pro Leu Lys Asp Asp Val Leu Leu Leu Glu
           115                 120                 125
   Thr Arg Gly Pro Gln Asp Asn Arg Ser Leu Cys Ala Leu Glu Pro Ser
       130                 135                 140
   Gly Cys Thr Ser Leu Pro Ser Lys Ala Ser Thr Arg Ala Ala Arg Leu
   145                 150                 155                 160
   Gly Glu Tyr Leu Leu Gln Asp Leu Gln Ser Gly Gln Cys Leu Gln Leu
                   165                 170                 175
   Trp Asp Asp Asp Leu Gly Ala Leu Trp Ala Cys Pro Met Asp Lys Tyr
               180                 185                 190
   Ile His Lys
           195
<210> 101
   <211> 1281
   <212> DNA
   <213> Artificial Sequence
<220>
   <223> IL-17RC signal peptide and exons 11-16 of human IL-17RC, and Fc5
<400> 101
   atgcctgtgc cctggttctt gctgtccttg gcactgggcc gaagcccagt ggtcctttct 60
   gaggaccccc gcgcacacca gaacctctgg caagccgccc gactgcgact gctgaccctg 120
   cagagctggc tgctggacgc accgtgctcg ctgcccgcag aagcggcact gtgctggcgg 180
   gctccgggtg gggacccctg ccagccactg gtcccaccgc tttcctggga gaacgtcact 240
   gtggacaagg ttctcgagtt cccattgctg aaaggccacc ctaacctctg tgttcaggtg 300
   aacagctcgg agaagctgca gctgcaggag tgcttgtggg ctgactccct ggggcctctc 360
   aaagacgatg tgctactgtt ggagacacga ggcccccagg acaacagatc cctctgtgcc 420
   ttggaaccca gtggctgtac ttcactaccc agcaaagcct ccacgagggc agctcgcctt 480
   ggagagtact tactacaaga cctgcagtca ggccagtgtc tgcagctatg ggacgatgac 540
   ttgggagcgc tatgggcctg ccccatggac aaatacatcc acaaggagcc caaatcttca 600
   gacaaaactc acacatgccc accgtgccca gcacctgaag ccgagggggc accgtcagtc 660
   ttcctcttcc ccccaaaacc caaggacacc ctcatgatct cccggacccc tgaggtcaca 720
   tgcgtggtgg tggacgtgag ccacgaagac cctgaggtca agttcaactg gtacgtggac 780
   ggcgtggagg tgcataatgc caagacaaag ccgcgggagg agcagtacaa cagcacgtac 840
   cgtgtggtca gcgtcctcac cgtcctgcac caggactggc tgaatggcaa ggagtacaag 900
   tgcaaggtct ccaacaaagc cctcccatcc tccatcgaga aaaccatctc caaagccaaa 960
   gggcagcccc gagaaccaca ggtgtacacc ctgcccccat cccgggatga gctgaccaag 1020
   aaccaggtca gcctgacctg cctggtcaaa ggcttctatc ccagcgacat cgccgtggag 1080
   tgggagagca atgggcagcc ggagaacaac tacaagacca cgcctcccgt gctggactcc 1140
   gacggctcct tcttcctcta cagcaagctc accgtggaca agagcaggtg gcagcagggg 1200
   aacgtcttct catgctccgt gatgcatgag gctctgcaca accactacac gcagaagagc 1260
   ctctccctgt ctccgggtaa a                                           1281
<210> 102
   <211> 427
   <212> PRT
   <213> Artificial Sequence
<220>
   <223> IL-17RC signal peptide and exons 11-16 of human IL-17RC, and Fc5
<400> 102
   Met Pro Val Pro Trp Phe Leu Leu Ser Leu Ala Leu Gly Arg Ser Pro
   1               5                   10                  15
   Val Val Leu Ser Glu Asp Pro Arg Ala His Gln Asn Leu Trp Gln Ala
               20                  25                  30
   Ala Arg Leu Arg Leu Leu Thr Leu Gln Ser Trp Leu Leu Asp Ala Pro
           35                  40                  45
   Cys Ser Leu Pro Ala Glu Ala Ala Leu Cys Trp Arg Ala Pro Gly Gly
       50                  55                  60
   Asp Pro Cys Gln Pro Leu Val Pro Pro Leu Ser Trp Glu Asn Val Thr
   65                  70                  75                  80
   Val Asp Lys Val Leu Glu Phe Pro Leu Leu Lys Gly His Pro Asn Leu
                   85                  90                  95
   Cys Val Gln Val Asn Ser Ser Glu Lys Leu Gln Leu Gln Glu Cys Leu
               100                 105                 110
   Trp Ala Asp Ser Leu Gly Pro Leu Lys Asp Asp Val Leu Leu Leu Glu
           115                 120                 125
   Thr Arg Gly Pro Gln Asp Asn Arg Ser Leu Cys Ala Leu Glu Pro Ser
       130                 135                 140
   Gly Cys Thr Ser Leu Pro Ser Lys Ala Ser Thr Arg Ala Ala Arg Leu
   145                 150                 155                 160
   Gly Glu Tyr Leu Leu Gln Asp Leu Gln Ser Gly Gln Cys Leu Gln Leu
                   165                 170                 175
   Trp Asp Asp Asp Leu Gly Ala Leu Trp Ala Cys Pro Met Asp Lys Tyr
               180                 185                 190
   Ile His Lys Glu Pro Lys Ser Ser Asp Lys Thr His Thr Cys Pro Pro
           195                 200                 205
   Cys Pro Ala Pro Glu Ala Glu Gly Ala Pro Ser Val Phe Leu Phe Pro
       210                 215                 220
   Pro Lys Pro Lys Asp Thr Leu Met Ile Ser Arg Thr Pro Glu Val Thr
   225                 230                 235                 240
   Cys Val Val Val Asp Val Ser His Glu Asp Pro Glu Val Lys Phe Asn
                   245                 250                 255
   Trp Tyr Val Asp Gly Val Glu Val His Asn Ala Lys Thr Lys Pro Arg
               260                 265                 270
   Glu Glu Gln Tyr Asn Ser Thr Tyr Arg Val Val Ser Val Leu Thr Val
           275                 280                 285
   Leu His Gln Asp Trp Leu Asn Gly Lys Glu Tyr Lys Cys Lys Val Ser
       290                 295                 300
   Asn Lys Ala Leu Pro Ser Ser Ile Glu Lys Thr Ile Ser Lys Ala Lys
   305                 310                 315                 320
   Gly Gln Pro Arg Glu Pro Gln Val Tyr Thr Leu Pro Pro Ser Arg Asp
                   325                 330                 335
   Glu Leu Thr Lys Asn Gln Val Ser Leu Thr Cys Leu Val Lys Gly Phe
               340                 345                 350
   Tyr Pro Ser Asp Ile Ala Val Glu Trp Glu Ser Asn Gly Gln Pro Glu
           355                 360                 365
   Asn Asn Tyr Lys Thr Thr Pro Pro Val Leu Asp Ser Asp Gly Ser Phe
       370                 375                 380
   Phe Leu Tyr Ser Lys Leu Thr Val Asp Lys Ser Arg Trp Gln Gln Gly
   385                 390                 395                 400
   Asn Val Phe Ser Cys Ser Val Met His Glu Ala Leu His Asn His Tyr
                   405                 410                 415
   Thr Gln Lys Ser Leu Ser Leu Ser Pro Gly Lys
               420                 425
<210> 103
   <211> 882
   <212> DNA
   <213> homo sapians
<400> 103
   atgcctgtgc cctggttctt gctgtccttg gcactgggcc gaagcccagt ggtcctttct 60
   gactgcaggg ggctcgaagt ctggaacagc atcccgagct gctgggccct gccctggctc 120
   aacgtgtcag cagatggtga caacgtgcat ctggttctga atgtctctga ggagcagcac 180
   ttcggcctct ccctgtactg gaatcaggtc cagggccccc caaaaccccg gtggcacaaa 240
   aacctgactg gaccgcagat cattaccttg aaccacacag acctggttcc ctgcctctgt 300
   attcaggtgt ggcctctgga acctgactcc gttaggacga acatctgccc cttcagggag 360
   gacccccgcg cacaccagaa cctctggcaa gccgcccgac tgcgactgct gaccctgcag 420
   agctggctgc tggacgcacc gtgctcgctg cccgcagaag cggcactgtg ctggcgggct 480
   ccgggtgggg acccctgcca gccactggtc ccaccgcttt cctgggagaa cgtcactgtg 540
   gacaaggttc tcgagttccc attgctgaaa ggccacccta acctctgtgt tcaggtgaac 600
   agctcggaga agctgcagct gcaggagtgc ttgtgggctg actccctggg gcctctcaaa 660
   gacgatgtgc tactgttgga gacacgaggc ccccaggaca acagatccct ctgtgccttg 720
   gaacccagtg gctgtacttc actacccagc aaagcctcca cgagggcagc tcgccttgga 780
   gagtacttac tacaagacct gcagtcaggc cagtgtctgc agctatggga cgatgacttg 840
   ggagcgctat gggcctgccc catggacaaa tacatccaca ag                    882
<210> 104
   <211> 294
   <212> PRT
   <213> homo sapians
<400> 104
   Met Pro Val Pro Trp Phe Leu Leu Ser Leu Ala Leu Gly Arg Ser Pro
   1               5                   10                  15
   Val Val Leu Ser Asp Cys Arg Gly Leu Glu Val Trp Asn Ser Ile Pro
               20                  25                  30
   Ser Cys Trp Ala Leu Pro Trp Leu Asn Val Ser Ala Asp Gly Asp Asn
           35                  40                  45
   Val His Leu Val Leu Asn Val Ser Glu Glu Gln His Phe Gly Leu Ser
       50                  55                  60
   Leu Tyr Trp Asn Gln Val Gln Gly Pro Pro Lys Pro Arg Trp His Lys
   65                  70                  75                  80
   Asn Leu Thr Gly Pro Gln Ile Ile Thr Leu Asn His Thr Asp Leu Val
                       85                  90                  95
   Pro Cys Leu Cys Ile Gln Val Trp Pro Leu Glu Pro Asp Ser Val Arg
               100                 105                 110
   Thr Asn Ile Cys Pro Phe Arg Glu Asp Pro Arg Ala His Gln Asn Leu
           115                 120                 125
   Trp Gln Ala Ala Arg Leu Arg Leu Leu Thr Leu Gln Ser Trp Leu Leu
       130                 135                 140
   Asp Ala Pro Cys Ser Leu Pro Ala Glu Ala Ala Leu Cys Trp Arg Ala
   145                 150                 155                 160
   Pro Gly Gly Asp Pro Cys Gln Pro Leu Val Pro Pro Leu Ser Trp Glu
                   165                 170                 175
   Asn Val Thr Val Asp Lys Val Leu Glu Phe Pro Leu Leu Lys Gly His
               180                 185                 190
   Pro Asn Leu Cys Val Gln Val Asn Ser Ser Glu Lys Leu Gln Leu Gln
           195                 200                 205
   Glu Cys Leu Trp Ala Asp Ser Leu Gly Pro Leu Lys Asp Asp Val Leu
       210                 215                 220
   Leu Leu Glu Thr Arg Gly Pro Gln Asp Asn Arg Ser Leu Cys Ala Leu
   225                 230                 235                 240
   Glu Pro Ser Gly Cys Thr Ser Leu Pro Ser Lys Ala Ser Thr Arg Ala
                   245                 250                 255
   Ala Arg Leu Gly Glu Tyr Leu Leu Gln Asp Leu Gln Ser Gly Gln Cys
               260                 265                 270
   Leu Gln Leu Trp Asp Asp Asp Leu Gly Ala Leu Trp Ala Cys Pro Met
           275                 280                 285
   Asp Lys Tyr Ile His Lys
       290
<210> 105
   <211> 1578
   <212> DNA
   <213> Artificial Sequence
<220>
   <223> IL-17RC signal peptide and exons 7-16 of human IL-17RC, and Fc5
<400> 105
   atgcctgtgc cctggttctt gctgtccttg gcactgggcc gaagcccagt ggtcctttct 60
   gactgcaggg ggctcgaagt ctggaacagc atcccgagct gctgggccct gccctggctc 120
   aacgtgtcag cagatggtga caacgtgcat ctggttctga atgtctctga ggagcagcac 180
   ttcggcctct ccctgtactg gaatcaggtc cagggccccc caaaaccccg gtggcacaaa 240
   aacctgactg gaccgcagat cattaccttg aaccacacag acctggttcc ctgcctctgt 300
   attcaggtgt ggcctctgga acctgactcc gttaggacga acatctgccc cttcagggag 360
   gacccccgcg cacaccagaa cctctggcaa gccgcccgac tgcgactgct gaccctgcag 420
   agctggctgc tggacgcacc gtgctcgctg cccgcagaag cggcactgtg ctggcgggct 480
   ccgggtgggg acccctgcca gccactggtc ccaccgcttt cctgggagaa cgtcactgtg 540
   gacaaggttc tcgagttccc attgctgaaa ggccacccta acctctgtgt tcaggtgaac 600
   agctcggaga agctgcagct gcaggagtgc ttgtgggctg actccctggg gcctctcaaa 660
   gacgatgtgc tactgttgga gacacgaggc ccccaggaca acagatccct ctgtgccttg 720
   gaacccagtg gctgtacttc actacccagc aaagcctcca cgagggcagc tcgccttgga 780
   gagtacttac tacaagacct gcagtcaggc cagtgtctgc agctatggga cgatgacttg 840
   ggagcgctat gggcctgccc catggacaaa tacatccaca aggagcccaa atcttcagac 900
   aaaactcaca catgcccacc gtgcccagca cctgaagccg agggggcacc gtcagtcttc 960
   ctcttccccc caaaacccaa ggacaccctc atgatctccc ggacccctga ggtcacatgc 1020
   gtggtggtgg acgtgagcca cgaagaccct gaggtcaagt tcaactggta cgtggacggc 1080
   gtggaggtgc ataatgccaa gacaaagccg cgggaggagc agtacaacag cacgtaccgt 1140
   gtggtcagcg tcctcaccgt cctgcaccag gactggctga atggcaagga gtacaagtgc 1200
   aaggtctcca acaaagccct cccatcctcc atcgagaaaa ccatctccaa agccaaaggg 1260
   cagccccgag aaccacaggt gtacaccctg cccccatccc gggatgagct gaccaagaac 1320
   caggtcagcc tgacctgcct ggtcaaaggc ttctatccca gcgacatcgc cgtggagtgg 1380
   gagagcaatg ggcagccgga gaacaactac aagaccacgc ctcccgtgct ggactccgac 1440
   ggctccttct tcctctacag caagctcacc gtggacaaga gcaggtggca gcaggggaac 1500
   gtcttctcat gctccgtgat gcatgaggct ctgcacaacc actacacgca gaagagcctc 1560
   tccctgtctc cgggtaaa                                               1578
<210> 106
   <211> 526
   <212> PRT
   <213> Artificial Sequence
<220>
   <223> IL-17RC signal peptide and exons 7-16 of human IL-17RC, and Fc5
<400> 106
   Met Pro Val Pro Trp Phe Leu Leu Ser Leu Ala Leu Gly Arg Ser Pro
   1               5                   10                  15
   Val Val Leu Ser Asp Cys Arg Gly Leu Glu Val Trp Asn Ser Ile Pro
               20                  25                  30
   Ser Cys Trp Ala Leu Pro Trp Leu Asn Val Ser Ala Asp Gly Asp Asn
           35                  40                  45
   Val His Leu Val Leu Asn Val Ser Glu Glu Gln His Phe Gly Leu Ser
       50                  55                  60
   Leu Tyr Trp Asn Gln Val Gln Gly Pro Pro Lys Pro Arg Trp His Lys
   65                  70                  75                  80
   Asn Leu Thr Gly Pro Gln Ile Ile Thr Leu Asn His Thr Asp Leu Val
                   85                  90                  95
   Pro Cys Leu Cys Ile Gln Val Trp Pro Leu Glu Pro Asp Ser Val Arg
               100                 105                 110
   Thr Asn Ile Cys Pro Phe Arg Glu Asp Pro Arg Ala His Gln Asn Leu
           115                 120                 125
   Trp Gln Ala Ala Arg Leu Arg Leu Leu Thr Leu Gln Ser Trp Leu Leu
       130                 135                 140
   Asp Ala Pro Cys Ser Leu Pro Ala Glu Ala Ala Leu Cys Trp Arg Ala
   145                 150                 155                 160
   Pro Gly Gly Asp Pro Cys Gln Pro Leu Val Pro Pro Leu Ser Trp Glu
                   165                 170                 175
   Asn Val Thr Val Asp Lys Val Leu Glu Phe Pro Leu Leu Lys Gly His
               180                 185                 190
   Pro Asn Leu Cys Val Gln Val Asn Ser Ser Glu Lys Leu Gln Leu Gln
           195                 200                 205
   Glu Cys Leu Trp Ala Asp Ser Leu Gly Pro Leu Lys Asp Asp Val Leu
       210                 215                 220
   Leu Leu Glu Thr Arg Gly Pro Gln Asp Asn Arg Ser Leu Cys Ala Leu
   225                 230                 235                 240
   Glu Pro Ser Gly Cys Thr Ser Leu Pro Ser Lys Ala Ser Thr Arg Ala
                   245                 250                 255
   Ala Arg Leu Gly Glu Tyr Leu Leu Gln Asp Leu Gln Ser Gly Gln Cys
               260                 265                 270
   Leu Gln Leu Trp Asp Asp Asp Leu Gly Ala Leu Trp Ala Cys Pro Met
           275                 280                 285
   Asp Lys Tyr Ile His Lys Glu Pro Lys Ser Ser Asp Lys Thr His Thr
       290                 295                 300
   Cys Pro Pro Cys Pro Ala Pro Glu Ala Glu Gly Ala Pro Ser Val Phe
   305                 310                 315                 320
   Leu Phe Pro Pro Lys Pro Lys Asp Thr Leu Met Ile Ser Arg Thr Pro
                   325                 330                 335
   Glu Val Thr Cys Val Val Val Asp Val Ser His Glu Asp Pro Glu Val
                   340                 345                 350
   Lys Phe Asn Trp Tyr Val Asp Gly Val Glu Val His Asn Ala Lys Thr
               355                 360                 365
   Lys Pro Arg Glu Glu Gln Tyr Asn Ser Thr Tyr Arg Val Val Ser Val
           370                 375                 380
   Leu Thr Val Leu His Gln Asp Trp Leu Asn Gly Lys Glu Tyr Lys Cys
   385                 390                 395                 400
   Lys Val Ser Asn Lys Ala Leu Pro Ser Ser Ile Glu Lys Thr Ile Ser
                   405                 410                 415
   Lys Ala Lys Gly Gln Pro Arg Glu Pro Gln Val Tyr Thr Leu Pro Pro
               420                 425                 430
   Ser Arg Asp Glu Leu Thr Lys Asn Gln Val Ser Leu Thr Cys Leu Val
           435                 440                 445
   Lys Gly Phe Tyr Pro Ser Asp Ile Ala Val Glu Trp Glu Ser Asn Gly
       450                 455                 460
   Gln Pro Glu Asn Asn Tyr Lys Thr Thr Pro Pro Val Leu Asp Ser Asp
   465                 470                 475                 480
   Gly Ser Phe Phe Leu Tyr Ser Lys Leu Thr Val Asp Lys Ser Arg Trp
                   485                 490                 495
   Gln Gln Gly Asn Val Phe Ser Cys Ser Val Met His Glu Ala Leu His
               500                 505                 510
   Asn His Tyr Thr Gln Lys Ser Leu Ser Leu Ser Pro Gly Lys
           515                 520                 525
<210> 107
   <211> 864
   <212> DNA
   <213> homo sapians
<400> 107
   atgcctgtgc cctggttctt gctgtccttg gcactgggcc gaagcccagt ggtcctttct 60
   ctggagaggc ttgtggggcc tcaggacgct acccactgct ctccgggcct ctcctgccgc 120
   ctctgggaca gtgacatact ctgcctgcct ggggacatcg tgcctgctcc gggccccgtg 180
   ctggcgccta cgcacctgca gacagagctg gtgctgaggt gccagaagga gaccgactgt 240
   gacctctgtc tgcgtgtggc tgtccacttg gccgtgcatg ggcactggga agagcctgaa 300
   gatgaggaaa agtttggagg agcagctgac tcaggggtgg aggagcctag gaatgcctct 360
   ctccaggccc aagtcgtgct ctccttccag gcctacccta ctgcccgctg cgtcctgctg 420
   gaggtgcaag tgcctgctgc ccttgtgcag tttggtcagt ctgtgggctc tgtggtatat 480
   gactgcttcg aggctgccct agggagtgag gtacgaatct ggtcctatac tcagcccagg 540
   tacgagaagg aactcaacca cacacagcag ctgcctgact gcagggggct cgaagtctgg 600
   aacagcatcc cgagctgctg ggactccctg gggcctctca aagacgatgt gctactgttg 660
   gagacacgag gcccccagga caacagatcc ctctgtgcct tggaacccag tggctgtact 720
   tcactaccca gcaaagcctc cacgagggca gctcgccttg gagagtactt actacaagac 780
   ctgcagtcag gccagtgtct gcagctatgg gacgatgact tgggagcgct atgggcctgc 840
   cccatggaca aatacatcca caag                                       864
<210> 108
   <211> 288
   <212> PRT
   <213> homo sapians
<400> 108
   Met Pro Val Pro Trp Phe Leu Leu Ser Leu Ala Leu Gly Arg Ser Pro
   1 5 10 15
   Val Val Leu Ser Leu Glu Arg Leu Val Gly Pro Gln Asp Ala Thr His
   20 25 30
   Cys Ser Pro Gly Leu Ser Cys Arg Leu Trp Asp Ser Asp Ile Leu Cys
   35 40 45
   Leu Pro Gly Asp Ile Val Pro Ala Pro Gly Pro Val Leu Ala Pro Thr
   50 55 60
   His Leu Gln Thr Glu Leu Val Leu Arg Cys Gln Lys Glu Thr Asp Cys
   65 70 75 80
   Asp Leu Cys Leu Arg Val Ala Val His Leu Ala Val His Gly His Trp
   85 90 95
   Glu Glu Pro Glu Asp Glu Glu Lys Phe Gly Gly Ala Ala Asp Ser Gly
   100 105 110
   Val Glu Glu Pro Arg Asn Ala Ser Leu Gln Ala Gln Val Val Leu Ser
   115 120 125
   Phe Gln Ala Tyr Pro Thr Ala Arg Cys Val Leu Leu Glu Val Gln Val
   130 135 140
   Pro Ala Ala Leu Val Gln Phe Gly Gln Ser Val Gly Ser Val Val Tyr
   145 150 155 160
   Asp Cys Phe Glu Ala Ala Leu Gly Ser Glu Val Arg Ile Trp Ser Tyr
   165 170 175
   Thr Gln Pro Arg Tyr Glu Lys Glu Leu Asn His Thr Gln Gln Leu Pro
   180 185 190
   Asp Cys Arg Gly Leu Glu Val Trp Asn Ser Ile Pro Ser Cys Trp Asp
   195 200 205
   Ser Leu Gly Pro Leu Lys Asp Asp Val Leu Leu Leu Glu Thr Arg Gly
   210 215 220
   Pro Gln Asp Asn Arg Ser Leu Cys Ala Leu Glu Pro Ser Gly Cys Thr
   225 230 235 240
   Ser Leu Pro Ser Lys Ala Ser Thr Arg Ala Ala Arg Leu Gly Glu Tyr
   245 250 255
   Leu Leu Gln Asp Leu Gln Ser Gly Gln Cys Leu Gln Leu Trp Asp Asp
   260 265 270
   Asp Leu Gly Ala Leu Trp Ala Cys Pro Met Asp Lys Tyr Ile His Lys
   275 280 285
<210> 109
   <211> 1560
   <212> DNA
   <213> Artificial Sequence
<220>
   <223> IL-17RC signal peptide and exons 1-7 and 14-16 of human IL-17RC, and Fc5
<400> 109
   atgcctgtgc cctggttctt gctgtccttg gcactgggcc gaagcccagt ggtcctttct 60
   ctggagaggc ttgtggggcc tcaggacgct acccactgct ctccgggcct ctcctgccgc 120
   ctctgggaca gtgacatact ctgcctgcct ggggacatcg tgcctgctcc gggccccgtg 180
   ctggcgccta cgcacctgca gacagagctg gtgctgaggt gccagaagga gaccgactgt 240
   gacctctgtc tgcgtgtggc tgtccacttg gccgtgcatg ggcactggga agagcctgaa 300
   gatgaggaaa agtttggagg agcagctgac tcaggggtgg aggagcctag gaatgcctct 360
   ctccaggccc aagtcgtgct ctccttccag gcctacccta ctgcccgctg cgtcctgctg 420
   gaggtgcaag tgcctgctgc ccttgtgcag tttggtcagt ctgtgggctc tgtggtatat 480
   gactgcttcg aggctgccct agggagtgag gtacgaatct ggtcctatac tcagcccagg 540
   tacgagaagg aactcaacca cacacagcag ctgcctgact gcagggggct cgaagtctgg 600
   aacagcatcc cgagctgctg ggactccctg ggcctctcca aagacgatgt gctactgttg 660
   gagacacgag gcccccagga caacagatcc ctctgtgcct tggaacccag tggctgtact 720
   tcactaccca gcaaagcctc cacgagggca gctcgccttg gagagtactt actacaagac 780
   ctgcagtcag gccagtgtct gcagctatgg gacgatgact tgggagcgct atgggcctgc 840
   cccatggaca aatacatcca caaggagccc aaatcttcag acaaaactca cacatgccca 900
   ccgtgcccag cacctgaagc cgagggggca ccgtcagtct tcctcttccc cccaaaaccc 960
   aaggacaccc tcatgatctc ccggacccct gaggtcacat gcgtggtggt ggacgtgagc 1020
   cacgaagacc ctgaggtcaa gttcaactgg tacgtggacg gcgtggaggt gcataatgcc 1080
   aagacaaagc cgcgggagga gcagtacaac agcacgtacc gtgtggtcag cgtcctcacc 1140
   gtcctgcacc aggactggct gaatggcaag gagtacaagt gcaaggtctc caacaaagcc 1200
   ctcccatcct ccatcgagaa aaccatctcc aaagccaaag ggcagccccg agaaccacag 1260
   gtgtacaccc tgcccccatc ccgggatgag ctgaccaaga accaggtcag cctgacctgc 1320
   ctggtcaaag gcttctatcc cagcgacatc gccgtggagt gggagagcaa tgggcagccg 1380
   gagaacaact acaagaccac gcctcccgtg ctggactccg acggctcctt cttcctctac 1440
   agcaagctca ccgtggacaa gagcaggtgg cagcagggga acgtcttctc atgctccgtg 1500
   atgcatgagg ctctgcacaa ccactacacg cagaagagcc tctccctgtc tccgggtaaa 1560
<210> 110
   <211> 520
   <212> PRT
   <213> Artificial Sequence
<220>
   <223> IL-17RC signal peptide and exons 1-7 and 14-16 of human IL-17RC, and Fc5
<400> 110
   Met Pro Val Pro Trp Phe Leu Leu Ser Leu Ala Leu Gly Arg Ser Pro
   1               5                   10                  15
   Val Val Leu Ser Leu Glu Arg Leu Val Gly Pro Gln Asp Ala Thr His
               20                  25                  30
   Cys Ser Pro Gly Leu Ser Cys Arg Leu Trp Asp Ser Asp Ile Leu Cys
           35                  40                  45
   Leu Pro Gly Asp Ile Val Pro Ala Pro Gly Pro Val Leu Ala Pro Thr
       50                  55                  60
   His Leu Gln Thr Glu Leu Val Leu Arg Cys Gln Lys Glu Thr Asp Cys
   65                  70                  75                  80
   Asp Leu Cys Leu Arg Val Ala Val His Leu Ala Val His Gly His Trp
                   85                  90                  95
   Glu Glu Pro Glu Asp Glu Glu Lys Phe Gly Gly Ala Ala Asp Ser Gly
               100                 105                 110
   Val Glu Glu Pro Arg Asn Ala Ser Leu Gln Ala Gln Val Val Leu Ser
           115                 120                 125
   Phe Gln Ala Tyr Pro Thr Ala Arg Cys Val Leu Leu Glu Val Gln Val
       130                 135                 140
   Pro Ala Ala Leu Val Gln Phe Gly Gln Ser Val Gly Ser Val Val Tyr
   145                 150                 155                 160
   Asp Cys Phe Glu Ala Ala Leu Gly Ser Glu Val Arg Ile Trp Ser Tyr
                   165                 170                 175
   Thr Gln Pro Arg Tyr Glu Lys Glu Leu Asn His Thr Gln Gln Leu Pro
               180                 185                 190
   Asp Cys Arg Gly Leu Glu Val Trp Asn Ser Ile Pro Ser Cys Trp Asp
           195                 200                 205
   Ser Leu Gly Pro Leu Lys Asp Asp Val Leu Leu Leu Glu Thr Arg Gly
       210                 215                 220
   Pro Gln Asp Asn Arg Ser Leu Cys Ala Leu Glu Pro Ser Gly Cys Thr
   225                 230                 235                 240
   Ser Leu Pro Ser Lys Ala Ser Thr Arg Ala Ala Arg Leu Gly Glu Tyr
                   245                 250                 255
   Leu Leu Gln Asp Leu Gln Ser Gly Gln Cys Leu Gln Leu Trp Asp Asp
               260                 265                 270
   Asp Leu Gly Ala Leu Trp Ala Cys Pro Met Asp Lys Tyr Ile His Lys
           275                 280                 285
   Glu Pro Lys Ser Ser Asp Lys Thr His Thr Cys Pro Pro Cys Pro Ala
       290                 295                 300
   Pro Glu Ala Glu Gly Ala Pro Ser Val Phe Leu Phe Pro Pro Lys Pro
   305                 310                 315                 320
   Lys Asp Thr Leu Met Ile Ser Arg Thr Pro Glu Val Thr Cys Val Val
                           325                     330                     335
   Val Asp Val Ser His Glu Asp Pro Glu Val Lys Phe Asn Trp Tyr Val
                   340                     345                     350
   Asp Gly Val Glu Val His Asn Ala Lys Thr Lys Pro Arg Glu Glu Gln
               355                     360                     365
   Tyr Asn Ser Thr Tyr Arg Val Val Ser Val Leu Thr Val Leu His Gln
       370                     375                     380
   Asp Trp Leu Asn Gly Lys Glu Tyr Lys Cys Lys Val Ser Asn Lys Ala
   385                     390                     395                     400
   Leu Pro Ser Ser Ile Glu Lys Thr Ile Ser Lys Ala Lys Gly Gln Pro
                   405                     410                     415
   Arg Glu Pro Gln Val Tyr Thr Leu Pro Pro Ser Arg Asp Glu Leu Thr
               420                     425                     430
   Lys Asn Gln Val Ser Leu Thr Cys Leu Val Lys Gly Phe Tyr Pro Ser
           435                     440                     445
   Asp Ile Ala Val Glu Trp Glu Ser Asn Gly Gln Pro Glu Asn Asn Tyr
       450                     455                     460
   Lys Thr Thr Pro Pro Val Leu Asp Ser Asp Gly Ser Phe Phe Leu Tyr
   465                     470                     475                     480
   Ser Lys Leu Thr Val Asp Lys Ser Arg Trp Gln Gln Gly Asn Val Phe
                   485                     490                     495
   Ser Cys Ser Val Met His Glu Ala Leu His Asn His Tyr Thr Gln Lys
               500                     505                     510
   Ser Leu Ser Leu Ser Pro Gly Lys
           515                     520
<210> 111
   <211> 1146
   <212> DNA
   <213> homo sapians
<400> 111
   atgcctgtgc cctggttctt gctgtccttg gcactgggcc gaagcccagt ggtcctttct 60
   ctggagaggc ttgtggggcc tcaggacgct acccactgct ctccgggcct ctcctgccgc 120
   ctctgggaca gtgacatact ctgcctgcct ggggacatcg tgcctgctcc gggccccgtg 180
   ctggcgccta cgcacctgca gacagagctg gtgctgaggt gccagaagga gaccgactgt 240
   gacctctgtc tgcgtgtggc tgtccacttg gccgtgcatg ggcactggga agagcctgaa 300
   gatgaggaaa agtttggagg agcagctgac tcaggggtgg aggagcctag gaatgcctct 360
   ctccaggccc aagtcgtgct ctccttccag gcctacccta ctgcccgctg cgtcctgctg 420
   gaggtgcaag tgcctgctgc ccttgtgcag tttggtcagt ctgtgggctc tgtggtatat 480
   gactgcttcg aggctgccct agggagtgag gtacgaatct ggtcctatac tcagcccagg 540
   tacgagaagg aactcaacca cacacagcag ctgcctgact gcagggggct cgaagtctgg 600
   aacagcatcc cgagctgctg ggaggacccc cgcgcacacc agaacctctg gcaagccgcc 660
   cgactgcgac tgctgaccct gcagagctgg ctgctggacg caccgtgctc gctgcccgca 720
   gaagcggcac tgtgctggcg ggctccgggt ggggacccct gccagccact ggtcccaccg 780
   ctttcctggg agaacgtcac tgtggacaag gttctcgagt tcccattgct gaaaggccac 840
   cctaacctct gtgttcaggt gaacagctcg gagaagctgc agctgcagga gtgcttgtgg 900
   gctgactccc tggggcctct caaagacgat gtgctactgt tggagacacg aggcccccag 960
   gacaacagat ccctctgtgc cttggaaccc agtggctgta cttcactacc cagcaaagcc 1020
   tccacgaggg cagctcgcct tggagagtac ttactacaag acctgcagtc aggccagtgt 1080
   ctgcagctat gggacgatga cttgggagcg ctatgggcct gccccatgga caaatacatc 1140
   cacaag                                                            1146
<210> 112
   <211> 382
   <212> PRT
   <213> homo sapians
<400> 112
   Met Pro Val Pro Trp Phe Leu Leu Ser Leu Ala Leu Gly Arg Ser Pro
   1               5                   10                  15
   Val Val Leu Ser Leu Glu Arg Leu Val Gly Pro Gln Asp Ala Thr His
               20                  25                  30
   Cys Ser Pro Gly Leu Ser Cys Arg Leu Trp Asp Ser Asp Ile Leu Cys
           35                  40                  45
   Leu Pro Gly Asp Ile Val Pro Ala Pro Gly Pro Val Leu Ala Pro Thr
       50                  55                  60
   His Leu Gln Thr Glu Leu Val Leu Arg Cys Gln Lys Glu Thr Asp Cys
   65                  70                  75                  80
   Asp Leu Cys Leu Arg Val Ala Val His Leu Ala Val His Gly His Trp
                   85                  90                  95
   Glu Glu Pro Glu Asp Glu Glu Lys Phe Gly Gly Ala Ala Asp Ser Gly
               100                 105                 110
   Val Glu Glu Pro Arg Asn Ala Ser Leu Gln Ala Gln Val Val Leu Ser
           115                 120                 125
   Phe Gln Ala Tyr Pro Thr Ala Arg Cys Val Leu Leu Glu Val Gln Val
       130                 135                 140
   Pro Ala Ala Leu Val Gln Phe Gly Gln Ser Val Gly Ser Val Val Tyr
   145                 150                 155                 160
   Asp Cys Phe Glu Ala Ala Leu Gly Ser Glu Val Arg Ile Trp Ser Tyr
                   165                 170                 175
   Thr Gln Pro Arg Tyr Glu Lys Glu Leu Asn His Thr Gln Gln Leu Pro
               180                 185                 190
   Asp Cys Arg Gly Leu Glu Val Trp Asn Ser Ile Pro Ser Cys Trp Glu
           195                 200                 205
   Asp Pro Arg Ala His Gln Asn Leu Trp Gln Ala Ala Arg Leu Arg Leu
       210                 215                 220
   Leu Thr Leu Gln Ser Trp Leu Leu Asp Ala Pro Cys Ser Leu Pro Ala
   225                 230                 235                 240
   Glu Ala Ala Leu Cys Trp Arg Ala Pro Gly Gly Asp Pro Cys Gln Pro
                   245                 250                 255
   Leu Val Pro Pro Leu Ser Trp Glu Asn Val Thr Val Asp Lys Val Leu
               260                 265                 270
   Glu Phe Pro Leu Leu Lys Gly His Pro Asn Leu Cys Val Gln Val Asn
           275                 280                 285
   Ser Ser Glu Lys Leu Gln Leu Gln Glu Cys Leu Trp Ala Asp Ser Leu
       290                 295                 300
   Gly Pro Leu Lys Asp Asp Val Leu Leu Leu Glu Thr Arg Gly Pro Gln
   305                 310                 315                 320
   Asp Asn Arg Ser Leu Cys Ala Leu Glu Pro Ser Gly Cys Thr Ser Leu
                   325                 330                 335
   Pro Ser Lys Ala Ser Thr Arg Ala Ala Arg Leu Gly Glu Tyr Leu Leu
               340                 345                 350
   Gln Asp Leu Gln Ser Gly Gln Cys Leu Gln Leu Trp Asp Asp Asp Leu
           355                 360                 365
   Gly Ala Leu Trp Ala Cys Pro Met Asp Lys Tyr Ile His Lys
       370                 375                 380
<210> 113
   <211> 1842
   <212> DNA
   <213> Artificial Sequence
<220>
   <223> IL-17RC signal peptide and exons 1-7 and 11-16 of human IL-17RC, and Fc5
<400> 113
   atgcctgtgc cctggttctt gctgtccttg gcactgggcc gaagcccagt ggtcctttct 60
   ctggagaggc ttgtggggcc tcaggacgct acccactgct ctccgggcct ctcctgccgc 120
   ctctgggaca gtgacatact ctgcctgcct ggggacatcg tgcctgctcc gggccccgtg 180
   ctggcgccta cgcacctgca gacagagctg gtgctgaggt gccagaagga gaccgactgt 240
   gacctctgtc tgcgtgtggc tgtccacttg gccgtgcatg ggcactggga agagcctgaa 300
   gatgaggaaa agtttggagg agcagctgac tcaggggtgg aggagcctag gaatgcctct 360
   ctccaggccc aagtcgtgct ctccttccag gcctacccta ctgcccgctg cgtcctgctg 420
   gaggtgcaag tgcctgctgc ccttgtgcag tttggtcagt ctgtgggctc tgtggtatat 480
   gactgcttcg aggctgccct agggagtgag gtacgaatct ggtcctatac tcagcccagg 540
   tacgagaagg aactcaacca cacacagcag ctgcctgact gcagggggct cgaagtctgg 600
   aacagcatcc cgagctgctg ggaggacccc cgcgcacacc agaacctctg gcaagccgcc 660
   cgactgcgac tgctgaccct gcagagctgg ctgctggacg caccgtgctc gctgcccgca 720
   gaagcggcac tgtgctggcg ggctccgggt ggggacccct gccagccact ggtcccaccg 780
   ctttcctggg agaacgtcac tgtggacaag gttctcgagt tcccattgct gaaaggccac 840
   cctaacctct gtgttcaggt gaacagctcg gagaagctgc agctgcagga gtgcttgtgg 900
   gctgactccc tggggcctct caaagacgat gtgctactgt tggagacacg aggcccccag 960
   gacaacagat ccctctgtgc cttggaaccc agtggctgta cttcactacc cagcaaagcc 1020
   tccacgaggg cagctcgcct tggagagtac ttactacaag acctgcagtc aggccagtgt 1080
   ctgcagctat gggacgatga cttgggagcg ctatgggcct gccccatgga caaatacatc 1140
   cacaaggagc ccaaatcttc agacaaaact cacacatgcc caccgtgccc agcacctgaa 1200
   gccgaggggg caccgtcagt cttcctcttc cccccaaaac ccaaggacac cctcatgatc 1260
   tcccggaccc ctgaggtcac atgcgtggtg gtggacgtga gccacgaaga ccctgaggtc 1320
   aagttcaact ggtacgtgga cggcgtggag gtgcataatg ccaagacaaa gccgcgggag 1380
   gagcagtaca acagcacgta ccgtgtggtc agcgtcctca ccgtcctgca ccaggactgg 1440
   ctgaatggca aggagtacaa gtgcaaggtc tccaacaaag ccctcccatc ctccatcgag 1500
   aaaaccatct ccaaagccaa agggcagccc cgagaaccac aggtgtacac cctgccccca 1560
   tcccgggatg agctgaccaa gaaccaggtc agcctgacct gcctggtcaa aggcttctat 1620
   cccagcgaca tcgccgtgga gtgggagagc aatgggcagc cggagaacaa ctacaagacc 1680
   acgcctcccg tgctggactc cgacggctcc ttcttcctct acagcaagct caccgtggac 1740
   aagagcaggt ggcagcaggg gaacgtcttc tcatgctccg tgatgcatga ggctctgcac 1800
   aaccactaca cgcagaagag cctctccctg tctccgggta aa                   1842
<210> 114
   <211> 614
   <212> PRT
   <213> Artificial Sequence
<220>
   <223> IL-17RC signal peptide and exons 1-7 and 11-16 of human IL-17RC, and Fc5
<400> 114 <210> 115
   <211> 1524
   <212> DNA
   <213> Artificial Sequence
<220>
   <223> IL-17RC signal peptide and exons 1-13 of human IL-17RC, and exons 7-9 of human IL-17RA
<400> 115
<210> 116
   <211> 508
   <212> PRT
   <213> Artificial Sequence
<220>
   <223> IL-17RC signal peptide and exons 1-13 of human IL-17RC, and exons 7-9 of human IL-17RA
<400> 116
   Met Pro Val Pro Trp Phe Leu Leu Ser Leu Ala Leu Gly Arg Ser Pro
    1              5                   10                  15
   Val Val Leu Ser Leu Glu Arg Leu Val Gly Pro Gln Asp Ala Thr His
               20                  25                  30
   Cys Ser Pro Gly Leu Ser Cys Arg Leu Trp Asp Ser Asp Ile Leu Cys
           35                  40                  45
   Leu Pro Gly Asp Ile Val Pro Ala Pro Gly Pro Val Leu Ala Pro Thr
       50                  55                  60
   His Leu Gln Thr Glu Leu Val Leu Arg Cys Gln Lys Glu Thr Asp Cys
   65                  70                  75                  80
   Asp Leu Cys Leu Arg Val Ala Val His Leu Ala Val His Gly His Trp
                       85                      90                      95
   Glu Glu Pro Glu Asp Glu Glu Lys Phe Gly Gly Ala Ala Asp Ser Gly
               100                     105                     110
   Val Glu Glu Pro Arg Asn Ala Ser Leu Gln Ala Gln Val Val Leu Ser
           115                     120                     125
   Phe Gln Ala Tyr Pro Thr Ala Arg Cys Val Leu Leu Glu Val Gln Val
       130                     135                     140
   Pro Ala Ala Leu Val Gln Phe Gly Gln Ser Val Gly Ser Val Val Tyr
   145                     150                     155                     160
   Asp Cys Phe Glu Ala Ala Leu Gly Ser Glu Val Arg Ile Trp Ser Tyr
                   165                     170                     175
   Thr Gln Pro Arg Tyr Glu Lys Glu Leu Asn His Thr Gln Gln Leu Pro
               180                     185                     190
   Asp Cys Arg Gly Leu Glu Val Trp Asn Ser Ile Pro Ser Cys Trp Ala
           195                     200                     205
   Leu Pro Trp Leu Asn Val Ser Ala Asp Gly Asp Asn Val His Leu Val
       210                     215                     220
   Leu Asn Val Ser Glu Glu Gln His Phe Gly Leu Ser Leu Tyr Trp Asn
   225                     230                     235                     240
   Gln Val Gln Gly Pro Pro Lys Pro Arg Trp His Lys Asn Leu Thr Gly
                   245                     250                     255
   Pro Gln Ile Ile Thr Leu Asn His Thr Asp Leu Val Pro Cys Leu Cys
               260                     265                     270
   Ile Gln Val Trp Pro Leu Glu Pro Asp Ser Val Arg Thr Asn Ile Cys
           275                     280                     285
   Pro Phe Arg Glu Asp Pro Arg Ala His Gln Asn Leu Trp Gln Ala Ala
       290                     295                     300
   Arg Leu Arg Leu Leu Thr Leu Gln Ser Trp Leu Leu Asp Ala Pro Cys
   305                     310                     315                     320
   Ser Leu Pro Ala Glu Ala Ala Leu Cys Trp Arg Ala Pro Gly Gly Asp
                   325                     330                     335
   Pro Cys Gln Pro Leu Val Pro Pro Leu Ser Trp Glu Asn Val Thr Val
               340                     345                     350
   Asp Lys Val Leu Glu Phe Pro Leu Leu Lys Gly His Pro Asn Leu Cys
           355                     360                     365
   Val Gln Val Asn Ser Ser Glu Lys Leu Gln Leu Gln Glu Cys Leu Trp
       370                     375                     380
   Ala Gly Ser Leu Trp Asp Pro Asn Ile Thr Val Glu Thr Leu Asp Thr
   385                     390                     395                     400
   Gln His Leu Arg Val Asp Phe Thr Leu Trp Asn Glu Ser Thr Pro Tyr
                   405                     410                     415
   Gln Val Leu Leu Glu Ser Phe Ser Asp Ser Glu Asn His Ser Cys Phe
               420                     425                     430
   Asp Val Val Lys Gln Ile Phe Ala Pro Arg Gln Glu Glu Phe His Gln
           435                     440                     445
   Arg Ala Asn Val Thr Phe Thr Leu Ser Lys Phe His Trp Cys Cys His
       450                     455                     460
   His His Val Gln Val Gln Pro Phe Phe Ser Ser Cys Leu Asn Asp Cys
   465                     470                     475                     480
   Leu Arg His Ala Val Thr Val Pro Cys Pro Val Ile Ser Asn Thr Thr
                   485                     490                     495
   Val Pro Lys Pro Val Ala Asp Tyr Ile Pro Leu Trp
               500                     505
<210> 117
   <211> 2220
   <212> DNA
   <213> Artificial Sequence
<220>
   <223> IL-17RC signal peptide and exons 1-13 of human IL-17RC, and exons 7-9 of human IL-17RA, and Fc5
<400> 117
   atgcctgtgc cctggttctt gctgtccttg gcactgggcc gaagcccagt ggtcctttct 60
   ctggagaggc ttgtggggcc tcaggacgct acccactgct ctccgggcct ctcctgccgc 120
   ctctgggaca gtgacatact ctgcctgcct ggggacatcg tgcctgctcc gggccccgtg 180
   ctggcgccta cgcacctgca gacagagctg gtgctgaggt gccagaagga gaccgactgt 240
   gacctctgtc tgcgtgtggc tgtccacttg gccgtgcatg ggcactggga agagcctgaa 300
   gatgaggaaa agtttggagg agcagctgac tcaggggtgg aggagcctag gaatgcctct 360
   ctccaggccc aagtcgtgct ctccttccag gcctacccta ctgcccgctg cgtcctgctg 420
   gaggtgcaag tgcctgctgc ccttgtgcag tttggtcagt ctgtgggctc tgtggtatat 480
   gactgcttcg aggctgccct agggagtgag gtacgaatct ggtcctatac tcagcccagg 540
   tacgagaagg aactcaacca cacacagcag ctgcctgact gcagggggct cgaagtctgg 600
   aacagcatcc cgagctgctg ggccctgccc tggctcaacg tgtcagcaga tggtgacaac 660
   gtgcatctgg ttctgaatgt ctctgaggag cagcacttcg gcctctccct gtactggaat 720
   caggtccagg gccccccaaa accccggtgg cacaaaaacc tgactggacc gcagatcatt 780
   accttgaacc acacagacct ggttccctgc ctctgtattc aggtgtggcc tctggaacct 840
   gactccgtta ggacgaacat ctgccccttc agggaggacc cccgcgcaca ccagaacctc 900
   tggcaagccg cccgactgcg actgctgacc ctgcagagct ggctgctgga cgcaccgtgc 960
   tcgctgcccg cagaagcggc actgtgctgg cgggctccgg gtggggaccc ctgccagcca 1020
   ctggtcccac cgctttcctg ggagaacgtc actgtggaca aggttctcga gttcccattg 1080
   ctgaaaggcc accctaacct ctgtgttcag gtgaacagct cggagaagct gcagctgcag 1140
   gagtgcttgt gggctggcag cctttgggat cccaacatca ctgtggagac cttggacaca 1200
   cagcatctgc gagtggactt caccctgtgg aatgaatcca ccccctacca ggtcctgctg 1260
   gaaagtttct ccgactcaga gaaccacagc tgctttgatg tcgttaaaca aatatttgcg 1320
   cccaggcaag aagaattcca tcagcgagct aatgtcacat tcactctaag caagtttcac 1380
   tggtgctgcc atcaccacgt gcaggtccag cccttcttca gcagctgcct aaatgactgt 1440
   ttgagacacg ctgtgactgt gccctgccca gtaatctcaa ataccacagt tcccaagcca 1500
   gttgcagact acattcccct gtgggagccc aaatcttcag acaaaactca cacatgccca 1560
   ccgtgcccag cacctgaagc cgagggggca ccgtcagtct tcctcttccc cccaaaaccc 1620
   aaggacaccc tcatgatctc ccggacccct gaggtcacat gcgtggtggt ggacgtgagc 1680
   cacgaagacc ctgaggtcaa gttcaactgg tacgtggacg gcgtggaggt gcataatgcc 1740
   aagacaaagc cgcgggagga gcagtacaac agcacgtacc gtgtggtcag cgtcctcacc 1800
   gtcctgcacc aggactggct gaatggcaag gagtacaagt gcaaggtctc caacaaagcc 1860
   ctcccatcct ccatcgagaa aaccatctcc aaagccaaag ggcagccccg agaaccacag 1920
   gtgtacaccc tgcccccatc ccgggatgag ctgaccaaga accaggtcag cctgacctgc 1980
   ctggtcaaag gcttctatcc cagcgacatc gccgtggagt gggagagcaa tgggcagccg 2040
   gagaacaact acaagaccac gcctcccgtg ctggactccg acggctcctt cttcctctac 2100
   agcaagctca ccgtggacaa gagcaggtgg cagcagggga acgtcttctc atgctccgtg 2160
   atgcatgagg ctctgcacaa ccactacacg cagaagagcc tctccctgtc tccgggtaaa 2220
<210> 118
   <211> 740
   <212> PRT
   <213> Artificial Sequence
<220>
   <223> IL-17RC signal peptide and exons 1-13 of human IL-17RC, and exons 7-9 of human IL-17RA, and Fc5
<400> 118
   Met Pro Val Pro Trp Phe Leu Leu Ser Leu Ala Leu Gly Arg Ser Pro
    1               5                  10                  15
   Val Val Leu Ser Leu Glu Arg Leu Val Gly Pro Gln Asp Ala Thr His
               20                  25                  30
   Cys Ser Pro Gly Leu Ser Cys Arg Leu Trp Asp Ser Asp Ile Leu Cys
           35                  40                  45
   Leu Pro Gly Asp Ile Val Pro Ala Pro Gly Pro Val Leu Ala Pro Thr
       50                  55                  60
   His Leu Gln Thr Glu Leu Val Leu Arg Cys Gln Lys Glu Thr Asp Cys
   65                  70                  75                  80
   Asp Leu Cys Leu Arg Val Ala Val His Leu Ala Val His Gly His Trp
                   85                  90                  95
   Glu Glu Pro Glu Asp Glu Glu Lys Phe Gly Gly Ala Ala Asp Ser Gly
               100                 105                 110
   Val Glu Glu Pro Arg Asn Ala Ser Leu Gln Ala Gln Val Val Leu Ser
           115                 120                 125
   Phe Gln Ala Tyr Pro Thr Ala Arg Cys Val Leu Leu Glu Val Gln Val
       130                 135                 140
   Pro Ala Ala Leu Val Gln Phe Gly Gln Ser Val Gly Ser Val Val Tyr
   145                 150                 155                 160
   Asp Cys Phe Glu Ala Ala Leu Gly Ser Glu Val Arg Ile Trp Ser Tyr
                   165                 170                 175
   Thr Gln Pro Arg Tyr Glu Lys Glu Leu Asn His Thr Gln Gln Leu Pro
               180                 185                 190
   Asp Cys Arg Gly Leu Glu Val Trp Asn Ser Ile Pro Ser Cys Trp Ala
           195                 200                 205
   Leu Pro Trp Leu Asn Val Ser Ala Asp Gly Asp Asn Val His Leu Val
       210                 215                 220
   Leu Asn Val Ser Glu Glu Gln His Phe Gly Leu Ser Leu Tyr Trp Asn
   225                 230                 235                 240
   Gln Val Gln Gly Pro Pro Lys Pro Arg Trp His Lys Asn Leu Thr Gly
                   245                 250                 255
   Pro Gln Ile Ile Thr Leu Asn His Thr Asp Leu Val Pro Cys Leu Cys
               260                 265                 270
   Ile Gln Val Trp Pro Leu Glu Pro Asp Ser Val Arg Thr Asn Ile Cys
           275                 280                 285
   Pro Phe Arg Glu Asp Pro Arg Ala His Gln Asn Leu Trp Gln Ala Ala
       290                 295                 300
   Arg Leu Arg Leu Leu Thr Leu Gln Ser Trp Leu Leu Asp Ala Pro Cys
   305                 310                 315                 320
   Ser Leu Pro Ala Glu Ala Ala Leu Cys Trp Arg Ala Pro Gly Gly Asp
                   325                 330                 335
   Pro Cys Gln Pro Leu Val Pro Pro Leu Ser Trp Glu Asn Val Thr Val
               340                 345                 350
   Asp Lys Val Leu Glu Phe Pro Leu Leu Lys Gly His Pro Asn Leu Cys
           355                 360                 365
   Val Gln Val Asn Ser Ser Glu Lys Leu Gln Leu Gln Glu Cys Leu Trp
       370                 375                 380
   Ala Gly Ser Leu Trp Asp Pro Asn Ile Thr Val Glu Thr Leu Asp Thr
   385                 390                 395                 400
   Gln His Leu Arg Val Asp Phe Thr Leu Trp Asn Glu Ser Thr Pro Tyr
                   405                 410                 415
   Gln Val Leu Leu Glu Ser Phe Ser Asp Ser Glu Asn His Ser Cys Phe
               420                 425                 430
   Asp Val Val Lys Gln Ile Phe Ala Pro Arg Gln Glu Glu Phe His Gln
           435                 440                 445
   Arg Ala Asn Val Thr Phe Thr Leu Ser Lys Phe His Trp Cys Cys His
       450                 455                 460
   His His Val Gln Val Gln Pro Phe Phe Ser Ser Cys Leu Asn Asp Cys
   465                 470                 475                 480
   Leu Arg His Ala Val Thr Val Pro Cys Pro Val Ile Ser Asn Thr Thr
                   485                 490                 495
   Val Pro Lys Pro Val Ala Asp Tyr Ile Pro Leu Trp Glu Pro Lys Ser
               500                 505                 510
   Ser Asp Lys Thr His Thr Cys Pro Pro Cys Pro Ala Pro Glu Ala Glu
           515                 520                 525
   Gly Ala Pro Ser Val Phe Leu Phe Pro Pro Lys Pro Lys Asp Thr Leu
       530                 535                 540
   Met Ile Ser Arg Thr Pro Glu Val Thr Cys Val Val Val Asp Val Ser
   545                 550                 555                 560
   His Glu Asp Pro Glu Val Lys Phe Asn Trp Tyr Val Asp Gly Val Glu
                   565                 570                 575
   Val His Asn Ala Lys Thr Lys Pro Arg Glu Glu Gln Tyr Asn Ser Thr
               580                 585                 590
   Tyr Arg Val Val Ser Val Leu Thr Val Leu His Gln Asp Trp Leu Asn
           595                 600                 605
   Gly Lys Glu Tyr Lys Cys Lys Val Ser Asn Lys Ala Leu Pro Ser Ser
       610                 615                 620
   Ile Glu Lys Thr Ile Ser Lys Ala Lys Gly Gln Pro Arg Glu Pro Gln
   625                 630                 635                 640
   Val Tyr Thr Leu Pro Pro Ser Arg Asp Glu Leu Thr Lys Asn Gln Val
                   645                 650                 655
   Ser Leu Thr Cys Leu Val Lys Gly Phe Tyr Pro Ser Asp Ile Ala Val
               660                 665                 670
   Glu Trp Glu Ser Asn Gly Gln Pro Glu Asn Asn Tyr Lys Thr Thr Pro
           675                 680                 685
   Pro Val Leu Asp Ser Asp Gly Ser Phe Phe Leu Tyr Ser Lys Leu Thr
       690                 695                 700
   Val Asp Lys Ser Arg Trp Gln Gln Gly Asn Val Phe Ser Cys Ser Val
   705                 710                 715                 720
   Met His Glu Ala Leu His Asn His Tyr Thr Gln Lys Ser Leu Ser Leu
                   725                 730                 735
   Ser Pro Gly Lys
               740
<210> 119
   <211> 1500
   <212> DNA
   <213> Artificial Sequence
<220>
   <223> Murine IL-17RA signal peptide and exons 1-6 of murine IL-17RA, exons 8-13 of human IL-17RC, and exons 7-9 of murine Il-17RA
<400> 119
   atggcgattc ggcgctgctg gccacgggtc gtccccgggc ccgcgctggg atggctgctt 60
   ctgctgctga acgttctggc cccgggccgc gcctccccgc gcctcctcga cttcccggct 120
   ccggtctgcg cgcaggaggg gctgagctgc agagtcaaga atagtacttg tctggatgac 180
   agctggatcc accccaaaaa cctgaccccg tcttccccaa aaaacatcta tatcaatctt 240
   agtgtttcct ctacccagca cggagaatta gtccctgtgt tgcatgttga gtggaccctg 300
   cagacagatg ccagcatcct gtacctcgag ggtgcagagc tgtccgtcct gcagctgaac 360
   accaatgagc ggctgtgtgt caagttccag tttctgtcca tgctgcagca tcaccgtaag 420
   cggtggcggt tttccttcag ccactttgtg gtagatcctg gccaggagta tgaagtgact 480
   gttcaccacc tgccgaagcc catccctgat ggggacccaa accacaaatc caagatcatc 540
   tttgtgcctg actgtgagga cagcaagatg aagatgacta cctcatgcgt gagctcagcc 600
   ctgccctggc tcaacgtgtc agcagatggt gacaacgtgc atctggttct gaatgtctct 660
   gaggagcagc acttcggcct ctccctgtac tggaatcagg tccagggccc cccaaaaccc 720
   cggtggcaca aaaacctgac tggaccgcag atcattacct tgaaccacac agacctggtt 780
   ccctgcctct gtattcaggt gtggcctctg gaacctgact ccgttaggac gaacatctgc 840
   cccttcaggg aggacccccg cgcacaccag aacctctggc aagccgcccg actgcgactg 900
   ctgaccctgc agagctggct gctggacgca ccgtgctcgc tgcccgcaga agcggcactg 960
   tgctggcggg ctccgggtgg ggacccctgc cagccactgg tcccaccgct ttcctgggag 1020
   aacgtcactg tggacaaggt tctcgagttc ccattgctga aaggccaccc taacctctgt 1080
   gttcaggtga acagctcgga gaagctgcag ctgcaggagt gcttgtgggc tggcagcctt 1140
   tgggatccca acatcactgt ggagaccttg gacacacagc atctgcgagt ggacttcacc 1200
   ctgtggaatg aatccacccc ctaccaggtc ctgctggaaa gtttctccga ctcagagaac 1260
   cacagctgct ttgatgtcgt taaacaaata tttgcgccca ggcaagaaga attccatcag 1320
   cgagctaatg tcacattcac tctaagcaag tttcactggt gctgccatca ccacgtgcag 1380
   gtccagccct tcttcagcag ctgcctaaat gactgtttga gacacgctgt gactgtgccc 1440
   tgcccagtaa tctcaaatac cacagttccc aagccagttg cagactacat tcccctgtgg 1500

<210> 120
   <211> 500
   <212> PRT
   <213> Artificial Sequence
<220>
   <223> Murine IL-17RA signal peptide and exon 1-6 of murine IL-17RA, exons 8-13 of human IL-17RC, and exons 7-9 of murine Il-17RA
<400> 120
   Met Ala Ile Arg Arg Cys Trp Pro Arg Val Val Pro Gly Pro Ala Leu
    1               5                  10                  15
   Gly Trp Leu Leu Leu Leu Leu Asn Val Leu Ala Pro Gly Arg Ala Ser
               20                  25                  30
   Pro Arg Leu Leu Asp Phe Pro Ala Pro Val Cys Ala Gln Glu Gly Leu
           35                  40                  45
   Ser Cys Arg Val Lys Asn Ser Thr Cys Leu Asp Asp Ser Trp Ile His
       50                  55                  60
   Pro Lys Asn Leu Thr Pro Ser Ser Pro Lys Asn Ile Tyr Ile Asn Leu
   65                  70                  75                  80
   Ser Val Ser Ser Thr Gln His Gly Glu Leu Val Pro Val Leu His Val
                   85                  90                  95
   Glu Trp Thr Leu Gln Thr Asp Ala Ser Ile Leu Tyr Leu Glu Gly Ala
               100                 105                 110
   Glu Leu Ser Val Leu Gln Leu Asn Thr Asn Glu Arg Leu Cys Val Lys
           115                 120                 125
   Phe Gln Phe Leu Ser Met Leu Gln His His Arg Lys Arg Trp Arg Phe
       130                 135                 140
   Ser Phe Ser His Phe Val Val Asp Pro Gly Gln Glu Tyr Glu Val Thr
   145                 150                 155                 160
   Val His His Leu Pro Lys Pro Ile Pro Asp Gly Asp Pro Asn His Lys
                   165                 170                 175
   Ser Lys Ile Ile Phe Val Pro Asp Cys Glu Asp Ser Lys Met Lys Met
               180                 185                 190
   Thr Thr Ser Cys Val Ser Ser Ala Leu Pro Trp Leu Asn Val Ser Ala
           195                 200                 205
   Asp Gly Asp Asn Val His Leu Val Leu Asn Val Ser Glu Glu Gln His
       210                 215                 220
   Phe Gly Leu Ser Leu Tyr Trp Asn Gln Val Gln Gly Pro Pro Lys Pro
   225                 230                 235                 240
   Arg Trp His Lys Asn Leu Thr Gly Pro Gln Ile Ile Thr Leu Asn His
                   245                 250                 255
   Thr Asp Leu Val Pro Cys Leu Cys Ile Gln Val Trp Pro Leu Glu Pro
               260                 265                 270
   Asp Ser Val Arg Thr Asn Ile Cys Pro Phe Arg Glu Asp Pro Arg Ala
           275                 280                 285
   His Gln Asn Leu Trp Gln Ala Ala Arg Leu Arg Leu Leu Thr Leu Gln
       290                 295                 300
   Ser Trp Leu Leu Asp Ala Pro Cys Ser Leu Pro Ala Glu Ala Ala Leu
   305                 310                 315                 320
   Cys Trp Arg Ala Pro Gly Gly Asp Pro Cys Gln Pro Leu Val Pro Pro
                           325                         330                         335
   Leu Ser Trp Glu Asn Val Thr Val Asp Lys Val Leu Glu Phe Pro Leu
                   340                         345                         350
   Leu Lys Gly His Pro Asn Leu Cys Val Gln Val Asn Ser Ser Glu Lys
               355                         360                         365
   Leu Gln Leu Gln Glu Cys Leu Trp Ala Gly Ser Leu Trp Asp Pro Asn
       370                         375                         380
   Ile Thr Val Glu Thr Leu Asp Thr Gln His Leu Arg Val Asp Phe Thr
   385                     390                         395                         400
   Leu Trp Asn Glu Ser Thr Pro Tyr Gln Val Leu Leu Glu Ser Phe Ser
                       405                         410                         415
   Asp Ser Glu Asn His Ser Cys Phe Asp Val Val Lys Gln Ile Phe Ala
                   420                         425                         430
   Pro Arg Gln Glu Glu Phe His Gln Arg Ala Asn Val Thr Phe Thr Leu
               435                         440                         445
   Ser Lys Phe His Trp Cys Cys His His His Val Gln Val Gln Pro Phe
       450                         455                         460
   Phe Ser Ser Cys Leu Asn Asp Cys Leu Arg His Ala Val Thr Val Pro
   465                     470                         475                         480
   Cys Pro Val Ile Ser Asn Thr Thr Val Pro Lys Pro Val Ala Asp Tyr
                       485                         490                         495
   Ile Pro Leu Trp
                   500
<210> 121
   <211> 2196
   <212> DNA
   <213> Artificial Sequence
<220>
   <223> Murine IL-17RA signal peptide and exons 1-6 of murine IL-17RA, exons 8-13 of human IL-17RC, and exons 7-9 of murine Il-17RA and Fc5
<400> 121
   atggcgattc ggcgctgctg gccacgggtc gtccccgggc ccgcgctggg atggctgctt 60
   ctgctgctga acgttctggc cccgggccgc gcctccccgc gcctcctcga cttcccggct 120
   ccggtctgcg cgcaggaggg gctgagctgc agagtcaaga atagtacttg tctggatgac 180
   agctggatcc accccaaaaa cctgaccccg tcttccccaa aaaacatcta tatcaatctt 240
   agtgtttcct ctacccagca cggagaatta gtccctgtgt tgcatgttga gtggaccctg 300
   cagacagatg ccagcatcct gtacctcgag ggtgcagagc tgtccgtcct gcagctgaac 360
   accaatgagc ggctgtgtgt caagttccag tttctgtcca tgctgcagca tcaccgtaag 420
   cggtggcggt tttccttcag ccactttgtg gtagatcctg gccaggagta tgaagtgact 480
   gttcaccacc tgccgaagcc catccctgat ggggacccaa accacaaatc caagatcatc 540
   tttgtgcctg actgtgagga cagcaagatg aagatgacta cctcatgcgt gagctcagcc 600
   ctgccctggc tcaacgtgtc agcagatggt gacaacgtgc atctggttct gaatgtctct 660
   gaggagcagc acttcggcct ctccctgtac tggaatcagg tccagggccc cccaaaaccc 720
   cggtggcaca aaaacctgac tggaccgcag atcattacct tgaaccacac agacctggtt 780
   ccctgcctct gtattcaggt gtggcctctg gaacctgact ccgttaggac gaacatctgc 840
   cccttcaggg aggacccccg cgcacaccag aacctctggc aagccgcccg actgcgactg 900
   ctgaccctgc agagctggct gctggacgca ccgtgctcgc tgcccgcaga agcggcactg 960
   tgctggcggg ctccgggtgg ggacccctgc cagccactgg tcccaccgct ttcctgggag 1020
   aacgtcactg tggacaaggt tctcgagttc ccattgctga aaggccaccc taacctctgt 1080
   gttcaggtga acagctcgga gaagctgcag ctgcaggagt gcttgtgggc tggcagcctt 1140
   tgggatccca acatcactgt ggagaccttg gacacacagc atctgcgagt ggacttcacc 1200
   ctgtggaatg aatccacccc ctaccaggtc ctgctggaaa gtttctccga ctcagagaac 1260
   cacagctgct ttgatgtcgt taaacaaata tttgcgccca ggcaagaaga attccatcag 1320
   cgagctaatg tcacattcac tctaagcaag tttcactggt gctgccatca ccacgtgcag 1380
   gtccagccct tcttcagcag ctgcctaaat gactgtttga gacacgctgt gactgtgccc 1440
   tgcccagtaa tctcaaatac cacagttccc aagccagttg cagactacat tcccctgtgg 1500
   gagcccaaat cttcagacaa aactcacaca tgcccaccgt gcccagcacc tgaagccgag 1560
   ggggcaccgt cagtcttcct cttcccccca aaacccaagg acaccctcat gatctcccgg 1620
   acccctgagg tcacatgcgt ggtggtggac gtgagccacg aagaccctga ggtcaagttc 1680
   aactggtacg tggacggcgt ggaggtgcat aatgccaaga caaagccgcg ggaggagcag 1740
   tacaacagca cgtaccgtgt ggtcagcgtc ctcaccgtcc tgcaccagga ctggctgaat 1800
   ggcaaggagt acaagtgcaa ggtctccaac aaagccctcc catcctccat cgagaaaacc 1860
   atctccaaag ccaaagggca gccccgagaa ccacaggtgt acaccctgcc cccatcccgg 1920
   gatgagctga ccaagaacca ggtcagcctg acctgcctgg tcaaaggctt ctatcccagc 1980
   gacatcgccg tggagtggga gagcaatggg cagccggaga acaactacaa gaccacgcct 2040
   cccgtgctgg actccgacgg ctccttcttc ctctacagca agctcaccgt ggacaagagc 2100
   aggtggcagc aggggaacgt cttctcatgc tccgtgatgc atgaggctct gcacaaccac 2160
   tacacgcaga agagcctctc cctgtctccg ggtaaa 2196
<210> 122
   <211> 2196
   <212> PRT
   <213> Artificial Sequence
<220>
   <223> Murine IL-17RA signal peptide and exons 1-6 of murine IL-17RA, exons 8-13 of human IL-17RC, and exons 7-9 of murine Il-17RA and Fc5
<400> 122
   Ala Thr Gly Gly Cys Gly Ala Thr Thr Cys Gly Gly Cys Gly Cys Thr
    1               5                  10                  15
   Gly Cys Thr Gly Gly Cys Cys Ala Cys Gly Gly Gly Thr Cys Gly Thr
               20                  25                  30
   Cys Cys Cys Cys Gly Gly Gly Cys Cys Cys Gly Cys Gly Cys Thr Gly
           35                  40                  45
   Gly Gly Ala Thr Gly Gly Cys Thr Gly Cys Thr Thr Cys Thr Gly Cys
       50                  55                  60
   Thr Gly Cys Thr Gly Ala Ala Cys Gly Thr Thr Cys Thr Gly Gly Cys
   65                  70                  75                  80
   Cys Cys Cys Gly Gly Gly Cys Cys Gly Cys Gly Cys Cys Thr Cys Cys
                   85                  90                  95
   Cys Cys Gly Cys Gly Cys Cys Thr Cys Cys Thr Cys Gly Ala Cys Thr
               100                 105                 110
   Thr Cys Cys Cys Gly Gly Cys Thr Cys Cys Gly Gly Thr Cys Thr Gly
           115                 120                 125
   Cys Gly Cys Gly Cys Ala Gly Gly Ala Gly Gly Gly Gly Cys Thr Gly
       130                 135                 140
   Ala Gly Cys Thr Gly Cys Ala Gly Ala Gly Thr Cys Ala Ala Gly Ala
   145                 150                 155                 160
   Ala Thr Ala Gly Thr Ala Cys Thr Thr Gly Thr Cys Thr Gly Gly Ala
                   165                 170                 175
   Thr Gly Ala Cys Ala Gly Cys Thr Gly Gly Ala Thr Cys Cys Ala Cys
               180                 185                 190
   Cys Cys Cys Ala Ala Ala Ala Ala Cys Cys Thr Gly Ala Cys Cys Cys
           195                 200                 205
   Cys Gly Thr Cys Thr Thr Cys Cys Cys Cys Ala Ala Ala Ala Ala Ala
       210                 215                 220
   Cys Ala Thr Cys Thr Ala Thr Ala Thr Cys Ala Ala Thr Cys Thr Thr
   225                 230                 235                 240
   Ala Gly Thr Gly Thr Thr Thr Cys Cys Thr Cys Thr Ala Cys Cys Cys
                   245                 250                 255
   Ala Gly Cys Ala Cys Gly Gly Ala Gly Ala Ala Thr Thr Ala Gly Thr
               260                 265                 270
   Cys Cys Cys Thr Gly Thr Gly Thr Thr Gly Cys Ala Thr Gly Thr Thr
   275 280 285
   Gly Ala Gly Thr Gly Gly Ala Cys Cys Cys Thr Gly Cys Ala Gly Ala
   290 295 300
   Cys Ala Gly Ala Thr Gly Cys Cys Ala Gly Cys Ala Thr Cys Cys Thr
   305 310 315 320
   Gly Thr Ala Cys Cys Thr Cys Gly Ala Gly Gly Gly Thr Gly Cys Ala
   325 330 335
   Gly Ala Gly Cys Thr Gly Thr Cys Cys Gly Thr Cys Cys Thr Gly Cys
   340 345 350
   Ala Gly Cys Thr Gly Ala Ala Cys Ala Cys Cys Ala Ala Thr Gly Ala
   355 360 365
   Gly Cys Gly Gly Cys Thr Gly Thr Gly Thr Gly Thr Cys Ala Ala Gly
   370 375 380
   Thr Thr Cys Cys Ala Gly Thr Thr Thr Cys Thr Gly Thr Cys Cys Ala
   385 390 395 400
   Thr Gly Cys Thr Gly Cys Ala Gly Cys Ala Thr Cys Ala Cys Cys Gly
   405 410 415
   Thr Ala Ala Gly Cys Gly Gly Thr Gly Gly Cys Gly Gly Thr Thr Thr
   420 425 430
   Thr Cys Cys Thr Thr Cys Ala Gly Cys Cys Ala Cys Thr Thr Thr Gly
   435 440 445
   Thr Gly Gly Thr Ala Gly Ala Thr Cys Cys Thr Gly Gly Cys Cys Ala
   450 455 460
   Gly Gly Ala Gly Thr Ala Thr Gly Ala Ala Gly Thr Gly Ala Cys Thr
   465 470 475 480
   Gly Thr Thr Cys Ala Cys Cys Ala Cys Cys Thr Gly Cys Cys Gly Ala
   485 490 495
   Ala Gly Cys Cys Cys Ala Thr Cys Cys Cys Thr Gly Ala Thr Gly Gly
   500 505 510
   Gly Gly Ala Cys Cys Cys Ala Ala Ala Cys Cys Ala Cys Ala Ala Ala
   515 520 525
   Thr Cys Cys Ala Ala Gly Ala Thr Cys Ala Thr Cys Thr Thr Thr Gly
   530 535 540
   Thr Gly Cys Cys Thr Gly Ala Cys Thr Gly Thr Gly Ala Gly Gly Ala
   545 550 555 560
   Cys Ala Gly Cys Ala Ala Gly Ala Thr Gly Ala Ala Gly Ala Thr Gly
   565 570 575
   Ala Cys Thr Ala Cys Cys Thr Cys Ala Thr Gly Cys Gly Thr Gly Ala
   580 585 590
   Gly Cys Thr Cys Ala Gly Cys Cys Cys Thr Gly Cys Cys Cys Thr Gly
   595 600 605
   Gly Cys Thr Cys Ala Ala Cys Gly Thr Gly Thr Cys Ala Gly Cys Ala
   610 615 620
   Gly Ala Thr Gly Gly Thr Gly Ala Cys Ala Ala Cys Gly Thr Gly Cys
   625 630 635 640
   Ala Thr Cys Thr Gly Gly Thr Thr Cys Thr Gly Ala Ala Thr Gly Thr
   645 650 655
   Cys Thr Cys Thr Gly Ala Gly Gly Ala Gly Cys Ala Gly Cys Ala Cys
   660 665 670
   Thr Thr Cys Gly Gly Cys Cys Thr Cys Thr Cys Cys Cys Thr Gly Thr
   675 680 685
   Ala Cys Thr Gly Gly Ala Ala Thr Cys Ala Gly Gly Thr Cys Cys Ala
   690 695 700
   Gly Gly Gly Cys Cys Cys Cys Cys Cys Ala Ala Ala Ala Cys Cys Cys
   705 710 715 720
   Cys Gly Gly Thr Gly Gly Cys Ala Cys Ala Ala Ala Ala Ala Cys Cys
   725 730 735
   Thr Gly Ala Cys Thr Gly Gly Ala Cys Cys Gly Cys Ala Gly Ala Thr
   740 745 750
   Cys Ala Thr Thr Ala Cys Cys Thr Thr Gly Ala Ala Cys Cys Ala Cys
   755 760 765
   Ala Cys Ala Gly Ala Cys Cys Thr Gly Gly Thr Thr Cys Cys Cys Thr
   770 775 780
   Gly Cys Cys Thr Cys Thr Gly Thr Ala Thr Thr Cys Ala Gly Gly Thr
   785 790 795 800
   Gly Thr Gly Gly Cys Cys Thr Cys Thr Gly Gly Ala Ala Cys Cys Thr
   805 810 815
   Gly Ala Cys Thr Cys Cys Gly Thr Thr Ala Gly Gly Ala Cys Gly Ala
   820 825 830
   Ala Cys Ala Thr Cys Thr Gly Cys Cys Cys Cys Thr Thr Cys Ala Gly
   835 840 845
   Gly Gly Ala Gly Gly Ala Cys Cys Cys Cys Cys Gly Cys Gly Cys Ala
   850 855 860
   Cys Ala Cys Cys Ala Gly Ala Ala Cys Cys Thr Cys Thr Gly Gly Cys
   865 870 875 880
   Ala Ala Gly Cys Cys Gly Cys Cys Cys Gly Ala Cys Thr Gly Cys Gly
   885 890 895
   Ala Cys Thr Gly Cys Thr Gly Ala Cys Cys Cys Thr Gly Cys Ala Gly
   900 905 910
   Ala Gly Cys Thr Gly Gly Cys Thr Gly Cys Thr Gly Gly Ala Cys Gly
   915 920 925
   Cys Ala Cys Cys Gly Thr Gly Cys Thr Cys Gly Cys Thr Gly Cys Cys
   930 935 940
   Cys Gly Cys Ala Gly Ala Ala Gly Cys Gly Gly Cys Ala Cys Thr Gly
   945 950 955 960
   Thr Gly Cys Thr Gly Gly Cys Gly Gly Gly Cys Thr Cys Cys Gly Gly
   965 970 975
   Gly Thr Gly Gly Gly Gly Ala Cys Cys Cys Cys Thr Gly Cys Cys Ala
   980 985 990
   Gly Cys Cys Ala Cys Thr Gly Gly Thr Cys Cys Cys Ala Cys Cys Gly
   995 1000 1005
   Cys Thr Thr Thr Cys Cys Thr Gly Gly Gly Ala Gly Ala Ala Cys Gly
   1010 1015 1020
   Thr Cys Ala Cys Thr Gly Thr Gly Gly Ala Cys Ala Ala Gly Gly Thr
   1025 1030 1035 1040
   Thr Cys Thr Cys Gly Ala Gly Thr Thr Cys Cys Cys Ala Thr Thr Gly
   1045 1050 1055
   Cys Thr Gly Ala Ala Ala Gly Gly Cys Cys Ala Cys Cys Cys Thr Ala
   1060 1065 1070
   Ala Cys Cys Thr Cys Thr Gly Thr Gly Thr Thr Cys Ala Gly Gly Thr
   1075 1080 1085
   Gly Ala Ala Cys Ala Gly Cys Thr Cys Gly Gly Ala Gly Ala Ala Gly
   1090 1095 1100
   Cys Thr Gly Cys Ala Gly Cys Thr Gly Cys Ala Gly Gly Ala Gly Thr
   1105 1110 1115 1120
   Gly Cys Thr Thr Gly Thr Gly Gly Gly Cys Thr Gly Gly Cys Ala Gly
   1125 1130 1135
   Cys Cys Thr Thr Thr Gly Gly Gly Ala Thr Cys Cys Cys Ala Ala Cys
   1140 1145 1150
   Ala Thr Cys Ala Cys Thr Gly Thr Gly Gly Ala Gly Ala Cys Cys Thr
   1155 1160 1165
   Thr Gly Gly Ala Cys Ala Cys Ala Cys Ala Gly Cys Ala Thr Cys Thr
   1170 1175 1180
   Gly Cys Gly Ala Gly Thr Gly Gly Ala Cys Thr Thr Cys Ala Cys Cys
   1185 1190 1195 1200
   Cys Thr Gly Thr Gly Gly Ala Ala Thr Gly Ala Ala Thr Cys Cys Ala
   1205 1210 1215
   Cys Cys Cys Cys Cys Thr Ala Cys Cys Ala Gly Gly Thr Cys Cys Thr
   1220 1225 1230
   Gly Cys Thr Gly Gly Ala Ala Ala Gly Thr Thr Thr Cys Thr Cys Cys
           1235                    1240                    1245
   Gly Ala Cys Thr Cys Ala Gly Ala Gly Ala Ala Cys Cys Ala Cys Ala
       1250                    1255                    1260
   Gly Cys Thr Gly Cys Thr Thr Thr Gly Ala Thr Gly Thr Cys Gly Thr
   1265                    1270                    1275                1280
   Thr Ala Ala Ala Cys Ala Ala Ala Thr Ala Thr Thr Thr Gly Cys Gly
                   1285                    1290                    1295
   Cys Cys Cys Ala Gly Gly Cys Ala Ala Gly Ala Ala Gly Ala Ala Thr
               1300                    1305                    1310
   Thr Cys Cys Ala Thr Cys Ala Gly Cys Gly Ala Gly Cys Thr Ala Ala
           1315                    1320                    1325
   Thr Gly Thr Cys Ala Cys Ala Thr Thr Cys Ala Cys Thr Cys Thr Ala
       1330                    1335                    1340
   Ala Gly Cys Ala Ala Gly Thr Thr Thr Cys Ala Cys Thr Gly Gly Thr
   1345                    1350                    1355                1360
   Gly Cys Thr Gly Cys Cys Ala Thr Cys Ala Cys Cys Ala Cys Gly Thr
                   1365                    1370                    1375
   Gly Cys Ala Gly Gly Thr Cys Cys Ala Gly Cys Cys Cys Thr Thr Cys
               1380                    1385                    1390
   Thr Thr Cys Ala Gly Cys Ala Gly Cys Thr Gly Cys Cys Thr Ala Ala
           1395                    1400                    1405
   Ala Thr Gly Ala Cys Thr Gly Thr Thr Thr Gly Ala Gly Ala Cys Ala
       1410                    1415                    1420
   Cys Gly Cys Thr Gly Thr Gly Ala Cys Thr Gly Thr Gly Cys Cys Cys
   1425                    1430                    1435                1440
   Thr Gly Cys Cys Cys Ala Gly Thr Ala Ala Thr Cys Thr Cys Ala Ala
                   1445                    1450                    1455
   Ala Thr Ala Cys Cys Ala Cys Ala Gly Thr Thr Cys Cys Cys Ala Ala
               1460                    1465                    1470
   Gly Cys Cys Ala Gly Thr Thr Gly Cys Ala Gly Ala Cys Thr Ala Cys
           1475                    1480                    1485
   Ala Thr Thr Cys Cys Cys Cys Thr Gly Thr Gly Gly Gly Ala Gly Cys
       1490                    1495                    1500
   Cys Cys Ala Ala Ala Thr Cys Thr Thr Cys Ala Gly Ala Cys Ala Ala
   1505                    1510                    1515                1520
   Ala Ala Cys Thr Cys Ala Cys Ala Cys Ala Thr Gly Cys Cys Cys Ala
                   1525                    1530                    1535
   Cys Cys Gly Thr Gly Cys Cys Cys Ala Gly Cys Ala Cys Cys Thr Gly
               1540                    1545                    1550
   Ala Ala Gly Cys Cys Gly Ala Gly Gly Gly Gly Gly Cys Ala Cys Cys
           1555                    1560                    1565
   Gly Thr Cys Ala Gly Thr Cys Thr Thr Cys Cys Thr Cys Thr Thr Cys
       1570                    1575                    1580
   Cys Cys Cys Cys Cys Ala Ala Ala Ala Cys Cys Cys Ala Ala Gly Gly
   1585                    1590                    1595                1600
   Ala Cys Ala Cys Cys Cys Thr Cys Ala Thr Gly Ala Thr Cys Thr Cys
                   1605                    1610                    1615
   Cys Cys Gly Gly Ala Cys Cys Cys Cys Thr Gly Ala Gly Gly Thr Cys
               1620                    1625                    1630
   Ala Cys Ala Thr Gly Cys Gly Thr Gly Gly Thr Gly Gly Thr Gly Gly
           1635                    1640                    1645
   Ala Cys Gly Thr Gly Ala Gly Cys Cys Ala Cys Gly Ala Ala Gly Ala
       1650                    1655                    1660
   Cys Cys Cys Thr Gly Ala Gly Gly Thr Cys Ala Ala Gly Thr Thr Cys
   1665                    1670                    1675                1680
   Ala Ala Cys Thr Gly Gly Thr Ala Cys Gly Thr Gly Gly Ala Cys Gly
                   1685                    1690                    1695
   Gly Cys Gly Thr Gly Gly Ala Gly Gly Thr Gly Cys Ala Thr Ala Ala
               1700                    1705                    1710
   Thr Gly Cys Cys Ala Ala Gly Ala Cys Ala Ala Ala Gly Cys Cys Gly
               1715                    1720                    1725
   Cys Gly Gly Gly Ala Gly Gly Ala Gly Cys Ala Gly Thr Ala Cys Ala
           1730                    1735                    1740
   Ala Cys Ala Gly Cys Ala Cys Gly Thr Ala Cys Cys Gly Thr Gly Thr
   1745                    1750                    1755                    1760
   Gly Gly Thr Cys Ala Gly Cys Gly Thr Cys Cys Thr Cys Ala Cys Cys
                   1765                    1770                    1775
   Gly Thr Cys Cys Thr Gly Cys Ala Cys Cys Ala Gly Gly Ala Cys Thr
               1780                    1785                    1790
   Gly Gly Cys Thr Gly Ala Ala Thr Gly Gly Cys Ala Ala Gly Gly Ala
           1795                    1800                    1805
   Gly Thr Ala Cys Ala Ala Gly Thr Gly Cys Ala Ala Gly Gly Thr Cys
       1810                    1815                    1820
   Thr Cys Cys Ala Ala Cys Ala Ala Ala Gly Cys Cys Cys Thr Cys Cys
   1825                    1830                    1835                    1840
   Cys Ala Thr Cys Cys Thr Cys Cys Ala Thr Cys Gly Ala Gly Ala Ala
                   1845                    1850                    1855
   Ala Ala Cys Cys Ala Thr Cys Thr Cys Cys Ala Ala Ala Gly Cys Cys
               1860                    1865                    1870
   Ala Ala Ala Gly Gly Gly Cys Ala Gly Cys Cys Cys Cys Gly Ala Gly
           1875                    1880                    1885
   Ala Ala Cys Cys Ala Cys Ala Gly Gly Thr Gly Thr Ala Cys Ala Cys
       1890                    1895                    1900
   Cys Cys Thr Gly Cys Cys Cys Cys Cys Ala Thr Cys Cys Cys Gly Gly
   1905                    1910                    1915                    1920
   Gly Ala Thr Gly Ala Gly Cys Thr Gly Ala Cys Cys Ala Ala Gly Ala
                   1925                    1930                    1935
   Ala Cys Cys Ala Gly Gly Thr Cys Ala Gly Cys Cys Thr Gly Ala Cys
               1940                    1945                    1950
   Cys Thr Gly Cys Cys Thr Gly Gly Thr Cys Ala Ala Ala Gly Gly Cys
           1955                    1960                    1965
   Thr Thr Cys Thr Ala Thr Cys Cys Cys Ala Gly Cys Gly Ala Cys Ala
       1970                    1975                    1980
   Thr Cys Gly Cys Cys Gly Thr Gly Gly Ala Gly Thr Gly Gly Gly Ala
   1985                    1990                    1995                    2000
   Gly Ala Gly Cys Ala Ala Thr Gly Gly Gly Cys Ala Gly Cys Cys Gly
                   2005                    2010                    2015
   Gly Ala Gly Ala Ala Cys Ala Ala Cys Thr Ala Cys Ala Ala Gly Ala
               2020                    2025                    2030
   Cys Cys Ala Cys Gly Cys Cys Thr Cys Cys Cys Gly Thr Gly Cys Thr
           2035                    2040                    2045
   Gly Gly Ala Cys Thr Cys Cys Gly Ala Cys Gly Gly Cys Thr Cys Cys
       2050                    2055                    2060
   Thr Thr Cys Thr Thr Cys Cys Thr Cys Thr Ala Cys Ala Gly Cys Ala
   2065                    2070                    2075                    2080
   Ala Gly Cys Thr Cys Ala Cys Cys Gly Thr Gly Gly Ala Cys Ala Ala
                   2085                    2090                    2095
   Gly Ala Gly Cys Ala Gly Gly Thr Gly Gly Cys Ala Gly Cys Ala Gly
               2100                    2105                    2110
   Gly Gly Gly Ala Ala Cys Gly Thr Cys Thr Thr Cys Thr Cys Ala Thr
           2115                    2120                    2125
   Gly Cys Thr Cys Cys Gly Thr Gly Ala Thr Gly Cys Ala Thr Gly Ala
       2130                    2135                    2140
   Gly Gly Cys Thr Cys Thr Gly Cys Ala Cys Ala Ala Cys Cys Ala Cys
   2145                    2150                    2155                    2160
   Thr Ala Cys Ala Cys Gly Cys Ala Gly Ala Ala Gly Ala Gly Cys Cys
                   2165                    2170                    2175
   Thr Cys Thr Cys Cys Cys Thr Gly Thr Cys Thr Cys Cys Gly Gly Gly
               2180                    2185                    2190
   Thr Ala Ala Ala
   2195
<210> 123
   <211> 1272
   <212> DNA
   <213> Artificial Sequence
<220>
   <223> IL-17RC signal peptide and exons 1-6 of human IL-17RC, and Fc5
<400> 123
   atgcctgtgc cctggttctt gctgtccttg gcactgggcc gaagcccagt ggtcctttct 60
   ctggagaggc ttgtggggcc tcaggacgct acccactgct ctccgggcct ctcctgccgc 120
   ctctgggaca gtgacatact ctgcctgcct ggggacatcg tgcctgctcc gggccccgtg 180
   ctggcgccta cgcacctgca gacagagctg gtgctgaggt gccagaagga gaccgactgt 240
   gacctctgtc tgcgtgtggc tgtccacttg gccgtgcatg ggcactggga agagcctgaa 300
   gatgaggaaa agtttggagg agcagctgac tcaggggtgg aggagcctag gaatgcctct 360
   ctccaggccc aagtcgtgct ctccttccag gcctacccta ctgcccgctg cgtcctgctg 420
   gaggtgcaag tgcctgctgc ccttgtgcag tttggtcagt ctgtgggctc tgtggtatat 480
   gactgcttcg aggctgccct agggagtgag gtacgaatct ggtcctatac tcagcccagg 540
   tacgagaagg aactcaacca cacacagcag ctgcctgagc ccaaatcttc agacaaaact 600
   cacacatgcc caccgtgccc agcacctgaa gccgaggggg caccgtcagt cttcctcttc 660
   cccccaaaac ccaaggacac cctcatgatc tcccggaccc ctgaggtcac atgcgtggtg 720
   gtggacgtga gccacgaaga ccctgaggtc aagttcaact ggtacgtgga cggcgtggag 780
   gtgcataatg ccaagacaaa gccgcgggag gagcagtaca acagcacgta ccgtgtggtc 840
   agcgtcctca ccgtcctgca ccaggactgg ctgaatggca aggagtacaa gtgcaaggtc 900
   tccaacaaag ccctcccatc ctccatcgag aaaaccatct ccaaagccaa agggcagccc 960
   cgagaaccac aggtgtacac cctgccccca tcccgggatg agctgaccaa gaaccaggtc 1020
   agcctgacct gcctggtcaa aggcttctat cccagcgaca tcgccgtgga gtgggagagc 1080
   aatgggcagc cggagaacaa ctacaagacc acgcctcccg tgctggactc cgacggctcc 1140
   ttcttcctct acagcaagct caccgtggac aagagcaggt ggcagcaggg gaacgtcttc 1200
   tcatgctccg tgatgcatga ggctctgcac aaccactaca cgcagaagag cctctccctg 1260
   tctccgggta aa                                                     1272
<210> 124
   <211> 424
   <212> PRT
   <213> Artificial Sequence
<220>
   <223> IL-17RC signal peptide and exons 1-6 of human IL-17RC, and Fc5
<400> 124
   Met Pro Val Pro Trp Phe Leu Leu Ser Leu Ala Leu Gly Arg Ser Pro
   1               5                   10                  15
   Val Val Leu Ser Leu Glu Arg Leu Val Gly Pro Gln Asp Ala Thr His
               20                  25                  30
   Cys Ser Pro Gly Leu Ser Cys Arg Leu Trp Asp Ser Asp Ile Leu Cys
           35                  40                  45
   Leu Pro Gly Asp Ile Val Pro Ala Pro Gly Pro Val Leu Ala Pro Thr
       50                  55                  60
   His Leu Gln Thr Glu Leu Val Leu Arg Cys Gln Lys Glu Thr Asp Cys
   65                  70                  75                  80
   Asp Leu Cys Leu Arg Val Ala Val His Leu Ala Val His Gly His Trp
                   85                  90                  95
   Glu Glu Pro Glu Asp Glu Glu Lys Phe Gly Gly Ala Ala Asp Ser Gly
               100                 105                 110
   Val Glu Glu Pro Arg Asn Ala Ser Leu Gln Ala Gln Val Val Leu Ser
           115                 120                 125
   Phe Gln Ala Tyr Pro Thr Ala Arg Cys Val Leu Leu Glu Val Gln Val
       130                 135                 140
   Pro Ala Ala Leu Val Gln Phe Gly Gln Ser Val Gly Ser Val Val Tyr
   145                 150                 155                 160
   Asp Cys Phe Glu Ala Ala Leu Gly Ser Glu Val Arg Ile Trp Ser Tyr
                   165                 170                 175
   Thr Gln Pro Arg Tyr Glu Lys Glu Leu Asn His Thr Gln Gln Leu Pro
               180                 185                 190
   Glu Pro Lys Ser Ser Asp Lys Thr His Thr Cys Pro Pro Cys Pro Ala
           195                 200                 205
   Pro Glu Ala Glu Gly Ala Pro Ser Val Phe Leu Phe Pro Pro Lys Pro
       210                 215                 220
   Lys Asp Thr Leu Met Ile Ser Arg Thr Pro Glu Val Thr Cys Val Val
   225                 230                 235                 240
   Val Asp Val Ser His Glu Asp Pro Glu Val Lys Phe Asn Trp Tyr Val
                   245                 250                 255
   Asp Gly Val Glu Val His Asn Ala Lys Thr Lys Pro Arg Glu Glu Gln
               260                 265                 270
   Tyr Asn Ser Thr Tyr Arg Val Val Ser Val Leu Thr Val Leu His Gln
           275                 280                 285
   Asp Trp Leu Asn Gly Lys Glu Tyr Lys Cys Lys Val Ser Asn Lys Ala
       290                 295                 300
   Leu Pro Ser Ser Ile Glu Lys Thr Ile Ser Lys Ala Lys Gly Gln Pro
   305                 310                 315                 320
   Arg Glu Pro Gln Val Tyr Thr Leu Pro Pro Ser Arg Asp Glu Leu Thr
                   325                 330                 335
   Lys Asn Gln Val Ser Leu Thr Cys Leu Val Lys Gly Phe Tyr Pro Ser
               340                 345                 350
   Asp Ile Ala Val Glu Trp Glu Ser Asn Gly Gln Pro Glu Asn Asn Tyr
           355                 360                 365
   Lys Thr Thr Pro Pro Val Leu Asp Ser Asp Gly Ser Phe Phe Leu Tyr
       370                 375                 380
   Ser Lys Leu Thr Val Asp Lys Ser Arg Trp Gln Gln Gly Asn Val Phe
   385                 390                 395                 400
   Ser Cys Ser Val Met His Glu Ala Leu His Asn His Tyr Thr Gln Lys
                   405                 410                 415
   Ser Leu Ser Leu Ser Pro Gly Lys
               420
<210> 125
   <211> 1794
   <212> DNA
   <213> Artificial Sequence
<220>
   <223> IL-17RC signal peptide and exons 1-6 and 11-16 of human IL-17RC, and Fc5
<400> 125
   atgcctgtgc cctggttctt gctgtccttg gcactgggcc gaagcccagt ggtcctttct 60
   ctggagaggc ttgtggggcc tcaggacgct acccactgct ctccgggcct ctcctgccgc 120
   ctctgggaca gtgacatact ctgcctgcct ggggacatcg tgcctgctcc gggccccgtg 180
   ctggcgccta cgcacctgca gacagagctg gtgctgaggt gccagaagga gaccgactgt 240
   gacctctgtc tgcgtgtggc tgtccacttg gccgtgcatg ggcactggga agagcctgaa 300
   gatgaggaaa agtttggagg agcagctgac tcaggggtgg aggagcctag gaatgcctct 360
   ctccaggccc aagtcgtgct ctccttccag gcctacccta ctgcccgctg cgtcctgctg 420
   gaggtgcaag tgcctgctgc ccttgtgcag tttggtcagt ctgtgggctc tgtggtatat 480
   gactgcttcg aggctgccct agggagtgag gtacgaatct ggtcctatac tcagcccagg 540
   tacgagaagg aactcaacca cacacagcag ctgcctgacc cccgcgcaca ccagaacctc 600
   tggcaagccg cccgactgcg actgctgacc ctgcagagct ggctgctgga cgcaccgtgc 660
   tcgctgcccg cagaagcggc actgtgctgg cgggctccgg gtggggaccc ctgccagcca 720
   ctggtcccac cgctttcctg ggagaacgtc actgtggaca aggttctcga gttcccattg 780
   ctgaaaggcc accctaacct ctgtgttcag gtgaacagct cggagaagct gcagctgcag 840
   gagtgcttgt gggctgactc cctggggcct ctcaaagacg atgtgctact gttggagaca 900
   cgaggccccc aggacaacag atccctctgt gccttggaac ccagtggctg tacttcacta 960
   cccagcaaag cctccacgag ggcagctcgc cttggagagt acttactaca agacctgcag 1020
   tcaggccagt gtctgcagct atgggacgat gacttgggag cgctatgggc ctgccccatg 1080
   gacaaataca tccacaagga gcccaaatct tcagacaaaa ctcacacatg cccaccgtgc 1140
   ccagcacctg aagccgaggg ggcaccgtca gtcttcctct tccccccaaa acccaaggac 1200
   accctcatga tctcccggac ccctgaggtc acatgcgtgg tggtggacgt gagccacgaa 1260
   gaccctgagg tcaagttcaa ctggtacgtg gacggcgtgg aggtgcataa tgccaagaca 1320
   aagccgcggg aggagcagta caacagcacg taccgtgtgg tcagcgtcct caccgtcctg 1380
   caccaggact ggctgaatgg caaggagtac aagtgcaagg tctccaacaa agccctccca 1440
   tcctccatcg agaaaaccat ctccaaagcc aaagggcagc cccgagaacc acaggtgtac 1500
   accctgcccc catcccggga tgagctgacc aagaaccagg tcagcctgac ctgcctggtc 1560
   aaaggcttct atcccagcga catcgccgtg gagtgggaga gcaatgggca gccggagaac 1620
   aactacaaga ccacgcctcc cgtgctggac tccgacggct ccttcttcct ctacagcaag 1680
   ctcaccgtgg acaagagcag gtggcagcag gggaacgtct tctcatgctc cgtgatgcat 1740
   gaggctctgc acaaccacta cacgcagaag agcctctccc tgtctccggg taaa       1794
<210> 126
   <211> 598
   <212> PRT
   <213> Artificial Sequence
<220>
   <223> IL-17RC signal peptide and exons 1-6 and 11-16 of human IL-17RC, and Fc5
<400> 126
   Met Pro Val Pro Trp Phe Leu Leu Ser Leu Ala Leu Gly Arg Ser Pro
   1               5                   10                  15
   Val Val Leu Ser Leu Glu Arg Leu Val Gly Pro Gln Asp Ala Thr His
               20                  25                  30
   Cys Ser Pro Gly Leu Ser Cys Arg Leu Trp Asp Ser Asp Ile Leu Cys
           35                  40                  45
   Leu Pro Gly Asp Ile Val Pro Ala Pro Gly Pro Val Leu Ala Pro Thr
       50                  55                  60
   His Leu Gln Thr Glu Leu Val Leu Arg Cys Gln Lys Glu Thr Asp Cys
   65                  70                  75                  80
   Asp Leu Cys Leu Arg Val Ala Val His Leu Ala Val His Gly His Trp
                   85                  90                  95
   Glu Glu Pro Glu Asp Glu Glu Lys Phe Gly Gly Ala Ala Asp Ser Gly
               100                 105                 110
   Val Glu Glu Pro Arg Asn Ala Ser Leu Gln Ala Gln Val Val Leu Ser
           115                 120                 125
   Phe Gln Ala Tyr Pro Thr Ala Arg Cys Val Leu Leu Glu Val Gln Val
       130                 135                 140
   Pro Ala Ala Leu Val Gln Phe Gly Gln Ser Val Gly Ser Val Val Tyr
   145                 150                 155                 160
   Asp Cys Phe Glu Ala Ala Leu Gly Ser Glu Val Arg Ile Trp Ser Tyr
                   165                 170                 175
   Thr Gln Pro Arg Tyr Glu Lys Glu Leu Asn His Thr Gln Gln Leu Pro
               180                 185                 190
   Asp Pro Arg Ala His Gln Asn Leu Trp Gln Ala Ala Arg Leu Arg Leu
           195                 200                 205
   Leu Thr Leu Gln Ser Trp Leu Leu Asp Ala Pro Cys Ser Leu Pro Ala
       210                     215                     220
   Glu Ala Ala Leu Cys Trp Arg Ala Pro Gly Gly Asp Pro Cys Gln Pro
   225                 230                 235                 240
   Leu Val Pro Pro Leu Ser Trp Glu Asn Val Thr Val Asp Lys Val Leu
                   245                 250                 255
   Glu Phe Pro Leu Leu Lys Gly His Pro Asn Leu Cys Val Gln Val Asn
               260                 265                 270
   Ser Ser Glu Lys Leu Gln Leu Gln Glu Cys Leu Trp Ala Asp Ser Leu
           275                 280                 285
   Gly Pro Leu Lys Asp Asp Val Leu Leu Leu Glu Thr Arg Gly Pro Gln
       290                 295                 300
   Asp Asn Arg Ser Leu Cys Ala Leu Glu Pro Ser Gly Cys Thr Ser Leu
   305                 310                 315                 320
   Pro Ser Lys Ala Ser Thr Arg Ala Ala Arg Leu Gly Glu Tyr Leu Leu
                   325                 330                 335
   Gln Asp Leu Gln Ser Gly Gln Cys Leu Gln Leu Trp Asp Asp Asp Leu
               340                 345                 350
   Gly Ala Leu Trp Ala Cys Pro Met Asp Lys Tyr Ile His Lys Glu Pro
           355                 360                 365
   Lys Ser Ser Asp Lys Thr His Thr Cys Pro Pro Cys Pro Ala Pro Glu
       370                 375                 380
   Ala Glu Gly Ala Pro Ser Val Phe Leu Phe Pro Pro Lys Pro Lys Asp
   385                 390                 395                 400
   Thr Leu Met Ile Ser Arg Thr Pro Glu Val Thr Cys Val Val Val Asp
                   405                 410                 415
   Val Ser His Glu Asp Pro Glu Val Lys Phe Asn Trp Tyr Val Asp Gly
               420                 425                 430
   Val Glu Val His Asn Ala Lys Thr Lys Pro Arg Glu Glu Gln Tyr Asn
           435                 440                 445
   Ser Thr Tyr Arg Val Val Ser Val Leu Thr Val Leu His Gln Asp Trp
       450                 455                 460
   Leu Asn Gly Lys Glu Tyr Lys Cys Lys Val Ser Asn Lys Ala Leu Pro
   465                 470                 475                 480
   Ser Ser Ile Glu Lys Thr Ile Ser Lys Ala Lys Gly Gln Pro Arg Glu
                   485                 490                 495
   Pro Gln Val Tyr Thr Leu Pro Pro Ser Arg Asp Glu Leu Thr Lys Asn
               500                 505                 510
   Gln Val Ser Leu Thr Cys Leu Val Lys Gly Phe Tyr Pro Ser Asp Ile
           515                 520                 525
   Ala Val Glu Trp Glu Ser Asn Gly Gln Pro Glu Asn Asn Tyr Lys Thr
       530                 535                 540
   Thr Pro Pro Val Leu Asp Ser Asp Gly Ser Phe Phe Leu Tyr Ser Lys
   545                 550                 555                 560
   Leu Thr Val Asp Lys Ser Arg Trp Gln Gln Gly Asn Val Phe Ser Cys
                   565                 570                 575
   Ser Val Met His Glu Ala Leu His Asn His Tyr Thr Gln Lys Ser Leu
               580                 585                 590
   Ser Leu Ser Pro Gly Lys
           595
<210> 127
   <211> 1515
   <212> DNA
   <213> Artificial Sequence
<220>
   <223> IL-17RC signal peptide and exons 1-6 and 14-16 of human IL-17RC, and Fc5
<400> 127
   atgcctgtgc cctggttctt gctgtccttg gcactgggcc gaagcccagt ggtcctttct 60
   ctggagaggc ttgtggggcc tcaggacgct acccactgct ctccgggcct ctcctgccgc 120
   ctctgggaca gtgacatact ctgcctgcct ggggacatcg tgcctgctcc gggccccgtg 180
   ctggcgccta cgcacctgca gacagagctg gtgctgaggt gccagaagga gaccgactgt 240
   gacctctgtc tgcgtgtggc tgtccacttg gccgtgcatg ggcactggga agagcctgaa 300
   gatgaggaaa agtttggagg agcagctgac tcaggggtgg aggagcctag gaatgcctct 360
   ctccaggccc aagtcgtgct ctccttccag gcctacccta ctgcccgctg cgtcctgctg 420
   gaggtgcaag tgcctgctgc ccttgtgcag tttggtcagt ctgtgggctc tgtggtatat 480
   gactgcttcg aggctgccct agggagtgag gtacgaatct ggtcctatac tcagcccagg 540
   tacgagaagg aactcaacca cacacagcag ctgcctgact ccctggggcc tctcaaagac 600
   gatgtgctac tgttggagac acgaggcccc caggacaaca gatccctctg tgccttggaa 660
   cccagtggct gtacttcact acccagcaaa gcctccacga gggcagctcg ccttggagag 720
   tacttactac aagacctgca gtcaggccag tgtctgcagc tatgggacga tgacttggga 780
   gcgctatggg cctgccccat ggacaaatac atccacaagg agcccaaatc ttcagacaaa 840
   actcacacat gcccaccgtg cccagcacct gaagccgagg gggcaccgtc agtcttcctc 900
   ttccccccaa aacccaagga caccctcatg atctcccgga cccctgaggt cacatgcgtg 960
   gtggtggacg tgagccacga agaccctgag gtcaagttca actggtacgt ggacggcgtg 1020
   gaggtgcata atgccaagac aaagccgcgg gaggagcagt acaacagcac gtaccgtgtg 1080
   gtcagcgtcc tcaccgtcct gcaccaggac tggctgaatg gcaaggagta caagtgcaag 1140
   gtctccaaca aagccctccc atcctccatc gagaaaacca tctccaaagc caaagggcag 1200
   ccccgagaac cacaggtgta caccctgccc ccatcccggg atgagctgac caagaaccag 1260
   gtcagcctga cctgcctggt caaaggcttc tatcccagcg acatcgccgt ggagtgggag 1320
   agcaatgggc agccggagaa caactacaag accacgcctc ccgtgctgga ctccgacggc 1380
   tccttcttcc tctacagcaa gctcaccgtg gacaagagca ggtggcagca ggggaacgtc 1440
   ttctcatgct ccgtgatgca tgaggctctg cacaaccact acacgcagaa gagcctctcc 1500
   ctgtctccgg gtaaa                                                  1515
<210> 128
   <211> 505
   <212> PRT
   <213> Artificial Sequence
<220>
   <223> IL-17RC signal peptide and exons 1-6 and 14-16 of human IL-17RC, and Fc5
<400> 128
   Met Pro Val Pro Trp Phe Leu Leu Ser Leu Ala Leu Gly Arg Ser Pro
   1               5                   10                  15
   Val Val Leu Ser Leu Glu Arg Leu Val Gly Pro Gln Asp Ala Thr His
               20                  25                  30
   Cys Ser Pro Gly Leu Ser Cys Arg Leu Trp Asp Ser Asp Ile Leu Cys
           35                  40                  45
   Leu Pro Gly Asp Ile Val Pro Ala Pro Gly Pro Val Leu Ala Pro Thr
       50                  55                  60
   His Leu Gln Thr Glu Leu Val Leu Arg Cys Gln Lys Glu Thr Asp Cys
   65                  70                  75                  80
   Asp Leu Cys Leu Arg Val Ala Val His Leu Ala Val His Gly His Trp
                   85                  90                  95
   Glu Glu Pro Glu Asp Glu Glu Lys Phe Gly Gly Ala Ala Asp Ser Gly
               100                 105                 110
   Val Glu Glu Pro Arg Asn Ala Ser Leu Gln Ala Gln Val Val Leu Ser
           115                 120                 125
   Phe Gln Ala Tyr Pro Thr Ala Arg Cys Val Leu Leu Glu Val Gln Val
       130                 135                 140
   Pro Ala Ala Leu Val Gln Phe Gly Gln Ser Val Gly Ser Val Val Tyr
   145                 150                 155                 160
   Asp Cys Phe Glu Ala Ala Leu Gly Ser Glu Val Arg Ile Trp Ser Tyr
                   165                 170                 175
   Thr Gln Pro Arg Tyr Glu Lys Glu Leu Asn His Thr Gln Gln Leu Pro
               180                 185                 190
   Asp Ser Leu Gly Pro Leu Lys Asp Asp Val Leu Leu Leu Glu Thr Arg
           195                 200                 205
   Gly Pro Gln Asp Asn Arg Ser Leu Cys Ala Leu Glu Pro Ser Gly Cys
       210                 215                 220
   Thr Ser Leu Pro Ser Lys Ala Ser Thr Arg Ala Ala Arg Leu Gly Glu
   225                 230                 235                 240
   Tyr Leu Leu Gln Asp Leu Gln Ser Gly Gln Cys Leu Gln Leu Trp Asp
                   245                 250                 255
   Asp Asp Leu Gly Ala Leu Trp Ala Cys Pro Met Asp Lys Tyr Ile His
               260                 265                 270
   Lys Glu Pro Lys Ser Ser Asp Lys Thr His Thr Cys Pro Pro Cys Pro
           275                 280                 285
   Ala Pro Glu Ala Glu Gly Ala Pro Ser Val Phe Leu Phe Pro Pro Lys
       290                 295                 300
   Pro Lys Asp Thr Leu Met Ile Ser Arg Thr Pro Glu Val Thr Cys Val
   305                 310                 315                 320
   Val Val Asp Val Ser His Glu Asp Pro Glu Val Lys Phe Asn Trp Tyr
                   325                 330                 335
   Val Asp Gly Val Glu Val His Asn Ala Lys Thr Lys Pro Arg Glu Glu
               340                 345                 350
   Gln Tyr Asn Ser Thr Tyr Arg Val Val Ser Val Leu Thr Val Leu His
           355                 360                 365
   Gln Asp Trp Leu Asn Gly Lys Glu Tyr Lys Cys Lys Val Ser Asn Lys
       370                 375                 380
   Ala Leu Pro Ser Ser Ile Glu Lys Thr Ile Ser Lys Ala Lys Gly Gln
   385                 390                 395                 400
   Pro Arg Glu Pro Gln Val Tyr Thr Leu Pro Pro Ser Arg Asp Glu Leu
                   405                 410                 415
   Thr Lys Asn Gln Val Ser Leu Thr Cys Leu Val Lys Gly Phe Tyr Pro
               420                 425                 430
   Ser Asp Ile Ala Val Glu Trp Glu Ser Asn Gly Gln Pro Glu Asn Asn
           435                 440                 445
   Tyr Lys Thr Thr Pro Pro Val Leu Asp Ser Asp Gly Ser Phe Phe Leu
       450                 455                 460
   Tyr Ser Lys Leu Thr Val Asp Lys Ser Arg Trp Gln Gln Gly Asn Val
   465                 470                 475                 480
   Phe Ser Cys Ser Val Met His Glu Ala Leu His Asn His Tyr Thr Gln
                   485                 490                 495
   Lys Ser Leu Ser Leu Ser Pro Gly Lys
               500                 505
<210> 129
   <211> 1335
   <212> DNA
   <213> Artificial Sequence
<220>
   <223> Optimized tissue Plasminogen Activator (otPA) pre-pro signal sequence and exons 8-13 of human IL-17RC, and Fc5
<400> 129
   atggatgcaa tgaagagagg gctctgctgt gtgctgctgc tgtgtggcgc cgtcttcgtt 60
   tcgctcagcc aggaaatcca tgccgagttg agacgcttcc gtagagccct gccctggctc 120
   aacgtgtcag cagatggtga caacgtgcat ctggttctga atgtctctga ggagcagcac 180
   ttcggcctct ccctgtactg gaatcaggtc cagggccccc caaaaccccg gtggcacaaa 240
   aacctgactg gaccgcagat cattaccttg aaccacacag acctggttcc ctgcctctgt 300
   attcaggtgt ggcctctgga acctgactcc gttaggacga acatctgccc cttcagggag 360
   gacccccgcg cacaccagaa cctctggcaa gccgcccgac tgcgactgct gaccctgcag 420
   agctggctgc tggacgcacc gtgctcgctg cccgcagaag cggcactgtg ctggcgggct 480
   ccgggtgggg acccctgcca gccactggtc ccaccgcttt cctgggagaa cgtcactgtg 540
   gacaaggttc tcgagttccc attgctgaaa ggccacccta acctctgtgt tcaggtgaac 600
   agctcggaga agctgcagct gcaggagtgc ttgtgggctg agcccaaatc ttcagacaaa 660
   actcacacat gcccaccgtg cccagcacct gaagccgagg gggcaccgtc agtcttcctc 720
   ttccccccaa aacccaagga caccctcatg atctcccgga cccctgaggt cacatgcgtg 780
   gtggtggacg tgagccacga agaccctgag gtcaagttca actggtacgt ggacggcgtg 840
   gaggtgcata atgccaagac aaagccgcgg gaggagcagt acaacagcac gtaccgtgtg 900
   gtcagcgtcc tcaccgtcct gcaccaggac tggctgaatg gcaaggagta caagtgcaag 960
   gtctccaaca aagccctccc atcctccatc gagaaaacca tctccaaagc caaagggcag 1020
   ccccgagaac cacaggtgta caccctgccc ccatcccggg atgagctgac caagaaccag 1080
   gtcagcctga cctgcctggt caaaggcttc tatcccagcg acatcgccgt ggagtgggag 1140
   agcaatgggc agccggagaa caactacaag accacgcctc ccgtgctgga ctccgacggc 1200
   tccttcttcc tctacagcaa gctcaccgtg gacaagagca ggtggcagca ggggaacgtc 1260
   ttctcatgct ccgtgatgca tgaggctctg cacaaccact acacgcagaa gagcctctcc 1320
   ctgtctccgg gtaaa                                                  1335
<210> 130
   <211> 445
   <212> PRT
   <213> Artificial Sequence
<220>
   <223> Optimized tissue Plasminogen Activator (otPA) pre-pro signal sequence and exons 8-13 of human IL-17RC, and Fc5
<400> 130
   Met Asp Ala Met Lys Arg Gly Leu Cys Cys Val Leu Leu Leu Cys Gly
   1               5                   10                  15
   Ala Val Phe Val Ser Leu Ser Gln Glu Ile His Ala Glu Leu Arg Arg
               20                  25                  30
   Phe Arg Arg Ala Leu Pro Trp Leu Asn Val Ser Ala Asp Gly Asp Asn
           35                  40                  45
   Val His Leu Val Leu Asn Val Ser Glu Glu Gln His Phe Gly Leu Ser
       50                  55                  60
   Leu Tyr Trp Asn Gln Val Gln Gly Pro Pro Lys Pro Arg Trp His Lys
   65                  70                  75                  80
   Asn Leu Thr Gly Pro Gln Ile Ile Thr Leu Asn His Thr Asp Leu Val
                   85                  90                  95
   Pro Cys Leu Cys Ile Gln Val Trp Pro Leu Glu Pro Asp Ser Val Arg
               100                 105                 110
   Thr Asn Ile Cys Pro Phe Arg Glu Asp Pro Arg Ala His Gln Asn Leu
           115                 120                 125
   Trp Gln Ala Ala Arg Leu Arg Leu Leu Thr Leu Gln Ser Trp Leu Leu
       130                 135                 140
   Asp Ala Pro Cys Ser Leu Pro Ala Glu Ala Ala Leu Cys Trp Arg Ala
   145                 150                 155                 160
   Pro Gly Gly Asp Pro Cys Gln Pro Leu Val Pro Pro Leu Ser Trp Glu
                   165                 170                 175
   Asn Val Thr Val Asp Lys Val Leu Glu Phe Pro Leu Leu Lys Gly His
               180                 185                 190
   Pro Asn Leu Cys Val Gln Val Asn Ser Ser Glu Lys Leu Gln Leu Gln
           195                 200                 205
   Glu Cys Leu Trp Ala Glu Pro Lys Ser Ser Asp Lys Thr His Thr Cys
       210                 215                 220
   Pro Pro Cys Pro Ala Pro Glu Ala Glu Gly Ala Pro Ser Val Phe Leu
   225                 230                 235                 240
   Phe Pro Pro Lys Pro Lys Asp Thr Leu Met Ile Ser Arg Thr Pro Glu
                   245                 250                 255
   Val Thr Cys Val Val Val Asp Val Ser His Glu Asp Pro Glu Val Lys
               260                 265                 270
   Phe Asn Trp Tyr Val Asp Gly Val Glu Val His Asn Ala Lys Thr Lys
           275                 280                 285
   Pro Arg Glu Glu Gln Tyr Asn Ser Thr Tyr Arg Val Val Ser Val Leu
       290                 295                 300
   Thr Val Leu His Gln Asp Trp Leu Asn Gly Lys Glu Tyr Lys Cys Lys
   305                 310                 315                 320
   Val Ser Asn Lys Ala Leu Pro Ser Ser Ile Glu Lys Thr Ile Ser Lys
                   325                 330                 335
   Ala Lys Gly Gln Pro Arg Glu Pro Gln Val Tyr Thr Leu Pro Pro Ser
               340                 345                 350
   Arg Asp Glu Leu Thr Lys Asn Gln Val Ser Leu Thr Cys Leu Val Lys
           355                 360                 365
   Gly Phe Tyr Pro Ser Asp Ile Ala Val Glu Trp Glu Ser Asn Gly Gln
       370                 375                 380
   Pro Glu Asn Asn Tyr Lys Thr Thr Pro Pro Val Leu Asp Ser Asp Gly
   385                 390                 395                 400
   Ser Phe Phe Leu Tyr Ser Lys Leu Thr Val Asp Lys Ser Arg Trp Gln
                   405                 410                 415
   Gln Gly Asn Val Phe Ser Cys Ser Val Met His Glu Ala Leu His Asn
               420                 425                 430
   His Tyr Thr Gln Lys Ser Leu Ser Leu Ser Pro Gly Lys
           435                 440                 445
<210> 131
   <211> 1299
   <212> DNA
   <213> Artificial Sequence
<220>
   <223> Optimized tissue Plasminogen Activator (otPA) pre-pro signal sequence and exons 8-10 and 14-16 of human IL-17RC, and Fc5
<400> 131
   atggatgcaa tgaagagagg gctctgctgt gtgctgctgc tgtgtggcgc cgtcttcgtt 60
   tcgctcagcc aggaaatcca tgccgagttg agacgcttcc gtagagccct gccctggctc 120
   aacgtgtcag cagatggtga caacgtgcat ctggttctga atgtctctga ggagcagcac 180
   ttcggcctct ccctgtactg gaatcaggtc cagggccccc caaaaccccg gtggcacaaa 240
   aacctgactg gaccgcagat cattaccttg aaccacacag acctggttcc ctgcctctgt 300
   attcaggtgt ggcctctgga acctgactcc gttaggacga acatctgccc cttcagggag 360
   gactccctgg ggcctctcaa agacgatgtg ctactgttgg agacacgagg cccccaggac 420
   aacagatccc tctgtgcctt ggaacccagt ggctgtactt cactacccag caaagcctcc 480
   acgagggcag ctcgccttgg agagtactta ctacaagacc tgcagtcagg ccagtgtctg 540
   cagctatggg acgatgactt gggagcgcta tgggcctgcc ccatggacaa atacatccac 600
   aaggagccca aatcttcaga caaaactcac acatgcccac cgtgcccagc acctgaagcc 660
   gagggggcac cgtcagtctt cctcttcccc ccaaaaccca aggacaccct catgatctcc 720
   cggacccctg aggtcacatg cgtggtggtg gacgtgagcc acgaagaccc tgaggtcaag 780
   ttcaactggt acgtggacgg cgtggaggtg cataatgcca agacaaagcc gcgggaggag 840
   cagtacaaca gcacgtaccg tgtggtcagc gtcctcaccg tcctgcacca ggactggctg 900
   aatggcaagg agtacaagtg caaggtctcc aacaaagccc tcccatcctc catcgagaaa 960
   accatctcca aagccaaagg gcagccccga gaaccacagg tgtacaccct gcccccatcc 1020
   cgggatgagc tgaccaagaa ccaggtcagc ctgacctgcc tggtcaaagg cttctatccc 1080
   agcgacatcg ccgtggagtg ggagagcaat gggcagccgg agaacaacta caagaccacg 1140
   cctcccgtgc tggactccga cggctccttc ttcctctaca gcaagctcac cgtggacaag 1200
   agcaggtggc agcaggggaa cgtcttctca tgctccgtga tgcatgaggc tctgcacaac 1260
   cactacacgc agaagagcct ctccctgtct ccgggtaaa 1299
<210> 132
   <211> 433
   <212> PRT
   <213> Artificial Sequence
<220>
   <223> Optimized tissue Plasminogen Activator (otPA) pre-pro signal sequence and exons 8-10 and 14-16 of human IL-17RC, and Fc5
<400> 132
   Met Asp Ala Met Lys Arg Gly Leu Cys Cys Val Leu Leu Leu Cys Gly
   1 5 10 15
   Ala Val Phe Val Ser Leu Ser Gln Glu Ile His Ala Glu Leu Arg Arg
   20 25 30
   Phe Arg Arg Ala Leu Pro Trp Leu Asn Val Ser Ala Asp Gly Asp Asn
   35 40 45
   Val His Leu Val Leu Asn Val Ser Glu Glu Gln His Phe Gly Leu Ser
   50 55 60
   Leu Tyr Trp Asn Gln Val Gln Gly Pro Pro Lys Pro Arg Trp His Lys
   65 70 75 80
   Asn Leu Thr Gly Pro Gln Ile Ile Thr Leu Asn His Thr Asp Leu Val
   85 90 95
   Pro Cys Leu Cys Ile Gln Val Trp Pro Leu Glu Pro Asp Ser Val Arg
   100 105 110
   Thr Asn Ile Cys Pro Phe Arg Glu Asp Ser Leu Gly Pro Leu Lys Asp
   115 120 125
   Asp Val Leu Leu Leu Glu Thr Arg Gly Pro Gln Asp Asn Arg Ser Leu
   130 135 140
   Cys Ala Leu Glu Pro Ser Gly Cys Thr Ser Leu Pro Ser Lys Ala Ser
   145 150 155 160
   Thr Arg Ala Ala Arg Leu Gly Glu Tyr Leu Leu Gln Asp Leu Gln Ser
   165 170 175
   Gly Gln Cys Leu Gln Leu Trp Asp Asp Asp Leu Gly Ala Leu Trp Ala
   180 185 190
   Cys Pro Met Asp Lys Tyr Ile His Lys Glu Pro Lys Ser Ser Asp Lys
   195 200 205
   Thr His Thr Cys Pro Pro Cys Pro Ala Pro Glu Ala Glu Gly Ala Pro
   210 215 220
   Ser Val Phe Leu Phe Pro Pro Lys Pro Lys Asp Thr Leu Met Ile Ser
   225 230 235 240
   Arg Thr Pro Glu Val Thr Cys Val Val Val Asp Val Ser His Glu Asp
   245 250 255
   Pro Glu Val Lys Phe Asn Trp Tyr Val Asp Gly Val Glu Val His Asn
   260 265 270
   Ala Lys Thr Lys Pro Arg Glu Glu Gln Tyr Asn Ser Thr Tyr Arg Val
   275 280 285
   Val Ser Val Leu Thr Val Leu His Gln Asp Trp Leu Asn Gly Lys Glu
   290 295 300
   Tyr Lys Cys Lys Val Ser Asn Lys Ala Leu Pro Ser Ser Ile Glu Lys
   305 310 315 320
   Thr Ile Ser Lys Ala Lys Gly Gln Pro Arg Glu Pro Gln Val Tyr Thr
   325 330 335
   Leu Pro Pro Ser Arg Asp Glu Leu Thr Lys Asn Gln Val Ser Leu Thr
   340 345 350
   Cys Leu Val Lys Gly Phe Tyr Pro Ser Asp Ile Ala Val Glu Trp Glu
   355 360 365
   Ser Asn Gly Gln Pro Glu Asn Asn Tyr Lys Thr Thr Pro Pro Val Leu
   370 375 380
   Asp Ser Asp Gly Ser Phe Phe Leu Tyr Ser Lys Leu Thr Val Asp Lys
   385 390 395 400
   Ser Arg Trp Gln Gln Gly Asn Val Phe Ser Cys Ser Val Met His Glu
   405 410 415
   Ala Leu His Asn His Tyr Thr Gln Lys Ser Leu Ser Leu Ser Pro Gly
   420 425 430
   Lys
<210> 133
   <211> 1056
   <212> DNA
   <213> Artificial Sequence
<220>
   <223> Optimized tissue Plasminogen Activator (otPA) pre-pro signal sequence and exons 8-10 of human IL-17RC, and Fc5
<400> 133
   atggatgcaa tgaagagagg gctctgctgt gtgctgctgc tgtgtggcgc cgtcttcgtt 60
   tcgctcagcc aggaaatcca tgccgagttg agacgcttcc gtagagccct gccctggctc 120
   aacgtgtcag cagatggtga caacgtgcat ctggttctga atgtctctga ggagcagcac 180
   ttcggcctct ccctgtactg gaatcaggtc cagggccccc caaaaccccg gtggcacaaa 240
   aacctgactg gaccgcagat cattaccttg aaccacacag acctggttcc ctgcctctgt 300
   attcaggtgt ggcctctgga acctgactcc gttaggacga acatctgccc cttcagggag 360
   gagcccaaat cttcagacaa aactcacaca tgcccaccgt gcccagcacc tgaagccgag 420
   ggggcaccgt cagtcttcct cttcccccca aaacccaagg acaccctcat gatctcccgg 480
   acccctgagg tcacatgcgt ggtggtggac gtgagccacg aagaccctga ggtcaagttc 540
   aactggtacg tggacggcgt ggaggtgcat aatgccaaga caaagccgcg ggaggagcag 600
   tacaacagca cgtaccgtgt ggtcagcgtc ctcaccgtcc tgcaccagga ctggctgaat 660
   ggcaaggagt acaagtgcaa ggtctccaac aaagccctcc catcctccat cgagaaaacc 720
   atctccaaag ccaaagggca gccccgagaa ccacaggtgt acaccctgcc cccatcccgg 780
   gatgagctga ccaagaacca ggtcagcctg acctgcctgg tcaaaggctt ctatcccagc 840
   gacatcgccg tggagtggga gagcaatggg cagccggaga acaactacaa gaccacgcct 900
   cccgtgctgg actccgacgg ctccttcttc ctctacagca agctcaccgt ggacaagagc 960
   aggtggcagc aggggaacgt cttctcatgc tccgtgatgc atgaggctct gcacaaccac 1020
   tacacgcaga agagcctctc cctgtctccg ggtaaa                           1056
<210> 134
   <211> 352
   <212> PRT
   <213> Artificial Sequence
<220>
   <223> Optimized tissue Plasminogen Activator (otPA) pre-pro signal sequence and exons 8-10 of human IL-17RC, and Fc5
<400> 134
   Met Asp Ala Met Lys Arg Gly Leu Cys Cys Val Leu Leu Leu Cys Gly
   1               5                   10                  15
   Ala Val Phe Val Ser Leu Ser Gln Glu Ile His Ala Glu Leu Arg Arg
   20 25 30
   Phe Arg Arg Ala Leu Pro Trp Leu Asn Val Ser Ala Asp Gly Asp Asn
   35 40 45
   Val His Leu Val Leu Asn Val Ser Glu Glu Gln His Phe Gly Leu Ser
   50 55 60
   Leu Tyr Trp Asn Gln Val Gln Gly Pro Pro Lys Pro Arg Trp His Lys
   65 70 75 80
   Asn Leu Thr Gly Pro Gln Ile Ile Thr Leu Asn His Thr Asp Leu Val
   85 90 95
   Pro Cys Leu Cys Ile Gln Val Trp Pro Leu Glu Pro Asp Ser Val Arg
   100 105 110
   Thr Asn Ile Cys Pro Phe Arg Glu Glu Pro Lys Ser Ser Asp Lys Thr
   115 120 125
   His Thr Cys Pro Pro Cys Pro Ala Pro Glu Ala Glu Gly Ala Pro Ser
   130 135 140
   Val Phe Leu Phe Pro Pro Lys Pro Lys Asp Thr Leu Met Ile Ser Arg
   145 150 155 160
   Thr Pro Glu Val Thr Cys Val Val Val Asp Val Ser His Glu Asp Pro
   165 170 175
   Glu Val Lys Phe Asn Trp Tyr Val Asp Gly Val Glu Val His Asn Ala
   180 185 190
   Lys Thr Lys Pro Arg Glu Glu Gln Tyr Asn Ser Thr Tyr Arg Val Val
   195 200 205
   Ser Val Leu Thr Val Leu His Gln Asp Trp Leu Asn Gly Lys Glu Tyr
   210 215 220
   Lys Cys Lys Val Ser Asn Lys Ala Leu Pro Ser Ser Ile Glu Lys Thr
   225 230 235 240
   Ile Ser Lys Ala Lys Gly Gln Pro Arg Glu Pro Gln Val Tyr Thr Leu
   245 250 255
   Pro Pro Ser Arg Asp Glu Leu Thr Lys Asn Gln Val Ser Leu Thr Cys
   260 265 270
   Leu Val Lys Gly Phe Tyr Pro Ser Asp Ile Ala Val Glu Trp Glu Ser
   275 280 285
   Asn Gly Gln Pro Glu Asn Asn Tyr Lys Thr Thr Pro Pro Val Leu Asp
   290 295 300
   Ser Asp Gly Ser Phe Phe Leu Tyr Ser Lys Leu Thr Val Asp Lys Ser
   305 310 315 320
   Arg Trp Gln Gln Gly Asn Val Phe Ser Cys Ser Val Met His Glu Ala
   325 330 335
   Leu His Asn His Tyr Thr Gln Lys Ser Leu Ser Leu Ser Pro Gly Lys
   340 345 350
<210> 135
   <211> 1080
   <212> DNA
   <213> Artificial Sequence
<220>
   <223> Optimized tissue Plasminogen Activator (otPA) pre-pro signal sequence and exons 11-13 of human IL-17RC, and Fc5
<400> 135
   atggatgcaa tgaagagagg gctctgctgt gtgctgctgc tgtgtggcgc cgtcttcgtt 60
   tcgctcagcc aggaaatcca tgccgagttg agacgcttcc gtagagaccc ccgcgcacac 120
   cagaacctct ggcaagccgc ccgactgcga ctgctgaccc tgcagagctg gctgctggac 180
   gcaccgtgct cgctgcccgc agaagcggca ctgtgctggc gggctccggg tggggacccc 240
   tgccagccac tggtcccacc gctttcctgg gagaacgtca ctgtggacaa ggttctcgag 300
   ttcccattgc tgaaaggcca ccctaacctc tgtgttcagg tgaacagctc ggagaagctg 360
   cagctgcagg agtgcttgtg ggctgagccc aaatcttcag acaaaactca cacatgccca 420
   ccgtgcccag cacctgaagc cgagggggca ccgtcagtct tcctcttccc cccaaaaccc 480
   aaggacaccc tcatgatctc ccggacccct gaggtcacat gcgtggtggt ggacgtgagc 540
   cacgaagacc ctgaggtcaa gttcaactgg tacgtggacg gcgtggaggt gcataatgcc 600
   aagacaaagc cgcgggagga gcagtacaac agcacgtacc gtgtggtcag cgtcctcacc 660
   gtcctgcacc aggactggct gaatggcaag gagtacaagt gcaaggtctc caacaaagcc 720
   ctcccatcct ccatcgagaa aaccatctcc aaagccaaag ggcagccccg agaaccacag 780
   gtgtacaccc tgcccccatc ccgggatgag ctgaccaaga accaggtcag cctgacctgc 840
   ctggtcaaag gcttctatcc cagcgacatc gccgtggagt gggagagcaa tgggcagccg 900
   gagaacaact acaagaccac gcctcccgtg ctggactccg acggctcctt cttcctctac 960
   agcaagctca ccgtggacaa gagcaggtgg cagcagggga acgtcttctc atgctccgtg 1020
   atgcatgagg ctctgcacaa ccactacacg cagaagagcc tctccctgtc tccgggtaaa 1080
<210> 136
   <211> 360
   <212> PRT
   <213> Artificial Sequence
<220>
   <223> Optimized tissue Plasminogen Activator (otPA) pre-pro signal sequence and exons 11-13 of human IL-17RC, and Fc5
<400> 136
   Met Asp Ala Met Lys Arg Gly Leu Cys Cys Val Leu Leu Leu Cys Gly
   1               5                   10                  15
   Ala Val Phe Val Ser Leu Ser Gln Glu Ile His Ala Glu Leu Arg Arg
               20                  25                  30
   Phe Arg Arg Asp Pro Arg Ala His Gln Asn Leu Trp Gln Ala Ala Arg
           35                  40                  45
   Leu Arg Leu Leu Thr Leu Gln Ser Trp Leu Leu Asp Ala Pro Cys Ser
       50                  55                  60
   Leu Pro Ala Glu Ala Ala Leu Cys Trp Arg Ala Pro Gly Gly Asp Pro
   65                  70                  75                  80
   Cys Gln Pro Leu Val Pro Pro Leu Ser Trp Glu Asn Val Thr Val Asp
                   85                  90                  95
   Lys Val Leu Glu Phe Pro Leu Leu Lys Gly His Pro Asn Leu Cys Val
               100                 105                 110
   Gln Val Asn Ser Ser Glu Lys Leu Gln Leu Gln Glu Cys Leu Trp Ala
           115                 120                 125
   Glu Pro Lys Ser Ser Asp Lys Thr His Thr Cys Pro Pro Cys Pro Ala
       130                 135                 140
   Pro Glu Ala Glu Gly Ala Pro Ser Val Phe Leu Phe Pro Pro Lys Pro
   145                 150                 155                 160
   Lys Asp Thr Leu Met Ile Ser Arg Thr Pro Glu Val Thr Cys Val Val
                   165                 170                 175
   Val Asp Val Ser His Glu Asp Pro Glu Val Lys Phe Asn Trp Tyr Val
               180                 185                 190
   Asp Gly Val Glu Val His Asn Ala Lys Thr Lys Pro Arg Glu Glu Gln
           195                 200                 205
   Tyr Asn Ser Thr Tyr Arg Val Val Ser Val Leu Thr Val Leu His Gln
       210                 215                 220
   Asp Trp Leu Asn Gly Lys Glu Tyr Lys Cys Lys Val Ser Asn Lys Ala
   225                 230                 235                 240
   Leu Pro Ser Ser Ile Glu Lys Thr Ile Ser Lys Ala Lys Gly Gln Pro
                   245                 250                 255
   Arg Glu Pro Gln Val Tyr Thr Leu Pro Pro Ser Arg Asp Glu Leu Thr
               260                 265                 270
   Lys Asn Gln Val Ser Leu Thr Cys Leu Val Lys Gly Phe Tyr Pro Ser
           275                 280                 285
   Asp Ile Ala Val Glu Trp Glu Ser Asn Gly Gln Pro Glu Asn Asn Tyr
       290                 295                 300
   Lys Thr Thr Pro Pro Val Leu Asp Ser Asp Gly Ser Phe Phe Leu Tyr
   305                 310                 315                 320
   Ser Lys Leu Thr Val Asp Lys Ser Arg Trp Gln Gln Gly Asn Val Phe
                   325                 330                 335
   Ser Cys Ser Val Met His Glu Ala Leu His Asn His Tyr Thr Gln Lys
               340                 345                 350
   Ser Leu Ser Leu Ser Pro Gly Lys
           355                 360
<210> 137
   <211> 1164
   <212> DNA
   <213> Artificial Sequence
<220>
   <223> Optimized tissue Plasminogen Activator (otPA) pre-pro signal sequence and exons 7-10 of human IL-17RC, and Fc5
<400> 137
   atggatgcaa tgaagagagg gctctgctgt gtgctgctgc tgtgtggcgc cgtcttcgtt 60
   tcgctcagcc aggaaatcca tgccgagttg agacgcttcc gtagaggcag cctgtgggac 120
   cccaacatca ccgtggagac cctggaggcc caccagctgc gtgtgagctt caccctgtgg 180
   aacgaatcta cccattacca gatcctgctg accagttttc cgcacatgga gaaccacagt 240
   tgctttgagc acatgcacca catacctgcg cccagaccag aagagttcca ccagcgatcc 300
   aacgtcacac tcactctacg caaccttaaa gggtgctgtc gccaccaagt gcagatccag 360
   cccttcttca gcagctgcct caatgactgc ctcagacact ccgcgactgt ttcctgccca 420
   gaaatgccag acactccaga accaattccg gactacatgc cctgtgggga gcccaaatct 480
   tcagacaaaa ctcacacatg cccaccgtgc ccagcacctg aagccgaggg ggcaccgtca 540
   gtcttcctct tccccccaaa acccaaggac accctcatga tctcccggac ccctgaggtc 600
   acatgcgtgg tggtggacgt gagccacgaa gaccctgagg tcaagttcaa ctggtacgtg 660
   gacggcgtgg aggtgcataa tgccaagaca aagccgcggg aggagcagta caacagcacg 720
   taccgtgtgg tcagcgtcct caccgtcctg caccaggact ggctgaatgg caaggagtac 780
   aagtgcaagg tctccaacaa agccctccca tcctccatcg agaaaaccat ctccaaagcc 840
   aaagggcagc cccgagaacc acaggtgtac accctgcccc catcccggga tgagctgacc 900
   aagaaccagg tcagcctgac ctgcctggtc aaaggcttct atcccagcga catcgccgtg 960
   gagtgggaga gcaatgggca gccggagaac aactacaaga ccacgcctcc cgtgctggac 1020
   tccgacggct ccttcttcct ctacagcaag ctcaccgtgg acaagagcag gtggcagcag 1080
   gggaacgtct tctcatgctc cgtgatgcat gaggctctgc acaaccacta cacgcagaag 1140
   agcctctccc tgtctccggg taaa                                       1164
<210> 138
   <211> 388
   <212> PRT
   <213> Artificial Sequence
<220>
   <223> Optimized tissue Plasminogen Activator (otPA) pre-pro signal sequence and exons 7-10 of human IL-17RC, and Fc5
<400> 138
   Met Asp Ala Met Lys Arg Gly Leu Cys Cys Val Leu Leu Leu Cys Gly
   1 5 10 15
   Ala Val Phe Val Ser Leu Ser Gln Glu Ile His Ala Glu Leu Arg Arg
   20 25 30
   Phe Arg Arg Gly Ser Leu Trp Asp Pro Asn Ile Thr Val Glu Thr Leu
   35 40 45
   Glu Ala His Gln Leu Arg Val Ser Phe Thr Leu Trp Asn Glu Ser Thr
   50 55 60
   His Tyr Gln Ile Leu Leu Thr Ser Phe Pro His Met Glu Asn His Ser
   65 70 75 80
   Cys Phe Glu His Met His His Ile Pro Ala Pro Arg Pro Glu Glu Phe
   85 90 95
   His Gln Arg Ser Asn Val Thr Leu Thr Leu Arg Asn Leu Lys Gly Cys
   100 105 110
   Cys Arg His Gln Val Gln Ile Gln Pro Phe Phe Ser Ser Cys Leu Asn
   115 120 125
   Asp Cys Leu Arg His Ser Ala Thr Val Ser Cys Pro Glu Met Pro Asp
   130 135 140
   Thr Pro Glu Pro Ile Pro Asp Tyr Met Pro Leu Trp Glu Pro Lys Ser
   145 150 155 160
   Ser Asp Lys Thr His Thr Cys Pro Pro Cys Pro Ala Pro Glu Ala Glu
   165 170 175
   Gly Ala Pro Ser Val Phe Leu Phe Pro Pro Lys Pro Lys Asp Thr Leu
   180 185 190
   Met Ile Ser Arg Thr Pro Glu Val Thr Cys Val Val Val Asp Val Ser
   195 200 205
   His Glu Asp Pro Glu Val Lys Phe Asn Trp Tyr Val Asp Gly Val Glu
   210 215 220
   Val His Asn Ala Lys Thr Lys Pro Arg Glu Glu Gln Tyr Asn Ser Thr
   225 230 235 240
   Tyr Arg Val Val Ser Val Leu Thr Val Leu His Gln Asp Trp Leu Asn
   245 250 255
   Gly Lys Glu Tyr Lys Cys Lys Val Ser Asn Lys Ala Leu Pro Ser Ser
   260 265 270
   Ile Glu Lys Thr Ile Ser Lys Ala Lys Gly Gln Pro Arg Glu Pro Gln
   275 280 285
   Val Tyr Thr Leu Pro Pro Ser Arg Asp Glu Leu Thr Lys Asn Gln Val
   290 295 300
   Ser Leu Thr Cys Leu Val Lys Gly Phe Tyr Pro Ser Asp Ile Ala Val
   305 310 315 320
   Glu Trp Glu Ser Asn Gly Gln Pro Glu Asn Asn Tyr Lys Thr Thr Pro
   325 330 335
   Pro Val Leu Asp Ser Asp Gly Ser Phe Phe Leu Tyr Ser Lys Leu Thr
   340 345 350
   Val Asp Lys Ser Arg Trp Gln Gln Gly Asn Val Phe Ser Cys Ser Val
   355 360 365
   Met His Glu Ala Leu His Asn His Tyr Thr Gln Lys Ser Leu Ser Leu
   370 375 380
   Ser Pro Gly Lys
   385
<210> 139
   <211> 2433
   <212> DNA
   <213> Artificial Sequence
<220>
   <223> IL-17RA signal sequence and exons 1-10 of IL-17RA and exons 8-16 of human IL-17RC, and Fc5

<400> 139
   atgggggccg cacgcagccc gccgtccgct gtcccggggc ccctgctggg gctgctcctg 60
   ctgctcctgg gcgtgctggc cccgggtggc gcctccctgc gactcctgga ccaccgggcg 120
   ctggtctgct cccagccggg gctaaactgc acggtcaaga atagtacctg cctggatgac 180
   agctggattc accctcgaaa cctgaccccc tcctccccaa aggacctgca gatccagctg 240
   cactttgccc acacccaaca aggagacctg ttccccgtgg ctcacatcga atggacactg 300
   cagacagacg ccagcatcct gtacctcgag ggtgcagagt tatctgtcct gcagctgaac 360
   accaatgaac gtttgtgcgt caggtttgag tttctgtcca aactgaggca tcaccacagg 420
   cggtggcgtt ttaccttcag ccactttgtg gttgaccctg accaggaata tgaggtgacc 480
   gttcaccacc tgcccaagcc catccctgat ggggacccaa accaccagtc caagaatttc 540
   cttgtgcctg actgtgagca cgccaggatg aaggtaacca cgccatgcat gagctcaggc 600
   agcctgtggg accccaacat caccgtggag accctggagg cccaccagct gcgtgtgagc 660
   ttcaccctgt ggaacgaatc tacccattac cagatcctgc tgaccagttt tccgcacatg 720
   gagaaccaca gttgctttga gcacatgcac cacatacctg cgcccagacc agaagagttc 780
   caccagcgat ccaacgtcac actcactcta cgcaacctta aagggtgctg tcgccaccaa 840
   gtgcagatcc agcccttctt cagcagctgc ctcaatgact gcctcagaca ctccgcgact 900
   gtttcctgcc cagaaatgcc agacactcca gaaccaattc cggactacat gcccctgtgg 960
   gccctgccct ggctcaacgt gtcagcagat ggtgacaacg tgcatctggt tctgaatgtc 1020
   tctgaggagc agcacttcgg cctctccctg tactggaatc aggtccaggg ccccccaaaa 1080
   ccccggtggc acaaaaacct gactggaccg cagatcatta ccttgaacca cacagacctg 1140
   gttccctgcc tctgtattca ggtgtggcct ctggaacctg actccgttag gacgaacatc 1200
   tgccccttca gggaggaccc ccgcgcacac cagaacctct ggcaagccgc ccgactgcga 1260
   ctgctgaccc tgcagagctg gctgctggac gcaccgtgct cgctgcccgc agaagcggca 1320
   ctgtgctggc gggctccggg tggggacccc tgccagccac tggtcccacc gctttcctgg 1380
   gagaacgtca ctgtggacaa ggttctcgag ttcccattgc tgaaaggcca ccctaacctc 1440
   tgtgttcagg tgaacagctc ggagaagctg cagctgcagg agtgcttgtg ggctgactcc 1500
   ctggggcctc tcaaagacga tgtgctactg ttggagacac gaggccccca ggacaacaga 1560
   tccctctgtg ccttggaacc cagtggctgt acttcactac ccagcaaagc ctccacgagg 1620
   gcagctcgcc ttggagagta cttactacaa gacctgcagt caggccagtg tctgcagcta 1680
   tgggacgatg acttgggagc gctatgggcc tgccccatgg acaaatacat ccacaaggag 1740
   cccaaatctt cagacaaaac tcacacatgc ccaccgtgcc cagcacctga agccgagggg 1800
   gcaccgtcag tcttcctctt ccccccaaaa cccaaggaca ccctcatgat ctcccggacc 1860
   cctgaggtca catgcgtggt ggtggacgtg agccacgaag accctgaggt caagttcaac 1920
   tggtacgtgg acggcgtgga ggtgcataat gccaagacaa agccgcggga ggagcagtac 1980
   aacagcacgt accgtgtggt cagcgtcctc accgtcctgc accaggactg gctgaatggc 2040
   aaggagtaca agtgcaaggt ctccaacaaa gccctcccat cctccatcga gaaaaccatc 2100
   tccaaagcca aagggcagcc ccgagaacca caggtgtaca ccctgccccc atcccgggat 2160
   gagctgacca agaaccaggt cagcctgacc tgcctggtca aaggcttcta tcccagcgac 2220
   atcgccgtgg agtgggagag caatgggcag ccggagaaca actacaagac cacgcctccc 2280
   gtgctggact ccgacggctc cttcttcctc tacagcaagc tcaccgtgga caagagcagg 2340
   tggcagcagg ggaacgtctt ctcatgctcc gtgatgcatg aggctctgca caaccactac 2400
   acgcagaaga gcctctccct gtctccgggt aaa 2433
<210> 140
   <211> 811
   <212> PRT
   <213> Artificial Sequence
<220>
   <223> IL-17RA signal sequence and exons 1-10 of IL-17RA and exons 8-16 of human IL-17RC, and Fc5
<400> 140
<210> 141
   <211> 2190
   <212> DNA
   <213> Artificial Sequence
<220>
   <223> IL-17RA signal sequence and exons 1-6 of IL-17RA and exons 8-13 of human IL-17RC and exons 7-10 of IL-17RA, and Fc5
<400> 141
   atgggggccg cacgcagccc gccgtccgct gtcccggggc ccctgctggg gctgctcctg 60
   ctgctcctgg gcgtgctggc cccgggtggc gcctccctgc gactcctgga ccaccgggcg 120
   ctggtctgct cccagccggg gctaaactgc acggtcaaga atagtacctg cctggatgac 180
   agctggattc accctcgaaa cctgaccccc tcctccccaa aggacctgca gatccagctg 240
   cactttgccc acacccaaca aggagacctg ttccccgtgg ctcacatcga atggacactg 300
   cagacagacg ccagcatcct gtacctcgag ggtgcagagt tatctgtcct gcagctgaac 360
   accaatgaac gtttgtgcgt caggtttgag tttctgtcca aactgaggca tcaccacagg 420
   cggtggcgtt ttaccttcag ccactttgtg gttgaccctg accaggaata tgaggtgacc 480
   gttcaccacc tgcccaagcc catccctgat ggggacccaa accaccagtc caagaatttc 540
   cttgtgcctg actgtgagca cgccaggatg aaggtaacca cgccatgcat gagctcagcc 600
   ctgccctggc tcaacgtgtc agcagatggt gacaacgtgc atctggttct gaatgtctct 660
   gaggagcagc acttcggcct ctccctgtac tggaatcagg tccagggccc cccaaaaccc 720
   cggtggcaca aaaacctgac tggaccgcag atcattacct tgaaccacac agacctggtt 780
   ccctgcctct gtattcaggt gtggcctctg gaacctgact ccgttaggac gaacatctgc 840
   cccttcaggg aggacccccg cgcacaccag aacctctggc aagccgcccg actgcgactg 900
   ctgaccctgc agagctggct gctggacgca ccgtgctcgc tgcccgcaga agcggcactg 960
   tgctggcggg ctccgggtgg ggacccctgc cagccactgg tcccaccgct ttcctgggag 1020
   aacgtcactg tggacaaggt tctcgagttc ccattgctga aaggccaccc taacctctgt 1080
   gttcaggtga acagctcgga gaagctgcag ctgcaggagt gcttgtgggc tggcagcctg 1140
   tgggacccca acatcaccgt ggagaccctg gaggcccacc agctgcgtgt gagcttcacc 1200
   ctgtggaacg aatctaccca ttaccagatc ctgctgacca gttttccgca catggagaac 1260
   cacagttgct ttgagcacat gcaccacata cctgcgccca gaccagaaga gttccaccag 1320
   cgatccaacg tcacactcac tctacgcaac cttaaagggt gctgtcgcca ccaagtgcag 1380
   atccagccct tcttcagcag ctgcctcaat gactgcctca gacactccgc gactgtttcc 1440
   tgcccagaaa tgccagacac tccagaacca attccggact acatgcccct gtgggagccc 1500
   aaatcttcag acaaaactca cacatgccca ccgtgcccag cacctgaagc cgagggggca 1560
   ccgtcagtct tcctcttccc cccaaaaccc aaggacaccc tcatgatctc ccggacccct 1620
   gaggtcacat gcgtggtggt ggacgtgagc cacgaagacc ctgaggtcaa gttcaactgg 1680
   tacgtggacg gcgtggaggt gcataatgcc aagacaaagc cgcgggagga gcagtacaac 1740
   agcacgtacc gtgtggtcag cgtcctcacc gtcctgcacc aggactggct gaatggcaag 1800
   gagtacaagt gcaaggtctc caacaaagcc ctcccatcct ccatcgagaa aaccatctcc 1860
   aaagccaaag ggcagccccg agaaccacag gtgtacaccc tgcccccatc ccgggatgag 1920
   ctgaccaaga accaggtcag cctgacctgc ctggtcaaag gcttctatcc cagcgacatc 1980
   gccgtggagt gggagagcaa tgggcagccg gagaacaact acaagaccac gcctcccgtg 2040
   ctggactccg acggctcctt cttcctctac agcaagctca ccgtggacaa gagcaggtgg 2100
   cagcagggga acgtcttctc atgctccgtg atgcatgagg ctctgcacaa ccactacacg 2160
   cagaagagcc tctccctgtc tccgggtaaa                                 2190
<210> 142
   <211> 730
   <212> PRT
   <213> Artificial Sequence
<220>
   <223> IL-17RA signal sequence and exons 1-6 of IL-17RA and exons 8-13 of human IL-17RC and exons 7-10 of IL-17RA, and Fc5
<400> 142
   Met Gly Ala Ala Arg Ser Pro Pro Ser Ala Val Pro Gly Pro Leu Leu
   1               5                   10                  15
   Gly Leu Leu Leu Leu Leu Leu Gly Val Leu Ala Pro Gly Gly Ala Ser
               20                  25                  30
   Leu Arg Leu Leu Asp His Arg Ala Leu Val Cys Ser Gln Pro Gly Leu
           35                  40                  45
   Asn Cys Thr Val Lys Asn Ser Thr Cys Leu Asp Asp Ser Trp Ile His
       50                  55                  60
   Pro Arg Asn Leu Thr Pro Ser Ser Pro Lys Asp Leu Gln Ile Gln Leu
   65                  70                  75                  80
   His Phe Ala His Thr Gln Gln Gly Asp Leu Phe Pro Val Ala His Ile
                   85                  90                  95
   Glu Trp Thr Leu Gln Thr Asp Ala Ser Ile Leu Tyr Leu Glu Gly Ala
               100                 105                 110
   Glu Leu Ser Val Leu Gln Leu Asn Thr Asn Glu Arg Leu Cys Val Arg
           115                 120                 125
   Phe Glu Phe Leu Ser Lys Leu Arg His His His Arg Arg Trp Arg Phe
       130                 135                 140
   Thr Phe Ser His Phe Val Val Asp Pro Asp Gln Glu Tyr Glu Val Thr
   145                 150                 155                 160
   Val His His Leu Pro Lys Pro Ile Pro Asp Gly Asp Pro Asn His Gln
                   165                 170                 175
   Ser Lys Asn Phe Leu Val Pro Asp Cys Glu His Ala Arg Met Lys Val
               180                 185                 190
   Thr Thr Pro Cys Met Ser Ser Ala Leu Pro Trp Leu Asn Val Ser Ala
           195                 200                 205
   Asp Gly Asp Asn Val His Leu Val Leu Asn Val Ser Glu Glu Gln His
       210                 215                 220
   Phe Gly Leu Ser Leu Tyr Trp Asn Gln Val Gln Gly Pro Pro Lys Pro
   225                 230                 235                 240
   Arg Trp His Lys Asn Leu Thr Gly Pro Gln Ile Ile Thr Leu Asn His
                   245                 250                 255
   Thr Asp Leu Val Pro Cys Leu Cys Ile Gln Val Trp Pro Leu Glu Pro
               260                 265                 270
   Asp Ser Val Arg Thr Asn Ile Cys Pro Phe Arg Glu Asp Pro Arg Ala
           275                 280                 285
   His Gln Asn Leu Trp Gln Ala Ala Arg Leu Arg Leu Leu Thr Leu Gln
       290                 295                 300
   Ser Trp Leu Leu Asp Ala Pro Cys Ser Leu Pro Ala Glu Ala Ala Leu
   305                 310                 315                 320
   Cys Trp Arg Ala Pro Gly Gly Asp Pro Cys Gln Pro Leu Val Pro Pro
                   325                 330                 335
   Leu Ser Trp Glu Asn Val Thr Val Asp Lys Val Leu Glu Phe Pro Leu
               340                 345                 350
   Leu Lys Gly His Pro Asn Leu Cys Val Gln Val Asn Ser Ser Glu Lys
           355                 360                 365
   Leu Gln Leu Gln Glu Cys Leu Trp Ala Gly Ser Leu Trp Asp Pro Asn
       370                 375                 380
   Ile Thr Val Glu Thr Leu Glu Ala His Gln Leu Arg Val Ser Phe Thr
   385                 390                 395                 400
   Leu Trp Asn Glu Ser Thr His Tyr Gln Ile Leu Leu Thr Ser Phe Pro
                   405                 410                 415
   His Met Glu Asn His Ser Cys Phe Glu His Met His His Ile Pro Ala
               420                 425                 430
   Pro Arg Pro Glu Glu Phe His Gln Arg Ser Asn Val Thr Leu Thr Leu
           435                 440                 445
   Arg Asn Leu Lys Gly Cys Cys Arg His Gln Val Gln Ile Gln Pro Phe
       450                 455                 460
   Phe Ser Ser Cys Leu Asn Asp Cys Leu Arg His Ser Ala Thr Val Ser
   465                 470                 475                 480
   Cys Pro Glu Met Pro Asp Thr Pro Glu Pro Ile Pro Asp Tyr Met Pro
                   485                 490                 495
   Leu Trp Glu Pro Lys Ser Ser Asp Lys Thr His Thr Cys Pro Pro Cys
               500                 505                 510
   Pro Ala Pro Glu Ala Glu Gly Ala Pro Ser Val Phe Leu Phe Pro Pro
           515                 520                 525
   Lys Pro Lys Asp Thr Leu Met Ile Ser Arg Thr Pro Glu Val Thr Cys
       530                 535                 540
   Val Val Val Asp Val Ser His Glu Asp Pro Glu Val Lys Phe Asn Trp
   545                 550                 555                 560
   Tyr Val Asp Gly Val Glu Val His Asn Ala Lys Thr Lys Pro Arg Glu
                   565                 570                 575
   Glu Gln Tyr Asn Ser Thr Tyr Arg Val Val Ser Val Leu Thr Val Leu
               580                 585                 590
   His Gln Asp Trp Leu Asn Gly Lys Glu Tyr Lys Cys Lys Val Ser Asn
           595                 600                 605
   Lys Ala Leu Pro Ser Ser Ile Glu Lys Thr Ile Ser Lys Ala Lys Gly
       610                 615                 620
   Gln Pro Arg Glu Pro Gln Val Tyr Thr Leu Pro Pro Ser Arg Asp Glu
   625                 630                 635                 640
   Leu Thr Lys Asn Gln Val Ser Leu Thr Cys Leu Val Lys Gly Phe Tyr
                   645                 650                 655
   Pro Ser Asp Ile Ala Val Glu Trp Glu Ser Asn Gly Gln Pro Glu Asn
               660                 665                 670
   Asn Tyr Lys Thr Thr Pro Pro Val Leu Asp Ser Asp Gly Ser Phe Phe
           675                 680                 685
   Leu Tyr Ser Lys Leu Thr Val Asp Lys Ser Arg Trp Gln Gln Gly Asn
       690                 695                 700
   Val Phe Ser Cys Ser Val Met His Glu Ala Leu His Asn His Tyr Thr
   705                 710                 715                 720
   Gln Lys Ser Leu Ser Leu Ser Pro Gly Lys
                   725                 730
<210> 143
   <211> 2124
   <212> DNA
   <213> Artificial Sequence
<220>
   <223> IL-17RA signal sequence and exons 1-3 of IL-17RA and exons 4-16 of human IL-17RC and Fc5
<400> 143
   atgggggccg cacgcagccc gccgtccgct gtcccggggc ccctgctggg gctgctcctg 60
   ctgctcctgg gcgtgctggc cccgggtggc gcctccctgc gactcctgga ccaccgggcg 120
   ctggtctgct cccagccggg gctaaactgc acggtcaaga atagtacctg cctggatgac 180
   agctggattc accctcgaaa cctgaccccc tcctccccaa aggacctgca gatccagctg 240
   cactttgccc acacccaaca aggagacctg ttccccgtgg ctcacatcga atggacactg 300
   cagacagacg ggcactggga agagcctgaa gatgaggaaa agtttggagg agcagctgac 360
   tcaggggtgg aggagcctag gaatgcctct ctccaggccc aagtcgtgct ctccttccag 420
   gcctacccta ctgcccgctg cgtcctgctg gaggtgcaag tgcctgctgc ccttgtgcag 480
   tttggtcagt ctgtgggctc tgtggtatat gactgcttcg aggctgccct agggagtgag 540
   gtacgaatct ggtcctatac tcagcccagg tacgagaagg aactcaacca cacacagcag 600
   ctgcctgact gcagggggct cgaagtctgg aattccatcc cgagctgctg ggccctgccc 660
   tggctcaacg tgtcagcaga tggtgacaac gtgcatctgg ttctgaatgt ctctgaggag 720
   cagcacttcg gcctctccct gtactggaat caggtccagg gccccccaaa accccggtgg 780
   cacaaaaacc tgactggacc gcagatcatt accttgaacc acacagacct ggttccctgc 840
   ctctgtattc aggtgtggcc tctggaacct gactccgtta ggacgaacat ctgccccttc 900
   agggaggacc cccgcgcaca ccagaacctc tggcaagccg cccgactgcg actgctgacc 960
   ctgcagagct ggctgctgga cgcaccgtgc tcgctgcccg cagaagcggc actgtgctgg 1020
   cgggctccgg gtggggaccc ctgccagcca ctggtcccac cgctttcctg ggagaacgtc 1080
   actgtggaca aggttctcga gttcccattg ctgaaaggcc accctaacct ctgtgttcag 1140
   gtgaacagct cggagaagct gcagctgcag gagtgcttgt gggctgactc cctggggcct 1200
   ctcaaagacg atgtgctact gttggagaca cgaggccccc aggacaacag atccctctgt 1260
   gccttggaac ccagtggctg tacttcacta cccagcaaag cctccacgag ggcagctcgc 1320
   cttggagagt acttactaca agacctgcag tcaggccagt gtctgcagct atgggacgat 1380
   gacttgggag cgctatgggc ctgccccatg gacaaataca tccacaagga gcccaaatct 1440
   tcagacaaaa ctcacacatg cccaccgtgc ccagcacctg aagccgaggg ggcaccgtca 1500
   gtcttcctct tccccccaaa acccaaggac accctcatga tctcccggac ccctgaggtc 1560
   acatgcgtgg tggtggacgt gagccacgaa gaccctgagg tcaagttcaa ctggtacgtg 1620
   gacggcgtgg aggtgcataa tgccaagaca aagccgcggg aggagcagta caacagcacg 1680
   taccgtgtgg tcagcgtcct caccgtcctg caccaggact ggctgaatgg caaggagtac 1740
   aagtgcaagg tctccaacaa agccctccca tcctccatcg agaaaaccat ctccaaagcc 1800
   aaagggcagc cccgagaacc acaggtgtac accctgcccc catcccggga tgagctgacc 1860
   aagaaccagg tcagcctgac ctgcctggtc aaaggcttct atcccagcga catcgccgtg 1920
   gagtgggaga gcaatgggca gccggagaac aactacaaga ccacgcctcc cgtgctggac 1980
   tccgacggct ccttcttcct ctacagcaag ctcaccgtgg acaagagcag gtggcagcag 2040
   gggaacgtct tctcatgctc cgtgatgcat gaggctctgc acaaccacta cacgcagaag 2100
   agcctctccc tgtctccggg taaa                                        2124
<210> 144
   <211> 708
   <212> PRT
   <213> Artificial Sequence
<220>
   <223> IL-17RA signal sequence and exons 1-3 of IL-17RA and exons 4-16 of human IL-17RC and Fc5
<400> 144
   Met Gly Ala Ala Arg Ser Pro Pro Ser Ala Val Pro Gly Pro Leu Leu
   1               5                   10                  15
   Gly Leu Leu Leu Leu Leu Leu Gly Val Leu Ala Pro Gly Gly Ala Ser
               20                  25                  30
   Leu Arg Leu Leu Asp His Arg Ala Leu Val Cys Ser Gln Pro Gly Leu
           35                  40                  45
   Asn Cys Thr Val Lys Asn Ser Thr Cys Leu Asp Asp Ser Trp Ile His
       50                  55                  60
   Pro Arg Asn Leu Thr Pro Ser Ser Pro Lys Asp Leu Gln Ile Gln Leu
   65                  70                  75                  80
   His Phe Ala His Thr Gln Gln Gly Asp Leu Phe Pro Val Ala His Ile
                   85                  90                  95
   Glu Trp Thr Leu Gln Thr Asp Gly His Trp Glu Glu Pro Glu Asp Glu
               100                 105                 110
   Glu Lys Phe Gly Gly Ala Ala Asp Ser Gly Val Glu Glu Pro Arg Asn
           115                 120                 125
   Ala Ser Leu Gln Ala Gln Val Val Leu Ser Phe Gln Ala Tyr Pro Thr
       130                 135                 140
   Ala Arg Cys Val Leu Leu Glu Val Gln Val Pro Ala Ala Leu Val Gln
   145                 150                 155                 160
   Phe Gly Gln Ser Val Gly Ser Val Val Tyr Asp Cys Phe Glu Ala Ala
                   165                 170                 175
   Leu Gly Ser Glu Val Arg Ile Trp Ser Tyr Thr Gln Pro Arg Tyr Glu
               180                 185                 190
   Lys Glu Leu Asn His Thr Gln Gln Leu Pro Asp Cys Arg Gly Leu Glu
           195                 200                 205
   Val Trp Asn Ser Ile Pro Ser Cys Trp Ala Leu Pro Trp Leu Asn Val
       210                 215                 220
   Ser Ala Asp Gly Asp Asn Val His Leu Val Leu Asn Val Ser Glu Glu
   225                 230                 235                 240
   Gln His Phe Gly Leu Ser Leu Tyr Trp Asn Gln Val Gln Gly Pro Pro
                   245                 250                 255
   Lys Pro Arg Trp His Lys Asn Leu Thr Gly Pro Gln Ile Ile Thr Leu
               260                 265                 270
   Asn His Thr Asp Leu Val Pro Cys Leu Cys Ile Gln Val Trp Pro Leu
           275                 280                 285
   Glu Pro Asp Ser Val Arg Thr Asn Ile Cys Pro Phe Arg Glu Asp Pro
       290                 295                 300
   Arg Ala His Gln Asn Leu Trp Gln Ala Ala Arg Leu Arg Leu Leu Thr
   305                 310                 315                 320
   Leu Gln Ser Trp Leu Leu Asp Ala Pro Cys Ser Leu Pro Ala Glu Ala
                   325                 330                 335
   Ala Leu Cys Trp Arg Ala Pro Gly Gly Asp Pro Cys Gln Pro Leu Val
               340                 345                 350
   Pro Pro Leu Ser Trp Glu Asn Val Thr Val Asp Lys Val Leu Glu Phe
           355                 360                 365
   Pro Leu Leu Lys Gly His Pro Asn Leu Cys Val Gln Val Asn Ser Ser
       370                 375                 380
   Glu Lys Leu Gln Leu Gln Glu Cys Leu Trp Ala Asp Ser Leu Gly Pro
   385                 390                 395                 400
   Leu Lys Asp Asp Val Leu Leu Leu Glu Thr Arg Gly Pro Gln Asp Asn
                   405                 410                 415
   Arg Ser Leu Cys Ala Leu Glu Pro Ser Gly Cys Thr Ser Leu Pro Ser
               420                 425                 430
   Lys Ala Ser Thr Arg Ala Ala Arg Leu Gly Glu Tyr Leu Leu Gln Asp
           435                 440                 445
   Leu Gln Ser Gly Gln Cys Leu Gln Leu Trp Asp Asp Asp Leu Gly Ala
       450                 455                 460
   Leu Trp Ala Cys Pro Met Asp Lys Tyr Ile His Lys Glu Pro Lys Ser
   465                 470                 475                 480
   Ser Asp Lys Thr His Thr Cys Pro Pro Cys Pro Ala Pro Glu Ala Glu
                   485                 490                 495
   Gly Ala Pro Ser Val Phe Leu Phe Pro Pro Lys Pro Lys Asp Thr Leu
               500                 505                 510
   Met Ile Ser Arg Thr Pro Glu Val Thr Cys Val Val Val Asp Val Ser
           515                 520                 525
   His Glu Asp Pro Glu Val Lys Phe Asn Trp Tyr Val Asp Gly Val Glu
       530                 535                 540
   Val His Asn Ala Lys Thr Lys Pro Arg Glu Glu Gln Tyr Asn Ser Thr
   545                 550                 555                 560
   Tyr Arg Val Val Ser Val Leu Thr Val Leu His Gln Asp Trp Leu Asn
                   565                 570                 575
   Gly Lys Glu Tyr Lys Cys Lys Val Ser Asn Lys Ala Leu Pro Ser Ser
               580                 585                 590
   Ile Glu Lys Thr Ile Ser Lys Ala Lys Gly Gln Pro Arg Glu Pro Gln
           595                 600                 605
   Val Tyr Thr Leu Pro Pro Ser Arg Asp Glu Leu Thr Lys Asn Gln Val
       610                 615                 620
   Ser Leu Thr Cys Leu Val Lys Gly Phe Tyr Pro Ser Asp Ile Ala Val
   625                 630                 635                 640
   Glu Trp Glu Ser Asn Gly Gln Pro Glu Asn Asn Tyr Lys Thr Thr Pro
                   645                 650                 655
   Pro Val Leu Asp Ser Asp Gly Ser Phe Phe Leu Tyr Ser Lys Leu Thr
               660                 665                 670
   Val Asp Lys Ser Arg Trp Gln Gln Gly Asn Val Phe Ser Cys Ser Val
           675                 680                 685
   Met His Glu Ala Leu His Asn His Tyr Thr Gln Lys Ser Leu Ser Leu
       690                 695                 700
   Ser Pro Gly Lys
   705
<210> 145
   <211> 2127
   <212> DNA
   <213> Artificial Sequence
<220>
   <223> IL-17RA signal sequence and exon 1 of IL-17RA and exons 2-16 of human IL-17RC and Fc5
<400> 145
   atgggggccg cacgcagccc gccgtccgct gtcccggggc ccctgctggg gctgctcctg 60
   ctgctcctgg gcgtgctggc cccgggtggc gcctccctgc gactcctgga ccaccgggcg 120
   ctggtctgct cccagccggg cctctcctgc cgcctctggg acagtgacat actctgcctg 180
   cctggggaca tcgtgcctgc tccgggcccc gtgctggcgc ctacgcacct gcagacagag 240
   ctggtgctga ggtgccagaa ggagaccgac tgtgacctct gtctgcgtgt ggctgtccac 300
   ttggccgtgc atgggcactg ggaagagcct gaagatgagg aaaagtttgg aggagcagct 360
   gactcagggg tggaggagcc taggaatgcc tctctccagg cccaagtcgt gctctccttc 420
   caggcctacc ctactgcccg ctgcgtcctg ctggaggtgc aagtgcctgc tgcccttgtg 480
   cagtttggtc agtctgtggg ctctgtggta tatgactgct tcgaggctgc cctagggagt 540
   gaggtacgaa tctggtccta tactcagccc aggtacgaga aggaactcaa ccacacacag 600
   cagctgcctg actgcagggg gctcgaagtc tggaattcca tcccgagctg ctgggccctg 660
   ccctggctca acgtgtcagc agatggtgac aacgtgcatc tggttctgaa tgtctctgag 720
   gagcagcact tcggcctctc cctgtactgg aatcaggtcc agggcccccc aaaaccccgg 780
   tggcacaaaa acctgactgg accgcagatc attaccttga accacacaga cctggttccc 840
   tgcctctgta ttcaggtgtg gcctctggaa cctgactccg ttaggacgaa catctgcccc 900
   ttcagggagg acccccgcgc acaccagaac ctctggcaag ccgcccgact gcgactgctg 960
   accctgcaga gctggctgct ggacgcaccg tgctcgctgc ccgcagaagc ggcactgtgc 1020
   tggcgggctc cgggtgggga cccctgccag ccactggtcc caccgctttc ctgggagaac 1080
   gtcactgtgg acaaggttct cgagttccca ttgctgaaag gccaccctaa cctctgtgtt 1140
   caggtgaaca gctcggagaa gctgcagctg caggagtgct tgtgggctga ctccctgggg 1200
   cctctcaaag acgatgtgct actgttggag acacgaggcc cccaggacaa cagatccctc 1260
   tgtgccttgg aacccagtgg ctgtacttca ctacccagca aagcctccac gagggcagct 1320
   cgccttggag agtacttact acaagacctg cagtcaggcc agtgtctgca gctatgggac 1380
   gatgacttgg gagcgctatg ggcctgcccc atggacaaat acatccacaa ggagcccaaa 1440
   tcttcagaca aaactcacac atgcccaccg tgcccagcac ctgaagccga gggggcaccg 1500
   tcagtcttcc tcttcccccc aaaacccaag gacaccctca tgatctcccg gacccctgag 1560
   gtcacatgcg tggtggtgga cgtgagccac gaagaccctg aggtcaagtt caactggtac 1620
   gtggacggcg tggaggtgca taatgccaag acaaagccgc gggaggagca gtacaacagc 1680
   acgtaccgtg tggtcagcgt cctcaccgtc ctgcaccagg actggctgaa tggcaaggag 1740
   tacaagtgca aggtctccaa caaagccctc ccatcctcca tcgagaaaac catctccaaa 1800
   gccaaagggc agccccgaga accacaggtg tacaccctgc ccccatcccg ggatgagctg 1860
   accaagaacc aggtcagcct gacctgcctg gtcaaaggct tctatcccag cgacatcgcc 1920
   gtggagtggg agagcaatgg gcagccggag aacaactaca agaccacgcc tcccgtgctg 1980
   gactccgacg gctccttctt cctctacagc aagctcaccg tggacaagag caggtggcag 2040
   caggggaacg tcttctcatg ctccgtgatg catgaggctc tgcacaacca ctacacgcag 2100
   aagagcctct ccctgtctcc gggtaaa                                     2127
<210> 146
   <211> 709
   <212> PRT
   <213> Artificial Sequence
<220>
   <223> IL-17RA signal sequence and exon 1 of IL-17RA and exons 2-16 of human IL-17RC and Fc5
<400> 146
   Met Gly Ala Ala Arg Ser Pro Pro Ser Ala Val Pro Gly Pro Leu Leu
   1               5                   10                  15
   Gly Leu Leu Leu Leu Leu Leu Gly Val Leu Ala Pro Gly Gly Ala Ser
               20                  25                  30
   Leu Arg Leu Leu Asp His Arg Ala Leu Val Cys Ser Gln Pro Gly Leu
           35                  40                  45
   Ser Cys Arg Leu Trp Asp Ser Asp Ile Leu Cys Leu Pro Gly Asp Ile
       50                  55                  60
   Val Pro Ala Pro Gly Pro Val Leu Ala Pro Thr His Leu Gln Thr Glu
   65                  70                  75                  80
   Leu Val Leu Arg Cys Gln Lys Glu Thr Asp Cys Asp Leu Cys Leu Arg
                   85                  90                  95
   Val Ala Val His Leu Ala Val His Gly His Trp Glu Glu Pro Glu Asp
               100                     105                     110
   Glu Glu Lys Phe Gly Gly Ala Ala Asp Ser Gly Val Glu Glu Pro Arg
           115                     120                     125
   Asn Ala Ser Leu Gln Ala Gln Val Val Leu Ser Phe Gln Ala Tyr Pro
       130                     135                     140
   Thr Ala Arg Cys Val Leu Leu Glu Val Gln Val Pro Ala Ala Leu Val
   145                     150                     155                     160
   Gln Phe Gly Gln Ser Val Gly Ser Val Val Tyr Asp Cys Phe Glu Ala
                   165                     170                     175
   Ala Leu Gly Ser Glu Val Arg Ile Trp Ser Tyr Thr Gln Pro Arg Tyr
               180                     185                     190
   Glu Lys Glu Leu Asn His Thr Gln Gln Leu Pro Asp Cys Arg Gly Leu
           195                     200                     205
   Glu Val Trp Asn Ser Ile Pro Ser Cys Trp Ala Leu Pro Trp Leu Asn
       210                     215                     220
   Val Ser Ala Asp Gly Asp Asn Val His Leu Val Leu Asn Val Ser Glu
   225                     230                     235                     240
   Glu Gln His Phe Gly Leu Ser Leu Tyr Trp Asn Gln Val Gln Gly Pro
                   245                     250                     255
   Pro Lys Pro Arg Trp His Lys Asn Leu Thr Gly Pro Gln Ile Ile Thr
               260                     265                     270
   Leu Asn His Thr Asp Leu Val Pro Cys Leu Cys Ile Gln Val Trp Pro
           275                     280                     285
   Leu Glu Pro Asp Ser Val Arg Thr Asn Ile Cys Pro Phe Arg Glu Asp
       290                     295                     300
   Pro Arg Ala His Gln Asn Leu Trp Gln Ala Ala Arg Leu Arg Leu Leu
   305                     310                     315                     320
   Thr Leu Gln Ser Trp Leu Leu Asp Ala Pro Cys Ser Leu Pro Ala Glu
                   325                     330                     335
   Ala Ala Leu Cys Trp Arg Ala Pro Gly Gly Asp Pro Cys Gln Pro Leu
               340                     345                     350
   Val Pro Pro Leu Ser Trp Glu Asn Val Thr Val Asp Lys Val Leu Glu
           355                     360                     365
   Phe Pro Leu Leu Lys Gly His Pro Asn Leu Cys Val Gln Val Asn Ser
       370                     375                     380
   Ser Glu Lys Leu Gln Leu Gln Glu Cys Leu Trp Ala Asp Ser Leu Gly
   385                     390                     395                     400
   Pro Leu Lys Asp Asp Val Leu Leu Leu Glu Thr Arg Gly Pro Gln Asp
                   405                     410                     415
   Asn Arg Ser Leu Cys Ala Leu Glu Pro Ser Gly Cys Thr Ser Leu Pro
               420                     425                     430
   Ser Lys Ala Ser Thr Arg Ala Ala Arg Leu Gly Glu Tyr Leu Leu Gln
           435                     440                     445
   Asp Leu Gln Ser Gly Gln Cys Leu Gln Leu Trp Asp Asp Asp Leu Gly
       450                     455                     460
   Ala Leu Trp Ala Cys Pro Met Asp Lys Tyr Ile His Lys Glu Pro Lys
   465                     470                     475                     480
   Ser Ser Asp Lys Thr His Thr Cys Pro Pro Cys Pro Ala Pro Glu Ala
                   485                     490                     495
   Glu Gly Ala Pro Ser Val Phe Leu Phe Pro Pro Lys Pro Lys Asp Thr
               500                     505                     510
   Leu Met Ile Ser Arg Thr Pro Glu Val Thr Cys Val Val Val Asp Val
           515                     520                     525
   Ser His Glu Asp Pro Glu Val Lys Phe Asn Trp Tyr Val Asp Gly Val
       530                     535                     540
   Glu Val His Asn Ala Lys Thr Lys Pro Arg Glu Glu Gln Tyr Asn Ser
   545                     550                     555                     560
   Thr Tyr Arg Val Val Ser Val Leu Thr Val Leu His Gln Asp Trp Leu
                   565                     570                     575
   Asn Gly Lys Glu Tyr Lys Cys Lys Val Ser Asn Lys Ala Leu Pro Ser
           580                 585                 590
   Ser Ile Glu Lys Thr Ile Ser Lys Ala Lys Gly Gln Pro Arg Glu Pro
           595                 600                 605
   Gln Val Tyr Thr Leu Pro Pro Ser Arg Asp Glu Leu Thr Lys Asn Gln
       610                 615                 620
   Val Ser Leu Thr Cys Leu Val Lys Gly Phe Tyr Pro Ser Asp Ile Ala
   625                 630                 635                 640
   Val Glu Trp Glu Ser Asn Gly Gln Pro Glu Asn Asn Tyr Lys Thr Thr
                   645                 650                 655
   Pro Pro Val Leu Asp Ser Asp Gly Ser Phe Phe Leu Tyr Ser Lys Leu
               660                 665                 670
   Thr Val Asp Lys Ser Arg Trp Gln Gln Gly Asn Val Phe Ser Cys Ser
           675                 680                 685
   Val Met His Glu Ala Leu His Asn His Tyr Thr Gln Lys Ser Leu Ser
       690                 695                 700
   Leu Ser Pro Gly Lys
   705
<210> 147
   <211> 2094
   <212> DNA
   <213> Artificial Sequence
<220>
   <223> IL-17RC signal sequence and exons 1-16 of IL-17RCx4 with Cys194Ser and Cys202Ser substitutions and exons 2-16 of human IL-17RC and Fc5
<400> 147
   atgcctgtgc cctggttctt gctgtccttg gcactgggcc gaagcccagt ggtcctttct 60
   ctggagaggc ttgtggggcc tcaggacgct acccactgct ctccgggcct ctcctgccgc 120
   ctctgggaca gtgacatact ctgcctgcct ggggacatcg tgcctgctcc gggccccgtg 180
   ctggcgccta cgcacctgca gacagagctg gtgctgaggt gccagaagga gaccgactgt 240
   gacctctgtc tgcgtgtggc tgtccacttg gccgtgcatg ggcactggga agagcctgaa 300
   gatgaggaaa agtttggagg agcagctgac tcaggggtgg aggagcctag gaatgcctct 360
   ctccaggccc aagtcgtgct ctccttccag gcctacccta ctgcccgctg cgtcctgctg 420
   gaggtgcaag tgcctgctgc ccttgtgcag tttggtcagt ctgtgggctc tgtggtatat 480
   gactgcttcg aggctgccct agggagtgag gtacgaatct ggtcctatac tcagcccagg 540
   tacgagaagg aactcaacca cacacagcag ctgcctgact ccagggggct cgaagtctgg 600
   aattccatcc cgagctcctg ggccctgccc tggctcaacg tgtcagcaga tggtgacaac 660
   gtgcatctgg ttctgaatgt ctctgaggag cagcacttcg gcctctccct gtactggaat 720
   caggtccagg gccccccaaa accccggtgg cacaaaaacc tgactggacc gcagatcatt 780
   accttgaacc acacagacct ggttccctgc ctctgtattc aggtgtggcc tctggaacct 840
   gactccgtta ggacgaacat ctgccccttc agggaggacc cccgcgcaca ccagaacctc 900
   tggcaagccg cccgactgcg actgctgacc ctgcagagct ggctgctgga cgcaccgtgc 960
   tcgctgcccg cagaagcggc actgtgctgg cgggctccgg gtggggaccc ctgccagcca 1020
   ctggtcccac cgctttcctg ggagaacgtc actgtggaca aggttctcga gttcccattg 1080
   ctgaaaggcc accctaacct ctgtgttcag gtgaacagct cggagaagct gcagctgcag 1140
   gagtgcttgt gggctgactc cctggggcct ctcaaagacg atgtgctact gttggagaca 1200
   cgaggccccc aggacaacag atccctctgt gccttggaac ccagtggctg tacttcacta 1260
   cccagcaaag cctccacgag ggcagctcgc cttggagagt acttactaca agacctgcag 1320
   tcaggccagt gtctgcagct atgggacgat gacttgggag cgctatgggc ctgccccatg 1380
   gacaaataca tccacaagga gcccaaatct tcagacaaaa ctcacacatg cccaccgtgc 1440
   ccagcacctg aagccgaggg ggcaccgtca gtcttcctct tccccccaaa acccaaggac 1500
   accctcatga tctcccggac ccctgaggtc acatgcgtgg tggtggacgt gagccacgaa 1560
   gaccctgagg tcaagttcaa ctggtacgtg gacggcgtgg aggtgcataa tgccaagaca 1620
   aagccgcggg aggagcagta caacagcacg taccgtgtgg tcagcgtcct caccgtcctg 1680
   caccaggact ggctgaatgg caaggagtac aagtgcaagg tctccaacaa agccctccca 1740
   tcctccatcg agaaaaccat ctccaaagcc aaagggcagc cccgagaacc acaggtgtac 1800
   accctgcccc catcccggga tgagctgacc aagaaccagg tcagcctgac ctgcctggtc 1860
   aaaggcttct atcccagcga catcgccgtg gagtgggaga gcaatgggca gccggagaac 1920
   aactacaaga ccacgcctcc cgtgctggac tccgacggct ccttcttcct ctacagcaag 1980
   ctcaccgtgg acaagagcag gtggcagcag gggaacgtct tctcatgctc cgtgatgcat 2040
   gaggctctgc acaaccacta cacgcagaag agcctctccc tgtctccggg taaa       2094
<210> 148
   <211> 698
   <212> PRT
   <213> Artificial Sequence
<220>
   <223> IL-17RC signal sequence and exons 1-16 of IL-17RCx4 with Cys194Ser and Cys202Ser substitutions and exons 2-16 of human IL-17RC and Fc5
<400> 148
   Met Pro Val Pro Trp Phe Leu Leu Ser Leu Ala Leu Gly Arg Ser Pro
    1               5                  10                  15
   Val Val Leu Ser Leu Glu Arg Leu Val Gly Pro Gln Asp Ala Thr His
               20                  25                  30
   Cys Ser Pro Gly Leu Ser Cys Arg Leu Trp Asp Ser Asp Ile Leu Cys
           35                  40                  45
   Leu Pro Gly Asp Ile Val Pro Ala Pro Gly Pro Val Leu Ala Pro Thr
       50                  55                  60
   His Leu Gln Thr Glu Leu Val Leu Arg Cys Gln Lys Glu Thr Asp Cys
   65                  70                  75                  80
   Asp Leu Cys Leu Arg Val Ala Val His Leu Ala Val His Gly His Trp
                   85                  90                  95
   Glu Glu Pro Glu Asp Glu Glu Lys Phe Gly Gly Ala Ala Asp Ser Gly
               100                 105                 110
   Val Glu Glu Pro Arg Asn Ala Ser Leu Gln Ala Gln Val Val Leu Ser
           115                 120                 125
   Phe Gln Ala Tyr Pro Thr Ala Arg Cys Val Leu Leu Glu Val Gln Val
       130                 135                 140
   Pro Ala Ala Leu Val Gln Phe Gly Gln Ser Val Gly Ser Val Val Tyr
   145                 150                 155                 160
   Asp Cys Phe Glu Ala Ala Leu Gly Ser Glu Val Arg Ile Trp Ser Tyr
                   165                 170                 175
   Thr Gln Pro Arg Tyr Glu Lys Glu Leu Asn His Thr Gln Gln Leu Pro
               180                 185                 190
   Asp Ser Arg Gly Leu Glu Val Trp Asn Ser Ile Pro Ser Ser Trp Ala
           195                 200                 205
   Leu Pro Trp Leu Asn Val Ser Ala Asp Gly Asp Asn Val His Leu Val
       210                 215                 220
   Leu Asn Val Ser Glu Glu Gln His Phe Gly Leu Ser Leu Tyr Trp Asn
   225                 230                 235                 240
   Gln Val Gln Gly Pro Pro Lys Pro Arg Trp His Lys Asn Leu Thr Gly
                   245                 250                 255
   Pro Gln Ile Ile Thr Leu Asn His Thr Asp Leu Val Pro Cys Leu Cys
               260                 265                 270
   Ile Gln Val Trp Pro Leu Glu Pro Asp Ser Val Arg Thr Asn Ile Cys
           275                 280                 285
   Pro Phe Arg Glu Asp Pro Arg Ala His Gln Asn Leu Trp Gln Ala Ala
       290                 295                 300
   Arg Leu Arg Leu Leu Thr Leu Gln Ser Trp Leu Leu Asp Ala Pro Cys
   305                 310                 315                 320
   Ser Leu Pro Ala Glu Ala Ala Leu Cys Trp Arg Ala Pro Gly Gly Asp
                   325                 330                 335
   Pro Cys Gln Pro Leu Val Pro Pro Leu Ser Trp Glu Asn Val Thr Val
               340                 345                 350
   Asp Lys Val Leu Glu Phe Pro Leu Leu Lys Gly His Pro Asn Leu Cys
           355                 360                 365
   Val Gln Val Asn Ser Ser Glu Lys Leu Gln Leu Gln Glu Cys Leu Trp
       370                 375                 380
   Ala Asp Ser Leu Gly Pro Leu Lys Asp Asp Val Leu Leu Leu Glu Thr
   385                 390                 395                 400
   Arg Gly Pro Gln Asp Asn Arg Ser Leu Cys Ala Leu Glu Pro Ser Gly
                   405                 410                 415
   Cys Thr Ser Leu Pro Ser Lys Ala Ser Thr Arg Ala Ala Arg Leu Gly
               420                 425                 430
   Glu Tyr Leu Leu Gln Asp Leu Gln Ser Gly Gln Cys Leu Gln Leu Trp
           435                 440                 445
   Asp Asp Asp Leu Gly Ala Leu Trp Ala Cys Pro Met Asp Lys Tyr Ile
       450                 455                 460
   His Lys Glu Pro Lys Ser Ser Asp Lys Thr His Thr Cys Pro Pro Cys
   465                 470                 475                 480
   Pro Ala Pro Glu Ala Glu Gly Ala Pro Ser Val Phe Leu Phe Pro Pro
                   485                 490                 495
   Lys Pro Lys Asp Thr Leu Met Ile Ser Arg Thr Pro Glu Val Thr Cys
               500                 505                 510
   Val Val Val Asp Val Ser His Glu Asp Pro Glu Val Lys Phe Asn Trp
           515                 520                 525
   Tyr Val Asp Gly Val Glu Val His Asn Ala Lys Thr Lys Pro Arg Glu
       530                 535                 540
   Glu Gln Tyr Asn Ser Thr Tyr Arg Val Val Ser Val Leu Thr Val Leu
   545                 550                 555                 560
   His Gln Asp Trp Leu Asn Gly Lys Glu Tyr Lys Cys Lys Val Ser Asn
                   565                 570                 575
   Lys Ala Leu Pro Ser Ser Ile Glu Lys Thr Ile Ser Lys Ala Lys Gly
               580                 585                 590
   Gln Pro Arg Glu Pro Gln Val Tyr Thr Leu Pro Pro Ser Arg Asp Glu
           595                 600                 605
   Leu Thr Lys Asn Gln Val Ser Leu Thr Cys Leu Val Lys Gly Phe Tyr
       610                 615                 620
   Pro Ser Asp Ile Ala Val Glu Trp Glu Ser Asn Gly Gln Pro Glu Asn
   625                 630                 635                 640
   Asn Tyr Lys Thr Thr Pro Pro Val Leu Asp Ser Asp Gly Ser Phe Phe
                   645                 650                 655
   Leu Tyr Ser Lys Leu Thr Val Asp Lys Ser Arg Trp Gln Gln Gly Asn
               660                 665                 670
   Val Phe Ser Cys Ser Val Met His Glu Ala Leu His Asn His Tyr Thr
           675                 680                 685
   Gln Lys Ser Leu Ser Leu Ser Pro Gly Lys
       690                 695
<210> 149
   <211> 2061
   <212> DNA
   <213> Artificial Sequence
<220>
   <223> IL-17RC signal sequence and exons 1-6 and 8-16 of IL-17RC with GlyGlyGlySer linker between exons 6 and 8, and Fc5
<400> 149
   atgcctgtgc cctggttctt gctgtccttg gcactgggcc gaagcccagt ggtcctttct 60
   ctggagaggc ttgtggggcc tcaggacgct acccactgct ctccgggcct ctcctgccgc 120
   ctctgggaca gtgacatact ctgcctgcct ggggacatcg tgcctgctcc gggccccgtg 180
   ctggcgccta cgcacctgca gacagagctg gtgctgaggt gccagaagga gaccgactgt 240
   gacctctgtc tgcgtgtggc tgtccacttg gccgtgcatg ggcactggga agagcctgaa 300
   gatgaggaaa agtttggagg agcagctgac tcaggggtgg aggagcctag gaatgcctct 360
   ctccaggccc aagtcgtgct ctccttccag gcctacccta ctgcccgctg cgtcctgctg 420
   gaggtgcaag tgcctgctgc ccttgtgcag tttggtcagt ctgtgggctc tgtggtatat 480
   gactgcttcg aggctgccct agggagtgag gtacgaatct ggtcctatac tcagcccagg 540
   tacgagaagg aactcaacca cacacagcag ctgcctggag gaggatccgc cctgccctgg 600
   ctcaacgtgt cagcagatgg tgacaacgtg catctggttc tgaatgtctc tgaggagcag 660
   cacttcggcc tctccctgta ctggaatcag gtccagggcc ccccaaaacc ccggtggcac 720
   aaaaacctga ctggaccgca gatcattacc ttgaaccaca cagacctggt tccctgcctc 780
   tgtattcagg tgtggcctct ggaacctgac tccgttagga cgaacatctg ccccttcagg 840
   gaggaccccc gcgcacacca gaacctctgg caagccgccc gactgcgact gctgaccctg 900
   cagagctggc tgctggacgc accgtgctcg ctgcccgcag aagcggcact gtgctggcgg 960
   gctccgggtg ggaccccctg ccagccactg gtcccaccgc tttcctggga gaacgtcact 1020
   gtggacaagg ttctcgagtt cccattgctg aaaggccacc ctaacctctg tgttcaggtg 1080
   aacagctcgg agaagctgca gctgcaggag tgcttgtggg ctgactccct ggggcctctc 1140
   aaagacgatg tgctactgtt ggagacacga ggcccccagg acaacagatc cctctgtgcc 1200
   ttggaaccca gtggctgtac ttcactaccc agcaaagcct ccacgagggc agctcgcctt 1260
   ggagagtact tactacaaga cctgcagtca ggccagtgtc tgcagctatg ggacgatgac 1320
   ttgggagcgc tatgggcctg ccccatggac aaatacatcc acaaggagcc caaatcttca 1380
   gacaaaactc acacatgccc accgtgccca gcacctgaag ccgagggggc accgtcagtc 1440
   ttcctcttcc ccccaaaacc caaggacacc ctcatgatct cccggacccc tgaggtcaca 1500
   tgcgtggtgg tggacgtgag ccacgaagac cctgaggtca agttcaactg gtacgtggac 1560
   ggcgtggagg tgcataatgc caagacaaag ccgcgggagg agcagtacaa cagcacgtac 1620
   cgtgtggtca gcgtcctcac cgtcctgcac caggactggc tgaatggcaa ggagtacaag 1680
   tgcaaggtct ccaacaaagc cctcccatcc tccatcgaga aaaccatctc caaagccaaa 1740
   gggcagcccc gagaaccaca ggtgtacacc ctgcccccat cccgggatga gctgaccaag 1800
   aaccaggtca gcctgacctg cctggtcaaa ggcttctatc ccagcgacat cgccgtggag 1860
   tgggagagca atgggcagcc ggagaacaac tacaagacca cgcctcccgt gctggactcc 1920
   gacggctcct tcttcctcta cagcaagctc accgtggaca agagcaggtg gcagcagggg 1980
   aacgtcttct catgctccgt gatgcatgag gctctgcaca accactacac gcagaagagc 2040
   ctctccctgt ctccgggtaa a                                           2061
<210> 150
   <211> 687
   <212> PRT
   <213> Artificial Sequence
<220>
   <223> IL-17RC signal sequence and exons 1-6 and 8-16 of IL-17RC with GlyGlyGlySer linker between exons 6 and 8, and Fc5
<400> 150
   Met Pro Val Pro Trp Phe Leu Leu Ser Leu Ala Leu Gly Arg Ser Pro
   1               5                   10                  15
   Val Val Leu Ser Leu Glu Arg Leu Val Gly Pro Gln Asp Ala Thr His
               20                  25                  30
   Cys Ser Pro Gly Leu Ser Cys Arg Leu Trp Asp Ser Asp Ile Leu Cys
           35                  40                  45
   Leu Pro Gly Asp Ile Val Pro Ala Pro Gly Pro Val Leu Ala Pro Thr
       50                  55                  60
   His Leu Gln Thr Glu Leu Val Leu Arg Cys Gln Lys Glu Thr Asp Cys
   65                  70                  75                  80
   Asp Leu Cys Leu Arg Val Ala Val His Leu Ala Val His Gly His Trp
                   85                  90                  95
   Glu Glu Pro Glu Asp Glu Glu Lys Phe Gly Gly Ala Ala Asp Ser Gly
               100                 105                 110
   Val Glu Glu Pro Arg Asn Ala Ser Leu Gln Ala Gln Val Val Leu Ser
           115                 120                 125
   Phe Gln Ala Tyr Pro Thr Ala Arg Cys Val Leu Leu Glu Val Gln Val
       130                 135                 140
   Pro Ala Ala Leu Val Gln Phe Gly Gln Ser Val Gly Ser Val Val Tyr
   145                 150                 155                 160
   Asp Cys Phe Glu Ala Ala Leu Gly Ser Glu Val Arg Ile Trp Ser Tyr
                   165                 170                 175
   Thr Gln Pro Arg Tyr Glu Lys Glu Leu Asn His Thr Gln Gln Leu Pro
               180                 185                 190
   Gly Gly Gly Ser Ala Leu Pro Trp Leu Asn Val Ser Ala Asp Gly Asp
           195                 200                 205
   Asn Val His Leu Val Leu Asn Val Ser Glu Glu Gln His Phe Gly Leu
       210                 215                 220
   Ser Leu Tyr Trp Asn Gln Val Gln Gly Pro Pro Lys Pro Arg Trp His
   225                 230                 235                 240
   Lys Asn Leu Thr Gly Pro Gln Ile Ile Thr Leu Asn His Thr Asp Leu
                   245                 250                 255
   Val Pro Cys Leu Cys Ile Gln Val Trp Pro Leu Glu Pro Asp Ser Val
               260                 265                 270
   Arg Thr Asn Ile Cys Pro Phe Arg Glu Asp Pro Arg Ala His Gln Asn
           275                 280                 285
   Leu Trp Gln Ala Ala Arg Leu Arg Leu Leu Thr Leu Gln Ser Trp Leu
       290                 295                 300
   Leu Asp Ala Pro Cys Ser Leu Pro Ala Glu Ala Ala Leu Cys Trp Arg
   305                 310                 315                 320
   Ala Pro Gly Gly Asp Pro Cys Gln Pro Leu Val Pro Pro Leu Ser Trp
                   325                 330                 335
   Glu Asn Val Thr Val Asp Lys Val Leu Glu Phe Pro Leu Leu Lys Gly
               340                 345                 350
   His Pro Asn Leu Cys Val Gln Val Asn Ser Ser Glu Lys Leu Gln Leu
           355                 360                 365
   Gln Glu Cys Leu Trp Ala Asp Ser Leu Gly Pro Leu Lys Asp Asp Val
       370                 375                 380
   Leu Leu Leu Glu Thr Arg Gly Pro Gln Asp Asn Arg Ser Leu Cys Ala
   385                 390                 395                 400
   Leu Glu Pro Ser Gly Cys Thr Ser Leu Pro Ser Lys Ala Ser Thr Arg
                   405                 410                 415
   Ala Ala Arg Leu Gly Glu Tyr Leu Leu Gln Asp Leu Gln Ser Gly Gln
               420                 425                 430
   Cys Leu Gln Leu Trp Asp Asp Asp Leu Gly Ala Leu Trp Ala Cys Pro
           435                 440                 445
   Met Asp Lys Tyr Ile His Lys Glu Pro Lys Ser Ser Asp Lys Thr His
       450                 455                 460
   Thr Cys Pro Pro Cys Pro Ala Pro Glu Ala Glu Gly Ala Pro Ser Val
   465                 470                 475                 480
   Phe Leu Phe Pro Pro Lys Pro Lys Asp Thr Leu Met Ile Ser Arg Thr
                   485                 490                 495
   Pro Glu Val Thr Cys Val Val Val Asp Val Ser His Glu Asp Pro Glu
               500                 505                 510
   Val Lys Phe Asn Trp Tyr Val Asp Gly Val Glu Val His Asn Ala Lys
           515                 520                 525
   Thr Lys Pro Arg Glu Glu Gln Tyr Asn Ser Thr Tyr Arg Val Val Ser
       530                 535                 540
   Val Leu Thr Val Leu His Gln Asp Trp Leu Asn Gly Lys Glu Tyr Lys
   545                 550                 555                 560
   Cys Lys Val Ser Asn Lys Ala Leu Pro Ser Ser Ile Glu Lys Thr Ile
                   565                 570                 575
   Ser Lys Ala Lys Gly Gln Pro Arg Glu Pro Gln Val Tyr Thr Leu Pro
               580                 585                 590
   Pro Ser Arg Asp Glu Leu Thr Lys Asn Gln Val Ser Leu Thr Cys Leu
           595                 600                 605
   Val Lys Gly Phe Tyr Pro Ser Asp Ile Ala Val Glu Trp Glu Ser Asn
       610                 615                 620
   Gly Gln Pro Glu Asn Asn Tyr Lys Thr Thr Pro Pro Val Leu Asp Ser
   625                 630                 635                 640
   Asp Gly Ser Phe Phe Leu Tyr Ser Lys Leu Thr Val Asp Lys Ser Arg
                   645                 650                 655
   Trp Gln Gln Gly Asn Val Phe Ser Cys Ser Val Met His Glu Ala Leu
               660                 665                 670
   His Asn His Tyr Thr Gln Lys Ser Leu Ser Leu Ser Pro Gly Lys
           675                 680                 685
<210> 151
   <211> 2094
   <212> DNA
   <213> Artificial Sequence
<220>
   <223> otPA pre-pro signal sequence and exons 1-6 and 8-16 of IL-17RC with a Leu21Ala substitution, and Fc5
<400> 151
   atggatgcaa tgaagagagg gctctgctgt gtgctgctgc tgtgtggcgc cgtcttcgtt 60
   tcgctcagcc aggaaatcca tgccgagttg agacgcttcc gtagagcaga gaggcttgtg 120
   gggcctcagg acgctaccca ctgctctccg ggcctctcct gccgcctctg ggacagtgac 180
   atactctgcc tgcctgggga catcgtgcct gctccgggcc ccgtgctggc gcctacgcac 240
   ctgcagacag agctggtgct gaggtgccag aaggagaccg actgtgacct ctgtctgcgt 300
   gtggctgtcc acttggccgt gcatgggcac tgggaagagc ctgaagatga ggaaaagttt 360
   ggaggagcag ctgactcagg ggtggaggag cctaggaatg cctctctcca ggcccaagtc 420
   gtgctctcct tccaggccta ccctactgcc cgctgcgtcc tgctggaggt gcaagtgcct 480
   gctgcccttg tgcagtttgg tcagtctgtg ggctctgtgg tatatgactg cttcgaggct 540
   gccctaggga gtgaggtacg aatctggtcc tatactcagc ccaggtacga gaaggaactc 600
   aaccacacac agcagctgcc tgccctgccc tggctcaacg tgtcagcaga tggtgacaac 660
   gtgcatctgg ttctgaatgt ctctgaggag cagcacttcg gcctctccct gtactggaat 720
   caggtccagg gccccccaaa accccggtgg cacaaaaacc tgactggacc gcagatcatt 780
   accttgaacc acacagacct ggttccctgc ctctgtattc aggtgtggcc tctggaacct 840
   gactccgtta ggacgaacat ctgccccttc agggaggacc cccgcgcaca ccagaacctc 900
   tggcaagccg cccgactgcg actgctgacc ctgcagagct ggctgctgga cgcaccgtgc 960
   tcgctgcccg cagaagcggc actgtgctgg cgggctccgg gtggggaccc ctgccagcca 1020
   ctggtcccac cgctttcctg ggagaacgtc actgtggaca aggttctcga gttcccattg 1080
   ctgaaaggcc accctaacct ctgtgttcag gtgaacagct cggagaagct gcagctgcag 1140
   gagtgcttgt gggctgactc cctggggcct ctcaaagacg atgtgctact gttggagaca 1200
   cgaggccccc aggacaacag atccctctgt gccttggaac ccagtggctg tacttcacta 1260
   cccagcaaag cctccacgag ggcagctcgc cttggagagt acttactaca agacctgcag 1320
   tcaggccagt gtctgcagct atgggacgat gacttgggag cgctatgggc ctgccccatg 1380
   gacaaataca tccacaagga gcccaaatct tcagacaaaa ctcacacatg cccaccgtgc 1440
   ccagcacctg aagccgaggg ggcaccgtca gtcttcctct tccccccaaa acccaaggac 1500
   accctcatga tctcccggac ccctgaggtc acatgcgtgg tggtggacgt gagccacgaa 1560
   gaccctgagg tcaagttcaa ctggtacgtg gacggcgtgg aggtgcataa tgccaagaca 1620
   aagccgcggg aggagcagta caacagcacg taccgtgtgg tcagcgtcct caccgtcctg 1680
   caccaggact ggctgaatgg caaggagtac aagtgcaagg tctccaacaa agccctccca 1740
   tcctccatcg agaaaaccat ctccaaagcc aaagggcagc cccgagaacc acaggtgtac 1800
   accctgcccc catcccggga tgagctgacc aagaaccagg tcagcctgac ctgcctggtc 1860
   aaaggcttct atcccagcga catcgccgtg gagtgggaga gcaatgggca gccggagaac 1920
   aactacaaga ccacgcctcc cgtgctggac tccgacggct ccttcttcct ctacagcaag 1980
   ctcaccgtgg acaagagcag gtggcagcag gggaacgtct tctcatgctc cgtgatgcat 2040
   gaggctctgc acaaccacta cacgcagaag agcctctccc tgtctccggg taaa       2094
<210> 152
   <211> 698
   <212> PRT
   <213> Artificial Sequence
<220>
   <223> otPA pre-pro signal sequence and exons 1-6 and 8-16 of IL-17RC with.a Leu21Ala substitution, and Fc5
<400> 152
   Met Asp Ala Met Lys Arg Gly Leu Cys Cys Val Leu Leu Leu Cys Gly
    1               5                  10                  15
   Ala Val Phe Val Ser Leu Ser Gln Glu Ile His Ala Glu Leu Arg Arg
               20                  25                  30
   Phe Arg Arg Ala Glu Arg Leu Val Gly Pro Gln Asp Ala Thr His Cys
           35                  40                  45
   Ser Pro Gly Leu Ser Cys Arg Leu Trp Asp Ser Asp Ile Leu Cys Leu
       50                  55                  60
   Pro Gly Asp Ile Val Pro Ala Pro Gly Pro Val Leu Ala Pro Thr His
   65                  70                  75                  80
   Leu Gln Thr Glu Leu Val Leu Arg Cys Gln Lys Glu Thr Asp Cys Asp
                   85                  90                  95
   Leu Cys Leu Arg Val Ala Val His Leu Ala Val His Gly His Trp Glu
               100                 105                 110
   Glu Pro Glu Asp Glu Glu Lys Phe Gly Gly Ala Ala Asp Ser Gly Val
           115                 120                 125
   Glu Glu Pro Arg Asn Ala Ser Leu Gln Ala Gln Val Val Leu Ser Phe
       130                 135                 140
   Gln Ala Tyr Pro Thr Ala Arg Cys Val Leu Leu Glu Val Gln Val Pro
   145                 150                 155                 160
   Ala Ala Leu Val Gln Phe Gly Gln Ser Val Gly Ser Val Val Tyr Asp
                   165                 170                 175
   Cys Phe Glu Ala Ala Leu Gly Ser Glu Val Arg Ile Trp Ser Tyr Thr
               180                 185                 190
   Gln Pro Arg Tyr Glu Lys Glu Leu Asn His Thr Gln Gln Leu Pro Ala
           195                 200                 205
   Leu Pro Trp Leu Asn Val Ser Ala Asp Gly Asp Asn Val His Leu Val
       210                 215                 220
   Leu Asn Val Ser Glu Glu Gln His Phe Gly Leu Ser Leu Tyr Trp Asn
   225                 230                 235                 240
   Gln Val Gln Gly Pro Pro Lys Pro Arg Trp His Lys Asn Leu Thr Gly
                   245                 250                 255
   Pro Gln Ile Ile Thr Leu Asn His Thr Asp Leu Val Pro Cys Leu Cys
               260                 265                 270
   Ile Gln Val Trp Pro Leu Glu Pro Asp Ser Val Arg Thr Asn Ile Cys
           275                 280                 285
   Pro Phe Arg Glu Asp Pro Arg Ala His Gln Asn Leu Trp Gln Ala Ala
       290                 295                 300
   Arg Leu Arg Leu Leu Thr Leu Gln Ser Trp Leu Leu Asp Ala Pro Cys
   305                 310                 315                 320
   Ser Leu Pro Ala Glu Ala Ala Leu Cys Trp Arg Ala Pro Gly Gly Asp
                           325                     330                     335
   Pro Cys Gln Pro Leu Val Pro Pro Leu Ser Trp Glu Asn Val Thr Val
               340                     345                     350
   Asp Lys Val Leu Glu Phe Pro Leu Leu Lys Gly His Pro Asn Leu Cys
           355                     360                     365
   Val Gln Val Asn Ser Ser Glu Lys Leu Gln Leu Gln Glu Cys Leu Trp
       370                     375                     380
   Ala Asp Ser Leu Gly Pro Leu Lys Asp Asp Val Leu Leu Leu Glu Thr
   385                     390                     395                     400
   Arg Gly Pro Gln Asp Asn Arg Ser Leu Cys Ala Leu Glu Pro Ser Gly
                   405                     410                     415
   Cys Thr Ser Leu Pro Ser Lys Ala Ser Thr Arg Ala Ala Arg Leu Gly
               420                     425                     430
   Glu Tyr Leu Leu Gln Asp Leu Gln Ser Gly Gln Cys Leu Gln Leu Trp
           435                     440                     445
   Asp Asp Asp Leu Gly Ala Leu Trp Ala Cys Pro Met Asp Lys Tyr Ile
       450                     455                     460
   His Lys Glu Pro Lys Ser Ser Asp Lys Thr His Thr Cys Pro Pro Cys
   465                     470                     475                     480
   Pro Ala Pro Glu Ala Glu Gly Ala Pro Ser Val Phe Leu Phe Pro Pro
                   485                     490                     495
   Lys Pro Lys Asp Thr Leu Met Ile Ser Arg Thr Pro Glu Val Thr Cys
               500                     505                     510
   Val Val Val Asp Val Ser His Glu Asp Pro Glu Val Lys Phe Asn Trp
           515                     520                     525
   Tyr Val Asp Gly Val Glu Val His Asn Ala Lys Thr Lys Pro Arg Glu
       530                     535                     540
   Glu Gln Tyr Asn Ser Thr Tyr Arg Val Val Ser Val Leu Thr Val Leu
   545                     550                     555                     560
   His Gln Asp Trp Leu Asn Gly Lys Glu Tyr Lys Cys Lys Val Ser Asn
                   565                     570                     575
   Lys Ala Leu Pro Ser Ser Ile Glu Lys Thr Ile Ser Lys Ala Lys Gly
               580                     585                     590
   Gln Pro Arg Glu Pro Gln Val Tyr Thr Leu Pro Pro Ser Arg Asp Glu
           595                     600                     605
   Leu Thr Lys Asn Gln Val Ser Leu Thr Cys Leu Val Lys Gly Phe Tyr
       610                     615                     620
   Pro Ser Asp Ile Ala Val Glu Trp Glu Ser Asn Gly Gln Pro Glu Asn
   625                     630                     635                     640
   Asn Tyr Lys Thr Thr Pro Pro Val Leu Asp Ser Asp Gly Ser Phe Phe
                   645                     650                     655
   Leu Tyr Ser Lys Leu Thr Val Asp Lys Ser Arg Trp Gln Gln Gly Asn
               660                     665                     670
   Val Phe Ser Cys Ser Val Met His Glu Ala Leu His Asn His Tyr Thr
           675                     680                     685
   Gln Lys Ser Leu Ser Leu Ser Pro Gly Lys
       690                     695
<210> 153
   <211> 2097
   <212> DNA
   <213> Artificial Sequence
<220>
   <223> Exons 1-6 and 8-16 of IL17RC with Ser215Thr and Ser228Thr substitutions
<400> 153
   atggatgcaa tgaagagagg gctctgctgt gtgctgctgc tgtgtggcgc cgtcttcgtt 60
   tcgctcagcc aggaaatcca tgccgagttg agacgcttcc gtagactgga gaggcttgtg 120
   gggcctcagg acgctaccca ctgctctccg ggcctctcct gccgcctctg ggacagtgac 180
   atactctgcc tgcctgggga catcgtgcct gctccgggcc ccgtgctggc gcctacgcac 240
   ctgcagacag agctggtgct gaggtgccag aaggagaccg actgtgacct ctgtctgcgt 300
   gtggctgtcc acttggccgt gcatgggcac tgggaagagc ctgaagatga ggaaaagttt 360
   ggaggagcag ctgactcagg ggtggaggag cctaggaatg cctctctcca ggcccaagtc 420
   gtgctctcct tccaggccta ccctactgcc cgctgcgtcc tgctggaggt gcaagtgcct 480
   gctgcccttg tgcagtttgg tcagtctgtg ggctctgtgg tatatgactg cttcgaggct 540
   gccctaggga gtgaggtacg aatctggtcc tatactcagc ccaggtacga gaaggaactc 600
   aaccacacac agcagctgcc tgccctgccc tggctcaacg tgacagcaga tggtgacaac 660
   gtgcatctgg ttctgaatgt cacagaggag cagcacttcg gcctctccct gtactggaat 720
   caggtccagg gccccccaaa accccggtgg cacaaaaacc tgactggacc gcagatcatt 780
   accttgaacc acacagacct ggttccctgc ctctgtattc aggtgtggcc tctggaacct 840
   gactccgtta ggacgaacat ctgccccttc agggaggacc cccgcgcaca ccagaacctc 900
   tggcaagccg cccgactgcg actgctgacc ctgcagagct ggctgctgga cgcaccgtgc 960
   tcgctgcccg cagaagcggc actgtgctgg cgggctccgg gtggggaccc ctgccagcca 1020
   ctggtcccac cgctttcctg ggagaacgtc actgtggaca aggttctcga gttcccattg 1080
   ctgaaaggcc accctaacct ctgtgttcag gtgaacagct cggagaagct gcagctgcag 1140
   gagtgcttgt gggctgactc cctggggcct ctcaaagacg atgtgctact gttggagaca 1200
   cgaggccccc aggacaacag atccctctgt gccttggaac ccagtggctg tacttcacta 1260
   cccagcaaag cctccacgag ggcagctcgc cttggagagt acttactaca agacctgcag 1320
   tcaggccagt gtctgcagct atgggacgat gacttgggag cgctatgggc ctgccccatg 1380
   gacaaataca tccacaagga gcccaaatct tcagacaaaa ctcacacatg cccaccgtgc 1440
   ccagcacctg aagccgaggg ggcaccgtca gtcttcctct tccccccaaa acccaaggac 1500
   accctcatga tctcccggac ccctgaggtc acatgcgtgg tggtggacgt gagccacgaa 1560
   gaccctgagg tcaagttcaa ctggtacgtg gacggcgtgg aggtgcataa tgccaagaca 1620
   aagccgcggg aggagcagta caacagcacg taccgtgtgg tcagcgtcct caccgtcctg 1680
   caccaggact ggctgaatgg caaggagtac aagtgcaagg tctccaacaa agccctccca 1740
   tcctccatcg agaaaaccat ctccaaagcc aaagggcagc cccgagaacc acaggtgtac 1800
   accctgcccc catcccggga tgagctgacc aagaaccagg tcagcctgac ctgcctggtc 1860
   aaaggcttct atcccagcga catcgccgtg gagtgggaga gcaatgggca gccggagaac 1920
   aactacaaga ccacgcctcc cgtgctggac tccgacggct ccttcttcct ctacagcaag 1980
   ctcaccgtgg acaagagcag gtggcagcag gggaacgtct tctcatgctc cgtgatgcat 2040
   gaggctctgc acaaccacta cacgcagaag agcctctccc tgtctccggg taaataa    2097
<210> 154
   <211> 698
   <212> PRT
   <213> Artificial Sequence
<220>
   <223> Exons 1-6 and 6-16 of IL17RC with Ser215Thr and Ser228Thr substitutions and Fc5
<400> 154
   Met Asp Ala Met Lys Arg Gly Leu Cys Cys Val Leu Leu Leu Cys Gly
    1               5                   10                  15
   Ala Val Phe Val Ser Leu Ser Gln Glu Ile His Ala Glu Leu Arg Arg
               20                  25                  30
   Phe Arg Arg Leu Glu Arg Leu Val Gly Pro Gln Asp Ala Thr His Cys
           35                  40                  45
   Ser Pro Gly Leu Ser Cys Arg Leu Trp Asp Ser Asp Ile Leu Cys Leu
       50                  55                  60
   Pro Gly Asp Ile Val Pro Ala Pro Gly Pro Val Leu Ala Pro Thr His
   65                  70                  75                  80
   Leu Gln Thr Glu Leu Val Leu Arg Cys Gln Lys Glu Thr Asp Cys Asp
                   85                  90                  95
   Leu Cys Leu Arg Val Ala Val His Leu Ala Val His Gly His Trp Glu
               100                 105                 110
   Glu Pro Glu Asp Glu Glu Lys Phe Gly Gly Ala Ala Asp Ser Gly Val
           115                 120                 125
   Glu Glu Pro Arg Asn Ala Ser Leu Gln Ala Gln Val Val Leu Ser Phe
       130                 135                 140
   Gln Ala Tyr Pro Thr Ala Arg Cys Val Leu Leu Glu Val Gln Val Pro
   145                 150                 155                 160
   Ala Ala Leu Val Gln Phe Gly Gln Ser Val Gly Ser Val Val Tyr Asp
                   165                 170                 175
   Cys Phe Glu Ala Ala Leu Gly Ser Glu Val Arg Ile Trp Ser Tyr Thr
               180                 185                 190
   Gln Pro Arg Tyr Glu Lys Glu Leu Asn His Thr Gln Gln Leu Pro Ala
           195                 200                 205
   Leu Pro Trp Leu Asn Val Thr Ala Asp Gly Asp Asn Val His Leu Val
       210                 215                 220
   Leu Asn Val Thr Glu Glu Gln His Phe Gly Leu Ser Leu Tyr Trp Asn
   225                 230                 235                 240
   Gln Val Gln Gly Pro Pro Lys Pro Arg Trp His Lys Asn Leu Thr Gly
                   245                 250                 255
   Pro Gln Ile Ile Thr Leu Asn His Thr Asp Leu Val Pro Cys Leu Cys
               260                 265                 270
   Ile Gln Val Trp Pro Leu Glu Pro Asp Ser Val Arg Thr Asn Ile Cys
           275                 280                 285
   Pro Phe Arg Glu Asp Pro Arg Ala His Gln Asn Leu Trp Gln Ala Ala
       290                 295                 300
   Arg Leu Arg Leu Leu Thr Leu Gln Ser Trp Leu Leu Asp Ala Pro Cys
   305                 310                 315                 320
   Ser Leu Pro Ala Glu Ala Ala Leu Cys Trp Arg Ala Pro Gly Gly Asp
                   325                 330                 335
   Pro Cys Gln Pro Leu Val Pro Pro Leu Ser Trp Glu Asn Val Thr Val
               340                 345                 350
   Asp Lys Val Leu Glu Phe Pro Leu Leu Lys Gly His Pro Asn Leu Cys
           355                 360                 365
   Val Gln Val Asn Ser Ser Glu Lys Leu Gln Leu Gln Glu Cys Leu Trp
       370                 375                 380
   Ala Asp Ser Leu Gly Pro Leu Lys Asp Asp Val Leu Leu Leu Glu Thr
   385                 390                 395                 400
   Arg Gly Pro Gln Asp Asn Arg Ser Leu Cys Ala Leu Glu Pro Ser Gly
                   405                 410                 415
   Cys Thr Ser Leu Pro Ser Lys Ala Ser Thr Arg Ala Ala Arg Leu Gly
               420                 425                 430
   Glu Tyr Leu Leu Gln Asp Leu Gln Ser Gly Gln Cys Leu Gln Leu Trp
           435                 440                 445
   Asp Asp Asp Leu Gly Ala Leu Trp Ala Cys Pro Met Asp Lys Tyr Ile
       450                 455                 460
   His Lys Glu Pro Lys Ser Ser Asp Lys Thr His Thr Cys Pro Pro Cys
   465                 470                 475                 480
   Pro Ala Pro Glu Ala Glu Gly Ala Pro Ser Val Phe Leu Phe Pro Pro
                   485                 490                 495
   Lys Pro Lys Asp Thr Leu Met Ile Ser Arg Thr Pro Glu Val Thr Cys
               500                 505                 510
   Val Val Val Asp Val Ser His Glu Asp Pro Glu Val Lys Phe Asn Trp
           515                 520                 525
   Tyr Val Asp Gly Val Glu Val His Asn Ala Lys Thr Lys Pro Arg Glu
       530                 535                 540
   Glu Gln Tyr Asn Ser Thr Tyr Arg Val Val Ser Val Leu Thr Val Leu
   545                 550                 555                 560
   His Gln Asp Trp Leu Asn Gly Lys Glu Tyr Lys Cys Lys Val Ser Asn
                   565                 570                 575
   Lys Ala Leu Pro Ser Ser Ile Glu Lys Thr Ile Ser Lys Ala Lys Gly
                   580                 585                 590
   Gln Pro Arg Glu Pro Gln Val Tyr Thr Leu Pro Pro Ser Arg Asp Glu
               595                 600                 605
   Leu Thr Lys Asn Gln Val Ser Leu Thr Cys Leu Val Lys Gly Phe Tyr
       610                 615                 620
   Pro Ser Asp Ile Ala Val Glu Trp Glu Ser Asn Gly Gln Pro Glu Asn
   625                 630                 635                 640
   Asn Tyr Lys Thr Thr Pro Pro Val Leu Asp Ser Asp Gly Ser Phe Phe
                   645                 650                 655
   Leu Tyr Ser Lys Leu Thr Val Asp Lys Ser Arg Trp Gln Gln Gly Asn
               660                 665                 670
   Val Phe Ser Cys Ser Val Met His Glu Ala Leu His Asn His Tyr Thr
           675                 680                 685
   Gln Lys Ser Leu Ser Leu Ser Pro Gly Lys
       690                 695
<210> 155
   <211> 2094
   <212> DNA
   <213> Artificial Sequence
<220>
   <223> Exons 1-6 and 8-16 of IL17RC with Ser to Thr substitutions and at residues 120, 215, 228, 374, and 408 and Fc5
<400> 155
   atggatgcaa tgaagagagg gctctgctgt gtgctgctgc tgtgtggcgc cgtcttcgtt 60
   tcgctcagcc aggaaatcca tgccgagttg agacgcttcc gtagactgga gaggcttgtg 120
   gggcctcagg acgctaccca ctgctctccg ggcctctcct gccgcctctg ggacagtgac 180
   atactctgcc tgcctgggga catcgtgcct gctccgggcc ccgtgctggc gcctacgcac 240
   ctgcagacag agctggtgct gaggtgccag aaggagaccg actgtgacct ctgtctgcgt 300
   gtggctgtcc acttggccgt gcatgggcac tgggaagagc ctgaagatga ggaaaagttt 360
   ggaggagcag ctgactcagg ggtggaggag cctaggaatg ccacactcca ggcccaagtc 420
   gtgctctcct tccaggccta ccctactgcc cgctgcgtcc tgctggaggt gcaagtgcct 480
   gctgcccttg tgcagtttgg tcagtctgtg ggctctgtgg tatatgactg cttcgaggct 540
   gccctaggga gtgaggtacg aatctggtcc tatactcagc ccaggtacga gaaggaactc 600
   aaccacacac agcagctgcc tgccctgccc tggctcaacg tgacagcaga tggtgacaac 660
   gtgcatctgg ttctgaatgt cacagaggag cagcacttcg gcctctccct gtactggaat 720
   caggtccagg gccccccaaa accccggtgg cacaaaaacc tgactggacc gcagatcatt 780
   accttgaacc acacagacct ggttccctgc ctctgtattc aggtgtggcc tctggaacct 840
   gactccgtta ggacgaacat ctgccccttc agggaggacc cccgcgcaca ccagaacctc 900
   tggcaagccg cccgactgcg actgctgacc ctgcagagct ggctgctgga cgcaccgtgc 960
   tcgctgcccg cagaagcggc actgtgctgg cgggctccgg gtggggaccc ctgccagcca 1020
   ctggtcccac cgctttcctg ggagaacgtc actgtggaca aggttctcga gttcccattg 1080
   ctgaaaggcc accctaacct ctgtgttcag gtgaacagca cagagaagct gcagctgcag 1140
   gagtgcttgt gggctgactc cctggggcct ctcaaagacg atgtgctact gttggagaca 1200
   cgaggccccc aggacaacag aacactctgt gccttggaac ccagtggctg tacttcacta 1260
   cccagcaaag cctccacgag ggcagctcgc cttggagagt acttactaca agacctgcag 1320
   tcaggccagt gtctgcagct atgggacgat gacttgggag cgctatgggc ctgccccatg 1380
   gacaaataca tccacaagga gcccaaatct tcagacaaaa ctcacacatg cccaccgtgc 1440
   ccagcacctg aagccgaggg ggcaccgtca gtcttcctct tccccccaaa acccaaggac 1500
   accctcatga tctcccggac ccctgaggtc acatgcgtgg tggtggacgt gagccacgaa 1560
   gaccctgagg tcaagttcaa ctggtacgtg gacggcgtgg aggtgcataa tgccaagaca 1620
   aagccgcggg aggagcagta caacagcacg taccgtgtgg tcagcgtcct caccgtcctg 1680
   caccaggact ggctgaatgg caaggagtac aagtgcaagg tctccaacaa agccctccca 1740
   tcctccatcg agaaaaccat ctccaaagcc aaagggcagc cccgagaacc acaggtgtac 1800
   accctgcccc catcccggga tgagctgacc aagaaccagg tcagcctgac ctgcctggtc 1860
   aaaggcttct atcccagcga catcgccgtg gagtgggaga gcaatgggca gccggagaac 1920
   aactacaaga ccacgcctcc cgtgctggac tccgacggct ccttcttcct ctacagcaag 1980
   ctcaccgtgg acaagagcag gtggcagcag gggaacgtct tctcatgctc cgtgatgcat 2040
   gaggctctgc acaaccacta cacgcagaag agcctctccc tgtctccggg taaa 2094
<210> 156
   <211> 698
   <212> PRT
   <213> Artificial Sequence
<220>
   <223> Exons 1-6 and 8-16 of IL17RC with Ser to Thr substitutions at residues 120, 215, 228, 374, and 408 and Fc5
<400> 156
   Met Asp Ala Met Lys Arg Gly Leu Cys Cys Val Leu Leu Leu Cys Gly
    1               5                  10                  15
   Ala Val Phe Val Ser Leu Ser Gln Glu Ile His Ala Glu Leu Arg Arg
               20                  25                  30
   Phe Arg Arg Leu Glu Arg Leu Val Gly Pro Gln Asp Ala Thr His Cys
           35                  40                  45
   Ser Pro Gly Leu Ser Cys Arg Leu Trp Asp Ser Asp Ile Leu Cys Leu
       50                  55                  60
   Pro Gly Asp Ile Val Pro Ala Pro Gly Pro Val Leu Ala Pro Thr His
   65                  70                  75                  80
   Leu Gln Thr Glu Leu Val Leu Arg Cys Gln Lys Glu Thr Asp Cys Asp
                   85                  90                  95
   Leu Cys Leu Arg Val Ala Val His Leu Ala Val His Gly His Trp Glu
               100                 105                 110
   Glu Pro Glu Asp Glu Glu Lys Phe Gly Gly Ala Ala Asp Ser Gly Val
           115                 120                 125
   Glu Glu Pro Arg Asn Ala Thr Leu Gln Ala Gln Val Val Leu Ser Phe
       130                 135                 140
   Gln Ala Tyr Pro Thr Ala Arg Cys Val Leu Leu Glu Val Gln Val Pro
   145                 150                 155                 160
   Ala Ala Leu Val Gln Phe Gly Gln Ser Val Gly Ser Val Val Tyr Asp
                   165                 170                 175
   Cys Phe Glu Ala Ala Leu Gly Ser Glu Val Arg Ile Trp Ser Tyr Thr
               180                 185                 190
   Gln Pro Arg Tyr Glu Lys Glu Leu Asn His Thr Gln Gln Leu Pro Ala
           195                 200                 205
   Leu Pro Trp Leu Asn Val Thr Ala Asp Gly Asp Asn Val His Leu Val
       210                 215                 220
   Leu Asn Val Thr Glu Glu Gln His Phe Gly Leu Ser Leu Tyr Trp Asn
   225                 230                 235                 240
   Gln Val Gln Gly Pro Pro Lys Pro Arg Trp His Lys Asn Leu Thr Gly
                   245                 250                 255
   Pro Gln Ile Ile Thr Leu Asn His Thr Asp Leu Val Pro Cys Leu Cys
               260                 265                 270
   Ile Gln Val Trp Pro Leu Glu Pro Asp Ser Val Arg Thr Asn Ile Cys
           275                 280                 285
   Pro Phe Arg Glu Asp Pro Arg Ala His Gln Asn Leu Trp Gln Ala Ala
       290                 295                 300
   Arg Leu Arg Leu Leu Thr Leu Gln Ser Trp Leu Leu Asp Ala Pro Cys
   305                 310                 315                 320
   Ser Leu Pro Ala Glu Ala Ala Leu Cys Trp Arg Ala Pro Gly Gly Asp
                   325                 330                 335
   Pro Cys Gln Pro Leu Val Pro Pro Leu Ser Trp Glu Asn Val Thr Val
               340                 345                 350
   Asp Lys Val Leu Glu Phe Pro Leu Leu Lys Gly His Pro Asn Leu Cys
           355                 360                 365
   Val Gln Val Asn Ser Thr Glu Lys Leu Gln Leu Gln Glu Cys Leu Trp
       370                 375                 380
   Ala Asp Ser Leu Gly Pro Leu Lys Asp Asp Val Leu Leu Leu Glu Thr
   385                 390                 395                 400
   Arg Gly Pro Gln Asp Asn Arg Thr Leu Cys Ala Leu Glu Pro Ser Gly
                   405                 410                 415
   Cys Thr Ser Leu Pro Ser Lys Ala Ser Thr Arg Ala Ala Arg Leu Gly
               420                 425                 430
   Glu Tyr Leu Leu Gln Asp Leu Gln Ser Gly Gln Cys Leu Gln Leu Trp
           435                 440                 445
   Asp Asp Asp Leu Gly Ala Leu Trp Ala Cys Pro Met Asp Lys Tyr Ile
       450                 455                 460
   His Lys Glu Pro Lys Ser Ser Asp Lys Thr His Thr Cys Pro Pro Cys
   465                 470                 475                 480
   Pro Ala Pro Glu Ala Glu Gly Ala Pro Ser Val Phe Leu Phe Pro Pro
                   485                 490                 495
   Lys Pro Lys Asp Thr Leu Met Ile Ser Arg Thr Pro Glu Val Thr Cys
               500                 505                 510
   Val Val Val Asp Val Ser His Glu Asp Pro Glu Val Lys Phe Asn Trp
           515                 520                 525
   Tyr Val Asp Gly Val Glu Val His Asn Ala Lys Thr Lys Pro Arg Glu
       530                 535                 540
   Glu Gln Tyr Asn Ser Thr Tyr Arg Val Val Ser Val Leu Thr Val Leu
   545                 550                 555                 560
   His Gln Asp Trp Leu Asn Gly Lys Glu Tyr Lys Cys Lys Val Ser Asn
                   565                 570                 575
   Lys Ala Leu Pro Ser Ser Ile Glu Lys Thr Ile Ser Lys Ala Lys Gly
               580                 585                 590
   Gln Pro Arg Glu Pro Gln Val Tyr Thr Leu Pro Pro Ser Arg Asp Glu
           595                 600                 605
   Leu Thr Lys Asn Gln Val Ser Leu Thr Cys Leu Val Lys Gly Phe Tyr
       610                 615                 620
   Pro Ser Asp Ile Ala Val Glu Trp Glu Ser Asn Gly Gln Pro Glu Asn
   625                 630                 635                 640
   Asn Tyr Lys Thr Thr Pro Pro Val Leu Asp Ser Asp Gly Ser Phe Phe
                   645                 650                 655
   Leu Tyr Ser Lys Leu Thr Val Asp Lys Ser Arg Trp Gln Gln Gly Asn
               660                 665                 670
   Val Phe Ser Cys Ser Val Met His Glu Ala Leu His Asn His Tyr Thr
           675                 680                 685
   Gln Lys Ser Leu Ser Leu Ser Pro Gly Lys
       690                 695
<210> 157
   <211> 2070
   <212> DNA
   <213> Artificial Sequence
<220>
   <223> IL-17RA signal peptide and exons 1-6 of IL-17RA and exons 8-16 of IL-17RC and Fc5
<400> 157
   atgggggccg cacgcagccc gccgtccgct gtcccggggc ccctgctggg gctgctcctg 60
   ctgctcctgg gcgtgctggc cccgggtggc gcctccctgc gactcctgga ccaccgggcg 120
   ctggtctgct cccagccggg gctaaactgc acggtcaaga atagtacctg cctggatgac 180
   agctggattc accctcgaaa cctgaccccc tcctccccaa aggacctgca gatccagctg 240
   cactttgccc acacccaaca aggagacctg ttccccgtgg ctcacatcga atggacactg 300
   cagacagacg ccagcatcct gtacctcgag ggtgcagagt tatctgtcct gcagctgaac 360
   accaatgaac gtttgtgcgt caggtttgag tttctgtcca aactgaggca tcaccacagg 420
   cggtggcgtt ttaccttcag ccactttgtg gttgaccctg accaggaata tgaggtgacc 480
   gttcaccacc tgcccaagcc catccctgat ggggacccaa accaccagtc caagaatttc 540
   cttgtgcctg actgtgagca cgccaggatg aaggtaacca cgccatgcat gagctcagcc 600
   ctgccctggc tcaacgtgtc agcagatggt gacaacgtgc atctggttct gaatgtctct 660
   gaggagcagc acttcggcct ctccctgtac tggaatcagg tccagggccc cccaaaaccc 720
   cggtggcaca aaaacctgac tggaccgcag atcattacct tgaaccacac agacctggtt 780
   ccctgcctct gtattcaggt gtggcctctg gaacctgact ccgttaggac gaacatctgc 840
   cccttcaggg aggacccccg cgcacaccag aacctctggc aagccgcccg actgcgactg 900
   ctgaccctgc agagctggct gctggacgca ccgtgctcgc tgcccgcaga agcggcactg 960
   tgctggcggg ctccgggtgg ggacccctgc cagccactgg tcccaccgct ttcctgggag 1020
   aacgtcactg tggacaaggt tctcgagttc ccattgctga aaggccaccc taacctctgt 1080
   gttcaggtga acagctcgga gaagctgcag ctgcaggagt gcttgtgggc tgactccctg 1140
   gggcctctca aagacgatgt gctactgttg gagacacgag gcccccagga caacagatcc 1200
   ctctgtgcct tggaacccag tggctgtact tcactaccca gcaaagcctc cacgagggca 1260
   gctcgccttg gagagtactt actacaagac ctgcagtcag gccagtgtct gcagctatgg 1320
   gacgatgact tgggagcgct atgggcctgc cccatggaca aatacatcca caaggagccc 1380
   aaatcttcag acaaaactca cacatgccca ccgtgcccag cacctgaagc cgagggggca 1440
   ccgtcagtct tcctcttccc cccaaaaccc aaggacaccc tcatgatctc ccggacccct 1500
   gaggtcacat gcgtggtggt ggacgtgagc cacgaagacc ctgaggtcaa gttcaactgg 1560
   tacgtggacg gcgtggaggt gcataatgcc aagacaaagc cgcgggagga gcagtacaac 1620
   agcacgtacc gtgtggtcag cgtcctcacc gtcctgcacc aggactggct gaatggcaag 1680
   gagtacaagt gcaaggtctc caacaaagcc ctcccatcct ccatcgagaa aaccatctcc 1740
   aaagccaaag ggcagccccg agaaccacag gtgtacaccc tgcccccatc ccgggatgag 1800
   ctgaccaaga accaggtcag cctgacctgc ctggtcaaag gcttctatcc cagcgacatc 1860
   gccgtggagt gggagagcaa tgggcagccg gagaacaact acaagaccac gcctcccgtg 1920
   ctggactccg acggctcctt cttcctctac agcaagctca ccgtggacaa gagcaggtgg 1980
   cagcagggga acgtcttctc atgctccgtg atgcatgagg ctctgcacaa ccactacacg 2040
   cagaagagcc tctccctgtc tccgggtaaa 2070
<210> 158
   <211> 690
   <212> PRT
   <213> Artificial Sequence
<220>
   <223> IL-17RA signal peptide and exons 1-6 of IL-17RA and exons 8-16 of IL-17RC and Fc5
<400> 158
   Met Gly Ala Ala Arg Ser Pro Pro Ser Ala Val Pro Gly Pro Leu Leu
    1               5                  10                  15
   Gly Leu Leu Leu Leu Leu Leu Gly Val Leu Ala Pro Gly Gly Ala Ser
               20                  25                  30
   Leu Arg Leu Leu Asp His Arg Ala Leu Val Cys Ser Gln Pro Gly Leu
           35                  40                  45
   Asn Cys Thr Val Lys Asn Ser Thr Cys Leu Asp Asp Ser Trp Ile His
       50                  55                  60
   Pro Arg Asn Leu Thr Pro Ser Ser Pro Lys Asp Leu Gln Ile Gln Leu
   65                  70                  75                  80
   His Phe Ala His Thr Gln Gln Gly Asp Leu Phe Pro Val Ala His Ile
                   85                  90                  95
   Glu Trp Thr Leu Gln Thr Asp Ala Ser Ile Leu Tyr Leu Glu Gly Ala
               100                 105                 110
   Glu Leu Ser Val Leu Gln Leu Asn Thr Asn Glu Arg Leu Cys Val Arg
                       115                 120                 125
   Phe Glu Phe Leu Ser Lys Leu Arg His His His Arg Arg Trp Arg Phe
       130                 135                 140
   Thr Phe Ser His Phe Val Val Asp Pro Asp Gln Glu Tyr Glu Val Thr
   145                 150                 155                 160
   Val His His Leu Pro Lys Pro Ile Pro Asp Gly Asp Pro Asn His Gln
                   165                 170                 175
   Ser Lys Asn Phe Leu Val Pro Asp Cys Glu His Ala Arg Met Lys Val
               180                 185                 190
   Thr Thr Pro Cys Met Ser Ser Ala Leu Pro Trp Leu Asn Val Ser Ala
           195                 200                 205
   Asp Gly Asp Asn Val His Leu Val Leu Asn Val Ser Glu Glu Gln His
       210                 215                 220
   Phe Gly Leu Ser Leu Tyr Trp Asn Gln Val Gln Gly Pro Pro Lys Pro
   225                 230                 235                 240
   Arg Trp His Lys Asn Leu Thr Gly Pro Gln Ile Ile Thr Leu Asn His
                   245                 250                 255
   Thr Asp Leu Val Pro Cys Leu Cys Ile Gln Val Trp Pro Leu Glu Pro
               260                 265                 270
   Asp Ser Val Arg Thr Asn Ile Cys Pro Phe Arg Glu Asp Pro Arg Ala
           275                 280                 285
   His Gln Asn Leu Trp Gln Ala Ala Arg Leu Arg Leu Leu Thr Leu Gln
       290                 295                 300
   Ser Trp Leu Leu Asp Ala Pro Cys Ser Leu Pro Ala Glu Ala Ala Leu
   305                 310                 315                 320
   Cys Trp Arg Ala Pro Gly Gly Asp Pro Cys Gln Pro Leu Val Pro Pro
                   325                 330                 335
   Leu Ser Trp Glu Asn Val Thr Val Asp Lys Val Leu Glu Phe Pro Leu
               340                 345                 350
   Leu Lys Gly His Pro Asn Leu Cys Val Gln Val Asn Ser Ser Glu Lys
           355                 360                 365
   Leu Gln Leu Gln Glu Cys Leu Trp Ala Asp Ser Leu Gly Pro Leu Lys
       370                 375                 380
   Asp Asp Val Leu Leu Leu Glu Thr Arg Gly Pro Gln Asp Asn Arg Ser
   385                 390                 395                 400
   Leu Cys Ala Leu Glu Pro Ser Gly Cys Thr Ser Leu Pro Ser Lys Ala
                   405                 410                 415
   Ser Thr Arg Ala Ala Arg Leu Gly Glu Tyr Leu Leu Gln Asp Leu Gln
               420                 425                 430
   Ser Gly Gln Cys Leu Gln Leu Trp Asp Asp Asp Leu Gly Ala Leu Trp
           435                 440                 445
   Ala Cys Pro Met Asp Lys Tyr Ile His Lys Glu Pro Lys Ser Ser Asp
       450                 455                 460
   Lys Thr His Thr Cys Pro Pro Cys Pro Ala Pro Glu Ala Glu Gly Ala
   465                 470                 475                 480
   Pro Ser Val Phe Leu Phe Pro Pro Lys Pro Lys Asp Thr Leu Met Ile
                   485                 490                 495
   Ser Arg Thr Pro Glu Val Thr Cys Val Val Val Asp Val Ser His Glu
               500                 505                 510
   Asp Pro Glu Val Lys Phe Asn Trp Tyr Val Asp Gly Val Glu Val His
           515                 520                 525
   Asn Ala Lys Thr Lys Pro Arg Glu Glu Gln Tyr Asn Ser Thr Tyr Arg
       530                 535                 540
   Val Val Ser Val Leu Thr Val Leu His Gln Asp Trp Leu Asn Gly Lys
   545                 550                 555                 560
   Glu Tyr Lys Cys Lys Val Ser Asn Lys Ala Leu Pro Ser Ser Ile Glu
                   565                 570                 575
   Lys Thr Ile Ser Lys Ala Lys Gly Gln Pro Arg Glu Pro Gln Val Tyr
               580                 585                 590
   Thr Leu Pro Pro Ser Arg Asp Glu Leu Thr Lys Asn Gln Val Ser Leu
                   595                     600                     605
   Thr Cys Leu Val Lys Gly Phe Tyr Pro Ser Asp Ile Ala Val Glu Trp
       610                     615                     620
   Glu Ser Asn Gly Gln Pro Glu Asn Asn Tyr Lys Thr Thr Pro Pro Val
   625                     630                 635                 640
   Leu Asp Ser Asp Gly Ser Phe Phe Leu Tyr Ser Lys Leu Thr Val Asp
                   645                     650                 655
   Lys Ser Arg Trp Gln Gln Gly Asn Val Phe Ser Cys Ser Val Met His
               660                     665                 670
   Glu Ala Leu His Asn His Tyr Thr Gln Lys Ser Leu Ser Leu Ser Pro
           675                     680                     685
   Gly Lys
       690
<210> 159
   <211> 252
   <212> DNA
   <213> Artificial Sequence
<220>
   <223> Domain 1
<400> 159
   gccctgccct ggctcaacgt gtcagcagat ggtgacaacg tgcatctggt tctgaatgtc 60
   tctgaggagc agcacttcgg cctctccctg tactggaatc aggtccaggg ccccccaaaa 120
   ccccggtggc acaaaaacct gactggaccg cagatcatta ccttgaacca cacagacctg 180
   gttccctgcc tctgtattca ggtgtggcct ctggaacctg actccgttag gacgaacatc 240
   tgccccttca gg                                                     252
<210> 160
   <211> 84
   <212> PRT
   <213> Artificial Sequence
<220>
   <223> Domain 1
<400> 160
   Ala Leu Pro Trp Leu Asn Val Ser Ala Asp Gly Asp Asn Val His Leu
   1               5                   10                  15
   Val Leu Asn Val Ser Glu Glu Gln His Phe Gly Leu Ser Leu Tyr Trp
               20                  25                  30
   Asn Gln Val Gln Gly Pro Pro Lys Pro Arg Trp His Lys Asn Leu Thr
           35                  40                  45
   Gly Pro Gln Ile Ile Thr Leu Asn His Thr Asp Leu Val Pro Cys Leu
       50                  55                  60
   Cys Ile Gln Val Trp Pro Leu Glu Pro Asp Ser Val Arg Thr Asn Ile
   65                  70                  75                  80
   Cys Pro Phe Arg
<210> 161
   <211> 282
   <212> DNA
   <213> Artificial Sequence
<220>
   <223> Domain 2
<400> 161
   gaggaccccc gcgcacacca gaacctctgg caagccgccc gactgcgact gctgaccctg 60
   cagagctggc tgctggacgc accgtgctcg ctgcccgcag aagcggcact gtgctggcgg 120
   gctccgggtg gggacccctg ccagccactg gtcccaccgc tttcctggga gaacgtcact 180
   gtggacaagg ttctcgagtt cccattgctg aaaggccacc ctaacctctg tgttcaggtg 240
   aacagctcgg agaagctgca gctgcaggag tgcttgtggg ct                    282
<210> 162
   <211> 94
   <212> PRT
   <213> Artificial Sequence
<220>
   <223> Domain 2
<400> 162
   Glu Asp Pro Arg Ala His Gln Asn Leu Trp Gln Ala Ala Arg Leu Arg
    1               5                  10                  15
   Leu Leu Thr Leu Gln Ser Trp Leu Leu Asp Ala Pro Cys Ser Leu Pro
               20                  25                  30
   Ala Glu Ala Ala Leu Cys Trp Arg Ala Pro Gly Gly Asp Pro Cys Gln
           35                  40                  45
   Pro Leu Val Pro Pro Leu Ser Trp Glu Asn Val Thr Val Asp Lys Val
       50                  55                  60
   Leu Glu Phe Pro Leu Leu Lys Gly His Pro Asn Leu Cys Val Gln Val
   65                  70                  75                  80
   Asn Ser Ser Glu Lys Leu Gln Leu Gln Glu Cys Leu Trp Ala
                   85                  90
<210> 163
   <211> 231
   <212> DNA
   <213> Artificial Sequence
<220>
   <223> Domain 3
<400> 163
   gactccctgg ggcctctcaa agacgatgtg ctactgttgg agacacgagg cccccaggac 60
   aacagatccc tctgtgcctt ggaacccagt ggctgtactt cactacccag caaagcctcc 120
   acgagggcag ctcgccttgg agagtactta ctacaagacc tgcagtcagg ccagtgtctg 180
   cagctatggg acgatgactt gggagcgcta tgggcctgcc ccatggacaa a          231
<210> 164
   <211> 77
   <212> PRT
   <213> Artificial Sequence
<220>
   <223> Domain 3
<400> 164
   Asp Ser Leu Gly Pro Leu Lys Asp Asp Val Leu Leu Leu Glu Thr Arg
   1               5                   10                  15
   Gly Pro Gln Asp Asn Arg Ser Leu Cys Ala Leu Glu Pro Ser Gly Cys
               20                  25                  30
   Thr Ser Leu Pro Ser Lys Ala Ser Thr Arg Ala Ala Arg Leu Gly Glu
           35                  40                  45
   Tyr Leu Leu Gln Asp Leu Gln Ser Gly Gln Cys Leu Gln Leu Trp Asp
       50                  55                  60
   Asp Asp Leu Gly Ala Leu Trp Ala Cys Pro Met Asp Lys
   65                  70                  75
<210> 165
   <211> 2124
   <212> DNA
   <213> homo sapians
<400> 165
   atgcctgtgc cctggttctt gctgtccttg gcactgggcc gaagcccagt ggtcctttct 60
   ctggagaggc ttgtggggcc tcaggacgct acccactgct ctccgggcct ctcctgccgc 120
   ctctgggaca gtgacatact ctgcctgcct ggggacatcg tgcctgctcc gggccccgtg 180
   ctggcgccta cgcacctgca gacagagctg gtgctgaggt gccagaagga gaccgactgt 240
   gacctctgtc tgcgtgtggc tgtccacttg gccgtgcatg ggcactggga agagcctgaa 300
   gatgaggaaa agtttggagg agcagctgac tcaggggtgg aggagcctag gaatgcctct 360
   ctccaggccc aagtcgtgct ctccttccag gcctacccta ctgcccgctg cgtcctgctg 420
   gaggtgcaag tgcctgctgc ccttgtgcag tttggtcagt ctgtgggctc tgtggtatat 480
   gactgcttcg aggctgccct agggagtgag gtacgaatct ggtcctatac tcagcccagg 540
   tacgagaagg aactcaacca cacacagcag ctgcctgact gcagggggct cgaagtctgg 600
   aacagcatcc cgagctgctg ggccctgccc tggctcaacg tgtcagcaga tggtgacaac 660
   gtgcatctgg ttctgaatgt ctctgaggag cagcacttcg gcctctccct gtactggaat 720
   caggtccagg gccccccaaa accccggtgg cacaaaaacc tgactggacc gcagatcatt 780
   accttgaacc acacagacct ggttccctgc ctctgtattc aggtgtggcc tctggaacct 840
   gactccgtta ggacgaacat ctgccccttc agggaggacc cccgcgcaca ccagaacctc 900
   tggcaagccg cccgactgcg actgctgacc ctgcagagct ggctgctgga cgcaccgtgc 960
   tcgctgcccg cagaagcggc actgtgctgg cgggctccgg gtggggaccc ctgccagcca 1020
   ctggtcccac cgctttcctg ggagaacgtc actgtggaca aggttctcga gttcccattg 1080
   ctgaaaggcc accctaacct ctgtgttcag gtgaacagct cggagaagct gcagctgcag 1140
   gagtgcttgt gggctgactc cctggggcct ctcaaagacg atgtgctact gttggagaca 1200
   cgaggccccc aggacaacag atccctctgt gccttggaac ccagtggctg tacttcacta 1260
   cccagcaaag cctccacgag ggcagctcgc cttggagagt acttactaca agacctgcag 1320
   tcaggccagt gtctgcagct atgggacgat gacttgggag cgctatgggc ctgccccatg 1380
   gacaaataca tccacaagcg ctgggccctc gtgtggctgg cctgcctact ctttgccgct 1440
   gcgctttccc tcatcctcct tctcaaaaag gatcacgcga aagcggccgc caggggccgc 1500
   gcggctctgc tcctctactc agccgatgac tcgggtttcg agcgcctggt gggcgccctg 1560
   gcgtcggccc tgtgccagct gccgctgcgc gtggccgtag acctgtggag ccgtcgtgaa 1620
   ctgagcgcgc aggggcccgt ggcttggttt cacgcgcagc ggcgccagac cctgcaggag 1680
   ggcggcgtgg tggtcttgct cttctctccc ggtgcggtgg cgctgtgcag cgagtggcta 1740
   caggatgggg tgtccgggcc cggggcgcac ggcccgcacg acgccttccg cgcctcgctc 1800
   agctgcgtgc tgcccgactt cttgcagggc cgggcgcccg gcagctacgt gggggcctgc 1860
   ttcgacaggc tgctccaccc ggacgccgta cccgcccttt tccgcaccgt gcccgtcttc 1920
   acactgccct cccaactgcc agacttcctg gggcccctgc agcagcctcg cgccccgcgt 1980
   tccgggcggc tccaagagag agcggagcaa gtgtcccggg cccttcagcc agccctggat 2040
   agctacttcc atcccccggg gactcccgcg ccgggacgcg gggtgggacc aggggcggga 2100
   cctggggcgg gggacgggac ttaa                                        2124
<210> 166
   <211> 707
   <212> PRT
   <213> homo sapians
<400> 166
   Met Pro Val Pro Trp Phe Leu Leu Ser Leu Ala Leu Gly Arg Ser Pro
    1               5                  10                  15
   Val Val Leu Ser Leu Glu Arg Leu Val Gly Pro Gln Asp Ala Thr His
               20                  25                  30
   Cys Ser Pro Gly Leu Ser Cys Arg Leu Trp Asp Ser Asp Ile Leu Cys
           35                  40                  45
   Leu Pro Gly Asp Ile Val Pro Ala Pro Gly Pro Val Leu Ala Pro Thr
       50                  55                  60
   His Leu Gln Thr Glu Leu Val Leu Arg Cys Gln Lys Glu Thr Asp Cys
   65                  70                  75                  80
   Asp Leu Cys Leu Arg Val Ala Val His Leu Ala Val His Gly His Trp
                   85                  90                  95
   Glu Glu Pro Glu Asp Glu Glu Lys Phe Gly Gly Ala Ala Asp Ser Gly
               100                 105                 110
   Val Glu Glu Pro Arg Asn Ala Ser Leu Gln Ala Gln Val Val Leu Ser
           115                 120                 125
   Phe Gln Ala Tyr Pro Thr Ala Arg Cys Val Leu Leu Glu Val Gln Val
       130                 135                 140
   Pro Ala Ala Leu Val Gln Phe Gly Gln Ser Val Gly Ser Val Val Tyr
   145                 150                 155                 160
   Asp Cys Phe Glu Ala Ala Leu Gly Ser Glu Val Arg Ile Trp Ser Tyr
                   165                 170                 175
   Thr Gln Pro Arg Tyr Glu Lys Glu Leu Asn His Thr Gln Gln Leu Pro
               180                 185                 190
   Asp Cys Arg Gly Leu Glu Val Trp Asn Ser Ile Pro Ser Cys Trp Ala
           195                 200                 205
   Leu Pro Trp Leu Asn Val Ser Ala Asp Gly Asp Asn Val His Leu Val
       210                 215                 220
   Leu Asn Val Ser Glu Glu Gln His Phe Gly Leu Ser Leu Tyr Trp Asn
   225                 230                 235                 240
   Gln Val Gln Gly Pro Pro Lys Pro Arg Trp His Lys Asn Leu Thr Gly
                   245                 250                 255
   Pro Gln Ile Ile Thr Leu Asn His Thr Asp Leu Val Pro Cys Leu Cys
               260                 265                 270
   Ile Gln Val Trp Pro Leu Glu Pro Asp Ser Val Arg Thr Asn Ile Cys
           275                 280                 285
   Pro Phe Arg Glu Asp Pro Arg Ala His Gln Asn Leu Trp Gln Ala Ala
       290                 295                 300
   Arg Leu Arg Leu Leu Thr Leu Gln Ser Trp Leu Leu Asp Ala Pro Cys
   305                 310                 315                 320
   Ser Leu Pro Ala Glu Ala Ala Leu Cys Trp Arg Ala Pro Gly Gly Asp
                   325                 330                 335
   Pro Cys Gln Pro Leu Val Pro Pro Leu Ser Trp Glu Asn Val Thr Val
               340                 345                 350
   Asp Lys Val Leu Glu Phe Pro Leu Leu Lys Gly His Pro Asn Leu Cys
           355                 360                 365
   Val Gln Val Asn Ser Ser Glu Lys Leu Gln Leu Gln Glu Cys Leu Trp
       370                 375                 380
   Ala Asp Ser Leu Gly Pro Leu Lys Asp Asp Val Leu Leu Leu Glu Thr
   385                 390                 395                 400
   Arg Gly Pro Gln Asp Asn Arg Ser Leu Cys Ala Leu Glu Pro Ser Gly
                   405                 410                 415
   Cys Thr Ser Leu Pro Ser Lys Ala Ser Thr Arg Ala Ala Arg Leu Gly
               420                 425                 430
   Glu Tyr Leu Leu Gln Asp Leu Gln Ser Gly Gln Cys Leu Gln Leu Trp
           435                 440                 445
   Asp Asp Asp Leu Gly Ala Leu Trp Ala Cys Pro Met Asp Lys Tyr Ile
       450                 455                 460
   His Lys Arg Trp Ala Leu Val Trp Leu Ala Cys Leu Leu Phe Ala Ala
   465 470 475 480
   Ala Leu Ser Leu Ile Leu Leu Leu Lys Lys Asp His Ala Lys Ala Ala
   485 490 495
   Ala Arg Gly Arg Ala Ala Leu Leu Leu Tyr Ser Ala Asp Asp Ser Gly
   500 505 510
   Phe Glu Arg Leu Val Gly Ala Leu Ala Ser Ala Leu Cys Gln Leu Pro
   515 520 525
   Leu Arg Val Ala Val Asp Leu Trp Ser Arg Arg Glu Leu Ser Ala Gln
   530 535 540
   Gly Pro Val Ala Trp Phe His Ala Gln Arg Arg Gln Thr Leu Gln Glu
   545 550 555 560
   Gly Gly Val Val Val Leu Leu Phe Ser Pro Gly Ala Val Ala Leu Cys
   565 570 575
   Ser Glu Trp Leu Gln Asp Gly Val Ser Gly Pro Gly Ala His Gly Pro
   580 585 590
   His Asp Ala Phe Arg Ala Ser Leu Ser Cys Val Leu Pro Asp Phe Leu
   595 600 605
   Gln Gly Arg Ala Pro Gly Ser Tyr Val Gly Ala Cys Phe Asp Arg Leu
   610 615 620
   Leu His Pro Asp Ala Val Pro Ala Leu Phe Arg Thr Val Pro Val Phe
   625 630 635 640
   Thr Leu Pro Ser Gln Leu Pro Asp Phe Leu Gly Ala Leu Gln Gln Pro
   645 650 655
   Arg Ala Pro Arg Ser Gly Arg Leu Gln Glu Arg Ala Glu Gln Val Ser
   660 665 670
   Arg Ala Leu Gln Pro Ala Leu Asp Ser Tyr Phe His Pro Pro Gly Thr
   675 680 685
   Pro Ala Pro Gly Arg Gly Val Gly Pro Gly Ala Gly Pro Gly Ala Gly
   690 695 700
   Asp Gly Thr
   705
<210> 167
   <211> 3120
   <212> DNA
   <213> homo sapians
<400> 167
   ggggccgagc cctccgcgac gccacccggg ccatgggggc cgcacgcagc ccgccgtccg 60
   ctgtcccggg gcccctgctg gggctgctcc tgctgctcct gggcgtgctg gccccgggtg 120
   gcgcctccct gcgactcctg gaccaccggg cgctggtctg ctcccagccg gggctaaact 180
   gcacggtcaa gaatagtacc tgcctggatg acagctggat tcaccctcga aacctgaccc 240
   cctcctcccc aaaggacctg cagatccagc tgcactttgc ccacacccaa caaggagacc 300
   tgttccccgt ggctcacatc gaatggacac tgcagacaga cgccagcatc ctgtacctcg 360
   agggtgcaga gttatctgtc ctgcagctga acaccaatga acgtttgtgc gtcaggtttg 420
   agtttctgtc caaactgagg catcaccaca ggcggtggcg ttttaccttc agccactttg 480
   tggttgaccc tgaccaggaa tatgaggtga ccgttcacca cctgcccaag cccatccctg 540
   atggggaccc aaaccaccag tccaagaatt tccttgtgcc tgactgtgag cacgccagga 600
   tgaaggtaac cacgccatgc atgagctcag gcagcctgtg ggaccccaac atcaccgtgg 660
   agaccctgga ggcccaccag ctgcgtgtga gcttcaccct gtggaacgaa tctacccatt 720
   accagatcct gctgaccagt tttccgcaca tggagaacca cagttgcttt gagcacatgc 780
   accacatacc tgcgcccaga ccagaagagt tccaccagcg atccaacgtc acactcactc 840
   tacgcaacct taaagggtgc tgtcgccacc aagtgcagat ccagcccttc ttcagcagct 900
   gcctcaatga ctgcctcaga cactccgcga ctgtttcctg cccagaaatg ccagacactc 960
   cagaaccaat tccggactac atgcccctgt gggtgtactg gttcatcacg ggcatctcca 1020
   tcctgctggt gggctccgtc atcctgctca tcgtctgcat gacctggagg ctagctgggc 1080
   ctggaagtga aaaatacagt gatgacacca aatacaccga tggcctgcct gcggctgacc 1140
   tgatcccccc accgctgaag cccaggaagg tctggatcat ctactcagcc gaccaccccc 1200
   tctacgtgga cgtggtcctg aaattcgccc agttcctgct caccgcctgc ggcacggaag 1260
   tggccctgga cctgctggaa gagcaggcca tctcggaggc aggagtcatg acctgggtgg 1320
   gccgtcagaa gcaggagatg gtggagagca actctaagat catcgtcctg tgctcccgcg 1380
   gcacgcgcgc caagtggcag gcgctcctgg gccggggggc gcctgtgcgg ctgcgctgcg 1440
   accacggaaa gcccgtgggg gacctgttca ctgcagccat gaacatgatc ctcccggact 1500
   tcaagaggcc agcctgcttc ggcacctacg tagtctgcta cttcagcgag gtcagctgtg 1560
   acggcgacgt ccccgacctg ttcggcgcgg cgccgcggta cccgctcatg gacaggttcg 1620
   aggaggtgta cttccgcatc caggacctgg agatgttcca gccgggccgc atgcaccgcg 1680
   taggggagct gtcgggggac aactacctgc ggagcccggg cggcaggcag ctccgcgccg 1740
   ccctggacag gttccgggac tggcaggtcc gctgtcccga ctggttcgaa tgtgagaacc 1800
   tctactcagc agatgaccag gatgccccgt ccctggacga agaggtgttt gaggagccac 1860
   tgctgcctcc gggaaccggc atcgtgaagc gggcgcccct ggtgcgcgag cctggctccc 1920
   aggcctgcct ggccatagac ccgctggtcg gggaggaagg aggagcagca gtggcaaagc 1980
   tggaacctca cctgcagccc cggggtcagc cagcgccgca gcccctccac accctggtgc 2040
   tcgccgcaga ggagggggcc ctggtggccg cggtggagcc tgggcccctg gctgacggtg 2100
   ccgcagtccg gctggcactg gcgggggagg gcgaggcctg cccgctgctg ggcagcccgg 2160
   gcgctgggcg aaatagcgtc ctcttcctcc ccgtggaccc cgaggactcg ccccttggca 2220
   gcagcacccc catggcgtct cctgacctcc ttccagagga cgtgagggag cacctcgaag 2280
   gcttgatgct ctcgctcttc gagcagagtc tgagctgcca ggcccagggg ggctgcagta 2340
   gacccgccat ggtcctcaca gacccacaca cgccctacga ggaggagcag cggcagtcag 2400
   tgcagtctga ccagggctac atctccagga gctccccgca gccccccgag ggactcacgg 2460
   aaatggagga agaggaggaa gaggagcagg acccagggaa gccggccctg ccactctctc 2520
   ccgaggacct ggagagcctg aggagcctcc agcggcagct gcttttccgc cagctgcaga 2580
   agaactcggg ctgggacacg atggggtcag agtcagaggg gcccagtgca tgagggcggc 2640
   tccccaggga ccgcccagat cccagctttg agagaggagt gtgtgtgcac gtattcatct 2700
   gtgtgtacat gtctgcatgt gtatatgttc gtgtgtgaaa tgtaggcttt aaaatgtaaa 2760
   tgtctggatt ttaatcccag gcatccctcc taacttttct ttgtgcagcg gtctggttat 2820
   cgtctatccc caggggaatc cacacagccc gctcccagga gctaatggta gagcgtcctt 2880
   gaggctccat tattcgttca ttcagcattt attgtgcacc tactatgtgg cgggcatttg 2940
   ggataccaag ataaattgca tgcggcatgg ccccagccat gaaggaactt aaccgctagt 3000
   gccgaggaca cgttaaacga acaggatggg ccgggcacgg tggctcacgc ctgtaatccc 3060
   agcacactgg gaggccgagg caggtggatc actctgaggt caggagtttg agccagcctg 3120
<210> 168
   <211> 78
   <212> DNA
   <213> Artificial Sequence
<220>
   <223> human growth hormone signal peptide
<220>
   <221> CDS
   <222> (1)...(78)
<400> 168
   atg gct aca ggc tcc cgg acg tcc ctg ctc ctg gct ttt ggc ctg ctc    48
   Met Ala Thr Gly Ser Arg Thr Ser Leu Leu Leu Ala Phe Gly Leu Leu
    1               5                   10                  15
   tgc ctg ccc tgg ctt caa gag ggc agt gcc                            78
   Cys Leu Pro Trp Leu Gln Glu Gly Ser Ala
               20                  25
<210> 169
   <211> 26
   <212> PRT
   <213> Artificial Sequence
<220>
   <223> human growth hormone signal peptide
<400> 169
   Met Ala Thr Gly Ser Arg Thr Ser Leu Leu Leu Ala Phe Gly Leu Leu
    1               5                   10                  15
   Cys Leu Pro Trp Leu Gln Glu Gly Ser Ala
               20                  25
<210> 170
   <211> 57
   <212> DNA
   <213> Artificial Sequence
<220>
   <223> Mouse Immunoglobulin Heavy Chain Variable Region (VH 26-10) Signal Peptide
<220>
   <221> CDS
   <222> (1)...(57)
<400> 170
   atg gga tgg agc tgg atc ttt ctc ttt ctt ctg tca gga act gca ggt    48
   Met Gly Trp Ser Trp Ile Phe Leu Phe Leu Leu Ser Gly Thr Ala Gly
    1               5                   10                  15
   gtc ctc tct                                                        57
   Val Leu Ser
<210> 171
   <211> 19
   <212> PRT
   <213> Artificial Sequence

<220>
   <223> Mouse Immunoglobulin Heavy Chain Variable Region (VH 26-10) Signal Peptide
<400> 171
   Met Gly Trp Ser Trp Ile Phe Leu Phe Leu Leu Ser Gly Thr Ala Gly
    1               5                  10                  15
   Val Leu Ser
<210> 172
   <211> 48
   <212> DNA
   <213> Artificial Sequence
<220>
   <223> Human CD33 Signal Peptide
<220>
   <221> CDS
   <222> (1)...(48)
<400> 172
   atg ccg ctg ctg cta ctg ctg ccc ctg ctg tgg gca ggg gcc ctg gct   48
   Met Pro Leu Leu Leu Leu Leu Pro Leu Leu Trp Ala Gly Ala Leu Ala
    1               5                  10                  15
<210> 173
   <211> 16
   <212> PRT
   <213> Artificial Sequence
<220>
   <223> Human CD33 Signal Peptide
<400> 173
   Met Pro Leu Leu Leu Leu Leu Pro Leu Leu Trp Ala Gly Ala Leu Ala
    1               5                  10                  15
<210> 174
   <211> 696
   <212> DNA
   <213> Artificial Sequence
<220>
   <223> Fc10 immunoglobulin heavy chain constant region
<220>
   <221> CDS
   <222> (0)...(696)
<400> 174
   gag ccc aaa tct tca gac aaa act cac aca tgc cca ccg tgc cca gca      48
   Glu Pro Lys Ser Ser Asp Lys Thr His Thr Cys Pro Pro Cys Pro Ala
    1               5                  10                  15
   cct gaa ctc ctg ggg gga ccg tca gtc ttc ctc ttc ccc cca aaa ccc      96
   Pro Glu Leu Leu Gly Gly Pro Ser Val Phe Leu Phe Pro Pro Lys Pro
               20                  25                  30
   aag gac acc ctc atg atc tcc cgg acc cct gag gtc aca tgc gtg gtg     144
   Lys Asp Thr Leu Met Ile Ser Arg Thr Pro Glu Val Thr Cys Val Val
           35                  40                  45
   gtg gac gtg agc cac gaa gac cct gag gtc aag ttc aac tgg tac gtg     192
   Val Asp Val Ser His Glu Asp Pro Glu Val Lys Phe Asn Trp Tyr Val
       50                  55                  60
   gac ggc gtg gag gtg cat aat gcc aag aca aag ccg cgg gag gag cag     240
   Asp Gly Val Glu Val His Asn Ala Lys Thr Lys Pro Arg Glu Glu Gln
   65                  70                  75                  80
   tac aac agc acg tac cgt gtg gtc agc gtc ctc acc gtc ctg cac cag     288
   Tyr Asn Ser Thr Tyr Arg Val Val Ser Val Leu Thr Val Leu His Gln
                           85                      90                    95
   gac tgg ctg aat ggc aag gag tac aag tgc aag gtc tcc aac aaa gcc     336
   Asp Trp Leu Asn Gly Lys Glu Tyr Lys Cys Lys Val Ser Asn Lys Ala
               100                 105                 110
   ctc cca gcc ccc atc gag aaa acc atc tcc aaa gcc aaa ggg cag ccc     384
   Leu Pro Ala Pro Ile Glu Lys Thr Ile Ser Lys Ala Lys Gly Gln Pro
           115                 120                 125
   cga gaa cca cag gtg tac acc ctg ccc cca tcc cgg gat gag ctg acc     432
   Arg Glu Pro Gln Val Tyr Thr Leu Pro Pro Ser Arg Asp Glu Leu Thr
       130                 135                 140
   aag aac cag gtc agc ctg acc tgc ctg gtc aaa ggc ttc tat ccc agc     480
   Lys Asn Gln Val Ser Leu Thr Cys Leu Val Lys Gly Phe Tyr Pro Ser
   145                 150                 155                 160
   gac atc gcc gtg gag tgg gag agc aat ggg cag ccg gag aac aac tac     528
   Asp Ile Ala Val Glu Trp Glu Ser Asn Gly Gln Pro Glu Asn Asn Tyr
                   165                 170                 175
   aag acc acg cct ccc gtg ctg gac tcc gac ggc tcc ttc ttc ctc tac     576
   Lys Thr Thr Pro Pro Val Leu Asp Ser Asp Gly Ser Phe Phe Leu Tyr
               180                 185                 190
   agc aag ctc acc gtg gac aag agc agg tgg cag cag ggg aac gtc ttc     624
   Ser Lys Leu Thr Val Asp Lys Ser Arg Trp Gln Gln Gly Asn Val Phe
           195                 200                 205
   tca tgc tcc gtg atg cat gag gct ctg cac aac cac tac acg cag aag     672
   Ser Cys Ser Val Met His Glu Ala Leu His Asn His Tyr Thr Gln Lys
       210                 215                 220
   agc ctc tcc ctg tct ccg ggt aaa                                     696
   Ser Leu Ser Leu Ser Pro Gly Lys
   225                 230
<210> 175
   <211> 232
   <212> .PRT
   <213> Artificial Sequence
<220>
   <223> Fc10 immunoglobulin heavy chain constant region
<400> 175
   Glu Pro Lys Ser Ser Asp Lys Thr His Thr Cys Pro Pro Cys Pro Ala
   1               5                   10                  15
   Pro Glu Leu Leu Gly Gly Pro Ser Val Phe Leu Phe Pro Pro Lys Pro
               20                  25                  30
   Lys Asp Thr Leu Met Ile Ser Arg Thr Pro Glu Val Thr Cys Val Val
           35                  40                  45
   Val Asp Val Ser His Glu Asp Pro Glu Val Lys Phe Asn Trp Tyr Val
       50                  55                  60
   Asp Gly Val Glu Val His Asn Ala Lys Thr Lys Pro Arg Glu Glu Gln
   65                  70                  75                  80
   Tyr Asn Ser Thr Tyr Arg Val Val Ser Val Leu Thr Val Leu His Gln
                   85                  90                  95
   Asp Trp Leu Asn Gly Lys Glu Tyr Lys Cys Lys Val Ser Asn Lys Ala
               100                 105                 110
   Leu Pro Ala Pro Ile Glu Lys Thr Ile Ser Lys Ala Lys Gly Gln Pro
           115                 120                 125
   Arg Glu Pro Gln Val Tyr Thr Leu Pro Pro Ser Arg Asp Glu Leu Thr
       130                 135                 140
   Lys Asn Gln Val Ser Leu Thr Cys Leu Val Lys Gly Phe Tyr Pro Ser
   145                 150                 155                 160
   Asp Ile Ala Val Glu Trp Glu Ser Asn Gly Gln Pro Glu Asn Asn Tyr
                   165                 170                 175
   Lys Thr Thr Pro Pro Val Leu Asp Ser Asp Gly Ser Phe Phe Leu Tyr
               180                 185                 190
   Ser Lys Leu Thr Val Asp Lys Ser Arg Trp Gln Gln Gly Asn Val Phe
           195                 200                 205
   Ser Cys Ser Val Met His Glu Ala Leu His Asn His Tyr Thr Gln Lys
       210                 215                 220
   Ser Leu Ser Leu Ser Pro Gly Lys
   225                 230
<210> 176
   <211> 60
   <212> DNA
   <213> Artificial Sequence
<220>
   <223> linker
<220>
   <221> CDS
   <222> (1)...(60)
<400> 176
   gga ggt ggg ggc tcc ggc ggg ggt gga agc ggt gga ggc ggg tcg ggg     48
   Gly Gly Gly Gly Ser Gly Gly Gly Gly Ser Gly Gly Gly Gly Ser Gly
   1               5                   10                  15
   ggc gga ggt agt                                                     60
   Gly Gly Gly Ser
               20
<210> 177
   <211> 20
   <212> PRT
   <213> Artificial Sequence
<220>
   <223> linker
<400> 177
   Gly Gly Gly Gly Ser Gly Gly Gly Gly Ser Gly Gly Gly Gly Ser Gly
    1               5                  10                  15
   Gly Gly Gly Ser
               20
<210> 178
   <211> 35
   <212> PRT
   <213> Artificial Sequence
<220>
   <223> pre-pro signal sequence from otPA
<400> 178
   Met Asp Ala Met Lys Arg Gly Leu Cys Cys Val Leu Leu Leu Cys Gly
    1               5                  10                  15
   Ala Val Phe Val Ser Leu Ser Gln Glu Ile His Ala Glu Leu Arg Arg
               20                  25                  30
   Phe Arg Arg
           35
<210> 179
   <211> 696
   <212> DNA
   <213> Artificial Sequence
<220>
   <223> Fc5 immunoglobulin heavy chain constant region
<220>
   <221> CDS
   <222> (1)...(696)
<400> 179
   gag ccc aaa tct tca gac aaa act cac aca tgc cca ccg tgc cca gca      48
   Glu Pro Lys Ser Ser Asp Lys Thr His Thr Cys Pro Pro Cys Pro Ala
    1               5                  10                  15
   cct gaa gcc gag ggg gca ccg tca gtc ttc ctc ttc ccc cca aaa ccc      96
   Pro Glu Ala Glu Gly Ala Pro Ser Val Phe Leu Phe Pro Pro Lys Pro
               20                  25                  30
   aag gac acc ctc atg atc tcc cgg acc cct gag gtc aca tgc gtg gtg     144
   Lys Asp Thr Leu Met Ile Ser Arg Thr Pro Glu Val Thr Cys Val Val
           35                  40                  45
   gtg gac gtg agc cac gaa gac cct gag gtc aag ttc aac tgg tac gtg     192
   Val Asp Val Ser His Glu Asp Pro Glu Val Lys Phe Asn Trp Tyr Val
       50                  55                  60
   gac ggc gtg gag gtg cat aat gcc aag aca aag ccg cgg gag gag cag     240
   Asp Gly Val Glu Val His Asn Ala Lys Thr Lys Pro Arg Glu Glu Gln
   65                  70                  75                  80
   tac aac agc acg tac cgt gtg gtc agc gtc ctc acc gtc ctg cac cag     288
   Tyr Asn Ser Thr Tyr Arg Val Val Ser Val Leu Thr Val Leu His Gln
                   85                  90                  95
   gac tgg ctg aat ggc aag gag tac aag tgc aag gtc tcc aac aaa gcc     336
   Asp Trp Leu Asn Gly Lys Glu Tyr Lys Cys Lys Val Ser Asn Lys Ala
               100                 105                 110
   ctc cca tcc tcc atc gag aaa acc atc tcc aaa gcc aaa ggg cag ccc     384
   Leu Pro Ser Ser Ile Glu Lys Thr Ile Ser Lys Ala Lys Gly Gln Pro
           115                 120                 125
   cga gaa cca cag gtg tac acc ctg ccc cca tcc cgg gat gag ctg acc     432
   Arg Glu Pro Gln Val Tyr Thr Leu Pro Pro Ser Arg Asp Glu Leu Thr
       130                 135                 140
   aag aac cag gtc agc ctg acc tgc ctg gtc aaa ggc ttc tat ccc agc     480
   Lys Asn Gln Val Ser Leu Thr Cys Leu Val Lys Gly Phe Tyr Pro Ser
   145                 150                 155                 160
   gac atc gcc gtg gag tgg gag agc aat ggg cag ccg gag aac aac tac     528
   Asp Ile Ala Val Glu Trp Glu Ser Asn Gly Gln Pro Glu Asn Asn Tyr
                   165                 170                 175
   aag acc acg cct ccc gtg ctg gac tcc gac ggc tcc ttc ttc ctc tac     576
   Lys Thr Thr Pro Pro Val Leu Asp Ser Asp Gly Ser Phe Phe Leu Tyr
               180                 185                 190
   agc aag ctc acc gtg gac aag agc agg tgg cag cag ggg aac gtc ttc     624
   Ser Lys Leu Thr Val Asp Lys Ser Arg Trp Gln Gln Gly Asn Val Phe
           195                 200                 205
   tca tgc tcc gtg atg cat gag gct ctg cac aac cac tac acg cag aag     672
   Ser Cys Ser Val Met His Glu Ala Leu His Asn His Tyr Thr Gln Lys
       210                 215                 220
   agc ctc tcc ctg tct ccg ggt aaa                                     696
   Ser Leu Ser Leu Ser Pro Gly Lys
   225                 230
<210> 180
   <211> 232
   <212> PRT
   <213> Artificial Sequence
<220>
   <223> Fc5 immunoglobulin heavy chain constant region
<400> 180
   Glu Pro Lys Ser Ser Asp Lys Thr His Thr Cys Pro Pro Cys Pro Ala
   1               5                   10                  15
   Pro Glu Ala Glu Gly Ala Pro Ser Val Phe Leu Phe Pro Pro Lys Pro
               20                  25                  30
   Lys Asp Thr Leu Met Ile Ser Arg Thr Pro Glu Val Thr Cys Val Val
           35                  40                  45
   Val Asp Val Ser His Glu Asp Pro Glu Val Lys Phe Asn Trp Tyr Val
       50                  55                  60
   Asp Gly Val Glu Val His Asn Ala Lys Thr Lys Pro Arg Glu Glu Gln
   65                  70                  75                  80
   Tyr Asn Ser Thr Tyr Arg Val Val Ser Val Leu Thr Val Leu His Gln
                   85                  90                  95
   Asp Trp Leu Asn Gly Lys Glu Tyr Lys Cys Lys Val Ser Asn Lys Ala
               100                 105                 110
   Leu Pro Ser Ser Ile Glu Lys Thr Ile Ser Lys Ala Lys Gly Gln Pro
           115                 120                 125
   Arg Glu Pro Gln Val Tyr Thr Leu Pro Pro Ser Arg Asp Glu Leu Thr
       130                 135                 140
   Lys Asn Gln Val Ser Leu Thr Cys Leu Val Lys Gly Phe Tyr Pro Ser
   145                 150                 155                 160
   Asp Ile Ala Val Glu Trp Glu Ser Asn Gly Gln Pro Glu Asn Asn Tyr
                   165                 170                 175
   Lys Thr Thr Pro Pro Val Leu Asp Ser Asp Gly Ser Phe Phe Leu Tyr
               180                 185                 190
   Ser Lys Leu Thr Val Asp Lys Ser Arg Trp Gln Gln Gly Asn Val Phe
           195                 200                 205
   Ser Cys Ser Val Met His Glu Ala Leu His Asn His Tyr Thr Gln Lys
       210                 215                 220
   Ser Leu Ser Leu Ser Pro Gly Lys
   225                 230
<210> 181
   <211> 31
   <212> PRT
   <213> Artificial Sequence
<220>
   <223> Murine I1-17RA signal peptide
<400> 181
   Met Ala Ile Arg Arg Cys Trp Pro Arg Val Val Pro Gly Pro Ala Leu
   1               5                   10                  15
   Gly Trp Leu Leu Leu Leu Leu Asn Val Leu Ala Pro Gly Arg Ala
               20                  25                  30